# EUROPEAN PATENT SPECIFICATION

(11) **EP 3 802 850 B1**
(45) Date of publication and mention of the grant of the patent: **19.11.2025**
(21) Application number: 19731515.3
(22) Date of filing: 31.05.2019
(51) Int. Cl.: C12Q 1/18, A61B 10/04, A61B 10/00

(54) **DEVICES AND SYSTEMS FOR GASTROINTESTINAL MICROBIOME DETECTION AND MANIPULATION**
VORRICHTUNGEN UND SYSTEME ZUR DETEKTION UND MANIPULATION DES GASTROINTESTINALEN MIKROBIOMS
DISPOSITIFS ET SYSTÈMES DE DÉTECTION ET DE MANIPULATION DU MICROBIOME GASTRO-INTESTINAL

(30) Priority: 01.06.2018 US 201862679659 P; 26.10.2018 US 201862751209 P; 04.04.2019 US 201962829225 P
(43) Date of publication of application: 14.04.2021
(73) Proprietor: BT Bidco, Inc., San Diego, CA 92131 (US)
(72) Inventor: WAHL, Christopher Loren, San Diego, California 92103 (US); JONES, Mitchell Lawrence, La Jolla, California 92037 (US); SINGH, Sharat, Rancho Santa Fe, California 92067 (US); LEE, Shaoying, View Park, California 90043 (US)
(74) Representative: Mewburn Ellis LLP
(86) International application number: PCT/US2019/034795
(87) International publication number: WO 2019/232295

(56) References cited:
- WO-A1-2017/068576
- WO-A1-2018/106931
- WO-A1-2018/106931
- WO-A1-2018/112441
- WO-A1-2018/112441
- WO-A2-01/11077
- WO-A2-01/11077
- US-A1- 2010 331 641
- US-A1- 2014 163 416
- US-A1- 2018 015 100
- US-A1- 2018 015 100
- US-A1- 2018 049 725
- US-A1- 2018 070 928
- US-A1- 2018 164 221
- US-B2- 7 384 778
- RAZA AUN ET AL: "Oral meropenem for superbugs: challenges and opportunities", DRUG DISCOVERY TODAY, vol. 26, no. 2, 1 February 2021 (2021-02-01), AMSTERDAM, NL, pages 551 - 560, XP055876240, ISSN: 1359-6446, DOI: 10.1016/j.drudis.2020.11.004
- AKIRA OHNO ET AL: "Antibacterial activity and PK/PD of ceftriaxone against penicillin-resistant Streptococcus pneumoniae and [beta]-lactamase-negative ampicillin-resistant Haemophilus influenzae isolates from patients with community-acquired pneumonia", JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 13, no. 5, 30 October 2007 (2007-10-30), pages 296 - 301, XP019546167, ISSN: 1437-7780, DOI: 10.1007/S10156-007-0539-2
- MAJEWSKI MAREK ET AL: "Efficacy of Rifaximin, a Nonabsorbed Oral Antibiotic, in the Treatment of Small Intestinal Bacterial Overgrowth", AMERICAN JOURNAL OF MEDICAL SCIENCES, vol. 333, no. 5, 1 May 2007 (2007-05-01), USA, pages 266 - 270, XP055876288, ISSN: 0002-9629, DOI: 10.1097/MAJ.0b013e3180536784
- RAZA AUN ET AL: "Oral meropenem for superbugs: challenges and opportunities", DRUG DISCOVERY TODAY, vol. 26, no. 2, 1 February 2021 (2021-02-01), AMSTERDAM, NL, pages 551 - 560, XP055876240, ISSN: 1359-6446, DOI: 10.1016/j.drudis.2020.11.004
- AKIRA OHNO ET AL: "Antibacterial activity and PK/PD of ceftriaxone against penicillin-resistant Streptococcus pneumoniae and [beta]-lactamase-negative ampicillin-resistant Haemophilus influenzae isolates from patients with community-acquired pneumonia", JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 13, no. 5, 30 October 2007 (2007-10-30), pages 296 - 301, XP019546167, ISSN: 1437-7780, DOI: 10.1007/S10156-007-0539-2
- MAJEWSKI MAREK ET AL: "Efficacy of Rifaximin, a Nonabsorbed Oral Antibiotic, in the Treatment of Small Intestinal Bacterial Overgrowth", AMERICAN JOURNAL OF MEDICAL SCIENCES, vol. 333, no. 5, 1 May 2007 (2007-05-01), USA, pages 266 - 270, XP055876288, ISSN: 0002-9629, DOI: 10.1097/MAJ.0b013e3180536784
- DATABASE EMBASE [online] ELSEVIER SCIENCE PUBLISHERS, AMSTERDAM, NL; 2001, DERMOUMI H L ET AL: "Isolation and antimicrobial susceptibility testing of fecal strains of the archaeon Methanobrevibacter smithii", Database accession no. EMB-2001145614
- DERMOUMI H L ET AL: "Isolation and antimicrobial susceptibility testing of fecal strains of the archaeon Methanobrevibacter smithii", CHEMOTHERAPY 2001 CH, vol. 47, no. 3, 2001, pages 177 - 183, XP009515282, ISSN: 0009-3157, DOI: 10.1159/000063219
- DATABASE BIOSIS [online] BIOSCIENCES INFORMATION SERVICE, PHILADELPHIA, PA, US; May 2001 (2001-05-01), DERMOUMI HEIDE L ET AL: "Isolation and antimicrobial susceptibility testing of fecal strains of the archaeon Methanobrevibacter smithii", XP002793552, Database accession no. PREV200100235112
- DERMOUMI HEIDE L ET AL: "Isolation and antimicrobial susceptibility testing of fecal strains of the archaeon Methanobrevibacter smithii", CHEMOTHERAPY, vol. 47, no. 3, May 2001 (2001-05-01), pages 177 - 183, XP009515282, ISSN: 0009-3157, DOI: 10.1159/000063219
- DERMOUMI H L ET AL: "Isolation and antimicrobial susceptibility testing of fecal strains of the archaeon Methanobrevibacter smithii", CHEMOTHERAPY 2001 CH, vol. 47, no. 3, 2001, pages 177 - 183, XP009515282, ISSN: 0009-3157
- SINGH SHARAT ET AL: "Sa1717 - Development of a Swallowable Diganostic Capsule to Monitor Gastrointestinal Health", GASTROENTEROLOGY : OFFICIAL PUBLICATION OF THE AMERICAN GASTROENTEROLOGICAL ASSOCIATION, vol. 156, no. 6, 1 May 2019 (2019-05-01), XP085677806, ISSN: 0016-5085, DOI: 10.1016/S0016-5085(19)37784-4

## Description

### RELATED APPLICATIONS

This application claims the benefit of U.S. Provisional Application Nos. 62/679,659, filed June 1, 2018; 62/751,209, filed October 26, 2018; and 62/829,225, filed April 4, 2019.

### TECHNICAL FIELD

The disclosure relates to gastrointestinal (GI) tract microbiome detection and manipulation devices, systems, and methods.

### BACKGROUND

The human gastrointestinal (GI) tract is colonized by a diverse and complex microbial community that includes many different microorganisms, particularly diverse species and strains of bacteria and archaea. These organisms form a commensal community that contributes to the health and well-being of the individual. While each individual is inhabited by their own signature microbial community, the diversity of microorganisms forming this community may have an effect on a plethora of diseases including allergy, diabetes, obesity, arthritis, neurological disorders, and gastrointestinal disorders (e.g., inflammatory bowel disease). For example, the microbial community interacts with the host immune system to educate it in order to form the necessary response mechanisms to combat external pathogens. This interaction is also necessary in preventing the development of autoimmune diseases.

WO 2018/106931 Al discloses gastrointestinal tract detection methods, devices and systems. WO 2018/112441 Al discloses an ingestible device and associated methods. WO 01/11077 A2 discloses methods of diagnosing or treating irritable bowel syndrome and other disorders caused by small intestinal bacterial overgrowth. US 2018/015100 Al describes combination products for the treatment of bacterial infections and method of producing or dosing of same. Raza Aun et al. "Oral meropenem for superbugs: challenges and opportunities", Drug Discovery Today, 1 February 2021, describes the challenges and opportunities of oral meropenem for superbugs. Akira Ohno et al. "Antibacterial activity and PK/PD of ceftriaxone against penicillin-resistant Streptococcus pneumoniae and [beta]-lactamase-negative ampicillin-resistant Haemophilus influenzae isolates from patients with community-acquired pneumonia", Journal of Infection and Chemotherapy, 30 October 2007, describes antibacterial activity and PK/PD of ceftriaxone against penicillin-resistant Streptococcus pneumoniae and [beta]-lactamase-negative ampicillin-resistant *Haemophilus influenzae* isolates from patients with community-acquired pneumonia.

Marek Majewski et al, "Efficacy of Rifaximin, a Nonabsorbed Oral Antibiotic, in the Treatment of Small Intestinal Bacterial Overgrowth", American Journal of Medical Sciences, 1 May 2007, describes the efficacy of rifaximin, a nonabsorbed oral antibiotic in the treatment of small intestinal bacterial overgrowth.

### SUMMARY

The invention is as defined in the appended claims. Hereinafter, the term "embodiment(s)" is to be considered as (an) embodiment(s) of the invention only if it falls within the scope of the claim, otherwise it refers to a mere instance of the disclosure.

The present disclosure relates to methods, devices, and systems for the detection and analysis of the gastrointestinal (GI) tract microbiome. The methods and devices described herein allow for the regio-specific analysis of analytes (e.g., microorganisms) in the GI tract of a subject that form the complex GI tract microbiota. For example, the devices described herein can directly analyze the microbiome of a subject *in vivo,* or can be used to obtain a sample from the GI tract which can then be analyzed *ex vivo.* The ability to obtain samples from specific regions of the GI tract may be particularly advantageous in developing personalized therapies for subjects having a gastrointestinal disorder (GID).

For example, a subject may present to a clinician with one or more symptoms of a GID. Regio-specific samples may be obtained from the GI tract of the subject using a device described herein. These samples can be analyzed *ex vivo* to identify and characterize the microorganisms in the sample. Individual microbial isolates obtained from the sample can be subjected to antibiotic resistance/sensitivity tests which can be used to develop customized antimicrobial regimens to treat the subject. The same device or a different device (e.g., any of the devices described herein) may then be used to administer a therapeutically effective amount of the antimicrobial regimen proximate to, proximal to, or directly onto the specific discrete locations of the GI tract from which the sample was obtained, or to monitor the GI tract of the subject.

In one aspect, this disclosure provides a pharmaceutical formulation for use in a method for treating small intestinal bacterial overgrowth (SIBO) in a subject in need thereof, the method comprising:
orally administering an effective amount of the pharmaceutical formulation, which comprises an antimicrobial agent, to the subject, thereby treating SIBO in the subject, wherein the antimicrobial agent is meropenem or ceftriaxone and wherein the pharmaceutical formulation is either: formulated for oral administration as a solid dosage form with an enteric coating; or is administered in an ingestible device and released into the small intestine.

Subsequent references to methods of treatment by therapy or surgery or *in vivo* diagnosis methods in this disclosure are to be interpreted as references to a pharmaceutical formulation of the invention for use in those methods.

In some embodiments of any of the methods described herein, the method includes determining antimicrobial susceptibility of a microorganism within the gastrointestinal (GI) tract of a subject including identifying an antimicrobial agent that inhibits the growth of the microorganism, wherein the microorganism has been obtained from a sample having been removed from a ingestible device that retrieved the sample from the GI tract of the subject.

In some embodiments of any of the methods described herein, the ingestible device includes a sampling chamber configured to collect the sample from the GI tract of the subject. In some embodiments, the sampling chamber includes the antimicrobial agent (e.g., a composition including one or more antibiotics). In some embodiments of any of the methods described herein, the sampling chamber includes an absorptive material.

In some embodiments of any of the methods described herein, identifying an antimicrobial agent that inhibits the growth of the microorganism includes quantitating and determining the viability of the microorganism obtained from the sample chamber, wherein reduced viability indicates that the microorganism is susceptible to the antimicrobial agent.

In some embodiments of any of the methods described herein, identifying an antimicrobial agent that inhibits the growth of the microorganism includes contacting a plurality of microorganisms derived from the microorganism with the antimicrobial agent for a predetermined period of time; and detecting growth of the plurality of microorganisms, wherein reduced growth in the presence of the microbial agent relative to a reference indicates that the microorganism is susceptible to the antimicrobial agent, and wherein non-reduced growth in the presence of the microbial agent relative to the reference indicates that the microorganism is resistant to the antimicrobial agent. In some embodiments, the reference includes a plurality of microorganisms derived from the microorganism incubated in the absence of the antimicrobial agent for the predetermined period of time.

In some embodiments of any of the methods described herein, the ingestible device includes a microprocessor.

In some embodiments of any of the methods described herein, the ingestible device includes a housing, and the sample chamber is configured within the housing such that, when the device is in the GI tract of the subject, the sample chamber is in selective fluid communication with the GI tract. In some embodiments, the housing is not biodegradable in the GI tract.

In some embodiments of any of the methods described herein, the ingestible device retrieved the sample from the mouth, the throat, the esophagus, the stomach, the rectum, the anus, the sphincter, the duodenum, the jejunum, the ileum, the ascending colon, the transverse colon, or the descending colon of the subject.

In some embodiments of any of the methods described herein, the subject may be a human subject. In some embodiments, the human subject has symptomology of a gastrointestinal disorder. In some embodiments, the human subject is a healthy subject. In some embodiments, the human subject has been diagnosed with a gastrointestinal disorder.

In some embodiments of any of the methods described herein, the microorganism may be a commensal microorganism or a pathogenic microorganism. The microorganism may be a bacterium, an archaeon, a protozoan, a parasite, a virus, or a fungus.

In some embodiments of any of the methods described herein, the microorganism may be a bacterium of a genus selected from the group consisting of *Acetanaerobacterium, Acetivibrio, Aeromonas, Alicyclobacillus, Alkaliphilus, Anaerofustis, Anaerosporobacter, Anaerostipes, Anaerotruncus, Anoxybacillus, Bacillus, Bacteroides, Blautia, Brachyspira, Brevibacillus, Bryantella, Bulleidia, Butyricicoccus, Butyrivibrio, Campylobacter, Catenibacterium, Chlamydiales, Citrobacter, Clostridiales, Clostridium, Collinsella, Coprobacillus, Coprococcus, Coxiella, Deferribacteres, Desulfitobacterium, Desulfotomaculum, Dorea, Eggerthella, Enterobacter, Enterococcus, Escherichia, Erysipelothrix, Erysipelotrichaceae, Ethanoligenens, Eubacterium, Faecalibacterium, Filifactor, Flavonifractor, Flexistipes, Fulvimonas, Fusobacterium, Gemmiger, Geobacillus, Gloeobacter, Haemophilus, Helicobacter, Holdemania, Hydrogenoanaerobacterium, Klebsiella, Kocuria, Lachnobacterium, Lachnospira, Lactobacillus, Lactonifactor, Leptospira, Lutispora, Lysinibacillus, Mollicutes, Moorella, Nocardia, Oscillibacter, Oscillospira, Paenibacillus, Papillibacter, Plesiomonas, Proteus, Pseudoflavoniftactor, Pseudomonas, Robinsoniella, Roseburia, Ruminococcaceae, Ruminococcus, Saccharomonospora, Sarcina, Salmonella, Solobacterium, Shigella, Sporobacter, Sporolactobacillus, Streptococcus, Streptomyces, Subdoligranulum, Sutterella, Staphylococcus, Syntrophococcus, Thermoanaerobacter, Thermobifida, Turicibacter, Veillonella, Vibrio,* and *Yersinia.*

In some embodiments of any of the methods described herein, the microorganism may be a bacterium of a species selected from the group consisting of *Bacteroides fragilis, Bacteroides distasonis, Bacteroides melanogenicus, Bacteroides ovatus, Bacteroides thetaiotamicron, Bacteroides uniformis, Bacteroides urolyticus, Bacteroides vulgatus, Citrobacter diversus, Citrobacter freundii, Citrobacter koseri, Escherichia coli, Enterobacter aerogenes, Klebsiella pneumoniae, Staphylococcus aureus, Shigella dysenteriae, Shigella flexneri, Shigella boydii, Shigella sonnei, Salmonella enterica, Salmonella bongori, Vibrio cholerae, Vibrio vulnificus, Vibrio parahemolyticus, Aeromonas hydrophila, Plesiomonas shigelloides, Prevotella bivia, Prevotella intermedia, Prevotella melanogenica, Proteus mirabilis, Pseudomonas aeruginosa, Haemophilus influenzae, Haemophilus parainfluenzae, Streptococcus agalactiae, Streptococcus mutans, Streptococcus pneumoniae, Streptococcus pyogenes, Enterococcus faecalis, Campylobacter jejuni, Clostridium sporogenes, Helicobacter pylori, Bacillus cereus, Yersinia enterocolitica,* and *Yersinia pseudotuberculosis.*

In some embodiments of any of the methods described herein, the microorganism may be an archeon of a species selected from the group consisting of *Methanobrevibacter smithii, Methanosphaera stadtmanae,* and *Methanobacterium ruminatum.*

In some embodiments of any of the methods described herein, the methods includes inoculating a culture media with a portion of the sample.

In some embodiments of any of the methods described herein, the methods include separating (e.g., isolating individual strains) of the microorganisms in the sample.

In some embodiments of any of the methods described herein, the plurality of microorganisms derived from the microorganism are contacted with the antimicrobial agent in a liquid culture media.

In some embodiments of any of the methods described herein, the plurality of microorganisms derived from the microorganism are contacted with the antimicrobial agent in a solid culture media.

In some embodiments of any of the methods described herein, the antimicrobial agent is impregnated on a device having a concentration gradient of the antimicrobial agent.

In some embodiments of any of the methods described herein, the antimicrobial agent is impregnated on a device having a fixed concentration of the antimicrobial agent.

Also described herein are antimicrobial agents from the group consisting of a betalactam antibiotic, an aminoglycoside, an ansa-type antibiotic, an anthraquinone, an antibiotic azole, an antibiotic glycopeptide, a macrolide, an antibiotic nucleoside, an antibiotic peptide, an antibiotic polyene, an antibiotic polyether, a quinolone, an antibiotic steroid, a sulfonamide, a carbapenem, tetracycline, a dicarboxylic acid, an antibiotic metal, an oxidizing agent, a substance that releases free radicals, a substance that releases active oxygen, a cationic antimicrobial agent, a quaternary ammonium compound, a biguanide, a triguanide, a bisbiguanide, a naturally-occurring antibiotic compound, an analog thereof, a polymer thereof, or a combination thereof. In some embodiments of any of the methods described herein, the methods include administering the ingestible device to a subject.

In some embodiments of any of the methods described herein, the methods include collecting the ingestible device from the subject.

In some embodiments of any of the methods described herein, the methods include removing the sample from the ingestible device.

In some embodiments of any of the methods described herein, the methods include identifying the microorganism. The microorganism may be identified using dark-field microscopy, electron microscopy, microcolony detection by autofluorescence, fluorescence *in situ* hybridization (FISH), flow cytometry, a differential system reactivity assays, 16S ribosomal RNA sequencing, 23S ribosomal RNA sequencing, 18S ribosomal RNA sequencing, whole genome sequencing, *rpoB* gene sequencing, serological testing, PCR, real time PCR, matrix assisted laser desorption ionization time-of-flight (MALDI-TOF), polymerase chain reaction/electrospray ionization mass spectrometry (PCR/ESI-MS), or a combination thereof.

In some embodiments of any of the methods described herein, the methods include quantifying the amount of the microorganism (e.g., dead and/or live cells) present in the sample.

In some embodiments of any of the methods described herein, the methods include determining the viability of the microorganism present in the sample.

In another aspect, the disclosure provides a pharmaceutical formulation for use in a method of preventing regrowth of a bacterium implicated in the pathogenesis of SIBO in a subject in need thereof, the method comprising:
orally administering an effective amount of the pharmaceutical formulation, which comprises an antimicrobial agent, to the subject, wherein the antimicrobial agent exhibits antimicrobial activity against a bacterium implicated in the pathogenesis of SIBO; and
decreasing the amount of bacterium by at least a 3-log reduction in CFU/mL, as compared to the amount of bacterium at the time of beginning administration of the formulation,
wherein exposure of the bacterium to the antimicrobial agent prevents regrowth of the bacterium, wherein the antimicrobial agent is selected from meropenem and ceftriaxone and wherein the pharmaceutical formulation is either: formulated for oral administration as a solid dosage form with an enteric coating; or is administered in an ingestible device and released into the small intestine.

In some embodiments, the method includes identifying an antimicrobial agent that inhibits the growth of the microorganism, wherein the microorganism has been obtained from a sample having been removed from a ingestible device that retrieved the sample from the GI tract of the subject; and administering an effective amount of a pharmaceutical formulation that includes the identified antimicrobial agent to the subject, thereby treating the infection in the subject.

In some embodiments, the bacterium is selected from the group consisting of *E. coli, Streptococcus* spp., *Bacteroides* spp, or any combination thereof.

In some embodiments of any of the methods described herein, the microorganism may be a pathogenic microorganism.

In some embodiments of any of the methods described herein, the microorganism is a protozoan, a bacterium, a parasite, an archeon, or a fungus. In some embodiments, the microorganism is a bacterium of a species selected from the group consisting of *Staphylococcus aureus, Shigella dysenteriae, Shigella flexneri, Shigella boydii, Shigella sonnei, Salmonella enterica, Salmonella bongori, Escherichia coli, Vibrio cholerae, Vibrio vulnificus, Vibrio parahemolyticus, Aeromonas hydrophila, Plesiomonas shigelloides, Campylobacter jejuni, Helicobacter pylori, Bacillus cereus, Yersinia enterocolitica,* and *Yersinia pseudotuberculosis.*

In some embodiments of any of the methods described herein, the ingestible device retrieved the sample from the mouth, the throat, the esophagus, the stomach, the rectum, the anus, the sphincter, the duodenum, the jejunum, the ileum, the ascending colon, the transverse colon, or the descending colon of the subject.

In some embodiments of any of the methods described herein, the ingestible device retrieved the sample from the jejunum of the subject.

In some embodiments of any of the methods described herein, the microorganism may be a bacterium from a genus selected from the group consisting of *Acetanaerobacterium, Acetivibrio, Aeromonas, Alicyclobacillus, Alkaliphilus, Anaerofustis, Anaerosporobacter, Anaerostipes, Anaerotruncus, Anoxybacillus, Bacillus, Bacteroides, Blautia, Brachyspira, Brevibacillus, Bryantella, Bulleidia, Butyricicoccus, Butyrivibrio, Campylobacter, Catenibacterium, Chlamydiales, Citrobacter, Clostridiales, Clostridium, Collinsella, Coprobacillus, Coprococcus, Coxiella, Deferribacteres, Desulfitobacterium, Desulfotomaculum, Dorea, Eggerthella, Enterobacter, Enterococcus, Escherichia, Erysipelothrix, Erysipelotrichaceae, Ethanoligenens, Eubacterium, Faecalibacterium, Filifactor, Flavonifractor, Flexistipes, Fulvimonas, Fusobacterium, Gemmiger, Geobacillus, Gloeobacter, Haemophilus, Helicobacter, Holdemania, Hydrogenoanaerobacterium, Klebsiella, Kocuria, Lachnobacterium, Lachnospira, Lactobacillus, Lactonifactor, Leptospira, Lutispora, Lysinibacillus, Mollicutes, Moorella, Nocardia, Oscillibacter, Oscillospira, Paenibacillus, Papillibacter, Plesiomonas, Pseudoflavonifractor, Proteus, Pseudomonas, Robinsoniella, Roseburia, Ruminococcaceae, Ruminococcus, Saccharomonospora, Sarcina, Salmonella, Solobacterium, Shigella, Sporobacter, Sporolactobacillus, Streptomyces, Subdoligranulum, Sutterella, Staphylococcus, Streptococcus, Syntrophococcus, Thermoanaerobacter, Thermobifida, Turicibacter, Veillonella, Vibrio,* and *Yersinia.*

In some embodiments of any of the methods described herein, the microorganism may be a bacterium from a species selected from the group consisting of *Bacteroides fragilis, Bacteroides distasonis, Bacteroides melanogenicus, Bacteroides ovatus, Bacteroides thetaiotamicron, Bacteroides uniformis, Bacteroides urolyticus, Bacteroides vulgatus, Citrobacter diversus, Citrobacter freundii, Citrobacter koseri, Escherichia coli, Enterobacter aerogenes, Klebsiella pneumoniae, Staphylococcus aureus, Prevotella bivia, Prevotella intermedia, Prevotella melanogenica, Proteus mirabilis, Pseudomonas aeruginosa, Haemophilus influenzae, Haemophilus parainfluenzae, Streptococcus agalactiae, Streptococcus mutans, Streptococcus pneumoniae, Streptococcus pyogenes, Enterococcus faecalis,* and *Clostridium sporogenes.*

In some embodiments of any of the methods described herein, the microorganism may be an archeon (e.g., a methanogenic archeon) from a species selected from the group consisting of *Methanobrevibacter smithii, Methanosphaera stadtmanae,* and *Methanobacterium ruminatum.*

In some embodiments of any of the methods described herein, the ingestible device includes a sampling chamber configured to collect the sample from the GI tract of the subject. In some embodiments of any of the methods described herein, the sampling chamber includes the antimicrobial agent.

In some embodiments of any of the methods described herein, identifying an antimicrobial agent that inhibits the growth of the microorganism includes quantitating and determining the viability of the microorganism obtained from the sample chamber, wherein reduced viability indicates that the microorganism is susceptible to the antimicrobial agent.

In some embodiments of any of the methods described herein, identifying an antimicrobial agent that inhibits the growth of the microorganism includes contacting a plurality of microorganisms derived from the microorganism with the antimicrobial agent for a predetermined period of time; and detecting growth of the plurality of microorganisms, wherein reduced growth in the presence of the microbial agent relative to a reference indicates that the microorganism is susceptible to the antimicrobial agent, and wherein non-reduced growth in the presence of the microbial agent relative to the reference indicates that the microorganism is resistant to the antimicrobial agent. In some embodiments, the reference includes a plurality of microorganisms derived from the microorganism incubated in the absence of the antimicrobial agent for the predetermined period of time.

In some embodiments of any of the methods described herein, the ingestible device includes a microprocessor.

In some embodiments of any of the methods described herein, the ingestible device includes a housing, and the sample chamber is configured within the housing such that, when the device is in the GI tract of the subject, the sample chamber is in selective fluid communication with the GI tract. In some embodiments of any of the methods described herein, the housing is not biodegradable in the GI tract.

In some embodiments of any of the methods described herein, the sampling chamber of the ingestible device includes an absorptive material.

In some embodiments of any of the methods described herein, the subject is a human subject.

In some embodiments of any of the methods described herein, the methods include inoculating a culture media with a portion of the sample.

In some embodiments of any of the methods described herein, the methods include separating the microorganisms in the sample.

In some embodiments of any of the methods described herein, the plurality of microorganisms derived from the microorganism are contacted with the antimicrobial agent in a solid culture media (e.g., an agar plate). In some embodiments of any of the methods described herein, the plurality of microorganisms derived from the microorganism are contacted with the antimicrobial agent in a liquid culture media (e.g., a liquid culture broth).

In some embodiments of any of the methods described herein, the antimicrobial agent is impregnated on a device having a concentration gradient of the antimicrobial agent.

In some embodiments of any of the methods described herein, the antimicrobial agent is impregnated on a device having a fixed concentration of the antimicrobial agent.

Also described herein are antimicrobial agents from the group consisting of a betalactam antibiotic, an aminoglycoside, an ansa-type antibiotic, an anthraquinone, an antibiotic azole, an antibiotic glycopeptide, a macrolide, an antibiotic nucleoside, an antibiotic peptide, an antibiotic polyene, an antibiotic polyether, a quinolone, an antibiotic steroid, a sulfonamide, a carbapenem, tetracycline, a dicarboxylic acid, an antibiotic metal, an oxidizing agent, a substance that releases free radicals, a substance that releases active oxygen, a cationic antimicrobial agent, a quaternary ammonium compound, a biguanide, a triguanide, a bisbiguanide, a naturally-occurring antibiotic compound, an analog thereof, a polymer thereof, and a combination thereof.

Also described herein are antimicrobial agents from the group consisting of a cephalosporin, a quinolone, tetracycline, ampicillin, erythromycin, rifaximin, metronidazole, erythromycin, amoxicillin-clavulanic acid, cefoxitin, ciprofloxacin, norfloxacin, neomycin, doxycycline, lincomycin, chloramphenicol, ertapenem, meropenem, ceftriaxone, piperacillin, and tazobactam.

The antimicrobial agent of the present invention is selected from the group consisting of meropenem or ceftriaxone. Also described herein are antimicrobial agents such as ertapenem, and piperacillin-tazobactam.

In some embodiments of any of the methods described herein, the methods include administering the ingestible device to the subject.

In some embodiments of any of the methods described herein, the methods include collecting the ingestible device from the subject.

In some embodiments of any of the methods described herein, the methods include removing the sample from the ingestible device.

In some embodiments of any of the methods described herein, the methods include identifying the microorganism. The microorganism may be identified using dark-field microscopy, electron microscopy, microcolony detection by autofluorescence, fluorescence *in situ* hybridization (FISH), flow cytometry, a differential system reactivity assays, 16S ribosomal RNA sequencing, 23S ribosomal RNA sequencing, 18S ribosomal RNA sequencing, whole genome sequencing, *rpoB* gene sequencing, serological testing, PCR, real time PCR, matrix assisted laser desorption ionization time-of-flight (MALDI-TOF), polymerase chain reaction/electrospray ionization mass spectrometry (PCR/ESI-MS), or a combination thereof.

In some embodiments of any of the methods described herein, the methods include quantifying the amount of the microorganism present in the sample.

In some embodiments of any of the methods described herein, the methods include determining the viability of the microorganism present in the sample.

Also described herein are pharmaceutical composition that can be administered to the subject orally, rectally, or intravenously. Also described herein are pharmaceutical composition that can be administered to the subject parenterally. In some embodiments of any of the methods described herein, the pharmaceutical formulation may be in a solid dosage form or a liquid dosage form.

The pharmaceutical formulation may be administered in an ingestible device described herein.

The pharmaceutical formulation is released from an ingestible device described herein. In some disclosures, the pharmaceutical formulation is released at the mouth, the throat, the esophagus, the stomach, the rectum, the anus, the sphincter, the duodenum, the jejunum, the ileum, the ascending colon, the transverse colon, or the descending colon of the subject. disclosures, the pharmaceutical formulation is released at a location in the gastrointestinal tract of the subject that is proximate to one or more sites of disease (e.g., the small intestine (e.g., jejunum) of the subject).

In some embodiments of any of the methods described herein, the ingestible device includes a housing, a reservoir containing the pharmaceutical formulation, and a release mechanism for releasing the pharmaceutical formulation from the second ingestible device. In some embodiments, the ingestible device may include a microprocessor. In some embodiments, the housing that is not biodegradable in the GI tract.

The disclosure also provides methods for identifying a microorganism present in the gastrointestinal (GI) tract of a subject, the methods include performing a test to identify the microorganism, wherein the microorganism has been obtained from a sample having been removed from a ingestible device that retrieved the sample from the GI tract of the subject.

In some embodiments of any of the methods described herein, the ingestible device includes a sampling chamber configured to collect the sample from the GI tract of the subject. In some embodiments of any of the methods described herein, the sampling chamber includes an absorptive material. In some embodiments of any of the methods described herein, the ingestible device includes a microprocessor.

In some embodiments of any of the methods described herein, the test to identify the microorganism may be dark-field microscopy, electron microscopy, microcolony detection by autofluorescence, fluorescence in situ hybridization (FISH), flow cytometry, a differential system reactivity assays, 16S ribosomal RNA sequencing, 23S ribosomal RNA sequencing, 18S ribosomal RNA sequencing, whole genome sequencing, *rpoB* gene sequencing, serological testing, PCR, real time PCR, matrix assisted laser desorption ionization time-of-flight (MALDI-TOF), polymerase chain reaction/electrospray ionization mass spectrometry (PCR/ESI-MS), or a combination thereof.

In some embodiments of any of the methods described herein, the ingestible device includes a housing, and the sample chamber is configured within the housing such that, when the device is in the GI tract of the subject, the sample chamber is in selective fluid communication with the GI tract. In some embodiments, the housing is not biodegradable in the GI tract.

In some embodiments of any of the methods described herein, the ingestible device retrieved the sample from the mouth, the throat, the esophagus, the stomach, the rectum, the anus, the sphincter, the duodenum, the jejunum, the ileum, the ascending colon, the transverse colon, or the descending colon of the subject.

In some embodiments of any of the methods described herein, the subject is a human subject. In some embodiments, the human subject has symptomology of a gastrointestinal disorder. In some embodiments, the human subject has been diagnosed with a gastrointestinal disorder.

In some embodiments of any of the methods described herein, the microorganism may be a commensal microorganism or a pathogenic microorganism. In some embodiments, the microorganism may be a bacterium, an archaeon, a protozoan, a parasite, or a fungus.

In some embodiments of any of the methods described herein, the methods include inoculating a culture media with a portion of the sample.

In some embodiments of any of the methods described herein, the methods include separating the microorganisms in the sample.

In some embodiments of any of the methods described herein, the methods include administering the ingestible device to a subject.

In some embodiments of any of the methods described herein, the methods include collecting the ingestible device from the subject.

In some embodiments of any of the methods described herein, the methods include removing the sample from the ingestible device.

In some embodiments of any of the methods described herein, the methods include quantifying the amount of the microorganism (e.g., live and/or dead cells) present in the sample. In some embodiments of any of the methods described herein, the methods include determining the viability of the microorganism present in the sample.

In yet another aspect, the disclosure provides a pharmaceutical formulation for use in a method of preventing the development of resistance to treatment by an antimicrobial agent of a bacterium implicated in the pathogenesis of SIBO in a subject in need thereof, the method comprising:
orally administering an effective amount of a pharmaceutical formulation, which comprises the antimicrobial agent, to the subject, wherein the antimicrobial agent is meropenem or ceftriaxone and wherein the pharmaceutical formulation is either: formulated for oral administration as a solid dosage form with an enteric coating; or is administered in an ingestible device and released into the small intestine, and wherein the bacterium has a spontaneous mutation frequency of less than 7.45×10⁻⁹, 5.75×10⁻⁹, 5.15×10⁻⁹, 9.55×10⁻¹⁰, 1.85×10⁻¹⁰, 1.75×10⁻¹⁰, 1.50×10⁻¹⁰, or 1.05×10⁻¹⁰.

### BRIEF DESCRIPTION OF THE DRAWINGS

Exemplary embodiments of the disclosure are provided below with reference to the drawings.
FIG. 1 shows an ingestible device.
FIG. 2 shows an ingestible device.
FIG. 3 shows a valve.
FIGs. 4 and 5 illustrate operation of a valve.
FIG. 6 shows an ingestible device.
FIG. 7 shows valve designs.
FIG. 8 shows a sampling chamber.
FIG. 9 shows a pumping mechanism.
FIG. 10 shows an ingestible device.
FIG. 11 shows an ingestible device.
FIG. 12 illustrates a valve system.
FIGs. 13A and 13B illustrate a portion of a two-stage valve system in its first and second stages, respectively.
FIGs. 14A and 14B illustrate a portion of a two-stage valve system in its first and second stages, respectively.
FIGs. 15A and 15B illustrate a portion of a two-stage valve system in its first and second stages, respectively.
FIG. 16 illustrates a more detailed view of an ingestible device.
FIGs. 17A-17C illustrate a portion of a three-stage valve system in its first, second and third stages, respectively.
FIGs. 18A-18C illustrate a portion of a three-stage valve system in its first, second and third stages, respectively.
FIGs. 19A-19C illustrate a portion of a three-stage valve system in its first, second and third stages, respectively.
FIG. 20 illustrates a three-stage valve system in its first stage.
FIG. 21A illustrates a portion of an ingestible device.
FIG. 21B illustrates a portion of an ingestible device.
FIG. 22 illustrates an ingestible device.
FIG. 23 illustrates an ingestible device.
FIG. 24 illustrates an ingestible device.
FIG. 25 illustrates an ingestible device.
FIG. 26 is an exploded view of an ingestible device.
FIG. 27 illustrates a portion of an ingestible device.
FIG. 28 illustrates a portion of an ingestible device.
FIG. 29 illustrates a member forming part of a set of five incubation chambers suitable for an ingestible device.
FIG. 30 illustrates a partial cross-sectional view of optics in an ingestible device.
FIG. 31 illustrates components of the optics and flow chamber systems in an ingestible device.
FIG. 32 shows a partial view of an ingestible device
FIGs. 33A, 33B and 33C illustrate operation of ingestible device.
FIG. 34 illustrates an exploded view of the components of ingestible device.
FIG. 35 illustrates an ingestible device.
FIG. 36 illustrates aspects of a mechanism for an ingestible device.
FIG. 37 illustrates an ingestible device.
FIG. 38 illustrates an ingestible device.
FIG. 39 illustrates an ingestible device.
FIGs. 40, 41 and 42 illustrate exemplary anchoring mechanisms of an ingestible device.
FIG. 43 illustrates an ingestible device.
FIG. 44A illustrates a portion of an ingestible device.
FIG. 44B illustrates a partial sectional view of a burst disc holder.
FIG. 45 illustrates an ingestible device.
FIG. 46 illustrates an ingestible device.
FIG. 47 illustrates an ingestible device.
FIG. 48 illustrates an ingestible device.
FIG. 49 illustrates an ingestible device.
FIG. 50 illustrates an ingestible device.
FIG. 51 illustrates an ingestible device.
FIG. 52 illustrates an ingestible device.
FIG. 53 illustrates an ingestible device.
FIG. 54 illustrates an ingestible device.
FIG. 55 illustrates an ingestible device.
FIG. 56 is a view of an ingestible device.
FIG. 57 is an exploded view of an ingestible device.
FIG. 58 is a diagram of an ingestible device during an example transit through a GI tract.
FIG. 59 is a diagram of an ingestible device during an example transit through a jejunum.
FIG. 60 is a flowchart of illustrative steps for determining a location of an ingestible device as it transits through a GI tract.
FIG. 61 is a flowchart of illustrative steps for detecting transitions from a stomach to a duodenum and from a duodenum back to a stomach.
FIG. 62 is a plot illustrating data collected during an example operation of an ingestible device.
FIG. 63 is another plot illustrating data collected during an example operation of an ingestible device.
FIG. 64 is a flowchart of illustrative steps for detecting a transition from a duodenum to a jejunum.
FIG. 65 is a plot illustrating data collected during an example operation of an ingestible device.
FIG. 66 is a plot illustrating muscle contractions detected by an ingestible device over time.
FIG. 67 is a flowchart of illustrative steps for detecting a transition from a jejunum to an ileum.
FIG. 68 is a flowchart of illustrative steps for detecting a transition from a jejunum to an ileum.
FIG. 69 is a flowchart of illustrative steps for detecting a transition from an ileum to a cecum.
FIG. 70 is a flowchart of illustrative steps for detecting a transition from a cecum to a colon.
FIG. 71 shows an embodiment of an exemplary ingestible device including a spectrometer and a separate base station.
FIG. 72 illustrates an exemplary system for collecting, communicating and/or analyzing data about a subject.
FIG. 73 shows an illustrative embodiment of a method for extracting a sample from an ingestible device.
FIG. 74 shows an illustrative embodiment of a method for creating an opening in an ingestible device using a heated lancet, which may be used in conjunction with the method illustrated by FIG. 73.
FIG. 75 shows an illustrative embodiment of a lancing device, which may be used to create an opening in an ingestible device, and may be used in conjunction with the method illustrated by FIG. 73.
FIG. 76 shows an illustrative embodiment of a grating device, which may be used to create an opening in an ingestible device, and may be used in conjunction with the method illustrated by FIG. 73.
FIG. 77 shows an illustrative embodiment of a sleeve device, which may connect the ingestible device to a tube, and may be used in conjunction with the method illustrated by FIG. 73.
FIG. 78 shows an illustrative embodiment of a centrifuge mechanism, which may allow the sleeve device and the ingestible device to be inserted into a centrifuge, and may be used in conjunction with the method illustrated by FIG. 73.
FIG. 79 shows another illustrative embodiment of a centrifuge mechanism, which may allow the sleeve device and the ingestible device to be inserted into a centrifuge, and may be used in conjunction with the method illustrated by FIG. 73.
FIG. 80 shows an illustrative embodiment of a method for separating the ingestible device into multiple portions, and extracting a sample from a portion of the ingestible device.
FIG. 81 shows an illustrative embodiment of an adapter device, which may connect a portion of an ingestible device to a tube, and may be used in conjunction with the method illustrated by FIG. 80.
FIG. 82 illustrates a system for extracting a sample from an ingestible device.
FIG. 83 is a partial exploded view of the system illustrated in FIG. 82.
FIGs. 84A and 84B illustrate views of an ingestible device with holes for allowing sample to exit samples chambers in the device.
FIG. 85 illustrates a cross-sectional view of an ingestible device with holes for allowing sample to exit samples chambers in the device.

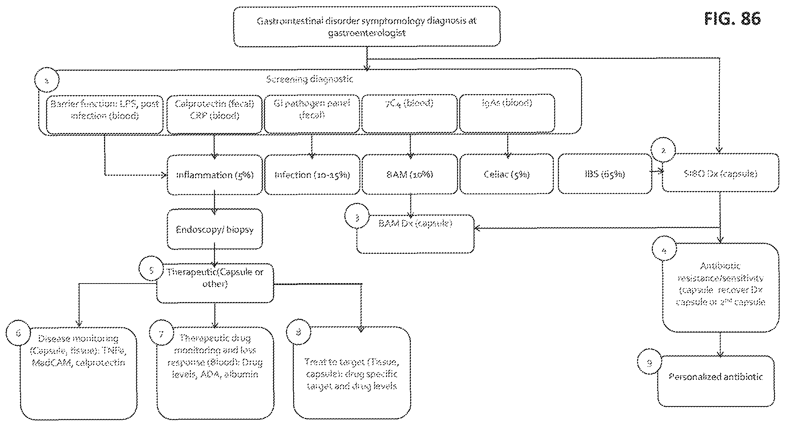

FIG. 86 illustrates an exemplary pathway for the diagnosis and treatment of gastrointestinal disorder symptomology.
FIG. 87 illustrates an exemplary pathway for the diagnosis and treatment of SIBO.

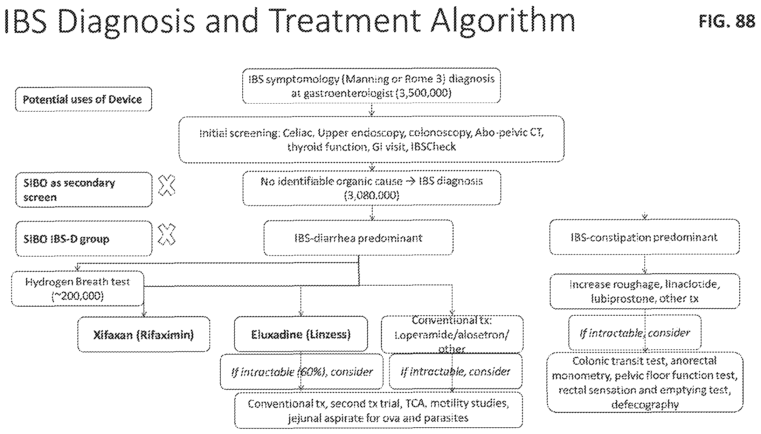

FIG. 88 illustrates an exemplary pathway for the diagnosis and treatment of IBS.

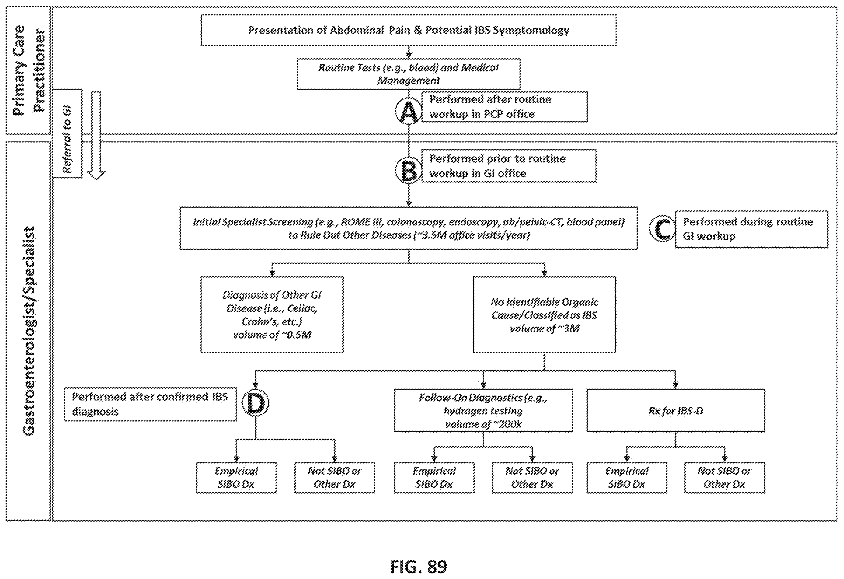

FIG. 89 illustrates an exemplary pathway for the diagnosis and treatment of a subject having IBS symptomology.

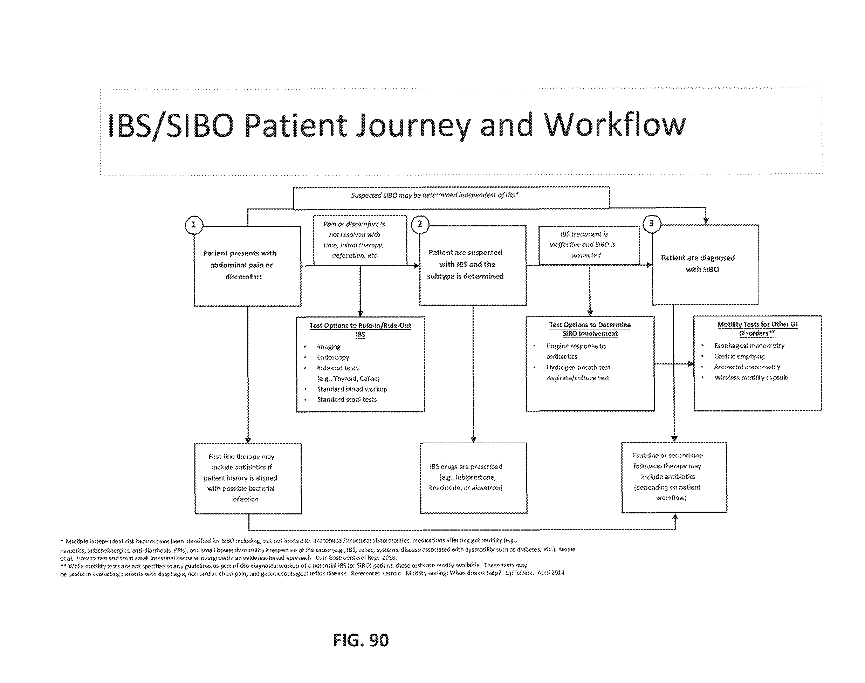

FIG. 90 illustrates an exemplary pathway for the diagnosis and treatment of a patient having abdominal pain or discomfort.
FIG. 91 shows meropenem (MEM), ceftriaxone (CTR), and rifaximin (RFX) MIC distribution against 50 *Enterobacter aerogenes* clinical isolates (µg/mL).
FIG. 92 shows meropenem (MEM), ceftriaxone (CTR), and rifaximin (RFX) MIC distribution against 50 *Escherichia coli* clinical isolates (µg/mL).
FIG. 93 shows meropenem (MEM), ceftriaxone (CTR), and rifaximin (RFX) MIC distribution against 50 *Klebsiella* spp. clinical isolates (µg/mL).
FIG. 94 shows meropenem (MEM), ceftriaxone (CTR), and rifaximin (RFX) MIC distribution against 50 *Proteus mirabilis* clinical isolates (µg/mL).
FIG. 95 shows meropenem (MEM), ceftriaxone (CTR), and rifaximin (RFX) MIC distribution against 50 *Pseudomonas aeruginosa* clinical isolates (µg/mL).
FIG. 96 shows meropenem (MEM), ceftriaxone (CTR), and rifaximin (RFX) MIC distribution against 50 *Staphylococcus aureus* clinical isolates (µg/mL).
FIG. 97 shows meropenem (MEM), ceftriaxone (CTR), and rifaximin (RFX) MIC distribution against 50 *Enterococcus faecalis* clinical isolates (µg/mL).
FIG. 98 shows meropenem (MEM), ceftriaxone (CTR), and rifaximin (RFX) MIC distribution against 54 *Streptococcus* spp. (n = 19 *S. pyogenes,* n = 20 *S. agalactiae* and n = 20 Viridans group *Streptococcus)* clinical isolates (µg/mL).
FIG. 99 shows meropenem (MEM), ceftriaxone (CTR), and rifaximin (RFX) MIC distribution against 19 *Streptococcus pyogenes* clinical isolates (µg/mL).
FIG. 100 shows meropenem (MEM), ceftriaxone (CTR), and rifaximin (RFX) MIC distribution against 20 *Streptococcus agalactiae* clinical isolates (µg/mL).
FIG. 101 shows meropenem (MEM), ceftriaxone (CTR), and rifaximin (RFX) MIC distribution against 15 Viridans group *Streptococci* clinical isolates (µg/mL).
FIG. 102 shows meropenem (MEM), ceftriaxone (CTR), and rifaximin (RFX) MIC distribution against 10 *Clostridium* spp. (n = 4 C. *sporogenes, n* = 2 *C. ramosum* and n = 4 C. *innocuum)* clinical isolates (µg/mL).
FIG. 103 Meropen shows meropenem (MEM), ceftriaxone (CTR), and rifaximin (RFX) MIC distribution against 30 *Prevotella* spp. (n = 5 *P. melaninogenica, n* = 7 *P. bivia, n* = 11 *P. buccae,* and n = 1 each *P. intermedia, P. nanceiensis, P. denticola, P. nigrescens, P. corporis, P. bergensis,* and *P. disiens)* clinical isolates (µg/mL).
FIG. 104 shows meropenem (MEM), ceftriaxone (CTR), and rifaximin (RFX) MIC distribution against 30 *Veillonella* spp. (n = 19 *V. parvula,* n = 9 *Veillonella* spp., n = 1 *Veillonella dispr., n* = 1 *V. atypica)* clinical isolates (µg/mL).
FIG. 105 shows meropenem (MEM), ceftriaxone (CTR), and rifaximin (RFX) MIC distribution against 30 *Bacteroides fragilis* clinical isolates (µg/mL).
FIG. 106 shows meropenem (MEM), ceftriaxone (CTR), and rifaximin (RFX) MIC distribution against 29 *Bacteroides non-fragilis* (n = 3 *B. caccae, n* = 7 *B. thetaiotaomicron, n = 4 B. ovatus,* n = 5 *B. vulgatus, n* = 2 *B. uniformis, n* = 2 *B. stercoris, n* = 1 *B. salversiae, n* = *1 B. intestinalis,* n = 3 *B. xylanisolveris,* and n = 1 *B. faecis)* clinical isolates (µg/mL).
FIGs. 107A-107C show the time-kill kinetics of meropenem (MEM; FIG. 107A), ceftriaxone (CRO; FIG. 107B) and rifaximin (RFX; FIG. 107C) against *E*. *coli* ATCC 25922.
FIGs. 108A-108C show the time-kill kinetics of meropenem (MEM; FIG. 108A), ceftriaxone (CRO; FIG. 108B) and rifaximin (RFX; FIG. 108C) against *E. coli* ATCC 35218.

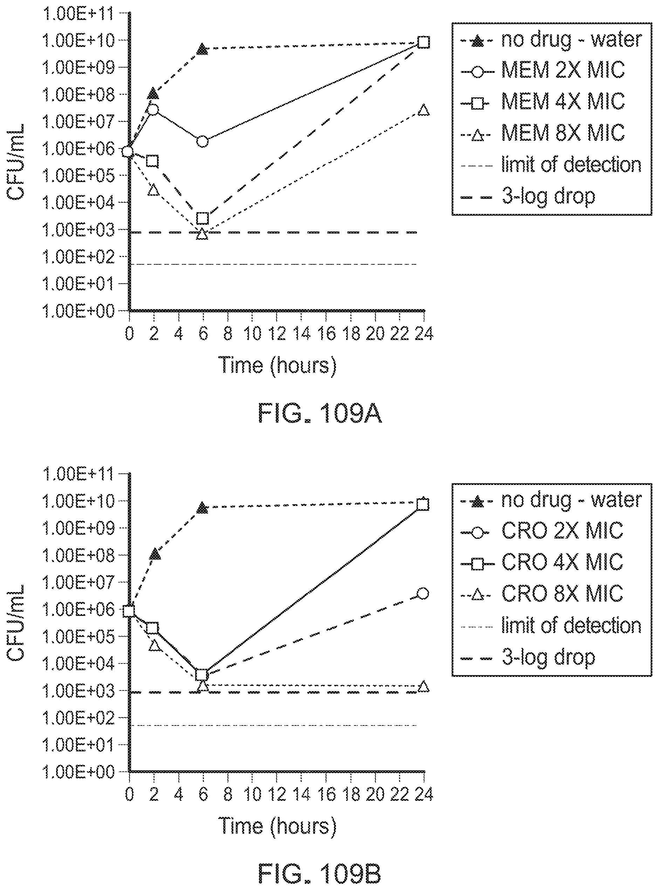

FIGs. 109A-109C show the time-kill kinetics of meropenem (MEM; FIG. 109A), ceftriaxone (CRO; FIG. 109B) and rifaximin (RFX; FIG. 109C) against *E. coli* MMX 1312.
FIGs. 110A-110C show the time-kill kinetics of meropenem (MEM; FIG. 110A), ceftriaxone (CRO; FIG. 110B) and rifaximin (RFX; FIG. 110C) against *S. pneumoniae* ATCC 49619.
FIGs. 111A-111C show the time-kill kinetics of meropenem (MEM; FIG. 111A), ceftriaxone (CRO; FIG. 111B) and rifaximin (RFX; FIG. 111C) against *S. pyogenes* ATCC 49399.
FIGs. 112A-112C show the time-kill kinetics of meropenem (MEM; FIG. 112A), ceftriaxone (CRO; FIG. 112B) and rifaximin (RFX; FIG. 112C) against *S. agalactiae* ATCC 13813.
FIGs. 113A-113B show the time-kill kinetics of meropenem (MEM; FIG. 113A) and rifaximin (RFX; FIG. 113B) against *B. fragilis* ATCC 25285.
FIGs. 114A-114C show the time-kill kinetics of meropenem (MEM; FIG. 114A), ceftriaxone (CRO; FIG. 114B) and rifaximin (RFX; FIG. 114C) against *B. vulgatus* ATCC 8482.
FIGs. 115A-115B show the time-kill kinetics of meropenem (MEM; FIG. 115A) and rifaximin (RFX; FIG. 115B) against *B. ovatus* MMX 3503.

### DETAILED DESCRIPTION

Various apparatuses, systems, devices, components and/or processes will be described below to provide illustrative and non-limiting examples. No embodiment described below limits the subject matter covered by any claim, and any claim may cover processes or apparatuses that differ from those described below. As an example, the subject matter covered by the claims is not limited to apparatuses, systems, devices, components and/or processes having all of the features of any one apparatus, system, device, component and/or process described below or to features common to multiple or all of the apparatuses or processes described below. It is possible that a given apparatus, system, device, component and/or or process described below is not covered by a given claim. Any embodiment disclosed herein that is not covered by one or more claims in this document may be covered by one or more claims in one or more other protective instruments, such as, for example, one or more continuing patent applications and/or one or more divisional patent applications. The Applicants, inventors and/or owners do not necessarily intend to abandon, disclaim or dedicate to the public any subject matter disclosed herein but not covered by a claim herein.

Furthermore, it will be appreciated that for simplicity and clarity of illustration, where considered appropriate, reference numerals may be repeated among the figures to indicate corresponding or analogous elements. In addition, numerous specific details are set forth in order to provide a thorough understanding of the embodiments described herein. However, it is to be understood that the embodiments described herein may be practiced without these specific details. In other instances, well-known methods, procedures and components may have not been described in detail so as not to obscure the embodiments described herein. Also, the description is not to be considered as limiting the scope of the embodiments described herein.

### Definitions

Unless otherwise defined herein, scientific and technical terms used in this disclosure shall have the meanings that are commonly understood by those of ordinary skill in the art. Generally, nomenclature used in connection with, and techniques of, chemistry, cell and tissue culture, molecular biology, cell and cancer biology, neurobiology, neurochemistry, virology, immunology, microbiology, pharmacology, genetics and protein and nucleic acid chemistry, described herein, are those well-known and commonly used in the art.

The methods and techniques of the present disclosure are generally performed, unless otherwise indicated, according to conventional methods well known in the art and as described in various general and more specific references that are cited and discussed throughout this specification.

Chemistry terms used herein are used according to conventional usage in the art, as exemplified by "The McGraw-Hill Dictionary of Chemical Terms," Parker S., Ed., McGraw-Hill, San Francisco, C.A. (1985).

In case of conflict between the disclosures of the publications, patents and published patents referred to herein, the present specification, including its specific definitions, will control.

A "patient," "subject," or "individual" are used interchangeably and refer to either a human or a non-human animal. These terms include mammals, such as humans, primates, livestock animals (including bovine, porcine, etc.), companion animals (e.g., canine, feline, etc.) and rodents (e.g., mice and rats). The term "animal" refers to humans (male or female), companion animals (e.g., dogs, cats and horses), food-source animals, zoo animals, marine animals, birds and other similar animal species. "Edible animals" refers to food-source animals such as cows, pigs, sheep and poultry.

The terms "treating," "treat," or "treatment" embrace both preventative, *i.e.,* prophylactic, and palliative treatment. For example, treating can refer to inhibiting the disease; e.g., inhibiting a disease, condition or disorder in an individual who is experiencing or displaying the pathology or symptomology of the disease, condition or disorder *(i.e.,* arresting further development of the pathology and/or symptomology). Treating can also refer to ameliorating the disease; e.g., ameliorating a disease, condition or disorder in an individual who is experiencing or displaying the pathology or symptomology of the disease, condition or disorder *(i.e.,* reversing the pathology and/or symptomology) such as decreasing the severity of disease. Treating can also include preventing or reducing the risk of developing the disease; e.g., preventing or reducing the risk of developing a disease, condition or disorder in an individual who may be predisposed to the disease, condition or disorder but does not yet experience or display the pathology or symptomology of the disease.

The methods described herein include the use of an ingestible device for detecting markers (e.g., bacteria) associated with symptomology of a functional bowel disorder in a subject who has or is at risk of developing a functional bowel disorder. In some disclosures, the subject has been previously identified as having a functional bowel disorder. In some disclosures, the subject has one or more symptoms of a functional bowel disorder. Some disclosures of any of the methods provided herein further include, prior to the providing an ingestible device step, determining that the subject has a functional bowl disorder. Some disclosures of any of the methods can further include identifying or diagnosing a subject as having a functional bowel disorder.

"Eukaryotic" as recited herein relates to any type of eukaryotic organism excluding fungi, such as animals, in particular animals containing blood, and includes invertebrate animals such as crustaceans and vertebrates. Vertebrates include both cold-blooded (fish, reptiles, amphibians) and warm blooded animal (birds and mammals). Mammals include in particular primates and more particularly humans.

"Microbiota" or "microbiome" as used herein refers to the community of microorganisms present in and on a human subject, including single cell and multicellular eukaryotes such as protozoan, helminthic and fungal eukaryotes, archaea, bacteria, and viruses (including bacterial viruses, i.e., phage).

"Selective lysis" as used in the present disclosure is obtained in a sample when a certain type of cell (e.g., a bacterial and/or archaeal cell (e.g., a Gram-positive or a Gram-negative bacterial cell) or a eukaryotic cell) is preferentially lysed over a different type of cell in the sample (e.g., eukaryotic cell or a bacterial cell). In some disclosures, cells of a particular genera, species or strain are preferentially lysed over cells of a different genera, species or strain. In some disclosures, the percentage of cells of a first genera, species, or strain in the sample that remain intact is significantly higher (e.g., 2, 5, 10, 20, 50, 100, 250, 500, or 1,000 times more) than the percentage of cells of a second genera, species, or strain in the sample that remain intact, upon treatment of or contact with a composition or device as described herein. In some disclosures, the percentage of the bacterial and/or archaeal cells in the sample is significantly lower (e.g., 2, 5, 10, 20, 50, 100, 250, 500, or 1,000 times less) than the percentage of the eukaryotic cells in the sample that remain intact, upon treatment of or contact with a composition or device described herein. In some disclosures, the percentage of bacterial and/or archaeal cells in the sample that remain intact is significantly higher (e.g., 2, 5, 10, 20, 50, 100, 250, 500, or 1,000 times more) than the percentage of the eukaryotic cells in the sample that remain intact, upon treatment of or contact with a composition or device as described herein. In some disclosures, the percentage of Gram-positive bacterial cell in the sample that remain intact is significantly higher (e.g., 2, 5, 10, 20, 50, 100, 250, 500, or 1,000 times more) than the percentage of the Gram-negative bacterial cells in the sample that remain intact, upon treatment of or contact with a composition or device as described herein. In some disclosures, the percentage of Gram-negative bacterial cell in the sample that remain intact is significantly higher (e.g., 2, 5, 10, 20, 50, 100, 250, 500, or 1,000 times more) than the percentage of the Gram-positive bacterial cells in the sample that remain intact, upon treatment of or contact with a composition or device as described herein.

A "sample" as used in the present disclosure may be a biological sample or an environmental sample. Such samples may be obtained from any organism or environmental site desired. For example, the compositions, methods and devices of this disclosure may be used for detecting and quantifying bacterial and/or archaeal cells in a sample obtained from, without limitation, soil, rock, plants, animals, cell or tissue culture, biofilms, organic debris, or water. In some disclosures, samples are obtained from mammals such as humans. In some embodiments, samples are obtained from a human's GI tract. In some disclosures, samples are body fluid samples including, but not limited to urine, blood, plasma, serum, saliva, semen, stool, sputum, cerebral spinal fluid, tears, mucus, and the like. In some disclosures, a single device collects multiple samples, for example, 2, 3, 4, 5, 6, 7, 8, 9, 10, 11, 12, 13, 14, 15, 16, 17, 18, 19, 20, 21, 22, 23, 24, 25, 30, 35, 40, 45, 50, 100 or more samples. In some disclosures, the sample is between 1-2000 µL (e.g., 1-1500 µL, 1-1900 µL, 1-1000 µL, 1-500 µL, 1-250 µL, 1-100 µL, 1-50 µL, 1-10 µL, and 1-5 µL). In some disclosures, samples are tissue samples, for example, from the gastrointestinal tract collected during endoscopy using punch biopsy forceps.

A "colony-forming unit" or "CFU" refers to a unit used to estimate the number of viable bacterial, archaeal and/or fungal cells in a sample. Viable is defined by the cell's ability to divide and form a population (or colony).

As used herein, the term "coupled" indicates that two elements can be directly coupled to one another or coupled to one another through one or more intermediate elements.

The term "saturate" means to permeate or be permeated with a liquid. In some disclosures, an absorptive sponge of the present disclosure may be fully saturated with an amount of a liquid such that no more liquid can be held. In some disclosures, an absorptive sponge of the present disclosure may be partially saturated with a liquid at an amount that is less than the maximum amount of the liquid that can be held by the sponge. For instance, in some disclosures, a sponge is half-saturated with a liquid at half of the maximum amount of the liquid that can be held by the sponge.

The term "semi-solid" means a material that is neither solid (elastic behavior) nor liquid (viscous behavior) and possesses the characteristics of both viscosity and elasticity. Examples of semi-solid materials include gels, ointments, creams, and highly viscous liquids.

As used herein "culturing" refers to maintaining cells in an environment that allows a population of one or more cells to increase in number through cell division. For example, in some disclosures "culturing" may include combining the cells with media in a dilution chamber at a temperature that permits cell growth, optionally a temperature found *in vivo* within the GI tract of a subject. In some disclosures, the cells are cultured at a temperature between about 35 °C and 42 °C. In some disclosures, the cells are cultured at a temperature of about 37 °C.

As used herein "dilution fluid" refers to a fluid within the device for diluting a fluid sample from the GI tract. In some disclosures, the dilution fluid is an aqueous solution. In some disclosures, the dilution fluid includes one or more agents that promote or inhibit the growth of an organism, such as a fungus or bacteria. In some disclosures, the dilution fluid includes one or more agents that facilitate the detection of an analyte, such as dyes or binding agents for analytes.

In some disclosures, a dilution fluid is a sterile media. As used herein, "sterile media" refers to media that does not contain any viable bacteria, archaea, or other cells that would grow and increase in number through cell division. Media may be rendered sterile by various techniques known in the art such as, but not limited to, autoclaving and/or preparing the media using aseptic techniques. In some disclosures, the media is a liquid media. Examples of media suitable for culturing bacteria and/or archaea include nutrient broth, Lysogeny Broth (LB) (also known as Luria Broth), Wilkins chalgren, and Tryptic Soy Broth (TSB). Other growth or culture media known in the art may also be used in the methods and devices described herein. In some disclosures, the media has a carbon source, such as glucose or glycerol, a nitrogen source such as ammonium salts or nitrates or amino acids, as well as salts and/or trace elements and vitamins for microbial growth. In some disclosures, the media is suitable for maintaining eukaryotic cells. In some disclosures, the media includes one or more agents that promote or inhibit the growth of bacteria and/or archaea, optionally agents that promote or inhibit the growth of specific types of bacteria and/or archaea.

In some disclosures, the media is a selective media. As used herein, "selective media" refers to a media that allows certain types of cells to grow and inhibits the growth of other organisms. Accordingly, the growth of cells in a selective media indicates the presence of certain types of cells within the cultured sample. For example, in some disclosures the media is selective for Gram-positive or Gram-negative bacteria. In some disclosures, the media contains crystal violet and bile salts (such as found in MacConkey agar) that inhibit the growth of Gram-positive organisms and allows for the selection and isolation of Gram-negative bacteria. In another disclosures, the media contains a high concentration of salt (e.g., NaCl) (such as found in Mannitol salt agar) and is selective for Gram-positive bacteria. In some disclosures, the media selectively kills eukaryotic cells or only grows prokaryotic cells. In another disclosures, the media selectively kills prokaryotic cells (or alternatively only grows eukaryotic cells), for example, using a media that includes antibiotics.

In some disclosures, the media is an indicator media. As used herein, "indicator media" refers to a media that contains specific nutrients or indicators (such as, but not limited to neutral red, phenol red, eosin y, or methylene blue) that produce a detectable signal when a certain type of cells are cultured in the indicator media.

As used herein, "detecting bacteria and/or archaea" refers to determining the presence or absence of bacteria and/or archaea within a sample or estimating the concentration of bacteria and/or archaea within a sample. For example, in some disclosures, bacterial and/or archaeal growth can be determined based on the concentration of bacteria and/or archaea within a sample. In some disclosures, the detection system detects and/or quantitates a particular bacterial and/or archaeal genus, species or strain within a sample. In some disclosures, the detection system detects the products of bacterial and/or archaeal growth within the cultured and/or diluted sample or a change in concentration of certain components within the media due to bacterial and/or archaeal growth. In some disclosures, products of bacterial and/or archaeal growth include analytes produced and/or secreted by the bacteria and/or archaea that are present in the media, including, but not limited to, bacterial toxins, exosomes, secreted proteins, and metabolites.

A "photosensitizer" as used herein refers to a sensitizer for generation of singlet oxygen usually by excitation with light. Exemplary photosensitizers suitable for use in the present application include those described in U.S. Patent Nos. 6,251,581, 5,516,636, 8,907,081, 6,545,012, 6,331,530, 8,247,180, 5,763,602, 5,705,622, 5,516,636, 7,217,531, and U.S. Patent Publication No. 2007/0059316. The photosensitizer can be photoactivatable (e.g., dyes and aromatic compounds) or chemiactivated (e.g., enzymes and metal salts). When excited by light, the photosensitizer is usually a compound included of covalently bonded atoms, usually with multiple conjugated double or triple bonds. The compound should absorb light in the wavelength range of 200-1100 nm, usually 300-1000 nm, *e.g.,* 450-950 nm, with an extinction coefficient at its absorbance maximum greater than 500 M⁻¹cm⁻¹, *e.g.,* at least 5000 M⁻¹cm-¹, or at least 50,000 M⁻¹cm⁻¹ at the excitation wavelength. The lifetime of an excited state produced following absorption of light in the absence of oxygen will usually be at least 100 nsec, *e.g.,* at least 1 µsec. In general, the lifetime is desirably sufficiently long to permit energy transfer to oxygen, which will normally be present at concentrations in the range of 10⁻⁵ to 10⁻¹³ M depending on the medium. The sensitizer excited state will usually have a different spin quantum number (s) than its ground state and will usually be a triplet (s=l) when, as is usually the case, the ground state is a singlet (s=0). In some disclosures, the sensitizer will have a high intersystem crossing yield. That is, photoexcitation of a sensitizer will produce the long lived state (usually triplet) with an efficiency of at least 10%, at least 40%, *e.g.,* greater than 80%. The photosensitizer will usually be at most weakly fluorescent under the assay conditions (quantum yield usually less than 0.5, or less than 0.1).

As used herein, the term "gastrointestinal tract" or "GI tract" refers to all portions of an organ system responsible for consuming and digesting foodstuffs, absorbing nutrients, and expelling waste. This includes orifices and organs such as the mouth, throat, esophagus, stomach, small intestine, large intestine, rectum, anus, and the like, as well as the various passageways and sphincters connecting the aforementioned parts. The device may be used to detect, analyze and/or quantify an analyte, e.g., bacterial cells, in a sample from the GI tract (e.g., in one or more of the mouth, throat, esophagus, stomach, small intestine, large intestine, rectum, anus, sphincter, duodenum, jejunum, ileum, ascending colon, transverse colon, and descending colon, or subsections thereof, for example the proximal jejunum or terminal ileum) of a subject. The device may also be used to detect or quantify bacterial and/or archaeal cells from outside the GI tract. In some disclosures, the samples from the subject are environmental samples that do not contain eukaryotic cells.

The GI tract is a large organ that extends from the buccal cavity to the anus. The primary function of the GI tract is to digest food, absorb nutrients and eliminated any waste. The GI tract is composed of the esophagus, the stomach, and the intestines. The different segments of the GI tract are generally associated with different characteristics. Chewed food flows through the esophagus, and into the stomach where it is temporarily stored and mixed with gastric acid. Involuntary muscle contractions, termed peristalsis, push the food out of the stomach and into the small intestine. The small intestine can be divided into the duodenum, the jejunum and the ileum. The majority of food digestion and absorption occurs in the ileum. Waste and unwanted products are passed into the colon, or large intestine. Typically, food resides for 10 to 14 seconds in the esophagus, and travels within the small intestine for 2 to 4 hours. Half of the contents of the stomach is emptied within 60 to 90 minutes (Khutoryanskiy (2015) Nature Materials 14:963-964). While food enters the esophagus at approximately pH 7.0, foods are acidified within the stomach (pH 1-5). The pH in the proximal small intestine is between 6.8 and 7.88; between 5.26 and 6.72 in the distal small intestine, between 5.26-6.72 in the ascending colon, and between 5.20 and 7.02 in the descending colon (Khutoryanskiy (2015) Nature Materials 14:963-964).

Over 1000 different microbial species have been identified that can live in the human GI tract, e.g., *Actinobacteria, Bifidobacterium* spp., *Coriobacteriales, Eggerthella, Slackia* spp., *Actinomycetales, Bacteroidetes, Firmicutes, Gemella, Clostridia, Lachnospiraceae, Negativicutes, Fusobacteria,* and fungi (e.g., *Eukarya).* See, e.g., Rajilic-Stojanovic and de Vos (2014) FEMS Microbiol. Rev. 38(5):996-1047; and Carroll et al. (2015) Mamm. Genome 20(7):395-403. Whereas the small intestine contains very few bacteria, the colon comprises between 10¹³ and 10¹⁴ commensal bacteria (Johansson et al. (2013) Nat. Rev. Gastroenterol. Hepatol. 10(6):352-361).

The intestinal fluid can contain a variety of digestive enzymes (e.g., pepsin, lipase, amylase, enterokinase, sucrose, maltase, lactase, secretin, motilin). See, e.g., Ulleberg et al. (2011) Food Dig. 2(1-3):52-61.

As used herein, "minimum inhibitory concentration" or "MIC" refers to the lowest concentration of an antimicrobial agent, for example, an antimicrobial agent as described herein, required to inhibit the growth of an organism. The organism is a bacterium implicated in the pathogenesis of SIBO. "MIC₅₀," as used herein, is the MIC value at which ≥50% of the isolates in a test population are inhibited; it is equivalent to the median MIC value. "MIC₉₀" represents the MIC value at which ≥90% of the strains within a test population are inhibited; the 90th percentile.

"Bactericidal activity," as used herein, refers to a reduction in colony forming units (CFU) per mL after drug (e.g., antimicrobial agent) exposure. In some embodiments, the reduction is a ≥3 log reduction in CFU/mL after about 24 hours of drug (e.g., antimicrobial agent) exposure.

As used herein, "spontaneous mutation frequency" refers to the rate at which mutations arise in a bacterium that can lead to resistance to develop to an antimicrobial agent. "Mutation prevention concentration" or "MPC" refers to the lowest concentration of antimicrobial agent above which the selective proliferation of resistant mutants is expected to occur only rarely.

### Small Intestinal Bacterial Overgrowth

The devices and methods described herein can be used in the diagnosis and/or treatment of small intestinal bacterial overgrowth (SIBO) in a subject. In some embodiments, a subject having or presenting symptoms of SIBO can be diagnosed and/or a course of treatment can be determined for the subject using the methods described herein. The course of treatment comprises orally administering an effective amount of a pharmaceutical formulation comprising an antimicrobial agent to a subject, thereby treating SIBO in the subject.

A "symptom" or "symptomology" of SIBO refers to any morbid phenomenon or departure from the normal in structure, function, or sensation, experienced by a subject and indicative of disease. Exemplary symptoms include, but are not limited to, abdominal pain, abdominal cramps, abdominal discomfort, bloating, flatulence, distension, disturbed bowel function (e.g., constipation, diarrhea, or mixed constipation and diarrhea), excessive mucous in stool, slow bowel transit, nausea, and upper gastrointestinal symptoms (e.g., dyspepsia, heartburn, nausea, or vomiting), poor appetite, blood in stool (e.g., hematochezia or melena), weight loss, fever, abdominal tenderness, gastric stasis, and steatorrhea.

In some embodiments, SIBO presents chronic or semichronic symptomology. In some embodiments, SIBO presents acute symptomology. In some embodiments, the symptoms of SIBO have a temporal duration of about 12 hours, 24 hours, 36 hours, 48 hours, 72 hours, 84 hours, 96 hours, or more. In some embodiments, the symptoms of SIBO have a temporal duration of about 1 day, 2 days, 3 days, 4 days, 5 days, 6 days, 7 days *(i.e.,* 1 week), 2 weeks, 3 weeks, 4 weeks, 5 weeks, 6 weeks, or more. In some embodiments, the symptoms of SIBO have a temporal duration of about 1 month, 2 months, 3 months, 4 months, 5 months, or more. In some embodiments, the symptoms of SIBO have a temporal duration ≥ 6 months (e.g., 6 months, 7 months, 8 months, 9 months, 12 months, 1 year, 2 years, 3 years, or more).

The methods and devices described herein can be used to diagnose, treat and/or monitor subjects having or presenting symptoms of SIBO.

The subject has or is presenting symptoms of small intestinal bacterial overgrowth (SIBO). The small intestine houses less than 10³ bacteria/mL under healthy conditions. When the homeostasis of the gut microbiome is disrupted or aberrant, various functions of the gut microbiota are uncontrolled. See, e.g., Shreiner et al. (2016) Curr. Opin. Gastroenterol. 31(1):69-75; Bures et al. (2010) World J. Gastroenterol. 16(24):2978-2990; and Adike and DiBaise (2018) Gastroenterol. Clin. North Am. 47(1):193-208. Excessive levels of bacteria and/or archaea (e.g., over 10⁵ bacteria /mL) and abnormal types of bacteria and/or archaea in the small intestine may lead to the development of SIBO. SIBO is associated with chronic diarrhea, abdominal discomfort, bloating, malabsorption, flatulence, and unintentional weight loss. While Gram-positive bacteria are typically found in the small intestine, subjects suffering from SIBO have a variety of bacteria in the small intestine including Gram-negative bacteria, which are normally only present in very small numbers or not at all within the small intestine. For example, bacteria present in SIBO may secrete mucosal damaging toxins or metabolize bile salts, which can lead to malabsorption and bloating. A study comparing the prevalence of SIBO in subjects aged 24 to 50 and in subjects aged 61 or older found that SIBO was more prevalent in older subjects as compared to younger subjects (15.6% and 5.9% respectively) (Parlesak et al. (2003) J. Am. Geriatr. Soc. 51(6):768-773). SIBO was also seen more frequently in subjects with reduced body weight. Risk factors for developing SIBO include: metabolic disorders (e.g., diabetes, hypochloryhydria), malnutrition, irritable bowel syndrome (IBS), Celiac disease, Crohn's disease, cirrhosis, renal failure, gastroparesis, small bowel dysmotility, structural abnormalities of the GI tract (e.g., jejunal diverticula), gastric resection and immuno-deficiency. Additional risk factors include the use of certain medications (e.g., antibiotics, gastric acid secretion inhibitors). See, e.g., Dukowicz et al. (2007) Gastroenterol. Hepatol. 3(2):112-122. In some embodiments, subjects having SIBO have delayed intestinal transit times (Cuoco et al. (2002) Hepatogastroenterology 49:1582-1586). In some embodiments, subjects having SIBO have accelerated intestinal transit times (Van Citters and Lin (2006) Clin. Nutrition in Gastrointestinal Disease. Thorofare: Slack Inc; 2006; 271-280).

As used herein, a subject has or is at risk of having SIBO if the subject has intestinal bacteria and/or archaea levels that are greater than 10³ colony forming units (CFU)/ mL, e.g., greater than 10⁴ CFU/ mL, greater than 10⁵ CFU/ mL, greater than 10⁶ CFU/ mL, greater than 10⁷ CFU/ mL, greater than 10⁸ CFU/ mL, greater than 10⁹ CFU/ mL, greater than 10¹⁰ CFU/ mL. In some embodiments, the bacteria are both Gram-positive and Gram-negative bacteria. In some embodiments, the bacteria are Gram-positive bacteria. In some embodiments, the bacteria are Gram-negative bacteria. In some embodiments, the archaea are methane-producing (i.e., methanogenic) archaea.

The prevalence of SIBO in healthy individuals varies from about 0-20% (see, e.g., Lombardo et at. (2010) Clin. Gastroenterol. Hepatol. 8:504-8; Sabaté et al. (2008) Obes. Surg. 18:371-7; Posserud et al. (2007) Gut 56:802-8; Teo (2004) J. Gastroenterol. Hepatol. 19:904-9; Lewis et al. (1999) Age Ageing 28:181-5; Pimentel et al. (2003) Am. J. Gastroenterol. 98:412-9; Rana et al. (2011) Diabetes Technol. Ther. 13:1115-20; Bratten et al. (2008) Am. J. Gastroenterol. 103:958-63; and Scarpellini et al. (2009) J. Pediatr. 155:416-20).

Several clinical conditions are associated with SIBO and are referred to herein as "SIBO-related conditions." Exemplary SIBO-related conditions include, but are not limited to, coeliac disease (see, e.g., Rana et al. (2007) Trop. Gastroenterol. 28:159-61; Rubio-Tapia et al. (2009) J. Clin. Gastroenterol. 43:157-61; and Tursi et al. (2003) Am. J. Gastroenterol. 98:839-43), connective tissue diseases such as scleroderma (see, e.g., Levesque et al. (2009) Rheumatology 48:1314-9; and Parodi et al. (2008) Am. J. Gastroenterol. 103:1257-62), Crohn's disease (see, *e.g.,* Fukushima et al. (1999) Dis. Colon Rectum 42:1072-7; Klaus et al. (2009) Gastroenterol. 9:61; and U.S. Publication No. 2002/0039599), diabetes mellitus (see, e.g., Rana et al. (2011) Diabetes Technol Ther 13:1115-20, and Zaccardi et al. (2009) Eur. Rev. Med. Pharmacol. Sci. 13:419-23), hypothyroidism (see, e.g., Lauritano et al. (2007) J. Clin. Endocr. Metab. 92:4180-4), nonspecific dysmotility (see, e.g., Jacobs et al. (2013) Aliment. Pharmacol. Ther. 37:1103-11), radiation enteropathy (see, e.g., Wedlake et al. (2008) Eur. J Cancer 44:2212-7), ulcerative colitis (see, e.g., Ibanez et al. (2008) Gastroenterology 134:A-350), chronic fatigue syndrome (see, e.g., Ojetti et al. (2009) Eur. Rev. Med. Pharmacol. Sci. 13:419-23), chronic pancreatitis (see, e.g., Mancilla *et al*. (2008) 136:976-80; and Trespi et al (1999) Curr. Med. Res. Opin. 15:47-52), drug-induced inhibition of acid secretion (see, e.g., Jacobs (2013) Aliment. Pharmacol. Ther. 37:1103-11; Compare et al. (2010) Eur. J. Clin. Invest. 41:380-6; and Lombardo et al. (2010) Clin. Gastroenterol. Hepatol. 8:504-8), end-stage renal failure (see, e.g., Strid et al. (2003) Digestion 67:129-37), fibromyalgia (see, e.g., U.S. Publication No. 2002/0039599), irritable bowel syndrome (Posserud et al. (2007) Gut 56:802-8; Bratten et al. (2008) Am. J. Gastroenterol. 103:958-63; 30. Pimentel et al. (2000) Am. J. Gastroenterol. 95:3503-6; Nucera et al. (2005) Aliment. Pharmacol. Ther. 21:1391-5; Lupascu et al. (2005) Aliment. Pharmacol. Ther. 22:1157-60; and Grover et al. (2008) Neurogastroenterol. Motil. 20:998-1008), immunodeficiency syndromes such as **HIV-**infection and chronic lymphocytic leukaemia (see, e.g., Chave et al. Am. J. Gastroenterol. 89:2168-71; and Smith et al. (1990) J. Clin. Pathol. 43:57-9), liver cirrhosis (see, e.g., Yang et al. (1998) Scand. J. Gastroenterol. 33:867-71; and Gunnarsdottir (2003) Am. J. Gastroenterol. 98:1362-70), obesity (see, e.g., Sabaté et al. (2008) Obes. Surg. 18:371-7; and Madrid et al. (2011) Dig. Dis. Sci. 56:155-60), parenteral nutrition (see, e.g., Gutierrez et al. (2012) J. Pediatr. Surg. 47:1150-4), rosacea (Parodi et al. Clin. Gastroenterol. Hepatol. 6:759-64*),* muscular dystrophy (see, e.g., Tarnopolsky et al. (2010) Muscle Nerve 42:853-5), and Parkinson's disease (see, e.g., Gabrielli (2011) Movement Disord. 26:889-92), and coronary artery disease (CAD) (Fialho et al. (2018) Dig. Dis. Sci. 63(2):412-421). **In** a recent study, CAD was significantly more prevalent in patients with SIBO as compared to those without SIBO (78.9% vs. 38.6%, P < 0.001) (Fialho *et al*. (2018)). Moreover, an increased numbers of vessels were affected by CAD among patients with SIBO as compared to those without SIBO. The mechanism by which SIBO is associated with CAD is unknown. Without wishing to be bound by any particular theory, SIBO may contribute to CAD through an increased production of bacterial byproducts such as lipopolysaccharide (LPS) and trimethylamine-N-oxide (TMAO) due to the high bacterial burden in the gut, which may induce a highly inflammatory and proatherogenic state. Microbes convert dietary nutrients that possess a TMA moiety (such as choline, phosphatidylcholine, ybutyrobetaine (yBB), and L-carnitine) into TMAO using specific microbial enzymes (e.g., TMA lyase) via a several metabolic pathways (see Tang and Hazen (2014) J. Clin. Invest. 124:4204-11). TMA is absorbed by the host subject, converted to TMAO by hepatic flavin monooxygenase 3 (FMO3), and excreted by the kidneys. In some embodiments of any of the methods described herein, one or more analytes including TMA, TMAO, carnitine, and ybutyrobetaine are measured in the GI tract of the subject (either *in vivo* using an ingestible device described herein or *ex vivo* in a sample obtained from the GI tract of the subject using an ingestible device described herein). Assays for measuring TMA, TMAO, carnitine, and ybutyrobetaine are known in the art.

In some embodiments of any of the methods described herein, a subject identified as having SIBO is further screened to determine whether the subject has or is at risk of developing a cardiovascular disease. Methods for detecting cardiovascular disease or a risk of developing cardiovascular disease in a subject are known in the art and include, for example, blood pressure, blood tests including a lipid profile, high density cholesterol, low density, cholesterol, triglycerides, cardiac biomarkers (enzymes, proteins, and hormones, such as troponin, myoglobin, b-type natriuretic peptide and creatine phosphokinase, that are associated with heart function, damage or failure), electrocardiograms (ECG or EKG), stress tests, chest x-ray, MUGA scan, computed tomography (CT), nuclear scanning (nuclear heart scan), echocardiogram (heart ultrasound), cardiac catheterization (coronary angiography), duplex/doppler ultrasound, magnetic resonance angiography (MRA) and magnetic resonance imaging (MRI) (see e.g., U.S. Patent Publication No. US 2006/0051873).

Thus, in some embodiments of any of the methods described herein, the subject has or is presenting symptoms of a SIBO-related condition selected from the group consisting of coeliac disease, a connective tissue disease (e.g., scleroderma), Crohn's disease, diabetes mellitus, hypothyroidism, nonspecific dysmotility, radiation enteropathy, ulcerative colitis, chronic fatigue syndrome, chronic pancreatitis, drug-induced inhibition of acid secretion, end-stage renal failure, fibromyalgia, irritable bowel syndrome, an immunodeficiency syndrome (e.g., HIV-infection and chronic lymphocytic leukaemia), obesity, parenteral nutrition, rosacea, muscular dystrophy, Parkinson's disease, and coronary artery disease.

In some embodiments, the SIBO-related condition is an autoimmune disease.

In some embodiments, the SIBO-related condition is selected from the group consisting of irritable bowel syndrome, fibromyalgia, chronic fatigue syndrome, depression; attention deficit/hyperactivity disorder, multiple sclerosis, systemic lupus erythematosus, and Crohn's disease.

In some embodiments, the SIBO-related condition is hyperalgesia.

The skilled medical practitioner is aware of suitable up-to-date diagnostic criteria by which a diagnosis for any of the SIBO-related conditions is made. These diagnostic criteria are based on a presentation of symptom(s) by a human subject. For example, these criteria include, but are not limited to, the Rome criteria for IBS (W. G. Thompson, Lancet 341:1569-72 (1993)) and the criteria for CFS established by the Centers for Disease Control and Prevention (CDC). (K. Fukuda et al., Ann. Intern. Med. 121:953-59 (1994)). The diagnostic criteria for fibromyalgia of the American College of Rheumatology will also be familiar (F. Wolfe et al., Arthritis Rheum. 33:160-72 (1990)), as will be the criteria for depression or ADHD provided for example, by the Diagnostic and Statistical Manual (DSM)-IV or its current version. (e.g., G. Tripp et al., J. Am. Acad. Child Adolesc. Psychiatry 38(2):156-64 (1999)). Symptoms of systemic lupus erythematosus include the 11 revised criteria of the American College of Rheumatology, such as a typical malar or discoid rash, photosensitivity, oral ulcers, arthritis, serositis, or disorders of blood, kidney or nervous system. (E. M Tan et al., Arthritis Rheum. 25:1271-77 (1982)). Appropriate diagnostic criteria for multiple sclerosis are also familiar (e.g., L. A. Rolak, Neuronal Clin. 14(1):27-43 (1996)), as are symptoms of Crohn's disease useful in reaching a suspected diagnosis. (e.g., J. M. Bozdech and R. G. Farmer, Hepatogastroenterol. 37(1):8-17 (1990); M. Tanaka and R. H. Riddell, Hepatogastroenterol. 37(1):18-31 (1990); A. B. Price and B. C. Morson, Hum. Pathol. 6(1):7-29 (1975)). The practitioner is, of course not limited to these illustrative examples for diagnostic criteria, but should use criteria that are current.

The methods described herein may be used to detect SIBO in a subject having a SIBO-related condition.

In some embodiment of any of the methods described herein, the subject is suspected of having SIBO or a SIBO-related condition. In some embodiments of any of the methods described herein, the subject has one or more symptoms selected from the group consisting of bloating, diarrhea, flatulence, stool frequency, abdominal pain, constipation, weight loss, fever, abdominal tenderness, nausea, gastric stasis, and steatorrhea.

In some embodiments of any of the methods described herein, the subject has been subjected to a surgical intervention. For example, SIBO is prevalent in subjects that have undergone abdominal surgery, bilateral vagotomy, gastrectomy, ileocaecal valve resection, and roux-en-Y reconstruction (see, e.g., Grace et al. (2013) Aliment. Pharmacol. Ther. 38(7):674-88). In some embodiment of any of the methods described herein, the subject has been subjected to a surgical intervention selected from the group consisting of abdominal surgery, bilateral vagotomy, gastrectomy, ileocaecal valve resection, and roux-en-Y reconstruction.

In some embodiments of any of the methods described herein, the subject has a GID associated with anomalous bacterial and/or archaeal populations. The bacteria may include, but are not limited to, the types of bacteria and/or archaea present in the fluid sample or the concentration of bacteria and/or archaea in specific regions of the GI tract. Data obtained using the methods described herein may be used to determine whether a subject has an infection, such as small intestinal bacterial overgrowth (SIBO), or to characterize bacterial and/or archaeal populations within the GI tract for diagnostic or other purposes.

In some embodiments, the methods described herein can be used to determine whether a subject has an elevated level of one or more bacterial species and/or strains associated with SIBO in a fluid obtained from the small intestine (e.g., jejunum) of the subject. Bacteria associated with SIBO include, but are not limited to, bacteria of the following genera: *Actinobacillus, Actinomyces, Bacteroides, Campylobacter, Citrobacter, Clostridium, Corynebacterium, Escherichia, Enterobacter, Enterococcus, Fusobacterium, Gemella, Granulicatella, Haemophilus, Klebsiella, Lactobacillus, Lachnoclostridium, Leptotrichia, Megasphaera, Neisseria, Oribacterium, Parascardovia, Porphyromonas, Prevotella, Proteus, Pseudomonas, Ralstonia, Rothia, Staphylococcus, Streptococcus,* and *Veillonella.* Exemplary species of bacterial species associated with SIBO include *Bacteroides fragilis, Bacteroides distasonis, Bacteroides melanogenicus, Bacteroides ovatus, Bacteroides thetaiotamicron, Bacteroides uniformis, Bacteroides urolyticus, Bacteroides vulgatus, Citrobacter diversus, Citrobacter freundii, Citrobacter koseri, Escherichia coli, Enterobacter aerogenes, Klebsiella pneumoniae, Staphylococcus aureus, Prevotella bivia, Prevotella intermedia, Prevotella melanogenica, Proteus mirabilis, Pseudomonas aeruginosa, Haemophilus influenzae, Haemophilus parainfluenzae, Streptococcus agalactiae, Streptococcus mutans, Streptococcus pneumoniae, Streptococcus pyogenes, Enterococcus faecalis,* and *Clostridium sporogenes.* Thus, in some embodiments of the methods described herein, elevated levels of one or more (for example, two or more, three or more, four or more, five or more) bacterial species and/or strains associated with SIBO (either alone or in combination) indicates that a subject has or is at risk of developing SIBO.

In some embodiments, an elevated level of one or more bacterial species and/or strains associated with SIBO is greater than about 10³ colony forming units (CFU)/mL, e.g., greater than about 10⁴ CFU/mL, greater than about 10⁵ CFU/mL, greater than about 10⁶ CFU/mL, greater than about 10⁷ CFU/mL, greater than about 10⁸ CFU/mL, greater than about 10⁹ CFU/mL, or greater than about 10¹⁰ CFU/mL.

In other embodiments, the devices and methods described herein are used to identify, characterize and/or quantify bacteria in the GI tract of a subject (e.g., in vivo or ex vivo) associated with SIBO. In some embodiments, the relative abundance of Proteobacteria phylum and Firmicutes phylum is determined using the devices and methods described herein, wherein a Firmicutes/Proteobacteria ratio (F/P) is lower in SIBO vs. non-SIBO subjects. In another embodiment, bacterial analysis (e.g., characterization or quantification) is done on a sample from the duodenum or the proximal jejunum.

In other embodiments, the devices and methods described herein are used to identify, characterize and/or quantify bacteria in the GI tract of a subject (e.g., in vivo or ex vivo) associated with IBD. For example, *Proteus,* a Gram-negative facultative anaerobic bacilli, has been identified as a key genus in Crohn's disease (CD) recurrence after intestinal resection. Thus, in some embodiments, the relative abundance of *Proteus,* particularly those belonging to the *P. mirabilis* lineages, is determined using the devices and methods described herein, wherein a higher abundance of *Proteus* is seen in CD patients compared to healthy controls.

In yet other embodiments, the devices and methods described herein are used to identify, characterize and/or quantify bacteria in the GI tract of a subject (e.g., in vivo or ex vivo) associated with microscopic colitis. For example, global and *Alistipesfinegoldii-specific* peak-to-trough ratios (PTRs) are significantly higher in active microscopic colitis compared to healthy controls. In multivariable analyses, *Haemophilus parainfluenzae, Veillonella parvula,* and *Veillonella* unclassified species are more abundant in microscopic colitis than healthy controls, while Alistipes putredinis are less abundant in active microscopic colitis patients. Thus, in some embodiments, the global and *Alistipesfinegoldii-specific* peak-to-trough ratios (PTRs) are determined using the devices and methods described herein, wherein higher PTRs are seen in active microscopic colitis compared to healthy controls. In other embodiments, the relative abundance of *Haemophilus parainfluenzae, Veillonella parvula,* and *Veillonella* unclassified species are determined using the devices and methods described herein, wherein a higher abundance of *Haemophilus parainfluenzae, Veillonella parvula,* and *Veillonella* unclassified species are seen in active microscopic colitis compared to healthy controls.

Elevated levels of methane have been observed in subjects having SIBO (referred to as "methane-dominant SIBO" or "methane-predominant SIBO") and other GI disorders such as IBS *(see,* e.g., Low et al. (2010) J. Clin. Gastroenterol. 44(8):547-50; Goettlieb et al. (2016) Aliment Pharmacol Ther.43(2):197-212*;* and Adike and DiBaise (2018) Gastroenterol. Clin. North Am. 47(1):193-208). In some embodiments, the methods described herein may be used to determine the concentration of methane in the GI tract of a subject having SIBO using an ingestible device described herein. In some embodiments, the methods described herein may be used to determine whether a subject has an elevated level of one or more methanogenic archaeal species and/or strains associated with methane-dominant SIBO (e.g., *Methanobrevibacter smithii)* in a sample obtained from the gastrointestinal intestinal tract (e.g., jejunum) of the subject. In some embodiments, an elevated level of one or more methanogenic archaeal species and/or strains associated with SIBO is greater than about 10³ colony forming units (CFU)/mL, e.g., greater than about 10⁴ CFU/mL, greater than about 10⁵ CFU/mL, greater than about 10⁶ CFU/mL, greater than about 10⁷ CFU/mL, greater than about 10⁸ CFU/mL, greater than about 10⁹ CFU/mL, or greater than about 10¹⁰ CFU/mL. Subjects identified as having elevated levels of methane or elevated levels of methanogenic archaea can be treated with an antimicrobial agent that is effective against methanogenic archaea. In some embodiments, the subject can be administered a pharmaceutical formulation comprising at least one antibiotic described herein. Exemplary antibiotics effective against methanogenic archaea include, but are not limited to, lovastatin (e.g., lovastatin lactone), neomycin, rifaximin, and combinations thereof.

### Methods of Diagnosing and Treating a Subject Having SIBO

In some embodiments, the methods described herein include performing one or more assays to determine and/or identify whether a subject having SIBO symptomology has or is at risk of developing SIBO. The assays allow for a skilled practitioner (e.g., a physician) to screen the subject in order to assess the GI tract function of the subject, and the presence and absence of one or more biomarkers associated with SIBO, to accurately diagnose the subject and/or provide an adequate treatment. For example, the assays/tests described herein will allow a skilled practitioner to diagnose a subject presenting with SIBO symptomology based on the identification of specific biomarkers or functional characteristics.

One or more of these assays/tests may be performed before, concurrently, or after commencing an intervention. In some embodiments, one or more of the assays/tests are performed using a sample that has been obtained from the subject (e.g., a fecal, tissue, or blood sample) from the subject. In some embodiments, the subject is administered an ingestible device described herein to obtain a sample from the subject and the assay is performed *ex vivo.* In some embodiments, the subject is administered an ingestible device described herein to obtain a sample from the subject and the assay is performed *in vivo.* In some embodiments, a sample is obtained from the subject without using an ingestible device described herein. In some embodiments, one or more additional tests is performed to screen the subject, including, for example, a colonoscopy, an endoscopy, an abdominal/pelvic CT scan, a colonic transit test, anorectal monometry, pelvic floor function test, rectal sensation and emptying test, defecography, a breath test to detect methane levels, esophageal manometry, gastric emptying, or anorectal manometry.

### Intestinal Barrier Function

In some embodiments, the methods described herein include performing an assay to determine the gastrointestinal barrier function of a subject. The intestinal barrier is a complex system that protects against intestinal luminal content which includes enteric flora, antigens and toxins, while permitting the absorption of nutrients, electrolytes, and water. As used herein, the term "intestinal barrier" refers to the functional system that separates the gut lumen from the subject's internal milieu, and includes mechanical components (e.g., mucus and the epithelial layer), immunological components (e.g., defensins, IgA, lymphocytes, and innate immune cells), muscular components, and neurological components. A defective intestinal barrier may result in increased intestinal permeability, thereby allowing for exposition of luminal content and the triggering of immunological responses that promote intestinal inflammation. Many factors can alter intestinal permeability including gut microbiota changes, mucus layer alterations, and epithelial damage, as well as the consumption of alcohol and energy-dense food (Bischoff et al. (2014) BMC Gastroenterol. 14:189). Generally, altered intestinal barrier function resulting in intestinal permeability is associated with inflammation. For example, altered intestinal permeability has been reported in patients having irritable bowel syndrome, steatoheptatitis, acute pancreatitis, multiple organ failure, major surgery, and trauma (Michielan and D'Inca (2015) Mediators Inflamm. 2015:628157). In addition, altered intestinal permeability plays an important role in the pathogenesis of intestinal inflammation and in the severity of several GFIDs including IBD, Crohn's disease, and ulcerative colitis (see, e.g., Antoni et al. (2014) World J. Gastroenterol. 20(5):1165-79). Altered intestinal barrier function may also be caused by intestinal infection, ingestion of allergenic foods or toxic compounds, deficient secretory IgA, trauma, endotoxemia, and nonsteroidal anti-inflammatory drugs (NSAIDs).

Multiple assays for determining intestinal barrier function are known in the art and are described, for example, in PCT Publication Nos. WO2014/039699, WO2016/036887A1, WO2017/136511A1, and WO2017/173203A1; Bischoff et al. (2014) BMC Gastroenterol. 14:189; and Kelly et al. (2015) Front Cell. Neurosci. 9:392. For example, intestinal barrier function may be measured by assessing biomarkers of epithelial integrity such as soluble adhesion molecules, immunological biomarkers, inflammation biomarkers, or bacterial markers such as circulating endotoxin. In addition, histological and microscopic analyses can be performed to assess intestinal permeability.

In some embodiments, the intestinal barrier function of a subject is determined using a lactulose/mannitol (L/M) test. The L/M test evaluates small intestinal permeability by measuring the urinary excretion of lactulose and mannitol after oral administration. Lactulose is a large disaccharide that only partially absorbed by a healthy gut but it absorption is increased when a subject exhibits decreased intestinal barrier function. Mannitol is a smaller monosaccharide that is easily absorbed since it freely crosses the intestinal epithelium. To perform the test, a subject is orally administered a solution containing both mannitol and lactulose, urine samples are collected over a 24-hour period, and samples are analyzed to detect sugar levels (e.g., using liquid chromatography-tandem mass spectrometry). The analysis of urine samples collected at different time points following administration is used to estimate permeability along the gastrointestinal tract. For example, sugar excretion measured in urine samples collected between 0-2 hours after administration of the reflects small intestine and colon permeability, while sugar excretion in urine samples collected between 6 and 24 hours after administration reflect colonic permeability (see, e.g., Camilleri et al. (2010) Neurogastroenterol. Motil. 22(1):e15-e26). High levels of both sugars in the urine samples is indicative of altered barrier function (e.g., leaky gut syndrome). Low levels of both sugars indicates malabsorption of nutrients by the subject. High levels of mannitol and low levels of lactulose in the samples indicate that the subject has normal intestinal barrier function.

In addition to the dual-sugar M/N test, a multi-sugar assay can be used to determine intestinal barrier function as described, for example, in van Wijck et al. (2013) Clin. Nutr. 32(2):245-51. Intestinal barrier function can also be assessed by measuring the absorption and excretion of polyethylene glycols (PEGs) (see, e.g., Bjarnason et al. (1995) Gastroenterology 108:1566-81). Like sugars, large molecular weight PEGs (400-4000 Da) will only cross the intestinal mucosa when intestinal barrier integrity is compromised. Urinary levels of large molecular weight PEGs can be measured using gas chromatography or high pressure liquid chromatography (HPLC) after oral administration to a subject (e.g., 24 hours post-administration). Increased urinary levels of high molecular weight PEGs is indicative of decreased barrier function (and intestinal permeability) (see, e.g., Grootjans et al. (2010) World J. Gastrointest. Surg. 2:61-69.

In some embodiments, intestinal barrier function can be assessed using a functional test such as the ⁵¹Cr-EDTA test (see, e.g., Jenkins et al. (1988) Clin. Invest. Med. 11(2):151-5*).* ⁵¹Cr-EDTA is easily detectable, has similar physiological properties to oligosaccharides, and is not degraded by the bacterial flora of the colon. In this test, a subject is administered ⁵¹Cr-EDTA, and the excretion of the molecule is monitored in urine samples from the subject. In a healthy gut, ⁵¹Cr-EDTA cannot cross the intestinal epithelia and therefore only minimal levels of the ⁵¹Cr-EDTA penetrate the circulation and can be detected in urine. However, if intestinal barrier function is disrupted, higher levels of ⁵¹Cr-EDTA are detected in urine.

Intestinal barrier function may also be assessed using passive measurements. When intestinal barrier function is compromised, bacteria and bacterial products can be found in the circulation of the subject (e.g., in plasma or serum). In some embodiments, the intestinal barrier function of a subject is assessed by measuring plasma endotoxin (e.g., LPS and/or lipoglycan) levels. Endotoxin is a lipopolysaccharide present in the outer membrane of Gran-negative bacteria. The limulus amebocyte lysate assay (LAL assay) can be used to measure plasma endotoxin levels. LAL is derived from extracts of primitive amebocytes from horseshoe crab. The lipid A component of endotoxin interacts with pro-clotting enzymes in LAL activating a cascade that results in gelation and clot formation. Two common variations of the LAL assay, the turbidometric LAL assay and the chromogenic LAL assay, are commercially available (Endosafe KTA2 lysate (Charles River Laboratories, France); Endochrome-K lysate (Charles River Laboratories, France); QCL-1000 kit; Lonza, Walkersville, MD, USA). Alternatively, plasma or serum endotoxin can be measured using reverse phase HPLC/MS/MS as described in Pais de Barros et al. (2015) J. Lipid Res. 56(7):1363-9.

Intestinal barrier function may also be assessed by measuring the concentration of immunoglobulins (e.g., IgG, IgM, or IgA) against the inner core of endotoxin, also known as "endotoxin core antibodies" (EndoCAb), in the whole blood, plasma and/or serum of a subject. In some embodiments, the concentration of EndoCAb can be measured using an enzyme-linked immunosorbent assay (ELISA) (see, e.g. Bennett-Guerrero et al. (1999) JAMA 277(8):646-50; Hamilton-Davies et al. (1997) Chest 112(5):1189-1196, 1997; Barclay (1995) "Endogenous Endotoxin-Core Antibody (EndoCAb) as a Marker of Endotoxin Exposure: A Review," In Levin et al., eds., "Bacterial Endotoxins: Lipopolysaccharides From Genes to Therapy," New York, NY, Wiley-Liss, 263-272; Barclay et al. (1987) Infect. Immun. 55:2706-14; and U.S. Patent Publication No. US 2003/0190313 A1). Immunoassay kits for the detection of EndoCAb are commercially available (DiaPharma EndoCAb kit, DiaPharma Group, Inc., West Chester, Ohio, USA; HBT Endocab test kit HK504, Canton, MA, USA).

Intestinal barrier function may be assessed by measuring the plasma levels of D-lactate. D-lactate is a fermentation product produced by many intestinal bacteria. In healthy subjects, circulating levels of D-lactate are low; however, when intestinal barrier function is compromised, increased circulating levels of D-lactate may be observed as a consequence of increased translocation of the compound across the intestinal barrier. Levels of D-lactate can be measured using a coupled enzymatic reaction utilizing D-lactate dehydrogenase and alanine aminotransferase, and measuring the formation of NADH as described in Fürst and Schiesser (1999) Anal Biochem. 269:214-5. Additional methods for analyzing plasma D-lactate levels have been described, for example, in Herrera et al. (2008) Ann. Clin. Biochem. 45(Pt. 2):177-83.

Intestinal barrier function of a subject may also be assessed using imaging techniques such as confocal laser endomicroscopy. Confocal laser endomicroscopy allows for the evaluation of gastrointestinal epithelial lining and vasculature using a molecular contrast agent (e.g., fluorescein) (see, e.g., Becker et al. (2008) Gastrointest. Endosc. 68(2):319-23; Kiesslich et al. (2007) Gastroenterol. 133(6):1769-78; and Liu et al. (2011) J. Clin. Gastroenterol. 45(3):240-5). In some embodiments, the intestinal barrier function of a subject is assessed by measuring autoantibody levels.

### Calprotectin

In some embodiments, the methods described herein include performing an assay to determine the concentration of calprotectin present in a fecal sample from a subject. Calprotectin (S100A8/A9) is a calcium and zinc-binding protein generally found in the cytosol of human neutrophils and macrophages. During cell stress or damage, calprotectin can be detected in stool. Therefore calprotectin levels are considered sensitive marker of intestinal inflammation.

Although fecal calprotectin is elevated in subjects having inflammatory bowel disease (e.g., ulcerative colitis and Crohn's disease), it is not a specific marker for IBD. Any inflammatory process within the gastrointestinal tract results in the activation of the innate immune response and calprotectin release (Smith and Gaya (2012) World J Gastroenterol. 18(46):6782-9 and Poullis et al. (2003) J. Gastroenterol. Hepatol. 18:756-762). Fecal calprotectin levels are useful in detecting active IBD and predicting recurrence of disease (see Angriman et al. (2007) Clin. Chim. Acta 381:63-8). Calprotectin is very suitable as a biomarker of inflammation since it is generally resistant to bacterial degradation in the gut, and is stable in stool samples at room temperature for about one week. Fecal calprotectin can be detected by methods known in the art, including the PhiCal^{®} Fecal Calprotectin Immunoassay (Genova Diagnostics, Inc., Asheville, NC, USA; Calpro AS, Oslo, Norway) which has been approved in the U.S.A. for use in diagnosing IBD. Additional human calprotectin detection kits are also commercially available including IDK^{®} Calprotectin ELISA, Immunodianostik AG, Bensheim, Germany; and Human Calprotectin ELISA Kit, Cell Sciences^{®}, Canton, Mass., USA.

Fecal calprotectin levels may be analyzed by using the PhiCal^{®} Fecal Calprotectin Immunoassay, as instructed by the manufacturer. Calprotectin levels below about 50 µg per gram of stool are indicative of no inflammation in the GI tract. Fecal calprotectin levels between about 50 to about 120 µg/g stool are associated with low-grade inflammation, which may be caused by post-infectious irritable bowel syndrome (IBS), infection, food allergies, polyps, neoplasia, non-steroidal anti-inflammatory drugs (NSAIDs) or IBD in remission. Fecal calprotectin levels above about 120 µg/g stool indicates significant inflammation which may be caused by IBD, infection, food allergies, NSAID use, polyps, adenomas, colorectal cancer, or diverticulitis. Fecal calprotectin levels greater than about 250 µg/g stool indicate a strong likelihood that the subject has active IBD or is at a high risk of relapse to active IBD within one year. In subjects that have been diagnosed with IBD, fecal calprotectin levels between 250-500 µg/g stool indicates low to moderate disease activity, and fecal calprotectin levels above 500 µg/g stool suggest high disease activity. Subjects with IBD in remission and fecal calprotectin levels above 250 µg/g stool have a high risk of relapse within one year.

### C-reactive protein (CRP)

In some embodiments, the methods described herein include performing an assay to determine the concentration of C-reactive protein present in a sample (e.g., blood, serum or plasma) from a subject. CRP (C-reactive protein) is an acute inflammatory protein biomarker that dramatically increases (up to 1,000-fold) at sites of inflammation or infection. Thus, in some embodiments, CRP levels may be used to determine whether a subject has inflammation. In some embodiments, CRP levels may be used to determine whether a subject has an infection. The protein is produced as a homopentameric protein referred to as native CRP (nCRP), but can dissociate into five monomers, called monomeric CRP (mCRP) (Sproston and Ashworth (2018) Front. Immunol. 9:754. Healthy subjects have low levels of CRP in circulation (less than 1 mg/L) but levels can rise 100-fold (even reaching 300-400 mg/L) in periods of acute inflammation (Chang et al. (2015) World J. Gastroenterol. 21(40):11246-59.

CRP levels may be determined using a less sensitive standard immunoassay (e.g., ELISA) that is useful for monitoring general inflammatory changes in patients where CRP levels are higher than 10 mg/L. Several of these immunoassays are commercially available including the Quantikine^{®} human CRP Immunoassay kit (R&D Systems^{®}, Minneapolis, MN, USA). A second type of detection assay referred to as a high sensitivity C-reactive protein test (hs-CRP) has a higher detection sensitivity and measures low levels of CRP. Blood or serum CRP levels less than 10 mg/L are typically tested using hs-CRP tests. Exemplary hs-CRP tests include the Cardio IQ^{®} hs-CRP (Quest Diagnostics^{®}, Seacacus, NJ, USA) CRP levels persistently above 10 mg/L may indicate an acute inflammatory process in a subject.

### 7a-hydroxy-4-cholesten-3-one (7αC4)

In some embodiments, the methods described herein include performing an assay to determine the concentration of 7α-hydroxy-4-cholesten-3-one (7αC4) present in a sample (e.g., blood, serum or plasma) from a subject. The measurement of 7αC4 allows for the monitoring of the enzymatic activity of hepatic cholesterol 7α-hydroxylase, the rate limiting enzyme in the synthesis of bile acids and can be used as a surrogate to detect bile acid malabsorption (BAM) (see, e.g., Galman et al. (2003) J. Lipid. Res. 44:859-66; and Camilleri et al. (2009) Neurogastroeterol. Motil. 21(7):734-43).

Bile acids are products of cholesterol synthesis that are synthesized in the liver, conjugated to taurine or glycine, and stored in the gallbladder until released into the small intestine. The primary bile acids are cholic acid, and chenodeoxycholic acid, which are deconjugated and dehydroxylated by intestinal bacteria to form the secondary bile acids deoxycholic acid and lithocholic acid, respectively. The majority of bile acids (about 95%) are reabsorbed in the distal ileum and returned to the liver (see, *e.g.,* U.S. Publication No. 2017/0343535). Impaired absorption of bile acids in the ileum can lead to excess bile acids in the colon which can cause symptoms of bile acid malabsorption (BAM; also known as bile acid diarrhea), including watery stool and fecal incontinence. Interestingly, up to 50% of patients with irritable bowel syndrome with diarrhea (IBS-D) also have BAM (see, e.g., Camilleri et al. (2009) Neurogastroeterol. Motil. 21(7):734-43).

In some embodiments, 7αC4 levels may be used to determine whether a subject has bile acid malabsorption. Serum 7αC4 concentration above about 65 ng/mL (e.g., above about 65.5 ng/mL, above about 66.0 ng/mL, above about 66.5 ng/mL, above about 67.0 ng/mL, above about 67.5 ng/mL, above about 68.0 ng/mL, above about 68.5 ng/mL, above about 69.0 ng/mL, above about 69.5 ng/mL, above about 70.0 ng/mL, above about 70.5 ng/mL, above about 71.0 ng/mL, above about 72.0 ng/mL, above about 73.0 ng/mL, above about 74.0 ng/mL, and above about 75.0 ng/mL) are indicative of bile acid malabsorption (see, e.g., Camilleri et al. (2009) Neurogastroenterol. Motil. 21(7):734-e43; and Sauter et al. (1999) Dig. Dis. Sci. 44(1):14-9). In some embodiments, the concentration of 7αC4 present in the blood, serum or plasma from the subject is analyzed using a high pressure liquid chromatography-tandem mass spectrometry (HPLC-MS/MS) assay as described in Camilleri et al. (2009) Neurogastroeterol. Motil. 21(7):734-43; or Honda et al. (2007) J. Lipid Res. 48(2):458-64. In some embodiments, subjects identified as having serum 7αC4 concentration above about 65 ng/mL indicates that the subject has bile acid malabsorption.

In some embodiments, the devices, methods and compositions described herein can be used to detect, quantitate, and/or analyze the presence and/or level of 7αC4 in the GI tract of a subject.

### Celiac Disease-Related Antibodies

In some embodiments, the methods described herein include performing a serological test (e.g., an ELISA) to detect and/or quantitate one or more of: anti-gliadin antibodies (AGA), anti-endomysial antibodies (EmA), anti-tissue transglutaminase antibodies (tTGA), anti-deamidated gliadin peptide antibodies (DGPA), and total serum IgA in a sample (e.g., blood, serum or plasma) from a subject. In some embodiments, AGA, EmA, tTGA, or DGPA are IgG antibodies (e.g., IgG AGA, IgG EmA, IgG tTGA, and IgG DGPA). In some embodiments, the AGA, EmA, tTGA, or DGPA are IgA antibodies (e.g., IgA AGA, IgA EmA, IgA tTGA, and IgA DGPA). AGA, EmA, tTGA, and DGPA are serological markers of Celiac disease. Thus, the detection and/or quantitation of these antibodies may be used to determine whether a subject has or is at risk of developing Celiac disease.

Serology assays are often used to diagnose Celiac disease in a subject (Dieterich et al. (1998) Gastroenterology 115:1317-21). Serology assays to detect IgA tTGA are considered the best strategy for Celiac disease serological screening as they are highly sensitive (up to 97%) (Volta and Villanacci (2011) Cell. Mol. Immunol. 8(2):96-102). Serology assays to detect IgA EmA are typically employed as confirmation tests in subjects that are positive for IgA tTGA due to their higher specificity (about 100 %, as compared to about 91% of the IgA tTGA assays) (Volta and Villanacci (2011)).

Serology assays to detect IgA AGA are recommended for the diagnosis of Celiac disease in young children (under 2 years of age) (Lagerqvist et al. (2008) J. Pediatr. Gastroenterol. Nutr. 47:428-35), while assays to detect IgG tTGA are recommended for detecting Celiac disease in subjects with an IgA deficiency (Cataldo et al. (1998) Gut 42:362-65).

Recently, serology assays to detect IgG and IgA DGPA have been developed. These assays have a lower sensitivity for Celiac disease than assays for IgA tTGA. Moreover, IgG DGPA serology assays show very high specificity for Celiac disease, and allow for the identification of the disease in most Celiac disease cases, including subjects having IgA-deficiency and children under 2 years of age (Volta et al. (2010) J. Clin. Gastroenterol. 44:186-19.

Kits for the detection and/or quantitation of IgG AGA, IgG EmA, IgG tTGA, IgG DGPA, IgA AGA, IgA EmA, IgA tTGA, and IgA DGPA are commercially available, for example from INOVA Diagnostics, San Diego, Calif. (see also, Prince (2006) Clin. Vaccine Immunol. 13(1):150-1; and U.S. Publication No. 2009/0176251 A1).

### GI Pathogen Screening

In some embodiments, the methods described herein include performing an assay to detect the presence or identity of a microorganism (e.g., a GI pathogen) in a sample from the subject (e.g., a fecal or blood sample). Any method known in the art may be used as described herein to assess whether a subject presenting a symptom of a GID has an infection with a GI pathogen (e.g., bacterial pathogens, protozoans, parasites, fungi, and viruses). Exemplary GI pathogens include, but are not limited to, a bacteria of one of the following genera: *Staphylococci* (e.g., *Staphylococcus aureus), Shigella* (e.g., *Shigella dysenteriae, Shigella flexneri, Shigella boydii,* and *Shigella sonnei), Salmonella* (e.g., *Salmonella enterica* and *Salmonella bongori), Escherichia* (e.g., *Escherichia coli* including enterotoxigenic *E. coli* (ETEC), enteroinvasive *E. coli* (EIEC), enteropathogenic *E. coli* (EPEC), or enterohemorrhagic *E. coli* (EHEC)), *Vibrio* (e.g., *V. cholerae, V. vulnificus* and *V. parahemolyticus), Aeromonas* (e.g., *Aeromonas hydrophila), Plesiomonas* (e.g., *Plesiomonas shigelloides), Campylobacter* (e.g., *Campylobacter jejuni), Clostridium* (e.g., *Clostridium difficile), Helicobacter* (e.g., *Helicobacter pylori), Bacillus* (e.g., *Bacillus cereus),* and *Yersinia* (e.g., *Y. enterocolitica* and *Y. pseudotuberculosis);* viruses, such as rotavirus A, norovirus GI/GII. and adenovirus 40/41; and parasites such as *Cryptosporidium* spp., *Entamoeba histolytica* and *Giardia lamblia.* For example, in some embodiments, the presence of a GI pathogen in a sample from the subject is detected using the xTAG^{®} gastrointestinal pathogen panel (GPP) assay (Luminex Corporation, Austin, TX, USA; see also luminexcorp.com/clinical/infectious-disease/gastrointestinal-pathogen-panel/). The GPP assay is a multiplexed nucleic acid test that detects up to 15 different pathogens that are responsible for gastroenteritis, including bacteria *(Salmonella* spp., *Shigella* spp., *Vibrio cholerae, Yersinia enterocolitica, Campylobacter* spp., *Clostridium difficile, Escherichia coli* 0157, Shiga toxin-producing *E. coli* and enterotoxigenic *E. coli),* viruses (rotavirus A, adenovirus 40/41 and norovirus GI/GII) and parasites *(Cryptosporidium* spp., *Entamoeba histolytica* and *Giardia lamblia).*

For example, traditional culture methods enable the identification and semi-quantitation of specific organisms through the utilization of differential growth media and solid plates, while microorganism classification and/or identification can be performed using Analytical Profile Index (API^{®}) strips (bioMerieux, Marcy l'Etoile, France; see also biomerieux-usa.com/clinical/api). This can also be accomplished using automated biochemical instruments such as Vitek. Other methods and techniques that can be used include mass spectrometry methods such as MALDI-TOF-MS (Matrix-assisted laser desorption/ionization time-of-flight mass spectrometry), which uses species- and strain-specific biomarkers to identify organisms. Microarray technology can also be used to evaluate nucleic acids, such as 16s rRNA, small peptides or molecules, such as toxins, that are specific for the microorganism, or carbohydrate (e.g., polysaccharide) profiles. Nucleic acid probe technology can also be used in combination with fluorescent microscopy for microbial quantification and identification. Other methods for identifying GI pathogens include nucleic acid amplification by PCR and RNA or DNA sequencing.

For example, Sanger sequencing or next generation sequencing (NGS) can be used to perform rapid microbial identification using 16S-23S rRNA regions or internal transcribed spacer regions (ITS) or whole genome sequencing (e.g., shotgun sequencing) can be performed (see, e.g., Deurenberg *et al*. (2017) *J*. *Biotech.* 243:16-24). Microorganisms can be identified through sequencing by identification of either segments of genetic material or the entire genome. In particular, ribosomal RNA (rRNA) sequences have been identified as a target for determining the phylogeny of an organism because it is present in all cells. Prokaryotic rRNA contains three main segments: 5S (~120 nt), 16S (~1.5 kb), and 23S (~2.9 kb). Bacterial identification can be achieved by sequencing the 16S small subunit (SSU) and 23S large subunit (LSU), which can be performed using either Sanger sequencing or next generation sequencing (see Sabat et al. (2017) Sci. Rep., 7(1), 3434). This enables identification of bacterial organisms based on sequencing these rRNA segments and comparing to existing rRNA databases, such as the Ribosomal Database Project (RDP-II) or SILVA (see, e.g., Cole et al. (2003). Nucleic Acids Res., 31(1):442-443; and Pruesse et al. (2007) Nucleic Acids Research 32(21):7188-96). The sequenced prokaryotic DNA or RNA can then be identified using pre-prepared libraries such as k-mer or BLAST (Deurenberg *et al*. (2017)). Various systems and products for the preparation of sample, sequencing, and analysis of genomic sequences derived from microbial organisms are commercially available including, for example, the Nextera^{®} XT DNA Library Preparation Kit, the MiSeq^{®} System and Reagent Kits, and the 16S Metagenomics software (all produced by Illumina^{®}, Inc.) and the Ion PGM^{™} (ThermoFischer) (see also Rapin et al. (2017) Curr. Protoc. Mouse Biol. 7(2):100-29).

Microbial whole genome sequencing is particularly advantageous as genes associated with resistance and/or virulence can be identified and used to predict antibiotic resistance, antibiotic susceptibility, and microbial virulence characteristics. Probes (e.g., oligonucleotides) specific for genes encoding genes associated with resistance and/or virulence of a microorganism can be used to identify and characterize microorganisms. Additional methods for identifying GI pathogens include competitive and non-competitive immunoassays, enzyme immunoassay (EIA), radioimmunoassay (RIA), antigen capture assays, two-antibody sandwich assays, Western blot analysis, enzyme linked immunosorbent assays (ELISAs), and the like. Staining techniques accompanied by microscopy may also be used (see, e.g., Garcia et al. (2017) Clin Microbiol Rev. 31(1):pii: e00025-17; McHardy et al. (2014) J. Clin. Microbiol. 52(3):712-20). Additional testing methods are described for example, in Drancourt et al. (2016) Clinical Microbiology Reviews 29(3):429-47.

### SIBO

In some embodiments, the methods described herein include performing an assay or test to determine whether a subject has SIBO. In some embodiments, a small intestine or jejunal aspirate is obtained from the subject (e.g., using a special sonde or via enteroscopy) and directly cultured. Sampling can be achieved by intubation followed by scrape, biopsy, or aspiration of the contents of the intestinal lumen, including the lumen of the duodenum, jejunum, or ileum. Further, any of the contents of the intestinal lumen including material of a cellular, fluid, fecal, or gaseous nature, or sampling is of the lumenal wall itself can be sampled. Analysis of the sample to detect bacterial overgrowth is by conventional microbiological techniques including microscopy, culturing, and/or cell numeration techniques. The detection of greater than about 1×10³ CFU/mL, greater than about 1×10⁴ CFU/mL, or greater than about 1×10⁵ CFU/mL of fluid can be indicative of SIBO.

In some embodiments, an ingestible device described herein may be administered to a subject to determine if the subject has SIBO. For example, a device described herein may be used to detect the presence of bacterial growth in a dilution from a jejunal fluid sample, and a bacterial concentration of about 10⁵ CFU/mL or greater in the jejunal fluid is indicative that the subject has SIBO.

Another exemplary method of detecting small intestinal bacterial overgrowth is by endoscopic visual inspection of the wall of the duodenum, jejunum, and/or ileum.

Breath hydrogen testing is another exemplary method of detecting SIBO in a patient (e.g., P. Kerlin and L. Wong, Gastroenterol. 95(4):982-88 (1988); A. Strocchi et al., Gastroenterol. 105(5):1404-1410 (1993); D. de Boissieu *et al.,* (1996); P. J. Lewindon et al., J. Paedatr. Child Health 34(1):79-82 (1998)). Breath hydrogen (and breath methane) tests are based on the fact that many obligately or facultatively fermentative bacteria found in the gastrointestinal tract produce detectable quantities of hydrogen or methane gas as fermentation products from a substrate consumed by the host under certain circumstances. Substrates include sugars such as lactulose, xylose, lactose, or glucose. The hydrogen (or methane) produced in the small intestine then enters the blood stream of the host and are gradually exhaled.

To perform the breath tests, the patient, after an overnight fast, swallows a controlled quantity of a suitable substrate, such as lactulose, xylose, lactose, or glucose, and breath samples are taken at frequent time intervals (e.g., every 10 to 15 minutes) for a two- to four-hour period. Samples are analyzed by gas chromatography or by other suitable techniques, singly or in combination. Plots of breath hydrogen in patients with SIBO typically show a double peak, i.e., a smaller early hydrogen peak followed by a larger hydrogen peak. A single hydrogen peak can also be an indicator of SIBO, if peak breath hydrogen exceeds the normal range of hydrogen for a particular testing protocol. (See, G. Mastropaolo and W. D. Rees, Gut 28(6):721-25 (1987)).

A variable fraction of the population fails to exhale appreciable hydrogen gas during intestinal fermentation of lactulose; the intestinal microflora of these individuals instead produce more methane. (G. Corazza et al., Dig. Dis. Sci. 38(11):2010-16 (1993); S. M. Riordan et al., Am. J. Gastroentrol. 91(9); 1795-1803 (1996)). Consequently, in the event of an initial negative result for breath hydrogen, or as a precaution, methane and/or carbon dioxide contents in each breath sample are optionally measured, in addition to hydrogen, or an alternative substrate is used.

In some embodiments, the presence of SIBO is demonstrated by a relative decrease in peak hydrogen exhalation values for an individual subject after antimicrobial treatment, in accordance with the present disclosure, compared to pretreatment values.

Another exemplary method of detecting bacterial overgrowth is by gas chromatography with mass spectrometry and/or radiation detection to measure breath emissions of isotope-labeled carbon dioxide, methane, or hydrogen, after administering an isotope-labeled substrate that is metabolizable by gastrointestinal bacteria but poorly digestible by the human host, such as lactulose, xylose, mannitol, or urea. (*e.g.,* G. R. Swart and J. W. van den Berg, Scand. J. Gastroenterol. [Suppl.] 225:13-18 (1998); S. F. Dellert et al., J. Pediatr. Gastroenterol. Nutr. 25(2):153-58 (1997); C. E. King and P. P. Toskes, Crit. Rev. Lab. Sci. 21(3):269-81 (1984)). A poorly digestible substrate is one for which there is a relative or absolute lack of capacity in a human for absorption or for enzymatic degradation or catabolism thereof.

Suitable isotopic labels include ¹³C or ¹⁴C. For measuring methane or carbon dioxide, suitable isotopic labels can also include ²H and ³H or ¹⁷O and ¹⁸O, as long as the substrate is synthesized with the isotopic label placed in a metabolically suitable location in the structure of the substrate, e.g., a location where enzymatic biodegradation by intestinal microflora results in the isotopic label being sequestered in the gaseous product. If the isotopic label selected is a radioisotope, such as ¹⁴C, ¹³H, or ¹⁵O, breath samples can be analyzed by gas chromatography with suitable radiation detection means. (*e.g.,* C. S. Chang et al., Eur. J. Nucl. Med. 22(10):1118-22 (1995); C. E. King and P. P. Toskes, Gastroenterol. 91(6):1447-51 (1986); A. Schneider et al., 32(2):86-91 (1985)).

In some embodiments, SIBO is diagnosed by a presentation of symptomology suggestive of SIBO (e.g., bloating, diarrhea, flatulence, increased or reduced stool frequency, abdominal pain, constipation, weight loss, fever, abdominal tenderness, nausea, gastric stasis, steatorrhea, and any combination thereof). In some embodiments, SIBO is diagnosed by a presentation of symptomology suggestive of SIBO in combination with any of the other diagnostic methods described herein (e.g., a greater than about 1×10³ CFU/mL in a small intestine fluid sample and/or the type of bacteria detected in the small intestine fluid sample).

As described herein, a patient diagnosed as having SIBO is administered one of ertapenem, meropenem, ceftriaxone, and piperacillin-tazobactam based on said diagnosis.

### Microbiome Characterization

Described herein are methods of characterizing the GI microbiome of a subject. As discussed above, the GI tract includes hundreds of microbial species, including bacteria, viruses, protozoan, and other parasites. The majority of gut bacteria belong to two phyla, the Bacteroidetes and Firmicutes; other phyla include Proteobacteria, Actinobacteria, Synergistetes and Fusobacteria. A healthy microbiota provides many benefits including resistance to colonization by harmful pathogens, metabolism of indigestible carbohydrates, vitamin production, and host immune response modulation (Browne et al. (2017) Nat. Rev. Microbiol. 15(9):531-43). Disruptions in the gut microbiota, also called dysbiosis, can lead to GI disorders, as well as metabolic disorders, and brain dysfunction (see Lin and Zhang (2017) BMC Immunol. 18:2). Thus, monitoring and characterizing the gut microbiota is an important tool in the armory of diagnostic/therapeutics tools relating to GIDs.

In some disclosuresof any of the methods described herein, an ingestible device described herein is administered to a subject to identify, characterize and/or analyze the GI microbiota. As described in detail below, the ingestible devices described herein may be used to collect a sample from the GI tract of the subject. Upon expulsion of the ingestible device from the subject, the device may be collected, and the sample therein may be further processed in order to characterize the microorganisms present at the site of collection. By collecting samples from different sites along the GI tract of a subject, the microbiota present at any location in the gastrointestinal tract can be mapped. For example, samples from one or more of the duodenum, jejunum, ileum, ascending colon, transverse colon or descending colon can be collected in order to analyze the microbiome of a subject. The location of particular species and strains of microorganisms present in the GI tract can be ascertained as described herein and a map of the general location of these microorganisms can be generated.

Any microorganisms can be identified and characterized using the methods described herein including, but not limited to bacteria, archaea, viruses, protozoa, parasites, and prions. In some disclosures, commensal bacteria are identified and characterized. In some disclosures, pathogenic bacteria are identified and characterized. Exemplary bacteria genera that may be identified and characterized include *Acetanaerobacterium, Acetivibrio, Actinobacillus, Actinomyces, Aeromonas, Aggregatibacter, Alicyclobacillus, Alkaliphilus, Alstipes, Anaerophaga, Anaerofustis, Anaerosporobacter, Anaerostipes, Anaerotruncus, Anoxybacillus, Atopobium, Bacillus, Bacteroides, Blautia, Brachyspira, Brevibacillus, Bryantella, Bulleidia, Butyricicoccus, Butyrivibrio, Campylobacter, Capnocytophaga, Catenibacterium, Catonella, Chlamydiales, Citrobacter, Clostridiales, Clostridium, Collinsella, Corynebacterium, Coprobacillus, Coprococcus, Coxiella, Deferribacteres, Desulfitobacterium, Desulfotomaculum, Dialister, Dorea, Eggerthella, Escherichia, Enterobacter, Enterococcus, Erysipelothrix, Erysipelotrichaceae, Ethanoligenens, Eubacterium, Faecalibacterium, Filifactor, Flavonifractor, Flexistipes, Fulvimonas, Fusobacterium, Gemella, Gemmiger, Geobacillus, Gloeobacter, Granulicatella, Haemophilus, Helicobacter, Holdemania, Hydrogenoanaerobacterium, Kingella, Klebsiella, Kocuria, Lachnobacterium, Lachnoclostridium, Lachnospira, Lactobacillus, Lactonifactor, Leptospira, Leptotrichia, Lutispora, Lysinibacillus, Megasphaera, Mollicutes, Moorella, Moryella, Neisseria, Nocardia, Oribacterium, Oscillibacter, Oscillospira, Paenibacillus, Paludibacter, Papillibacter, Parabacteroides, Parascardovia, Peptostreptococcus, Plesiomonas, Porphyromonas, Prevotella, Proteus, Pseudoflavoniftactor, Pseudomonas, Ralstonia, Robinsoniella, Roseburia, Rothia, Ruminococcaceae, Ruminococcus, Saccharomonospora, Sarcina, Salmonella, Selenomonas, Sporobacter, Staphylococcus, Streptococcus, Solobacterium, Shigella, Sporobacter, Sporolactobacillus, Streptomyces, Subdoligranulum, Sutterella, Staphylococcus, Syntrophococcus, Tanerella, Thermoanaerobacter, Thermobifida, Treponema, Turicibacter, Vibrio, Veillonella,* and *Yersinia.*

The ingestible devices described herein may directly characterize (without needing to process the sample *ex vivo)* microorganisms collected from the GI tract of a subject as provided in detail below. Moreover, the ingestible devices described herein may be used to collect a sample from the GI tract of a subject which can then be processed *ex vivo* in order to ascertain the identity, quantity, and characteristics of the microorganisms in the sample. In some disclosures, this analysis is performed using live cells (e.g., growing or non-growing cells) obtained from the sample. In some disclosures, this analysis is performed using dead cells obtained from the sample. For instance, samples collected from the device may be analyzed using methods known in the art for these purposes, including direct observation using dark-field microscopy (with and without staining), electron microscopy, microcolony detection by autofluorescence, fluorescence *in situ* hybridization (FISH), flow cytometry, differential system reactivity assays such as pH-based reactions, enzyme profiling, carbon source utilization, and the analysis of carbohydrate utilization, preformed enzymes, organic products, and cellular fatty acids, 16S ribosomal RNA sequencing, 23S ribosomal RNA sequencing, 18S ribosomal RNA sequencing, internal transcribed spacer (ITS) region sequencing, *rpoB* gene sequencing, serological testing, PCR, real time PCR, and matrix assisted laser desorption ionization time-of-flight (MALDI-TOF) (see, e.g., Lagier et al. (2015) Clin. Microbiol. Rev. 28(1):237-64). In addition, assays that detect microbial byproducts (e.g., metabolites), toxins and antigens may be used to determine the identity of microorganisms collected from the GI tract of a subject (e.g., ELISA assays and flow cytometry assays).

For instance, samples obtained using an ingestible device described herein can be used to inoculate a liquid or solid culture media for further expansion and identification of the microorganisms present in the sample. For example, to identify bacteria, plates holding one of the following culture media can be inoculated with the sample either manually (e.g. using a sterile loop or needle) or by an automated streaker instrument: blood agar (BAP), Hektoen-enteric agar, Maconkey (MAC) agar, colistin-nalidix acid (CNA), candida ID agar, and MCA bifidobacter agar. The plates are then incubated (e.g., at 30-37 °C) for a number of hours (e.g., 8-96 hours or more) prior to evaluation. Some plates (e.g., the bifidobacter agar plates can be incubated longer (e.g., at 35 °C for at least or about 72 hours). Candida ID agar plates can be incubated for at least or about 72 hours at 35 °C. Following incubation, the plates can be assessed in various ways. For example, changes in morphology can be evaluated. CNA plates can be evaluated for alpha-hemolysis (green), gamma-hemolysis (no hemolysis) and/or beta-hemolysis (clearing of agar immediately surrounding a colony). *Lactobacillus, Streptococcus,* and *Staphylococcus* are a few of the isolates that may be recovered from CNA plates. HE agar plates can be examined for the presence of lactose (yellow) and non-lactose fermenters, hydrogen sulfide producers (black pigment) and clearly mucoid colonies. These plates are useful in isolating *Salmonella,* which produce hydrogen sulfide, and *Shigella,* which do not ferment lactose and appear as clear colonies. Maconkey agar can be used to identify gram-negative organisms. Almost all enteric bacilli will grow on this media. Lactose fermenting colonies (pink), non-lactose fermenting colonies (grayish or colorless), and mucoid colonies may also grow on these plates. Additional media and conditions for culturing human GI microbiota are described in Table 3 of Lagier et al. (2015) Clin. Microbiol. Rev. 28(1):237-64).

In some , samples obtained from the ingestible devices are subjected to further analysis in order to characterize the isolates species, strain, and antibiotic susceptibility. It will be readily understood by those of skill in the art that depending on the analysis methodology used, culture steps may be necessary in order to obtain individual microbial isolates for analysis. For example, MALDI-TOF analysis is a rapid, low cost method for identifying microorganisms. Three systems are available for the identification of microorganisms, the Andromas database (Andromas SAS, Paris, France), the Vitek-MS platform (bioMerieux, Marcy l'Etoile, France), and the Bruker Biotyper (Bruker Daltonics, Heidelberg, Germany; and Becton Dickinson, Franklin Lakes, NJ, USA) (see, e.g., Clark et al. (2013) Clin. Microbiol. Rev. 26:547-603. In some disclosures, samples obtained from the ingestible device or isolates obtained from the samples can be analyzed using a combination of polymerase chain reaction/electrospray ionization mass spectrometry (PCR/ESI-MS) to identify the microorganisms in the samples, such as the IRIDICA System (Abbot Molecular, Des Plaines, IL, USA). In some disclosures, samples obtained from the ingestible device or isolates obtained from the samples can be characterized using API^{®} strips (bioMerieux, Marcy l'Etoile, France), which allow for the identification of bacteria (e.g., *Staphylococci, Enterococci, Streptococci, Enterobacteriaceae,* non-fermenting bacteria, and yeast) based on the detection of enzymatic activity.

### Sulfur-metabolizing bacteria

In some disclosures, the devices and methods described herein are used to identify, characterize and/or quantify sulfur-metabolizing microorganisms in the GI tract of a subject (e.g., *in vivo* or *ex vivo),* or the abundance of sulfated metabolites (e.g., bile acids, polyphenols and biogenic amines). For example, the microbiome of IBD patients with active disease may be enriched in microbial taxa involved in sulfur metabolism such as *Escherichia, Shigella* and *Fusobacterium,* and a high proportion of sulfate-reducing bacteria such as *Desulfovibrio* and *Campylobacter.*

### Mucin-degrading bacteria

In some disclosures, the devices and methods described herein are used to identify, characterize and/or quantify mucin-degrading microorganisms in the GI tract of a subject (e.g., *in vivo* or *ex vivo),* or the abundance of mucin or mucin metabolites. For example, the microbiome of ulcerative colitis and Crohn's disease patients may be enriched in *R. torques* and *R. gnavus,* while *A. muciniphila,* may be reduced in ulcerative colitis and Crohn's disease patients.

### Methane-producing archaea

In some disclosures, the devices and methods described herein may be used to identify, characterize and/or quantify methane-producing microorganism in the GI tract of a subject (e.g., *in vivo* or *ex vivo).* Archaea are the only confirmed, naturally-occurring biological sources of methane. Methanogenic archaea oxidize hydrogen to produce methane. *Methanobrevibacter smithii* is the predominant methanogen in the human intestine, although other methanogenic archaea may also be present, such as *Methanosphaera stadtmanae and . Methanobacterium ruminatum.* An association between IBS-C and high breath methane levels has been reported, and experimental evidence suggests that methane may delay intestinal transit and contractility (Triantafyllou et al. (2014) J. Neurogastroenterol. Motil. 20:31-40; and Goettlieb et al. (2016) Aliment. Pharmacol. Ther. 43(2):197-212. Further, a correlation between decreased methane production (assessed using methane on lactulose breath test) was observed in subjects who were responsive to antibiotic treatment for SIBO (Gatta and Scarpignato (2017) Aliment. Pharmacol. Ther. 45(5):604-16. Thus, methanogenic archaea may play an important role in many gastrointestinal diseases, including IBS and SIBO.

In some disclosures, the methods described herein include detecting the presence of and/or quantitating the amount of archaeal cells (e.g., methanogenic archaeal cells) present in a sample obtained from the GI tract of the subject. Assays for detecting the presence and /or quantity of bacteria in a sample obtained from the GI tract of a subject described herein may also be used to detect and/or quantify archaeal cells in a sample, including for example, the assays described below which detect (e.g., directly or indirectly) fluorescence emitted by coenzyme F-420.

In some disclosures, the methods described herein include determining the concentration of methane in the GI tract of a subj ect using an ingestible device described herein. For example, the concentration of methane can be detected at specific regions of the GI tract of a subject (e.g., one or more of the duodenum, jejunum, ileum, ascending colon, transverse colon or descending colon). In some disclosures, the methods described herein include identifying subject having an elevated level of methane in the GI tract as determined using a breath test, and further determining the concentration of methane in the GI tract of a subject using an ingestible device described herein. In some disclosures, the subject had or is at risk of developing a gastrointestinal disorder (e.g., SIBO and IBS).

In some disclosures, the methods described herein include methods for detecting volatile organic compounds (VOCs), such as methane, and other gases from a biological sample using resistive metal oxide gas sensors/mixed metal oxide gas sensors, electrochemical gas sensors, optical/IR gas sensors, conducting polymer/composite polymer resistive/capacitive gas sensors, quartz crystal microbalance gas sensors, carbon nanotubes, and pellister/calorimetric gas sensors in an ingestible device. Examples of ingestible gas sensors are described in US Patent Publication No. US 2013/0289368, which published on October 31, 2013, US Patent Publication No. US 2017/0284956, which published on October 5, 2017, and PCT Patent Publication No. WO 2016/197181, which published on December 15, 2016. Examples of gases that can be detected in the gastrointestinal tract using a sensor include, but are not limited to, oxygen, hydrogen, nitrogen, methane, and carbon dioxide.

In some disclosures, the methods described herein include generating spectral data of one or more regions of the GI tract of a subject using an ingestible device described herein. For example, the spectral data can be generated for specific regions of the GI tract of a subject (e.g., one or more of the duodenum, jejunum, ileum, ascending colon, transverse colon or descending colon).

In some disclosures, one or more of the following conditions may be determined for one or more regions of the GI tract of a subject using an ingestible device described herein: pH, gastrointestinal motility, temperature, heart rate, and respiration rate.

In some disclosures, the methods and devices described herein are used to generate a microbial profile of a subject. The microbial profile can include information such as the identity, location, abundance, antibiotic-resistance, antibiotic-sensitivity of microorganisms (e.g., commensal or pathogenic bacteria) present in the GI tract of a subject. A microbial profile can include information relating to any analyte described herein, pH, temperature, gastrointestinal motility, and others.

In some disclosures, a microbial profile can be generated for a subject having a GID (e.g., FBS or SIBO). In some embodiments, a microbial profile can be generated for a subject before and after treatment with a therapeutic agent (e.g., an antibiotic). The microbial profile of a subject may be used to predict a subject's response to a treatment (e.g., an antibiotic treatment for an infection; see, e.g., Khanna et al. (2016) Aliment. Pharmacol. Ther. 44(7):715-27).

In some disclosures, a microbial profile can be generated for a subject before and after consumption of an ingestible standard. Ingestible standards allow for the comparison of microbial profiles from the same individual at different time points as well as from different individuals (e.g., having different genetic profiles). Ingestible standards can be used to ascertain how particular foods or macronutrients affect the microbial composition of the microbiome of a subject. In some disclosures, a microbial profile can be generated for a healthy subject. In some disclosures, a microbial profile can be generated for a malnourished subject.

In some disclosures, a microbial profile can be generated for a subject before and after consumption of an medical food to develop a "nutrient profile" for the subject. The term "medical food" refers to a food which is formulated to be consumed or administered under the supervision of a physician and which is intended for the specific dietary management of a disease or condition for which distinctive nutritional requirements are established. In some disclosures, an ingestible device described herein may be administered to a subject before and/or after ingestion of a medical food, and a sample obtained from the GI tract of the subject using the ingestible device may be analyzed to determine levels of one or more micronutrients, macronutrients, enzymes, amino acids, fats, carbohydrates, vitamins (e.g., folic acid, vitamin B6, vitamin B12, biotin, thiamine, riboflavin, niacin, vitamin B5, vitamin A, vitamin C, vitamin D, and vitamin E), minerals (e.g., calcium, chromium, chloride, copper, iodide, fluoride, magnesium, manganese, molybdenum, potassium, phosphorous, selenium, sodium, and zinc) present in the GI tract of the subject. In some disclosures, an ingestible device described herein may be administered to a subject before and/or after ingestion of a medical food, and a sample obtained from the GI tract of the subject using the ingestible device may be analyzed to characterize the microbiome present in the GI tract of the subject before and/or after ingesting the medical food.

### Antimicrobial Susceptibility Testing

In some disclosures, individual bacterial isolates obtained from the cultures above are subjected to antimicrobial susceptibility testing to determine their resistance and/or susceptibility to specific antimicrobial agents. This information may be used to determine an appropriate antibiotic treatment to, for example, treat a GI infection with a specific pathogen, decrease/increase the amount of a particular bacterial strain/species in the GI tract of the subject, and/or treat SIBO in a subject. Thus, once an antimicrobial profile has been obtained for a bacterial isolate of interest, an appropriate antimicrobial can be administered to the subject as desired.

Susceptibility testing is particular useful to detect individual isolates that possess acquired antimicrobial resistance mechanisms. Many susceptibility testing methods are known in the art and can be used as described herein (see, e.g., Jorgensen and Ferraro (2009) Clinical Infectious Diseases 49:1749-1755; and Maurer et al. (2017) Infectious Disease Reports 9:6839). For example, antimicrobial susceptibility can be determined using conventional culture based methods such as the broth or agar dilution test. In these dilution tests, the bacterial isolate of interest is inoculated onto different media that include serial dilutions of an antimicrobial agent, and incubated under appropriate conditions (e.g., overnight at 37 °C) to allow for growth to occur. Following the incubation period, the media is examined to determine bacterial growth. The lowest concentration of antibiotic that prevents growth represents the minimal inhibitory concentration (MIC). Broth dilution tests can be miniaturized and mechanized allowing for reproducibility and high throughput.

The antimicrobial gradient method can also be used. The antimicrobial gradient method relies on the creation of an antimicrobial concentration gradient in an agar medium to determine susceptibility. The Etest^{®} (bioMerieux, Marcy l'Etoile, France) is a commercial version of the test that employs thin plastic strips impregnated on the underside with a dried antimicrobial concentration gradient, and include a marking of the concentration scale of the antimicrobial. The strips are placed in a radial fashion on a agar plate that has been inoculated with a bacterial isolate of interest. After incubation under appropriate conditions (e.g., overnight at 37 °C) to allow for growth to occur. If the antibiotic inhibits growth of the bacterial isolate, a growth inhibition area forms in the shape of an ellipse where no bacterial growth is detected. The MIC is determined as the concentration indicated on the strip corresponding to the lower part of the ellipse-shaped growth inhibition area.

Another type of susceptibility test is the disk diffusion test, often referred to as the Kirby-Bauer test. This is a standardized test that involves inoculating a gel plate (e.g., a 150-mm Mueller-Hinton agar plate) and placing thereon one or more disks impregnated with fixed concentrations of an antimicrobial. After incubation (e.g., 18-24 hours at 35 °C.), the diameter of zones of inhibition around the disks (if present) determine the sensitivity of the inoculated microorganism to the particular antimicrobial agent impregnated in each disk. Results of this method can be analyzed by comparing the diameter of the inhibition zone with information published by the National Committee on Clinical Laboratory Standards.

Commercial instruments for antimicrobial susceptibility testing, such as Phoenix 100 (BD Biosciences) and Vitek^{®} 2 (BioMérieux), allow automation and reduce hands-on and incubation time, and can be used in the methods described herein. Both instruments operate with colorimetric or fluorimetric indicators for bacterial identification and estimation of growth rate.

In some disclosures, individual microbial isolates (e.g., bacteria, archaea, protozoa, and parasites) obtained from a sample collected from the GI tract of a subject (e.g., a subject having a GID or GID symptomology) are analyzed to determine their resistance and/or susceptibility to particular antimicrobial agents (e.g., an antibiotic described herein). This analysis can be used to determine effective antimicrobial treatment regimens to treat against the microbial isolate. For example, if the microbial isolate is a pathogen, suitable antimicrobial treatments for the subject can be identified.

In some disclosures, the susceptibility and/or resistance of a microorganism in the GI tract of a subject to an antimicrobial agent can be determined by collecting a sample from the GI tract using an ingestible device described herein. The ingestible device may include one or more sampling chambers, comprising one or more antimicrobial agents. Samples may be recovered from the device after a period of time (e.g., about 1 hour, about 3 hours, about 6 hours, about 12 hours, about 18 hours, about 24 hours, about 36 hours, about 48 hours, about 72 hours or more) and analyzed to quantitate and/or determine the viability of the microorganism(s) in the sample. Reduced viability (e.g., determined by analyzing the dead microorganisms recovered from the sample) indicates that a microorganism is susceptible to the antimicrobial agent(s) that was in the sampling chamber. Viability indicates that the microorganism is resistant to the antimicrobial agent(s) that was in the sampling chamber. Microorganism viability can be determined by a variety of methods, and can include both methods that highlight viable organisms (vital stains) as well as dead organisms (mortal stains). Stains for assessing viability are known in the art and include ethidium or propidium dyes, hexidium iodide, SYTO nucleic acid stains, 7-aminiactinomycin D, SYTOX Green/Orange/Blue nucleic acid stains, and others (see, e.g., Lloyd and Hayes (1995) FEMS Microbiology Letters 133:1-7). Viable and dead microorganisms recovered from the sample can be identified using a method described herein.

Any antimicrobial may be tested using the methods described herein, including beta-lactam antibiotics, aminoglycosides, ansa-type antibiotics, anthraquinones, antibiotic azoles, antibiotic glycopeptides, macrolides, antibiotic nucleosides, antibiotic peptides, antibiotic polyenes, antibiotic polyethers, quinolones, antibiotic steroids, sulfonamides, carbapenems, tetracycline, dicarboxylic acids, antibiotic metals, oxidizing agents, substances that release free radicals and/or active oxygen, cationic antimicrobial agents, quaternary ammonium compounds, biguanides, triguanides, bisbiguanides and analogs and polymers thereof and naturally occurring antibiotic compounds.

Beta-lactam antibiotics include, but are not limited to, 2-(3-alanyl)clavam, 2-hydroxymethylclavam, 8-epi-thienamycin, acetyl-thienamycin, amoxicillin, amoxicillin sodium, amoxicillin trihydrate, amoxicillin-potassium clavulanate combination, ampicillin, ampicillin sodium, ampicillin trihydrate, ampicillin-sulbactam, apalcillin, aspoxicillin, azidocillin, azlocillin, aztreonam, bacampicillin, biapenem, carbenicillin, carbenicillin disodium, carfecillin, carindacillin, carpetimycin, cefacetril, cefaclor, cefadroxil, cefalexin, cefaloridine, cefalotin, cefamandole, cefamandole, cefapirin, cefatrizine, cefatrizine propylene glycol, cefazedone, cefazolin, cefbuperazone, cefcapene, cefcapene pivoxil hydrochloride, cefdinir, cefditoren, cefditoren pivoxil, cefepime, cefetamet, cefetamet pivoxil, cefixime, cefinenoxime, cefinetazole, cefminox, cefminox, cefmolexin, cefodizime, cefonicid, cefoperazone, ceforanide, cefoselis, cefotaxime, cefotetan, cefotiam, cefoxitin, cefozopran, cefpiramide, cefpirome, cefpodoxime, cefpodoxime proxetil, cefprozil, cefquinome, cefradine, cefroxadine, cefsulodin, ceftazidime, cefteram, cefteram pivoxil, ceftezole, ceftibuten, ceftizoxime, ceftriaxone, cefuroxime, cefuroxime axetil, cephalosporin, cephamycin, chitinovorin, ciclacillin, clavulanic acid, clometocillin, cloxacillin, cycloserine, deoxy pluracidomycin, dicloxacillin, dihydro pluracidomycin, epicillin, epithienamycin, ertapenem, faropenem, flomoxef, flucloxacillin, hetacillin, imipenem, lenampicillin, loracarbef, mecillinam, meropenem, metampicillin, meticillin, mezlocillin, moxalactam, nafcillin, northienamycin, oxacillin, panipenem, penamecillin, penicillin, phenethicillin, piperacillin, tazobactam, pivampicillin, pivcefalexin, pivmecillinam, pivmecillinam hydrochloride, pluracidomycin, propicillin, sarmoxicillin, sulbactam, sulbenicillin, talampicillin, temocillin, terconazole, thienamycin, ticarcillin and analogs, salts and derivatives thereof.

Beta-lactam antibiotics can be used in combination with other antibiotics or active agents to achieve an antimicrobial effect. For example, Beta-lactam antibiotics (such as carbapenems) can be co-administered with carbapenemase inhibitors (e.g., sulbactam, tazobactam, clavulanic acid, avibactam, vaborbactam). Use of such inhibitors restores or increases potency to carbapenem antibiotics by inhibiting the beta-lactamase enzymes that would otherwise degrade them. Other exemplary inhibitors are relebactam and boronic acid-based inhibitors, including PRX7009, b-lactamase inhibitory protein II, and Zinc01807204 and Zinc02318494 compounds. Metallo-b-lactamase inhibitors include EDTA, thioester derivatives, propionic acid, maleic acid, succinic acid and phthalic acid derivatives.

Aminoglycosides include, but are not limited to, 1,2'-N-DL-isoseryl-3',4'-dideoxykanamycin B, 1,2'-N-DL-isoseryl-kanamycin B, 1,2'-N-[(S)-4-amino-2-hydroxybutyryl]-3',4'-dideoxykanamycin B, 1,2'-N-[(S)-4-amino-2-hydroxybutyryl]-kanamycin B, 1-N-(2-Aminobutanesulfonyl) kanamycin A, 1-N-(2-aminoethanesulfonyl)3',4'-dideoxyribostamycin, 1-N-(2-Aminoethanesulfonyl)3'-deoxyribostamycin, 1-N-(2-aminoethanesulfonyl)3'4'-dideoxykanamycin B, 1-N-(2-aminoethanesulfonyl)kanamycin A, 1-N-(2-aminoethanesulfonyl)kanamycin B, 1-N-(2-aminoethanesulfonyl)ribostamycin, 1-N-(2-aminopropanesulfonyl)3'-deoxykanamycin B, 1-N-(2-aminopropanesulfonyl)3'4'-dideoxykanamycin B, 1-N-(2-aminopropanesulfonyl)kanamycin A, 1-N-(2-aminopropanesulfonyl)kanamycin B, 1-N-(L-4-amino-2-hydroxy-butyryl)2,'3'-dideoxy-2'-fluorokanamycin A, 1-N-(L-4-amino-2-hydroxy-propionyl)2,'3'-dideoxy-2'-fluorokanamycin A, 1-N-DL-3',4'-dideoxy-isoserylkanamycin B, 1-N-DL-isoserylkanamycin, 1-N-DL-isoserylkanamycin B, 1-N-[L-(-)-(alpha-hydroxy-gamma-aminobutyryl)]-XK-62-2,2',3'-dideoxy-2'-fluorokanamycin A,2-hydroxygentamycin A3,2-hydroxygentamycin B, 2-hydroxygentamycin B1, 2-hydroxygentamycin JI-20A, 2-hydroxygentamycin JI-20B, 3"-N-methyl-4"-C-methyl-3',4'-dodeoxy kanamycin A, 3"-N-methyl-4"-C-methyl-3',4'-dodeoxy kanamycin B, 3"-N-methyl-4"-C-methyl-3',4'-dodeoxy-6'-methyl kanamycin B, 3',4'-Dideoxy-3'-eno-ribostamycin,3',4'-dideoxyneamine,3',4'-dideoxyribostamycin, 3'-deoxy-6'-N-methyl-kanamycin B,3'-deoxyneamine,3'-deoxyribostamycin, 3'-oxysaccharocin,3,3'-nepotrehalosadiamine, 3-demethoxy-2"-N-formimidoylistamycin B disulfate tetrahydrate, 3-demethoxyistamycin B,3-O-demethyl-2-N-formimidoylistamycin B, 3-O-demethylistamycin B,3-trehalosamine,4",6"-dideoxydibekacin, 4-N-glycyl-KA-6606VI, 5"-Amino-3',4',5"-trideoxy-butirosin A, 6"-deoxydibekacin,6'-epifortimicin A, 6-deoxy-neomycin (structure 6-deoxy-neomycin B),6-deoxy-neomycin B, 6-deoxy-neomycin C, 6-deoxy-paromomycin, acmimycin, AHB-3',4'-dideoxyribostamycin, AHB-3'-deoxykanamycin B, AHB-3'-deoxyneamine, AHB-3'-deoxyribostamycin, AHB-4"-6"-dideoxydibekacin, AHB-6"-deoxydibekacin, AHB-dideoxyneamine, AHB-kanamycin B, AHB-methyl-3'-deoxykanamycin B, amikacin, amikacin sulfate, apramycin, arbekacin, astromicin, astromicin sulfate, bekanamycin, bluensomycin, boholmycin, butirosin, butirosin B, catenulin, coumamidine gamma1, coumamidine gamma2,D,L-1-N-(alpha-hydroxy-beta-aminopropionyl)-XK-62-2, dactimicin, de-O-methyl-4-N-glycyl-KA-6606VI, de-O-methyl-KA-6606I, de-O-methyl-KA-7038I, destomycin A, destomycin B, di-N6',O3-demethylistamycin A, dibekacin, dibekacin sulfate, dihydrostreptomycin, dihydrostreptomycin sulfate, epi-formamidoylglycidylfortimicin B, epihygromycin, formimidoyl-istamycin A, formimidoyl-istamycin B, fortimicin B, fortimicin C, fortimicin D, fortimicin KE, fortimicin KF, fortimicin KG, fortimicin KG1 (stereoisomer KG1/KG2), fortimicin KG2 (stereoisomer KG1/KG2), fortimicin KG3, framycetin, framycetin sulphate, gentamicin, gentamycin sulfate, globeomycin, hybrimycin A1, hybrimycin A2, hybrimycin B1, hybrimycin B2, hybrimycin C1, hybrimycin C2, hydroxystreptomycin, hygromycin, hygromycin B, isepamicin, isepamicin sulfate, istamycin, kanamycin, kanamycin sulphate, kasugamycin, lividomycin, marcomycin, micronomicin, micronomicin sulfate, mutamicin, myomycin, N-demethyl-7-O-demethylcelesticetin, demethylcelesticetin, methanesulfonic acid derivative of istamycin, nebramycin, nebramycin, neomycin, netilmicin, oligostatin, paromomycin, quintomycin, ribostamycin, saccharocin, seldomycin, sisomicin, sorbistin, spectinomycin, streptomycin, tobramycin, trehalosmaine, trestatin, validamycin, verdamycin, xylostasin, zygomycin and analogs, salts and derivatives thereof.

Ansa-type antibiotics include, but are not limited to, 21-hydroxy-25-demethyl-25-methyl-thioprotostreptovaricin, 3-methyl-thiorifamycin, ansamitocin, atropisostreptovaricin, awamycin, halomicin, maytansine, naphthomycin, rifabutin, rifamide, rifampicin, rifamycin, rifapentine, rifaximin (*e*.*g*., Xifaxan^{®}), rubradirin, streptovaricin, tolypomycin and analogs, salts and derivatives thereof.

Antibiotic anthraquinones include, but are not limited to, auramycin, cinerubin, ditrisarubicin, ditrisarubicin C, figaroic acid fragilomycin, minomycin, rabelomycin, rudolfomycin, sulfurmycin and analogs, salts and derivatives thereof.

Antibiotic azoles include, but are not limited to, azanidazole, bifonazole, butoconazol, chlormidazole, chlormidazole hydrochloride, cloconazole, cloconazole monohydrochloride, clotrimazol, dimetridazole, econazole, econazole nitrate, enilconazole, fenticonazole, fenticonazole nitrate, fezatione, fluconazole, flutrimazole, isoconazole, isoconazole nitrate, itraconazole, ketoconazole, lanoconazole, metronidazole, metronidazole benzoate, miconazole, miconazole nitrate, neticonazole, nimorazole, niridazole, omoconazol, ornidazole, oxiconazole, oxiconazole nitrate, propenidazole, secnidazol, sertaconazole, sertaconazole nitrate, sulconazole, sulconazole nitrate, tinidazole, tioconazole, voriconazol and analogs, salts and derivatives thereof.

Antibiotic glycopeptides include, but are not limited to, acanthomycin, actaplanin, avoparcin, balhimycin, bleomycin B (copper bleomycin), chloroorienticin, chloropolysporin, demethylvancomycin, enduracidin, galacardin, guanidylfungin, hachimycin, demethylvancomycin, N-nonanoyl-teicoplanin, phleomycin, platomycin, ristocetin, staphylocidin, talisomycin, teicoplanin, vancomycin, victomycin, xylocandin, zorbamycin and analogs, salts and derivatives thereof.

Macrolides include, but are not limited to, acetylleucomycin, acetylkitasamycin, angolamycin, azithromycin, bafilomycin, brefeldin, carbomycin, chalcomycin, cirramycin, clarithromycin, concanamycin, deisovaleryl-niddamycin, demycinosyl-mycinamycin, Di-O-methyltiacumicidin, dirithromycin, erythromycin, erythromycin estolate, erythromycin ethyl succinate, erythromycin lactobionate, erythromycin stearate, flurithromycin, focusin, foromacidin, haterumalide, haterumalide, josamycin, josamycin ropionate, juvenimycin, juvenimycin, kitasamycin, ketotiacumicin, lankavacidin, lankavamycin, leucomycin, machecin, maridomycin, megalomicin, methylleucomycin, methymycin, midecamycin, miocamycin, mycaminosyltylactone, mycinomycin, neutramycin, niddamycin, nonactin, oleandomycin, phenylacetyideltamycin, pamamycin, picromycin, rokitamycin, rosaramicin, roxithromycin, sedecamycin, shincomycin, spiramycin, swalpamycin, tacrolimus, telithromycin, tiacumicin, tilmicosin, treponemycin, troleandomycin, tylosin, venturicidin and analogs, salts and derivatives thereof.

Antibiotic nucleosides include, but are not limited to, amicetin, angustmycin, azathymidine, blasticidin S, epiroprim, flucytosine, gougerotin, mildiomycin, nikkomycin, nucleocidin, oxanosine, oxanosine, puromycin, pyrazomycin, showdomycin, sinefungin, sparsogenin, spicamycin, tunicamycin, uracil polyoxin, vengicide and analogs, salts and derivatives thereof.

Antibiotic peptides include, but are not limited to, actinomycin, aculeacin, alazopeptin, amfomycin, amythiamycin, antifungal from *Zalerion arboricola,* antrimycin, apid, apidaecin, aspartocin, auromomycin, bacileucin, bacillomycin, bacillopeptin, bacitracin, bagacidin, beminamycin, beta-alanyl-L-tyrosine, bottromycin, capreomycin, caspofungine, cepacidine, cerexin, cilofungin, circulin, colistin, cyclodepsipeptide, cytophagin, dactinomycin, daptomycin, decapeptide, desoxymulundocandin, echanomycin, echinocandin B, echinomycin, ecomycin, enniatin, etamycin, fabatin, ferrimycin, ferrimycin, ficellomycin, fluoronocathiacin, fusaricidin, gardimycin, gatavalin, globopeptin, glyphomycin, gramicidin, herbicolin, iomycin, iturin, iyomycin, izupeptin, janiemycin, janthinocin, jolipeptin, katanosin, killertoxin, lipopeptide antibiotic, lipopeptide from *Zalerion* sp., lysobactin, lysozyme, macromomycin, magainin, melittin, mersacidin, mikamycin, mureidomycin, mycoplanecin, mycosubtilin, neopeptifluorin, neoviridogrisein, netropsin, nisin, nocathiacin, nocathiacin 6-deoxyglycoside, nosiheptide, octapeptin, pacidamycin, pentadecapeptide, peptifluorin, permetin, phytoactin, phytostreptin, planothiocin, plusbacin, polcillin, polymyxin antibiotic complex, polymyxin B, polymyxin B1, polymyxin F, preneocarzinostatin, quinomycin, quinupristin-dalfopristin, safracin, salmycin, salmycin, salmycin, sandramycin, saramycetin, siomycin, sperabillin, sporamycin, a *Streptomyces* compound, subtilin, teicoplanin aglycone, telomycin, thermothiocin, thiopeptin, thiostrepton, tridecaptin, tsushimycin, tuberactinomycin, tuberactinomycin, tyrothricin, valinomycin, viomycin, virginiamycin, zervacin and analogs, salts and derivatives thereof.

In some disclosures, the antibiotic peptide is a naturally-occurring peptide that possesses an antibacterial and/or an antifungal activity. Such peptide can be obtained from an herbal or a vertebrate source.

Polyenes include, but are not limited to, amphotericin, amphotericin, aureofungin, ayfactin, azalomycin, blasticidin, candicidin, candicidin methyl ester, candimycin, candimycin methyl ester, chinopricin, filipin, flavofungin, fradicin, hamycin, hydropricin, levorin, lucensomycin, lucknomycin, mediocidin, mediocidin methyl ester, mepartricin, methyl amphotericin, natamycin, niphimycin, nystatin, nystatin methyl ester, oxypricin, partricin, pentamycin, perimycin, pimaricin, primycin, proticin, rimocidin, sistomycosin, sorangicin, trichomycin and analogs, salts and derivatives thereof.

Polyethers include, but are not limited to, 20-deoxy-epi-narasin, 20-deoxysalinomycin, carriomycin, dianemycin, dihydrolonomycin, etheromycin, ionomycin, iso-lasalocid, lasalocid, lenoremycin, lonomycin, lysocellin, monensin, narasin, oxolonomycin, a polycyclic ether antibiotic, salinomycin and analogs, salts and derivatives thereof.

Quinolones include, but are not limited to, an alkyl-methylendioxy-4(1H)-oxocinnoline-3-carboxylic acid, alatrofloxacin, cinoxacin, ciprofloxacin, ciprofloxacin hydrochloride, danofloxacin, dermofongin A, enoxacin, enrofloxacin, fleroxacin, flumequine, gatifloxacin, gemifloxacin, grepafloxacin, levofloxacin, lomefloxacin, lomefloxacin, hydrochloride, miloxacin, moxifloxacin, nadifloxacin, nalidixic acid, nifuroquine, norfloxacin, ofloxacin, orbifloxacin, oxolinic acid, pazufloxacine, pefloxacin, pefloxacin mesylate, pipemidic acid, piromidic acid, premafloxacin, rosoxacin, rufloxacin, sparfloxacin, temafloxacin, tosufloxacin, trovafloxacin and analogs, salts and derivatives thereof.

Antibiotic steroids include, but are not limited to, aminosterol, ascosteroside, cladosporide A, dihydrofusidic acid, dehydro-dihydrofusidic acid, dehydrofusidic acid, fusidic acid, squalamine and analogs, salts and derivatives thereof.

Sulfonamides include, but are not limited to, chloramine, dapsone, mafenide, phthalylsulfathiazole, succinylsulfathiazole, sulfabenzamide, sulfacetamide, sulfachlorpyridazine, sulfadiazine, sulfadiazine silver, sulfadicramide, sulfadimethoxine, sulfadoxine, sulfaguanidine, sulfalene, sulfamazone, sulfamerazine, sulfamethazine, sulfamethizole, sulfamethoxazole, sulfamethoxypyridazine, sulfamonomethoxine, sulfamoxol, sulfanilamide, sulfaperine, sulfaphenazol, sulfapyridine, sulfaquinoxaline, sulfasuccinamide, sulfathiazole, sulfathiourea, sulfatolamide, sulfatriazin, sulfisomidine, sulfisoxazole, sulfisoxazole acetyl, sulfacarbamide and analogs, salts and derivatives thereof.

Tetracyclines include, but are not limited to, dihydrosteffimycin, demethyltetracycline, aclacinomycin, akrobomycin, baumycin, bromotetracycline, cetocyclin, chlortetracycline, clomocycline, daunorubicin, demeclocycline, doxorubicin, doxorubicin hydrochloride, doxycycline, lymecyclin, marcellomycin, meclocycline, meclocycline sulfosalicylate, methacycline, minocycline, minocycline hydrochloride, musettamycin, oxytetracycline, rhodirubin, rolitetracycline, rubomycin, serirubicin, steffimycin, tetracycline and analogs, salts and derivatives thereof.

Dicarboxylic acids, having between about 6 and about 14 carbon atoms in their carbon atom skeleton are particularly useful in the treatment of disorders of the skin and mucosal membranes that involve microbial. Suitable dicarboxylic acid moieties include, but are not limited to, adipic acid, pimelic acid, suberic acid, azelaic acid, sebacic acid, 1,11-undecanedioic acid, 1,12-dodecanedioic acid, 1,13-tridecanedioic acid and 1, 14-tetradecanedioic acid. Thus, in one or more disclosures of the present disclosure, dicarboxylic acids, having between about 6 and about 14 carbon atoms in their carbon atom skeleton, as well as their salts and derivatives (*e.g*., esters, amides, mercapto-derivatives, anhydraides), are useful immunomodulators in the treatment of disorders of the skin and mucosal membranes that involve inflammation. Azelaic acid and its salts and derivatives are preferred. It has antibacterial effects on both aerobic and anaerobic organisms, particularly *Propionibacterium acnes* and *Staphylococcus epidermidis,* normalizes keratinization, and has a cytotoxic effect on malignant or hyperactive melanocytes. In a preferred disclosure, the dicarboxylic acid is azelaic acid in a concentration greater than 10%. Preferably, the concentration of azelaic acid is between about 10% and about 25%. In such concentrates, azelaic acid is suitable for the treatment of a variety of skin disorders, such as acne, rosacea and hyperpigmentation.

In some disclosures, the antibiotic agent is an antibiotic metal. A number of metals ions have been shown to possess antibiotic activity, including silver, copper, zinc, mercury, tin, lead, bismutin, cadmium, chromium and ions thereof. It has been theorized that these antibiotic metal ions exert their effects by disrupting respiration and electron transport systems upon absorption into bacterial or fungal cells. Anti-microbial metal ions of silver, copper, zinc, and gold, in particular, are considered safe for *in vivo* use. Anti-microbial silver and silver ions are particularly useful due to the fact that they are not substantially absorbed into the body. Thus, in one or more disclosure, the antibiotic metal consists of an elemental metal, selected from the group consisting of silver, copper, zinc, mercury, tin, lead, bismutin, cadmium, chromium and gold, which is suspended in the composition as particles, microparticles, nanoparticles or colloidal particles. The antibiotic metal can further be intercalated in a chelating substrate.

In further disclosures, the antibiotic metal is ionic. The ionic antibiotic metal can be presented as an inorganic or organic salt (coupled with a counterion), an organometallic complex or an intercalate. Non-binding examples of counter inorganic and organic ions are sulfadiazine, acetate, benzoate, carbonate, iodate, iodide, lactate, laurate, nitrate, oxide, and palmitate, a negatively charged protein. In preferred embodiments, the antibiotic metal salt is a silver salt, such as silver acetate, silver benzoate, silver carbonate, silver iodate, silver iodide, silver lactate, silver laurate, silver nitrate, silver oxide, silver palmitate, silver protein, and silver sulfadiazine.

In one or more disclosures, the antibiotic metal or metal ion is embedded into a substrate, such as a polymer, or a mineral (such as zeolite, clay and silica).

In one or more disclosures, the antibiotic agent includes strong oxidants and free radical liberating compounds, such as oxygen, hydrogen peroxide, benzoyl peroxide, elemental halogen species, as well as oxygenated halogen species, bleaching agents (e.g., sodium, calcium or magnesium hypochloride and the like), perchlorite species, iodine, iodate, and benzoyl peroxide. Organic oxidizing agents, such as quinones, are also included. Such agents possess a potent broad-spectrum activity.

In one or more disclosures, the antibiotic agent is a cationic antimicrobial agent. The outermost surface of bacterial cells universally carries a net negative charge, making them sensitive to cationic substances. Examples of cationic antibiotic agents include: quaternary ammonium compounds (QAC's)-QAC's are surfactants, generally containing one quaternary nitrogen associated with at least one major hydrophobic moiety; alkyltrimethyl ammonium bromides are mixtures of where the alkyl group is between 8 and 18 carbons long, such as cetrimide (tetradecyltrimethylammonium bromide); benzalkonium chloride, which is a mixture of n-alkyldimethylbenzyl ammonium chloride where the alkyl groups (the hydrophobic moiety) can be of variable length; dialkylmethyl ammonium halides; dialkylbenzyl ammonium halides; and QAC dimmers, which bear bi-polar positive charges in conjunction with interstitial hydrophobic regions.

In one or more disclosures, the cationic antimicrobial agent is a polymer. Cationic antimicrobial polymers include, for example, guanide polymers, biguanide polymers, or polymers having side chains containing biguanide moieties or other cationic functional groups, such as benzalkonium groups or quarternium groups (e.g., quaternary amine groups). It is understood that the term "polymer" as used herein includes any organic material including three or more repeating units, and includes oligomers, polymers, copolymers, block copolymers, terpolymers, etc. The polymer backbone may be, for example a polyethylene, polypropylene or polysilane polymer.

In one or more disclosures, the cationic antimicrobial polymer is a polymeric biguanide compound. When applied to a substrate, such a polymer is known to form a barrier film that can engage and disrupt a microorganism. An exemplary polymeric biguanide compound is polyhexamethylene biguanide (PHMB) salts. Other exemplary biguanide polymers include, but are not limited to poly(hexamethylenebiguanide), poly(hexamethylenebiguanide) hydrochloride, poly(hexamethylenebiguanide) gluconate, poly(hexamethylenebiguanide) stearate, or a derivative thereof. In one or disclosures embodiments, the antimicrobial material is substantially water-insoluble.

In some disclosures, the antibiotic agent is selected from the group of biguanides, triguanides, bisbiguanides and analogs thereof.

Guanides, biguanides, biguanidines and triguanides are unsaturated nitrogen containing molecules that readily obtain one or more positive charges, which make them effective antimicrobial agents. The basic structures a guanide, a biguanide, a biguanidine and a triguanide are provided below. 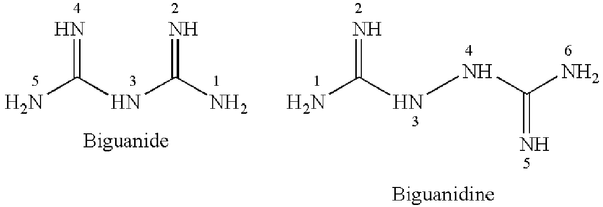 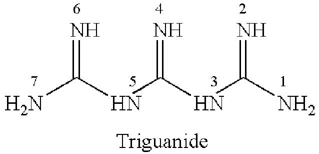 In some disclosures, the guanide, biguanide, biguanidine or triguanide, provide bi-polar configurations of cationic and hydrophobic domains within a single molecule.

Examples of guanides, biguanides, biguanidines and triguanides that are currently been used as antibacterial agents include chlorhexidine and chlorohexidine salts, analogs and derivatives, such as chlorhexidine acetate, chlorhexidine gluconate and chlorhexidine hydrochloride, picloxydine, alexidine and polihexanide. Other examples of guanides, biguanides, biguanidines and triguanides that can conceivably be used according to the present disclosure are chlorproguanil hydrochloride, proguanil hydrochloride (currently used as antimalarial agents), mefformin hydrochloride, phenformin and buformin hydrochloride (currently used as antidiabetic agents).

Yet, in one or more disclosures, the antibiotic is a non-classified antibiotic agent, including, without limitation, aabomycin, acetomycin, acetoxycycloheximide, acetylnanaomycin, an *Actinoplanes* sp. compound, actinopyrone, aflastatin, albacarcin, albacarcin, albofungin, albofungin, alisamycin, alpha-R,S-methoxycarbonylbenzylmonate, altromycin, amicetin, amycin, amycin demanoyl compound, amycine, amycomycin, anandimycin, anisomycin, anthramycin, anti-syphilis immune substance, anti-tuberculosis immune substance, an antibiotic from *Escherichia coli,* an antibiotic from *Streptomyces refuineus,* anticapsin, antimycin, aplasmomycin, aranorosin, aranorosinol, arugomycin, ascofuranone, ascomycin, ascosin, *Aspergillus flavus* antibiotic, asukamycin, aurantinin, an Aureolic acid antibiotic substance, aurodox, avilamycin, azidamfenicol, azidimycin, bacillaene, a *Bacillus larvae* antibiotic, bactobolin, benanomycin, benzanthrin, benzylmonate, bicozamycin, bravomicin, brodimoprim, butalactin, calcimycin, calvatic acid, candiplanecin, carumonam, carzinophilin, celesticetin, cepacin, cerulenin, cervinomycin, chartreusin, chloramphenicol, chloramphenicol palmitate, chloramphenicol succinate sodium, chlorflavonin, chlorobiocin, chlorocarcin, chromomycin, ciclopirox, ciclopirox olamine, citreamicin, cladosporin, clazamycin, clecarmycin, clindamycin, coliformin, collinomycin, copiamycin, corallopyronin, corynecandin, coumermycin, culpin, cuprimyxin, cyclamidomycin, cycloheximide, dactylomycin, danomycin, danubomycin, delaminomycin, demethoxyrapamycin, demethylscytophycin, dermadin, desdamethine, dexylosyl-benanomycin, pseudoaglycone, dihydromocimycin, dihydronancimycin, diumycin, dnacin, dorrigocin, dynemycin, dynemycin triacetate, ecteinascidin, efrotomycin, endomycin, ensanchomycin, equisetin, ericamycin, esperamicin, ethylmonate, everninomicin, feldamycin, flambamycin, flavensomycin, florfenicol, fluvomycin, fosfomycin, fosfonochlorin, fredericamycin, frenolicin, fumagillin, fumifungin, funginon, fusacandin, fusafungin, gelbecidine, glidobactin, grahamimycin, granaticin, griseofulvin, griseoviridin, grisonomycin, hayumicin, hayumicin, hazymicin, hedamycin, heneicomycin, heptelicid acid, holomycin, humidin, isohematinic acid, karnatakin, kazusamycin, kristenin, L-dihydrophenylalanine, a L-isoleucyl-L-2-amino-4-(4'-amino-2',5'-cyclohexadienyl) derivative, lanomycin, leinamycin, leptomycin, libanomycin, lincomycin, lomofungin, lysolipin, magnesidin, manumycin, melanomycin, methoxycarbonylmethylmonate, methoxycarbonylethylmonate, methoxycarbonylphenylmonate, methyl pseudomonate, methylmonate, microcin, mitomalcin, mocimycin, moenomycin, monoacetyl cladosporin, monomethyl cladosporin, mupirocin, mupirocin calcium, mycobacidin, myriocin, myxopyronin, pseudoaglycone, nanaomycin, nancimycin, nargenicin, neocarcinostatin, neoenactin, neothramycin, nifurtoinol, nocardicin, nogalamycin, novobiocin, octylmonate, olivomycin, orthosomycin, oudemansin, oxirapentyn, oxoglaucine methiodide, pactacin, pactamycin, papulacandin, paulomycin, phaeoramularia fungicide, phenelfamycin, phenyl, cerulenin, phenylmonate, pholipomycin, pirlimycin, pleuromutilin, a polylactone derivative, polynitroxin, polyoxin, porfiromycin, pradimicin, prenomycin, prop-2-enylmonate, protomycin, *Pseudomonas* antibiotic, pseudomonic acid, purpuromycin, pyrinodemin, pyrroInitrin, pyrrolomycin, amino, chloro pentenedioic acid, rapamycin, rebeccamycin, resistomycin, reuterin, reveromycin, rhizocticin, roridin, rubiflavin, naphthyridinomycin, saframycin, saphenamycin, sarkomycin, sarkomycin, sclopularin, selenomycin, siccanin, spartanamicin, spectinomycin, spongistatin, stravidin, streptolydigin, *Streptomyces arenae* antibiotic complex, streptonigrin, streptothricins, streptovitacin, streptozotocine, a strobilurin derivative, stubomycin, sulfamethoxazol-trimethoprim, sakamycin, tejeramycin, terpentecin, tetrocarcin, thermorubin, thermozymocidin, thiamphenicol, thioaurin, thiolutin, thiomarinol, thiomarinol, tirandamycin, tolytoxin, trichodermin, trienomycin, trimethoprim, trioxacarcin, tyrissamycin, umbrinomycin, unphenelfamycin, urauchimycin, usnic acid, uredolysin, variotin, vermisporin, verrucarin and analogs, salts and derivatives thereof.

In one or more disclosures, the antibiotic agent is a naturally occurring antibiotic compound. As used herein, the term "naturally-occurring antibiotic agent" includes all antibiotics that are obtained, derived or extracted from plant or vertebrate sources. Non-limiting examples of families of naturally-occurring antibiotic agents include phenol, resorcinol, antibiotic aminoglycosides, anamycin, quinines, anthraquinones, antibiotic glycopeptides, azoles, macrolides, avilamycin, agropyrene, cnicin, aucubin antibioticsaponin fractions, berberine (isoquinoline alkaloid), arctiopicrin (sesquiterpene lactone), lupulone, humulone (bitter acids), allicin, hyperforin, echinacoside, coniosetin, tetramic acid, imanine and novoimanine.

Ciclopirox and ciclopiroxolamine possess fungicidal, fungistatic and sporicidal activity. They are active against a broad spectrum of dermatophytes, yeasts, molds and other fungi, such as *Trichophytons* species, *Microsporum* species, *Epidermophyton* species and yeasts (*Candida albicans, Candida glabrata,* other candida species and *Cryptococcus neoformans*). Some *Aspergillus* species are sensitive to ciclopirox as are some *Penicillium.* Likewise, ciclopirox is effective against many Gram-positive and Gram-negative bacteria (e.g., *Escherichia coli, Proteus mirabilis, Pseudomonas aeruginosa, Staphylococcus* and *Streptococcus* species), as well as *Mycoplasma* species, *Trichomonas vaginalis* and *Actinomyces.*

Plant oils and extracts which contain antibiotic agents are also useful. Non-limiting examples of plants that contain agents include thyme, *Perilla,* lavender, tea tree, *Terfezia clayeryi, Micromonospora, Putterlickia verrucosa, Putterlickia pyracantha, Putterlickia retrospinosa, Maytenus ilicifolia, Maytenus evonymoides, Maytenus aquifolia, Faenia interjecta, Cordyceps sinensis,* couchgrass, holy thistle, plantain, burdock, hops, echinacea, buchu, chaparral, myrrh, red clover and yellow dock, garlic, and St. John's wort. Mixtures of the antibiotic agents as described herein may also be employed.

Antimicrobial susceptibility testing using the methods and compositions described herein is particularly advantageous in selecting adequate antimicrobials for the treatment of GI tract infections and SIBO. For example, some bacterial strains isolated from subjects having SIBO have been identified as being resistant to common antibiotics (see, e.g., Bouhnik et al. (1999) Amer. J. Gastroenterol. 94(5): 1327-31). Thus, antimicrobial susceptibility testing using the methods described herein can be used to select adequate antibiotic regimens and dosing for the treatment of SIBO.

In some disclosures of any of the methods described herein, a subject identified as having SIBO or a SIBO-related condition is administered a pharmaceutical formulation comprising at least one antimicrobial (e.g., an antibiotic provided herein). Dosing of the antimicrobial may be adjusted depending on bacterial and/or archaeal load (e.g., bacterial load in the small intestine of a subject) and/or the types of bacteria/archaea identified in the GI tract of the subject or in a portion (e.g., the jejunum) of the GI tract of the subject. In some disclosures, the at least one antimicrobial is selected from the group consisting of a cephalosporin, a quinolone, tetracycline, ampicillin, erythromycin, rifaximin, metronidazole, erythromycin, amoxicillin-clavulanic acid, cefoxitin, ciprofloxacin, norfloxacin, neomycin, doxycycline, lincomycin, chloramphenicol, a carbopenem (e.g., ertapenem, doripenem, and meropenem), ceftriaxone, piperacillin, and tazobactam.

For example, a subject identified as having SIBO (or a SIBO-related condition) that includes a bacterial overgrowth of one or more of strains of *Escherichia coli, Klebsiella* spp., *Enterobacter* spp., *Enterococcus faecalis, Enterococcus faecium,* and *Staphylococcus aureus* may be administered a pharmaceutical formulation comprising rifaximin.

In some disclosures, the at least one antimicrobial is selected from the group consisting of meropenem, ceftriaxone, ertapenem, and piperacillin-tazobactam. In a related disclosure, the antimicrobial is indicated for the treatment of symptomology suggestive of SIBO.

A subject identified as having SIBO (or a SIBO-related condition) that includes a bacterial overgrowth of one or more of *Staphylococcus aureus, Escherichia coli, Bacteroides fragilis, Streptococcus agalactiae, Haemophilus influenza, Bacteroides disasonis, Streptococcus pneumoniae, Klebsiella pneumoniae, Bacteroides ovatus, Streptococcus pyogenes, Moraxella catarrhalis, Bacteroides thetaiotaomicron, Proteus mirabilis, Bacteroides uniformis, Clostridium clostridioforme, Eubacterium lentum, Peptostreptococcus spp., Porphyromonas asaccharolytica, Prevotella bivia, Streptococcus pneumoniae, Citrobacter freundii, Clostridium perfringens, Staphylococcus epidermidis, Citrobacter koseri, Fusobacterium spp., Enterobacter aerogenes, Bacteroides vulgatus, Enterobacter cloacae, Haemophilus influenzae, Haemophilus parainfluenzae, Klebsiella oxytoca, Morganella morganii, Proteus vulgaris, Providencia stuartii, Providencia rettgeri* , *and Serratia marcescens* may be administered a pharmaceutical formulation comprising ertapenem.

A subject identified as having SIBO (or a SIBO-related condition) that includes a bacterial overgrowth of one or more of *Staphylococcus aureus, Escherichia coli, Bacteroides fragilis, Streptococcus agalactiae, Haemophilus influenza, Bacteroides thetaiotaomicron, Streptococcus pneumoniae, Klebsiella pneumoniae, Clostridium clostridioforme, Streptococcus pyogenes, Neisseria meningitides, Enterococcus faecalis, Proteus mirabilis, Peptostreptococcus* spp., Viridans group streptococci, *Pseudomonas aeruginosa, Streptococcus pneumoniae, Citrobacter freundii, Bacteroides distasonis, Staphylococcus epidermidis, Citrobacter diversus, Bacteroides ovatus, Acinetobacter* spp., *Bacteroides uniformis, Campylobacter jejuni, Bacteroides urolyticus, Aeromonas hydrophilia, Bacteroides vulgatus, Enterobacter cloacae,. Clostridium difficile, Hafnia alvei, Clostridium perfringens, Haemophilus influenza, Eubacterium lentum, Moraxella catarrhalis, Fusobacterium* spp., *Prevotella bivia, Klebsiella oxytoca, Prevotella intermedia, Morganella morganii, Prevotella melanogenica, Pasteurella multocida, Porphyomonas asaccharolytica, Proteus vulgaris, Propionibacterium acnes, Serratia marcescens, Salmonella* spp., *Shigella* spp., and *Yersinia enterocolitica* may be administered a pharmaceutical formulation comprising meropenem.

A subject identified as having SIBO (or a SIBO-related condition) that includes a bacterial overgrowth of one or more of *Staphylococcus aureus, Escherichia coli, Bacteroides fragilis, Haemophilus influenza, Bacteroides thetaiotaomicron, Klebsiella pneumoniae, Acinetobacter baumannii, Pseudomonas aeruginosa, Streptococcus pneumoniae, Serratia marcescens, Prevotella melanogenica, Staphylococcus epidermidis, Citrobacter koseri, Bacteroides distasonis, Enterococcus faecalis, Moraxella catarrhalis, Clostridium perfringens, Staphylococcus epidermidis, Morganella morganii, Streptococcus agalactiae, Proteus vulgaris, Streptococcus pneumoniae, Neisseria gonorrhoeae, Streptococcus pyogenes, Proteus mirabilis,* Viridans group streptococci, *Proteus vulgaris, Providencia stuartii, Providencia rettgeri,* and *Salmonella enterica* may be administered a pharmaceutical formulation comprising piperacillin and tazobactam.

A subject identified as having SIBO (or a SIBO-related condition) that includes a bacterial overgrowth of one or more of *Staphylococcus aureus, Escherichia coli, Bacteroides fragilis, Staphylococcus epidermidis, Haemophilus influenza, Clostridium* spp., *Streptococcus pneumoniae, Haemophilus parainfluenzae, Peptostreptococcus* spp., *Streptococcus pyogenes, Acinetobacter baumannii,* Viridans group streptococci, *Pseudomonas aeruginosa, Acinetobacter calcoaceticus, Enterobacter aerogenes, Klebsiella pneumoniae, Kelbsiella oxytoca, Moraxella catarrhalis, Morganella morganii, Neisseria gonorrhoeae, Neisseria meningitides, Proteus mirabilis, Proteus vulgaris, Serratia marcescens, Streptococcus agalactiae, Citrobacter diversus, Prevotella bivius, Citrobacter freundii, Bacteroides melanogenicus, Providencia* spp. (e.g., *Providencia rettgeri*), and *Salmonella* spp. may be administered a pharmaceutical formulation comprising ceftriaxone.

### Methods of Selecting and Optimizing Treatment

The methods described herein include the administration of one or more treatments, i.e. antibiotics, to a subject identified as having or being at risk of developing SIBO. The methods can also include selecting a treatment for a subject who has SIBO or is determined to be at risk for developing SIBO, based upon the presence or absence of an analyte (e.g., a particular microorganism), or based upon the amount of an analyte.

The methods can also include administering a treatment (e.g., a pharmaceutical formulation including at least one therapeutic agent) selected by a method described herein to a subject who has or is at risk of developing SIBO to treat, delay disease progression, or reduce the risk of developing of the disease. In some embodiments, the formulation is comprised in an ingestible device as disclosed herein. In some embodiments wherein the formulation is comprised in an ingestible device, the formulation may be suitable for oral administration. The formulation may be, for example, a solid dosage form or a liquid dosage form. In some embodiments, the formulation is suitable for introduction and optionally for storage in an ingestible device described herein. In some embodiments, the formulation is suitable for introduction and optionally for storage in a reservoir comprised in the ingestible device. In some embodiments, the formulation is suitable for introduction and optionally for storage in the reservoir comprised in the ingestible device.

In some disclosures of any of the methods described herein, a subject having SIBO is administered an ingestible device (e.g., an ingestible device as described herein), wherein the device comprises a pharmaceutical formulation that is released at a location in the gastrointestinal tract of the subject. In some disclosures, the pharmaceutical formulation is released at a location in the gastrointestinal tract of the subject proximate to one or more sites of disease. In some embodiments, the pharmaceutical formulation comprises a therapeutic agent (e.g., a therapeutic agent described herein), and a pharmaceutically acceptable excipient. In some embodiments, the pharmaceutical formulation is a personalized treatment for SIBO in the subject. In some embodiments, the ingestible device is configured to release the therapeutic agent according to desired (e.g., customized or optimized) dosage, timing, and/or location parameter.

Alternatively, the methods described herein can include administering a pharmaceutical formulation comprising a therapeutic agent (e.g., an antibiotic) in a suitable dosage form. The pharmaceutical formulation can be adapted for the chosen route of administration, e.g., orally or parenterally, or by intravenous, intramuscular, topical or subcutaneous routes. In some disclosures, for oral administration, the compounds can be formulated as a solid dosage form with or without an enteric coating.

The methods described herein include orally administering an effective amount of a pharmaceutical formulation comprising an antimicrobial agent (e.g., meropenem, ceftriaxone, ertapenem, or piperacillin-tazobactam) to a subject having SIBO. The pharmaceutical formulation may include a pharmaceutically acceptable vehicle such as an inert diluent, excipient or an assimilable edible carrier. Suitable dosage forms include hard or soft shell gelatin capsules, ingestible tablets, buccal tablets, troches, capsules, elixirs, suspensions, syrups, wafers, and the like.

The amount of active compound in the pharmaceutical formulation is such that an effective dosage level will be obtained.

Appropriate excipients for tablets, troches, pills, capsules, and the like include: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the therapeutic agent, sucrose or fructose as a sweetening agent, methyl and propylparabens as preservatives, a dye and flavoring such as cherry or orange flavor. In addition, the therapeutic agent may be incorporated into sustained-release preparations, particles, and devices.

Pharmaceutical formulations comprising a therapeutic agent (e.g., an antibiotic) may also be administered intravenously or intramuscularly by infusion or injection. Solutions of the therapeutic agent or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these formulations contain a preservative to prevent the growth of microorganisms. Pharmaceutically acceptable excipients and dosage forms are described, for example, in In Remington: The Science and Practice of Pharmacy, 21st edition, 2005, ed. D. B. Troy, Lippincott Williams & Wilkins, Philadelphia; Encyclopedia of Pharmaceutical Technology, eds. J. Swarbrick and J. C. Boylan, 1988-1999, Marcel Dekker, New York; and Handbook of Pharmaceutical Excipients, 3rd Edition (2000) edited by A. H. Kibbe, American Pharmaceutical Association and Pharmaceutical Press.

Also provided herein are methods of determining the efficacy of SIBO treatment. In some embodiments, providing an ingestible device can determine successful treatment of SIBO in a subject (e.g., the presence or absence of an analyte is determined; the levels of an analyte is decreased as compared to the levels of the analyte determined in the subject at an early period of time; the levels of an analyte is decreased as compared to the levels of the analyte determined in a control subject (e.g., a subject that does not have SIBO, or is not at risk of developing SIBO); the levels of an analyte is increased as compared to the levels of the analyte determined in the subject at an early period of time). In some embodiments, prior to the providing an ingestible device step, the subject received treatment for SIBO (e.g., any of the treatment described herein). For example, in some embodiments, the level of an analyte (e.g., any of the analytes described herein) is decreased as compared to the level of the analyte described herein prior to treatment for SIBO, and further treatment is discontinued. For example, in some embodiments, the level of an analyte (e.g., any of the analytes described herein) is increased as compared to the level of the analyte described herein prior to treatment for SIBO, and a different treatment is administered.

Non-limiting examples of therapeutic agents for treating or preventing SIBO include substances that suppress cytokine production, downregulate or suppress self-antigen expression, or mask MHC antigens, and medical foods. Examples of such agents include 2-amino-6-aryl-5 -substituted pyrimidines (see U.S. Patent No. 4,665,077); non-steroidal anti-inflammatory drugs (NSAIDs); ganciclovir; tacrolimus; glucocorticoids such as Cortisol or aldosterone; anti-inflammatory agents such as a cyclooxygenase inhibitor; a 5-lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6-mercaptopurine; steroids such as corticosteroids or glucocorticosteroids or glucocorticoid analogs, e.g., prednisone, methylprednisolone, including SOLU-MEDROL^{®}, methylprednisolone sodium succinate, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or 5 antagonists including anti-interferon-alpha, -beta, or -gamma antibodies, anti-tumor necrosis factor(TNF)-alpha antibodies (infliximab (REMICADE^{®}) or adalimumab), anti-TNF-alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CD 1 la and anti-CD 18 antibodies; anti-L3T4 antibodies; heterologous anti-lymphocyte globulin; pan-T antibodies, anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor (Cohen et al., U.S. Patent No. 5,114,721); T-cell receptor fragments (Offner et al., Science, 251:430-432 (1991); WO 90/11294; Janeway, Nature, 341:482 (1989); and WO 91/01133); BAFF antagonists such as BAFF or BR3 antibodies or immunoadhesins and zTNF4 antagonists (for review, see Mackay and Mackay, Trends Immunol., 23:113-5 (2002); biologic agents that interfere with T cell helper signals, such as anti-CD40 receptor or anti-CD40 ligand (CD 154), including blocking antibodies to CD40-CD40 ligand (e.g., Durie et al., Science, 261:1328-30 (1993); Mohan et al., J. Immunol., 154:1470-80 (1995)) and CTLA4-Ig (Finck et al., Science, 265:1225-7 (1994)); and T-cell receptor antibodies (EP340,109) such as T10B9. Non-limiting examples of adjunct agents also include the following: budesonide; epidermal growth factor; aminosalicylates; metronidazole; mesalamine; olsalazine; balsalazide; antioxidants; thromboxane inhibitors; IL-1 receptor antagonists; anti-IL-1 monoclonal antibodies; growth factors; elastase inhibitors; pyridinylimidazole compounds; TNF antagonists; IL-4, IL-10, IL-13 and/or TGFβ cytokines or agonists thereof (e.g., agonist antibodies); IL-11; glucuronide- or dextran-conjugated prodrugs of prednisolone, dexamethasone or budesonide; ICAM-I antisense phosphorothioate oligodeoxynucleotides (ISIS 2302; Isis Pharmaceuticals, Inc.); soluble complement receptor 1 (TPlO; T Cell Sciences, Inc.); slow-release mesalazine; antagonists of platelet activating factor (PAF); ciprofloxacin; and lignocaine. As disclosed herein, the agents for treating or preventing SIBO include any antibiotic described herein (e.g., rifaximin). Examples of agents for UC are sulfasalazine and related salicylate-containing drugs for mild cases and corticosteroid drugs in severe cases.

Topical administration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

Non-limiting examples of common therapeutic agents for the treatment of IBS-C, IB S-D, and bile acid diarrhea, as well as exemplary dosing regimens, are provided in the table below:

| | **Name** | **Mechanism** | **Dose** |
|---|---|---|---|
| IBS-C | | | |
| | Linaclotide (Linzess, Constella) | peptide agonist of the guanylate cyclase 2C. Reduces activation of colonic sensory neurons, reducing pain; and activates colonic motor neurons, which increases smooth muscle contraction and thus promotes bowel movements. | 290 mcg PO QD |
| | Lubiprostone (Amitiza) | Lubiprostone is a bicyclic fatty acid derived from prostaglandin E1 that acts by specifically activating ClC-2 chloride channels on the apical aspect of gastrointestinal epithelial cells, producing a chloride-rich fluid secretion. These secretions soften the stool, increase motility, and promote spontaneous bowel movements (SBM). | 8 mcg PO BID |
| | Laxatives | | |

| IBS-D | | | |
|---|---|---|---|
| | Rifaximin (Xifaxin) | Antibiotic | 550 mg PO TID x 14 days |
| | Eluxadoline (Viberzi) | Opioid receptor agonist | 100 mg PO BID |
| | Alonsetron (Lontronex) | Antagonist action on the 5-HT3 receptors of the enteric nervous system of the gastrointestinal tract | 0.5-1 mg PO BID |
| | Loperamide (Imodium) | Opioid-receptor agonist | |

| Bile acid diarrhea | | | |
|---|---|---|---|
| | Cholestyramine, colestipol (Questran) | | 4-8 g PO BID |

In some embodiments of any of the methods described herein, a subject may be administered more than one ingestible device. For example, in some embodiments, a subject is administered a first ingestible device and a second ingestible device. In some embodiments, the subject is administered a first, a second, and a third ingestible device. In some embodiments, a subject is administered more than three ingestible devices (either concurrently or consecutively). Each ingestible device administered to a subject may have a different or the same function. For example, in some embodiments, a first ingestible device is suitable for monitoring, identifying and/or characterizing an analyte, and a second ingestible device is suitable for the delivery of a therapeutic agent (e.g., a therapeutic agent described herein). In some embodiments, a first ingestible device is suitable for collecting a sample from the gastrointestinal tract of a subject, and a second ingestible device is suitable for the delivery of a therapeutic agent. In some embodiments, a first ingestible device is suitable for collecting a sample from the gastrointestinal tract of a subject, and a second ingestible device is suitable for performing antimicrobial susceptibility/resistance analysis as described herein. A third ingestible device may be administered to a subject in order to monitor a disease state in the subject.

In some disclosures of any of the methods described herein, an ingestible device described herein is administered to a subject to collect a sample from the GI tract of a subject in order to monitor a level of a specific microorganism in the GI tract of the subject (e.g., before and after treatment with a therapeutic agent (e.g., an antimicrobial agent)).

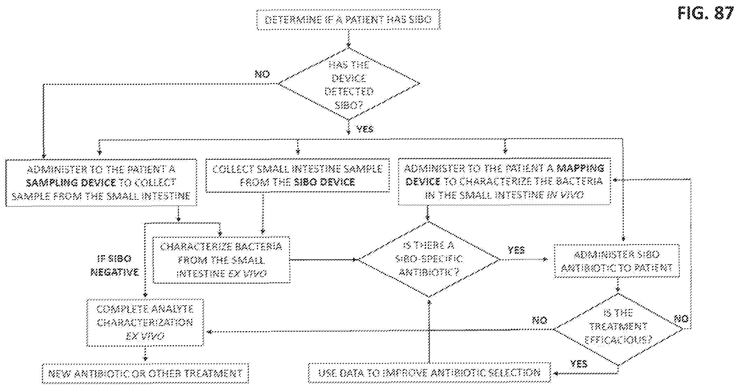

The methods described herein include administering a treatment (e.g., an antibiotic) selected by a method described herein to a subject who has SIBO to treat, delay disease progression, or reduce the risk of developing the disease. An exemplary method for the diagnosis and treatment of SIBO is provided at FIG. 87. In some embodiments, a subject identified as having SIBO is administered an ingestible device described herein, wherein the device includes a sampling chamber. At least one sample may be collected from the GI tract of the subject (e.g., jejunal fluid) using the ingestible device. Sample(s) may be obtained from different regions of the GI tract of the subject (e.g., one or more of the duodenum, jejunum, ileum, ascending colon, transverse colon or descending colon). Upon excretion of the ingestible device from the subject, the sample(s) may be collected and analyzed as described herein the characterize one or more analytes (e.g., bacteria). Microbial isolates from the sample may be subjected to analysis to characterize the microbe and/or antimicrobial susceptibility testing may be performed to identify antimicrobial agents (e.g., cytostatic or cytolytic agents) that can be effectively used against the microbial isolates. The subject can then be administered a pharmaceutical formulation comprising the identified antimicrobial agent. The formulation may be administered using an ingestible device as disclosed herein or it may be administered by other means (e.g., intravenously, rectally, orally, etc.). The efficacy of treatment may be monitored as described herein to improve the antibiotic selection. Additionally, the subject may be administered one or more ingestible devices to monitor, identify and/or characterize methane levels, pH, temperature, and one or more metabolites in the GI tract of the subject.

Accordingly, in some dislosures, the methods described herein comprise (a) diagnosing a subject as having an overgrowth of bacteria in the gastrointestinal tract; wherein the step of diagnosing comprises use of an ingestible device; and (b) administering one of ertapenem, meropenem, ceftriaxone, and piperacillin-tazobactam based on the diagnosis of step (a).

### Methods Relating to Subjects Having Small Intestinal Bacterial Overgrowth (SIBO)

In an aspect, provided herein are methods for treating small intestinal bacterial overgrowth (SIBO) in a subject in need thereof, the method comprising: orally administering an effective amount of a pharmaceutical formulation comprising an antimicrobial agent to the subject, thereby treating SIBO in the subject, wherein the antimicrobial agent is meropenem or ceftriaxone.

In some embodiments, treating SIBO comprises ameliorating or decreasing the severity of one or more symptoms associated with SIBO. In some embodiments, the one or more symptoms associated with SIBO are selected from bloating, diarrhea, flatulence, increased or decreased stool frequency, abdominal pain, constipation, weight loss, fever, abdominal tenderness, nausea, gastric stasis, and steatorrhea.

In some embodiments, treating SIBO comprises partially eradicating the bacterial overgrowth of the small intestine.

In some embodiments, the method further comprises the step of identifying a subject having SIBO.

In some embodiments, identifying a subject having SIBO comprises obtaining a fluid sample from the small intestine and measuring the bacterial concentration, wherein a bacterial concentration of about 10³ CFU/mL or greater in the fluid is indicative that the subject has small intestinal bacterial overgrowth (SIBO).

In some embodiments, identifying a subject having SIBO comprises obtaining a fluid sample from the small intestine and measuring the bacterial concentration, wherein a bacterial concentration of about 10⁴ CFU/mL or greater in the fluid is indicative that the subject has small intestinal bacterial overgrowth (SIBO).

In some embodiments, identifying a subject having SIBO comprises obtaining a fluid sample from the small intestine and measuring the bacterial concentration, wherein a bacterial concentration of about 10⁵ CFU/mL or greater in the fluid is indicative that the subject has small intestinal bacterial overgrowth (SIBO).

In some embodiments, identifying a subject having SIBO comprises measuring an amount of hydrogen or methane, or both, in the breath of a subject after the subject has consumed a substrate selected from the group consisting of lactulose, xylose, lactose, and glucose, wherein an amount of hydrogen or methane, or both, that exceeds a normal range of hydrogen or methane, or both, is indicative that the subject has SIBO.

In some embodiments, an effective amount of the antimicrobial for the treatment SIBO is from about one-quarter to about two-times of a standard dose of the antimicrobial. A standard dose is that regularly used by clinicians for the treatment of other types of infection, and can be informed by the dosing included in, e.g., an FDA label.

As described herein, the antimicrobial is meropenem or ertapenem and the antimicrobial is co-administered with one or more carbapenemase inhibitors. In some embodiments, the carbapenemase inhibitor is selected from sulbactam tazobactam, clavulanic acid, avibactam, and vaborbactam. In some embodiments, the carbapenemase inhibitor is selected from relebactam, a boronic acid-based inhibitor (e.g., PRX7009), a b-lactamase inhibitory protein II, and Zinc01807204 and Zinc02318494 compounds. In some embodiments, the carbapenemase inhibitor is a metallo-b-lactamase inhibitor selected from EDTA, thioester derivatives, propionic acid, maleic acid, succinic acid and phthalic acid derivatives.

In some embodiments, the method of treating SIBO further comprises administering intestinal lavage or enema to the subject.

In some embodiments, the method of treating SIBO further comprises administering a probiotic to the subject.

In some embodiments, the method of treating SIBO further comprises modifying the subject's diet. In some embodiments, modifying a subject' diet comprises increasing the subject's consumption of dietary fiber.

In some embodiments, the method of treating SIBO further comprises administering a chemical prokinetic agent to the subject. Non-limiting examples of prokinetic agents include motilin or functional analogues thereof, a macrolide compound such as erythromycin or azithromycin, or a bile acid, or a bile salt derived therefrom. Example bile acids include ursodeoxycholic acid and chenodeoxycholic acid, and their pharmaceutically acceptable salts (e.g., sodium or potassium salts). Other prokinetic agents are compounds with cholinergic activity such as cisapride. Other prokinetic agents are dopamine antagonists such as metoclopramide, domperidone, or bethanechol.

In some embodiments, the method of treating SIBO further comprises administering bile acid replacement therapy to the subject.

In some embodiments, the method of treating SIBO further comprises administering a nitric oxide altering agent such as nitroglycerin, N-omega-nitro-L-arginine methylester (L-NAME), N-monomethyl-L-arginine (L-NMMA), or a 5-hydroxytryptamine (HT or serotonin) receptor antagonist, such as ondansetron or alosetron to the subject.

In some embodiments, the method of treating SIBO further comprises administering an antihistamine to the subject. Suitable antihistamines include, but are not limited to, promethazine and meclizine.

In some embodiments, the method of treating SIBO further comprises administering a neuroleptic agent to the subject. Suitable neuroleptic agents include, but are not limited to, prochlorperazine, chlorpromazine, or haloperidol.

In some embodiments, the method of treating SIBO further comprises administering a kappa agonist (e.g., fedotozine) to the subject.

In some embodiments, the method of treating SIBO further comprises administering an anti-inflammatory cytokine or an agonist thereof, substantially simultaneously with or after at least partially eradicating the bacterial overgrowth of the small intestine to further ameliorate the symptoms of SIBO. Anti-inflammatory cytokines include human IL-4, IL- 10, IL-11, or TGF-β, derived from a human source or a transgenic non-human source expressing a human gene. The anti-inflammatory cytokine is injected or infused intravenously or subcutaneously.

In some embodiments, the method of treating SIBO further comprises administering a pro-inflammatory cytokine or an antibody that specifically binds a pro-inflammatory cytokine substantially simultaneously with or after at least partially eradicating the bacterial overgrowth of the small intestine to further ameliorate the symptoms of SIBO. The antagonist or antibody is one that binds to a pro-inflammatory cytokine or antagonizes the activity or receptor binding of a pro inflammatory cytokine. Pro-inflammatory cytokines include TNF-α, IL-1α, IL-1β, IL-6, IL-8, IL-12, or LIF. The cytokine antagonist or antibody can be derived from a human source or is a chimeric protein having a human protein constituent. The cytokine antagonist or antibody can be delivered to the human subject by intravenous infusion.

In some embodiments, the method of treating SIBO further comprises administering an agent that modifies afferent neural feedback or sensory perception. Agents that modify afferent neural feedback or sensory perception include 5-HT receptor antagonists, such as ondansetron and alosetron; opiate agonists, such as fedotozine; peppermint oil; cisapride; a dopamine antagonist, such as domperidone; an antidepressant agent; an anxiolytic agent; or a combination of any of these. Useful antidepressant agents include tricyclic antidepressants, such as amitriptyline (Elavil); tetracyclic antidepressants, such as maprotiline; serotonin re-uptake inhibitors, such as fluoxetine (Prozac) or sertraline (Zoloft); monoamine oxidase inhibitors, such as phenezline; and miscellaneous antidepressants, such as trazodone, venlafaxine, mirtazapine, nefazodone, or bupropion (Wellbutrin). Typically, useful antidepressant agents are available in hydrochloride, sulfated, or other conjugated forms, and all of these conjugated forms are included among the useful antidepressant agents. Useful anxiolytic (anti-anxiety) agents include benzodiazepine compounds, such as Librium, Atavin, Xanax, Valium, Tranxene, and Serax, or other anxiolytic agents such as Paxil.

In some embodiments, SIBO is refractory (e.g., not responsive to prior, different course of treatment).

In some embodiments, SIBO is relapsed (e.g., progresses at the conclusion of a prior, different course of treatment).

In another aspect, provided herein are methods for treating SIBO, the method comprising orally administering an effective amount of a pharmaceutical formulation comprising an antimicrobial agent to the subject, wherein the antimicrobial agent exhibits antimicrobial activity against a bacterium implicated in the pathogenesis of SIBO, thereby treating SIBO in the subject.

In some embodiments, the antimicrobial agent exhibits antimicrobial activity against a bacterium implicated in the pathogenesis of SIBO. In some embodiments, the antimicrobial agent exhibits antimicrobial activity against the bacterium with a minimal inhibitory concentration (MIC) range of less than about 0.001 µg/mL to greater than about 128 µg/mL, such as about 0.001 µg/mL to about 128 µg/mL, about 0.001 µg/mL to about 64 µg/mL, about 0.001 µg/mL to about 32 µg/mL, about 0.001 µg/mL to about 16 µg/mL, about 0.001 µg/mL to about 8 µg/mL, about 0.001 µg/mL to about 4 µg/mL, about 0.001 µg/mL to about 2 µg/mL, about 0.001 µg/mL to about 1 µg/mL, about 0.001 µg/mL to about 0.5 µg/mL, about 0.001 µg/mL to about 0.3 µg/mL, about 0.001 µg/mL to about 0.15 µg/mL, about 0.001 µg/mL to about 0.1 µg/mL, about 0.001 µg/mL to about 0.05 µg/mL, about 0.001 µg/mL to about 0.015 µg/mL, about 0.015 µg/mL to about 128 µg/mL, about 0.015 µg/mL to about 64 µg/mL, about 0.015 µg/mL to about 32 µg/mL, about 0.015 µg/mL to about 16 µg/mL, about 0.015 µg/mL to about 8 µg/mL, about 0.015 µg/mL to about 4 µg/mL, about 0.015 µg/mL to about 2 µg/mL, about 0.015 µg/mL to about 1 µg/mL, about 0.015 µg/mL to about 0.5 µg/mL, about 0.015 µg/mL to about 0.3 µg/mL, about 0.015 µg/mL to about 0.15 µg/mL, about 0.015 µg/mL to about 0.1 µg/mL, about 0.015 µg/mL to about 0.05 µg/mL, about 0.03 µg/mL to about 128 µg/mL, about 0.03 µg/mL to about 64 µg/mL, about 0.03 µg/mL to about 32 µg/mL, about 0.03 µg/mL to about 16 µg/mL, about 0.03 µg/mL to about 8 µg/mL, about 0.03 µg/mL to about 4 µg/mL, about 0.03 µg/mL to about 2 µg/mL, about 0.03 µg/mL to about 1 µg/mL, about 0.03 µg/mL to about 0.5 µg/mL, about 0.03 µg/mL to about 0.3 µg/mL, about 0.03 µg/mL to about 0.15 µg/mL, about 0.03 µg/mL to about 0.1 µg/mL, about 0.03 µg/mL to about 0.05 µg/mL, about 0.12 µg/mL to about 128 µg/mL, about 0.12 µg/mL to about 64 µg/mL, about 0.12 µg/mL to about 32 µg/mL, about 0.12 µg/mL to about 16 µg/mL, about 0.12 µg/mL to about 8 µg/mL, about 0.12 µg/mL to about 4 µg/mL, about 0.12 µg/mL to about 2 µg/mL, about 0.12 µg/mL to about 1 µg/mL, about 0.12 µg/mL to about 0.5 µg/mL, about 0.12 µg/mL to about 0.3 µg/mL, about 0.12 µg/mL to about 0.15 µg/mL, about 0.5 µg/mL to about 128 µg/mL, about 0.5 µg/mL to about 64 µg/mL, about 0.5 µg/mL to about 32 µg/mL, about 0.5 µg/mL to about 16 µg/mL, about 0.5 µg/mL to about 8 µg/mL, about 0.5 µg/mL to about 4 µg/mL, about 0.5 µg/mL to about 2 µg/mL, about 0.5 µg/mL to about 1 µg/mL, about 1 µg/mL to about 128 µg/mL, about 1 µg/mL to about 64 µg/mL, about 1 µg/mL to about 32 µg/mL, about 1 µg/mL to about 16 µg/mL, about 1 µg/mL to about 8 µg/mL, about 1 µg/mL to about 4 µg/mL, about 1 µg/mL to about 2 µg/mL, about 2 µg/mL to about 128 µg/mL, about 2 µg/mL to about 64 µg/mL, about 2 µg/mL to about 32 µg/mL, about 2 µg/mL to about 16 µg/mL, about 2 µg/mL to about 8 µg/mL, about 2 µg/mL to about 4 µg/mL, about 4 µg/mL to about 128 µg/mL, about 4 µg/mL to about 64 µg/mL, about 4 µg/mL to about 32 µg/mL, about 4 µg/mL to about 16 µg/mL, about 4 µg/mL to about 8 µg/mL, about 8 µg/mL to about 128 µg/mL, about 8 µg/mL to about 64 µg/mL, about 8 µg/mL to about 32 µg/mL, or about 8 µg/mL to about 16 µg/mL. In some embodiments, the antimicrobial agent exhibits antimicrobial activity against the bacterium with a MIC range of about 0.002 µg/mL to about 0.5 µg/mL, about 0.002 µg/mL to about 128 µg/mL, about 0.008 µg/mL to about 0.3 µg/mL, about 0.008 µg/mL to about 1 µg/mL, about 0.008 µg/mL to about 2 µg/mL, about 0.008 µg/mL to about 8 µg/mL, about 0.008 µg/mL to about 128 µg/mL, about 0.015 µg/mL to 2 about 0.015 µg/mL to 128 µg/mL, about 0.03 µg/mL to about 8 µg/mL, about 0.03 µg/mL to about 64 µg/mL, about 0.06 µg/mL to about 2 µg/mL, about 0.06 µg/mL to about 16 µg/mL, about 0.12 µg/mL to about 8 µg/mL, about 0.12 µg/mL to about 128 µg/mL, about 0.5 µg/mL to about 16 µg/mL, about 0.5 µg/mL to about 128 µg/mL, about 1 µg/mL to about 64 µg/mL, about 1 µg/mL to about 128 µg/mL, about 2 µg/mL to about 64 µg/mL, about 4 µg/mL to about 128 µg/mL, or about 8 µg/mL to about 128 µg/mL. In some embodiments, the antimicrobial agent exhibits antimicrobial activity against the bacterium with a MIC range of about 0.001 µg/mL to about 128 µg/mL, about 0.001 µg/mL to about 64 µg/mL, about 0.004 µg/mL to about 32 µg/mL, about 0.015 µg/mL to about 16 µg/mL, about 0.03 µg/mL to about 8 µg/mL, or about 0.5 µg/mL to about 2 µg/mL. In some embodiments, the antimicrobial agent is meropenem. In some embodiments, the antimicrobial agent is ceftriaxone.

In some embodiments, the antimicrobial agent exhibits antimicrobial activity against the bacterium implicated in the pathogenesis of SIBO, with a MIC₅₀ value of less than about 0.001 µg/mL to greater than about 128 µg/mL, about 0.001 µg/mL to about 64 µg/mL, about 0.004 µg/mL to about 32 µg/mL, about 0.015 µg/mL to about 16 µg/mL, about 0.03 µg/mL to about 8 µg/mL, about 0.5 µg/mL to about 2 µg/mL, or about 0.001 µg/mL, about 0.002 µg/mL, about 0.004 µg/mL, about 0.015 µg/mL, about 0.03 µg/mL, about 0.06 µg/mL, about 0.12 µg/mL, about 0.25 µg/mL, about 0.5 µg/mL, about 1 µg/mL, about 4 µg/mL, about 8 µg/mL, about 16 µg/mL, about 32 µg/mL, about 64 µg/mL, or about 128 µg/mL. In some embodiments, the antimicrobial agent is meropenem. In some embodiments, the antimicrobial agent is ceftriaxone.

In some embodiments, the antimicrobial agent exhibits antimicrobial activity against the bacterium implicated in the pathogenesis of SIBO, with a MIC₉₀ value of less than about 0.001 µg/mL to greater than about 128 µg/mL, about 0.001 µg/mL to about 64 µg/mL, about 0.004 µg/mL to about 32 µg/mL, about 0.015 µg/mL to about 16 µg/mL, about 0.03 µg/mL to about 8 µg/mL, about 0.5 µg/mL to about 2 µg/mL, or about 0.001 µg/mL, about 0.002 µg/mL, about 0.008 µg/mL, about 0.015 µg/mL, about 0.03 µg/mL, about 0.06 µg/mL, about 0.12 µg/mL, about 0.25 µg/mL, about 0.5 µg/mL, about 2 µg/mL, about 4 µg/mL, about 8 µg/mL, about 16 µg/mL, about 32 µg/mL, about 64 µg/mL, or about 128 µg/mL. In some embodiments, the antimicrobial agent is meropenem. In some embodiments, the antimicrobial agent is ceftriaxone.

In some embodiments, the bacterium implicated in the pathogenesis of SIBO is selected from the group consisting of a gram-positive bacterium, a gram-negative bacterium, an anaerobic bacterium, and combinations thereof.

In some embodiments, the bacterium implicated in the pathogenesis of SIBO is a gram-negative bacterium. Examples of gram-negative bacterium implicated in the pathogenesis of SIBO include, but are not limited to, *Enterobacter aerogenes, Escherichia coli, Klebsiella* spp., for example, *K. oxytoca* and *K. pneumonia, Proteus mirabilis,* and *Pseudomonas aeruginosa.*

In some embodiments, the bacterium implicated in the pathogenesis of SIBO is a gram-positive bacterium. Examples of gram-positive bacterium implicated in the pathogenesis of SIBO include, but are not limited to, *Staphylococcus aureus, Enterococcus faecalis,* and *Streptococcus* spp., for example, *Streptococcus pyogenes, Streptococcus agalactiae,* and Viridans group *Streptococcus.*

In some embodiments, the bacterium implicated in the pathogenesis of SIBO is an anaerobic bacterium. Examples of anaerobic bacterium implicated in the pathogenesis of SIBO suggestive of SIBO include, but are not limited to, *Clostridium* spp., for example, C. *sporogenes, C. ramosum,* and *C*. *innocuum, Prevotella* spp., for example, *P. melanogenica, P. bivia, P. buccae, P. nanceiensis, P. intermedia, P. denticola, P. nigrescens, P. corporis, P. bergensis,* and *P. disiens, Veillonella* spp., for example, *V. parvula, Veillonella* dispr, and *V*. *atypica, Bacteroides fragilis,* and *Bacteroides non-fragilis,* for example, *B. caccae, B. thetaiotaomicron, B. ovatus, B. vulgatus, B. uniformis, B. stercoris, B. xylanisolvens, B salyersiae, B. intestinalis,* and *B. faecis.*

In some embodiments, the antimicrobial agent exhibits antimicrobial activity against a bacterium implicated in the pathogenesis of SIBO, where the bacterium is selected from the group consisting of *Enterobacter aerogenes, Escherichia coli, Klebsiella* spp., *K. oxytoca, K. pneumonia, Proteus mirabilis, Pseudomonas aeruginosa, Staphylococcus aureus, Enterococcus faecalis, Streptococcus* spp., *Streptococcus pyogenes, Streptococcus agalactiae,* Viridans group *Streptococcus, Clostridium* spp., *C*. *sporogenes, C. ramosum, C. innocuum, Prevotella* spp., *P. melanogenica, P. bivia, P. buccae, P. nanceiensis, P. intermedia, P. denticola, P. nigrescens, P. corporis, P. bergensis, P. disiens, Veillonella* spp., *V. parvula, Veillonella* dispr, *V. atypica, Bacteroides fragilis, Bacteroides non-fragilis, B. caccae, B. thetaiotaomicron, B. ovatus, B. vulgatus, B. uniformis, B. stercoris, B. xylanisolvens, B salyersiae, B. intestinalis,* and *B. faecis.* In some embodiments, the the antimicrobial agent is meropenem. In some embodiments, the antimicrobial agent is ceftriaxone.

In some embodiments, the antimicrobial agent exhibits bactericidal efficacy against a bacterium implicated in the pathogenesis of SIBO. For example, exposure of the bacterium to the antimicrobial agent for a period of time can result in reduction in colony forming units (CFU) per mL of the bacterium. In some embodiments, exposure of the bacterium to the antimicrobial agent for a period of time results in a reduction in CFU/mL of the bacterium of about 0.5-log to about 5-log or greater, such as about 0.5-log to about 4.5-log, about 0.5-log to about 4-log, about 0.5-log to about 3.5-log, about 0.5-log to about 3-log, about 0.5-log to about 2.5-log, about 0.5-log to about 2-log, about 0.5-log to about 1.5-log, about 0.5-log to about 1-log, about 1-log to about 5-log, about 1-log to about 4.5-log, about 1-log to about 4-log, about 1-log to about 3.5-log, about 1-log to about 3-log, about 1-log to about 2.5-log, about 1-log to about 2-log, about 1-log to about 1.5-log, about 1.5-log to about 5-log, about 1.5-log to about 4.5-log, about 1.5-log to about 4-log, about 1.5-log to about 3.5-log, about 1.5-log to about 3-log, about 1.5-log to about 2.5-log, about 1.5-log to about 2-log, about 2-log to about 5-log, about 2-log to about 4.5-log, about 2-log to about 4-log, about 2-log to about 3.5-log, about 2-log to about 3-log, about 2-log to about 2.5-log, about 2.5-log to about 5-log, about 2.5-log to about 4.5-log, about 2.5-log to about 4-log, about 2.5-log to about 3.5-log, about 2.5-log to about 3-log, about 3-log to about 5-log, about 3-log to about 4.5-log, about 3-log to about 4-log, about 3-log to about 3.5-log, about 3.5-log to about 5-log, about 3.5-log to about 4.5-log, about 3.5-log to about 4-log, about 4-log to about 5-log, about 4-log to about 4.5-log, about 4.5-log to about 5-log, or at least ≥0.5-log, ≥1-log, ≥1.5-log, ≥2-log, ≥2.5-log, ≥3-log, ≥3.5-log, ≥4-log, ≥4.5-log, ≥5-log, ≥6-log, ≥7-log, ≥8-log, ≥9-log, ≥10-log, or greater. In some embodiments, the antimicrobial agent exhibits bactericidal efficacy of at least a 3-log reduction in CFU/mL in about 1 hour, about 2 hours, about 4 hours, about 6 hours, about 8 hours, about 10 hours, about 12 hours, about 14 hours, about 16 hours, about 18 hours, about 20 hours, about 24 hours, about 30 hours, about 36 hours, about 42 hours, or about 48 hours. In some embodiments, the antimicrobial agent exhibits bactericidal efficacy of at least a 3-log reduction in CFU/mL in about 2 hours. In some embodiments, the antimicrobial agent exhibits bactericidal efficacy of at least a 3-log reduction in CFU/mL in about 6 hours. In some embodiments, the antimicrobial agent exhibits bactericidal efficacy of at least a 3-log reduction in CFU/mL in about 24 hours. In some embodiments, the bacterium is selected from *E. coli, Streptococcus* spp., and *Bacteroides* spp. In some embodiments, the the antimicrobial agent is meropenem. In some embodiments, the antimicrobial agent is ceftriaxone.

In some embodiments, the antimicrobial agent exhibits bactericidal efficacy against a bacterium implicated in the pathogenesis of SIBO at a concentration of about 0.5X MIC to about 8X MIC, such as about 0.5X MIC to about 6X MIC, about 0.5X MIC to about 4X MIC, about 0.5X MIC to about 2X MIC, about 0.5X MIC to about 1X MIC, about 1X MIC to about 8X MIC, about 1X MIC to about 6X MIC, about 1X MIC to about 4X MIC, about 1X MIC to about 2X MIC, about 2X MIC to about 8X MIC, about 2X MIC to about 6X MIC, about 2X MIC to about 4X MIC, about 4X MIC to about 8X MIC, about 4X MIC to about 6X MIC, about 6X MIC to about 8X MIC, or about 0.5X MIC, about 1X MIC, about 2X MIC, about 3X MIC, about 4X MIC, about 5X MIC, about 6X MIC, about 7X MIC, or about 8X MIC. In some embodiments, the the antimicrobial agent is meropenem. In some embodiments, the antimicrobial agent is ceftriaxone.

In some embodiments, exposure of the bacterium implicated in the pathogenesis of SIBO prevents regrowth of the bacterium. In some embodiments, the the antimicrobial agent is meropenem. In some embodiments, the antimicrobial agent is ceftriaxone.

In some embodiments, the antimicrobial agent is bacteriostatic with respect to one or more bacterium implicated in the pathogenesis of SIBO. "Bacteriostatic" refers to the ability of the antimicrobial agent to prevent or inhibit growth of a bacterium. In some embodiments, the antimicrobial agent is bactericidal with respect to one or more bacterium implicated in the pathogenesis of SIBO. "Bactericidal" refers to the ability of the antimicrobial agent to kill a bacterium. In some embodiments, the antimicrobial agent is both bacteriostatic and bactericidal with respect to one or more bacterium implicated in the pathogenesis of SIBO. In some embodiments, the bacterium is selected from *E. coli, Streptococcus* spp., and *Bacteroides* spp. In some embodiments, the the antimicrobial agent is meropenem. In some embodiments, the antimicrobial agent is ceftriaxone.

In some embodiments, the bacterium implicated in the pathogenesis of SIBO. For example, the bacterium can have a spontaneous mutation frequency of less than about 10⁻⁷, such as less than 10⁻⁸ or 10⁻⁹, such as less than 1×10⁻¹⁰. In some embodiments, the bacterium has a spontaneous mutation frequency of less than about 7.45×10⁻⁹, about 5.75×10⁻⁹, about 5.15×10⁻⁹, about 9.55×10⁻¹⁰, about 1.85×10⁻¹⁰, about 1.75×10⁻¹⁰, about 1.50×10⁻¹⁰, or about 1.05×10⁻¹⁰. In some embodiments, the bacterium is selected from *Escherichia coli, Streptococcus* spp., *S. pneumonia, S. pyogenes, S. agalactiae, Bacteroides* spp., *B. fragilis, B. vulgatus,* and *B. ovatus.* In some embodiments, the the antimicrobial agent is meropenem. In some embodiments, the antimicrobial agent is ceftriaxone.

In some embodiments, the antimicrobial agent exhibits a mutation prevention concentration (MPC) of about 0.01 µg/mL to about 32 µg/mL, about 0.05 µg/mL to about 1 µg/mL, or about 0.1 µg/mL to about 0.25 µg/mL against the bacterium implicated in the pathogenesis of SIBO is unable to or is not susceptible to develop resistance to the antimicrobial agent, such as about 0.01 µg/mL to about 1 µg/mL, about 0.01 µg/mL to about 0.5 µg/mL, about 0.01 µg/mL to about 0.25 µg/mL, about 0.01 µg/mL to about 0.12 µg/mL, about 0.01 µg/mL to about 0.06 µg/mL, about 0.06 µg/mL to about 1 µg/mL, about 0.06 µg/mL to about 0.5 µg/mL, about 0.06 µg/mL to about 0.25 µg/mL, about 0.06 µg/mL to about 0.12 µg/mL, about 0.12 µg/mL to about 1 µg/mL, about 0.12 µg/mL to about 0.5 µg/mL, about 0.12 µg/mL to about 0.25 µg/mL, about 0.25 µg/mL to about 1 µg/mL, about 0.25 µg/mL to about 0.5 µg/mL, or about 0.5 µg/mL to about 1 µg/mL. In some embodiments, the MPC is about 0.03 µg/mL, about 0.06 µg/mL, about 0.12 µg/mL, about 0.25 µg/mL, about 0.5 µg/mL, or about 32 µg/mL. In some embodiments, the bacterium is selected from *Escherichia coli, Streptococcus* spp., *S. pneumonia, S. pyogenes, S. agalactiae, Bacteroides* spp., *B. fragilis, B. vulgatus,* and *B. ovatus.* In some embodiments, the the antimicrobial agent is meropenem. In some embodiments, the antimicrobial agent is ceftriaxone.

### Analytes

The compositions and methods described herein can be used to detect, analyze, and/or quantitate a variety of analytes in a human subject. "Analyte" as used in the present application refers to a compound or composition to be detected in a sample. Exemplary analytes suitable for use in the present application include those described in U.S. Patent 6,251,581. Broadly speaking, an analyte can be any substance (e.g., a substance with one or more antigens) capable of being detected. An exemplary and non-limiting list of analytes includes ligands, proteins and fragments thereof, blood clotting factors, hormones, cytokines, polysaccharides, nucleic acids, carbohydrates, mucopolysaccharides, lipids, fatty acids, microorganisms (e.g., bacteria), microbial antigens, and therapeutic agents (including fragments and metabolites thereof).

For instance, the analyte may be a substance that binds to an analyte-binding agent (e.g., a biomolecule) and forms a complex. In some disclosures, the analyte may be monovalent (monoepitopic) or polyvalent (polyepitopic), usually antigenic or haptenic. In some disclosures, the analyte is a single compound or plurality of compounds. In some disclosures, the analyte is a plurality of compounds which share at least one common epitopic or determinant site. The analyte can be a part of a cell such as bacteria or a cell bearing a blood group antigen such as A, B, D, etc., a human leukocyte antigen (HLA), or other cell surface antigen. The analyte can also be a microorganism (*e*.*g*., bacterium (e.g. a pathogenic bacterium), a fungus, protozoan, or a virus), a protein, a nucleic acid, a lipid, or a hormone. In some disclosures, the analyte can be an exosome or a part of an exosome (*e*.*g*., a bacterial exosome). In some disclosures, the analyte is derived from a subject (e.g., a human subject). In some disclosures, the analyte is derived from a microorganism present in the subject. In some disclosures, the analyte is a nucleic acid (e.g., a DNA molecule or a RNA molecule), a protein (e.g., a soluble protein, a cell surface protein), or a fragment thereof, that can be detected using any of the devices and methods provided herein.

The polyvalent ligand analytes will normally be poly(amino acids), *e.g.,* a polypeptide (*e.g*., protein) or a peptide, polysaccharides, nucleic acids (e.g., DNA or RNA), and combinations thereof. Such combinations include components of bacteria, viruses, chromosomes, genes, mitochondria, nuclei, cell membranes, and the like.

In some disclosures, the polyepitopic ligand analytes have a molecular weight of at least about 5,000 Da, more usually at least about 10,000 Da. In the poly(amino acid) category, the poly(amino acids) of interest may generally have a molecular weight from about 5,000 Da to about 5,000,000 Da, more usually from about 20,000 Da to about 1,000,000 Da; among the hormones of interest, the molecular weights will usually range from about 5,000 Da to about 60,000 Da.

In some disclosures, the monoepitopic ligand analytes generally have a molecular weight of from about 100 to about 2,000 Da, more usually from about 125 to about 1,000 Da.

A wide variety of proteins may be considered as to the family of proteins having similar structural features, proteins having particular biological functions, proteins related to specific microorganisms, particularly disease causing microorganisms, etc. Such proteins include, for example, immunoglobulins, cytokines, enzymes, hormones, cancer antigens, nutritional markers, tissue specific antigens, etc.

In some disclosures, the analyte is a protein. In some disclosures, the analyte is a protein, e.g., an enzyme (e.g., a hemolysin, a protease, a phospholipase), a soluble protein, a membrane-bound protein, or an exotoxin. In some disclosures, the analyte is a fragment of a protein, a peptide, or an antigen. In some disclosures, the analyte is a peptide of at least 5 amino acids (e.g., at least 6, at least 7, at least 8, at least 9, at least 10, at least 25, at least, 50, or at least 100 amino acids). Exemplary lengths include 6, 7, 8, 9, 10, 11, 12, 13, 14, 15, 16, 17, 18, 19, 20, 21, 22, 23, 24, 25, 26, 27, 28, 29, 30, 50, 75, or 100 amino acids. Exemplary classes of protein analytes include, but are not limited to: protamines, histones, albumins, globulins, scleroproteins, phosphoproteins, antibodies, affimers, mucoproteins, chromoproteins, lipoproteins, nucleoproteins, glycoproteins, T-cell receptors, proteoglycans, cell surface receptors, membrane-anchored proteins, transmembrane proteins, secreted proteins, HLA, and unclassified proteins. In some disclosures, the analyte is an affimer (see, e.g., Tiede et al. (2017) eLife 6:e24903).

Exemplary analytes include: Prealbumin, Albumin, α₁-Lipoprotein, α₁-Antitrypsin, α₁-Glycoprotein, Transcortin, 4.6S-Postalbumin, α₁-glycoprotein, α_{1X}-Glycoprotein, Thyroxin-binding globulin, Inter-α-trypsin-inhibitor, Gc-globulin (Gc 1-1, Gc 2-1, Gc 2-2), Haptoglobin (Hp 1-1, Hp 2-1, Hp 2-2), Ceruloplasmin, Cholinesterase, α₂-Lipoprotein(s), Myoglobin, C-Reactive Protein, α₂-Macroglobulin, α₂-HS-glycoprotein, Zn-α₂-glycoprotein, α₂-Neuramino-glycoprotein, Erythropoietin, β-lipoprotein, Transferrin, Hemopexin, Fibrinogen, Plasminogen, β₂-glycoprotein I, β₂-glycoprotein II, Immunoglobulin G (IgG) or γG-globulin, Immunoglobulin A (IgA) or γA-globulin, Immunoglobulin M (IgM) or γM-globulin, Immunoglobulin D (IgD) or γD-Globulin (γD), Immunoglobulin E (IgE) or γE-Globulin (γE), Free κ and λ light chains, and Complement factors: C'1, (C'1q, C'1r, C'1s, C'2, C'3 (β₁A, α₂D), C'4, C'5, C'6, C'7, C'8, C'9.

Additional examples of analytes include tumor necrosis factor-α (TNFα), interleukin-12 (IL-12), IL-23, IL-6, α2β1 integrin, α1β1 integrin, α4β7 integrin, integrin α4β1 (VLA-4), E-selectin, ICAM-1, α5β1 integrin, α4β1 integrin, VLA-4, α2β1 integrin, α5β3 integrin, α5β5 integrin, αIIbβ3 integrin, MAdCAM-1, SMAD7, JAK1, JAK2, JAK3, TYK-2, CHST15, IL-1, IL-1α, IL-1ϑ, IL-18, IL-36α, IL-36ϑ, IL-36K, IL-38, IL-33, IL-13, CD40L, CD40, CD3K, CD3δ, CD3ε, CD3ζ, TCR, TCRα, TCRS, TCRδ, TCRK, CD14, CD20, CD25, IL-2, IL-2 ϑ chain, IL-2 K chain, CD28, CD80, CD86, CD49, MMP1, CD89, IgA, CXCL10, CCL11, an ELR chemokine, CCR2, CCR9, CXCR3, CCR3, CCR5, CCL2, CCL8, CCL16, CCL25, CXCR1m CXCR2m CXCL1, CXCL2, CXCL3, CXCL4, CXCL5, CXCL6, CXCL7, and CXCL8, and a nucleic acid (e.g., mRNA) encoding any of the same.

In some disclosures, the analyte is a blood clotting factor. Exemplary blood clotting factors include, but are not limited to:

| International designation | Name |
|---|---|
| I | Fibrinogen |
| II | Prothrombin |
| IIa | Thrombin |
| III | Tissue thromboplastin |
| V and VI | Proaccelerin, accelerator globulin |
| VII | Proconvertin |
| VIII | Antihemophilic globulin (AHG) |
| IX | Christmas factor plasma thromboplastin component (PTC) |
| X | Stuart-Prower factor, autoprothrombin III |
| XI | Plasma thromboplastin antecedent (PTA) |
| XII | Hagemann factor |
| XIII | Fibrin-stabilizing factor |

In some disclosures, the analyte is a hormone. Exemplary hormones include, but are not limited to: Peptide and Protein Hormones, Parathyroid hormone, (parathromone), Thyrocalcitonin, Insulin, Glucagon, Relaxin, Erythropoietin, Melanotropin (melancyte-stimulating hormone; intermedin), Somatotropin (growth hormone), Corticotropin (adrenocorticotropic hormone), Thyrotropin, Follicle-stimulating hormone, Luteinizing hormone (interstitial cell-stimulating hormone), Luteomammotropic hormone (luteotropin, prolactin), Gonadotropin (chorionic gonadotropin), Secretin, Gastrin, Angiotensin I and II, Bradykinin, and Human placental lactogen, thyroxine, cortisol, triiodothyronine, testosterone, estradiol, estrone, progestrone, luteinizing hormone-releasing hormone (LHRH), and immunosuppressants such as cyclosporine, FK506, mycophenolic acid, and so forth.

In some disclosures, the analyte is a peptide hormone (e.g., a peptide hormone from the neurohypophysis). Exemplary peptide hormones from the neurohypophysis include, but are not limited to: Oxytocin, Vasopressin, and releasing factors (RF) (e.g., corticotropin releasing factor (CRF), luteinizing hormone releasing factor (LRF), thyrotropin releasing factor (TRF), Somatotropin-RF, growth hormone releasing factor (GRF), follicle stimulating hormone-releasing factor (FSH-RF), prolactin inhibiting factor (PIF), and melanocyte stimulating hormone inhibiting factor (MIF)).

In some disclosures, the analyte is a cytokine or a chemokine. Exemplary cytokines include, but are not limited to: interleukin-1 (IL-1), interleukin-2 (IL-2), interleukin-6 (IL-6), epidermal growth factor (EGF), tumor necrosis factor (TNF, e.g., TNF-α or TNF-β), and nerve growth factor (NGF).

In some disclosures, the analyte is a cancer antigen. Exemplary cancer antigens include, but are not limited to: prostate-specific antigen (PSA), carcinoembryonic antigen (CEA), α-fetoprotein, Acid phosphatase, CA19.9, CA125, CD19, WT-1, CD22, L1-CAM, ROR-1, CD30, CD125, AFP, CEA, ETA, MAGE, and MUC16.

In some disclosures, the analyte is a tissue-specific antigen. Exemplary tissue specific antigens include, but are not limited to: alkaline phosphatase, myoglobin, CPK-MB, calcitonin, and myelin basic protein.

In some disclosures, the analyte is a mucopolysaccharide or a polysaccharide.

In some disclosures, the analyte is a microorganism, or a molecule derived from or produced by a microorganism (e.g., a bacteria, a virus, prion, or a protozoan). For example, in some disclosures, the analyte is a molecule (e.g., a protein or a nucleic acid) that is specific for a particular microbial genus, species, or strain (e.g., a specific bacterial genus, species, or strain). In some disclosures, the microorganism is pathogenic (*i.e*., causes disease). In some disclosures, the microorganism is non-pathogenic (*e.g*., a commensal microorganism). Exemplary microorganisms include, but are not limited to:

| | |
|---|---|
| **Corynebacteria** | |
| ***Corynebacterium diphtheria*** | |
| **Pneumococci** | |
| ***Diplococcus pneumoniae*** | |
| **Streptococci** | |
| ***Streptococcus pyrogenes*** | |
| ***Streptococcus salivarus*** | |
| **Staphylococci** | |
| ***Staphylococcus aureus*** | |
| ***Staphylococcus albus*** | |
| **Neisseria** | |
| ***Neisseria meningitidis*** | |
| ***Neisseria gonorrhea*** | |
| **Enterobacteriaciae** | |
| ***Escherichia coli*** | |
| ***Aerobacter aerogenes*** | **The coliform bacteria** |
| ***Klebsiella pneumoniae*** | |
| ***Salmonella typhosa*** | |
| ***Salmonella choleraesuis*** | **The Salmonellae** |
| ***Salmonella typhimurium*** | |
| ***Shigella dysenteria*** | |
| ***Shigella schmitzii*** | |
| ***Shigella arabinotarda*** | |
| | **The Shigellae** |
| ***Shigella flexneri*** | |
| ***Shigella boydii*** | |
| ***Shigella sonnei*** | |
| **Other enteric bacilli** | |
| ***Proteus vulgaris*** | |
| ***Proteus mirabilis*** | **Proteus species** |
| ***Proteus morgani*** | |
| ***Pseudomonas aeruginosa*** | |
| ***Alcaligenes faecalis*** | |
| ***Vibrio cholerae*** | |
| **Hemophilus-Bordetella group** | ***Rhizopus oryzae*** |
| **Hemophilus influenza, *H. ducryi*** | ***Rhizopus arrhizua*** |
| | **Phycomycetes** |
| ***Hemophilus hemophilus*** | ***Rhizopus nigricans*** |
| ***Hemophilus aegypticus*** | ***Sporotrichum schenkii*** |
| ***Hemophilus parainfluenza*** | ***Flonsecaea pedrosoi*** |
| ***Bordetella pertussis*** | ***Fonsecacea compact*** |
| **Pasteurellae** | ***Fonsecacea dermatidis*** |
| ***Pasteurella pestis*** | ***Cladosporium carrionii*** |
| ***Pasteurella tulareusis*** | ***Phialophora verrucosa*** |
| **Brucellae** | ***Aspergillus nidulans*** |
| ***Brucella melltensis*** | ***Madurella mycetomi*** |
| ***Brucella abortus*** | ***Madurella grisea*** |
| ***Brucella suis*** | ***Allescheria boydii*** |
| **Aerobic Spore-forming Bacilli** | ***Phialophora jeanselmei*** |
| ***Bacillus anthracis*** | ***Microsporum gypseum*** |
| ***Bacillus subtilis*** | ***Trichophyton mentagrophytes*** |
| ***Bacillus megaterium*** | ***Keratinomyces ajelloi*** |
| ***Bacillus cereus*** | ***Microsporum canis*** |
| **Anaerobic Spore-forming Bacilli** | ***Trichophyton rubrum*** |
| ***Clostridium botulinum*** | ***Microsporum adouini*** |
| ***Clostridium tetani*** | **Viruses** |
| ***Clostridium perfringens*** | **Adenoviruses** |
| ***Clostridium novyi*** | **Herpes Viruses** |
| ***Clostridium septicum*** | ***Herpes simplex*** |
| ***Clostridium histoyticum*** | **Varicella (Chicken pox)** |
| ***Clostridium tertium*** | **Herpes Zoster (Shingles)** |
| ***Clostridium bifermentans*** | **Virus B** |
| ***Clostridium sporogenes*** | **Cytomegalovirus** |
| **Mycobacteria** | **Pox Viruses** |
| ***Mycobacterium tuberculosis* hominis** | **Variola (smallpox)** |
| ***Mycobacterium bovis*** | **Vaccinia** |
| ***Mycobacterium avium*** | ***Poxvirus bovis*** |
| ***Mycobacterium leprae*** | **Paravaccinia** |
| ***Mycobacterium paratuberculosis*** | ***Molluscum contagiosum*** |
| **Actinomycetes (fungus-ike bacteria)** | **Picornaviruses** |
| ***Actinomyces Isaeli*** | **Poliovirus** |
| ***Actinomyces bovis*** | **Coxsackievirus** |
| ***Actinomyces naeslundii*** | **Echoviruses** |
| ***Nocardia asteroides*** | **Rhinoviruses** |
| ***Nocardia brasiliensis*** | **Myxoviruses** |
| **The Spirochetes** | **Influenza(A, B, and C)** |
| ***Treponema pallidum*** | **Parainfluenza (1-4)** |
| ***Treponema pertenue*** | **Mumps Virus** |
| ***Spirillum minus*** | |
| ***Streptobacillus monoiliformis*** | **Newcastle Disease Virus** |
| ***Treponema carateum*** | **Measles Virus** |
| ***Borrelia recurrentis*** | **Rinderpest Virus** |
| ***Leptospira icterohemorrhagiae*** | **Canine Distemper Virus** |
| ***Leptospira canicola*** | **Respiratory Syncytial Virus** |
| **Trypanasomes** | **Rubella Virus** |
| **Mycoplasmas** | **Arboviruses** |
| ***Mycoplasma pneumoniae*** | |
| **Other pathogens** | **Eastern Equine Encephalitis Virus** |
| ***Listeria monocytogenes*** | **Western Equine Encephalitis Virus** |
| ***Erysipeothrix rhusiopathiae*** | **Sindbis Virus** |
| ***Streptobacillus moniliformis*** | **Chikugunya Virus** |
| ***Donvania granulomatis*** | **Semliki Forest Virus** |
| ***Entamoeba histolytica*** | **Mayora Virus** |
| ***Plasmodium falciparum*** | **St. Louis Encephalitis** |
| ***Plasmodium japonicum*** | **California Encephalitis Virus** |
| ***Bartonella bacilliformis*** | **Colorado Tick Fever Virus** |
| **Rickettsia (bacteria-like parasites)** | **Yellow Fever Virus** |
| ***Rickettsia prowazekii*** | **Dengue Virus** |
| ***Rickettsia mooseri*** | **Reoviruses** |
| ***Rickettsia rickettsii*** | **Reovirus Types 1-3** |
| ***Rickettsia conori*** | **Retroviruses** |
| ***Rickettsia australis*** | **Human Immunodeficiency** |
| ***Rickettsia sibiricus*** | **Viruses I and II (HTLV)** |
| ***Rickettsia akari*** | **Human T-cell Lymphotrophic** |
| ***Rickettsia tsutsugamushi*** | **Virus I & II (HIV)** |
| ***Rickettsia burnetti*** | **Hepatitis** |
| ***Rickettsia quintana*** | **Hepatitis A Virus** |
| **Chlamydia (unclassifiable parasites bacterial/viral)** | **Hepatitis B Virus** |
| | **Hepatitis C Virus** |
| **Chlamydia agents (naming uncertain)** | **Tumor Viruses** |
| ***Chlamydia trachomatis*** | |
| **Fungi** | **Rauscher Leukemia Virus** |
| ***Cryptococcus neoformans*** | **Gross Virus** |
| ***Blastomyces dermatidis*** | **Maloney Leukemia Virus** |
| ***Histoplasma capsulatum*** | |
| ***Coccidioides immitis*** | **Human Papilloma Virus** |
| ***Paracoccidioides brasliensis*** | |
| ***Candida albicans*** | |
| ***Aspergillus fumigatus*** | |
| ***Mucor corymbifer* (*Absidia corymbifera*)** | |

In some disclosures, the analyte is a bacterium. Exemplary bacteria include, but are not limited to: *Escherichia coli* (or *E. coli*), *Bacillus anthracis, Bacillus cereus, Clostridium botulinum, Clostridium difficile, Yersinia pestis, Yersinia enterocolitica, Francisella tularensis, Brucella* species, *Clostridium perfringens, Burkholderia mallei, Burkholderia pseudomallei, Staphylococcus* species, *Mycobacterium* species, Group A *Streptococcus,* Group *B Streptococcus, Streptococcus pneumoniae, Helicobacter pylori, Salmonella enteritidis, Mycoplasma hominis, Mycoplasma orale, Mycoplasma salivarium, Mycoplasma fermentans, Mycoplasma pneumoniae, Mycobacterium bovis, Mycobacterium tuberculosis, Mycobacterium avium, Mycobacterium leprae, Rickettsia rickettsii, Rickettsia akari, Rickettsia prowazekii, Rickettsia canada, Bacillus subtilis, Bacillus subtilis niger, Bacillus thuringiensis, Coxiella burnetti, Faecalibacterium prausnitzii* (also known as *Bacteroides praussnitzii*), *Roseburia hominis, Eubacterium rectale, Dialister invisus, Ruminococcus albus, Ruminococcus callidus,* and *Ruminococcus bromii.* Additional exemplary bacteria include bacteria of the phyla Firmicutes (e.g., *Clostridium* clusters XIVa and IV), bacteria of the phyla Bacteroidetes (e.g., *Bacteroides fragilis* or *Bacteroides vulgatus*), and bacteria of the phyla Actinobacteria (e.g., *Coriobacteriaceae* spp. or *Bifidobacterium adolescentis*). Bacteria of the *Clostridium* cluster XIVa includes species belonging to, for example, the *Clostridium, Ruminococcus, Lachnospira, Roseburia, Eubacterium, Coprococcus, Dorea,* and *Butyrivibrio* genera. Bacteria of the *Clostridium* cluster IV includes species belonging to, for example, the *Clostridium, Ruminococcus, Eubacterium* and *Anaerofilum* genera. In some disclosures, the analyte is *Candida, e.g., Candida albicans.* In some disclosures, the analyte is a byproduct from a bacterium or other microorganism, e.g., helminth ova, enterotoxin (*Clostridium difficile* toxin A; TcdA) or cytotoxin (*Clostridium difficile* toxin B; TcdB).

In some disclosures, the analyte is a pathogenic bacterium. Non-limiting examples of pathogenic bacteria belong to the genera *Bacillus, Bordetella, Borrelia, Brucella, Campylobacter, Chlamydia, Chlamydophila, Clostridium, Corynebacterium, Enterobacter, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio,* and *Yersinia.* Non-limiting examples of specific pathogenic bacterial species include a strain of *Bacillus anthracis,* a strain of a strain of *Bordetella pertussis,* a strain of a strain of *Borrelia burgdorferi,* a strain of a strain of *Brucella abortus,* a strain of a strain of *Brucella canis,* a strain of a strain of *Brucella melitensis,* a strain of a strain of *Brucella suis,* a strain of a strain of *Campylobacter jejuni, a* strain of *Chlamydia pneumoniae,* a strain of *Chlamydia trachomatis,* a strain of *Chlamydophila psittaci,* a strain of *Clostridium botulinum,* a strain of *Clostridium difficile,* a strain of *Clostridium perfringens,* a strain of *Clostridium tetani,* a strain of *Corynebacterium diphtheria,* a strain of *Enterobacter sakazakii,* a strain of *Enterococcus faecalis,* a strain of *Enterococcus faecium,* a strain of *Escherichia coli* (e.g., *E. coli* 0157 H7), a strain of *Francisella tularensis,* a strain of *Haemophilus influenza,* a strain of *Helicobacter pylori,* a strain of *Legionella pneumophila,* a strain of *Leptospira interrogans,* a strain of *Listeria monocytogenes,* a strain of *Mycobacterium leprae,* a strain of *Mycobacterium tuberculosis,* a strain of *Mycobacterium ulcerans,* a strain of *Mycoplasma pneumonia,* a strain of *Neisseria gonorrhoeae,* a strain of *Neisseria meningitides,* a strain of *Pseudomonas aeruginosa,* a strain of *Rickettsia rickettsia, a* strain of *Salmonella typhi* and *Salmonella typhimurium,* a strain of *Shigella sonnei,* a strain of *Staphylococcus aureus,* a strain of *Staphylococcus epidermidis,* a strain of *Staphylococcus saprophyticus,* a strain of *Streptococcus agalactiae,* a strain of *Streptococcus pneumonia, a* strain of *Streptococcus pyogenes,* a strain of *Treponema pallidum,* a strain of *Vibrio cholera, a* strain of *Yersinia enterocolitica,* and, a strain of *Yersinia pestis.*

In some disclosures, the analyte is a commensal bacterium (e.g., a probiotic). In some disclosures, the bacterium has been previously administered to a subject, e.g., as a live biotherapeutic agent. Exemplary commensal bacteria include, but are not limited to, *Faecalibacterium prausnitzii* (also referred to as *Bacteroides praussnitzii*), *Roseburia hominis, Eubacterium rectale, Dialister invisus, Ruminococcus albus, Ruminococcus gnavus, Ruminococcus torques, Ruminococcus callidus,* and *Ruminococcus bromii.*

In some disclosures, the analyte is a virus. In some disclosures, the virus is a pathogenic virus. Non-limiting examples of pathogenic viruses belong to the families Adenoviridae, Picornaviridae, Herpesviridae, Hepadnaviridae, Flaviviridae, Retroviridae, Orthomyxoviridae, Paramyxoviridae, Papovaviridae, Polyomavirus, Rhabdoviridae, and Togaviridae. In some disclosures, the analyte is a bacteriophage. In some disclosures, the devices and methods described herein are used to identify, characterize and/or quantify the virome in the GI tract of a subject (e.g., in vivo or ex vivo). In some disclosures, the relative abundance of Escherichia phage, Enterobacteria phage, and Caudovirales bacteriophages is determined using the devices and methods described herein, wherein Escherichia phage and Enterobacteria phage are more abundant in the mucosa of IBD patients than healthy controls.

In some disclosures, the analyte is a fungus. In some disclosures, the fungi is a pathogenic fungus. Non-limiting examples of pathogenic fungi belong to the genera *Asperfillus, Canidia, Cryptococcus, Histoplasma, Pneumocystis,* and *Stachybotrys.* Non-limiting examples of specific pathogenic fungi species include a strain of *Aspergillus clavatus, Aspergillus fumigatus, Aspergillus flavus, Canidia albicans, Cryptococcus albidus, Cryptococcus gattii, Cryptococcus laurentii, Cryptococcus neoformans, Histoplasma capsulatum, Pneumocystis jirovecii, Pneumocystis carinii,* and *Stachybotrys chartarum.*

In some disclosures, the analyte is a protozoan. In some disclosures, the analyte is a pathogenic protozoan. Non-limiting examples of pathogenic protozoa belong to the genera *Acanthamoeba, Balamuthia, Cryptosporidium, Dientamoeba, Endolimax, Entamoeba, Giardia, Iodamoeba, Leishmania, Naegleria, Plasmodium, Sappinia, Toxoplasma, Trichomonas,* and *Trypanosoma.* Non-limiting examples of specific pathogenic protozoa species include a strain of *Acanthamoeba* spp., *Balamuthia mandrillaris, Cryptosporidium canis, Cryptosporidium felis, Cryptosporidium hominis, Cryptosporidium meleagridis, Cryptosporidium muris, Cryptosporidium parvum, Dientamoeba fragilis, Endolimax nana, Entamoeba dispar, Entamoeba hartmanni, Entamoeba histolytica, Entamoeba coli, Entamoeba moshkovskii, Giardia lamblia, Iodamoeba butschlii, Leishmania aethiopica, Leishmania braziliensis, Leishmania chagasi, Leishmania donovani, Leishmania infantum, Leishmania major, Leishmania mexicana, Leishmania tropica, Naegleria fowleri, Plasmodium falciparum, Plasmodium knowlesi, Plasmodium malariae, Plasmodium ovale, Plasmodium vivax, Sappinia diploidea, Toxoplasma gondii, Trichomonas vaginalis, Trypanosoma brucei,* and *Trypanosoma cruzi.*

In some disclosures, the analyte is secreted by or expressed on the cell surface of a microorganism (e.g., a bacterium, a colonic bacterium, a viable bacterium, a dead bacterium, a parasite (e.g., *Giardia lamblia, Cryptosporidium, Cystoisosporiasis belli,* and *Balantidium coli*), a virus (e.g., a herpes virus, a cytomegalovirus, a herpes simplex virus, an Epstein-Barr virus, a human papilloma virus, a rotavirus, a human herpesvirus-8; Goodgame (1999) Curr. Gastroenterol. Rep. 1(4):292-300). In some disclosures, the analyte is secreted by or expressed on the cell surface of a Gram-negative bacterium (e.g., *E. coli, Helicobacter pylori*). In some disclosures, the analyte is secreted by or expressed on the cell surface (e.g., a bacterial surface epitope) of a Gram-positive bacterium (e.g., *Staphylococcus aureus, Clostridium botulinum, Clostridium difficile).*

In some disclosures, the analyte is a molecule expressed on the surface of a bacterial cell (e.g., a bacterial cell surface protein). In some disclosures, the analyte is a bacterial toxin (e.g., TcdA and/or TcdB from *Clostridium difficile*). In some disclosures, the analyte is CFA/I fimbriae, flagella, lipopolysaccharide (LPS), lipoteichoic acid, or a peptidoglycan. Non-limiting examples of bacterium that may express an analyte that can be detected using any of the devices and methods described herein include: *Bacillus anthracis, Bacillus cereus, Clostridium botulinum, Clostridium difficile, Escherichia coli, Yersinia pestis, Yersinia enterocolitica, Francisella tularensis, Brucella* species, *Clostridium perfringens, Burkholderia mallei, Burkholderia pseudomallei, Helicobacter pylori, Staphylococcus* species, *Mycobacterium* species, Group A *Streptococcus,* Group B *Streptococcus, Streptococcus pneumoniae, Francisella tularensis, Salmonella enteritidis, Mycoplasma hominis, Mycoplasma orale, Mycoplasma salivarium, Mycoplasma fermentans, Mycoplasma pneumoniae, Mycobacterium bovis, Mycobacterium tuberculosis, Mycobacterium avium, Mycobacterium leprae, Rickettsia rickettsii, Rickettsia akari, Rickettsia prowazekii, Rickettsia canada, Bacillus subtilis, Bacillus subtilis niger, Bacillus thuringiensis, Coxiella bumetti, Candida albicans, Bacteroides fragilis, Leptospira interrogans, Listeria monocytogenes, Pasteurella multocida, Salmonella typhi, Salmonella typhimurium, Shigella dysenteriae, Shigella flexneria, Shigella sonnei, Vibrio cholera,* and *Vibrio parahaemolyticus.*

In some disclosures, the analyte is a byproduct from a bacterium or another microorganism, e.g., helminth ova, enterotoxin *(Clostridium difficile* toxin A; TcdA), cytotoxin *(Clostridium difficile toxin* B; TcdB), and ammonia. In some disclosures, the analyte is an antigen from a microorganism (e.g., a bacteria, virus, prion, fungus, protozoan or a parasite).

In some disclosures, the analytes include drugs, metabolites, pesticides, pollutants, and the like. Included among drugs of interest are the alkaloids. Among the alkaloids are morphine alkaloids, which includes morphine, codeine, heroin, dextromethorphan, their derivatives and metabolites; cocaine alkaloids, which include cocaine and benzyl ecgonine, their derivatives and metabolites; ergot alkaloids, which include the diethylamide of lysergic acid; steroid alkaloids; iminazoyl alkaloids; quinazoline alkaloids; isoquinoline alkaloids; quinoline alkaloids, which include quinine and quinidine; diterpene alkaloids, their derivatives and metabolites.

In some disclosures, the analyte is a steroid selected from the estrogens, androgens, adrenocortical steroids, bile acids, cardiotonic glycosides and aglycones, which includes digoxin and digoxigenin, saponins and sapogenins, their derivatives and metabolites. Also included are the steroid mimetic substances, such as diethylstilbestrol.

In some disclosures, the analyte is a bile acid or a bile salt (also known as a conjugated bile acid). Bile acids are products of cholesterol synthesis that are synthesized in the liver, conjugated to taurine or glycine, and stored in the gallbladder until released into the small intestine. The primary bile acids are cholic acid, and chenodeoxycholic acid, which are deconjugated and dehydroxylated by intestinal bacteria (bile acid (BA)-metabolizing microbiota (BAMM)) to form the secondary bile acids deoxycholic acid and lithocholic acid, respectively. BA-metabolizing microbiota (BAMM) have been shown to influence host homeostasis through changes in BA composition. The devices and methods described herein may be used to identify, characterize and/or quantify BAMM in the GI tract of a subject (e.g., in vivo or ex vivo), for example, using the Clusters of Orthologous Groups (COG) database, which provides the distribution of bacterial functions in the core and dispensable genomes (Tatusov, R. L. et al., "The COG database: an updated version includes eukaryotes," BMC Bioinformatics 4:41 (2003)). In some disclosures, higher abundance of BAMM associated with bile salt hydrolase (BSH) activity (e.g., COG3049) is associated with IBD, higher abundance of BAMM associated with hydroxysteroid dehydrogenase (HSDH) activity (e.g., COG1902) is associated with Crohn's disease, and higher abundance of BAMM associated with alphadehydoxylase (ADH) activity (e.g., COG1062) is associated with inflamed GI mucosa, wherein abundance is relative to levels seen in healthy controls. The majority of bile acids (about 95%) are reabsorbed in the distal ileum and returned to the liver (see, *e.g.,* U.S. Publication No. 2017/0343535). Impaired absorption of bile acids in the ileum can lead to excess bile acids in the colon which can cause symptoms of bile acid malabsorption (BAM; also known as bile acid diarrhea), including watery stool and fecal incontinence. Interestingly, up to 50% of patients with irritable bowel syndrome with diarrhea (IBS-D) also have BAM (see, e.g., Camilleri et al. (2009) Neurogastroeterol. Motil. 21(7):734-43). In some disclosures, the presence, absence, and/or a specific level of one or more bile acids or bile salts in the GI tract of a subject is indicative of a condition or disease state (e.g., a GI disorder and/or a non-GI disorder (e.g., a systemic disorder or a liver disease)). In some disclosures, the compositions, devices, and methods described herein may be used to detect, analyze and/or quantify at least one bile acid or bile salt in the GI tract of the subject to diagnose a GI disorder such as BAM or IBS (e.g., IBS-D). In some disclosures, the devices, methods and compositions described herein can be used to detect, quantitate, and/or analyze a bile acid or a bile salt in the GI tract of a subject. For instance, the presence and/or absence, and/or the concentration of a bile acid, a bile salt, or a combination thereof, may be determined at a specific region of the GI tract of a subject (e.g., one or more of the duodenum, jejunum, ileum, ascending colon, transverse colon or descending colon) to determine whether the subject has or is at risk of developing a GI disorder, such as BAM or IBS-D. In some disclosures, the devices, methods and compositions described herein can be used to determine the ratio of two or more bile acids or bile acid salts in the GI tract of a subject (e.g., a specific region of the GI tract of a subject including one or more of the duodenum, jejunum, ileum, ascending colon, transverse colon or descending colon). In some disclosures, the presence and/or absence, and/or the concentration of a bile acid, a bile salt, or a combination thereof, is determined in the ileum of a subject. In some disclosures, the presence and/or absence, and/or the concentration of a bile acid, a bile salt, or a combination thereof, is determined in the colon of a subject. In some disclosures, the concentration of a bile acid, a bile salt, or a combination thereof, is determined in specific regions of the GI tract of the subject, and for example, compared to determine where along the GI tract the compounds are accumulating. In some disclosures, the detection of a concentration of a bile acid, bile salt, or a combination thereof, in a specific region of the GI tract of the subject (e.g., the colon or the ileum) that is above a reference level of a bile acid, bile salt, or a combination thereof (e.g., the average level of a bile acid in healthy subjects) may be indicative of BAM and/or IBS-D in a subject. In some disclosures, the bile acid is selected from the group consisting of chenodeoxycholic acid, cholic acid, deoxycholate, lithocholate, and ursodeoxycholic acid. In some disclosures, the bile acid comprises cholesten-3-one or a structural variant thereof. In some disclosures, the bile acid is cholesten-3-one or a structural variant thereof. In some disclosures, the bile acid is cholesten-3-one. In some disclosures, the bile acid is a structural variant of cholesten-3-one. In some embodiments, the bile salt is selected from the group consisting of glycocholic acid, taurocholic acid, glycodeoxycholic acid, glycochenodeoxycholic acid, taurodeoxycholic acid, taurochenodeoxycholic acid, glycolithocholic acid, and taurolithocholic acid.

In some disclosures, the analyte is 7α-hydroxy-4-cholesten-3-one (7αC4). The measurement of 7αC4 allows for the monitoring of the enzymatic activity of hepatic cholesterol 7α-hydroxylase, the rate limiting enzyme in the synthesis of bile acids and can be used as a surrogate to detect BAM (see, e.g., Galman et al. (2003) J. Lipid. Res. 44:859-66; and Camilleri et al. (2009) Neurogastroeterol. Motil. 21(7):734-43).

In some disclosures, the analyte comprises cholesterol, a lipid, a fat soluble vitamin (e.g., ascorbic acid, cholecalciferol, ergocalciferol, a tocopherol, a tocotrienol, phylloquinone, and a menaquinone), bilirubin, fibroblast growth factor 19 (FGF19), TGR5 (also known as GP-BAR1 or M-BAR), glycine, taurine, or cholecystokinin (CCK or CCK-PZ). In some disclosures, the analyte comprises cholecystokinin. Cholecystokinin is a peptide hormone that contributes to control intestinal motility (see Rehfeld (2017) Front. Endocrinol. (Lausanne) 8:47). In some disclosures, the analyte comprises secretin. Secretin is a peptide hormone that regulates the pH of the duodenal content by controlling gastric acid secretion, regulates bile acid and bicarbonate secretion in the duodenum, and regulates water homeostasis (see, e.g., Afroze et al. (2013) Ann. Transl. Med. 1(3):29). In some disclosures, a subject has been administered cholecystokinin or secretin to induce the release of an analyte (e.g., from the liver and/or gall bladder into the GI tract).

In some disclosures, the analyte is a metabolite in the serotonin, tryptophan and/or kynurenine pathways, including but not limited to, serotonin (5-HT), 5-hydroxyindole acetic acid (5-HIAA), 5-hydroxytryptophan (5-HTP), kynurenine (K), kynurenic acid (KA), 3-hydroxykynurenine (3-HK), 3-hydroxyanthranilic acid (3-HAA), quinolinic acid, anthranilic acid, and combinations thereof. 5-HT is a molecule that plays a role in the regulation of gastrointestinal motility, secretion, and sensation. Imbalances in the levels of 5-HT are associated with several diseases including inflammatory bowel syndrome (IBS), autism, gastric ulcer formation, non-cardiac chest pain, and functional dyspepsia (see, *e.g.,* Faure et al. (2010) Gastroenterology 139(1):249-58 and Muller et al. (2016) Neuroscience 321:24-41, and International Publication No. WO 2014/188377). Conversion of metabolites within the serotonin, tryptophan and/or kynurenine pathways affects the levels of 5-HT in a subject. Therefore, measuring the levels of one or more of the metabolites in this pathway may be used for the diagnosis, management and treatment of a disease or disorder associated with 5-HT imbalance including but not limited to IBS, autism, carcinoid syndrome, depression, hypertension, Alzheimer's disease, constipation, migraine, and serotonin syndrome. One or more analytes in the serotonin, tryptophan and/or kynurenine pathways can be detected and/or quantitated using, for example, methods and analyte-binding agents that bind to these metabolites including, e.g., antibodies, known in the art (see, e.g., International Publication No. WO 2014/188377).

In some disclosures, the analyte is a lactam having from 5 to 6 annular members selected from barbiturates, *e*.*g*., phenobarbital and secobarbital, diphenylhydantoin, primidone, ethosuximide, and metabolites thereof.

In some disclosures, the analyte is an aminoalkylbenzene, with alkyl of from 2 to 3 carbon atoms, selected from the amphetamines; catecholamines, which includes ephedrine, L-dopa, epinephrine; narceine; papaverine; and metabolites thereof.

In some disclosures, the analyte is a benzheterocyclic selected from oxazepam, chlorpromazine, tegretol, their derivatives and metabolites, the heterocyclic rings being azepines, diazepines and phenothiazines.

In some disclosures, the analyte is a purine selected from theophylline, caffeine, their metabolites and derivatives.

In some disclosures, the analyte is marijuana, cannabinol or tetrahydrocannabinol.

In some disclosures, the analyte is a vitamin such as vitamin A, vitamin B, *e*.*g*. vitamin B₁₂, vitamin C, vitamin D, vitamin E and vitamin K, folic acid, thiamine.

In some disclosures, the analyte is selected from prostaglandins, which differ by the degree and sites of hydroxylation and unsaturation.

In some disclosures, the analyte is a tricyclic antidepressant selected from imipramine, dismethylimipramine, amitriptyline, nortriptyline, protriptyline, trimipramine, chlomipramine, doxepine, and desmethyldoxepin.

In some disclosures, the analyte is selected from anti-neoplastics, including methotrexate.

In some disclosures, the analyte is an antibiotic as described herein, including, but not limited to, penicillin, chloromycetin, actinomycetin, tetracycline, terramycin, and metabolites and derivatives.

In some disclosures, the analyte is a nucleoside or nucleotide selected from ATP, NAD, FMN, adenosine, guanosine, thymidine, and cytidine with their appropriate sugar and phosphate substituents.

In some disclosures, the analyte is selected from methadone, meprobamate, serotonin, meperidine, lidocaine, procainamide, acetylprocainamide, propranolol, griseofulvin, valproic acid, butyrophenones, antihistamines, chloramphenicol, anticholinergic drugs, such as atropine, their metabolites and derivatives.

In some disclosures, the analyte is a metabolite related to a diseased state. Such metabolites include, but are not limited to spermine, galactose, phenylpyruvic acid, and porphyrin Type 1.

In some disclosures, the analyte is an aminoglycoside, such as gentamicin, kanamicin, tobramycin, or amikacin.

In some disclosures, the analyte is a pesticide. Among pesticides of interest are polyhalogenated biphenyls, phosphate esters, thiophosphates, carbamates, polyhalogenated sulfenamides, their metabolites and derivatives.

In some disclosures, the analyte has a molecular weight of about 500 Da to about 1,000,000 Da (e.g., about 500 to about 500,000 Da, about 1,000 to about 100,000 Da).

In some disclosures, the analyte is a receptor, with a molecular weight ranging from about 10,000 to about 2 x 10⁸ Da, more usually from about 10,000 to about 10⁶ Da. For immunoglobulins, IgA, IgG, IgE and IgM, the molecular weights will generally vary from about 160,000 Da to about 10⁶ Da. Enzymes will normally range in molecular weight from about 10,000 Da to about 1,000,000 Da. Natural receptors vary widely, generally having a molecular weight of at least about 25,000 Da and may be about 10⁶ or higher Da, including such materials as avidin, DNA, RNA, thyroxine binding globulin, thyroxine binding prealbumin, transcortin, etc.

In some disclosures, the term "analyte" further includes polynucleotide analytes such as those polynucleotides defined below. These include m-RNA, r-RNA, t-RNA, DNA, DNA-DNA duplexes, DNA-RNA duplexes, nucleic acid molecules comprising modified bases, locked nucleic acid molecules (LNA molecules), antagomirs, peptide nucleic acid molecules (PNA molecules), antisense RNA or DNA molecules (e.g., antisense molecules including modifications to the sugars, bases, backbone linkages that allow for specific detection), chimeric antisense oligonucleotides, antisense oligonucleotides comprising modified linkages, interference RNA (RNAi), short interfering RNA (siRNA); a micro, interfering RNA (miRNA); a small, temporal RNA (stRNA); or a short, hairpin RNA (shRNA); small RNA-induced gene activation (RNAa); small activating RNAs (saRNAs), etc. The term analyte also includes polynucleotide-binding agents, such as, for example, restriction enzymes, transcription factors, transcription activators, transcription repressors, nucleases, polymerases, histones, DNA repair enzymes, intercalating agents, chemotherapeutic agents, and the like.

In some disclosures, the analyte may be a molecule found directly in a sample such as a body fluid from a host. The sample can be examined directly or may be pretreated to render the analyte more readily detectible. Furthermore, the analyte of interest may be determined by detecting an agent probative of the analyte of interest (e.g., an analyte-binding agent), such as a specific binding pair member complementary to the analyte of interest, whose presence will be detected only when the analyte of interest is present in a sample. Thus, the agent probative of the analyte becomes the analyte that is detected in an assay.

In some disclosures, the analyte a nucleic acid (e.g., a bacterial DNA molecule or a bacterial RNA molecule (e.g., a bacterial tRNA, a transfer-messenger RNA (tmRNA)). See, e.g., Sjostrom et al. (2015) Scientific Reports 5:15329; Ghosal (2017) Microbial Pathogenesis 104:161-163; Shen et al. (2012) Cell Host Microbe. 12(4):509-520.

In some disclosures, the analyte is a component of an outer membrane vesicle (OMV) (e.g., an OmpU protein, Elluri et al. (2014) PloS One 9:e106731). See, e.g., Kulp and Kuehn (2010) Annual Review of microbiology 64:163-184; Berleman and Auer (2013) Environmental microbiology 15:347-354; Wai et al. (1995) Microbiology and immunology 39:451-456; Lindmark et al. (2009) BMC Microbiology 9:220; Sjostrom et al. (2015) Scientific Reports 5:15329.

In some disclosures, the analyte is G-CSF, which can stimulate the bone marrow to produce granulocytes and stem cells and release them into the bloodstream.

In some disclosures, the analyte is an enzyme such as glutathione S-transferase. For example, the ingestible device can include P28GST, a 28 kDa helminth protein from *Schistosoma* with potent immunogenic and antioxidant properties. P28GST prevents intestinal inflammation in experimental colitis through a Th2-type response with mucosal eosinophils and can be recombinantly produced (e.g., in *S. cerevisiae*). See, for example, U.S. Patent No. 9,593,313, Driss et al., Mucosal Immunology, 2016 9, 322-335; and Capron et al., Gastroenterology, 146(5):S-638.

In some disclosures, the analyte is a metabolite in the serotonin, tryptophan and/or kynurenine pathways, including but not limited to, serotonin (5-HT), 5-hydroxyindole acetic acid (5-HIAA), 5-hydroxytryptophan (5-HTP), kynurenine (K), kynurenic acid (KA), 3-hydroxykynurenine (3-HK), 3-hydroxyanthranilic acid (3-HAA), quinolinic acid, anthranilic acid, and combinations thereof.

In some disclosures, analytes are therapeutic agents, fragments thereof, and metabolites thereof (e.g., antibiotics). In some disclosures, analytes are biomarkers. In some disclosures, the analytes are antibodies. In some disclosures, the analytes are antibiotics. Exemplary antibiotic agents are set forth in the U.S. Patent Publication US 2006/0269485. Additional exemplary analytes (e.g., therapeutic agents (e.g., drugs), antibodies, antibiotics and biomarkers) are provided below. Additional exemplary analytes (e.g., biomarkers, antibodies, antibiotics, and therapeutic agents) are described in International Publication No. PCT/US2017/065139, filed December 7, 2017.

### Analyte-Binding Agents

Certain detection methods described below can utilize at least one analyte-binding agent in order to detect an analyte in a sample. An "analyte-binding agent" is a molecule that binds to a specific analyte. Some analyte-binding agents may comprise analytes (e.g., the analytes described above) in accordance with the ability of the analyte to bind to another molecule to be detected using the methods described below. For example, in some disclosures, the analyte-binding agent comprises an antibody when used as a reagent to detect and/or quantify an antigen that the antibody specifically binds to. However, in some disclosures, the antibody is an analyte (e.g., an antibody which is a drug, such as a TNFα antibody) and the analyte-binding agent comprises an antigen to which the antibody specifically binds, thereby allowing for its use as a reagent to detect and/or quantify the antibody. In some disclosures, the analyte-binding agent binds to analyte that is specific to a particular genus, species, or strain of a microorganism (e.g., a pathogenic bacteria). In some embodiments, an analyte-binding agent has an area on the surface or in a cavity which specifically binds to and is thereby defined as complementary with a particular spatial and polar organization of the analyte. In some disclosures, the analyte-binding agent and the corresponding analyte form a binding pair, such as, but not limited to, an immunological pair (such as antigen-antibody), a biotin-avidin pair, a hormone-hormone receptor pair, a nucleic acid duplex, IgG-protein A pair, a polynucleotide pair such as DNA-DNA, DNA-RNA, and the like. In some embodiments, the analyte-binding agent comprises an antibody (*e*.*g*., a monoclonal antibody), an affimer, an aptamer, an antigen, a receptor, a small molecule, and a nucleic acid (*e.g*., a DNA molecule or an RNA molecule). In some disclosures, either member of the binding pair (e.g., the analyte-binding agent and/or the analyte) can be detectably labeled as described herein.

In some disclosures, the analyte-binding agent comprises a portion of a nucleic acid that is complementary to the nucleic acid sequence of the target analyte. As used herein, "complementary" refers to the capacity for pairing through hydrogen binding between two nucleic acid sequences. For example, if a nucleic acid base at one position of the target analyte is capable of hydrogen bonding with a nucleic acid base at a corresponding position of an analyte-binding agent, then the bases are considered to be complementary to each other at that position. In some disclosures, 100% complementarity is not required. In some disclosures, 100% complementarity is required. Routine methods can be used to design an analyte-binding agent that binds to a nucleic acid sequence of a target analyte. In some disclosures, the analyte-binding agent comprises a nucleic acid sequence that is complementary to at least about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65 or more contiguous nucleotides or nucleosides present in the nucleic acid sequence of the target analyte (e.g., a DNA molecule or an RNA molecule). In general, the analyte-binding agents useful in the devices and methods described herein have at least about 80% sequence complementarity to a nucleic acid sequence of a target analyte, e.g., at least about 85%, at least about 90%, at least about 92%, at least about 94%, at least about 95%, at least about 96%, at least about 97%, at least about 98%, at least about 99%, or are about 100% complementary to a nucleic acid sequence of a target analyte).

In some disclosures, the analyte-binding agent comprises a detectable moiety such as a photosensitizer, a fluorescent compound, and/or chemiluminescent compound described herein. In some disclosures, the analyte-binding agent is capable of being detected by a detection system of a device described herein, e.g., an optical detection system.

### Ingestible Devices

Ingestible devices and their use are described, for example, in the following U.S. patent applications: USSN 14/460,893, entitled "Ingestible Medical Device," and filed August 15, 2014; USSN 15/514,413, entitled "Electromechanical Pill Device with Localization Capabilities," and filed March 24, 2017; USSN 15/680,400, entitled "Systems and Methods for Obtaining Samples using Ingestible Devices," filed on August 18, 2017; USSN 15/680,430, entitled "Sampling Systems and Related Materials and Methods," filed on August 18, 2017; USSN 15/699,848, entitled "Electromechanical Ingestible Delivery of a Dispensable Substance," filed on September 8, 2017; USSN 15/835,270, entitled "Gastrointestinal Tract Detection Methods, Device and Systems," and filed December 7, 2017; USSN 15/835,237, entitled "Gastrointestinal Tract Detection Methods, Device and Systems," and filed December 7, 2017; USSN 15/835,292, entitled "Gastrointestinal Tract Detection Methods, Device and Systems," and filed December 7, 2017; USSN 15/844,349, entitled "Ingestible Device and Associated Methods," filed December 15, 2017; USSN 15/844,381, entitled "Ingestible Device and Associated Methods," filed December 15, 2017; USSN 15/844,427, entitled "Ingestible Device and Associated Methods," filed December 15, 2017; 15/694,458, entitled "Systems and Methods for Extracting a Sample from an Ingestible Device," filed on March 15, 2018; USSN 15/940,407, entitled "Localization Systems and Methods for an Optoelectromechanical Pill Device," filed on March 29, 2018; and and USSN 62/642,544, entitled "Ingestible Device With Relatively Large Payload Volume," and filed March 13, 2018.

In general, an ingestible device is configured to be able to enter the GI tract (e.g., via the mouth) and collect one or more samples while passing through one or more regions of the GI tract. Optionally, the device can include one or more additional functionalities, including the ability to analyze the sample while in the GI tract of the subject (*in vivo*), the ability to deliver a substance (e.g., a therapeutic agent) while in the GI tract of the subject (*in vivo*) and/or the ability to locate the device outside the GI tract of the subject (*ex vivo*).

The ingestible device described herein may generally be in the shape of a capsule, like a conventional pill. As described herein, the device is an ingestible device. Accordingly, the shape of the device provides for easier ingestion, or insertion and removal, and is also familiar to healthcare practitioners and patients.

Unlike a conventional pill, the device is designed to withstand the chemical and mechanical environment of the GI tract (e.g., effects of muscle contractile forces and concentrated hydrochloric acid in the stomach). However, unlike other devices that are intended to stay inside a patient's body (e.g., medical implants), the ingestible device is designed (in general) to only temporarily travel within the body. Accordingly, the regulatory rules governing the materials and manufacture of the ingestible device may be less strict than for the devices that are intended to stay inside the body. Nevertheless, since the ingestible device still enters the body, the material(s) used to manufacture the ingestible device are generally selected to at least comply with the standards for biocompatibility (e.g., ISO 10993). Furthermore, components inside the ingestible device are free of any restricted and/or toxic metals and are lead-free pursuant to the Directive 2002/95/EC, which is also known as the Restriction of Hazardous Substances (RoHS).

There is a broad range of materials that may be used for manufacturing the ingestible device. Different materials may be used for each of the different components of the ingestible device. Examples of these materials include, but are not limited to, thermoplastics, fluoropolymers, elastomers, stainless steel and glass complying with ISO 10993 and USP Class VI specifications for biocompatibility. In certain embodiments, these materials may further include liquid silicone rubber material with a hardness level of 10 to 90 as determined using a durometer (e.g., MED-4942^{™} manufactured by NuSil^{™}), a soft biocompatible polymer material such as, but not limited to, polyvinyl chloride (PVC), polyethersulfone (PES), polyethylene (PE), polyurethane (PU) or polytetrafluoroethylene (PTFE), and a rigid polymer material coated with a biocompatible material that is soft or pliable (e.g., a poly(methyl methacrylate) (PMMA) material coated with silicone polymer). Use of different materials for different components may enable functionalization of certain surfaces for interaction with proteins, antibodies, and other biomarkers. For example, Teflon^{®} may be used as a material in the ingestible device for any movable components in order to reduce friction between these components. Other example materials may include other materials commonly used in microfabrication, such as polydimethylsiloxane (PDMS), borosilicate glass, and/or silicon.

Generally, an enclosure of the ingestible device may be manufactured from a type of plastic, such as a photosensitive acrylic polymer material. The enclosure may be formed by coupling two enclosure ends together. The enclosure, in effect, protects the interior of the ingestible device from its external environment and also protects the external environment (e.g., the GI tract) from components inside the device.

Furthermore, the device may include one or more additional layers of protection. The additional protection may protect the patient against any adverse effects arising from any structural problems associated with the enclosure (e.g., the two enclosure ends falling apart or a fracture developing in the enclosure). For example, a power supply inside the device may be coated with an inert and pliable material (e.g., a thin layer of silicone polymer) so that only electrical contacts on the power supply are exposed. This additional protection to the power supply may prevent chemicals inside the device from seeping into the patient's body.

Also, a surface of the device and surfaces of the different components in the device may receive different treatments that vary according to their intended use. For example, the surface of the device may receive plasma activation for increasing hydrophilic behavior. Dilution chambers, storage components, ports, valves, pumps and/or conduits that are intended to come into contact with a fluid such as biological fluid or dilution fluid during normal operation of the device may also receive hydrophilic treatment while certain other components may receive hydrophobic treatments.

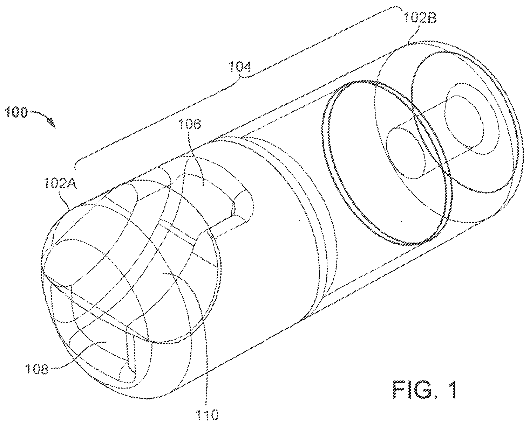

FIG. 1 illustrates an example ingestible device 100 with multiple openings in the housing. The ingestible device 100 has an outer housing with a first end 102A, a second end 102B, and a wall 104 extending longitudinally from the first end 102A to the second end 102B. Ingestible device 100 has a first opening 106 in the housing, which is connected to a second opening 108 in the housing. The first opening 106 of the ingestible device 100 is oriented substantially perpendicular to the second opening 108, and the connection between the first opening 106 and the second opening 108 forms a curved chamber 110 within the ingestible device 100.

The overall shape of the ingestible device 100, or any of the other ingestible devices discussed in this disclosure, may be similar to an elongated pill or capsule. This may make the ingestible device 100 easy to consume, and allow it to travel easily through the GI tract. In certain portions of the GI tract, such as the stomach, the ingestible device 100 may be free to move or rotate in any direction. In other portions of the GI tract, the movement of the ingestible device 100 may be restricted. For example, in the relatively narrow confines of the small intestine, the walls of the small intestine may squeeze down on the ingestible device, forcing the ingestible device 100 to orient itself longitudinally along the length of the small intestine. In this case, the walls of the small intestine wrap around the longitudinally extending wall 104 of the ingestible device 100, and the ingestible device 100 travels through the small intestine with one of the ends 102A or 102B in front.

For illustrative purposes, the ingestible device 100 of FIG. 1 shows the first opening 106 located in a portion of the wall 104 and oriented radially, and the second opening 108 located near the first end 102A and oriented longitudinally. However, in some embodiments, the exact location and orientation of the first opening 106 and the second opening 108 may be different from that shown in FIG. 1. During transit through the GI Tract, natural contractions within the small intestine may apply pressure radially to different portions of the wall 104 of the ingestible device 100, which may force solids or fluids into the first opening 106. As new material (e.g., fluid and solid particulates from the small intestine or other portions of the GI tract) enters the curved chamber 110 through the first opening 106, older material already located in the curved chamber 110 may be naturally forced out of the curved chamber 110 through the second opening 108.

In some embodiments, a portion of the curved chamber 110 may be used as a sampling chamber, which may hold samples obtained from the GI tract. In some embodiments the curved chamber 110 is subdivided into sub-chambers, each of which may be separated by a series of one or more valves or interlocks. For example, sub-chambers may be used to retain multiple samples within different portions of the curved chamber 110. In some embodiments, the curved chamber 110 is connected to other chambers within the ingestible device 100, or other openings located on the housing of the ingestible device 100. This may allow new samples to be acquired in the curved chamber 110 while older samples of interest are still stored within the ingestible device 100. In some embodiments, the ingestible device 100 is equipped with sensors to detect the properties a sample contained in the sampling chamber, or the results of an assay technique applied to the sample. In some embodiments, the ingestible device 100 is configured to obtain and retain a sample within the sampling chamber, which may be retrieved at a later time.

In some embodiments, the first opening 106, the second opening 108, or the curved chamber 110 include one or more of a hydrophilic or hydrophobic material, a sponge, a valve, or an air permeable membrane. For example, a one-way valve may prevent material from entering the curved chamber 110 through the second opening 108. As an alternate example, placing an air permeable membrane within the curved chamber 110 near the second opening 108 may allow unwanted gasses and air bubbles to pass through the air permeable membrane and exit the curved chamber 110, while solid or liquid samples may be prevented from passing through the air permeable membrane, and are retained within the curved chamber 110. The air permeable membrane may also prevent solid or liquid samples from entering the curved chamber 110 through the second opening 108.

The use of a hydrophilic material or sponge may allow samples to be retained within the curved chamber 110, and may reduce the amount of pressure needed for fluid to enter through the first opening 106 and dislodge air or gas in the curved chamber 110. Examples of hydrophilic materials that may be incorporated into the ingestible device 100 include hydrophilic polymers such as polyvinyl alcohol, polyvinyl pyrrolidone, and the like. Similarly, materials that have undergone various types of treatments, such as plasma treatments, may have suitable hydrophilic properties, and may be incorporated into the investible device 100. Sponges may be made of any suitable material or combination of materials, such as fibers of cotton, rayon, glass, polyester, polyethylene, polyurethane, and the like. Sponges generally may be made from commercially available materials, such as those produced by Porex^{®}.

In some embodiments, the sponges may be treated in order to change their absorbency or to help preserve samples. Examples of materials which may be used to treat the sponges, alone or in combination, include sorbic acid, propyl parabene, citric acid, surfactants such as Tween^{®} (polysorbate), DNA inhibitors and stabilizers, RNA inhibitors and stabilizers, protein inhibitors and stabilizers, and the like. In some embodiments, the sponges may be cut or abraded to change their absorbency or other physical properties.

Hydrophobic materials located near the second opening 108 may repel liquids, discouraging liquid samples from entering or exiting the curved chamber 110 through the second opening 108. This may serve a similar function as an air permeable membrane. Examples of hydrophobic materials which may be incorporated into the ingestible device 100 include polycarbonate, acrylics, fluorocarbons, styrenes, certain forms of vinyl, and the like.

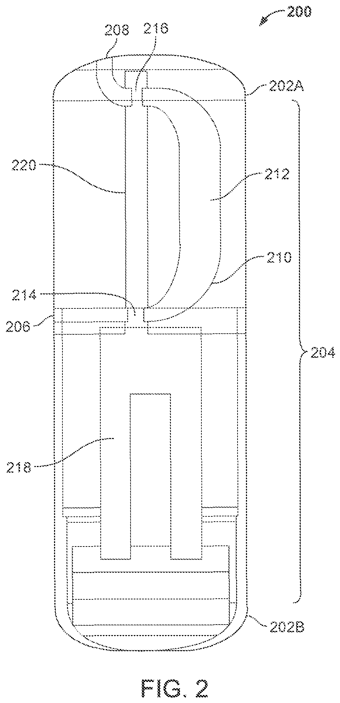

The various materials listed above are provided as examples, and are not limiting. In practice, any type of suitable hydrophilic, hydrophobic, or sample preserving material may be used in the ingestible device 100, and the teachings discussed in relation to ingestible device 100 may be incorporated into any of the other ingestible devices described in this disclosure. Various methods for taking samples, controlling the movement of samples, or removing unwanted gasses, are discussed in detail in relation to FIGs. 2-9, and any of the various structures or techniques described in connection with FIGs. 2-9 may be incorporated into the ingestible device 100.

FIG. 2 illustrates an example ingestible device 200 with multiple openings in the housing and various modifications that may be made to the ingestible device 100 (FIG. 1). Similar to the ingestible device 100, the ingestible device 200 has an outer housing with a first end 202A, a second end 202B, and a wall 204 extending longitudinally from the first end 202A to the second end 202B. Also similar to the ingestible device 100, the ingestible device 200 has a first opening 206 in the housing, which is connected to a second opening 208 in the housing. The connection between the first opening 206 and the second opening 208 forms a curved chamber 210 within the ingestible device 200.

In the ingestible device 200, a portion of the curved chamber 210 forms a sampling chamber 212. In some embodiments, the ingestible device 200 may include a sensor (not shown) within or proximate to the sampling chamber. This sensor may be used to detect a property of the sample. In some embodiments, an assay technique is applied to a sample within the sampling chamber, and the sensor may be used to detect the results of the assay technique. A first valve 214 is located between the first opening 206 and the sampling chamber 212. Similarly, a second valve 216 is located between the second opening 208 and the sampling chamber 212. In some embodiments, the valves 214 and 216 prevent a fluid from entering or exiting the sampling chamber 212, or may be used to isolate a sample within the sampling chamber 212.

The ingestible device 200 includes a mechanical actuator 218 coupled to the valves 214 and 216. In some embodiments, the mechanical actuator 218 is used to move one or both of the valves 214 and 216 between an open and a closed position. In some embodiments, the mechanical actuator 218 is controlled by a microcontroller, microprocessor, or other circuitry inside the ingestible device 200. In an open position, the first valve 214 may allow a sample to pass in and out of the sampling chamber 212 through the portion of the curved chamber 210 connected to the first opening 206. Similarly, in an open position, the second valve 216 may allow a sample to pass in and out of the sampling chamber 212 from the portion of the curved chamber 210 connected to the second opening 208. When the valves 214 and 216 are in the closed positions, they may not allow a sample to pass into or out of the sampling chamber 212.

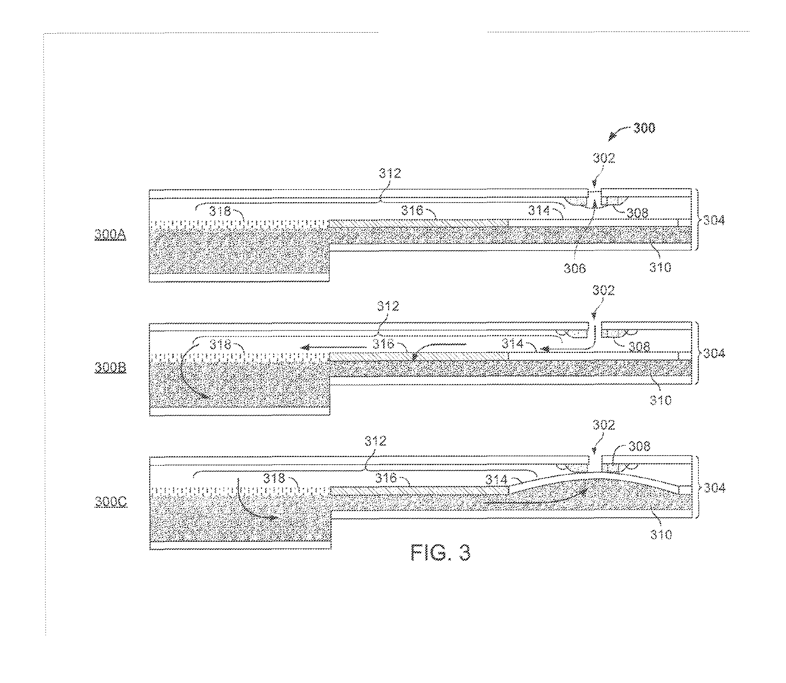

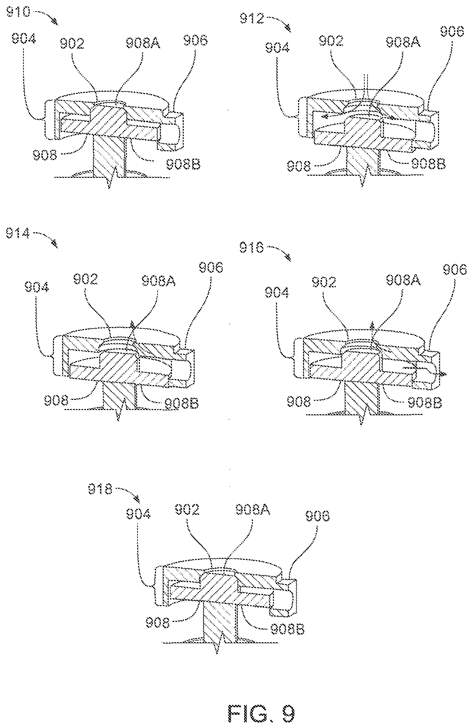

In some embodiments, the valves 214 and 216 are rotary valves, pin valves, flap valves, butterfly valves, ball valves, plug valves, or any other suitable type of one-way or two-way valves, and may be the same or different types of valves. In some embodiments, one or both of the valves 214 and 216 are automatic valves that reseal themselves after a sample has been obtained, similar to the osmotic valve mechanism discussed in relation to FIG. 3. In some embodiments, one or both of the valves 214 and 216 include a pumping mechanism, such as the pumping mechanism discussed in relation to FIG. 9. For illustrative purposes, the ingestible device 200 is depicted with both of the valves 214 and 216 as moveable two-way valves coupled to the mechanical actuator 218. However, in some embodiments, the mechanical actuator 218 is coupled to only one of the valves, and the other valve may be replaced with a passive one-way valve. For example, the mechanical actuator 218 may be coupled to only the first valve 214, and the second valve 216 may be replaced with a passive one-way valve that allows gases, fluids, or solids to exit the sampling chamber 212 through the portion of the curved chamber 210 connected to the second opening 208. This may restrict fluid from entering the sampling chamber 212 from the second opening 208, but allow unwanted material to be removed from the sampling chamber 212 as the sample is obtained.

In some embodiments, the ingestible device 200 may be able to detect the approximate location of the ingestible device 200 within the GI tract. For example, it may be possible to use various combinations of light emitting diodes and sensors positioned along the ingestible device 200 to determine whether the device is in the stomach, small intestine, or large intestine. Methods for determining the location of an ingestible device within a GI tract are described in greater detail elsewhere herein. In these embodiments, the ingestible device 200 may be configured to use the mechanical actuator 218 to move the valves 214 and 216 into an open position in response to determining that the ingestible device 200 has reached a predetermined location within the GI tract. For example, a microcontroller on board the ingestible device 200 may be configured to open the valves 214 and 216 only when the ingestible device 200 is within the small intestine, thereby obtaining a sample from within the small intestine.

For illustrative purposes, the ingestible device 200 is depicted with the mechanical actuator 218, the first valve 214, and the second valve 216 oriented in a substantially straight line, with a single shaft 220 being used to couple the mechanical actuator 218 to the valves 214 and 216. However, in some embodiments, the orientation and/or positioning of the valves 214 and 216 relative to the position of the mechanical actuator 218 may be different than that shown, and the coupling of the mechanical actuator 218 to the valves 214 and 216 may also be different. In some embodiments, the mechanical actuator 218 simultaneously moves the valves 214 and 216. For example, in some embodiments the valves 214 and 216 are rotary valves, and they may be simultaneously opened and closed by rotating the shaft 220 that extends from the mechanical actuator 218 along the length of the ingestible device 200. As an alternate example, the valves 214 and 216 may be pin valves, and the pins may be attached to the shaft 220 that extends from the mechanical actuator 218 along the length of the ingestible device 200. In this case, the mechanical actuator 218 may open and close the valves by moving the shaft 220 linearly. This may be accomplished either by configuring mechanical actuator 218 to be a linear actuator, such as a solenoid. Alternately, the mechanical actuator 218 may be a rotary actuator, and the rotation may be converted into a linear motion. One skilled in the art will understand that this may be done any number of ways, for example, by coupling the mechanical actuator 218 to a ball screw mechanism, a threaded lead nut and lead screw mechanism, a rack and pinion mechanism, or the like.

In some embodiments, the ingestible device 200 does not include the second valve 216 at all. In this case, fluids and solids contained within the sampling chamber 212 may be free to exit through the second opening 208. Alternately, the second valve 216 near the second opening 208 may be replaced by an air-permeable membrane, which may allow gasses and unwanted air bubbles to exit the sampling chamber 212 through the second opening 208, while still retaining fluids and/or solids within the sampling chamber 212. Alternately, the second valve 216 near the second opening 208 may be replaced with a hydrophobic material. Similar to an air permeable membrane, an appropriately positioned hydrophobic material may be used to line the walls of the curved chamber 210 proximate to the second opening 208, which may allow gasses or unwanted air bubbles to exit the sampling chamber 212 through the second opening 208, while restricting some fluids from entering or exiting the sampling chamber 212 through the second opening 208. In some embodiments, one or more of the above described mechanisms may be combined in the same ingestible device. For example, the ingestible device 200 may implement the second valve 216 as a two-way valve, and also have hydrophobic material and an air-permeable membrane located near the second opening 208.

**In** some embodiments, the curved chamber 210 is connected to one or more sub-chambers (not shown). Each of these sub-chambers may be configured to hold one or more samples, and isolate the samples from both the sampling chamber 212, and the other sub-chambers. For example, each sub-chamber may be connected to the curved chamber 210 through a one-way valve, allowing samples to enter the sub-chamber from the curved chamber 210, but preventing the obtained samples from exiting the sub-chamber and re-entering either the curved chamber 210 or the sampling chamber 212. **In** general, any type of valve or other suitable mechanism may be used to isolate samples contained in the sub-chambers. In some embodiments, the ingestible device 200 distributes different samples into different sub-chambers at different times, or from different locations of the GI tract. For example, the ingestible device 200 may obtain a sample from the duodenum and distribute it into a first sub-chamber, and the ingestible device 200 may later obtain a sample from the ileum and distribute it into a second sub-chamber. In some embodiments, different types of assay techniques or diagnostics are applied to some of the samples contained in the different sub-chambers.

FIG. 3 illustrates an example of an osmotic valve mechanism 300, which may be incorporated into an ingestible device in order to obtain samples. The osmotic valve mechanism 300 may be used in an ingestible device that features a first end, a second end, and a wall extending longitudinally between the first end and the second end, similar to the shape of the ingestible devices 100 (FIG. 1) and 200 (FIG. 2).

The osmotic valve mechanism 300 includes an inlet port 302, which is connected to a sampling chamber 304. In some embodiments, the inlet port 302 connects sampling chamber 304 directly or indirectly to an opening in the housing of an ingestible device.

The initial state of the osmotic valve mechanism 300 is shown in diagram 300A. As shown in diagram 300A, the inlet port 302 of the osmotic valve mechanism 300 is sealed using a single use sealing device 306 positioned within the inlet port 302. The single use sealing device 306 is positioned adjacent to a heating element 308. When it is time for the osmotic valve mechanism 300 to be opened (which may be determined by a localization mechanism that determines the ingestible device is located in a desirable portion of the **GI** tract), the heating element 308 applies heat to the sealing device 306, causing the sealing device 306 to deform and unseal the inlet port 302.

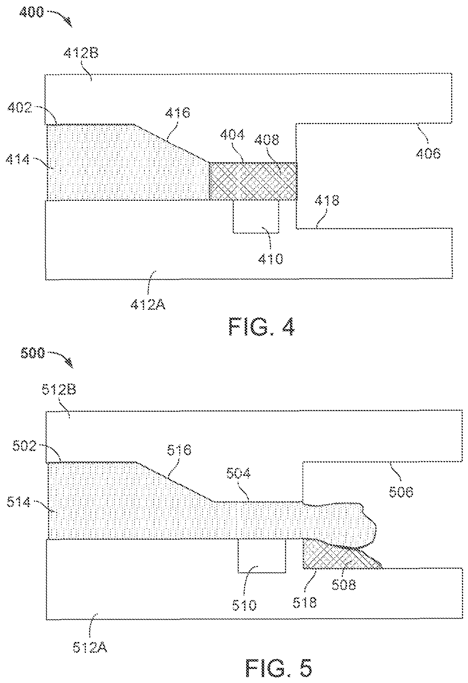

In some embodiments, the sealing device 306 may be a plug made out of a material that is meltable, deformable, and/or destroyable through the use of the heating element 308, such as wax. For example, in some embodiments, the heating element 308 may be a resistive heater that undergoes ohmic heating as an electrical current is passed through it, and the sealing device 306 is a wax plug. In some embodiments, the type of wax used to form the wax plug has a melting point between 38 degrees and 80 degrees Celsius, which is above the ambient temperature of a human body, but which may be easily achieved using the heating element 308. Some embodiments of the osmotic valve mechanism 300 may use a sealing device 306 that is melted or deformed at temperatures outside of the range described above, but practical considerations may be made to ensure that the osmotic valve mechanism 300 does not cause unwanted damage or burning to the GI tract. In some embodiments, a microprocessor is configured to control the heating element 308, causing it to generate heat. For example, the microprocessor may be configured to activate the heating element 308 once the ingestible device reaches a particular location within the GI tract. An example mechanism for unsealing the inlet port 302 is described in greater detail in relation to FIGs. 4 and 5. Although FIGs. 3, 4, and 5 depict the sealing device 306 as a type of plug, any type of suitable sealing device may be used. For example, in some embodiments, the sealing device includes a breakable membrane, which may be destroyed when heat is applied to the membrane. In some embodiments, the osmotic valve mechanism 300 does not include a heating element 308, and the sealing device 306 is melted, deformed, destroyed, or dislodged from the inlet port 302 by a mechanical actuator, or through electromagnetic fields. For example, the sealing device 306 may be a membrane that will rupture when a sufficiently large electrical current or magnetic field is applied to the membrane.

Inside the sampling chamber 304 of the osmotic valve mechanism 300 is made of a member including an absorptive material 310, and at least a portion of the absorptive material 310 is located near the inlet port 302. The absorptive material 310 may include any suitable sponge material or hydrophilic material, such as any of the materials described in relation to FIG. 1. The portion of the absorptive material 310 located near the inlet port 302 may have a tendency to expand when it comes into contact with fluids. The osmotic valve mechanism 300 has a barrier 312 inside the sampling chamber 304, which is divided into three portions. The first portion of the barrier 312 is a flexible membrane 314, the second portion of the barrier 312 adjacent to the flexible membrane 314 is a rigid portion 316, and the third portion of the barrier 312 adjacent to the rigid portion 316 is a semi-permeable membrane 318.

The barrier 312 within the sampling chamber 304 is positioned between the inlet port 302 and the absorptive material 310, covering a surface of the absorptive material 310. When the inlet port 302 is unsealed, a sample (e.g., a fluid sample containing solid particulates taken from the GI tract) enters the sampling chamber 304 through the inlet port 302, and begins to fill the sampling chamber 304. The absorptive material 310 may have a natural tendency to expand when it comes into contact with a fluid sample. However, by covering a surface of the absorptive material 310, the barrier 312 may allow only certain portions of absorptive material 310 to expand. The barrier 312 may also direct the flow of a fluid sample as it enters the sampling chamber 304, and allow the fluid sample to come into contact with only certain parts of the absorptive material 310.

Diagram 300B shows the osmotic valve mechanism 300 shortly after the inlet port 302 is unsealed. Once the inlet port 302 is unsealed, the sampling chamber 304 may be opened, and a sample may enter the sampling chamber 304 through the inlet port 302. In some embodiments, the sample cannot cross the flexible membrane 314 and contact the absorptive material 310. As a result, the flexible membrane 314 may be used to guide the sample as it enters the sampling chamber 304. Similarly, in some embodiments the sample cannot cross the rigid portion 316 of the barrier 312, and the rigid portion 316 may also be used to guide the sample as it enters the sampling chamber 304. The semi-permeable membrane 318 allows at least a portion of the sample to pass through the semi-permeable membrane and contact the absorptive material 310. This may allow the sample to be absorbed by the absorptive material 310 after the sample has filled the top portion of the sampling chamber 304, which in turn may cause the absorptive material 310 to begin to expand.

Diagram 300C shows the state of the osmotic valve mechanism 300 after the absorptive material 310 has absorbed a portion of the sample. The portion of the absorptive material 310 under the flexible membrane 314 expands when the absorptive material 310 absorbs the sample. As the absorptive material 310 expands, the flexible membrane 314 is forced up against the inlet port 302, effectively sealing the inlet port 302 from the sampling chamber 304. In some embodiments, the rigid portion 316 prevents the portion of the absorptive material 310 under the rigid portion 316 from expanding. In some embodiments, the semi-permeable membrane 318 may be rigid, and prevent the portion of the absorptive material 310 adjacent to the semi-permeable membrane 318 from expanding.

After the absorptive material 310 expands, causing the inlet port 302 to be resealed, a portion of the sample may be confined within the sampling chamber 304. Once a sample has been properly confined, it may be possible to apply a wide range of assay techniques or diagnostics to the sample. In some embodiments, the portion of the sampling chamber 304 between the rigid portion 316 and the wall of the sampling chamber forms a testing area. For example, a sensor may be placed within or proximate to the sampling chamber 304 in order to study the portion of the sample contained within the testing area located above the rigid portion 316. This sensor may be used to study properties of the sample, or it may be used to detect the results of an assay technique applied to the sample.

Diagram 300C is shown for illustrative purposes only, and is not limiting. In some embodiments, the osmotic valve mechanism 300 does not include the barrier 312, or one or more portions of the barrier 312 are excluded or rearranged within the sampling chamber 304. For example, the location of the rigid portion 316 and the semi-permeable membrane 318 may be reversed, or the rigid portion 316 may be removed and the semi-permeable membrane 318 extended so that it connects directly with the flexible membrane 314. When the osmotic valve mechanism 300 does not include a barrier 312 or does not include the flexible membrane 314, a portion of the absorptive material 310 near the inlet port 302 may expand and clog the inlet port 302, effectively resealing the inlet port 302.

In some embodiments, the material used to form the absorptive material 310 expands at a controlled rate, which may ensure that sufficient time has passed for the sample to enter the sampling chamber 304 and for the sampling chamber 304 to be filled before the inlet port 302 is resealed. This may be particularly useful for embodiments where the osmotic valve mechanism 300 does not include a flexible membrane 314 and/or the semi-permeable membrane 318. In some embodiments, a portion of the absorptive material 310 is covered by a dissolvable film or membrane, which may prevent the absorptive material 310 from expanding until a sufficient amount of time has passed for the film to dissolve.

In some embodiments, the sampling chamber 304 is connected to one or more sub-chambers (not shown). Each of these sub-chambers may be configured to hold samples, and isolate the samples from both the sampling chamber 304, and the other sub-chambers. For example, each sub-chamber may be connected to the sampling chamber 304 through a one-way valve, allowing samples to enter the sub-chamber from the sampling chamber, but preventing the obtained samples from exiting the sub-chamber. As an alternate example, each of the sub-chambers may employ a sealing device, heating element, and member made of absorptive material arranged similar to osmotic valve mechanism 300. In these embodiments, each of the sub-chambers may be opened by activating their respective heating elements, and may be automatically sealed off from the sampling chamber 304 after a sufficient amount of the sample has been obtained. In general, any type of valve or other suitable mechanism may be used to isolate samples contained in the sub-chambers. In some embodiments, similar to ingestible device 200, an ingestible device employing multiple sub-chambers in conjunction with the osmotic valve mechanism 300 may distribute different samples into different sub-chambers at different times, or from different locations of the GI tract.

It will be understood by one skilled in the art that variations of the osmotic valve mechanism 300 may be combined with any of the other ingestible devices described in this disclosure. For example, in some embodiments of the ingestible device 200 shown and described in relation to FIG. 2, one or both of the valves 214 and 216 may be replaced with certain embodiments of the osmotic valve mechanism 300. One or both of the valves 214 and 216 may include a sealing device that can be destroyed or deformed (e.g., by the mechanical actuator 218 or through a heating element), and one or both of the valves 214 and 216 may be automatically resealed by the expansion of absorptive material located within the sampling chamber 212.

FIGs. 4 and 5 illustrate in detail how some embodiments of the osmotic valve mechanism 300 (FIG. 3) may be operated in order to obtain a sample.

FIG. 4 shows a detailed view of an inlet port 400, which may be incorporated into osmotic valve mechanism 300, prior to being unsealed. The inlet port 400 features an exterior portion 402, which is separated by a middle portion 404 from an interior portion 406. The middle portion 404 of the inlet port 400 contains a sealing device 408, which may be the same as sealing device 306 shown and described in relation to FIG. 3. A heating element 410 is located near the middle portion 404, and adjacent to the sealing device 408. The sides of the inlet port 412A and 412B form the shape of the inlet port 400, and may be constructed from an insulating material, such as insulating ceramic, or polymers such as polyamide-imide, polyphenylene sulfide, polyphenylene oxide, and the like. For illustrative purposes, the exterior portion 402 of the inlet port 400 is depicted as being filled with a sample 414, which may be a fluid sample obtained from the GI tract. However, in some embodiments, the inlet port 400 may be operated regardless of whether a sample 414 is actually contained in the exterior portion 402. The exterior portion 402 and the interior portion 406 are wider than the middle portion 404. A sloped wall 416 gradually reduces the width of the exterior portion 402, to transition from the wider width of the exterior portion 402 to the narrower width of the middle portion 404. This configuration may reduce the overall volume of the sealing device 408 (compared to a configuration with a wider middle portion 404), and reduce the surface area of the sealing device 408 exposed to the sample 414, which may reduce the amount of heat lost from the sealing device 408 to the sample 414. In turn, this may make it easier to raise the temperature of the sealing device 408 using the heating element 410. In some embodiments, the geometry of the inlet port 400 may allow an air pocket (not shown) to form in the exterior portion 402, separating the sealing device 408 from fluid contained within the GI tract. This may act as an insulating barrier around the sealing device 408, and also make it easier to raise the temperature of the sealing device 408 using the heating element 410. Moreover, the larger width of the interior portion 406 relative to the middle portion 404 forms a remnant capture area 418, which may hold the remnants of the sealing device 408 after the inlet port 400 is unsealed.

In some embodiments, the exterior portion 402 of the inlet port 400 may be connected directly or indirectly to an opening in the housing of an ingestible device. In some embodiments, there is nothing to restrict a sample from entering the opening, and, at any given time, the exterior portion 402 of the inlet port 400 may be filled with a fluid sample 414 gathered from whatever portion of the GI tract the ingestible device is located within.

Sealing device 408 prevents the fluid sample 414 contained within the exterior portion 402 of the inlet port 400 from entering the interior portion 406 of the inlet port 400. For simplicity, FIGs. 4 and 5 depict the sealing device 408 as a plug, which forms a seal that may be broken by using a heating element 410. However, in some embodiments the sealing device 408 may be any other type of breakable seal or valve used within the middle portion 404 to separate the exterior portion 402 of the inlet port 400 and the interior portion 406 of the inlet port 400.

In some embodiments, the heating element 410 may be operated by a microcontroller. For example, the microcontroller may be configured to operate the heating element 410 and unseal the inlet port 400 when the ingestible device is in a certain portion of the GI tract. The sides of the inlet port 412A and 412B may be formed from an insulating material, which may shield the ingestible device and the fluid sample 414 from the heat generated by the heating element 410. This may also help to focus the heat produced by heating element 410 in the direction of the sealing device 408, and may reduce the total amount of power to drive the heating element 410 to melt, deform, or destroy the sealing device 408.

In some embodiments, the dimensions of the inlet port 400 are chosen such that a fluid sample 414 is naturally drawn into the exterior portion 402, and ultimately through the middle portion 404 into the interior portion 406, through capillary action. Typically, the cross-section of the exterior portion 402, the middle portion 404, and the interior portion 406 will be square, circular, or rectangular, but any type of cross-section may be used. The overall cross-sectional area of the exterior portion 402, the middle portion 404, and the interior portion 406 of the inlet port 400 is typically less than 50 square millimeters given the size constraints of the ingestible device, with .2 to 2 square millimeters being common. However, the cross-sectional areas listed above are only examples, and any cross-sectional area may be chosen in order to better draw in samples from the different portions of the GI tract. One skilled in the art will understand that the exact shape and dimensions will depend on the physical properties of the sample to be acquired, and some embodiments may use cross-sections other than the ones mentioned above.

FIG. 5, shows a detailed view of an inlet port 500, which may be incorporated into osmotic valve mechanism 300, after it has been unsealed.

After the heating element 510 has heated the sealing device 508 sufficiently, the sealing device 508 may deform, melt, or otherwise be destroyed, effectively unsealing the inlet port 500. Once the inlet port 500 is unsealed, the fluid sample 514 is able to flow naturally from the exterior portion 502 of the inlet port 500 to the interior portion 506 of the inlet port 500 through the middle portion 504. Similar to the embodiments described in relation to FIG. 4, the sides 512A and 512B of the inlet port may be made of an appropriate insulating material, and form the shape of the inlet port 500, the exterior portion 502 with the sloped wall 516, the middle portion 504, and the interior portion 506 along with the remnant capture area 518. As the fluid sample 514 enters the interior portion 506 of the inlet port 500, the natural flow of the fluid sample 514 may carry any of the remnants of the sealing device 508 into the remnant capture area 518 located within the interior portion 506. In some embodiments, once the melted or deformed remnants of the sealing device 508 cease to be in contact with the heating element 510 and instead come into contact with the insulating material that make up the walls of the remnant capture area 518, the remnants of the sealing device 508 re-solidifies or re-forms along the walls of the remnant capture area 518. As a result, the remnant capture area 518 may provide a location for the re-solidified remnants of the sealing device 508 to be stored, and may prevent the remnants of the sealing device 508 from impeding the flow of the sample 514.

In some embodiments, electromagnetic forces are used to attract the remnants of the sealing device 508 to the remnant capture area 518. For example, the sealing device (e.g., the sealing device 408) may be made from a magnetic material, and an induced or permanent magnetic field may be used to attract the remnants of the sealing device 508 to the remnant capture area 518. This magnetic field may be applied after the heating element 510 is activated, and until the remnants of the sealing device 508 re-solidify or re-form within the remnant capture area 518.

It will be understood that the embodiments described by FIGs. 3, 4, and 5, are merely illustrative, and they may be modified and combined with other techniques for drawing in or pumping fluid samples. For example, to encourage samples to be drawn into the sampling chamber 304, the sampling chamber 304 may contain a low-pressure vacuum, and samples may be forcibly drawn into the sampling chamber 304 when the inlet port 302 is unsealed. A similar effect may also be produced by connecting the sampling chamber 304 to a sub-chamber containing a low-pressure vacuum, or by using by using a mechanical actuator to either pump the fluid samples or to increase the volume of the sampling chamber 304. In some embodiments, the geometry and relative size of the exterior portions 402 and 502, the middle portions 404 and 504, and interior portions 406 and 506, may be different from those depicted in FIGs. 4 and 5. For example, the different portions 402, 404, 406, 502, 504, and 506 may have a uniform width, and the sloped walls 416 and 516 and/or the remnant capture areas 418 and 518 are not included. As another example, a sloped wall may be used to form the remnant capture areas 418 and 518.

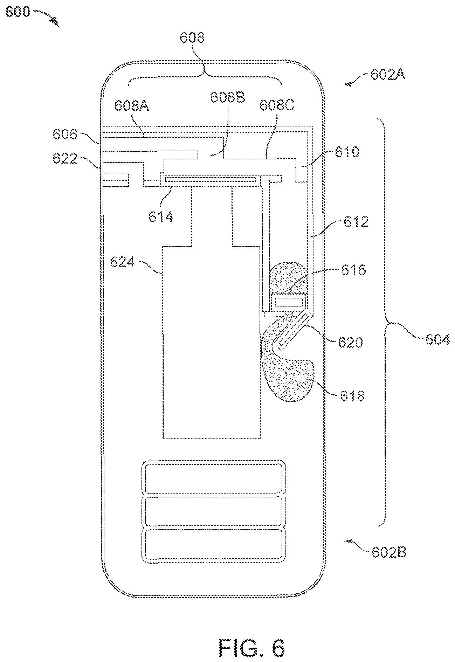

FIG. 6 illustrates another example of an ingestible device 600 with a sampling chamber that includes an exit port. Similar to the ingestible devices 100 and 200, the ingestible device 600 is designed to have an outer housing with a first end 602A, a second end 602B, and a wall 604 extending longitudinally from the first end 602A to the second end 602B. The ingestible device 600 has an opening 606 in the housing, which allows samples to enter the ingestible device 600 from the surrounding environment. The ingestible device 600 has an inlet region 608 connected to the opening 606. The inlet region 608 is connected to an entry port 610 of a sampling chamber 612. The inlet region 608 is divided into three portions. A first portion 608A of the inlet region 608 is connected to the opening 606 and a second portion 608B, and a third portion 608C is connected to the entry port 610 of the sampling chamber 612. The second portion 608B connects the first portion 608A to the third portion 608C, and may contain a moveable valve 614 that is used to prevent samples from flowing through the inlet region 608, and isolate the first portion 608A of the inlet region 608 from the third portion 608C of the inlet region 608.

The ingestible device 600 has a mechanical actuator 624 coupled to the moveable valve 614. In some embodiments, a microprocessor or microcontroller is configured to control the mechanical actuator 624 and move the moveable valve 614 between an open and a closed position. For example, the microcontroller may be configured to move the moveable valve 614 into an open position after the ingestible device reaches a particular location within the GI tract. In some embodiments, the mechanical actuator may be driven by a set of batteries or other power source located within the ingestible device 600. When the moveable valve 614 is moved into an open position, a sample may be allowed to flow through the inlet region 608, and enter the sampling chamber 612 through the entry port 610. When the moveable valve 614 is in a closed position, the sample is prevented from flowing through the inlet region 608 and reaching the sampling chamber 612 from the opening 606.

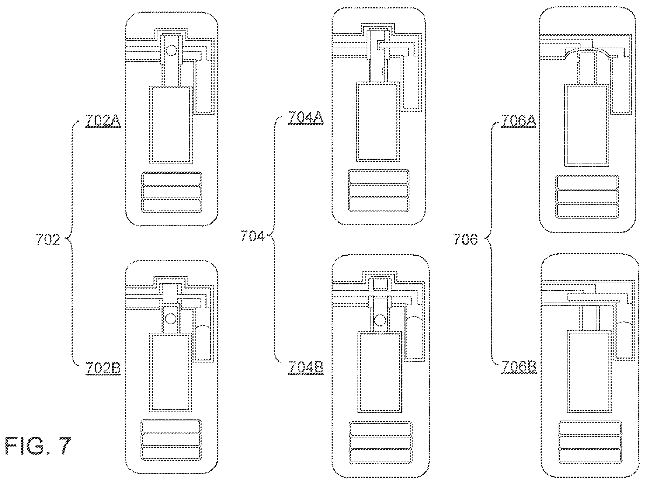

For illustrative purposes, FIG. 6 depicts the moveable valve 614 as a diaphragm valve, which uses a mechanical actuator 624 to move a flexible diaphragm in order to seal or unseal an aperture in the second portion 608B of the inlet region 608, which may effectively block or unblock the inlet region 608. However, it will be understood that, in some embodiments, the moveable valve 614 may be a different type of valve. For example, in some embodiments the moveable valve 614 may be replaced by a pumping mechanism, such as the pumping mechanism described in relation to FIG. 9. As another example, in some embodiments the moveable valve 614 is replaced with an osmotic valve, similar to the embodiments described in relation to FIGs. 3, 4, and 5. Several examples of other different valve types are described in relation to FIG. 7.

The sampling chamber 612 of the ingestible device 600 has an exit port 616 located on the opposite end of the sampling chamber 612 from the entry port 610. In general, the exit port 616 may be located anywhere within the sampling chamber 612. The exit port 616 is configured to allow air or gas 618 to exit the sampling chamber 612, while preventing at least a portion of the sample obtained by the ingestible device 600 from exiting the sampling chamber 612. For example, the exit port 616 may include a gas-permeable membrane, which allows the gas 618 to exit the sampling chamber 612, but which would prevent a liquid or solid sample from leaving the sampling chamber 612 through the exit port 616. Allowing the gas 618 to exit the sampling chamber 612 may prevent pressure from building up within the sampling chamber 612 as the sample enters through the entry port 610. This may result in the sample being drawn into the sampling chamber 612 more easily, and result in increasing the overall volume of the sample able to be collected by the ingestible device 600, and increasing the ease with which the sample is brought into the sampling chamber 612.

The ingestible device 600 includes a one-way valve 620 as part of the exit port 616. This valve may prevent the gas 618 from re-entering the sampling chamber 612. However, in some embodiments the one-way valve 620 may be excluded from the ingestible device 600. In some embodiments, the exit port 616 includes a gas permeable membrane. This gas permeable membrane may lose its permeability when it is placed in contact with the sample. For example, the gas permeable membrane may include a spongy material that allows the gas 618 to exit the sampling chamber 612 through the exit port 616. Once the spongy material becomes moist through contact with the sample, it may become no longer gas permeable, or the permeability may be greatly reduced, thereby preventing the gas 618 from reentering the sampling chamber 612. In some embodiments, the gas permeable membrane may include expanded polytetrafluoroethylene, polypropylene, or the like. In some embodiments, the material used to make the gas permeable membrane may be filter-like, as opposed to sponge-like materials. Generally, the gas permeable membrane may be made of any material that allow gas to permeate, but which prevents liquid from flowing through the membrane due to sufficient resistance or surface tension effects.

In the ingestible device 600, the exit port 616 is connected to a volume within the housing of ingestible device 600 outside of the sampling chamber. Depending on the manufacturing process used to produce the ingestible device 600, the volume within the housing of the ingestible device 600 may contain air or some other type of gas.

The ingestible device 600 includes an outlet port 622, which is connected to the volume within housing of the ingestible device 600. The outlet port 622 may provide a path for the gas 618 to exit the ingestible device 600 and be released into the environment surrounding the ingestible device 600. This may be advantageous when the volume of gas 618 is relatively large, since it may prevent pressure from building up within the housing of the ingestible device 600. In some embodiments, the ingestible device 600 does not include an outlet port 622, and the gas 618 stays inside the volume of the ingestible device 600. In some embodiments, the outlet port 622 is directly or indirectly connected to the exit port 616, for example, by a tube or channel. In some embodiments, the exit port 616 leads directly from the sampling chamber 612 to an opening in the ingestible device 600, and the exit port 616 may effectively replace the outlet port 622. In some embodiments, the outlet port 622 may contain a gas permeable membrane, a one-way valve, a hydrophobic channel, or some other mechanism to avoid unwanted material, (e.g., fluids and solid particulates from within the GI tract), from entering the ingestible device 600 through the outlet port 622.

In some embodiments, the ingestible device 600 may include a sensor within or proximate to the sampling chamber 612. For example, this sensor may be used to detect various properties of a sample contained within the sampling chamber 612, or this sensor may be used to detect the results of an assay technique applied to the sample contained within the sampling chamber 612.

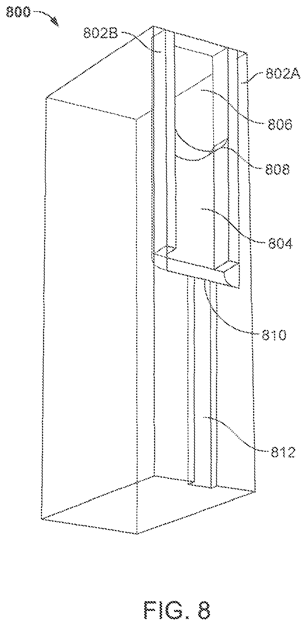

In some embodiments, a hydrophilic sponge is located within the sampling chamber 612, and the hydrophilic sponge may be configured to absorb the sample as the sample enters the sampling chamber 612. In some embodiments, the hydrophilic sponge fills a substantial portion of the sampling chamber 612, and holds the sample for an extended period of time. This may be particularly advantageous if the sample is collected from the ingestible device 600 after the ingestible device 600 exits the body. In some embodiments, the hydrophilic sponge is placed on only certain surfaces or fills only certain portions of the sampling chamber 612. For example, it may be possible to line certain walls (or all walls) of the sampling chamber 612 with a hydrophilic sponge to assist in drawing in the sample, while leaving some (or none) of the walls of the sampling chamber 612 uncovered. Leaving walls uncovered may allow the use of diagnostics or assay techniques that involve a relatively un-obscured optical path. An example of such an embodiment is described in detail in relation to FIG. 8. In some embodiments, the sponge material may be placed on all walls of the sampling chamber 612. This may prevent unwanted ambient light from entering the sampling chamber 612, which may be useful for certain types of low light detection assays. In some embodiments, an opaque material is used to cover some or all sides of the sampling chamber 612. This may also prevent unwanted ambient light from entering the sampling chamber 612.

In some embodiments, the ingestible device 600 may include a sealed vacuum chamber connected to the exit port 616, or connected directly or indirectly to the sampling chamber 612. The sealed vacuum chamber may have an internal pressure that is substantially lower than ambient pressure of the sampling chamber 612 and/or the inlet region 608. In these embodiments, the ingestible device 600 unseals the vacuum chamber in order to reduce the pressure within the sampling chamber. This change in pressure may force the sample to be sucked into the sampling chamber, or allow the sample to be drawn into the sampling chamber quickly.

For simplicity, FIG. 6 depicts only a single sampling chamber 612, but it will be understood that the inlet region 608 may be connected to multiple sampling chambers arranged throughout the device, each of which may be controlled independently through the use of one or more valves. For example, in some embodiments there may be one or more sub-chambers connected to the inlet region 608. Each of the sub-chambers may be configured to hold samples gathered from within the GI tract, and keep those samples isolated. In general, any type of valve or other suitable mechanism may be used to isolate samples contained in the sub-chambers, including any of the valves or mechanisms described in relation to FIGs. 1-5. In some embodiments, the ingestible device 600 distributes different samples into each of the different sub-chambers at different times, or from different locations within the GI tract. For example, the ingestible device 600 may accomplish this by opening up a valve to connect the interior of inlet region 608 to the appropriate sub-chamber before opening up the inlet region 608 to draw in the sample from the opening 606 in the housing.

FIG. 7 depicts different types of moveable valves that may be incorporated into an ingestible device, such as the ingestible devices 100, 200 or 600. The ingestible device 702 illustrates how a pin valve may be used as a moveable valve (e.g., as moveable valve 614 of ingestible device 600 (FIG. 6)), with diagram 702A showing the pin valve in a closed position, and diagram 702B showing the pin valve in an open position. In the ingestible device 702, a mechanical actuator may be configured to move the pin valve linearly in order to switch between an open position and a closed position. For example, in diagram 702A, the ingestible device 702 has a pin inserted into the inlet port, thereby preventing the sample from flowing into the sampling chamber from the opening in the ingestible device 702. In diagram 702B, the ingestible device 702 has a pin that has been removed from the inlet port, allowing the sample to flow freely into the sampling chamber from the opening in the ingestible device 702. In order to generate linear motion, the mechanical actuator may be a linear actuator, such as a solenoid. Alternately, the mechanical actuator may be a rotatory actuator, and the rotation may be converted into a linear motion. One skilled in the art will understand that this may be done any number of ways, for example, by coupling the mechanical actuator to a ball screw mechanism, a threaded lead nut and lead screw mechanism, a rack and pinion mechanism, or the like.

Ingestible device 704 illustrates how a rotary valve may be used as a moveable valve (e.g., as moveable valve 614 of ingestible device 600 (FIG. 6)), with diagram 704A showing the rotary valve in a closed position, and diagram 704B showing the rotary valve in an open position. In diagram 704A, the ingestible device 704 has a rotary pin oriented such that the sample is prevented from entering the sampling chamber from the opening in the ingestible device 704. In diagram 704B, the ingestible device 704 has a rotary pin that has been rotated into an orientation where the sample is free to flow into the sampling chamber from the opening in the ingestible device 704. In order to operate the rotary valve, the mechanical actuator in ingestible device 704 may be a rotatory actuator, which is capable of rotating the rotary pin to switch between the open position and the closed position.

Ingestible device 706 illustrates how a flexible diaphragm, or diaphragm valve, may be used as a moveable valve (e.g., as moveable valve 614 of ingestible device 600 (FIG. 6)), with diagram 706A showing the diaphragm valve in a closed position, and diagram 706B showing the diaphragm valve in an open position. In diagram 706A, the ingestible device 706 has a diaphragm valve in a closed position, with the flexible diaphragm being pressed against an aperture in the inlet region due to the pressure generated by the mechanical actuator against the flexible diaphragm. This may effectively block a sample from flowing through the inlet region, and thereby preventing a sample from entering the sampling chamber from the opening in the ingestible device 706. **In** diagram 706B, the ingestible device 706 has a diaphragm valve in an open position, with the pressure removed from the flexible diaphragm. The diaphragm returns to a position away from the aperture in the inlet region, allowing a sample to flow freely into the sampling chamber from the opening the in ingestible device 706.

**In** some embodiments, ingestible device 706 has a spring mechanism near the diaphragm or in direct contact with the diaphragm. The spring mechanism may apply pressure to the diaphragm to oppose the pressure applied by the mechanical actuator, which may cause the flexible diaphragm to be moved into an open position when the mechanical actuator is not applying pressure to the flexible diaphragm. Additionally, this may ensure that the diaphragm valve remains open when the mechanical actuator is not applying pressure across the flexible diaphragm.

**In** some embodiments, moving the mechanical actuator from a closed position to an open position causes a volume of the inlet region within the ingestible device to increase. This may cause the pressure within the inlet region to be reduced, generating suction to draw a sample into the inlet region. Similarly, moving the mechanical actuator from an open position to a closed position may cause the volume of the inlet region to be reduced. This may cause the pressure within the inlet region to be increased, pushing the sample out of the inlet region. Depending on the design of the inlet region, the mechanical actuator, and the moveable valve, this may push the sample into the sampling chamber rather than pushing the sample back through the opening in the ingestible device. An example of such a design is described in greater detail in relation to FIG. 9.

FIG. 8 illustrates an example of a sampling mechanism that may be incorporated into an ingestible device, such as the ingestible devices 100, 200, 600, and 702-706. The sampling mechanism 800 is partially lined with hydrophilic sponges 802A and 802B. In between the hydrophilic sponges 802A and 802B is a testing region 804 within the sampling mechanism 800. The hydrophilic sponges 802A and 802B attract a liquid or fluid sample 806, and may draw the sample 806 into the sampling mechanism 800. As the hydrophilic sponges 802A and 802B are saturated with the sample 806, a meniscus 808 is formed at the end of the sample 806, between the hydrophilic sponges 802A and 802B. This system may be useful for acquiring particularly viscous samples, which may have difficulty flowing into the sampling mechanism 800 naturally.

The sampling mechanism 800 includes an exit port 810 connected to a channel 812. As the sample 806 is drawn into the sampling mechanism 800, air or gas contained in the sampling mechanism 800 may be pushed out of the sampling mechanism 800 through the exit port 810 and into the channel 812. This may avoid gas being trapped within the sampling mechanism 800, which in turn may avoid pressure building inside of the sampling mechanism 800 and preventing the sample 806 from being drawn into the testing region 804.

In some embodiments, the sampling mechanism 800 may not include an exit port 810 or a channel 812, and any air or gas in the sampling mechanism 800 may be allowed to remain within the sampling mechanism 800. In some embodiments, the sampling mechanism 800 may be filled with a low pressure vacuum, attached to a pump or other mechanism to create a vacuum, or attached to a sealed chamber containing a low pressure vacuum that may be unsealed. The use of a vacuum may allow the sampling mechanism 800 to forcibly draw in a sample.

In some embodiments, an ingestible device may include sensors or diagnostics to study the sample 806 contained within the sampling mechanism 800. Because there is no sponge material on the front and back walls of the testing region 804, information about the sample 806 contained within the testing region 804 may be gathered by using sensors and/or assay techniques that involve a clear optical path, which would otherwise be obscured by a sponge (e.g., the hydrophilic sponges 802A and 802B). For example, light sources and/or optical sensors may be placed near the front and/or back walls in order to test optical properties of the sample, or to detect the results of certain assay techniques.

It will be understood by those skilled in the art that the sampling mechanism 800 depicted in FIG. 8 is merely illustrative, and the general techniques described in relation to FIG. 8 may be applied to a wide range of different chambers, channels, and fluid pathways, and incorporated into a wide range of different ingestible devices. Furthermore, in some embodiments, the overall geometry of FIG. 8 and the positioning of the sponges and the testing area may be altered. For example, the sponge may be formed in the shape of hollow tubes, with testing areas located in the middle of each tube. In this case, there would be a clear optical path from one end of the tube to the other.

FIG. 9 illustrates a pumping mechanism 900 that may be incorporated into an ingestible device, including certain embodiments of ingestible devices 100, 200, 600, and 702-706. For illustrative purposes, the pumping mechanism 900 may be described in the context of an ingestible device similar to ingestible device 600 (FIG. 6). When it is incorporated into an ingestible device similar to ingestible device 600, the pumping mechanism 900 may function as a moveable valve (e.g., moveable valve 614 of ingestible device 600), and control the ability of samples to flow between the opening 606 in the housing and the entry port 610 of the sampling chamber 612. Additionally, the pumping chamber 904 of the pumping mechanism 900 may form part of the second portion 608B of the inlet region 608. However, the general structure and principles of pumping mechanism 900 are not limited to the ingestible devices described in this disclosure, and they may be applied to a wide range of ingestible devices.

Pumping mechanism 900 is designed to draw in a sample through a first opening 902 into a pumping chamber 904, and push a portion of the sample out of the pumping chamber 904 through a second opening 906. In some embodiments, the first opening 902 may be connected directly or indirectly to an opening in the housing of an ingestible device. For example, an inlet region (e.g., the first portion 608A of the inlet region 608 of the ingestible device 600 (FIG. 6)) may connect an opening in the housing of an ingestible device (e.g., the opening 606 in the housing of ingestible device 600 (FIG. 6)) to the first opening 902. **In** some embodiments, the second opening 906 is connected directly or indirectly to a sampling chamber of an ingestible device. For example, the second opening 906 may be connected to an entry port of a sampling chamber (e.g., connected via the third portion 608C of the inlet region 608 to the entry port 610 of the sampling chamber 612 of the ingestible device 600 (FIG. 6)).

The pumping mechanism 900 features a moveable pump head 908 contained within the pumping chamber 904. The protrusion 908A of the moveable pump head 908 is shaped to fit within the first opening 902, or otherwise block the first opening 902. The base 908B of the moveable pump head 908 is able to cover the second opening 906 or otherwise block the second opening 906. Moreover, the protrusion 908A and the base 908B of the moveable pump head 908 are sized and oriented from each other in such a manner such that when the protrusion 908A blocks the first opening 902, the base 908B may simultaneously block the second opening 906 or leave the second opening 906 unblocked. Furthermore, when the base 908B blocks the second opening 906, the protrusion 908A may always be configured to also block the first opening 902.

As the moveable pump head 908 is moved up and down, the openings 902 and 906 may be sealed or unsealed, switching the pumping mechanism 900 across an open position, a partially closed position, and a closed position. In the open position (as is shown in the diagram 912), both the first opening 902 and the second opening 906 are unsealed or open. **In** the partially closed position (as is shown in the diagram 914, the moveable pump head 908 is positioned to only seal the first opening 902, while leaving the second opening 906 open. Finally, in the closed position (as is shown in the diagrams 910 and 918), both the first opening 902 and the second opening 906 are sealed.

In some embodiments, the moveable pump head 908 may be connected to a mechanical actuator (e.g., the mechanical actuator 624 of the ingestible device 600 (FIG. 6)), which may be configured to move the moveable pump head 908 linearly up and down. For example, the moveable pump head 908 may be located on the end of a shaft that is attached to the mechanical actuator. In some embodiments, the mechanical actuator and the positioning of the moveable pump head 908 may be controlled by a microcontroller or microprocessor located within the ingestible device. For example, a microcontroller may be configured to move the pump head 908 and begin pumping a sample through the pumping chamber 904 only after the ingestible device has reached a particular location within the GI tract.

Diagram 910 depicts the pumping mechanism 900 in a fully closed position. When the pumping mechanism 900 is in the fully closed position, the protrusion 908A of the moveable pump head 908 may be positioned within the first opening 902, and the base 908B of the moveable pump head 908 may be positioned adjacent to the second opening 906. **In** the fully closed position, the positioning of the moveable pump head 908 may effectively prevent a sample from entering or exiting the pumping chamber 904 from the openings 902 or 906.

Diagram 912 depicts the pumping mechanism 900 in an open position. When the pumping mechanism 900 is in the open position, the moveable pump head 908 is moved away from the first opening 902, moving the protrusion 908A of the moveable pump head 908 out of the first opening 902, and moving the base 908B of the moveable pump away from the second opening 906. In this position, the pumping mechanism 900 may allow one or more samples to enter the pumping chamber 904 through the first opening 902, and exit the pumping chamber 904 through the second opening 906. Because the effective volume of the pumping chamber 904 increases when the moveable pump head 908 is moved away from the first opening 902, the pumping mechanism 900 may draw a sample into the sampling chamber through the first opening 902 when transitioning from a closed position depicted in the diagram 910 to an open position depicted in the diagram 912. In some embodiments, a one-way valve may be incorporated into an ingestible device to prevent samples from being drawn into the pumping chamber 904 through the second opening 906 when the pumping mechanism 900 transitions between the closed position and the open position. This may ensure that the only sample entering the pumping chamber 904 is drawn in through the first opening 902.

Diagram 914 depicts the pumping mechanism 900 in a partially closed position. When the pumping mechanism 900 is in the partially closed position, the protrusion 908A of the moveable pump head 908 is positioned adjacent to the first opening 902, or just inside the first opening 902. In this position, the protrusion 908A of the moveable pump head 908 effectively seals off the first opening 902, preventing any of the sample remaining in the pumping chamber 904 from exiting pumping chamber 904 via the first opening 902. In this position, the base 908B of the moveable pump head 908 is positioned away from the second opening 906. This may allow any sample remaining in the pumping chamber 904 to exit the pumping chamber 904 through the second opening 906. For example, if the second opening 906 is connected to an entry port of a sampling chamber (e.g., connected via the third portion 608C of the inlet region 608 to the entry port 610 of the sampling chamber 612 of the ingestible device 600 (FIG. 6)), this may allow the sample to flow freely from the pumping mechanism 900 into the sampling chamber via the entry port.

Diagram 916 depicts the pumping mechanism 900 as it transitions between the partially closed position to the fully closed position. As the pumping mechanism 900 moves into the fully closed position, the moveable pump head 908 forces any of remaining sample contained within the pumping chamber 904 out of the pumping chamber 904 through the second opening 906. As this happens, the protrusion 908A of the moveable pump head 908 remains within the first opening 902, blocking it off and preventing the sample from exiting the pumping chamber 904 through first opening 902. By comparison, the base 908B of the moveable pump head 908 does not fully cover the second opening 906, and the sample is free to exit the pumping chamber 904 through the second opening 906. In combination, this may result in a majority of the sample remaining in the sampling chamber being forced through the second opening 906 as the pumping mechanism 900 moves from the partially closed position depicted in diagram 914 to the fully closed position depicted in diagram 918.

Diagram 918 depicts the pumping mechanism 900 in the fully closed position, similar to diagram 910. As noted before, in the fully closed position the moveable pump head 908 is positioned to seal off the openings 902 and 906, which may prevent a sample from entering or exiting the pumping chamber 904 from the openings 902 or 904. In general, the pumping mechanism 900 may cycle between the closed position depicted in diagrams 910 and 918 and the open position depicted in diagram 912 any number of times in order to draw additional samples into the pumping chamber 904 through the first opening 902, and force the samples out of the pumping chamber 904 through the second opening 906.

Although FIG. 9 depicts the protrusion 908A of the moveable pump head 908 located in the center of the moveable pump head 908, the location of the protrusion 908A may be anywhere on the moveable pump head 908. For example, the protrusion 908A of the moveable pump head 908 and the first opening 902 may be positioned on the side of the pumping chamber 904. In some embodiments, the moveable pump head 908 is split into two pieces, which may be controlled by one or more actuators. For example, the protrusion 908A and the base 908B may be two separate pieces, each of which is moved using a different actuator. This may allow the first opening 902 to be sealed and unsealed independently from the volume of the pumping mechanism 900 being increased or decreased.

For illustrative purposes, the diagrams 910-918 depict the base 908B of the moveable pump head 908 being used to cover or otherwise block the second opening 906. However, in some embodiments, the moveable pump head 908 may not cover, fit within, or otherwise block the second opening 906, and it will be understood by one skilled in the art that the second opening 906 does not need to be partially or fully blocked in order to push a sample through the second opening 906. For example, the moveable pump head 908 may not include a base 908B at all. Instead, the moveable pump head 908 may be made of a flexible material that forms a seal with the underside of the pumping chamber 904. In this case, the moveable pump head 908 may be moved up and down in a manner similar to a plunger in order to change the effective volume of the pumping chamber 904. When the volume decreases, the sample is at least partially forced out of the pumping chamber 904 through the second opening 906.

In general, incorporating the pumping mechanism 900 into an ingestible device may not impair the function of the openings, ports, valves, membranes, sampling chambers, or other structures of the ingestible device, and any of the teachings or embodiments described in conjunction with the ingestible devices 100, 200, 600, or 702-706 may be combined in different embodiments of an ingestible device along with the pumping mechanism 900. For example, the pumping mechanism 900 may replace the first valve 214 in the ingestible device 200 (FIG. 2), and may be used to force the sample into the sampling chamber 212. As an alternate example, the pumping mechanism 900 may be used to force samples into the sampling chamber 304 of the osmotic valve mechanism 300 (FIG. 3). As another example, the pumping mechanism 900 may be incorporated into an embodiment of the ingestible device 600 (FIG. 6) where the exit port 616 is not included, and the pumping mechanism 900 may be used to force the sample into the sampling chamber 612 despite the pressure that may result from air or gas 618 being trapped within the sampling chamber 612.

For illustrative purposes, the examples provided by this disclosure focus primarily on a number of different example embodiments of an ingestible device, such as the ingestible devices 100, 200, 600, and 702-706. However, it is understood that variations in the general shape and design of one or more embodiments of the ingestible devices described in relation to FIGs 1-9 may be made without significantly changing the functions and operations of the device. Furthermore, it should be noted that the features and limitations described in any one embodiment may be applied to any other embodiment herein, and the descriptions and examples relating to one embodiment may be combined with any other embodiment in a suitable manner. For example, any of the valves described in relation to FIG. 7 may be used as the valves 214 and 216 described in relation to FIG. 2. As an alternate example, the absorptive material 310 and flexible membrane 314 described in relation to FIG. 3 may be incorporated into any of the various sampling chambers described in various embodiments of ingestible devices 100, 200, 600, and 702-706 in order to automatically seal the sampling chamber. Moreover, the figures and examples provided in disclosure are intended to be only exemplary, and not limiting. It should also be noted that the systems and/or methods described above may be applied to, or used in accordance with, other systems and/or methods, including systems and/or methods that may or may not be directly related to ingestible devices.

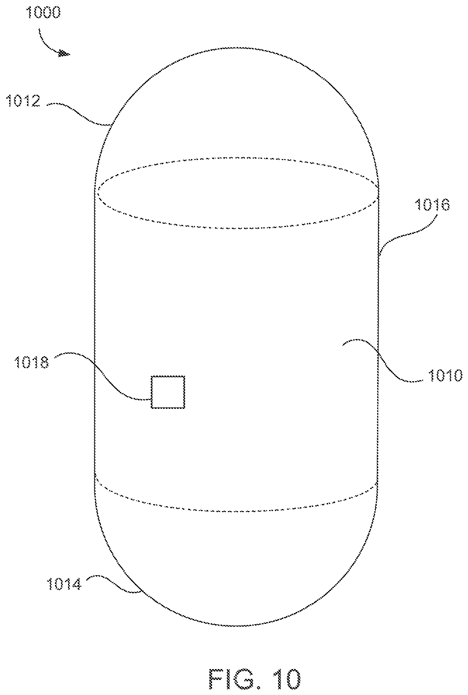

FIG. 10 illustrates, in a highly schematic fashion, an ingestible device 1000 having a housing 1010 that includes a first end 1012 and a second end 1014 opposite first end 1012. Housing 1010 also includes a wall 1016 that connects first end 1012 and second end 1014. Wall 1016 has an opening 1018 that allows fluid from an exterior of the ingestible device 1000 (e.g., from the GI tract) and into an interior of ingestible device 1000.

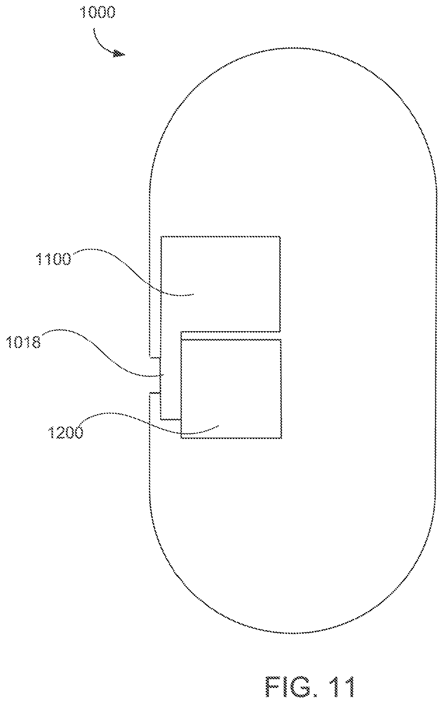

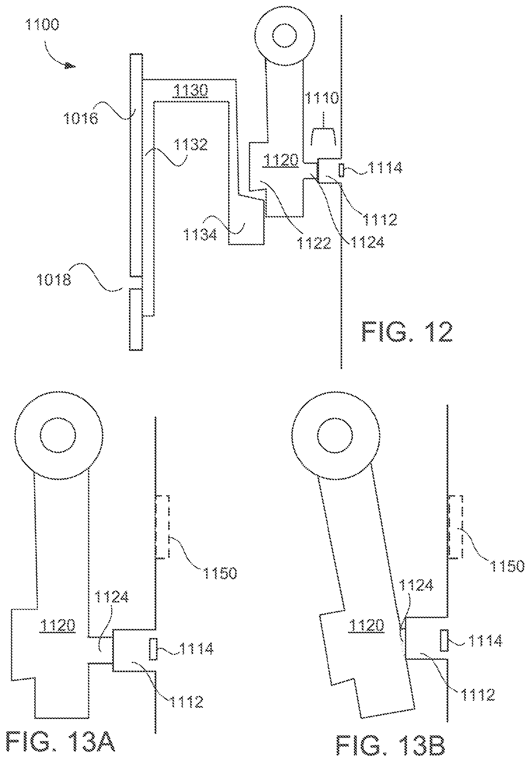

FIG. 11 depicts a cross-sectional view of a portion of the interior of ingestible device 1000. As shown in FIG. 11, the interior of ingestible device 1000 includes a valve system 1100 and a sampling system 1200. Valve system 1100 is depicted as having a portion that is flush with the opening 1018 so that valve system 1100 prevents fluid exterior to ingestible device 1000 from entering sampling system 1200. However, as described in more detail below with reference to FIGs. 12-16, valve system 1100 can change position so that valve system 1100 allows fluid exterior to ingestible device 1000 to enter sampling system 1200.

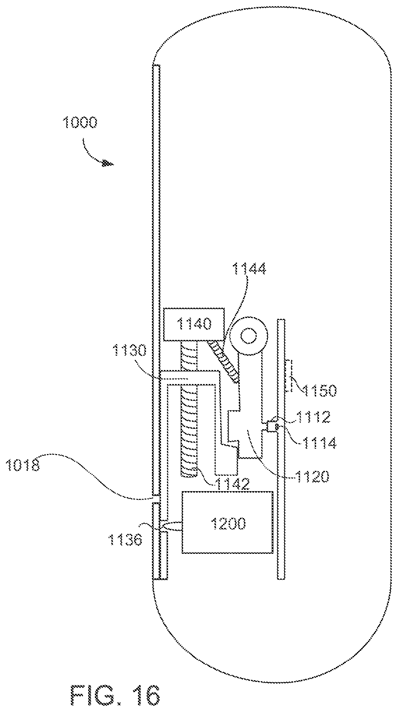

FIGs. 12 and 16 illustrate valve system 1100 in more detail. As shown in FIG. 12, valve system 1100 includes an actuation mechanism 1110, a trigger 1120, and a gate 1130. In FIGs. 12 and 16, a leg 1132 of gate 1130 is flush against, and parallel with, housing wall 1016 so that gate leg 1132 covers opening 1018 to prevent fluid exterior to ingestible device 1000 (e.g., fluid in the GI tract) from entering the interior of ingestible device 1000. A protrusion 1134 of gate 1130 engages a lip 1122 of trigger 1120. A peg 1124 of trigger 1120 engages a wax pot 1112 of actuation mechanism 1110. Referring to FIG. 16, a biasing mechanism 1140 includes a compression spring 1142 that applies an upward force on gate 1130. Biasing mechanism 1140 also includes a torsion spring 1144 that applies a force on trigger 1120 in the counter-clockwise direction. In FIGs. 12 and 16, the force applied by torsion spring 1144 is counter-acted by the solid wax in pot 1112, and the force applied by compression spring 1142 is counter-acted by lip 1122.

FIGs. 13A and FIG 13B show an embodiment of the manner in which actuation mechanism 1110 actuates movement of trigger 1120. Similar to FIGs. 12 and 16, FIG. 13A shows a configuration in which peg 1124 applies a force against solid wax pot 1112 due to torsion spring 1144, and in which the solid nature of wax pot 1112 resists the force applied by peg 1124. A control unit 1150 is in signal communication with valve system 1100. During use of ingestible device 1000, a control unit 1150 receives a signal, indicating that the position of valve system 1100 should change, e.g., so that ingestible device 1000 can take a sample of a fluid in the GI tract. Control unit 1150 sends a signal that causes a heating system 1114 of actuation system 1100 to heat the wax in pot 1112 so that the wax melts. As shown in FIG. 13B, the melted wax is not able to resist the force applied by peg 1124 so that, under the force of torsion spring 1144, trigger 1120 moves in a counter-clockwise fashion.

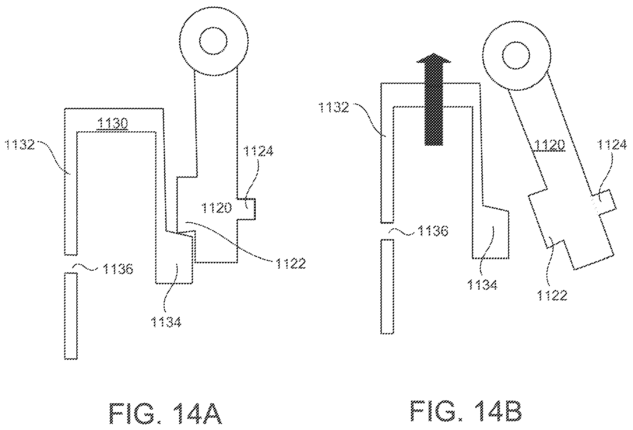

FIGs. 14A and 14B illustrate the interaction of trigger 1120 and gate 1130 before and after actuation. As shown in FIG 14A, when wax pot 1112 is solid (corresponding to the configuration shown in FIG. 13A), protrusion 1134 engages lip 1122, which prevents the force of compression spring 1142 from moving gate 1130 upward. As shown in FIG. 14B, when the wax in pot 1112 melts (FIG. 13B), trigger 1120 moves counter-clockwise, and lip 1122 disengages from protrusion 1134. This allows the force of compression spring 1142 to move gate 1130 upward. As seen by comparing FIG. 14A to FIG. 14B, the upward movement of gate 1130 results in an upward movement of an opening 1136 in gate leg 1132.

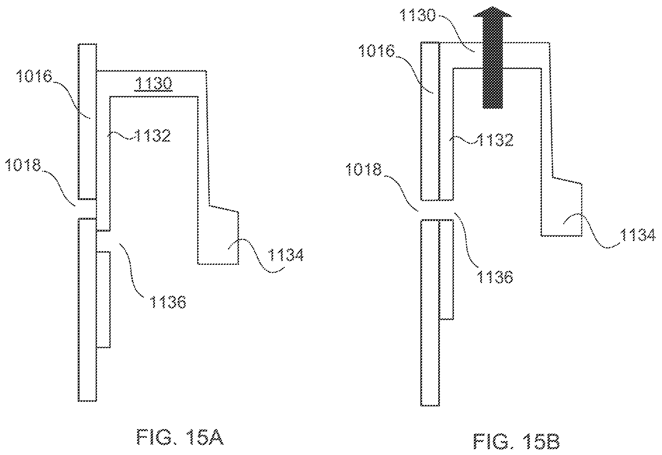

FIGs. 15A and 15B illustrate the impact of the upward movement of opening 1136 on the ability of ingestible device 1000 to obtain a sample. As shown in FIG. 15A, when the wax in pot 1112 is solid (FIGs. 13A and 14A), opening 1136 in is not aligned with opening 1018 in wall 1016 of ingestible device 1000. Instead, gate leg 1132 covers opening 1018 and blocks fluid from entering the interior of ingestible device 1000. As shown in FIG. 15B, when the wax in pot 1112 is melted and trigger 1120 and gate 1130 have moved (FIGs. 13B and 14B), opening 1136 in gate 1130 is aligned with opening 1018 in wall 1016. In this configuration, fluid that is exterior to ingestible device 1000 (e.g., in the GI tract) can enter the interior of ingestible device 1000 via openings 1018 and 1036.

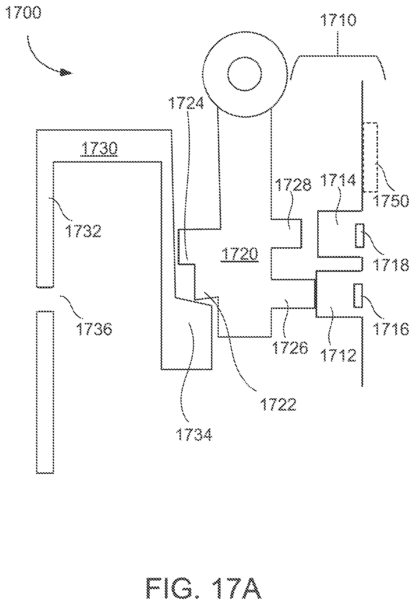

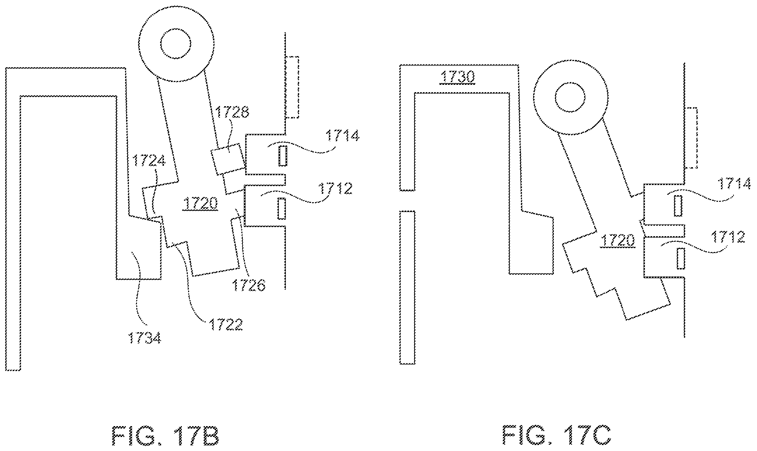

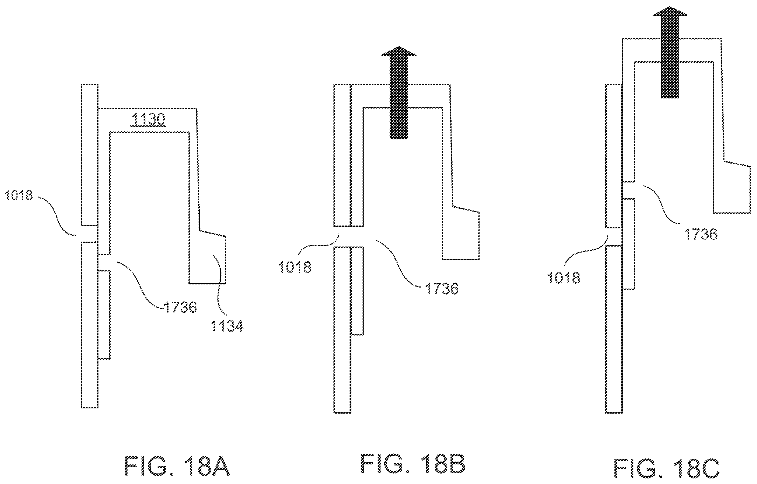

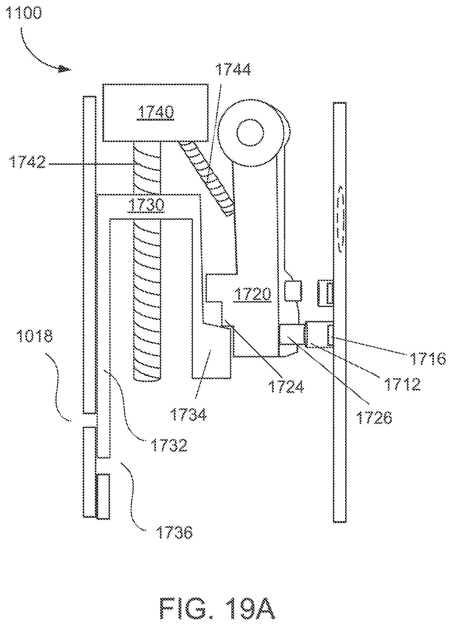

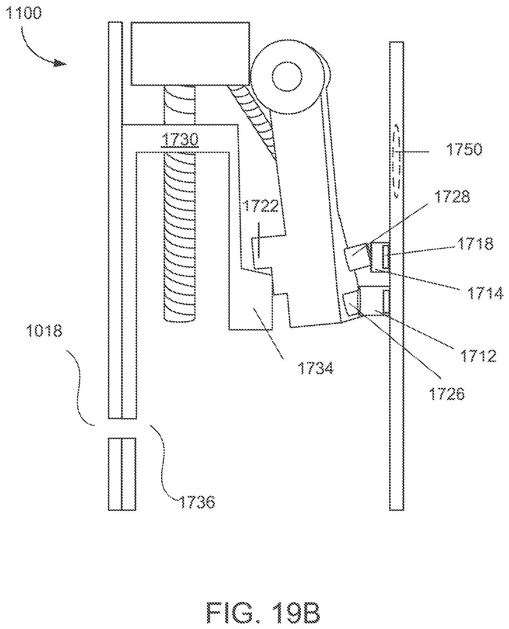

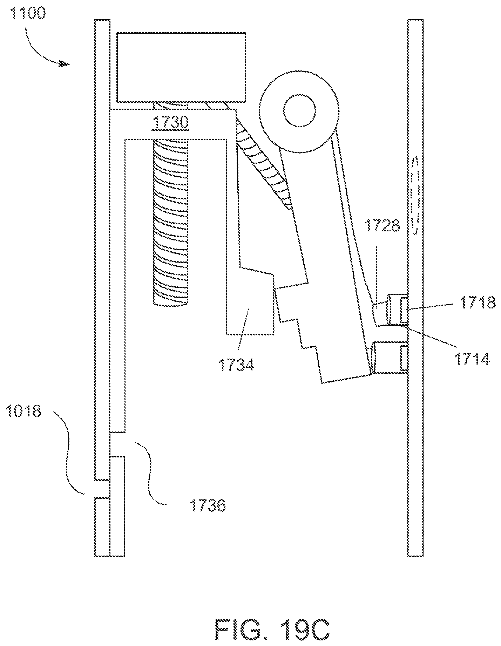

While the foregoing description is made with regard to a valve system having one open position and one closed position (e.g., a two-stage valve system), the disclosure is not limited in this sense. Rather, the concepts described above with regard to a two stage valve system can be implemented with a valve system have more than two stages (e.g., three stages, four stages, five stages, etc.). For example, FIGs. 17A-19C illustrate cross-sectional views of a three-stage valve system 1700. FIGs. 17A, 18A and 19A illustrate different views of components of valve system 1700 in the same position. FIGs. 17B, 18B and 19B illustrate different views of components of valve system 1700 in the same position. FIGs. 17C, 18C and 19C illustrate different views of components of valve system 1700 in the same position.

As shown in FIGs. 17A-19C, valve system 1700 includes an actuation system 1710, a trigger 1720, a gate 1730 and a biasing system 1740. Actuation system 1710 includes a first wax pot 1712, a second wax pot 1714, a first heating system 1716 and a second heating system 1718. Trigger 1720 includes a first lip 1722, a second lip 1724, a first peg 1726 and a second peg 1728. Gate 1730 includes a gate leg 1732 and a protrusion 1734. Gate leg 1732 has an opening 1736. Biasing system 1740 includes a compression spring 1742 and a torsion spring 1744. In addition, the ingestible device includes a control unit 1750.

As shown in FIGs. 17A, 18A and 19A, in the first stage, protrusion 1734 engages first lip 1722, and first peg 1726 engages first wax pot 1712. Compression spring 1742 applies an upward force on gate 1730, and torsion spring 1744 applies a force on trigger 1720 in the counter-clockwise direction. The force applied by torsion spring 1744 is counter-acted by the solid wax in first pot 1712, and the force applied by compression spring 1742 is counter-acted by first lip 1722. Opening 1736 is not aligned with opening 1018.

FIGs. 17B, 18B and 19B illustrate the configuration in a second stage, after control unit 1750 sends a signal to first heating system 1716 to melt the wax in first pot 1712. In the second stage, trigger 1720 has moved counter-clockwise relative to its position in the first stage. First peg 1726 is positioned in first pot 1712 because the melted wax cannot prevent this movement. Further counter-clockwise movement of trigger 1720 is prevented by the engagement of second peg 1728 with the solid wax in second pot 1714. With the counter-clockwise movement of trigger 1720, first lip 1722 disengages from protrusion 1734, and gate 1730 moves upward so that opening 1736 in leg 1732 is aligned with opening 1018. Further upward movement of gate 1730 is prevented by the engagement of protrusion 1734 with second lip 1724.

FIGs. 17C, 18C and 19C illustrate the configuration in a third stage, after control unit 1750 sends a signal to second heating system 1718 to melt the wax in second pot 1714. In the third stage, trigger 1720 has moved counter-clockwise relative to its position in the second stage. Second peg 1728 is positioned in second pot 1714 because the melted wax cannot prevent this movement. Further counter-clockwise rotation is prevented by the engagement of first and second pegs 1726 and 1728, respectively with first and second pots 1712 and 1714, respectively. Protrusion 1734 is disengaged from second lip 1724, allowing the force of compression spring 1742 to move gate 1730 upward so that opening 1736 is no longer aligned with opening 1018.

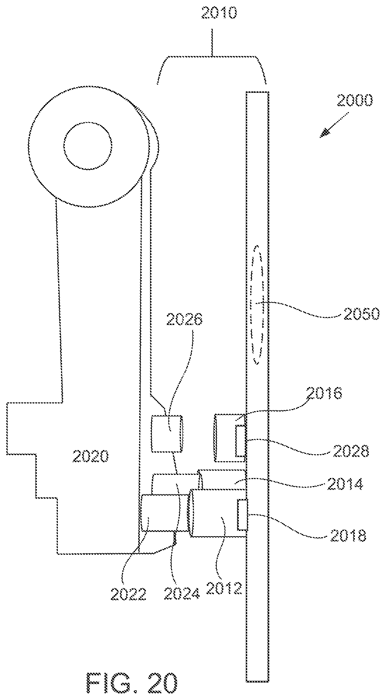

FIG. 20 illustrates another embodiment of a three stage valve system 2000 that can be used in an ingestible device. Valve system 2000 that is similar to valve system 1700 except that actuation system 2010 includes three includes wax pots 2012, 2014 and 2016, respectively, that define a triangle, and trigger 2020 includes three pegs 2022, 2024 and 2026, respectively, that define a corresponding triangle. Actuation system 2010 is controlled using a control unit 2050. Actuation system 2010 also includes a first heating system 2018 that heats the wax in pots 2012 and 2014 and so that pegs 2022 and 2024 enters their corresponding pot, causing valve system 2000 to move from its first stage to its second stage. Actuation system 2010 also includes a second heating system 2028 that heats the wax in pot 2016 so that pegs 2026 enters pot 2016, causing valve system 2000 to move from its second stage to its third stage.

In the foregoing discussion, embodiments actuating systems are described that include one or more wax pots and corresponding heating systems. But the disclosure is not limited to such actuating systems. Generally, any actuating system can be used that will provide an appropriate force to resist counter-clockwise movement of the trigger when desired and to remove that force when desired. Examples of such actuation systems include a pot with a silicon or wax seal. A control unit may be used to rupture the seal and allow counter clock-wise movement of the trigger. Additionally, or alternatively, the actuation mechanism may use dissolvable coating to that dissolves over time or in the presence of a substance. As the coating dissolves, the trigger may move further in the counter clock-wise direction. Other actuation mechanisms may also apply an attractive force rather than remove a resistive force. For example, the actuation mechanism may include magnetic pegs and slidable magnets The magnets may be located behind the pots or may slide to a position behind the pots when the valve system should change stages. As the magnets behind the pots slide into range of the magnetic trigger pegs, the trigger moves in the counterclockwise direction due to the attractive force between the magnetic peg and the magnets. The sliding mechanism to move the slidable magnets may be powered by an osmotic pump, a pressurized chamber, or any other applicable method of movement previously described in other embodiments.

In the discussion above, embodiments of triggers are disclosed that include one or more lips and one or more pegs. However, the disclosure is not limited to such triggers. **In** general, for example, any trigger design can be used that is capable of providing the step-wise movement of the trigger. Such trigger designs include, for example, a releasable latch coupling or a saw toothed engagement wall. A different embodiment may utilize a ball in socket joint to engage the trigger and gate, in which the "socket" is located on the trigger. It is to be noted that such designs need not be based on counter-clockwise movement and may be, for example, designed for the controlled movement of the trigger in one or more of various degrees of freedom. For example, rather than rotate, the trigger may be configured to slide laterally to push a peg of the trigger into a melted wax pot.

The discussion above describes embodiments of gates that include a protrusion and a leg with an opening. The disclosure is not limited to such designs. Generally, any appropriate arrangement can be used so long as it provides the desired step-wise controlled movement of an opening to the interior of the ingestible device. Exemplary designs include a gate that is capable of responding to or applying magnetic forces on the trigger. A saw toothed pattern may also provide a step-wise gate movement. Additionally, embodiments include a latch designed to releasably couple the gate to the trigger. A different embodiment may utilize a ball in socket joint in which the "ball" is located on the gate. Optionally, a gate can include one or regions that include one or more appropriate sealing materials positioned to cover the opening in the housing of the ingestible device when the gate is positioned to prevent fluid exterior to the ingestible device from entering the interior of the device via the opening in the housing of the ingestible device.

In the foregoing discussion, embodiments of biasing systems are described that include a compression spring and a biasing spring. However, the disclosure is not limited in this sense. In general, any biasing elements can be used to provide the counter-clockwise force to the trigger and/or to provide the upward force to the gate. Exemplary biasing elements include elastic bands, wherein a stretched elastic band acts similar to a stretched compression spring as described. Additional basing mechanisms may include magnets and/or magnetic forces to induce trigger or gate movement. For example, a magnet may be located above the gate, where, like the constant force of the stretched compression spring, the magnet also applies a constant attractive force on the gate.

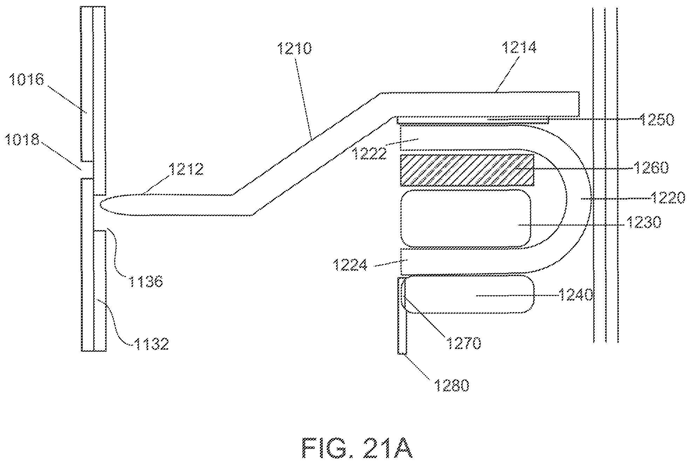

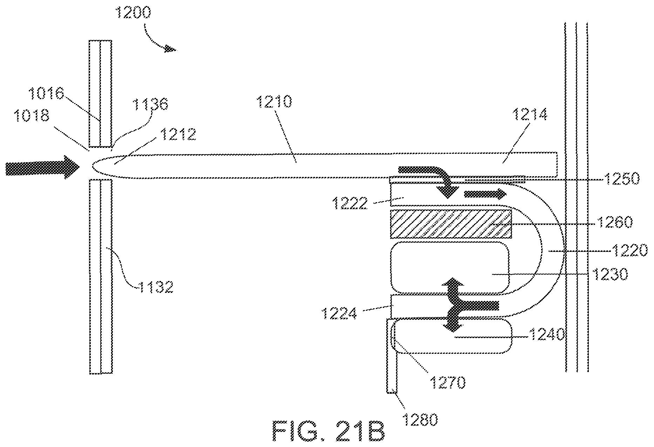

As noted above in addition to a valve system, an ingestible device includes a sampling system. FIGs. 21A and 21B illustrate a partial cross sectional view of ingestible device 1000 with sampling system 1200 and certain components of valve system 1100. Sampling system 1200 includes a series of sponges configured to absorb fluid from an opening, move the fluid to a location within the housing, and prepare the fluid for testing. Preparation for testing may include filtering the fluid and combining the fluid with a chemical assay. The assay may be configured to dye cells in the filtered sample. The series of sponges includes a wicking sponge 1210, a transfer sponge 1220, a volume sponge 1230, and an assay sponge 1240.

Wicking sponge 1210 is made of an absorptive material that absorbs the fluid form the opening in the housing when the valve is open (i.e., when the inlet and the housing are aligned). The wicking sponge transfers the fluid from the opening to a filter. Wicking sponge 1210 includes a wicking tongue 1212 extended towards the housing 1016. As shown in Fig. 21A, before actuation of the actuation system (FIGs. 13A, 14A, 15A), wicking tongue 1212 is not adjacent opening 1018 in wall 1016 of ingestible device 1000 so that wicking tongue 1212 does not absorb fluid exterior to ingestible device 1000. However, as shown in FIG. 21B, after actuation of the actuation system (FIGs. 13B, 14B, 15B), wicking tongue 1212 is adjacent opening 1018 so that wicking sponge 1212 is made of an absorptive material that absorbs fluid that passes through opening 1018, e.g., fluid from the GI tract. Fluid absorbed by wicking tongue 1212 can travel through wicking sponge 1210 to a distal end 1214 of wicking sponge 1210. The wicking sponge 1210 and wicking tongue 1212 may be made of a VF2 sponge, an Ahlstrom M13 sponge, MF/F material, a Carwild Ivalon Polyvinyl Alcohol material, or another suitable absorptive material. Optionally, the dimensions of the sponge material may be selected to enable all its desired functions while remaining precisely packaged within the capsule. In some embodiments, Carwild Ivalon Polyvinyl Alcohol material is cut to the dimensions 1.4 millimeters (height) x 6 millimeters (width) x 8.5 millimeters (length). In certain embodiments, one or more of the following parameters can be considered when selecting an appropriate material and/or its dimension: ability to load one more preservative materials; desired preservative material(s) to be loaded; capacity to hold one or more dried preservatives; ability to facilitate hydration of one or more dried preservative materials upon contact with one or more GI fluids; capacity to capture fluid (e.g., GI fluid); and swelling properties upon fluid uptake (generally, it is desirable to have little or no swelling upon fluid uptake). Typically, the preservative(s) is (are) selected based on the analyte of interest.

Nucleic acid preservatives can be used to prevent or reduce the rate of nucleic acid degradation or denaturation, and/or increase the stability of nucleic acids, e.g., to maintain nucleic acid structure. In some embodiments, the nucleic acid preservative is nuclease inhibitor (deoxyribonuclease inhibitor). In some embodiments, the nucleic acid preservative is a ribonuclease inhibitor. Nuclease inhibitors and ribonuclease inhibitors are known in the art, and have been described in, e.g., U.S. 6,224,379. In some embodiments, the nucleic acid preservative mixture can include EDTA, sodium citrate, an ammonium sulphate. In some embodiments, the RNA preservative mixture includes 2mL of 0.5M EDTA, 1.25ml of 1 M sodium citrate, 35g of ammonium sulphate, and 46.8 mL of dH20. In some embodiments, the RNA preservative is an RNAlater^{™} stabilization solution (ThermoFisher Scientific), as described in U.S. Patent No. 7,056,673. In some embodiments, an RNA preservative can include one or more of triphenylmethane dyes (such as methyl green, crystal violet, pararosaniline, or tris-(4-aminophenyl)methane), cresyl violet, polyamines, and cobalt ions. In some embodiments, an RNA preservative can include one or more of spermine, spermidine, 1,10-diamino-4,7-diazadecane, 1,11-diamino-4,8-diazaundecane, 1,13-diamino-4,10-diazatridecane, 1,14-diamino-4,11-diazatetradecane, 1,15-diamino-4,12-diazapentadecane, 1,16-diamino-4,13-diazahexadecane, 1,17-diamino-4,14-diazaheptadecane, 1,18-diamino-4,15-diazanonadecane, 1,19-diamino-4,16-diazaeicosane, and 1,20-diamino-4,17-diazaheneicosane.

Protein preservatives can be used to prevent or reduce the rate of protein degradation or denaturation, and/or increase the stability of proteins, e.g., to maintain protein structure. Preservatives can include, by way of example, protease inhibitors, surfactants (e.g., nonionic surfactants), emulsifiers, acids, parabens, esters and protein stabilizers.

In some embodiments, the preservative can prevent or reduce the digestion or degradation of proteins by one or more proteases. In some embodiments, the preservative can be a protease inhibitor. In some embodiments, the protease inhibitor is a serine protease inhibitor, a metalloprotease inhibitor, an aminopeptidase inhibitor, a cysteine peptidase inhibitor, or an aspartyl protease inhibitor. In some embodiments, the protease inhibitor can prevent or reduce digestion by proteases such as, but not limited to, trypsin, chymotrypsin, plasmin kallikrein, thrombin, papain, cathepsin B, cathepsin L, calpain and staphopain, endoproteinase Lys-C, Kallikrein, and thrombin. In some embodiments, the protease inhibitor can be 4-(2-aminoethyl)benzenesulfonyl fluoride hydrochloride (AEBSF, CAS 30827-99-7), aprotinin (CAS 9087-70-1), bestatin (CAS 58970-76-6), E-64 (CAS 66701-25-5), leupeptin (CAS 103476-89-7), pepstatin A (CAS 26305-03-3), or N-p-Tosyl-L-phenylalanine chloromethyl ketone (TPCK). In some embodiments, the protein biomarker preservative includes 4-(2-aminoethyl)benzenesulfonyl fluoride hydrochloride (AEBSF, CAS 30827-99-7), aprotinin (CAS 9087-70-1), bestatin (CAS 58970-76-6), E-64 (CAS 66701-25-5), leupeptin (CAS 103476-89-7), pepstatin A (CAS 26305-03-3), DMSA, and bovine serum albumin, and, optionally, N-p-Tosyl-L-phenylalanine chloromethyl ketone (TPCK).

In some embodiments, the preservative can be a protein stabilizer such as, for example, Trehalose or Dextran.

A preservative as disclosed herein can be an acid. In some embodiments, the preservative can be an acid with a pKa between 3 and 7. In some embodiments, the preservative can be citric acid, or sorbic acid.

In some embodiments, the preservative can be a surfactant such as a polysorbate. Exemplary polysorbates include, for example, polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), sorbitan monolaurate, sorbitan monopalmitate, sorbitan monostearate, sorbitan tristearate, and sorbitan monooleate.

In some embodiments, the preservative is a paraben, parahydroxybenzoate, or ester of parahydroxybenzoic acid (4-hydroxybenzoic acid). In some embodiments, the preservative can be propyl paraben.

In some embodiments, the preservative can include dimethyl sulfoxide (DMSA). In some embodiments, the preservative can include bovine serum albumin.

The preservative can be a mixture of two or more of a protease inhibitor, a surfactant, an emulsifier, an acid, a paraben, and an ester. For example, a preservative as described herein can include a mixture of two or more protease inhibitors. In some embodiments, a preservative as described herein can include a mixture of one or more protease inhibitors, and one or more acids. In some embodiments, a preservative as described herein can include a mixture of one or more protease inhibitors, one or more acids, and an ester, e.g., a paraben. In some embodiments, a preservative as described herein can include a mixture of one or more protease inhibitors, one or more acids, one or more esters, and one or more surfactants. In some embodiments, the preservative can include the HALT^{™} protease inhibitor cocktail (Thermo Fisher). In some embodiments, the preservative can include the HALT^{™} protease inhibitor cocktail (Thermo Fisher) and TPCK. In some embodiments, the preservative can be bactericidal to preserve a protein, e.g., a protein biomarker. In some embodiments, the preservative mixture that is bactericidal includes citric acid (CAS 77-92-9), sorbic acid (CAS 110-44-1), propylparaben (CAS 94-13-3), tween 80 (CAS 9005-65-6), ethanol, bovine serum albumin, and TPCK (CAS 402-71-1).

In some embodiments, a preservative mixture containing one or more protease inhibitors can be contacted with a protein in the gastrointestinal tract to stabilize the protein. In some embodiments, the protein is an immunoglobulin. In some embodiments, the protein is an IgA or IgM. In some embodiments, the protein is a secretory IgA. In an exemplary embodiment, a preservative mixture containing AEBSF, aprotinin, bestatin, E-64, leupeptin and pepstatin A protease inhibitors (HALT^{™}, Thermo Fisher), and N-p-Tosyl-L-phenylalanine chloromethyl ketone (TPCK, Sigma Aldrich) can be used to stabilize one or more immunoglobulin proteins in the gastrointestinal tract, e.g., secretory IgA.

In some embodiments, a preservative mixture containing one or more protease inhibitors, acids, parabens, and surfactants can be contacted with a protein in the gastrointestinal tract to stabilize the protein. In some embodiments, the protein is not an immunoglobulin. In an exemplary embodiment, a preservative mixture containing AEBSF, aprotinin, bestatin, E-64, leupeptin and pepstatin A protease inhibitors (HALT^{™}, Thermo Fisher), N-p-Tosyl-L-phenylalanine chloromethyl ketone (TPCK, Sigma Aldrich), citric acid, sorbic acid, propyl paraben, polysorbate 80 (Tween 80), BSA can be used to stabilize one or more non-immunoglobulin proteins in the gastrointestinal tract, e.g., a cytokine, calprotectin, S100A12, lactoferrin, M2-pyruvate kinase, neopterin, a metalloproteinase, a myeloperoxidase, polymorphonuclear elastase, and/or alpha 1 antitrypsin eosinophilic protein X.

In some embodiments, one or more internal controls are included in an ingestible device, as described herein, that is used to collect one or more analytes. The internal control can be used to monitor the stability and degradation of small molecules, nucleic acids, and/or proteins in the device over time. In some embodiments, the internal control can be a small molecule, a nucleic acid, and/or a protein. In some embodiments, the small molecule internal control can be 2,4 dinitrophenol (2,4, DNP), femocene, and/or a deuterium-labeled cholesterol. In some embodiments, the nucleic acid internal control can be a DNA internal control. In some embodiments, the nucleic acid internal control can be a RNA internal control. In some embodiments, the RNA internal control can be a G+C-rich (60%) RNA molecule with extensive secondary structure, based on a modified delta virus genome, as described in Dingle et al., J. Clin. Microbiol. 42(3):1003-1011, 2004. In some embodiments, the protein internal control can be human serum albumin (HSA), fluorescein isothiocyanate, and/or biotin.

In some embodiments, the preservative is a microbial preservative. In exemplary embodiments, the preservative prevents, inhibits, or reduces the growth and/or multiplication of a microorganism. In some embodiments, the preservative permanently prevents, inhibits, or reduces the growth and/or multiplication of a microorganism. In exemplary embodiments, the preservative prevents, inhibits, or reduces the growth and/or multiplication of bacteria. In some embodiments, the preservative permanently prevents, inhibits, or reduces the growth and/or multiplication of bacteria. In some embodiments, the preservative is one or more of a bacteriostatic, bacteriocidal, and/or fixative compound.

Bacteriostatic preservatives arrest the growth or multiplication of the bacteria. In some embodiments, the preservative kills the bacteria, thereby preventing growth and multiplication. Bactericidal preservatives kill bacteria. Bacteria enter a device as described herein in the GI tract of a subject, and are contacted with a bacteriostatic preservative that arrests bacterial growth and multiplication, or a bactericidal preservative that kills the bacteria. As a result, the numbers of bacteria in the device are representative of the bacterial microflora that was present in the GI tract at the time the bacteria first entered the device.

In some embodiments, the preservative can be a bacteriostatic food preservative, such as, but not limited to, sorbic acid, citric acid, propyl paraben, nisin, dimethyl dicarbonate, and ethylenediaminetetraacetic acid (EDTA). In some embodiments, the preservative can be sodium azide, hydroxyurea, fusidic acid, diazolidinyl urea, imidazolidinyl urea, salicylic acid, barium and nickel chloride, metallic copper, thimerosal, 2-phenoxyethanol, or ProClin^{™}. In some embodiments, the preservative can be one or more of sorbic acid, citric acid, propyl paraben, nisin, dimethyl dicarbonate, ethylenediaminetetraacetic acid (EDTA), sodium azide, hydroxyurea, fusidic acid, diazolidinyl urea, imidazolidinyl urea, salicylic acid, barium and nickle chloride, metallic copper, thimerosal, 2-phenoxyethanol, and ProClin^{™}.

In some embodiments, the preservative prevents or reduces nucleic acid degradation, in addition to preventing or inhibiting the growth and/or multiplication of bacteria. The preservation of nucleic acid integrity allows for the quantification of bacteria using PCR-based DNA or RNA analysis methods, e.g., 16S ribosomal RNA PCR and sequencing. In some embodiments, the preservative includes EDTA.

In some embodiments, the bactericidal preservative can include one or more of citric acid (CAS 77-92-9), sorbic acid (CAS 110-44-1), propylparaben (CAS 94-13-3), Tween 80 (CAS 9005-65-6), ethanol, bovine serum albumin, and TPCK (CAS 402-71-1). In some embodiments, the bactericidal preservative is a mixture of citric acid, sorbic acid, propyl-paraben, and Tween 80, e.g., the bactericidal preservative can include 2.5% (m/v) citric acid, 2.5% (m/v) sorbic acid, 2.5% (m/v) propyl-paraben), and 3.13% (m/v) Tween 80. In some embodiments, the bactericidal preservative is a mixture of sorbic acid, Tris, EDTA, Tween 80, and NaCl, e.g., the bactericidal preservative can include 2.0% (m/v) sorbic acid, tris, EDTA, 1.0% (m/v) Tween 80, and 1.0% (m/v) NaCl. In some embodiments, the bactericidal preservative is a heavy metal bactericidal mixture. In some embodiments, the bactericidal preservative is a mixture that includes barium chloride and nickel chloride. In some embodiments, the bactericidal preservative is thimerosal, e.g., a stabilizer that includes 0.1% thimerosal.

A cell filter 1250 is located between distal end 1214 of wicking sponge 1210 and a first end 1222 of transfer sponge 1220. The cell filter 1250 is configured to prevent undesired cells, such as Hela cells, from entering one or more downstream sponges in sampling system 1200, particularly sponges used in testing. In some embodiments, the filter can be used to filter and/or selectively kill eukaryotic cells. Excluding such undesired cells enhances the accuracy of various analytical results.

Fluid that passes from wicking sponge 1210 and through cell filter 1250 can enter transfer sponge 1220 via its first end first end 1222. Transfer sponge 1220 is configured to move the filtered fluid from cell filter 1250 to volume sponge 1230 and/or assay sponge 1240.

To allow transfer sponge 1220 (made of an absorptive material) to absorb a relatively large volume of fluid, transfer sponge 1220 is shaped (e.g., arc-shaped) to provide a relatively long distance between first end 1222 of transfer sponge 1220 and a second end 1224 of transfer sponge 1220. Second end 1224 contacts both volume sponge 1230 and assay sponge 1240 while preventing volume sponge 1230 and assay sponge 1240 from directly contacting each other. A barrier 1260 is located between first end 1222 and volume sponge 1230 to ensure that fluid absorbed in transfer sponge 1220 at first end 1222 travels to second end 1224 before being absorbed by volume sponge 1230. Although depicted as being arc-shaped, transfer sponge 1220 can have one or more different configurations, such as, for example, an extended straight line or multiple curves, depending, for example, on the desired volume of sample and/or desired transfer speed. In general, the shorter and/or thinner the path of transfer sponge 1220, the quicker the transfer speed from first end 1222 to second end 1224. The transfer sponge 1220 may be made of a VF2 sponge, an Ahlstrom M13 sponge, MF/F material, or another suitable absorptive material.

Volume sponge 1230 is made of an absorptive material that absorbs additional fluid for testing and is in fluid communication with assay sponge 1240 via second end 1224 of transfer sponge 1220. Volume sponge 1230 can be particularly useful when fluorescent or optical testing is used. In some embodiments, assay sponge 1240 and transfer sponge 1224 may not individually contain a sufficient volume of the sample to attain a confident test result. The volume of volume sponge 1230, assay sponge 1240, and second end 1224 of the transfer sponge 1220 sum to a sufficient testing volume for optical, and other, tests. Assay sponge 1240 contains a chemical assay that is used to test the sample or to prepare the sample for a test. Once assay sponge 1240 is saturated, the assay chemicals are free to flow from assay sponge 1240 and interact with sample absorbed by transfer sponge 1220 and volume sponge 1230. Volume sponge 1230 and the assay sponge 1240 may be made of a VF2 sponge, an Ahlstrom M13 sponge, MF/F material, or another suitable absorptive material. Preferably, the wicking sponge, wicking tongue, transfer sponge, and assay sponge are Ahlstrom M13 sponges, and the volume sponge is a VF2 sponge.

Cell filter 1250 can be made from any appropriate material and have any appropriate dimensions. Exemplary materials include polycarbonate (PCTE), polyethersulfone (PES), polyester (PETE) and polytetrafluoroethylene (PTFE). In some embodiments, the dimensions of cell filter 1250 can be about 9.5 millimeters by about 6.5 millimeters by about 0.05 millimeter.

Sampling system 1200 also includes a membrane 1270 located between assay sponge 1240 and a vent 1280 for gases to leave sampling system 1200. Membrane 1270 is configured to allow one or more gases to leave sampling system 1200 via an opening 1280, while maintaining liquid in sampling system 1200.

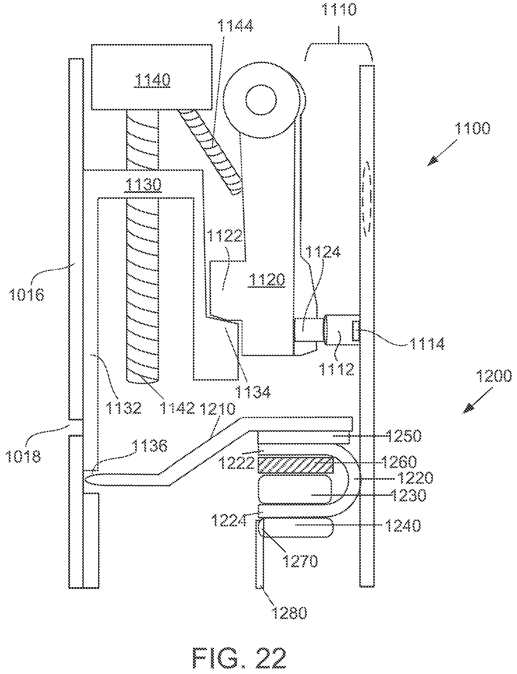

FIG. 22 illustrates an embodiment of ingestible device 1000 with a relatively detailed view of both valve system 1100 and sampling system 1200. FIG. 22 shows valve system 1100 positioned prior to actuation of actuation system 1110 (e.g., when configured as shown in FIGs. 13A, 14A, 15A and 20A).

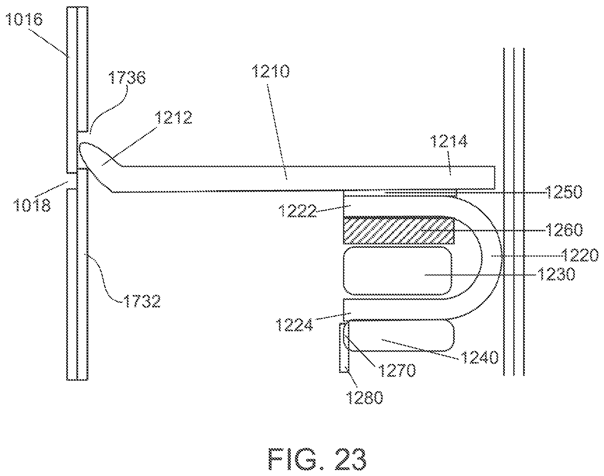

FIG. 23 illustrates an embodiment of an ingestible device including sampling system 1200 and three-stage valve system 1700 positioned in its third stage.

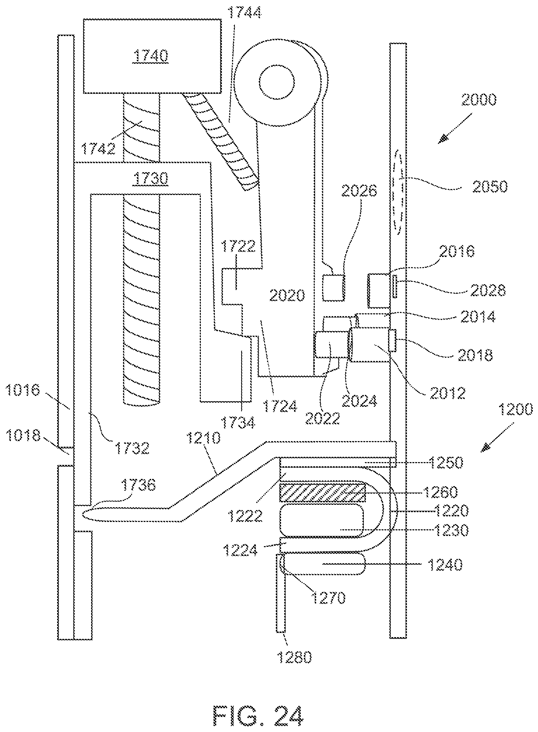

FIG. 24 illustrates an embodiment of an ingestible device 1000 including sampling system 1200 and valve system 2000 positioned in its third stage.

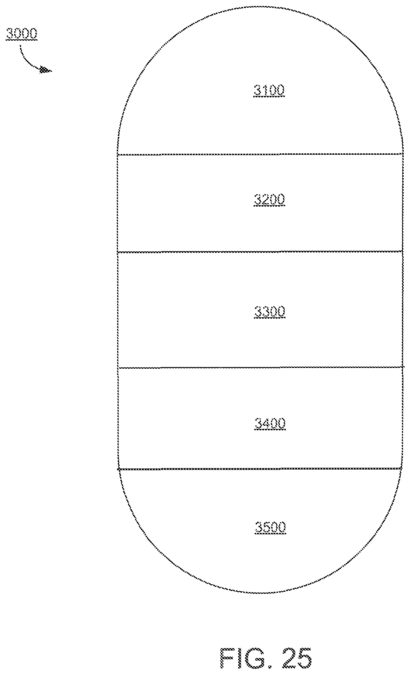

FIG. 25 is a highly schematic illustration of an ingestible device 3000 that contains multiple different systems that cooperate for obtaining a sample and analyzing a sample, e.g., within the GI tract of a subject. Ingestible device 3000 includes a power system 3100 (e.g., one or more batteries), configured to power an electronics system 3200 (e.g., including a control system, optionally in signal communication with an external base station), and an analytic system 3500.

Exemplary analytical systems include assay systems, such as, for example, optical systems containing one or more sources of radiation and/or one more detectors. Such systems may use, for example, a light source that illuminates and a sample and a detector configured to detect light that is emitted by the sample (e.g., fluorescence spectroscopy), optical density (e.g., the portion of light that passes through the sample), and/or light that is diffracted by sample (e.g., diffraction optics). An analytical system may use, for example, ELISA (enzyme-linked immunosorbent assay). An analytical system may use, for example, LOCI (luminescent oxygen channeling) or fluorescent oxygen channeling. An analytical technique may involve incubating and/or diluting a sample before or during the analysis/assaying of the sample. An analytical technique may involve the use of staining/dyeing a live cell.

Ingestible device 3000 also includes a sampling system 3400 for taking in a sample from the environment exterior to ingestible device 3000, and a valve system 3300 that regulates the ability of a fluid to access sampling system 3400.

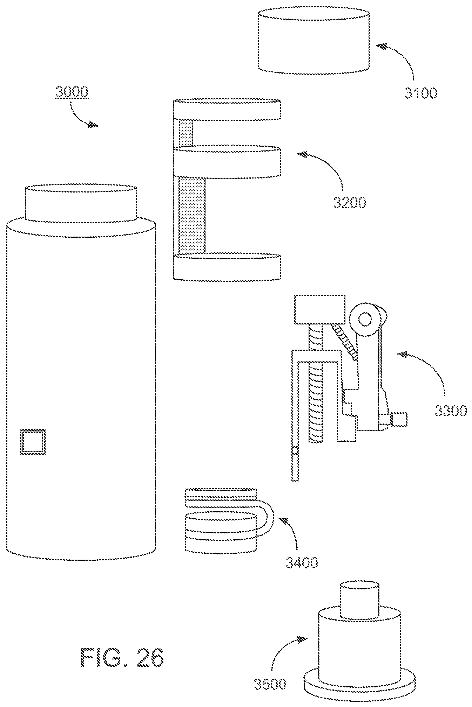

FIG. 26 provides an exploded view of the ingestible device 3000. FIG.26 includes the exploded view of ingestible device 3000, showing a general configuration of the systems in FIG. 25. FIG. 26 includes power system 3100 (e.g., a stack of batteries), electronic system 3200 (e.g., a PCB and associated wiring), valve system 3300, sampling system 3400, and analytic system 3500.

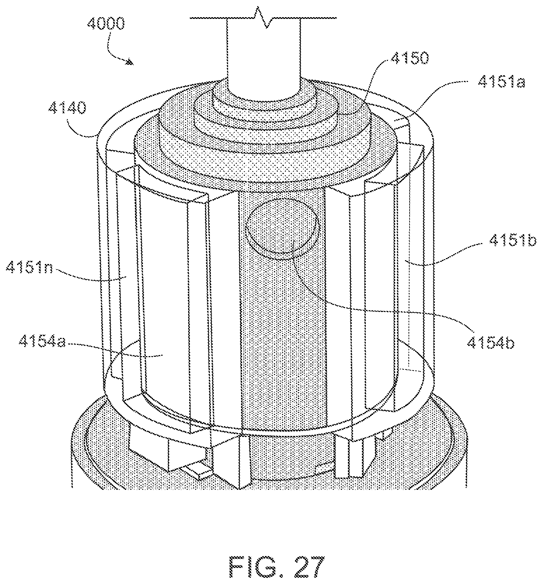

FIG. 27 illustrates a portion of an ingestible device 4000 with a port 4154b in an open position to the exterior of the ingestible device 4000. The ingestible device 4000 may include a cylinder-shaped rotatable element 4150 that includes sampling ports on the wall of the rotatable element 4150. The sampling chamber 4150 is wrapped by a shell element 4140 with dividers to form a series of dilution chambers 4151a-n between the shell element 4140 and the rotatable element 4150. In operation, when the ingestible device 4000 determines the device itself arrives at a target location within the GI tract, the rotatable element 4150 may be rotated into an open position such that an aperture of the shell element 4140 is aligned with the port 4154b on the wall of the rotatable element 4150 and the port 4154b is exposed to the exterior of the ingestible device 4000 through the aperture. In this way, fluid from the GI tract can enter the port 4154b and occupy the volume defined by the port 154b. In the embodiment shown in FIG. 24, the port 4154b may be a depression on the surface of a rotatable element 4150 and a number of dilution chambers 4151a-n are positioned circumferentially around the axis of rotation of the rotatable element 4150. As previously discussed, each of the dilution chambers 4151a-n may store a dilution fluid. In some embodiments, the depression is a cylindrical depression. Optionally, the depression may be a rectangular depression, or any concave depression forming a regular or irregular shape. In another embodiment, the port 4154b may be connected to a chamber (not shown) within the rotatable element 4150 to create an enlarged space to store the GI fluid sample from the external environment of the ingestible device.

In some embodiments, the ingestible device 4000 may further include a controller and an actuator. The controller may determine that the ingestible device 4000 is located at a target location of the GI tract, and then the actuator may trigger the rotation of the rotatable element 4150 to align the port 4154b at the open position to initiate the sampling. For example, the housing of ingestible device 4000 may have a pH-sensitive enteric coating to detect or otherwise be sensitive to a pH level of the environment external to the ingestible device 4000, based on which the controller may determine whether the ingestible device has arrived at a target location. For another example, the ingestible device 4000 may include an optical sensing unit that transmits an illumination to the environment and collects a reflectance, based on which, the regio-specific location of the ingestible device 4000 may be identified based on optical characteristics of the reflectance.

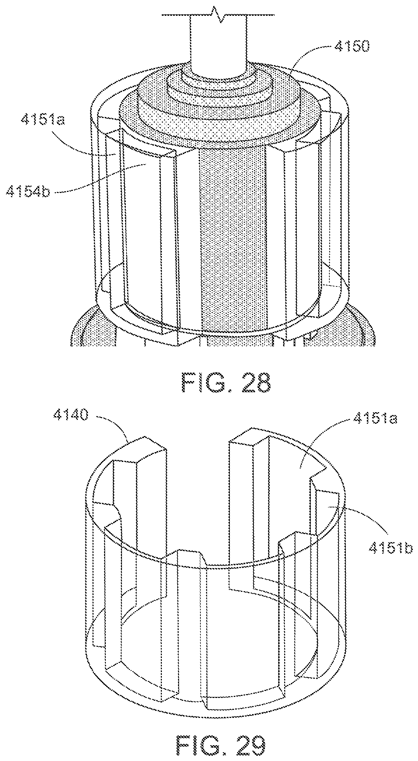

FIG. 28 shows one embodiment of a portion of an ingestible device with a port 4154b at a first position aligned with a first dilution chamber 415 1a. In operation, the rotatable element 4150 may be rotated to align the sampling port 4154b and the first dilution chamber 4151a such that the fluid sample from the GI tract stored within the volume of the sampling port 4154b can be combined with dilution fluid in the first dilution chamber to form a first dilution. The first dilution may then occupy the combined volume of the port 4154b and first dilution chamber 4151a. Optionally, the rotatable element 4150 may be subsequently rotated to a second position such that the port 4154b containing a portion of the first dilution is then moved to be aligned and in fluid communication with another dilution chamber, e.g., a second dilution chamber that is next to the first dilution chamber along the rotational direction. In this way, the first dilution stored within the port 4154b may then again be diluted with the dilution fluid stored within the second dilution chamber. Similarly, if the rotatable element 4150 keeps rotating and allows the port 4154b to be serially aligned with each dilution chamber, then the original GI fluid sample may be diluted serially and each dilution chambers 4151a-n may be left with a diluted GI fluid sample at a different dilution ratio.

FIG. 29 shows an embodiment of an element 4140 forming part of a set of five dilution chambers (e.g., including 4151a-b) for surrounding a rotatable element (e.g., 4150 in FIGs. 21-22) in an ingestible device as described herein. In some embodiments, the device may contain a single dilution chamber. Alternatively, the device may contain 2, 3, 4, 5, 6, 7, 8 or greater than 8 dilution chambers.

In some embodiments, each dilution chamber 4151a-n may be filled with a dilution fluid prior to the ingestible device 4000 being administered. In another embodiment, the dilution fluid may be stored in a separate reservoir (not shown) within the ingestible device 4000. At the time when the ingestible device 4000 is determined to be at a target location within the GI tract, a pumping mechanism may pump the dilution fluid into one or more dilution chambers 4151a-b via one or more outlet (not shown) of the reservoir.

In some embodiments, the shell element 4140 may have valves or pumps (not shown) between the dilution chambers 4151a-n. For example, the diluted fluid from a first dilution chamber may be pumped into a second dilution chamber via a valve between the two chambers.

Devices of the type depicted in FIGs. 27-29 optionally can include a sampling system as disclosed herein.

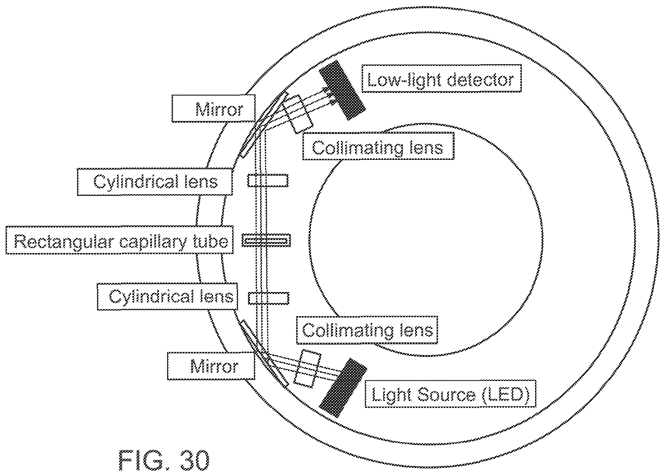

In certain embodiments, an ingestible device includes a microscopic evaluation system. In some embodiments, bacterial cells in a sample may be first labeled with fluorescent dyes (such as those described herein), and the fluorescently-labeled cells may be imaged and counted by the microscopic evaluation using an ingestible device as described herein. For example, in some embodiments, the bacterial cells in a sample may be labeled with multiple analyte-binding reagents (e.g., multiple antibodies each specific for different types of analytes (e.g., bacteria of different genera, species, and/or strains)), each conjugated to a different dye, thereby allowing for the imaging, detection and counting of the different types of analytes (e.g., bacteria) present in the sample. In other embodiments, the fluorescently-labeled cells are counted as they pass through an onboard flow system (e.g., microfluidic single cell channeling). Examples of flow cytometry systems include hydrodynamic focusing, small diameter capillary tube flow, and rectangular capillary tube flow. As described herein, live bacterial cells are labeled, and the principles of flow cytometry are used to quantify labeled cells. Generally speaking, the photons from an incident laser beam are absorbed by the fluorophore and raised to a higher, unstable energy level. Within less than a nanosecond, the fluorophore re-emits the light at a longer representative wavelength where it is passed through a series of dichroic filters. This reemitted light can be collected and interpreted as proportional to the number of labeled bacterial cells. In some embodiments, a sheath fluid is not used as part of the flow system to help accommodate the volume restrictions of the device. In some embodiments, a rectangular capillary tube is used to achieve a sufficiently large cross-sectional area and relatively thin inspection area. The flow cytometry optical system operates parallel to the fluidics system and serves to observe the redirection of light passing through the cell and delivers information about the bacterial cells. In some embodiments, rather than using a conventional laser and spherical lenses to focus the light to a point, an LED and cylindrical lenses are used to focus the light to a line across a rectangular capillary tube. In other embodiments, collimating lenses are used to make the light source parallel, while cylindrical lenses are used to refine the inspection area. An exemplary optical configuration for this arrangement can be seen in FIG. 30. In some embodiments, optical filters can be added to permit the use of fluorophores. The characteristic wavelength of reemitted light from the fluorophores can be isolated and detected with the use of dichroic, bandpass, and short or long wave pass filters. Generally, multiple dichroic lenses and photomultipliers are used, however, due to space limitations, only a single side-scatter detector and forward scatter detector may be used in certain embodiments.

One of the design challenges of integrating flow cytometry into the device is to provide a pumping mechanism. Without moving fluid, individual bacterial cells cannot be identified and accounted for by flow cytometry within a fixed volume of fluid. In some embodiments, a gear motor is to move fluid through the device. For example, a micromotor including a planetary gearhead (e.g., with a 25:1 reduction) can provide the desired amount of torque to create fluid flow. In another embodiment, a series of piezoelectric resistors embedded in the surface of a microfabricated plate is used to create flow. In yet another embodiment, a micropump that includes a pair of one-way valves and uses a magnetic pump membrane actuated by an external magnetic field is used to create flow.

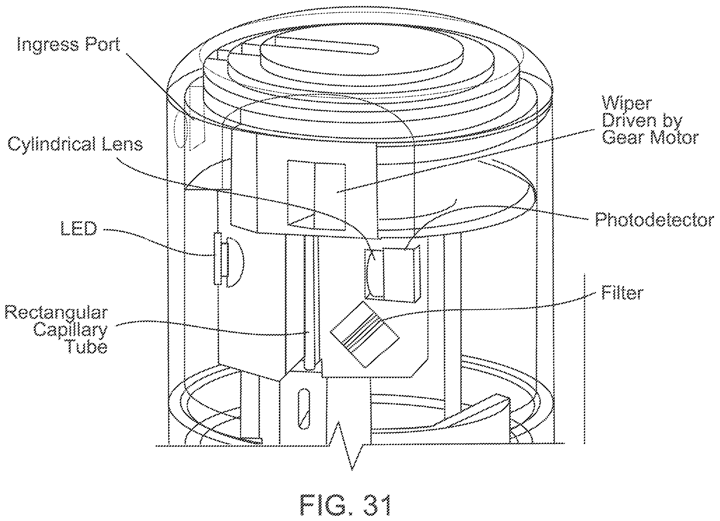

In some embodiments, the system architecture includes an opening and sealing mechanism combined with a rotary wiper which creates a pressure driven flow via a gear motor. The gear motor can be used for other functions in the device. As shown in FIG. 31, the components of the optics and flow chamber systems fit within the device. In some embodiments, the sample fluid is absorbed via a flexible membrane at the top of the capsule. In some embodiments, the gear motor has 270° of permissible travel which serves to open and fill the fluid chamber. During closure, the motor closes the ingress port while simultaneously pushing the fluid through the rectangular capillary tube where the optical system is located. The threaded component allows the flexible membrane to close and seal the ingress channel without changing the wiper height. In some embodiments, the volume of the sample chamber is 25µL, 50µL, 75µL or more. In some embodiments, two or more samples are taken from the GI tract to procure a sufficient sample size. Referring to FIG. 31, an LED on the left side of the capillary tube and the low-light photodetector on the right for capturing forward and side scatter are shown. Once the fluid passes through the capillary tube, it exits the capsule via a one-way valve. In certain embodiments, the flow system allows for the detection of cell size and internal cell complexity, in addition to cell quantitation.

The foregoing discussion is not exhaustive with respect to various ingestible device designs, either with respect to sampling componentry or absorbent (sponge) design.

As an example, while ingestible devices have been described that include one or more optical systems incorporated into the ingestible device, in some embodiments, an ingestible device does not include an optical system. Optionally, such ingestible devices may also not include any other analytical componentry. In embodiments of an ingestible device, which do not include an optical system and/or other analytical componentry, there may be more room inside the ingestible device to store one or more samples.

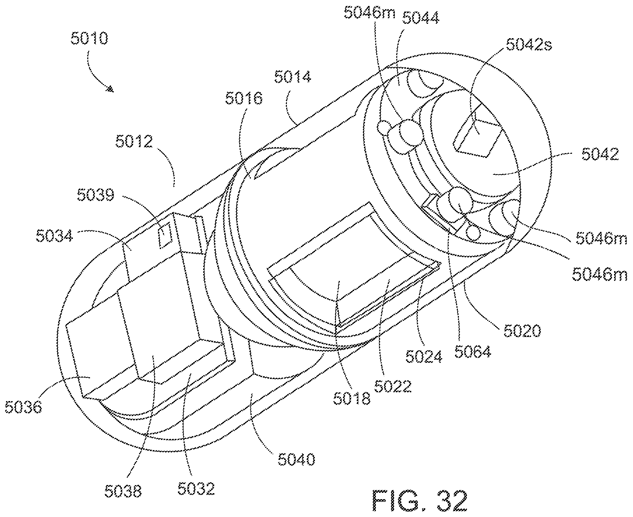

FIG. 32 shows a partial view of an exemplary embodiment of an ingestible device 5010 in which a portion of the enclosure of ingestible device 5010 has been removed. Ingestible device 5010 may be used for collecting substances. Ingestible device 5010 may generally be in the shape of a capsule, like a conventional pill. Accordingly, the shape of ingestible device 5010 provides for easier ingestion and is also familiar to healthcare practitioners and patients.

The structure of ingestible device 5010 includes first portion 5012 and second portion 5014. First portion 5012 includes control electronics, a power supply, and a communication system. Second portion 5014 is generally configured to interact with the GI tract, such as, for example but not limited to, sample collection, substance delivery and environmental monitoring. Second portion 5014 includes a storage sub-unit 5016 with one or more chambers 5018 and a chamber enclosure 5020 that encloses or overlays a storage sub-unit 5016. Each chamber 5018 has a corresponding chamber opening 5022. Chamber enclosure 5020 has an access port 5024. In this example embodiment, ingestible device 5010 includes three chambers 5018, but there can be other embodiments that have one, two or more than three chambers.

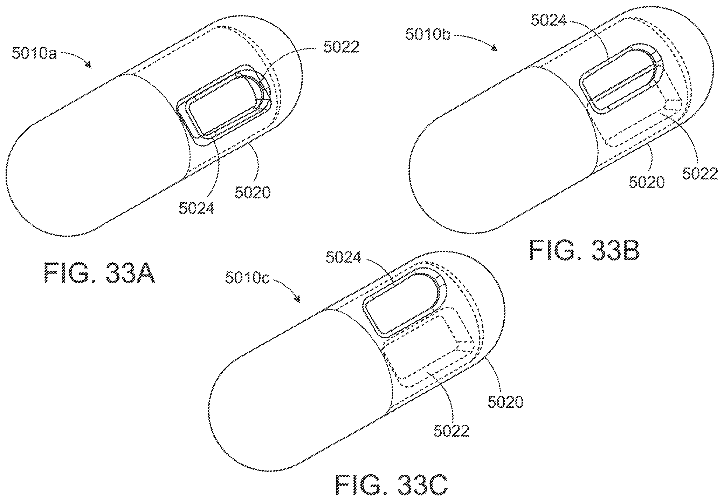

FIGs. 33A-33C illustrate operation of ingestible device 5010. Generally, chamber enclosure 5020 operates as a "closed-loop" revolver mechanism. Chamber enclosure 5020 rotates, in a controlled manner, to align the access port 5024 with each of chamber openings 5022 for collecting, at targeted locations, samples of the contents in the GI into corresponding chambers 5018 (shown in FIG. 32), and/or for delivering substances stored in chambers 5018 (shown in FIG. 32) to targeted locations within the body.

Generally, during collection of samples, the rotation of chamber enclosure 5020 may be described as a "closed-loop" revolver mechanism because each chamber opening 5022 is exposed only once during the passage of ingestible device 5010 within the body in order to avoid cross-contamination of the collected samples. In other words, in some embodiments, chamber enclosure 5020 ideally rotates only once when collecting samples during each usage of ingestible device 5010 so that access port 5024 aligns with each of chamber openings 5022 serially and only once. That is, during collection of samples, access port 2224 does not bypass any chamber opening 5022 and also does not return to a previous chamber opening 5022 during its rotation.

In some embodiments, chamber enclosure 5020 can rotate in a bidirectional motion before completing one revolution and/or perform multiple revolutions during one usage of the ingestible device 5010 so that at least one chamber opening 5022 is exposed multiple times. A chamber opening 5022 may need to be exposed multiple times if its corresponding chamber stores solids or semi-solid reagents, sensors or cleaning agents for cleaning the GI tract.

As illustrated in FIG. 33A, shown therein generally is ingestible device 5010 in an open position 5010a in which access port 5024 on chamber enclosure 5020 is aligned with a chamber opening 5022. In this configuration, ingestible device 5010 may collect substances through chamber opening 5022. In other words, the contents of the GI tract may be forced into exposed chamber 5018 (shown in FIG. 32) through muscular contractions (e.g., peristalsis).

Thereafter, chamber enclosure 5020 may rotate to seal chamber opening 5022. FIG. 33B shows ingestible device 5010 with a partially open/partially closed position 5010b in which access port 5024 has been rotated such that chamber enclosure 5020 partially seals chamber opening 5022.

FIG. 33C shows ingestible device 5010 in a closed position 5010c, in which the chamber enclosure 5020 has been rotated a distance such that access port 5024 completely seals chamber opening 5022. If chamber enclosure 5020 has not rotated one revolution, chamber enclosure 5020 may continue to rotate in the same direction in order to align access port 5024 with another chamber opening 5022 depending if ingestible device 5010 has been configured to perform another operation (e.g., sampling or distribution).

In another example embodiment, chamber enclosure 5020 may be stationary and storage sub-unit 5016 (shown in FIG. 32) may instead rotate to align its one or more chamber openings 5022 with access port 5024. Rotating storage sub-unit 5016 instead of chamber enclosure 5020 may provide greater control over the rotation motion and a more constant motion since storage sub-unit 5016 would not be subjected to a varying viscosity arising from the contents in the GI tract. This arrangement, however, may limit a volume of at least one of chambers 5018.

In some embodiments, chamber enclosure 5020 or storage sub-unit 5016 may rotate in a predetermined sequence of bidirectional rotational motions. As described above, when storage sub-unit 5016 is configured to rotate instead of chamber enclosure 5020, the volume of at least one of chambers 5018 can be limited. In order to avoid having to limit the volume of the chambers 5018, non-recess areas that may be used to separate different chambers 5018 in storage sub-unit 5016 may be minimized in volume or removed. Ingestible device 5010 can rotate in a first direction for aligning access port 5024 with one of the two adjacent chambers. Ingestible device 5010 can be configured to rotate in a second direction that is opposite to the first direction in order to avoid cross contamination between samples collected into or substances released from those two adjacent chambers.

Ingestible device 5010 may be used for collecting usable samples from the contents of the GI tract (e.g., 100 µL sized samples) and maintaining each sample in isolation from one another until the samples are extracted.

In some embodiments, ingestible device 5010 may also be configured to conduct in-vivo measurements. Ingestible device 5010 is introduced into the body with some of chambers 5018 being empty and some of chambers 5018 carrying at least one reagents. At a predefined location in the body, ingestible device 5010 is configured to collect a sample from the GI tract and to store the sample into a chamber carrying at least one reagent. After collection, in-vivo analysis may be conducted based on how the collected sample interacts with the reagent inside chamber 5018. For example, ingestible device 5010 may use a biochemistry assay, such as an enzyme-linked immunosorbent assay (ELISA), for performing in-situ experiments on collected samples. Alternatively, peripherals can be included into chambers 5018 for changing the dynamics of several in-vivo analysis and measurements. The peripherals may include a light source, a receiver, a transducer, a heater, and the like. In general, the in-vivo experiments vary according to the type of information that is being sought.

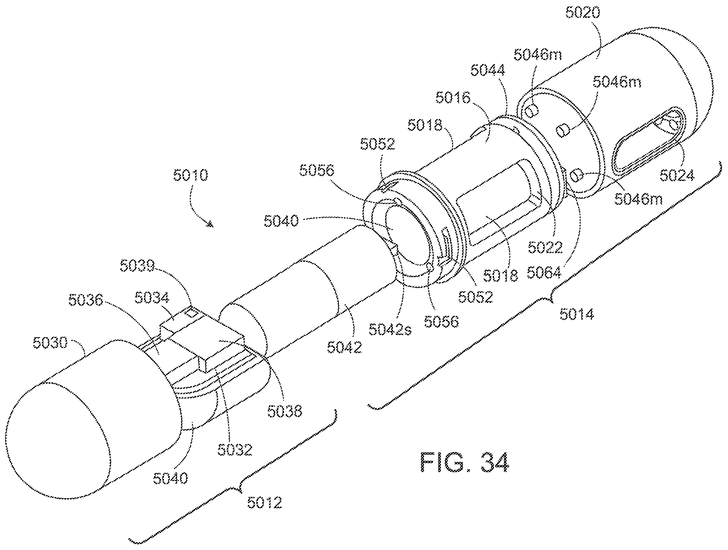

FIG. 34 illustrates an exploded view of the components of ingestible device 5010 in one example embodiment. First portion 5012 of ingestible device 5010 includes an end closure 5030, and electronic components embedded on a main printed circuit board (PCB) 5032 including a communication subsystem having communication peripherals 5034 and a transceiver 5036, a main microcontroller (i.e., processor) 5038, a power supply 5040 and other peripheral components, including a magnetic switch 5039, described in further detail below. Second portion 5014 of ingestible device 5010 generally includes a motor 5042 with a shaft 5042s protruding from motor 5042, storage sub-unit 5016, a secondary PCB 5044, an encoding magnet arrangement 5046m and the chamber enclosure 5020. Generally, by placing main PCB 5032 and secondary PCB 5044 in distinct regions inside ingestible device 5010, they may be prevented from experiencing the same electrical or physical hazards. Motor 5042 is inserted into a motor compartment 5054 that is located in the center of storage sub-unit 5016. PCB 5044 is annular and includes one or more peripheral electronic components (e.g., a capacitor and a resistor, which can be used as a pull-up resistor), and a sensor 5064. Storage sub-unit 5016 further includes chambers 5018, with chamber openings 5022, for storing one or more collected samples and/or for storing one or more dispensable substances. Access holes 5056 are also located on storage sub-unit 5016 oriented towards the first portion 5030.

End enclosure 5030 provides a hollow space defined by an inner wall that is cylindrical with a domed end portion. End enclosure 5030 also includes engagement members for aligning and releasably engaging with storage sub-unit 5016 to releasably lock end enclosure 5030 in place during operation. In particular, engagement members releasably engage complementary structures 5052 in storage sub-unit 5016. When end enclosure 5030 locks with storage sub-unit 5016, end enclosure 5030 overlaps with a rear of storage sub-unit 5016 and creates a seal. In some embodiments, the overlap between end enclosure 5030 and storage sub-unit 5016 may span a width of 3 millimeters.

Some or all of the sponges of the above-described sampling systems may contain one or more preservatives (see discussion above). Typically, the assay sponge and/or the volume sponge and/or the transfer sponge contain one or more preservatives. Typically, the preservative(s) are selected based on the analyte of interest, e.g., an analyte (such as a nucleic acid or protein biomarker) for a GI disorder.

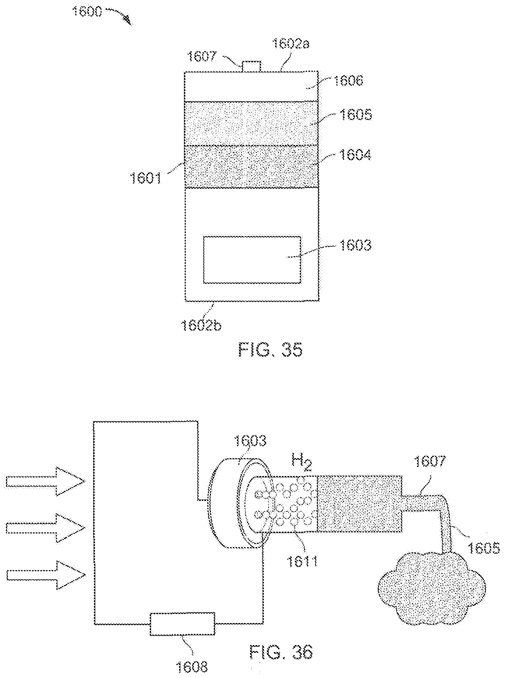

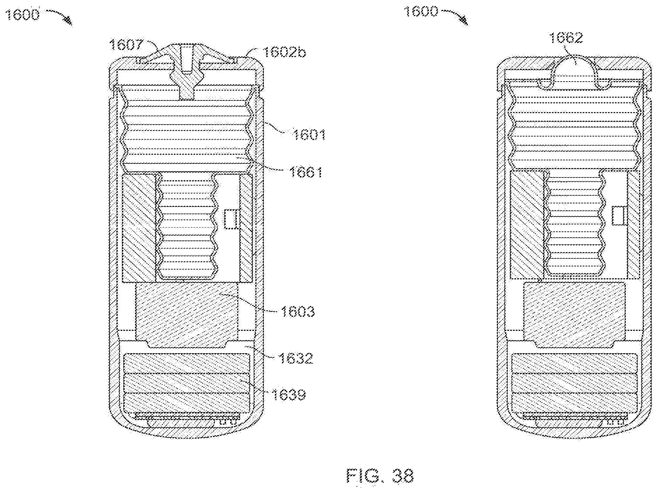

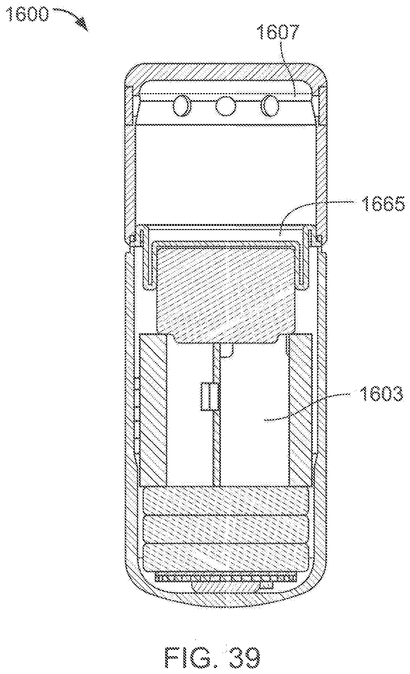

In some embodiments, an ingestible is configured to delivery one or more substances (e.g., one more therapeutic substances). FIGs. 35-55 provide illustrative and non-limiting examples of such ingestible devices. It is to be understood that one more features from such an ingestible device can be combined with one or more features of an ingestible device configured to take one more samples, such as, for example, described above with regarding to FIGs. 1-34.

FIG. 35 provides an example mock-up diagram illustrating aspects of a structure of an ingestible device 1600 for delivering a dispensable substance, according to some embodiments described herein. In some embodiments, the ingestible device 1600 may generally be in the shape of a capsule, a pill or any swallowable form that may be orally consumed by an individual. In this way, the ingestible device 1600 may be ingested by a patient and may be prescribed by healthcare practitioners and patients.

The ingestible device 1600 includes a housing 1601 that may take a shape similar to a capsule, a pill, and/or the like, which may include two ends 1602a-b. The housing 1601 may be designed to withstand the chemical and mechanical environment of the GI tract (e.g., effects of muscle contractile forces and concentrated hydrochloric acid in the stomach). A broad range of materials that may be used for the housing 1601. Examples of these materials include, but are not limited to, thermoplastics, fluoropolymers, elastomers, stainless steel and glass complying with ISO 10993 and USP Class VI specifications for biocompatibility; and any other suitable materials and combinations thereof.

In some embodiments, the wall of the housing 1601 may have a thickness of 0.5mm-1mm, which is sufficient to sustain an internal explosion (e.g., caused by hydrogen ignition or over pressure inside the housing).

The housing 1601 may or may not have a pH-sensitive enteric coating to detect or otherwise be sensitive to a pH level of the environment external to the ingestible device. As discussed elsewhere in the application in more detail, the ingestible device 1600 may additionally or alternatively include one more sensors, e.g., temperature sensor, pH sensor, impedance sensor, optical sensor.

The housing 1601 may be formed by coupling two enclosure portions together. The ingestible device 1600 may include an electronic component within the housing 1600. The electronic component may be placed proximally to an end 1602b of the housing, and includes a printed circuit board (PCB), a battery, an optical sensing unit, and/or the like.

The ingestible device 1600 further includes a gas generating cell 1603 that is configured to generate gas and thus cause an internal pressure within the housing 1601. In some embodiments, the gas generating cell may include or be connected to a separate channel or valve of the ingestible device such that gas may be release through the channel or valve to create a motion to alter the position of the ingestible device within the GI tract. Such gas release can also be used to position the ingestible device relative to the intestinal lining. In another embodiment, gas may be released through the separate channel or valve to alter the surface orientation of the intestinal tissue prior to delivery of the dispensable substance.

A traveling plunger 1604 may be placed on top of the gas generating cell 1603 within the housing 1601. The traveling plunger 1604 is a membrane that separates the gas generating cell 1603 and a storage reservoir that stores the dispensable substance 1605. In some embodiments, the traveling plunger 1604 may be a movable piston. In some embodiments, the traveling plunger 1604 may instead be a flexible membrane such as but not limited to a diaphragm. In some embodiments, the traveling plunger 1604, which may have the form of a flexible diaphragm, may be placed along an axial direction of the housing 1601, instead of being placed on top of the gas generating cell 1603. The traveling plunger or the membrane 1604 may move (when the membrane 1604 is a piston) or deform (when the membrane 1604 is a diaphragm) towards a direction of the end 1602a of the housing, when the gas generating cell 1603 generates gas to create an internal pressure that pushes the membrane 1604. In this way, the membrane or traveling plunger 1604 may push the dispensable substance 1605 out of the housing via a dispensing outlet 1607.

The housing 1601 may include a storage reservoir storing one or more dispensable substances 1605 adjacent to the traveling plunger 1604. The dispensable substance 1605 may take the form of a powder, a compressed powder, a fluid, a semi-liquid gel, or any other dispensable or deliverable form. The delivery of the dispensable substance 1605 may take a form such as but not limited to bolus, semi-bolus, continuous, systemic, burst delivery, and/or the like.

**In** some embodiments, the storage reservoir may include multiple chambers, and each chamber stores a different dispensable substance. For example, the different dispensable substances can be released at the same time via the dispensing outlet 1607. Alternatively, the multiple chambers may take a form of different layers within the storage reservoir such that the different dispensable substance from each chamber is delivered sequentially in an order. **In** one example, each of the multiple chambers is controlled by a separate traveling plunger, which may be propelled by gas generation. The electronic component may control the gas generating cell 1603 to generate gas to propel a specific traveling plunger, e.g., via a separate gas generation chamber, etc., to deliver the respective substance. In some embodiments, the content of the multiple chambers may be mixed or combined prior to release.

The ingestible device 1600 may include a dispensing outlet 1607 at one end 1602a of the housing 1601 to direct the dispensable substance 1605 out of the housing. The dispensing outlet 1607 may include an exit valve, a slit or a hole, a jet injection nozzle with a syringe, and/or the like. When the traveling plunger 1604 moves towards the end 1602a of the housing 1601, an internal pressure within the storage reservoir may increase and push the dispensing outlet to be open to let the dispensable substance 1605 be released out of the housing 1601.

In an embodiment, a pressure relief device 1606 may be placed within the housing 1601, e.g., at the end 1602a of the housing 1601.

In some embodiments, the housing 1601 may include small holes (e.g., with a diameter smaller than 2 mm), e.g., on the side of the housing 1601, or at the end 1602a to facilitate loading the dispensable substance into the storage reservoir.

In some embodiments, a feedback control circuit (e.g., a feedback resistor, etc.) may be added to send feedback from the gas generating cell 1603 to the electronic component such that when the internal pressure reaches a threshold level, the electronic component may control the gas generating cell 1603 to turn off gas generation, or to activate other safety mechanism (e.g., feedback-controlled release valve, etc.). For example, an internal pressure sensor may be used to measure the internal pressure within the ingestible device and generate feedback to the feedback control circuit.

FIG. 36 provides an example diagram illustrating aspects of a mechanism for a gas generating cell 1603 configured to generate a gas to dispense a substance, according to some embodiments described herein. As shown in FIG. 36, the gas generating cell 1603 generates a gas 1611 which can propel the dispensable substance 1605 out of the dispensing outlet 1607. A variable resistor 1608 may be connected to a circuit with the gas generating cell 1603 such that the variable resistor 1608 may be used to control an intensity and/or an amount of gas 1611 (e.g., hydrogen) generated by the cell 1603. Specifically, the gas generating cell 1603 may be a battery form factor cell that is capable of generating hydrogen when a resistor is applied. In this way, as the gas generating cell 1603 only needs the use of a resistor only without any active power requirements, the gas generating cell 1603 may be integrated into an ingestible device such as a capsule with limited energy/power available. For example, the gas generating cell 1603 may be compatible with a capsule at a size of 26mm x 13mm or smaller.

In some embodiments, based on the elution rate of gas from the cell, and an internal volume of the ingestible device, it may take time to generate sufficient gas 1611 to deliver the substance 1605, and the time may be 30 seconds or longer. For example, the time to generate a volume of hydrogen equivalent to 500µL of fluid would be approximately 5 minutes. A longer period of time may be needed based upon non-ideal conditions within the ingestible device, such as friction, etc. Thus, given that the production of gas (e.g., hydrogen) may take time, gas generation may need to start prior to the ingestible device arriving at the site of delivery to build pressure up within the device. The ingestible device may then need to know when it is approaching the site of delivery. For example, the device may start producing gas on an "entry transition," which is determined by temperature, so as to produce enough gas to be close to the pressure high enough to deliver the dispensable substance. The ingestible device may then only start producing gas again when it arrives at the site of delivery, which will cause the internal pressure within the ingestible device to reach a level required by the dispensing outlet to release the dispensable substance. Also, for regio-specific delivery, the ingestible device may estimate the time it takes to build up enough pressure to deliver the dispensable substance before the ingestible device arrives at a specific location, to activate gas generation.

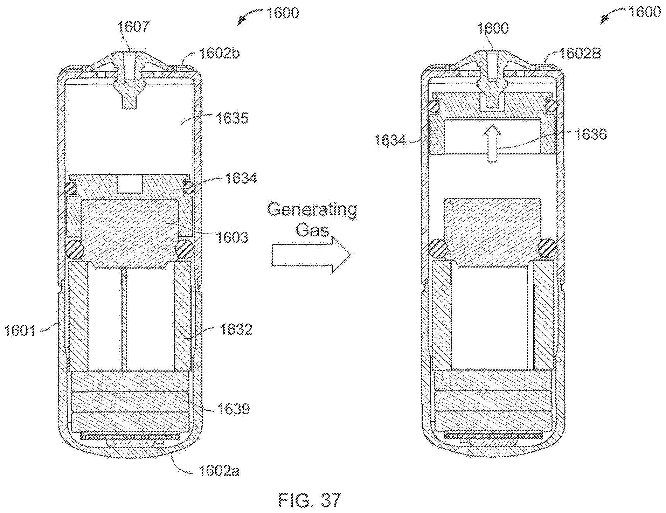

FIGs. 37-39 illustrate an example of an ingestible device for localized delivery of a dispensable substance. The ingestible device 1600 includes a piston or drive element 1634 to push for substance delivery, in accordance with particular implementations described herein. The ingestible device 1600 may have one or more batteries 1639 placed at one end 1602a of a housing 1601 to provide power for the ingestible device 1600. A printed circuit board (PCB) 1632 may be placed adjacent to a battery or other power source 1639, and a gas generating cell 1603 may be mounted on or above the PCB 1632. The gas generating cell 1603 may be sealed from the bottom chamber (e.g., space including 1639 and 1632) of the ingestible device 1600. A movable piston 1634 may be placed adjacent to the gas generating cell 1603. In this way, gas generation from the gas generating cell 1603 may propel a piston 1634 to move towards another end 1602b of the housing 1601 such that the dispensable substance in a reservoir compartment 1635 can be pushed out of the housing through a dispensing outlet 1607, e.g., the movement is shown at 1636, with the piston 1634 at a position after dispensing the substance. The dispensing outlet 1607 may include a plug. The reservoir compartment 1635 can store the dispensable substance, or alternatively the reservoir compartment can house a storage reservoir 1661 which includes the dispensable substance. The reservoir compartment 1635 or storage reservoir 1661 may have a volume of approximately 600µL or even more dispensable substance, which may be dispensed in a single bolus, or gradually over a period of time.

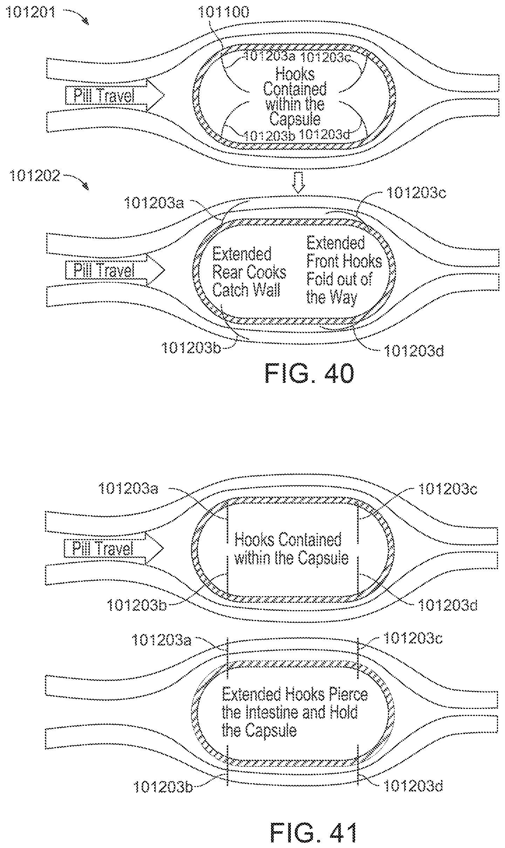

FIGs. 40-42 provide example structural diagrams illustrating aspects of anchoring mechanisms of an ingestible device to anchor the ingestible device to the intestine for dispensable substance delivery. As shown in FIG. 40, the ingestible device 101100 can be anchored within the intestine by extending hooks 101203a-d from the ingestible device 101100 after it has entered the region of interest. At 101201, as the ingestible device 101100 travels along the GI tract, the hooks 101203a-d are contained within the ingestible device. At 101202, when the ingestible device 101100 determines it has arrived at a location within the GI tract, the hooks 101203a-d can be actuated to extend outside of the ingestible device 101100 to catch in the intestinal wall and hold the ingestible device 101100 in the respective location. The hooks 101203a-d can be oriented to catch the intestinal wall regardless of the instant orientation of the ingestible device 101100. The hooks 101203a-d can also retract, dissolve, or detach from the intestinal wall after the dispensable substance has been delivered at the anchored location.

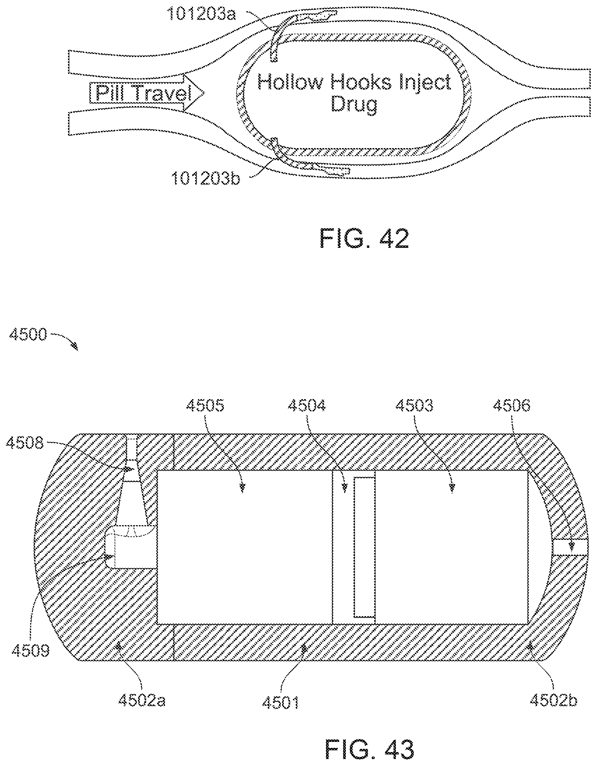

As shown in FIG. 41, the hooks 101203a-d could also extend radially from the ingestible device, and pierce into the intestinal wall to hold the ingestible device 101100 in place. As shown in FIG. 42, if the extending hooks (e.g., 101203a-b) are hollow, the hooks can be used to both anchor the ingestible device and inject the dispensable substance into the intestinal wall.

FIG. 43 illustrates an ingestible device 4500 including a pre-pressurized actuator chamber 4503 and a sliding piston 4504, according to some embodiments described herein.

Ingestible device 4500 includes a device housing 4501. The device housing 4501 is composed of a cap portion 4502a and a base portion 4502b in the illustrated embodiments. Ingestible device 4500 also includes a pre-pressurized actuator chamber 4503 that is pressurized to a target pressure, for example during manufacture or via air fill port 4506 prior to ingestion. The capsule incorporates an active release mechanism that activates as the capsule reaches the target location. As the release mechanism activates, sliding piston 4504 will rapidly move to the left, pushing a high pressure jet of dispensable substance through the nozzle.

Depending on the material used to form the walls of the device housing 4501, the material could diffuse the compressed gas in the pre-pressurized actuator chamber 4503 over time, decreasing the internal pressure. To ensure that pressure is maintained in the ingestible device 4500 over a period between fabrication and patient use, packaging could be pressurized to equal the internal pressure of the pill in certain embodiments; therefore, preventing the permeation of compressed gas from the ingestible device 4500. Assuming the gas expansion within the capsule occurs very fast and an adiabatic polytropic process takes place, gas laws are used to correlate the initial and final pressure of the gas with its volume change ratio.

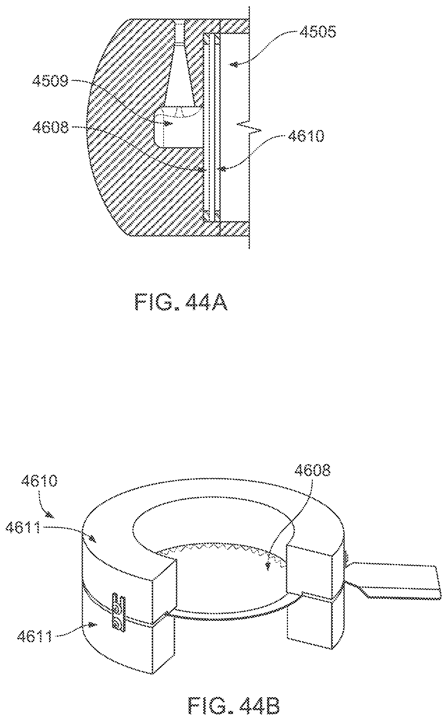

FIG. 44A illustrates a burst disc 4608 with an in line nozzle 4509. FIG. 44B illustrates a partial sectional view of a burst disc holder 4610, according to some embodiments described herein. A burst disc 4608 may enable the release of a dispensable substance, (for example from reservoir 4505) by purposefully fracturing at a targeted pressure allowing the dispensable substance to exit a nozzle 4509 to a target location within the GI tract. A burst disc 4608 can be used as the sole occlusion component in certain embodiments and can be used to provide isolation between upstream contamination and the dispensable substance payload in embodiments including another occlusion component. The burst disc 4608 can be held in place via clamped outer rings 4611 of disc holder 4610 as demonstrated in FIG. 44B.

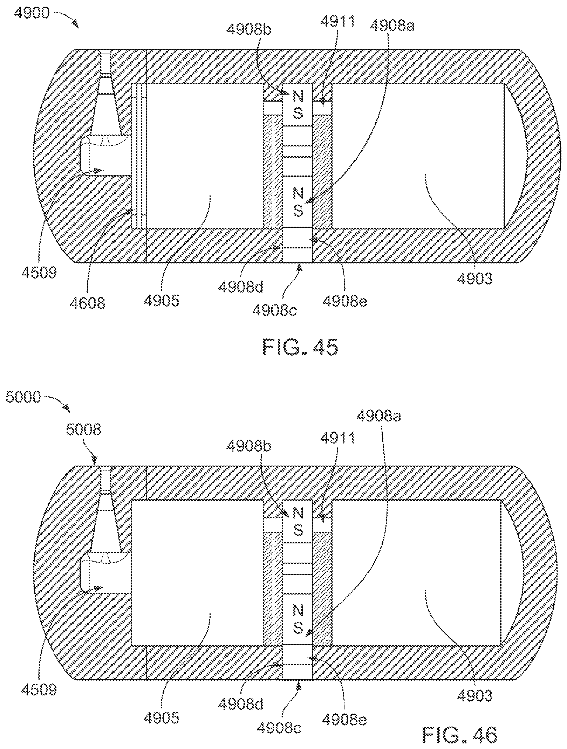

FIG. 45 illustrates an ingestible device 4900 including a magnetic occlusion component 4908b, a burst disc 4608, and a pre-pressurized actuator chamber 4903, according to some embodiments described herein. FIG. 46 illustrates an ingestible device 5000 including a magnetic occlusion component, a pre-pressurized actuator chamber 4903 and a bioabsorbable plug 5008, according to some embodiments described herein. A magnetic stack, which upon peristaltic or osmotic pressure application releases pneumatic pressure, allowing for the delivery of a jet of dispensable substance through a conduit 4509. Osmotic pressure may be used to reconfigure the occlusion component that includes magnets 4908a and 4908b. The enteric coating 4908c dissolves when exposed to luminal fluid, exposing the membrane 4908d and osmogen 4908e. The membrane 4908d and osmogen 4908e facilitate the movement of liquid to create osmotic pressure on the magnet 4908a. As the osmotic pressure builds up, magnet 4908a will be pushed up in proximity to magnet 4908b. Magnet 4908b will be pulled down providing a flow through path for a gas from pressurized chamber 4905 to interact with the reservoir 4905 via connecting conduit 4911. The advantage of this system is that the mechanism may be completely sealed from the exterior of the capsule, allowing for pressure to only project into the chamber 4905. Note that an enteric coating/membrane stack 4908c, 4908d could be replaced by a method of leveraging peristalsis for pushing magnet 4908a. FIG. 45 is implemented with a burst disc 4608 as the sealing/release mechanism once the chamber 4905 is exposed to the pressurized chamber 4903. FIG. 46 is implemented with a bioabsorbable plug 5008 (e.g. enteric coating) that is dissolved and expelled once the reservoir 4905 is exposed to the pressurized actuator chamber 4903.

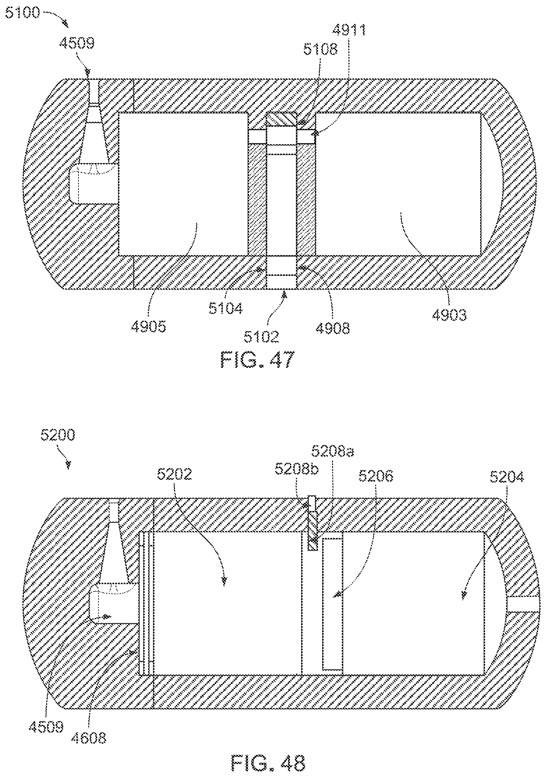

FIG. 47 illustrates an ingestible device 5100 including enteric sliding occlusion component 5102, a pre-pressurized actuator chamber 4903 and a sliding component 5108, according to some embodiments described herein. An osmotic drive 4908, including an enteric coating 5102 and semipermeable membrane 5104, is configured to move a sliding component 5108. The sliding component 5108, once pushed by the osmotic drive 4908, will allow a flow-through port 4911 to connect the pressurized actuator chamber 4903 to the reservoir 4905, providing dispensable substance delivery through the nozzle 5108.

FIG. 48 illustrates an ingestible device 5200 including dissolvable pin occlusion component, a chamber 5202, a pre-pressurized chamber 5204 and a sliding piston 5206, according to some embodiments described herein. In another embodiment, an enteric coating 5208b is dissolved, exposing a structural pin 5208a (such as a glucose spike or hydrogel) that dissolves in the presence of intestinal luminal fluid. With this design, as long as the pin 5208a is in place, the force exerted on the piston 5206 and the chamber 5202 is not large enough for the burst disk 4608 to rupture. The enteric coating 5208b and pin 5208a will dissolve as the capsule 5200 is ingested and as a result, the pressure force on the piston 5206 will increase. The full force of the pre-pressurized chamber 5204 translated onto the chamber 5202 via the piston 5206 is large enough to rupture the burst disk 4608. The rupture of the burst disk 4608 results in a pressurized jet of liquid being delivered from the chamber 5202 through the nozzle 4509.

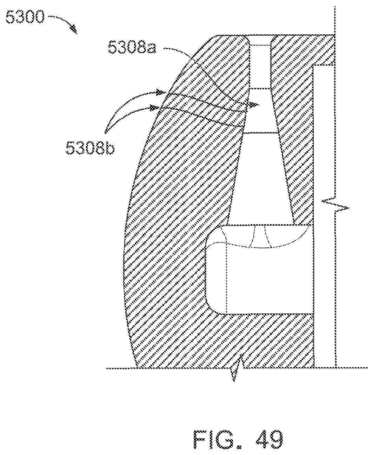

FIG. 49 illustrates an ingestible device 5300 including wax plug 5308a with wire lead activators 5308b, according to some embodiments described herein. In this method, the dispensing site is identified based on collected reflected light. The reflectance of light in green and red spectrums (with iterations to this methodology and algorithm actively being pursued) are measured and an algorithm is used to correlate the measured reflectance with the location in the Gastrointestinal (GI) tract. This method provides a non-pH based system to determine the anatomical locations of the capsule during fasted transit. As the capsule 5300 reaches the target location, a signal is generated which will be used to activate an alternative release mechanism.

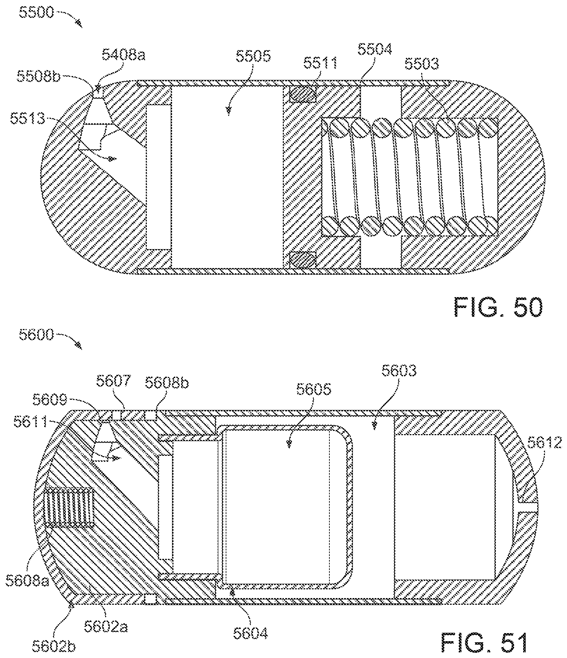

FIG. 50 illustrates an ingestible 5500 device including a spring actuator 5503 and a sliding piston 5504, according to some embodiments described herein. Ingestible device 5500 uses the potential energy stored in a spring 5503 when compressed as the driving or actuating mechanism for jet delivery of the dispensable substance. The occlusion component or release mechanism consists of bioabsorbable plug 5508a separated from the reservoir 5505 by a protectant layer 5508b. In this embodiment, the inner volume of the capsule 5500 is divided into two sections separated by a sliding piston 5504. The left section (e.g., reservoir 5505) is filled with dispensable substance and a spring 5503 is mounted in the right section. The piston 5504 can freely move to the right or left depending on the net force exerted on the piston 5504. An O-ring 5511 is used to provide the sealing desired between the two sections, with alternative sealing means possible. Compressed spring 5503 applies a force on the piston 5504 and the piston 5504 transfers this force to the liquid dispensable substance in form of pressure. The same pressure will be transferred to the plug 5508a sealing the nozzle 5513. However, this pressure acts on a small area (area of the plug 5508a). Therefore, the large force exerted by the spring 5503 translates into a small force on the sealing plug 5508a. As the capsule 5500 is digested, it moves through GI tract and the bioabsorbable sealing plug 5508a will start dissolving. After certain amount of time, the plug will weaken or fully dissolve in GI fluid. As soon as the plug 5508a weakens to the design threshold, the pressure inside the reservoir 5503 drops, the spring 5503 will expand delivering dispensable substance (e.g., in the form of a highpressure jet of fluid) through the opening.

FIG. 51 illustrates an ingestible device 5600 including a spring actuated slidable housing portion 5602b, according to some embodiments described herein. Ingestible device 5600 consists of a pressurized actuator 5603 chamber, a reservoir 5605 separated from the pressure actuator chamber 5603 by a deformable body 5604 such as bellows and a spring/enteric coating release mechanism The spring 5608a is mounted on the polycarbonate cap 5602a from one end and to a sliding cap 5602b on the other end. The stainless steel top slider 5602b can slide to the left and right opening and closing the nozzle 5611. An enteric ring 5608b is used to keep the top slider closed. An O-ring and a bioabsorbable plug 5609 are used to provide the desired sealing. An adhesive seal 5612 is located on the housing, on the opposite end of the capsule 5600 from the spring 5608a. Compressed gas applies a force on the bellows 5604 and the bellows 5604 transfer this force to the liquid dispensable substance in form of pressure. The same pressure will be transferred to the slider 5602b in form of a radial force. However, this pressure acts on a small area (area of the exit orifice 5607). Therefore, the transverse load on the slider 5602b is relatively small. When the capsule 5600 is assembled, the spring 5608a is compressed (slider 5602b in closed mode), and the enteric coating 5608b keeps the slider 5602b in position. As the capsule 5600 is digested, it moves through GI tract. The enteric coating 5608b will dissolve when the capsule 5600 passes through the intestinal fluid. With the dissolution of the enteric coating 5608b, the spring 5608a will push the slider 5602b back away from the capsule 5600 (open mode). As a result, the exit orifice 5607 becomes concentric with the nozzle 5611 and the jet of fluid will be released.

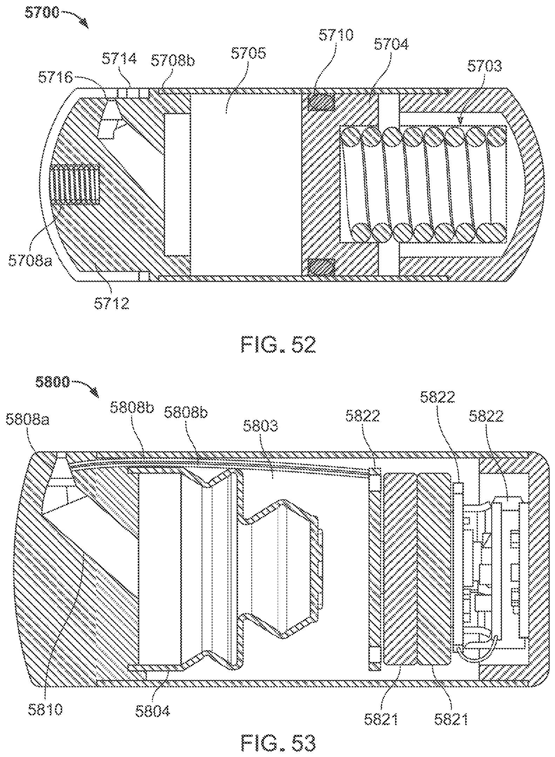

FIG. 52 illustrates an ingestible device 5700 with another spring actuated slidable housing portion 5712, according to some embodiments described herein. Ingestible device 5700 uses a compressed spring (spring 5703) as the drive mechanism and a compressed spring 5708a (spring with sliding top cap 5712 as the release mechanism. A piston 5704 separates the reservoir 5705 from the spring chamber and an enteric coating 5708b is used to initiate the release mechanism. An O-ring 5710 is used to provide sealing between the piston 5704 and cylinder. Compressed spring 5703 applies a force on the piston 5704 and the piston 5704 transfers this force to the liquid dispensable substance in the form of pressure. The same pressure will be transferred to the top cap slider 5712 in form of a radial force. However, this pressure acts on a small area (area of the exit orifice 5714) resulting in a small transverse force on the top slider 5712. When the capsule 5700 is assembled, spring 5703 is left in compressed mode (slider 5712 in closed position). As the capsule 5700 is digested, it moves through GI tract. The enteric coating 5708b will dissolve when the capsule 5700 passes through the intestinal fluid. With the dissolution of the enteric coating 5708b, the spring 5708a will push the slider 5712 back away from the capsule 5700 (open mode). As a result, the exit orifice 5714 becomes concentric with the nozzle 5716 and the jet of fluid will be released.

FIG. 53 illustrates an ingestible device 5800 including a melt away occlusion component 5808a and a pressurized chamber 5803, according to some embodiments described herein. Ingestible device 5800 consists of two chambers, one chamber is filled with dispensable substance and the other chamber is filled with pressurized gas. A wax valve 5808a actuated by localization board 5822 is used as the occlusion component. A large section of the pressure chamber 5803 is occupied by the release mechanism and the batteries 5821. Wax valve wires 5808b are connected to the wax valve 5808a and will melt the wax using an electric current. The timing of this operation is controlled by the localization board 5822. In this embodiment, a fully controlled release mechanism is used. As the capsule 5800 reaches target area, the localization kit will activate and direct a predetermined electric current toward the wax valve 5808a. A heating element will receive this current and will melt or weaken the wax valve 5808a. With weakening or removal of the wax from the nozzle 5810, gas pressure from the pressurized chamber 5803 will push the bellows 5804 resulting in a pressurized jet of liquid dispensable substance exiting the nozzle 5810, thus delivering the dispensable substance.

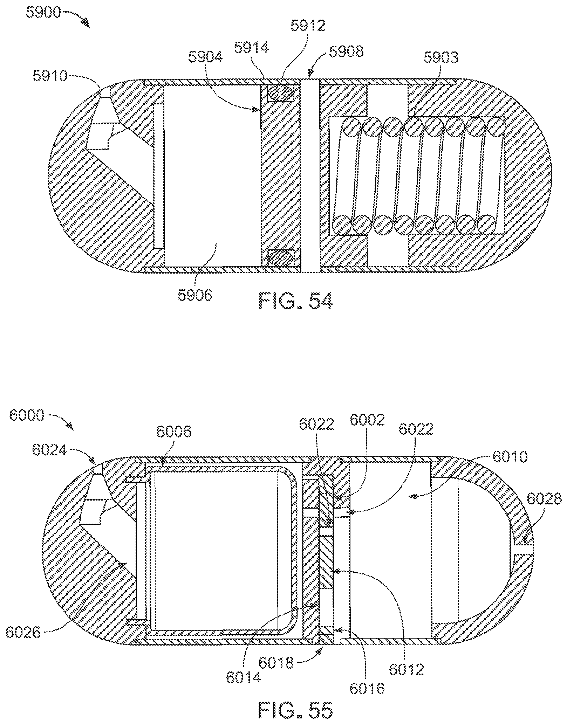

FIG. 54 illustrates an ingestible device 5900 including a dissolvable pin occlusion component 5908 and a spring actuated sliding piston 5914, according to some embodiments described herein. One of the main challenges of designing an effective capsule is the sealing between the two chambers inside the capsule since there is a significant pressure difference between the two chambers, the dispensable substance tends to move from the dispensable substance chamber into the pressure or spring chamber. Certain embodiments address this by reducing the pressure difference between the two chambers during the shelf life and before jet delivery. For example, ingestible device 5900 includes a compressed spring 5903 is retained using a dissolvable pin 5908. Additionally, an O-ring 5912 is used to provide sealing between the piston 5914 and housing. With this design, as long as the pin 5908 is in place, there is no force exerted on the piston 5904 and the liquid in chamber 5906. The force exerted by the spring 5903 will result in shear stress on the pin 5908. The pin 5908 will dissolve as the capsule 5900 is ingested and as a result, the spring force will translate into a pressurized jet of liquid. An enteric coating on the ends of the pin 5908 could further enhance the specificity of the triggering location. During the shelf life and before ingestion of the capsule 5900, there is not a significant amount of pressure acting on the dispensable substance and consequently, sealing challenges are easier to address. With a 200-psi design pressure, the pin would be expected to hold approximately 20 lbf, and would involve design consideration to the shear strength of the dissolvable pin. As the capsule 5900 passes through the GI tract, the pin 5908 will start dissolving. As the pin 5908 dissolves, there is no support for the piston 5904 to keep the piston 5904 in place. The force of the spring 5903 will result in a significant pressure in the fluid. At a certain point the pin 5908 will fail and the piston 5904 will move to the left releasing a highpressure jet of fluid through the nozzle 5910.

FIG. 55 illustrates an ingestible device 6000 including shuttle slider occlusion component 6012 and a pressurized chamber 6010, according to some embodiments described herein. Ingestible device 6000 includes two chambers separated by a wall 6002 made of polycarbonate. The right chamber is an adhesive seal 6028 and a pressurized chamber 6010, pressurized with gas, and a bellows 6006 is installed in the left chamber. There are no openings connecting the two chambers 6006, 6010. An osmotic release mechanism is used to connect the two chambers 6006, 6010 through a sliding valve 6012. Osmogen 6014 is contained within a small container below the sliding valve 6012. Osmogen 6014 is separated from the GI fluid by a water permeable membrane 6016 covered with enteric coating 6018. On the top of the osmogen 6014, a shuttle slider 6012 is mounted. The slider 6012 has an opening 6020 in the middle. The slider shuttle 6012 is sandwiched between two slabs of polycarbonate with a pressure through port 6022. When the slider shuttle 6012 is in closed form, the holes on the polycarbonate slabs are not concentric with the hole on the slider shuttle 6012. When the slider shuttle 6012 is in open mode, the holes of the slider and polycarbonate slabs surrounding it all will be concentric letting gas and pressure exchange between the two chambers 6006, 6010.

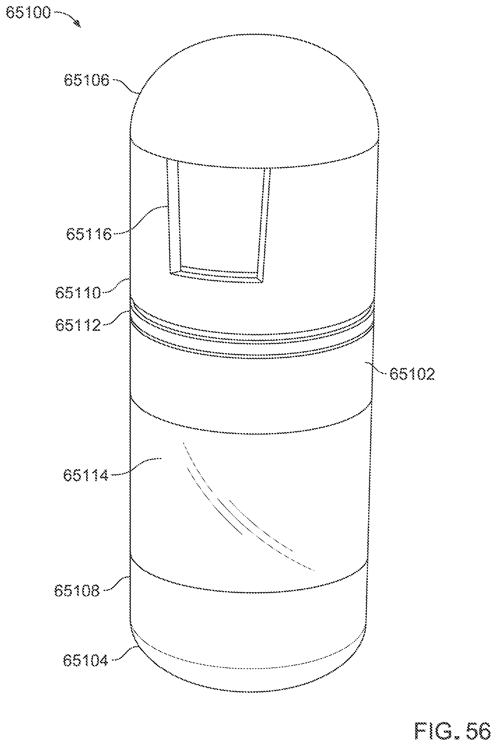

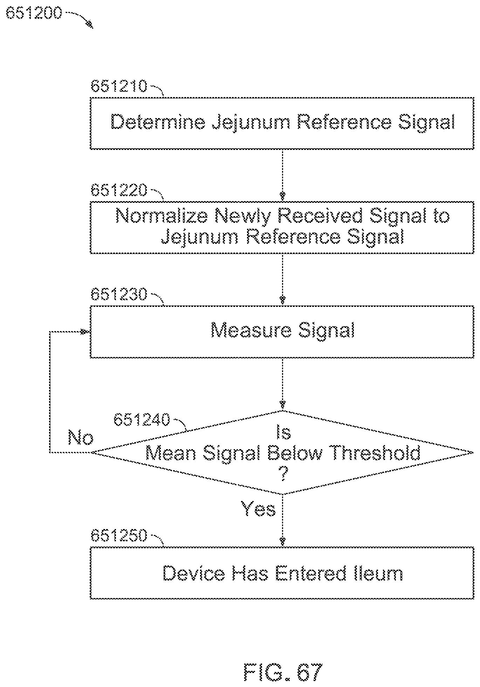

In certain embodiments, an ingestible device is configured to determine its location (e.g., within the GI tract of a subject). FIGs. 56-70 provide illustrative and non-limiting examples of such ingestible devices and associated methods. It is to be understood that one more features from such embodiments can be combined with one or more features of an ingestible device configured to take one more samples, such as, for example, described above with regarding to FIGs. 1-34, and/or with one or more features of an ingestible device configured to deliver one or more substances (e.g., one or more therapeutic substances), such as, for example, described above with respect to FIGs. 35-55.

In some embodiments, the location of the ingestible device within the GI tract of the subject can be determined to an accuracy of at least about 85%, e.g., at least about 90%, at least about 95%, at least about 97%, at least about 98%, at least about 99%, or about 100%. In such embodiments, the portion of the portion of the GI tract of the subject can include, for example, the esophagus, the stomach, duodenum, the jejunum, and/or the terminal ileum, cecum and colon.

In certain embodiments, the location of the ingestible device within the esophagus of the subject can be determined to an accuracy of at least about 85%, e.g., at least about 90%, at least about 95%, at least about 97%, at least about 98%, at least about 99%, or about 100%.

In some embodiments, the location of the ingestible device within the stomach of the subject can be determined to an accuracy of at least about 85%, e.g., at least about 90%, at least about 95%, at least about 97%, at least about 98%, at least about 99%, or about 100%.

In certain embodiments, the location of the ingestible device within the duodenum of the subject can be determined to an accuracy of at least about 85%, e.g., at least about 90%, at least about 95%, at least about 97%, at least about 98%, at least about 99%, or about 100%.

In some embodiments, the location of the ingestible device within the jejunum of the subject can be determined to an accuracy of at least about 85%, e.g., at least about 90%, at least about 95%, at least about 97%, at least about 98%, at least about 99%, or about 100%.

In certain embodiments, the location of the ingestible device within the terminal ileum, cecum and colon of the subject can be determined to an accuracy of at least about 85%, e.g., at least about 90%, at least about 95%, at least about 97%, at least about 98%, at least about 99%, or about 100%.

In some embodiments, the location of the ingestible device within the cecum of the subject can be determined to an accuracy of at least about 85%, e.g., at least about 90%, at least about 95%, at least about 97%, at least about 98%, at least about 99%, or about 100%.

As used herein, the term "reflectance" refers to a value derived from light emitted by the device, reflected back to the device, and received by a detector in or on the device. For example, in some embodiments this refers to light emitted by the device, wherein a portion of the light is reflected by a surface external to the device, and the light is received by a detector located in or on the device.

As used herein, the term "illumination" refers to any electromagnetic emission. In some embodiments, an illumination may be within the range of infrared light (IR), the visible spectrum and ultraviolet light (UV), and an illumination may have a majority of its power centered at a particular wavelength in the range of 100 nm to 1000 nm. In some embodiments, it may be advantageous to use an illumination with a majority of its power limited to one of the infrared (750 nm-1000 nm), red (600 nm-750 nm), green (495 nm-600 nm), blue (400 nm-495 nm), or ultraviolet (100 nm-400 nm) spectrums. In some embodiments a plurality of illuminations with different wavelengths may be used. For illustrative purposes, the embodiments described herein may refer to the use of green or blue spectrums of light. However, it is understood that these embodiments may use any suitable light having a wavelength that is substantially or approximately within the green or blue spectra defined above, and the localization systems and methods described herein may use any suitable spectra of light.

Referring now to FIG. 56, shown therein is a view of an example embodiment of an ingestible device 65100, which may be used to identify a location within a gastrointestinal (GI) tract. It is to be understood that certain details regarding the design of ingestible device 65100 are not shown in FIG. 56 and the following figures, and that, in general, various aspect of ingestible devices described elsewhere herein can be implemented in ingestible device 65100 and the ingestible devices shown in the following figures.

In some embodiments, ingestible device 65100 may be configured to autonomously determine whether it is located in the stomach, a particular portion of the small intestine such as a duodenum, jejunum, or ileum, or the large intestine by utilizing sensors operating with different wavelengths of light. Additionally, ingestible device 65100 may be configured to autonomously determine whether it is located within certain portions of the small intestine or large intestine, such as the duodenum, the jejunum, the cecum, or the colon.

Ingestible device 65100 may have a housing 65102 shaped similar to a pill or capsule. The housing 65102 of ingestible device 65100 may have a first end portion 65104, and a second end portion 65106. The first end portion 65104 may include a first wall portion 65108, and second end portion 65106 may include a second wall portion 65110. In some embodiments, first end portion 65104 and second end portion 65106 of ingestible device 65100 may be manufactured separately, and may be affixed together by a connecting portion 65112.

In some embodiments, ingestible device 65100 may include an optically transparent window 65114. Optically transparent window 65114 may be transparent to various types of illumination in the visible spectrum, infrared spectrum, or ultraviolet light spectrum, and ingestible device 65100 may have various sensors and illuminators located within the housing 65102, and behind the transparent window 65114. This may allow ingestible device 65100 to be configured to transmit illumination at different wavelengths through transparent window 65114 to an environment external to housing 65102 of ingestible device 65100, and to detect a reflectance from a portion of the illumination that is reflected back through transparent window 65114 from the environment external to housing 65102. Ingestible device 65100 may then use the detected level of reflectance in order to determine a location of ingestible device 65100 within a GI tract. In some embodiments, optically transparent window 65114 may be of any shape and size, and may wrap around the circumference of ingestible device 65100. In this case, ingestible device 65100 may have multiple sets of sensors and illuminators positioned at different locations azimuthally behind window 65114.

In some embodiments, ingestible device 65100 may optionally include an opening 65116 in the second wall portion 65110. In some embodiments, the second wall portion 65110 may be configured to rotate around the longitudinal axis of ingestible device 65100 (e.g., via a suitable motor or other actuator housed within ingestible device 65100). This may allow ingestible device 65100 to obtain a fluid sample from the GI tract, or release a substance into the GI tract, through opening 65116.

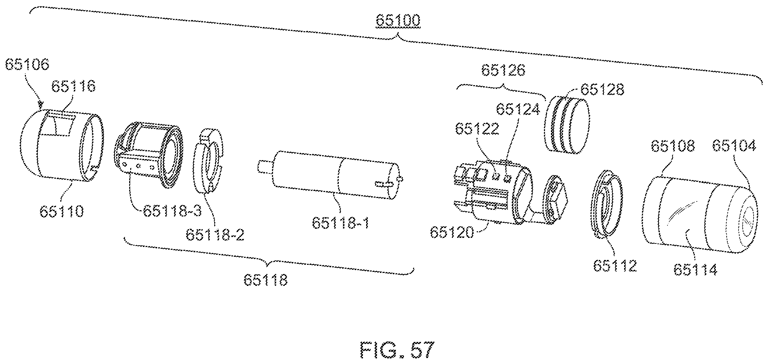

FIG. 57 shows an exploded view of ingestible device 65100. In some embodiments, ingestible device 65100 may optionally include a rotation assembly 65118. Optional rotation assembly 65118 may include a motor 65118-1 driven by a microcontroller (e.g., a microcontroller coupled to printed circuit board 65120), a rotation position sensing ring 65118-2, and a storage sub-unit 65118-3 configured to fit snugly within the second end portion 65104. In some embodiments, rotation assembly 65118 may cause second end portion 65104, and opening 65116, to rotate relative to the storage sub-unit 65118-3. In some embodiments, there may be cavities on the side of storage sub-unit 65118-3 that function as storage chambers. When the opening 65116 is aligned with a cavity on the side of the storage sub-unit 65118-3, the cavity on the side of the storage sub-unit 65118-3 may be exposed to the environment external to the housing 65102 of ingestible device 65100. In some embodiments, the storage sub-unit 65118-3 may be loaded with a medicament or other substance prior to the ingestible device 65100 being administered to a subject. In this case, the medicament or other substance may be released from the ingestible device 65100 by aligning opening 65116 with the cavity within storage sub-unit 65118-3. In some embodiments, the storage sub-unit 65118-3 may be configured to hold a fluid sample obtained from the GI tract. For example, ingestible device 65100 may be configured to align opening 65116 with the cavity within storage sub-unit 65118-3, thus allowing a fluid sample from the GI tract to enter the cavity within storage sub-unit 65118-3. Afterwards, ingestible device 65100 may be configured to seal the fluid sample within storage sub-unit 65118-3 by further rotating the second end portion 65106 relative to storage sub-unit 65118-3. In some embodiments, storage sub-unit 118-3 may also contain a hydrophilic sponge, which may enable ingestible device 65100 to better draw certain types of fluid samples into ingestible device 65100. In some embodiments, ingestible device 65100 may be configured to either obtain a sample from within the GI tract, or to release a substance into the GI tract, in response to determining that ingestible device 65100 has reached a predetermined location within the GI tract. For example, ingestible device 65100 may be configured to obtain a fluid sample from the GI tract in response to determining that the ingestible device has entered the jejunum portion of the small intestine (e.g., as determined by process 65900 discussed elsewhere herein). It is understood that any suitable method of obtaining samples or releasing substances may be incorporated into some of the embodiments of the ingestible devices disclosed herein, and that the systems and methods for determining a location of an ingestible device may be incorporated into any suitable type of ingestible device.

Ingestible device 65100 may include a printed circuit board (PCB) 65120, and a battery 65128 configured to power PCB 65120. PCB 65120 may include a programmable microcontroller, and control and memory circuitry for holding and executing firmware or software for coordinating the operation of ingestible device 65100, and the various components of ingestible device 65100. For example, PCB 65120 may include memory circuitry for storing data, such as data sets of measurements collected by sensing sub-unit 65126, or instructions to be executed by control circuitry to implement a localization process, such as, for example, one or more of the processes, discussed herein, including those discussed below in connection with one or more of the associated flow charts. PCB 65120 may include a detector 65122 and an illuminator 65124, which together form sensing sub-unit 65126. In some embodiments, control circuitry within PCB 65120 may include processing units, communication circuitry, or any other suitable type of circuitry for operating ingestible device 65100. For illustrative purposes, only a single detector 65122 and a single illuminator 65124 forming a single sensing sub-unit 65126 are shown. However, it is understood that in some embodiments there may be multiple sensing sub-units, each with a separate illuminator and detector, within ingestible device 65100. For example, there may be several sensing sub-units spaced azimuthally around the circumference of the PCB 65120, which may enable ingestible device 65100 to transmit illumination and detect reflectances or ambient light in all directions around the circumference of the device. In some embodiments, sensing sub-unit 65126 may be configured to generate an illumination using illuminator 65124, which is directed through the window 65114 in a radial direction away from ingestible device 65100. This illumination may reflect off of the environment external to ingestible device 65100, and the reflected light coming back into ingestible device 65100 through window 65114 may be detected as a reflectance by detector 65122.

In some embodiments, window 65114 may be of any suitable shape and size. For example, window 65114 may extend around a full circumference of ingestible device 65100. In some embodiments there may be a plurality of sensing sub-units (e.g., similar to sensing sub-unit 65126) located at different positions behind the window. For example, three sensing sub-units may be positioned behind the window at the same longitudinal location, but spaced 120 degrees apart azimuthally. This may enable ingestible device 65100 to transmit illuminations in all directions radially around ingestible device 65100, and to measure each of the corresponding reflectances.

In some embodiments, illuminator 65124 may be capable of producing illumination at a variety of different wavelengths in the ultraviolet, infrared, or visible spectrum. For example, illuminator 65124 may be implemented by using Red-Green-Blue Light-Emitting diode packages (RGB-LED). These types of RGB-LED packages are able to transmit red, blue, or green illumination, or combinations of red, blue, or green illumination. Similarly, detector 65122 may be configured to sense reflected light of the same wavelengths as the illumination produced by illuminator 65124. For example, if illuminator 65124 is configured to produce red, blue, or green illumination, detector 65122 may be configured to detect different reflectances produced by red, blue, or green illumination (e.g., through the use of an appropriately configured photodiode). These detected reflectances may be stored by ingestible device 65100 (e.g., within memory circuitry of PCB 65120 (FIG. 57)), and may then be used by ingestible device 65100 in determining a location of ingestible device 65100 within the GI tract (e.g., through the use of one or more processes described herein).

It is understood that ingestible device 65100 is intended to be illustrative, and not limiting. It will be understood that modifications to the general shape and structure of the various devices and mechanisms described in relation to FIG. 56 and FIG. 57 may be made without significantly changing the functions and operations of the devices and mechanisms. For example, ingestible device 65100 may have a housing formed from a single piece of molded plastic, rather than being divided into a first end portion 65104 and a second end portion 65106. As an alternate example, the location of window 65114 within ingestible device 65100 may be moved to some other location, such as the center of ingestible device 65100, or to one of the ends of ingestible device 65100. Moreover, the systems and methods discussed in relation to FIGs. 56-70 may be implemented on any suitable type of ingestible device, provided that the ingestible device is capable of detecting reflectances or levels of illumination in some capacity. For example, in some embodiments ingestible device 65100 may be modified to replace detector 65122 with an image sensor, and the ingestible device may be configured to measure relative levels of red, blue, or green light by decomposing a recorded image into its individual spectral components. It should be noted that the features and limitations described in any one embodiment may be applied to any other embodiment herein, and the descriptions and examples relating to one embodiment may be combined with any other embodiment in a suitable manner.

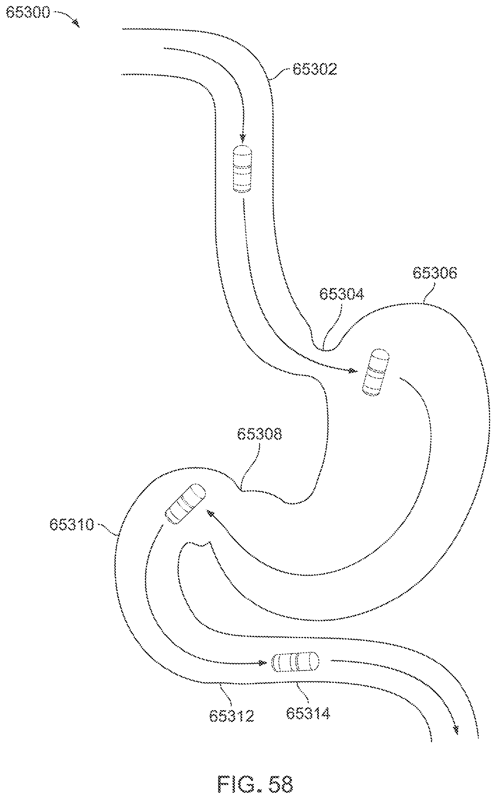

FIG. 58 is a diagram of an ingestible device during an example transit through a gastrointestinal (GI) tract, in accordance with some embodiments of the disclosure. The ingestible device may include any portion of any other ingestible device discussed in this disclosure, and may be any suitable type of ingestible device with localization capabilities. For example, the ingestible device may be without an optional opening for sampling or optional rotation assembly for sampling. In some embodiments, the ingestible device may be ingested by a subject, and as the ingestible device traverses the GI tract, the ingestible device determines its location within the GI tract. For example, the movement of the ingestible device and the amount of light detected by the ingestible device (e.g., via a detector as described elsewhere herein) may vary substantially depending on the location of the ingestible device within the GI tract, and the ingestible device may be configured to use this information to determine a location of the ingestible device within the GI tract. For instance, the ingestible device may detect ambient light from the surrounding environment, or reflectances based on illumination generated by the ingestible device (e.g., generated by an illuminator as described elsewhere herein), and use this information to determine a location of the ingestible device through processes, such as described herein. The current location of the ingestible device, and the time that the ingestible device detected each transition between the various portions of the GI tract, may then be stored by the ingestible device (e.g., in memory circuitry of a PCB as described elsewhere herein), and may be used for any suitable purpose.

Shortly after the ingestible device is ingested, the ingestible device will traverse the esophagus 65302, which may connect the subject's mouth to a stomach 65306. In some embodiments, the ingestible device may be configured to determine that it has entered the esophagus portion GI tract by measuring the amount and type of light (e.g., via a detector as described elsewhere herein) in the environment surrounding the ingestible device. For instance, the ingestible device may detect higher levels of light in the visible spectrum (e.g., via a detector as described elsewhere herein) while outside the subject's body, as compared to the levels of light detected while within the GI tract. In some embodiments, the ingestible device may have previously stored data (e.g., on memory circuitry of a PCB as described elsewhere herein) indicating a typical level of light detected when outside of the body, and the ingestible device may be configured to determine that entry to the body has occurred when a detected level of light (e.g., detected via a detector as described elsewhere herein) has been reduced beyond a threshold level (e.g., at least a 20-30% reduction) for a sufficient period of time (e.g., 5.0 seconds).

In some embodiments, the ingestible device may be configured to detect a transition from esophagus 65302 to stomach 65306 by passing through sphincter 65304. In some embodiments, ingestible device 65300 may be configured to determine whether it has entered stomach 65306 based at least in part on a plurality of parameters, such as but not limited to the use of light or temperature measurements (e.g., via a detector as described elsewhere herein or via a thermometer within the ingestible device), pH measurements (e.g., via a pH meter within the ingestible device), time measurements (e.g., as detected through the use of clock circuitry included within a PCB as described elsewhere herein), or any other suitable information. For instance, the ingestible device may be configured to determine that the ingestible device has entered stomach 65306 after detecting that a measured temperature of the ingestible device exceeds 31 degrees Celsius. Additionally, or alternately, the ingestible device may be configured to automatically determine it has entered stomach 65306 after one minute (or another pre-set time duration parameter, 80 seconds, 90 seconds, etc.) has elapsed from the time that the ingestible device was ingested, or one minute (or another pre-set time duration parameter, 80 seconds, 90 seconds, etc.) from the time that the ingestible device detected that it has entered the GI tract.

Stomach 65306 is a relatively large, open, and cavernous organ, and therefore the ingestible device may have a relatively large range of motion. By comparison, the motion of the ingestible device is relatively restricted within the tube-like structure of the duodenum 65310, the jejunum 65314, and the ileum (not shown), all of which collectively form the small intestine. Additionally, the interior of stomach 65306 has distinct optical properties from duodenum 65310 and jejunum 65314, which may enable the ingestible device to detect a transition from stomach 65306 to duodenum 65310 through the appropriate use of measured reflectances (e.g., through the use of reflectances measured by a detector as described elsewhere herein), as used in conjunction with a process 65600).

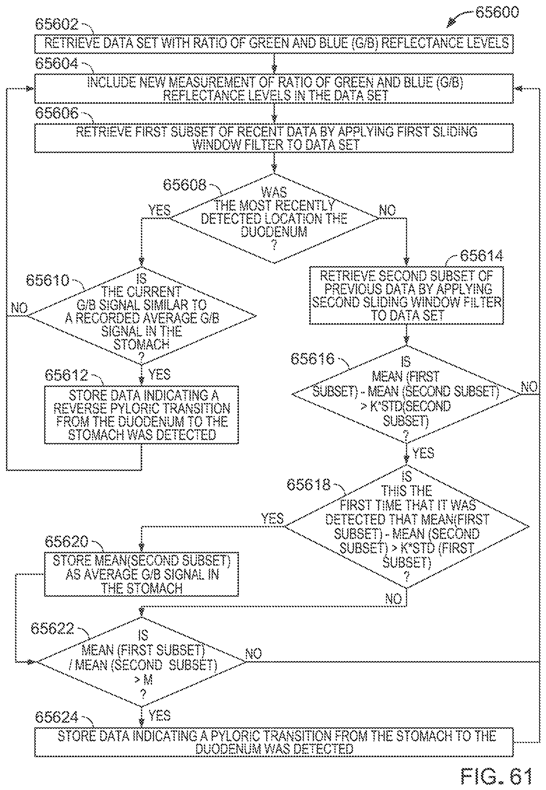

In some embodiments, the ingestible device may be configured to detect a pyloric transition from stomach 65306 to duodenum 65310 through the pylorus 65308. For instance, in some embodiments, the ingestible device may be configured to periodically generate illumination in the green and blue wavelengths (e.g., via an illuminator as described elsewhere herein), and measure the resulting reflectances (e.g., via a detector as described elsewhere herein). The ingestible device may be configured to then use a ratio of the detected green reflectance to the detected blue reflectance to determine whether the ingestible device is located within the stomach 65306, or duodenum 65310 (e.g., via process 65600). In turn, this may enable the ingestible device to detect a pyloric transition from stomach 65306 to duodenum 65310, an example of which is discussed in relation to FIG. 61.

Similarly, in some embodiments, the ingestible device may be configured to detect a reverse pyloric transition from duodenum 65310 to stomach 65306. The ingestible device will typically transition naturally from stomach 65306 to duodenum 65310, and onward to jejunum 65314 and the remainder of the GI tract. However, similar to other ingested substances, the ingestible device may occasionally transition from duodenum 65310 back to stomach 65306 as a result of motion of the subject, or due to the natural behavior of the organs with the GI tract. To accommodate this possibility, the ingestible device may be configured to continue to periodically generate illumination in the green and blue wavelengths (e.g., via an illuminator as described elsewhere herein), and measure the resulting reflectances (e.g., via a detector as described elsewhere herein) to detect whether or not the ingestible device has returned to stomach 65306. An exemplary detection process is described in additional detail in relation to FIG. 61.

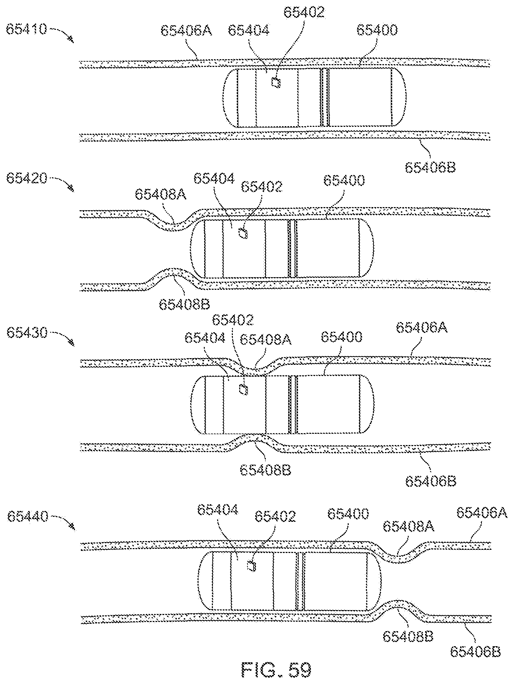

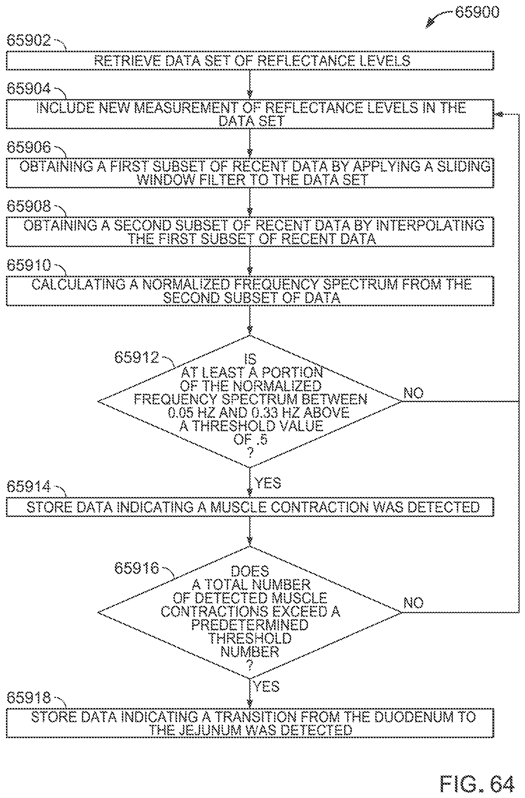

After entering duodenum 65310, the ingestible device may be configured to detect a transition to the jejunum 65314 through the duodenojejunal flexure 65312. For example, the ingestible device may be configured to use reflectances to detect peristaltic waves within the jejunum 65314, caused by the contraction of the smooth muscle tissue lining the walls of the jejunum 65314. In particular, the ingestible device may be configured to begin periodically transmitting illumination (and measuring the resulting reflectances (e.g., via a detector and an illuminator of a sensing sub-unit as described elsewhere herein) at a sufficiently high frequency in order to detect muscle contractions within the jejunum 65314. The ingestible device may then determine that it has entered the jejunum 65314 in response to having detected either a first muscle contraction, or a predetermined number of muscle contractions (e.g., after having detected three muscle contractions in sequence). The interaction of the ingestible device with the walls of jejunum 65314 is also discussed in relation to FIG. 59, and an example of this detection process is described in additional detail in relation to FIG. 64.

FIG. 59 is a diagram of an ingestible device during an example transit through a jejunum, in accordance with some embodiments of the disclosure. Diagrams 65410, 65420, 65430, and 65440 depict ingestible device 65400 as it traverses through a jejunum (e.g., jejunum 65314), and how ingestible device 65400 interacts with peristaltic waves formed by walls 65406A and 65406B (collectively, walls 65406) of the jejunum. In some implementations, ingestible device 65400 may include any portion of any other ingestible device discussed in this disclosure, and may be any suitable type of ingestible device with localization capabilities.

Diagram 65410 depicts ingestible device 65400 within the jejunum, when the walls 65406 of the jejunum are relaxed. In some embodiments, the confined tube-like structure of the jejunum naturally causes ingestible device 65400 to be oriented longitudinally along the length of the jejunum, with window 65404 facing walls 65406. In this orientation, ingestible device 65400 may use sensing sub-unit 65402 to generate illumination (e.g., via an illuminator as described elsewhere herein) oriented towards walls 65406, and to detect the resulting reflectances (e.g., via a detector as described elsewhere herein) from the portion of the illumination reflected off of walls 65406 and back through window 65404. In some embodiments, ingestible device 65400 may be configured to use sensing sub-unit 65402 to generate illumination and measure the resulting reflectance with sufficient frequency to detect peristaltic waves within the jejunum. For instance, in a healthy human subject, peristaltic waves may occur at a rate of approximately 0.05 Hz to 0.33 Hz. Therefore, the ingestible device 65400 may be configured to generate illumination and measure the resulting reflectance at least once every 2.5 seconds (i.e., potentially minimum rate to detect a 0.2 Hz signal), and preferably at a higher rate, such as once every 0.5 seconds, which may improve the overall reliability of the detection process due to more data points being available. It is understood that the ingestible device 65400 need not gather measurements at precise intervals, and in some embodiments the ingestible device 65400 may be adapted to analyze data gathered at more irregular intervals, provided that there are still a sufficient number of appropriately spaced data points to detect 0.05 Hz to 0.33 Hz signals.

Diagram 65420 depicts ingestible device 65400 within the jejunum, when the walls 65406 of the jejunum begin to contract and form a peristaltic wave. Diagram 65420 depicts contracting portion 65408A of wall 65406A and contracting portion 65408B of wall 65406B (collectively, contracting portion 65408 of wall 65406) that form a peristaltic wave within the jejunum. The peristaltic wave proceeds along the length of the jejunum as different portions of wall 65406 contract and relax, causing it to appear as if contracting portions 65408 of wall 65406 proceed along the length of the jejunum (i.e., as depicted by contracting portions 65408 proceeding from left to right in diagrams 65410-65430). While in this position, ingestible device 65400 may detect a similar level of reflectance (e.g., through the use of an illuminator and a detector of a sensing sub-unit as described elsewhere herein) as detected when there is no peristaltic wave occurring (e.g., as detected when ingestible device 65400 is in the position indicated in diagram 65410).

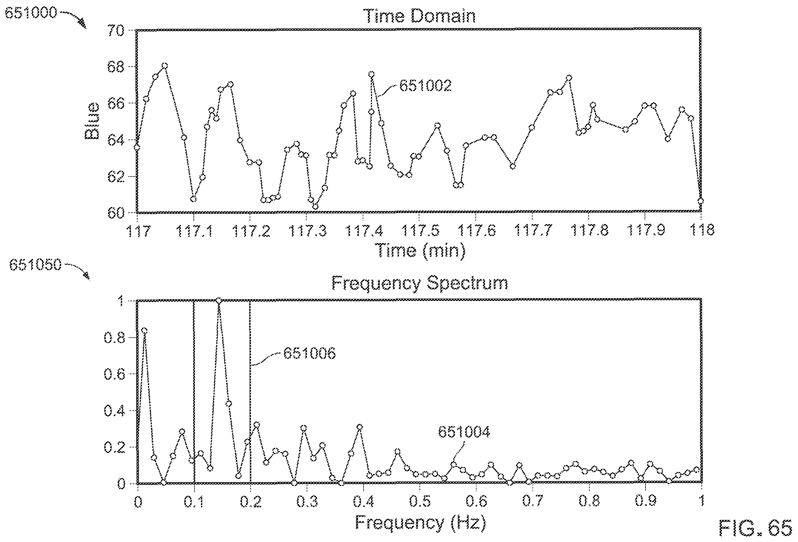

Diagram 65430 depicts ingestible device 65400 within the jejunum, when the walls 65406 of the jejunum continue to contract, squeezing around ingestible device 65400. As the peristaltic wave proceeds along the length of the jejunum, contracting portions 65408 of wall 65406 may squeeze tightly around ingestible device 65400, bringing the inner surface of wall 65406 into contact with window 65404. While in this position, ingestible device 65400 may detect a change in a reflectance detected as a result of illumination produced by sensing sub-unit 65402. The absolute value of the change in the measured reflectance may depend on several factors, such as the optical properties of the window 65404, the spectral components of the illumination, and the optical properties of the walls 65406. However, ingestible device 65400 may be configured to store a data set with the reflectance values over time, and search for periodic changes in the data set consistent with the frequency of the peristaltic waves (e.g., by analyzing the data set in the frequency domain, and searching for peaks between 0.05 Hz to 0.33 Hz). This may enable ingestible device 65400 to detect muscle contractions due to peristaltic waves without foreknowledge of the exact changes in reflectance signal amplitude that may occur as a result of detecting the muscle contractions of the peristaltic wave. An example procedure for detecting muscle contractions is discussed further in relation to FIG. 64, and an example of a reflectance data set gathered while ingestible device 65400 is located within the jejunum is discussed in relation to FIG. 65.

Diagram 65440 depicts ingestible device 65400 within the jejunum, when the peristaltic wave has moved past ingestible device 65400. Diagram 65440 depicts contracting portions 65408 that form the peristaltic wave within the jejunum having moved past the end of ingestible device 65400. The peristaltic wave proceeds along the length of the jejunum as different portions of wall 65406 contract and relax, causing it to appear as if contracting portions 65408 of wall 65406 proceed along the length of the jejunum (i.e., as depicted by contracting portions 65408 proceeding from left to right in diagrams 65410-65430). While in this position, ingestible device 65400 may detect a similar level of reflectance (e.g., through the use of an illuminator and a detector of a sensing sub-unit as described elsewhere herein) as detected when there is no peristaltic wave occurring (e.g., as detected when ingestible device 65400 is in the position indicated in diagram 65410, or diagram 65420).

Depending on the species of the subject, peristaltic waves may occur with relatively predictable regularity. After the peristaltic wave has passed over ingestible device 65400 (e.g., as depicted in diagram 65440), the walls 65406 of the jejunum may relax again (e.g., as depicted in diagram 65410), until the next peristaltic wave begins to form. In some embodiments, ingestible device 65400 may be configured to continue to gather reflectance value data while it is within the GI tract, and may store a data set with the reflectance values over time. This may allow ingestible device 65400 to detect each of the muscle contractions as the peristaltic wave passes over ingestible device 65400 (e.g., as depicted in diagram 65430), and may enable ingestible device 65400 to both count the number of muscle contractions that occur, and to determine that a current location of the ingestible device 65400 is within the jejunum. For example, ingestible device 65400 may be configured to monitor for possible muscle contractions while is inside either the stomach or the duodenum, and may determine that ingestible device 65400 has moved to the jejunum in response to detecting a muscle contraction consistent with a peristaltic wave.

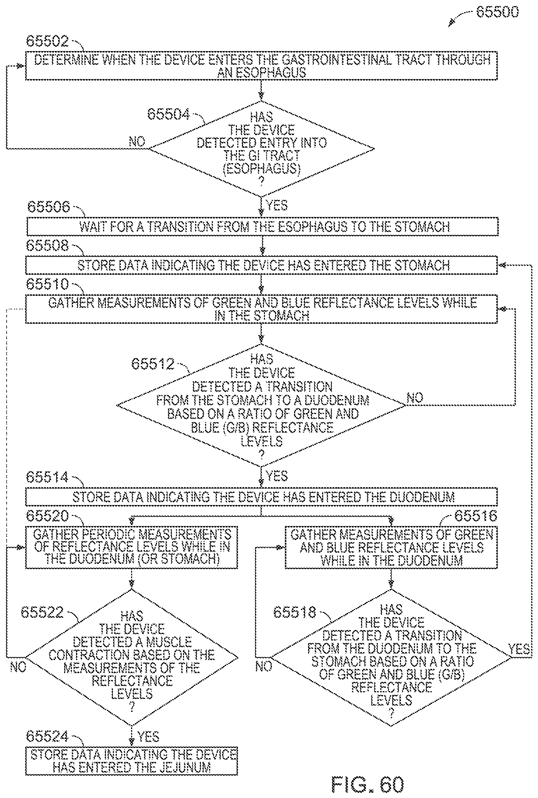

FIG. 60 is a flowchart illustrating some aspects of a localization process used by the ingestible device. In general, the process described in FIG. 60 can be used with any ingestible device disclosed herein. Furthermore, the features of FIG. 60 may be combined with any other systems, methods or processes described in this application. For example, portions of the process in FIG. 60 may be integrated into or combined with the pyloric transition detection procedure described by FIG. 61, or the jejunum detection process described by FIG. 64.

At 65502, the ingestible device gathers measurements (e.g., through a detector as described elsewhere herein) of ambient light. For example, the ingestible device may be configured to periodically measure (e.g., through a detector as described elsewhere herein) the level of ambient light in the environment surrounding the ingestible device. In some embodiments, the type of ambient light being measured may depend on the configuration of the detector within the ingestible device. For example, if the detector is configured to measure red, green, and blue wavelengths of light, the ingestible device may be configured to measure the ambient amount of red, green, and blue light from the surrounding environment. In some embodiments, the amount of ambient light measured by the ingestible device will be larger in the area external to the body (e.g., a well-lit room where the ingestible device is being administered to a subject) and in the oral cavity of the subject, as compared to the ambient level of light measured by the ingestible device when inside of an esophagus, stomach, or other portion of the GI tract (e.g., esophagus, stomach, duodenum, or jejunum).

At 65504, the ingestible device determines (e.g., via control circuitry within a PCB as described elsewhere herein) whether the ingestible device has detected entry into the GI tract. For example, the ingestible device may be configured to determine when the most recent measurement of ambient light (e.g., the measurement gathered at 65502) indicates that the ingestible device has entered the GI tract. For instance, the first time that the ingestible device gatherers a measurement of ambient light at 65502, the ingestible device may store that measurement (e.g., via storage circuitry within a PCB) as a typical level of ambient light external to the body. The ingestible device may be configured to then compare the most recent measurement of ambient light to the typical level of ambient light external to the body (e.g., via control circuitry within a PCB as described elsewhere herein), and determine that the ingestible device has entered the GI tract when the most recent measurement of ambient light is substantially smaller than the typical level of ambient light external to the body. For example, the ingestible device may be configured to detect that it has entered the GI tract in response to determining that the most recent measurement of ambient light is less than or equal to 20% of the typical level of ambient light external to the body. If the ingestible device determines that it has detected entry into the GI tract (e.g., that the ingestible device has entered at least the esophagus), process 65500 proceeds to 65506. Alternately, if the ingestible device determines that it has not detected entry into the GI tract (e.g., as a result of the most recent measurement being similar to the typical level of ambient light external to the body), process 65500 proceeds back to 65502 where the ingestible device gathers further measurements. For instance, the ingestible device may be configured to wait a predetermined amount of time (e.g., five seconds, ten seconds, etc.), and then gather another measurement of the level of ambient light from the environment surrounding the ingestible device.

At 65506, the ingestible device waits for a transition from the esophagus to the stomach (e.g., from the esophagus to the stomach). For example, the ingestible device may be configured to determine that it has entered the stomach (e.g., the stomach) after waiting a predetermined period of time after having entered the GI tract. For instance, a typical esophageal transit time in a human patient may be on the order of 15-30 seconds. In this case, after having detected that the ingestible device has entered the GI tract at 65504 (i.e., after detecting that the ingestible device has reached at least the esophagus), the ingestible device may be configured to wait one minute, or a similar amount of time longer than the typical esophageal transmit time (e.g., ninety-seconds), before automatically determining that the ingestible device has entered at least the stomach (e.g., the stomach).

In some embodiments, the ingestible device may also determine whether it has entered the stomach based on measurements of pH or temperature. For example, the ingestible device may be configured to determine that it has entered the stomach if a temperature of ingestible device has increased to at least 31 degrees Celsius (i.e., consistent with the temperature inside the stomach), or if a measured pH of the environment surrounding the ingestible device is sufficiently acidic (i.e., consistent with the acidic nature of gastric juices that may be found inside the stomach).

At 65508, the ingestible device (stores data indicating the ingestible device has entered the stomach (e.g., the stomach). For example, after having waited a sufficient amount of time at 65506, the ingestible device may store data (e.g., within storage circuitry of a PCB 65120 as described elsewhere herein) indicative of the ingestible device having entered at least the stomach. Once the ingestible device reaches at least the stomach, process 65500 proceeds to 65510 where the ingestible device may be configured to gather data to detect entry into the duodenum (e.g., the duodenum).

In some embodiments, process 65500 may also simultaneously proceed from 65508 to 65520, where the ingestible device may be configured to gather data in order to detect muscle contractions and detect entry into the jejunum (e.g., the jejunum). In some embodiments, the ingestible device may be configured to simultaneously monitor for entry into the duodenum at 65516-65518, as well as detect for entry into the jejunum at 65520-65524. This may allow the ingestible device to determine when it has entered the jejunum (e.g., as a result of detecting muscle contractions), even when it fails to first detect entry into the duodenum (e.g., as a result of very quick transit times of the ingestible device through the duodenum).

At 65510, the ingestible device gathers measurements of green and blue reflectance levels (e.g., through the use of an illuminator and a detector of a sensing sub-unit as described elsewhere herein) while in the stomach. For example, the ingestible device may be configured to periodically gather measurements of green and blue reflectance levels while in the stomach. For instance, the ingestible device may be configured to transmit a green illumination and a blue illumination (e.g., via an illuminator as described elsewhere herein) every five to fifteen seconds, and measure the resulting reflectance (e.g., via a detector as described elsewhere herein). Every time that the ingestible device gathers a new set of measurements, the measurements may be added to a stored data set (e.g., stored within memory circuitry of a PCB as described elsewhere herein). The ingestible device may then use this data set to determine whether or not the ingestible device is still within a stomach or a duodenum.

In some embodiments, the ingestible device may be configured to detect a first reflectance based on generating an illumination of a first wavelength in approximately the green spectrum of light (between 495-600 nm), and detecting a second reflectance based on generating an illumination of the second wavelength in approximately the blue spectrum of light (between 400-495 nm). In some embodiments, the ingestible device may ensure that the illumination in the green spectrum and the illumination in the blue spectrum have wavelengths separated by at least 50 nm. This may enable the ingestible device to sufficiently distinguish between the two wavelengths when detecting the reflectances (e.g., via a detector as described elsewhere herein). It is understood that the separation of 50 nm is intended to be illustrative, and not limiting, and depending on the accuracy of the detectors within the ingestible device, smaller separations may be possible to be used.

At 65512, the ingestible device determines (e.g., using control circuitry within a PCB as described elsewhere herein) whether the ingestible device has detected a transition from the stomach to a duodenum based on a ratio of green and blue (G/B) reflectance levels. For example, the ingestible device may obtain (e.g., from memory circuitry of a PCB as described elsewhere herein) a data set containing historical data for the respective ratio of the green reflectance to the blue reflectance as measured at a respective time. Generally speaking, a duodenum of a human subject reflects a higher ratio of green light to blue light, as compared to the ratio of green light to blue light that is reflected by a stomach. Based on this, the ingestible device may be configured to take a first set of ratios from the data set, representing the result of recent measurements, and compare them to a second set of ratios from the data set, representing the results of past measurements. When the ingestible device determines that the mean value of the first set of ratios is substantially larger than the mean value of the second set of ratios (i.e., that the ratio of reflected green light to reflected blue light has increased), the ingestible device may determine that it has entered the duodenum (from the stomach. If the ingestible device detects a transition from the stomach to a duodenum (process 65500 proceeds to 65514, where the ingestible device stores data indicating that the ingestible device has entered the duodenum. Alternatively, if the ingestible device determines that the ingestible device has not transitioned from the stomach to the duodenum, process 65500 proceeds back to 65510 to gather more measurements of green and blue reflectance levels while still in the stomach. An example procedure for using measurements of green and blue reflectances to monitor for transitions between the stomach and the duodenum is discussed in greater detail in relation to FIG. 61.

In some embodiments, the first time that detects a transition from the stomach to the duodenum, the ingestible device may be configured to take a mean of the second set of data, (e.g., the set of data previously recorded while in the stomach) and store this as a typical ratio of green light to blue light detected within the stomach (e.g., the stomach) (e.g., within memory circuitry of a PCB 65120 (FIG. 57) as described elsewhere herein). This stored information may later be used by the ingestible device to determine when the ingestible device re-enters the stomach from the duodenum as a result of a reverse pyloric transition.

At 65514, the ingestible device stores data indicating that the ingestible device has entered the duodenum. For example, the ingestible device may store a flag within local memory (e.g., memory circuitry of a PCB as described elsewhere herein) indicating that the ingestible device is currently in the duodenum. In some embodiments, the ingestible device may also store a timestamp indicating the time when the ingestible device entered the duodenum. Once the ingestible device reaches the duodenum, process 65500 proceeds to 65520 where the ingestible device may be configured to gather data in order to detect muscle contractions and detect entry into the jejunum. Process 65500 also proceeds from 65514 to 65516, where the ingestible device may be configured to gather data additional data in order to detect re-entry into the stomach from the duodenum.

At 65516, the ingestible device gathers measurements (e.g., via a sensing sub-unit as described elsewhere herein) of green and blue reflectance levels while in the duodenum. For example, the ingestible device may be configured to periodically gather measurements (e.g., via a sensing sub-unit as described elsewhere herein) of green and blue reflectance levels while in the duodenum, similar to the measurements made at 65510 while in the stomach. For instance, the ingestible device may be configured to transmit a green illumination and a blue illumination (e.g., via an illuminator as described elsewhere herein) every five to fifteen seconds, and measure the resulting reflectance (e.g., via a detector as described elsewhere herein). Every time that the ingestible device gathers a new set of measurements, the measurements may be added to a stored data set (e.g., stored within memory circuitry of a PCB). The ingestible device may then use this data set to determine whether or the ingestible device is still within the duodenum, or if the ingestible device has transitioned back into the stomach).

At 65518, the ingestible device determines a transition from the duodenum to the stomach based on a ratio of the measured green reflectance levels to the measured blue reflectance levels. In some embodiments, the ingestible device may compare the ratio of the measured green reflectance levels to the measured blue reflectance levels recently gathered by the ingestible device (e.g., measurements gathered at 65516), and determine whether or not the ratio of the measured green reflectance levels to the measured blue reflectance levels is similar to the average ratio of the measured green reflectance levels to the measured blue reflectance levels seen in the stomach. For instance, the ingestible device may retrieve data (e.g., from memory circuitry of a PCB (FIG. 57)) indicative of the average ratio of the measured green reflectance levels to the measured blue reflectance levels seen in the stomach, and determine that the ingestible device has transitioned back to the stomach if the recently measured ratio of the measured green reflectance levels to the measured blue reflectance levels is sufficiently similar to the average level in the stomach (e.g., within 20% of the average ratio of the measured green reflectance levels to the measured blue reflectance levels seen in the stomach, or within any other suitable threshold level). If the ingestible device detects a transition from the duodenum to the stomach, process 65500 proceeds to 65508 to store data indicating the ingestible device has entered the stomach, and continues to monitor for further transitions. Alternatively, if the ingestible device does not detect a transition from the duodenum to the stomach, process 65500 proceeds to 65516 to gather additional measurements of green and blue reflectance levels while in the duodenum, which may be used to continuously monitor for possible transitions back into the stomach. An example procedure for using measurements of green and blue reflectances to monitor for transitions between the stomach and the duodenum is discussed in greater detail in relation to FIG. 61.

At 65520, the ingestible device gathers periodic measurements of the reflectance levels (e.g., via a sensing sub-unit) while in the duodenum. In some embodiments, the ingestible device may gather similar periodic measurements while in the stomach as well. In some embodiments, these periodic measurements may enable the ingestible device to detect muscle contractions (e.g., muscle contractions due to a peristaltic wave as discussed in relation to FIG. 59), which may be indicative of entry into a jejunum. The ingestible device may be configured to gather periodic measurements using any suitable wavelength of illumination (e.g., by generating illumination using an illuminator and detecting the resulting reflectance using a detector), or combinations of wavelengths of illumination. For example, in some embodiments, the ingestible device may be configured to generate red, green, and blue illumination, store separate data sets indicative of red, green, and blue illumination, and analyze each of the data sets separately to search for frequency components in the recorded data indicative of detected muscle contractions. In some embodiments, the measurements gathered by the ingestible device at 65520 may be sufficiently fast as to detect peristaltic waves in a subject. For instance, in a healthy human subject, peristaltic waves may occur at a rate of approximately 0.05 Hz to 0.33 Hz. Therefore, the ingestible device may be configured to generate illumination and measure the resulting reflectance at least once every 2.5 seconds (i.e., potentially minimum rate to detect a 0.2 Hz signal), and preferably at a higher rate, such as once every 0.5 seconds or faster, and store values indicative of the resulting reflectances in a data set (e.g., within memory circuitry of a PCB). After gathering additional data (e.g., after gathering one new data point, or a predetermined number of new data points), process 65500 proceeds to 65522, where the ingestible device determines whether or not a muscle contraction has been detected.

At 65522, the ingestible device determines (e.g., via control circuitry within a PCB) whether the ingestible device detects a muscle contraction based on the measurements of reflectance levels (e.g., as gathered by a sensing sub-unit). For example, the ingestible device may obtain a fixed amount of data stored as a result of measurements made at 65520 (e.g., retrieve the past minute of data from memory circuitry within a PCB. The ingestible device may then convert the obtained data into the frequency domain, and search for peaks in a frequency range that would be consistent with peristaltic waves. For example, in a healthy human subject, peristaltic waves may occur at a rate of approximately 0.05 Hz to 0.33 Hz, and the ingestible device may be configured to search for peaks in the frequency domain representation of the data between 0.05 Hz to 0.33 Hz above a threshold value. If the ingestible device detects a contraction based on the reflectance levels (e.g., based on detecting peaks in the frequency domain representation of the data between 0.05 Hz to 0.33 Hz), process 65500 proceeds to 65524 to store data indicating that the device has entered the jejunum. Alternatively, if the ingestible device does not detect a muscle contraction, process 65500 proceeds to 65520 to gather periodic measurements of the reflectance levels while in the duodenum. In some embodiments, the ingestible device may store data (e.g., within memory circuitry of a PCB) indicating that a muscle contraction was detected, and process 65500 will not proceed from 65522 to 65524 until a sufficient number of muscle contractions have been detected.

At 65524, the ingestible device stores data (e.g., within memory circuitry of a PCB) indicating that the device has entered the jejunum). For example, in response to detecting that muscle contraction has occurred at 65522, the ingestible device may determine that it has entered the jejunum 65314, and is no longer inside of the duodenum or the stomach. In some embodiments, the ingestible device may continue to measure muscle contractions while in the jejunum, and may store data indicative of the frequency, number, or strength of the muscle contractions over time (e.g., within memory circuitry of a PCB). In some embodiments, the ingestible device may also be configured to monitor for one or more transitions. Such transitions can include a transition from the jejunum to the ileum, an ileoceacal transition from the ileum to the cecum, a transition from the cecum to the colon, or detect exit from the body (e.g., by measuring reflectances, temperature, or levels of ambient light).

In some embodiments, the ingestible device may also determine that it has entered the jejunum after a pre-determined amount of time has passed after having detected entry into the duodenum. For example, barring a reverse pyloric transition from the duodenum back to the stomach, the typical transit time for an ingestible device to reach the jejunum from the duodenum in a healthy human subject is less than three minutes. In some embodiments, the ingestible device may therefore be configured to automatically determine that it has entered the jejunum after spending at least three minutes within the duodenum. This determination may be made separately from the determination made based on measured muscle contractions (e.g., the determination made at 65522), and in some embodiments, the ingestible device may determine that it has entered the jejunum in response to either detecting muscle contractions, or after three minutes has elapsed from having entered the duodenum (e.g., as determined by storing data at 65514 indicative of the time that ingestible device entered the duodenum).

For illustrative purposes, 65512-65518 of process 65500 describe the ingestible device measuring green reflectances and blue reflectances, calculating a ratio of the two reflectances, and using this information to determine when the ingestible device has transitioned between the duodenum and stomach. However, in some embodiments, other wavelengths of light may be used other than green and blue, provided that the wavelengths of light chosen have different reflective properties within the stomach and the duodenum (e.g., as a result of different reflection coefficients of the stomach tissue and the tissue of the duodenum).

It will be understood that the steps and descriptions of the flowcharts of this disclosure, including FIG. 60, are merely illustrative. Any of the steps and descriptions of the flowcharts, including FIG. 60, may be modified, omitted, rearranged, and performed in alternate orders or in parallel, two or more of the steps may be combined, or any additional steps may be added, without departing from the scope of the present disclosure. For example, the ingestible device may calculate the mean and the standard deviation of multiple data sets in parallel in order to speed up the overall computation time. As another example, the ingestible device may gather data periodic measurements and detect possible muscle contractions (e.g., at 65520-65522) while simultaneously gathering green and blue reflectance levels to determine transitions to and from the stomach and duodenum (e.g., at 65510-65518). Furthermore, it should be noted that the steps and descriptions of FIG. 60 may be combined with any other system, device, or method described in this application, including processes 65600 and 65900, and any of the ingestible devices or systems discussed in this application could be used to perform one or more of the steps in FIG. 60.

FIG. 61 is a flowchart illustrating some aspects of a process for detecting transitions from a stomach to a duodenum and from a duodenum back to a stomach, which may be used when determining a location of an ingestible device as it transits through a gastrointestinal (GI) tract, in accordance with some embodiments of the disclosure. In some embodiments, process 65600 may begin when an ingestible device first detects that it has entered the stomach, and will continue as long as the ingestible device determines that it is within the stomach or the duodenum. In some embodiments, process 65600 may only be terminated when an ingestible device determines that it has entered the jejunum, or otherwise progressed past the duodenum and the stomach. The duodenum detection process 65600 described in FIG. 61 may be applied to any device discussed in this application, and any of the ingestible devices may be used to perform one or more parts of the process described in FIG. 61. Furthermore, the features of FIG. 61 may be combined with any other systems, methods or processes described in this application. For example, portions of the process described by the process in FIG. 61 may be integrated into process 65500 discussed in relation to FIG. 60.

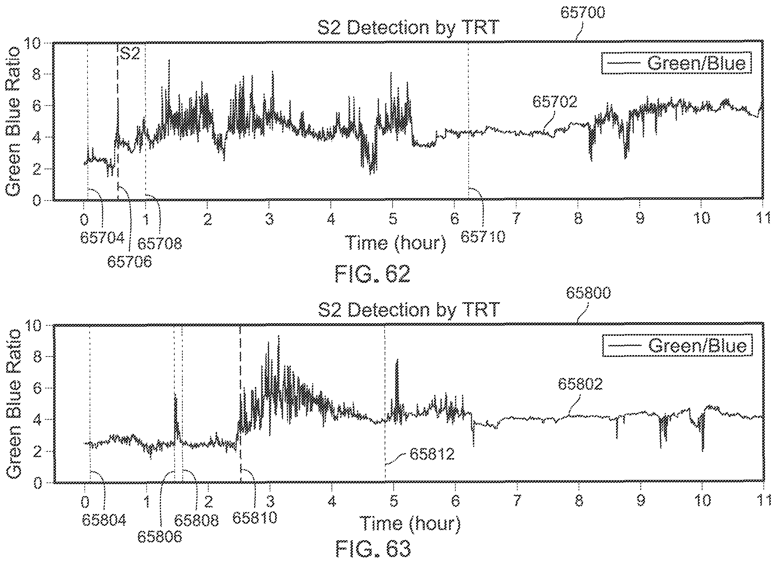

At 65602, the ingestible device retrieves a data set (e.g., from memory circuitry within a PCB) with ratios of the measured green reflectance levels to the measured blue reflectance levels over time. For example, the ingestible device may retrieve a data set from a PCB containing recently recorded ratios of the measured green reflectance levels to the measured blue reflectance levels (e.g., as recorded at 65510 or 65516 of process 65500). In some embodiments, the retrieved data set may include the ratios of the measured green reflectance levels to the measured blue reflectance levels over time. Example plots of data sets of ratios of the measured green reflectance levels to the measured blue reflectance levels are discussed further in relation to FIG. 62 and FIG. 63.

At 65604, the ingestible device includes a new measurement (e.g., as made with a sensing sub-unit) of a ratio of the measured green reflectance level to the measured blue reflectance level in the data set. For example, the ingestible device may be configured to occasionally record new data by transmitting green and blue illumination (e.g., via an illuminator), detecting the amount of reflectance received due to the green and blue illumination (e.g., via a detector), and storing data indicative of the amount of the received reflectance (e.g., in memory circuitry of a PCB). The ingestible device may be configured to record new data every five to fifteen seconds, or at any other convenient interval of time. For illustrative purposes, the ingestible device is described as storing and retrieving the ratio of the measured green reflectance levels to the measured blue reflectance levels (e.g., if the amount of detected green reflectance was identical to the amount of detected blue reflectance at a given time, the ratio of the green and blue reflectances would be "1.0" at that given time); however, it is understood that the green reflectance data and the blue reflectance data may be stored separately within the memory of the ingestible device (e.g., stored as two separate data sets within memory circuitry of a PCB).

At 65606, the ingestible device retrieves a first subset of recent data by applying a first sliding window filter to the data set. For example, the ingestible device may use a sliding window filter to obtain a predetermined amount of the most recent data within the data set, which may include any new values of the ratio of the measured green reflectance level to the measured blue reflectance level obtained at 65604. For instance, the ingestible device may be configured to select between ten and forty data points from the data set, or the ingestible device may be configured to select a predetermined range of data values between fifteen seconds of data and five minutes of data. In some embodiments, other ranges of data may be selected, depending on how frequently measurements are recorded, and the particular application at hand. For instance, any suitable amount of data may be selected in the sliding window, provided that it is sufficient to detect statistically significant differences between the data selected in a second sliding window (e.g., the second subset of data selected at 65614).

In some embodiments, the ingestible device may also be configured to remove outliers from the data set, or to smooth out unwanted noise in the data set. For example, the ingestible device may select the first subset of data, or any other subset of data, by first obtaining a raw set of values by applying a window filter to the data set (e.g., selecting a particular range of data to be included). The ingestible device may then be configured to identify outliers in the raw set of values; for instance, by identifying data points that are over three standard deviations away from the mean value of the raw set of values, or any other suitable threshold. The ingestible device may then determine the subset of data by removing outliers from the raw set of values. This may enable the ingestible device to avoid spurious information when determining whether or not it is located within the stomach or the duodenum.

At 65608, the ingestible device determines whether the most recently detected location was the duodenum. In some embodiments, the ingestible device may store a data flag (e.g., within memory circuitry of a PCB 65120 (FIG. 57)) indicating the most recent portion of the GI tract that the ingestible device detected itself to be within. For instance, every time the ingestible device detects entry to the stomach (e.g., detects entry into stomach 65306 as a result of the decision made at 65610), a flag is stored in memory indicating the ingestible device is in the stomach (e.g., as part of storing data at 65612). If the ingestible device subsequently detects entry into the duodenum (e.g., detects entry into the duodenum 65310 as a result of a decision made at 65624), another different flag is stored in memory indicating that the ingestible device is in the duodenum (e.g., as part of storing data at 65624). In this case, the ingestible device may retrieve the most recently stored flag at 65608, and determine whether or not the flag indicates that the ingestible device was most recently within the duodenum. If the ingestible device detects that it was most recently in the duodenum, process 65600 proceeds to 65610 where the ingestible device compares the recent measurements of the ratios of the measured green reflectance levels to the measured blue reflectance levels (e.g., measurements that include the recent measurement made at 65606) to the typical ratios measured within the stomach, and uses this information to determine whether a reverse pyloric transition from the duodenum back to the stomach has occurred. Alternately, if the ingestible device detects that it was not most recently in the duodenum (e.g., because it was in the stomach instead), process 65600 proceeds to 65614 where the ingestible device compares the recent measurements of the ratios of the measured green reflectance levels to the measured blue reflectance levels (e.g., measurements that include the recent measurement made at 65606) to past measurements, and uses this information to determine whether a pyloric transition from the stomach to the duodenum has occurred.

Process 65600 proceeds from 65608 to 65610 when the ingestible device determined that it was most recently in the duodenum. At 65610, the ingestible device determines (e.g., via control circuitry within a PCB) whether the current G/B signal is similar to a recorded average G/B signal in the stomach. For example, the ingestible device may be configured to have previously stored data (e.g., within memory circuitry of a PCB 65120 (FIG. 57)) indicative of the average ratio of the measured green reflectance levels to the measured blue reflectance levels measured in the stomach. The ingestible device may then retrieve this stored data indicative of the average ratio of the measured green reflectance levels to the measured blue reflectance levels in the stomach, and compare this against the recent measurements in order to determine whether or not the ingestible device has returned back to the stomach from the duodenum. For instance, the ingestible device may determine if the mean value of the first subset of recent data (i.e., the average value of the recently measured ratios of the measured green reflectance levels to the measured blue reflectance levels) is less than the average ratio of the measured green reflectance levels to the measured blue reflectance levels within the stomach, or less that the average ratio measured within the stomach plus a predetermined number times the standard deviation of the ratios measured within the stomach. For instance, if the average ratio of the measured green reflectance levels to the measured blue reflectance levels in the stomach was "1," with a standard deviation of "0.2," ingestible device may determine whether or not the mean value of the first subset of data is less than "1.0 + k*0.2," where "k" is a number between zero and five. It is understood that, in some embodiments, the ingestible device may be configured to use a different threshold level to determine whether or not the mean value of the first subset of recent data is sufficiently similar to the average ratio of the measured green reflectance levels to the measured blue reflectance levels within the stomach. In response to determining that the recent ratio of the measured green reflectance levels to the measured blue reflectance levels is similar to the average ratio of measured green and blue reflectance levels seen in the stomach, process 65600 proceeds to 65612 where the ingestible device stores data indicating that it has re-entered the stomach from the duodenum. Alternately, in response to determining that the recent ratio of measured green and blue reflectance levels is sufficiently different from the average ratio of measured green and blue reflectance levels seen in the stomach, the ingestible device proceeds directly to 65604, and continues to obtain new data on an ongoing basis.

At 65612, the ingestible device stores data indicating a reverse pyloric transition from the duodenum to the stomach was detected. For example, the ingestible device may store a data flag (e.g., within memory circuitry of a PCB) indicating that the ingestible device most recently detected itself to be within the stomach portion of the GI tract. In some embodiments, the ingestible device may also store data (e.g., within memory circuitry of a PCB) indicating a time that the ingestible device detected the reverse pyloric transition from the duodenum to the stomach. This information may be used by the ingestible device at 65608, and as a result process 65600 may proceed from 65608 to 65614, rather than proceeding from 65618 to 65610. After the ingestible device stores the data indicating a reverse pyloric transition from the duodenum to the stomach was detected, process 65600 proceeds to 65604 where the ingestible device continues to gather additional measurements, and continues to monitor for further transitions between the stomach and the duodenum.

Process 65600 proceeds from 65608 to 65614 when the ingestible device determined that it was not most recently in the duodenum (e.g., as a result of having most recently been in the stomach instead). At 65614, the ingestible device retrieves a second subset of previous data by applying a second sliding window filter to the data set. For example, the ingestible device may use a sliding window filter to obtain a predetermined amount of older data from a past time range, which may be separated from recent time range used to select the first subset of data gathered at 65606 by a predetermined period of time. In some embodiments, any suitable amount of data may be selected by the first and second window filters, and the first and second window filters may be separated by any appropriate predetermined amount of time. For example, in some embodiments, the first window filter and the second window filter may each be configured to select a predetermined range of data values from the data set, the predetermined range being between fifteen seconds of data and five minutes of data. In some embodiments, the recent measurements and the past measurements may then be separated by a predetermined period of time that is between one to five times the predetermined range of data values. For instance, the ingestible device may select the first subset of data and the second subset of data to each be one minute of data selected from the dataset (i.e., selected to have a predetermined range of one minute), and the first subset of data and the second subset of data are selected from recorded measurements that are at least two minutes apart (i.e., the predetermined period of time is two minutes, which is twice the range used to select the subsets of data using the window filters). As another example, the ingestible device may select the first subset of data and the second subset of data to each be five minutes of data selected from the dataset (i.e., selected to have a predetermined range of five minutes), and the first subset of data and the second subset of data are selected from recorded measurements that are at least 10 minutes apart (i.e., the predetermined period of time is two minutes, which is twice the range used to select the subsets of data using the window filters).

In some embodiments, if the ingestible device recently transitioned to the stomach from the duodenum (e.g., as determined by checking for recent data stored within the ingestible device at 65612), the ingestible device may select the second subset of data at 65614 from a time frame when the ingestible device is known to be within the stomach. In some embodiments, the ingestible device may alternately select a previously recorded average and standard deviation for ratios of green reflectances and blue reflectances within the stomach (e.g., an average and standard deviation typical of data recorded within the stomach, as previously recorded within memory circuitry of a PCB at 65620) in place of the second subset of data. In this case, the ingestible device may simply use the previously recorded average and previously recorded standard deviation when making a determination at 65616, rather than expending resources to calculate the mean and standard deviation of the second subset.

At 65616, the ingestible device determines whether the difference between the mean of the second subset and the mean of the first subset is greater than a predetermined multiple of the standard deviation of the first subset. For example, the ingestible device may compute a difference between a mean of the first subset of recent data and a mean of a second subset of past data, and determine whether this difference is greater than three times the standard deviation of the second subset of past data. In some embodiments, it is understood that any convenient threshold level may be used other than three times the standard deviation, such as any value between one and five times the standard deviation. Also, in some embodiments, the ingestible device may instead set the threshold level based on the standard deviation of the second subset instead of the first subset. In response to determining that the difference between the mean of the first subset and the mean of the second subset is greater than a predetermined multiple of the standard deviation of the second subset, process 65600 proceeds to 65618. Otherwise, process 65600 proceeds back to 65604, where the ingestible device 65604 continues to gather new data to be used in monitoring for transitions between the stomach and the duodenum.

At 65618, the ingestible device determines (e.g., via control circuitry within a PCB) whether the determination made at 65616 is the first time that the difference between the mean of the first subset of recent data and the mean of the second subset of past data is calculated to be greater than the standard deviation of the second subset. If the ingestible device determines that this is the first time that the difference between the mean of the first subset and the mean of the second subset is calculated to be greater than the standard deviation of the second subset, process 65600 proceeds to 65620 to store the mean of the second subset of past data as an average G/B signal in the stomach. Alternatively, if the ingestible device determines that the immediately preceding determination made at 65616 is not the first time that the difference between the mean of the first subset of recent data and the mean of the second subset of past data is calculated to be greater than the standard deviation of the second subset, process 65600 proceeds directly to 65622.

At 65620, the ingestible device stores the mean of the second subset as an average G/B signal in the stomach. For example, the ingestible device may be configured to store the mean of the second subset of past data (e.g., store within memory circuitry of a PCB 65120) as the average ratio of the measured green reflectance levels to the measured blue reflectance levels measured in the stomach. In some embodiments, the ingestible device may also store the standard deviation of the second subset of past data as a typical standard deviation of the ratios of the measured green reflectance levels to the measured blue reflectance levels detected within the stomach. This stored information may be used by the ingestible device later on (e.g., at 65610) to compare against future data, which may enable the ingestible device to detect reverse pyloric transitions from the duodenum back to the stomach, and may generally be used in place of other experimental data gathered from the stomach (e.g., in place of the second subset of data at 65616). After storing the mean of the second subset as an average G/B signal in the stomach, process 65600 proceeds to 65622.

At 65622, the ingestible device determines whether a difference of the mean of the first subset of recent data to the mean of the second subset of past data is greater than a predetermined threshold, "M". In some embodiments, the predetermined threshold, "M," will be sufficiently large to ensure that the mean of the first subset is substantially larger than the mean of the second subset, and may enable the ingestible device to ensure that it detected an actual transition to the duodenum. This may be particularly advantageous when the determination made at 65616 is potentially unreliable due to the standard deviation of the second subset of past data being abnormally small. For example, a typical value of the predetermined threshold "M," may be on the order of 0.1 to 0.5. If the ingestible device determines that the difference of the mean of the first subset of recent data to the second subset of past data is greater than a predetermined threshold, process 65600 proceeds to 65624 to store data indicating that a pyloric transition from the stomach to the duodenum was detected. Alternatively, if the ingestible device determines that the ratio of the mean of the first subset to the second subset is less than or equal to the predetermined threshold, "M" (i.e., determines that a transition to the duodenum has not occurred), process 65600 proceeds directly to 65604 where the ingestible device continues to make new measurements and monitor for possible transitions between the stomach and the duodenum.

In some embodiments, instead of using a difference of the mean of the first subset of recent data to the mean of the second subset of past data, the ingestible device determines whether the ratio of the mean of the first subset of recent data to the mean of the second subset of past data is greater than a predetermined threshold, "M". In some embodiments, the predetermined threshold, "M," will be sufficiently large to ensure that the mean of the first subset is substantially larger than the mean of the second subset, and may enable the ingestible device to ensure that it detected an actual transition to the duodenum. This may be particularly advantageous when the determination made at 65616 is potentially unreliable due to the standard deviation of the second subset of past data being abnormally small. For example, a typical value of the predetermined threshold "M," may be on the order of 1.2 to 2.0. It is understood any convenient type of threshold or calculation may be used to determine whether or not the first subset of data and the second subset of data are both statistically distinct from one another, and also substantially different from one another in terms of overall average value.

At 65624, the ingestible device stores data indicating a pyloric transition from the stomach to the duodenum was detected. For example, the ingestible device may store a data flag (e.g., within memory circuitry of a PCB) indicating that the ingestible device most recently detected itself to be within the duodenum portion of the GI tract. In some embodiments, the ingestible device may also store data (e.g., within memory circuitry of a PCB) indicating a time that the ingestible device detected the pyloric transition from the stomach to the duodenum. This information may be used by the ingestible device at 65608, and as a result process 65600 may proceed from 65608 to 65610, rather than proceeding from 65618 to 65614. After the ingestible device stores the data indicating a pyloric transition from the stomach to the duodenum was detected, process 65600 proceeds to 65604 where the ingestible device continues to gather additional measurements, and continues to monitor for further transitions between the stomach and the duodenum.

It will be understood that the steps and descriptions of the flowcharts of this disclosure, including FIG. 61, are merely illustrative. Any of the steps and descriptions of the flowcharts, including FIG. 61, may be modified, omitted, rearranged, and performed in alternate orders or in parallel, two or more of the steps may be combined, or any additional steps may be added, without departing from the scope of the present disclosure. For example, the ingestible device may calculate the mean and the standard deviation of multiple data sets in parallel in order to speed up the overall computation time. Furthermore, it should be noted that the steps and descriptions of FIG. 61 may be combined with any other system, device, or method described in this application, and any of the ingestible devices or systems discussed in this application could be used to perform one or more of the steps in FIG. 61. For example, portions of process 65600 may be incorporated into 65508-65516 of process 65500, and may be part of a more general process for determining a location of the ingestible device. As another example, the ratio of detected blue and green light (e.g., as measured and added to the data set at 65604) may continue even outside of the stomach or duodenum, and similar information may be recorded by the ingestible device throughout its transit in the GI tract. Example plots of data sets of ratios of measured green and blue reflectance levels, which may be gathered throughout the GI tract, are discussed further in relation to FIG. 62 and FIG. 62 below.

FIG. 62 is a plot illustrating data collected during an example operation of an ingestible device, which may be used when determining a location of an ingestible device as it transits through a gastrointestinal (GI) tract, in accordance with some embodiments of the disclosure.

Although FIG. 62 may be described in connection with the ingestible device for illustrative purposes, this is not intended to be limiting, and plot 65700 and data set 65702 may be typical of data gathered by any device discussed in this application. Plot 65700 depicts the ratios of the measured green reflectance levels to the measured blue reflectance levels over time. For example, the ingestible device may have computed the value for each point in the data set 65702 by transmitting green and blue illumination at a given time (e.g., via a illuminator), measuring the resulting green and blue reflectances (e.g., via a detector), calculating the ratio of the resulting reflectances, and storing the ratio in the data set along with a timestamp indicating the time that the reflectances were gathered.

At 65704, shortly after the ingestible device begins operation, the ingestible device determines that it has reached at least the stomach (e.g., as a result of making a determination similar to the determination discussed in relation to 65506 in process 65500). The ingestible device continues to gather additional measurements of green and blue reflectance levels, and at 65706 the ingestible device determines that a pyloric transition has occurred from the stomach to the duodenum (e.g., as a result of making a determination similar to the determinations discussed in relation to 65616-65624 of process 65600). Notably, the values in data set 65702 around 65706 jump up precipitously, which is indicative of the higher ratios of measured green reflectance levels to measured blue reflectance levels typical of the duodenum.

The remainder of the data set 65702 depicts the ratios of the measured green reflectance levels to the measured blue reflectance levels throughout the remainder of the GI tract. At 65708, the ingestible device has reached the jejunum (e.g., as determined through measurements of muscle contractions, as discussed in relation to FIG. 64), and by 65710, the ingestible device has reached the cecum. It is understood that, in some embodiments, the overall character and appearance of data set 65702 changes within the small intestine (i.e., the duodenum, jejunum, and ileum) versus the cecum. Within the jejunum and ileum, there may typically be a wide variation in the ratios of the measured green reflectance levels to the measured blue reflectance levels, resulting in relatively noisy data with a high standard deviation. By comparison, within the cecum the ingestible device may measure a relatively stable ratio of the measured green reflectance levels to the measured blue reflectance levels. In some embodiments, the ingestible device may be configured to determine transitions from the small intestine to the cecum based on these differences. For example, the ingestible device may compare recent windows of data to past windows of data, and detect a transition to the cecum in response to determining that the standard deviation of the ratios in the recent window of data is substantially less than the standard deviation of the ratios in the past window of data.

FIG. 63 is another plot illustrating data collected during an example operation of an ingestible device, which may be used when determining a location of an ingestible device as it transits through a gastrointestinal (GI) tract, in accordance with some embodiments of the disclosure. Similar to FIG. 62, FIG. 63 may be described in connection with the ingestible device for illustrative purposes. However, this is not intended to be limiting, and plot 65800 and data set 65802 may be typical of data gathered by any device discussed in this application.

At 65804, shortly after the ingestible device begins operation, the ingestible device determines that it has reached at least the stomach (e.g., as a result of making a determination similar to the determination discussed in relation to 65506 in process 65500). The ingestible device continues to gather additional measurements of green and blue reflectance levels (e.g., via a sensing sub-unit), and at 65806 the ingestible device determines that a pyloric transition has occurred from the stomach to the duodenum (e.g., as a result of making a determination similar to the determinations discussed in relation to 65616-65624 of process 65600). Notably, the values in data set 65802 around 65806 jump up precipitously, which is indicative of the higher ratios of measured green reflectance levels to measured blue reflectance levels typical of the duodenum, before falling shortly thereafter. As a result of the reduced values in data set 65802, the ingestible device determines that a reverse pyloric transition has occurred from the duodenum back to the stomach at 65808 (e.g., as a result of making a determination similar to the determinations discussed in relation to 65610-65612 of process 65600). At 65810, as a result of the values in data set 65802 increasing again, the ingestible device determines that another pyloric transition has occurred from the stomach to the duodenum, and shortly thereafter the ingestible device proceeds onwards to the jejunum, ileum, and cecum.

The remainder of the data set 65802 depicts the ratios of the measured green reflectance levels to the measured blue reflectance levels throughout the remainder of the GI tract. Notably, at 65812, ingestible device reaches the transition point between the ileum and the cecum. As discussed above in relation to FIG. 62, the transition to the cecum is marked by a reduced standard deviation in the ratios of measured green reflectances and measured blue reflectances over time, and the ingestible device may be configured to detect a transition to the cecum based on determining that the standard deviation of a recent set of measurements is substantially smaller than the standard deviation of past measurements taken from the jejunum or ileum.

FIG. 64 is a flowchart of illustrative steps for detecting a transition from a duodenum to a jejunum, which may be used when determining a location of an ingestible device as it transits through a gastrointestinal (GI) tract, in accordance with some embodiments of the disclosure. Although FIG. 64 may be described in connection with the ingestible device for illustrative purposes, this is not intended to be limiting, and either portions or the entirety of process 65900 described in FIG. 64 may be applied to any device discussed in this application, and any of these ingestible devices may be used to perform one or more parts of the process described in FIG. 64. Furthermore, the features of FIG.64 may be combined with any other systems, methods or processes described in this application. For example, portions of the process described by the process in FIG. 64 may be integrated into the localization process 65500 (e.g., as part of 65520-65524). In some embodiments, the ingestible device may perform process 65900 while in the duodenum, or in response to detecting entry to the duodenum. In other embodiments, the ingestible device may perform process 65900 while in the stomach, or in response to detecting entry into the GI tract. It is also understood that process 65900 may be performed in parallel with any other process described in this disclosure (e.g., process 65600), which may enable the ingestible device to detect entry into various portions of the GI tract, without necessarily detecting entry into a preceding portion of the GI tract.

For illustrative purposes, FIG. 64 may be discussed in terms of the ingestible device generating and making determinations based on a single set of reflectance levels generated at a single wavelength by a single sensing sub-unit (e.g., sensing sub-unit 65126). However, it is understood that the ingestible device may generate multiple wavelengths of illumination from multiple different sensing sub-units positioned around the circumference of ingestible device (e.g., multiple sensing sub-units positioned at different locations behind window 65114 of the ingestible device , and each of the resulting reflectances may be stored as a separate data set. Moreover, each of these sets of reflectance levels may be used to detect muscle contractions by running multiple versions of process 65900, each one of which processes data for a different set of reflectances corresponding to data sets obtained from measurements of different wavelengths or measurements made by different sensing sub-units.

At 65902, the ingestible device retrieves a set of reflectance levels. For example, the ingestible device may retrieve a data set of previously recorded reflectance levels from memory (e.g., from memory circuitry of a PCB). Each of the reflectance levels may correspond to reflectances previously detected by the ingestible device (e.g., via a detector) from illumination generated by the ingestible device (e.g., via an illuminator), and may represent a value indicative of an amount of light detected in a given reflectance. However, it is understood that any suitable frequency of light may be used, such as light in the infrared, visible, or ultraviolet spectrums. In some embodiments, the reflectance levels may correspond to reflectances previously detected by the ingestible device at periodic intervals.

At 65904, the ingestible device includes new measurements of reflectance levels in the data set. For example, the ingestible device may be configured to detect a new reflectance (e.g., transmit illumination and detect the resulting reflectance using a sensing sub-unit) at regular intervals, or with sufficient speed as to detect peristaltic waves. For example, the ingestible device may be configured to generate illumination and measure the resulting reflectance once every three seconds (i.e., potentially minimum rate to detect a 0.17 Hz signal), and preferably at a higher rate, as fast at 0.1 second or even faster. It is understood that the periodic interval between measurements may be adapted as needed based on the species of the subject, and the expected frequency of the peristaltic waves to be measured. Every time the ingestible device makes a new reflectance level measurement at 65904, the new data is included to the data set (e.g., a data set stored within memory circuitry of a PCB).

At 65906, the ingestible device obtains a first subset of recent data by applying a sliding window filter to the data set. For example, the ingestible device may retrieve a one-minute worth of data from the data set. If the data set includes values for reflectances measured every second, this would be approximately 60 data points worth of data. Any suitable type of window size may be used, provided that the size of the window is sufficiently large to detect peristaltic waves (e.g., fluctuations on the order of 0.05 Hz to 0.33 Hz for healthy human subjects). In some embodiments, the ingestible device may also clean the data, for example, by removing outliers from the first subset of data obtained through the use of the sliding window filter.

At 65908, the ingestible device obtains a second subset of recent data by interpolating the first subset of recent data. For example, the ingestible device may interpolate the first subset of data in order to generate a second subset of data with a sufficient number of data points (e.g., data points spaced every 0.5 seconds or greater). In some embodiments, this may enable the ingestible device to also replace any outlier data points that may have been removed as part of applying the window filter at 65906.

At 65910, the ingestible device calculates a normalized frequency spectrum from the second subset of data. For example, the ingestible device may be configured to perform a fast Fourier transform to convert the second subset of data from a time domain representation into a frequency domain representation. It is understood that depending on the application being used, and the nature of the subset of data, any number of suitable procedures (e.g., Fourier transform procedures) may be used to determine a frequency spectrum for the second subset of data. For example, the sampling frequency and size of the second subset of data may be known in advance, and the ingestible device may be configured to have pre-stored values of a normalized discreet Fourier transform (DFT) matrix, or the rows of the DFT matrix corresponding to the 0.05 Hz to 0.33 Hz frequency components of interest, within memory (e.g., memory circuitry of a PCB). In this case, the ingestible device may use matrix multiplication between the DFT matrix and the data set to generate an appropriate frequency spectrum. An example data set and corresponding frequency spectrum that may be obtained by the ingestible device is discussed in greater detail in relation to FIG. 65.

At 65912, the ingestible device determines whether at least a portion of the normalized frequency spectrum is between 00.05 Hz to 0.33 Hz above a threshold value of 0.5 Hz. Peristaltic waves in a healthy human subject occur at a rate between 0.05 Hz to 0.33 Hz, and an ingestible device experiencing peristaltic waves (e.g., an ingestible device detecting contractions in the walls of the jejunum) may detect sinusoidal variations in the amplitude of detected reflectances levels that follow a similar 0.05 Hz to 0.33 Hz frequency. If the ingestible device determines that a portion of the normalized frequency spectrum between 0.05 Hz to 0.33 Hz is above a threshold value of 0.5 Hz, this measurement may be consistent with peristaltic waves in a healthy human subject, and process 65900 proceeds to 65914 where the ingestible device stores data indicating a muscle contraction was detected. Alternatively, if the ingestible device determines that no portion of the normalized frequency spectrum between 0.05 Hz to 0.33 Hz above a threshold value of 0.5, process 65900 proceeds directly to 65904 to make new measurements and to continue to monitor for new muscle contractions. It is understood that a threshold value other than 0.5 may be used, and that the exact threshold may depend on the sampling frequency and type of frequency spectrum used by the ingestible device.

At 65914, the ingestible device stores data indicating a muscle contraction was detected. For example, the ingestible device may store data in memory (e.g., memory circuitry of a PCB) indicating that a muscle contraction was detected, and indicating the time that the muscle contraction was detected. In some embodiments, the ingestible device may also monitor the total number of muscle contractions detected, or the number of muscle contractions detected in a given time frame. In some embodiments, detecting a particular number of muscle contractions may be consistent with the ingestible device being within the jejunum) of a healthy human subject. After detecting a muscle contraction, process 65900 proceeds to 65916.

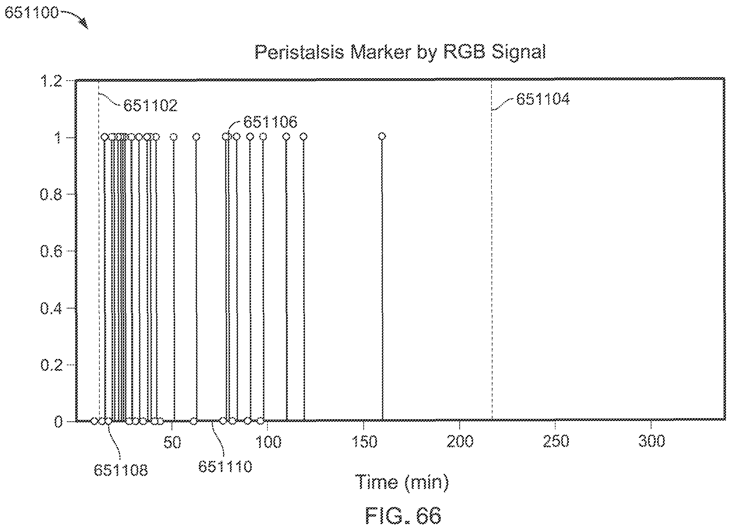

At 65916, the ingestible device determines whether a total number of muscle contractions exceeds a predetermined threshold number. For example, the ingestible device may retrieve the total number of muscle contractions detected from memory (e.g., from memory circuitry of a PCB), and compare the total number to a threshold value. In some embodiments, the threshold value may be one, or any number larger than one. The larger the threshold value, the more muscle contractions need to be detected before the ingestible device stores data indicating that it has entered the jejunum. In practice, setting the threshold value as three or higher may prevent the ingestible device from detecting false positives (e.g., due to natural movement of the GI tract organs, or due to movement of the subject). If the total number of contractions exceeds the predetermined threshold number, process 65900 proceeds to 65918 to store data indicating detection of a transition from the duodenum to the jejunum. Alternatively, if the total number of contractions does not exceed a predetermined threshold number, process 65900 proceeds to 65904 to include new measurements of reflectance levels in the data set. An example plot of the muscle contractions detected over time is discussed in greater detail in relation to FIG. 66.

At 65918, the ingestible device stores data indicating detection of a transition from the duodenum to the jejunum. For example, the ingestible device may store data in memory (e.g., from memory circuitry of a PCB) indicating that the jejunum has been reached. In some embodiments, if the ingestible device is configured to perform all or part of process 65900 while in the stomach, the ingestible device may store data at 65918 indicating detection of a transition from the stomach directly to the jejunum (e.g., as a result of transitioning too quickly through the duodenum for the pyloric transition to be detected using process 65600).

In some embodiments, the ingestible device may be configured to obtain a fluid sample from the environment external to a housing of the ingestible device in response to identifying a change in the location of the ingestible device. For example, the ingestible device may be configured to obtain a fluid sample from the environment external to the housing of the ingestible device (e.g., through the use of optional opening 65116 and optional rotating assembly 65118) in response to determining that the ingestible device is located within the jejunum. In some embodiments, the ingestible device may also be equipped with appropriate diagnostics to detect certain medical conditions based on the retrieved fluid sample, such as small intestinal bacterial overgrowth (SIBO).

In some embodiments, the ingestible device may be configured to deliver a dispensable substance that is pre-stored within the ingestible device from the ingestible device into the GI tract in response to identifying the change in the location of the ingestible device. For example, the ingestible device may have a dispensable substance pre-stored within the ingestible device (e.g., within a storage chamber or cavity on an optional storage sub-unitGI and the ingestible device may be configured to dispense the substance into the gastrointestinal tract (e.g., through the use of an optional opening and an optional rotating assembly) when the ingestible device detects that the ingestible device is located within the jejunum. In some embodiments, this may enable the ingestible device to deliver substances (e.g., therapeutics and medicaments) at targeted locations within the GI tract.

In some embodiments, the ingestible device may be configured to perform an action based on the total number of detected muscle contractions. For example, the ingestible device may be configured to retrieve data indicative of the total number of muscle contractions (e.g., from memory circuitry of a PCB), and compare that to an expected number of muscle contractions in a healthy individual. In response, the ingestible device may either dispense a substance into the GI tract (e.g., through the use of an optional opening and an optional rotating assembly), or may obtain a fluid sample from the environment external to the housing of the ingestible device (e.g., through the use of an optional opening and an optional rotating assembly). For instance, the ingestible device may be configured to obtain a sample in response to determining that a number of detected muscle contractions is abnormal, and differs greatly from the expected number. As another example, the ingestible device may be configured to deliver a substance into the GI tract (such as a medicament), in response to determining that the detected muscle contractions are consistent with a functioning GI tract in a healthy individual.

It will be understood that the steps and descriptions of the flowcharts of this disclosure are merely illustrative. Any of the steps and descriptions of the flowcharts may be modified, omitted, rearranged, and/or performed in alternate orders or in parallel, two or more of the steps may be combined, or any additional steps may be added, without departing from the scope of the present disclosure. For example, the ingestible device may calculate the mean and the standard deviation of multiple data sets in parallel (e.g., multiple data sets, each one corresponding to a different wavelength of reflectance or different sensing sub-unit used to detect the reflectance) in order to speed up the overall computation time. Furthermore, it should be noted that the steps and descriptions of FIG. 64 may be combined with any other system, device, or method described in this application, and any of the ingestible devices or systems discussed in this application could be used to perform one or more of the steps in FIG. 64.

FIG. 65 is a plot illustrating data collected during an example operation of an ingestible device, which may be used when detecting a transition from a duodenum to a jejunum, in accordance with some embodiments of the disclosure. Diagram 651000 depicts a time domain plot 651002 of a data set of reflectance levels measured by an ingestible device (e.g., the second subset of data discussed in relation to 65908). In some embodiments, the ingestible device may be configured to gather data points at semi-regular intervals approximately 0.5 seconds apart. By comparison, diagram 651050 depicts a frequency domain plot 651004 of the same data set of reflectance levels measured by an ingestible device (e.g., as a result of the ingestible device calculating a frequency spectrum at 65910). In some embodiments, the ingestible device may be configured to calculate the frequency spectrum through any convenient means.

In diagram 651050, the range of frequencies 651006 between 0.05 Hz to 0.33 Hz may be the range of frequencies that the ingestible device searches in order to detect muscle contractions. As shown in diagram 651050, there is a strong peak in the frequency domain plot 651004 around 0.14 Hz, which is consistent with the frequency of peristaltic motion in a healthy human individual. In this case, the ingestible device analyzing frequency domain plot 651004 may be configured to determine that the data is consistent with a detected muscle contraction (e.g., using a process similar to 65912 of process 65900), and may store data (e.g., in memory circuitry of a PCB) indicating that a muscle contraction has been detected. Because the muscle contraction was detected from the one-minute window of data ending at 118 minutes, the ingestible device may also store data indicating that the muscle contraction was detected at the 118-minute mark (i.e., which may indicate that the ingestible device was turned on and ingested by the subject 118 minutes ago).

FIG. 66 is a plot illustrating muscle contractions detected by an ingestible device over time, which may be used when determining a location of an ingestible device as it transits through a gastrointestinal (GI) tract, in accordance with some embodiments of the disclosure. In some embodiments, the ingestible device may be configured to detect muscle contractions, and store data indicative of when each muscle contraction is detected (e.g., as part of 65914 of process 65900). Plot 651100 depicts the detected muscle contractions 651106 over time, with each muscle contraction being represented by a vertical line reaching from "0" to "1" on the y-axis.

At 651102, around the 10-minute mark, the ingestible device first enters the duodenum (e.g., as determined by the ingestible device performing process 65600). Shortly thereafter, at 651108, the ingestible device begins to detect several muscle contractions 651106 in quick succession, which may be indicative of the strong peristaltic waves that form in the jejunum. Later, around 651110, the ingestible device continues to detect intermittent muscle contractions, which may be consistent with the ingestible device within the ileum. Finally, at 651104, the ingestible device transitions out of the small intestine, and into the cecum. Notably, the ingestible device detects more frequent muscle contractions in the jejunum portion of the small intestine as compared to the ileum portion of the small intestine, and the ingestible device does not measure any muscle contractions after having exited the small intestine. In some embodiments, the ingestible device may incorporate this information into a localization process. For example, the ingestible device may be configured to detect a transition from a jejunum to an ileum in response to determining that a frequency of detected muscle contractions (e.g., the number of muscle contractions measured in a given 10-minute window) has fallen below a threshold number. As another example, the ingestible device may be configured to detect a transition from an ileum to a cecum in response to determining that no muscle contractions have been detected for a threshold period of time. It is understood that these examples are intended to be illustrative, and not limiting, and that measurements of muscle contractions may be combined with any of the other processes, systems, or methods discussed in this disclosure.

FIG. 66 is a flowchart 651200 for certain embodiments for determining a transition of the device from the jejunum to the ileum. It is to be noted that, in general, the jejunum is redder and more vascular than the ileum. Moreover, generally, in compari son to the ileum, the jejunum has a thicker intestine wall with more mesentery fat. These differences between the jejunum and the ileum are expected to result in differences in optical responses in the jejunum relative to the ileum. Optionally, one or more optical signals may be used to investigate the differences in optical responses. For example, the process can include monitoring a change in optical response in reflected red light, blue light, green light, ratio of red light to green light, ratio of red light to blue light, and/or ratio of green light to blue light. In some embodiments, reflected red light is detected in the process.

Flowchart 651200 represents a single sliding window process. In step 651210, the jejunum reference signal is determined based on optical reflection. Typically, this signal is as the average signal (e.g., reflected red light) over a period of time since the device was determined to enter the jejunum. The period of time can be, for example, from five minutes to 40 minutes (e.g., from 10 minutes to 30 minutes, from 15 minutes to 25 minutes). In step 651220, the detected signal (e.g., reflected red light) just after the period of time used in step 651210 is normalized to the reference signal determined in step 651210. In step 651230, the signal (e.g., reflected red light) is detected. In step 651240, the mean signal detected based on the single sliding window is compared to a signal threshold. The signal threshold in step 651240 is generally a fraction of the reference signal of the jejunum reference signal determined in step 651210. For example, the signal threshold can be from 60% to 90% (e.g., from 70% to 80%) of the jejunum reference signal. If the mean signal exceeds the signal threshold, then the process determines that the device has entered the ileum at step 651250. If the mean signal does not exceed the signal threshold, then the process returns to step 651230.

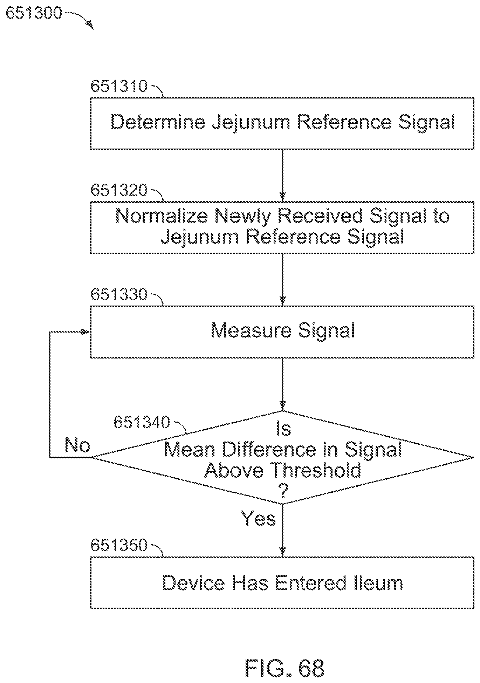

FIG. 68 is a flowchart 651200 for certain embodiments for determining a transition of the device from the jejunum to the ileum using a two sliding window process. In step 651310, the jejunum reference signal is determined based on optical reflection. Typically, this signal is as the average signal (e.g., reflected red light) over a period of time since the device was determined to enter the jejunum. The period of time can be, for example, from five minutes to 40 minutes (e.g., from 10 minutes to 30 minutes, from 15 minutes to 25 minutes). In step 651320, the detected signal (e.g., reflected red light) just after the period of time used in step 651310 is normalized to the reference signal determined in step 651310. In step 651330, the signal (e.g., reflected red light) is detected. In step 651340, the mean difference in the signal detected based on the two sliding windows is compared to a signal threshold. The signal threshold in step 651340 is based on whether the mean difference in the detected signal exceeds a multiple (e.g., from 1.5 times to five times, from two times to four times) of the detected signal of the first window. If signal threshold is exceeded, then the process determines that the device has entered the ileum at step 651350. If the signal threshold is not exceeded, then the process returns to step 651330.

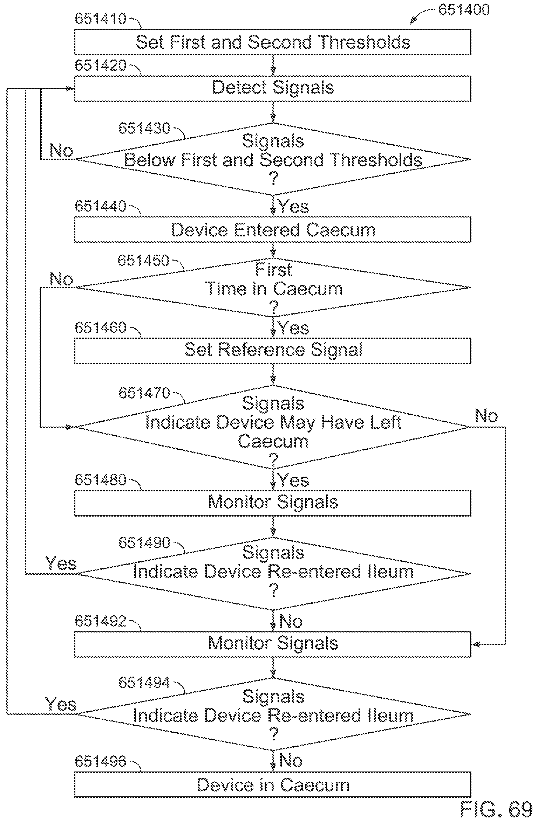

FIG. 69 is a flowchart 651400 for a process for certain embodiments for determining a transition of the device from the ileum to the cecum. In general, the process involves detecting changes in the reflected optical signal (e.g., red light, blue light, green light, ratio of red light to green light, ratio of red light to blue light, and/or ratio of green light to blue light). In some embodiments, the process includes detecting changes in the ratio of reflected red light to reflected green light, and also detecting changes in the ratio of reflected green light to reflected blue light. Generally, in the process 651400, the sliding window analysis (first and second windows) discussed with respect to process 65600 is continued.

Step 651410 includes setting a first threshold in a detected signal, e.g., ratio of detected red light to detected green light, and setting a second threshold for the coefficient of variation for a detected signal, e.g., the coefficient of variation for the ratio of detected green light to detected blue light. The first threshold can be set to a fraction (e.g., from 0.5 to 0.9, from 0.6 to 0.8) of the average signal (e.g., ratio of detected red light to detected green light) in the first window, or a fraction (e.g., from 0.4 to 0.8, from 0.5 to 0.7) of the mean difference between the detected signal (e.g., ratio of detected red light to detected green light) in the two windows. The second threshold can be set to 0.1 (e.g., 0.05, 0.02).

Step 651420 includes detecting the signals in the first and second windows that are to be used for comparing to the first and second thresholds.

Step 651430 includes comparing the detected signals to the first and second thresholds. If the corresponding value is not below the first threshold or the corresponding value is not below the second threshold, then it is determined that the device has not left the ileum and entered the cecum, and the process returns to step 651420. If the corresponding value is below the first threshold and the corresponding value is below the second threshold, then it is determined that the device has left the ileum and entered the cecum, and the proceeds to step 651440.

Step 651450 includes determining whether it is the first time that that the device was determined to leave the ileum and enter the cecum. If it is the first time that the device was determined to leave the ileum and enter the cecum, then the process proceeds to step 651460. If it is not the first time that the device has left the ileum and entered the cecum, then the process proceeds to step 651470.

Step 651460 includes setting a reference signal. In this step the optical signal (e.g., ratio of detected red light to detected green light) as a reference signal.

Step 651470 includes determining whether the device may have left the cecum and returned to the ileum. The device is determined to have left the cecum and returned to the ileum if the corresponding detected signal (e.g., ratio of detected red light to detected green light) is statistically comparable to the reference signal (determined in step 651460) and the coefficient of variation for the corresponding detected signal (e.g., ratio of detected green light to detected blue light) exceeds the second threshold. If it is determined that the device may have left the cecum and returned to the ileum, the process proceeds to step 651480.

Step 651480 includes continuing to detect the relevant optical signals for a period of time (e.g., at least one minute, from five minutes to 15 minutes).

Step 651490 includes determining whether the signals determined in step 651480 indicate (using the methodology discussed in step 651470) that the device re-entered the ileum. If the signals indicate that the device re-entered the ileum, the process proceeds to step 651420. If the signals indicate that the device is in the cecum, the process proceeds to step 651492.

Step 651492 includes continuing to monitor the relevant optical signals for a period of time (e.g., at least 30 minutes, at least one hour, at least two hours).

Step 651494 includes determining whether the signals determined in step 651492 indicate (using the methodology discussed in step 651470 ) that the device re-entered the ileum. If the signals indicate that the device re-entered the ileum, the process proceeds to step 651420. If the signals indicate that the device is in the cecum, the process proceeds to step 651496.

At step 651496, the process determines that the device is in the cecum.

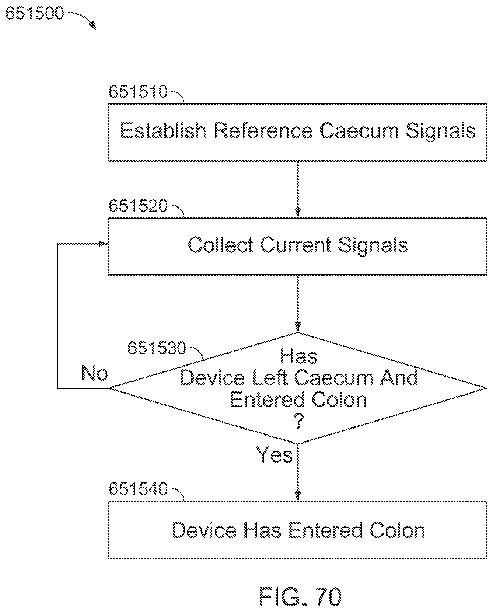

FIG. 70 is a flowchart 651500 for a process for certain embodiments for determining a transition of the device from the cecum to the colon. In general, the process involves detecting changes in the reflected optical signal (e.g., red light, blue light, green light, ratio of red light to green light, ratio of red light to blue light, and/or ratio of green light to blue light). In some embodiments, the process includes detecting changes in the ratio of reflected red light to reflected green light, and also detecting changes in the ratio of reflected blue light. Generally, in the process 651500, the sliding window analysis (first and second windows) discussed with respect to process 651400 is continued.

In step 651510, optical signals (e.g., the ratio of reflected red signal to reflected green signal, and reflected blue signal) are collected for a period of time (e.g., at least one minute, at least five minutes, at least 10 minutes) while the device is in the cecum (e.g., during step 651480). The average values for the recorded optical signals (e.g., the ratio of reflected red signal to reflected green signal, and reflected blue signal) establish the cecum reference signals.

In step 651520, the optical signals are detected after it has been determined that the device entered the cecum (e.g., at step 651440). The optical signals are normalized to the cecum reference signals.

Step 651530 involves determining whether the device has entered the colon. This includes determining whether any of three different criteria are satisfied. The first criterion is satisfied if the mean difference in the ratio of a detected optical signal (e.g., ratio of detected red signal to the detected green) is a multiple greater than one (e.g., 2X, 3X, 4X) the standard deviation of the corresponding signal (e.g., ratio of detected red signal to the detected green) in the second window. The second criterion is satisfied if the mean of a detected optical signal (e.g., a ratio of detected red light to detected green light) exceeds a given value (e.g., exceeds one). The third criterion is satisfied if the coefficient of variation of an optical signal (e.g., detected blue light) in the first window exceeds a given value (e.g., exceeds 0.2). If any of the three criteria are satisfied, then the process proceeds to step 651540. Otherwise, none of the three criteria are satisfied, the process returns to step 651520.

In some embodiments, the disclosure also provides an ingestible device configured to collect spectral data for one or more analytes (e.g., a macronutrient) present in the GI tract of a subject and/or to collect spectral data characterize one or more regions of the GI tract of a subject. In some embodiments, the ingestible device includes one more spectrometers configured to generate the spectral data. The disclosure also provides associated kits, methods and/or systems for characterizing the GI tract of a subject. Some embodiments are directed to an ingestible device which can produce spectral data of one or more analytes present in the GI tract. Certain embodiments are directed to associated methods for characterizing the GI tract of a subject which contains such analytes, as well as related kits and systems.

Generating spectral data at one or more wavelengths (e.g., pre-determined wavelengths) in accordance with the disclosure beneficially allows for the detection and/or quantitation, *in vivo,* of one or more particular analytes within a sample in the GI tract. For example, spectral data in the low NIR is suitable for detecting food and macronutrients. Spectral data at longer wavelengths, e.g., 2500-16000 nm, is suitable for dissolved gases (e.g., methane). In some embodiments, one or more of these observations is/are used to generate spectral data (e.g., hyperspectral data) for a sample. More generally, spectral data of a variety of different wavelengths can be used to analyze one or more analytes (e.g., one or more macronutrients) present in the GI tract. In some embodiments, spectral data is used to develop one or more digestion profiles of a subject, e.g., when the analyte(s) include one or more macronutrients. Generating spectral data at one or more pre-determined wavelengths allows for information to be obtained regarding the subject without necessarily identifying specific analytes. As an example, the spectral data gathered by the disclosed ingestible device is suitably used to search a database of spectral standards or digestion profiles, and it may be determined that similar spectral data is indicative of individuals with a vegetarian diet who have recently eaten a particular type of ingestible standard.

As used herein, the term "digestion profile" refers to spectral data from a subject and optionally information associated with the spectral data, associated with the sample, or associated with the subject. For example, in some embodiments, a digestion profile may include spectral data and information on the subject such as, but not limited to, weight, height, sex, diet, activity level, medical condition, medication, genotype, phenotype, BMI, race, age, exercise routine, heart rate, pulse, food ingested by the subject, and/or place of residence.

Hyperspectral imaging may be advantageously used in the methods and devices described herein as the additional spatial dimensions allow for images to be analyzed in order to identify non-homogeneous samples and/or obtain spectra from different areas of the image such as to focus on liquid sample or particular matter such as food particles, etc. The results of this analysis may be beneficially included in the digestion profile for a subject, or may be used in conjunction with a digestion profile to infer information about the subject or provide recommendations for the subject. For example, spectral data may be used in accordance with the disclosed technologies to detect the presence of a particular chemical or macronutrient present within the sample over time, which suitably indicates the subject's ability to absorb and/or metabolize the chemical or macronutrient from the sample as it transits through the GI tract of the subject. More generally, hyperspectral imaging may be used for a broad range of analytes to be analyzed at multiple time points and/or at multiple locations, for example, throughout the GI tract.

An ingestible standard (e.g., a set meal or a predetermined composition of one or more macronutrients) may be used with the disclosed ingestible device in order to generate spectral data that is associated with the ingestible standard. One advantage of using an ingestible standard is to facilitate the comparison of spectral data and/or digestion profiles from the same individual or from different individuals. For example, the ingestible standard serves as a control and differences in the spectral data and/or digestion profiles for a subject having ingested the same ingestible standard may be ascribed to changes in the physiology of the GI tract rather than differences in the ingested material. As another example, the use of an ingestible standard also allows for the identification of individuals with similar GI tract characteristics based on similarities in their spectral data. Information on those individuals may then be beneficially used to inform or predict the characteristics of a subject.

The disclosed technologies can be used to produce a digestion profile indicative of the relative or total amount of one or more analytes in one or more samples. For example, the digestion profile indicates to a user the relative concentration of macronutrients in the stomach of the subject, or the total amount in grams of fat in the intestine of the subject. Data indicative of the presence of one or more analytes is gathered by the disclosed ingestible device at several points in time, and is advantageously used to determine how effectively the subject digests absorbs and/or metabolizes particular analytes. As an example, if the level of a particular macronutrient detected in the sample is reduced over time, it may indicate that the subject has absorbed and/or metabolized the macronutrient. As another example, if the level of a particular macronutrient detected in the sample is reduced more quickly over time compared to that of one or more other macronutrients, then the subject may have a higher rate of absorption of the particular macronutrient relative to the one or more other macronutrients (the subject may absorb and/or metabolize the particular macronutrient faster than the one or more other macronutrients).

Spectral data associated with an ingestible standard for a subject may be used in accordance with the disclosed technologies to predict the absorption of calories or macronutrients by the subject for the substance, or whether the substance may be beneficial or detrimental to a criteria selected by a user such as wanting to lose weight, gain weight, lose body fat, gain body fat, lose muscle mass, gain muscle mass, manage a medical condition, treat a medical condition, increase absorption of a macronutrient, decrease absorption of a macronutrient, absorb carbohydrates (e.g., carb loading for sports performance) or gain lean muscle. For example, a subject's digestion profile may indicate that they are able to only partially digest certain types of vegetables, and this information may be used to infer how many calories and macronutrients the subject will actually absorb if they consume one of those types of vegetables.

A digestion profile may include spectral data and information associated with the spectral data, associated with the sample, or associated with the subject. For example, a digestion profile may include spectral data and location data identifying where in the GI tract the spectral data was generated. Optionally a digestion profile may include environmental data and one or more user inputs. User inputs may be information on the subject and/or criteria selected by a user such as an analyte, medical condition, a desired outcome, or information about the sample. For example, the digestion profile may include user-inputted information about a meal or ingestible standard that the subject consumed concurrently with the ingestible device, as well as general demographic information about the subject.

A digestion profile may be used to search a database of spectral standards and/or other digestion profiles in order to identify similar digestion profiles and/or generate additional information regarding the sample and/or subject. For example, information indicating that the sample was a particular ingestible standard and general demographic information for the subject may be obtained from the subject's digestion profile. This may be used to search a digestion profile database to identify digestion profiles for individuals with similar demographics, or digestion profiles where the sample was the same ingestible standard as the one consumed by the subject. As another example, spectral data from a digestion profile may be used to search for digestion profiles or spectral standards containing similar spectral data. Any suitable type of signal processing technique may be used to identify similarities between spectral data, and any suitable type of information may be generated based on the digestion profiles or spectral standards identified in the database. For example, this information may include average digestion time for a given sample, average number of macronutrients or calories absorbed from a given sample, level of variation between a subject's spectral data and typical spectral data available in the database, and the like. This additional information may be presented to a user and/or be incorporated into the digestion profile of the subject. For example, information may be stored in the digestion profile for a subject indicating that the subject's ability to absorb nutrients from a particular ingestible standard is superior to 90% of individuals of a similar age and weight. Accordingly, a plurality of different digestion profiles may be generated for a subject, including the same or different spectral data associated with the same or different information associated with the spectral data, information associated with the sample, or information associated with the subject. In some embodiments, the plurality of digestion profiles are obtained from the same subject at different time frames (e.g., prior to treatment with a specific regimen (e.g., a therapeutic regimen or surgical intervention) and may be used to assess the efficacy of a particular regimen.

In some embodiments, one or more of the analytes can be used to develop a digestion profile of a subject. In general, a digestion profile includes longitudinal data for the subject over time, such as a subject's daily caloric intake and fluctuations in weight. In some embodiments, a digestion profile may include spectral data and information indicative of one or more analytes in the sample. In some embodiments, a digestion profile may include spectral data and information indicative of the characteristics of the sample without necessarily identifying any analytes. In some embodiments, the information associated with the spectral data or associated with the subject includes criteria selected by a user, such as the identity of one or more analytes, medical conditions, or a desired outcome. Information associated with the spectral data may include the location within the GI tract where the spectral data was generated, environmental data from the GI tract, or the identity of an ingestible standard ingested by the subject. In some embodiments, a digestion profile may further include one or more desired outcomes or goals for the subject. In some embodiments, all or part of a digestion profile for a subject may be used to search a database of digestion profiles and identify subjects or groups of subjects with similar digestion profiles. Optionally, information obtained from identifying subjects or groups of subjects with similar digestion profiles may then be added to the digestion profile for the subject. A digestion profile may also include lifestyle and/or diet recommendations for the subject. In some embodiments, a digestion profile may include diet recommendations regarding food choices, time of day to eat and/or frequency of eating. In some embodiments, a digestion profile is predictive of the effect of ingesting one or more substances by the subject.

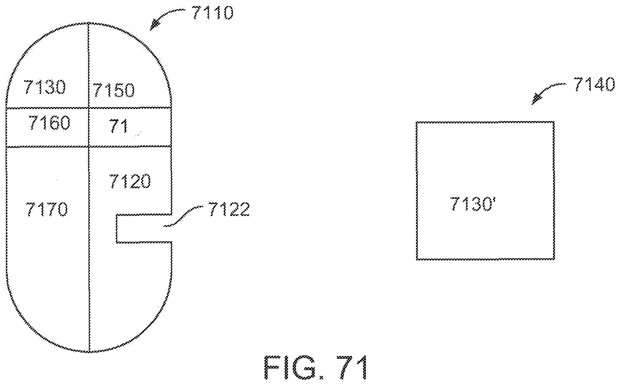

Referring to FIG. 71, there is shown an ingestible device 7110 including spectrometer 7120 operable to generate spectral data of a sample within the GI tract of a subject *in vivo.* Alternatively or additionally, in some embodiments, the ingestible device may be used to obtain spectral data relating to the tissue lining of the GI tract of a subject. Optionally, ingestible device 7110 may include communications unit 7130, processing unit 7150, memory 7160, one or more environmental sensors 7170, and/or microcontroller 7180. Optionally, ingestible device 7110 may include a detection chamber 7122 for containing a sample from the GI tract of the subject. In general, detection chamber 7122 may be exposed to the exterior of the device or contained within the device. In some embodiments, one or more ports, valves, pumps and/or conduits provide fluid communication between a detection chamber 7122 contained within the ingestible device 7110 and the exterior of ingestible device 7110. In general, a sample (e.g., a sample from the GI tract of the subject) is collected in the detection chamber 7122, and the spectrometer 7120 is used to generate spectral data for the sample.

Optionally, the spectrometer 7120 can be designed to generate spectral data of one or more samples external to the ingestible device 7110. As an example, the spectrometer 7120 can be designed to generate spectral data of one or more regions of the GI tract of the subject (e.g., one or more regions of tissue of the GI tract of the subject). In embodiments in which the spectrometer 7120 is designed to generate spectral data of one or more samples external to the ingestible device 7110, the ingestible device 7110 may or may not include the detection chamber 7122.

Base station 7140 contains a communications unit 7130' for communicating with the ingestible device 7120. In general, communications units 7130 and 7130' may exchange data through any suitable wired or wireless communication scheme, including radio communications (RF), WiFi communications, Bluetooth^{™} communications, universal serial bus (USB) communications, infrared or near infrared communications, acoustic signaling, and the like. In some embodiments, the ingestible device 7120 may be recovered after travelling though the GI tract of the subject, and the ingestible device 7120 exchanges data with the base station 7140 via the communications units 7130 and 7130' after the ingestible device 7120 has been recovered. In some embodiments, the ingestible device 7120 communicates with the base station 7140 while transiting the GI tract of the subject, and the base station 7140 received data either continuously or at predetermined intervals. For example, the ingestible device 7120 may be configured to transmit information to the base station 7140 every minute, or after reaching certain predetermined locations within the GI tract of the subject.

Communications unit 7130 may transmit spectral data and/or environmental data to base station 7140 located ex vivo that contains a corresponding communications unit 7130'. Base station 7140 may be any electronic device configured to communicate with ingestible device 7110 such as, but not limited to, a computer, or a personal electronic device such as a watch, physical activity tracker, fitness monitor, personal digital assistant (PDA), phone or tablet. Other examples of electronic devices that may be configured to communicate with ingestible device 7110 and serve as base station 7140 include a "smart" home appliance that is able to connect to a network such as the internet. In some embodiments, base station 7140 may be a home appliance connected to a network such as home speakers, a fridge or other kitchen appliance, a toilet and/or a scale, such as a bathroom scale. In some embodiments, the base station and/or ingestible device are connected to a network such as the internet. For example, base station 7140 may include network circuitry for communicating over an internet connection with a database of digestion profiles and/or spectral standards stored on a remote server. In some embodiments, base station 7140 may include a display unit for presenting information to a user. For example, base station 7140 may include a display for presenting information about the subject's digestion profile. In some embodiments, base station 7140 may include a user input interface for receiving user inputs. For example, base station 7140 may include a keyboard, mouse, touch-screen screen enabled display, or other suitable user interface.

Ingestible device 7110 and/or base station 7140 may include memory 7160 for storing the spectral data, environmental data and/or digestion profiles. For example, base station 7140 may store multiple sets of spectral data gathered from multiple ingestible devices (e.g., multiple instances of ingestible device 7110) in memory 7160, which may or may not be shared as part of public database of digestion profiles. In some embodiments, memory 7160 may also include operational parameters for controlling ingestible device 7110. In general, memory 7160 may be any form of computer-readable memory suitable for storing and retrieving data such as semiconductor memory or a secondary storage device such as a hard disk drive or solid state drive. In some embodiments, ingestible device 7110 and/or base station 7140 are connected to networked storage suitable for storing the spectral data, environmental data and/or digestion profiles for a subject. In general, this networked storage may include a searchable database of digestion profiles. For example, spectral data may be transmitted from the ingestible device 7110 to the base station 7140, the base station 7140 may use the spectral data to generate a digestion profile for the subject, and the base station 7140 may communicate the digestion profile to a remote server to be added to a digestion profile database.

In some embodiments, spectrometer 7120 is operable to generate spectral data for a sample within the GI tract of a subject. The spectral data may be intensity spectral data, absorbance spectral data, transmission spectral data, reflectance spectral data, fluorescence spectral data, Fourier transform spectral data and/or Raman spectral data. Optionally, ingestible device 7110 may include a plurality of spectrometers for generating different types of spectral data. In some embodiments, the spectrometer 7120 may be configured to gather spectral data from a portion of the sample within the GI tract of the subject surrounding the enclosure of the ingestible device. In some embodiments, where the ingestible device 7110 includes a detection chamber 7122, the spectrometer 7120 may be configured to gather spectral data for a sample contained with the detection chamber 7122. In certain embodiments, spectrometer 20 is operable to generate spectral data of a sample external to ingestible device 7110 (e.g., via a hyperspectral camera as the spectrometer). In such embodiments, ingestible device 7110 may or may not include detection chamber 7122.

Different types of spectrometers known in the art may be used to generate the spectral data. In some embodiments, spectrometer 7120 includes a light source and a photodetector. For example, the spectrometer 7120 may include a source for generating light in the near-infrared spectrum, and an accompanying photodetector sensitive to the near-infrared spectrum. In some embodiments, spectrometer 7120 further includes one or more of a dispersive element, lens and/or filter. In some embodiments, spectrometer 7120 includes one or more lenses, fisheye lenses, filters, color filters, polarization filters, optical filters, beam splitters, interferometers, tunable filters or spectral reformers.

In some embodiments, the spectrometer 7120 includes elements that are suitable for miniaturization and/or use on an ingestible device. In some embodiments, the spectrometer 7120 includes a tunable interferometer such as a Fabry-Perot interferometer (FPI). In some embodiments, the spectrometer 7120 includes a micro-electro-mechanical system (MEMS). Elements suitable for use in the ingestible device 7110 include FPI interferometers and other elements available from Hamamatsu Photonics K.K. and FPI tunable wavelength filters available from VTT Technical Research Centre of Finland Ltd.

In some embodiments, the light source is a lamp, such as a tungsten or xenon lamp, light emitting diode (LED), tunable LED or laser. In some embodiments, the light source produces light at one or more wavelengths in the Ultraviolet, Visible, Near-infrared and/or Mid-infrared. In some embodiments, the light source produces a continuous output and/or a pulsed output.

In some embodiments, the photodetector includes one or more photodiodes. Photodiodes may be made out of materials known in the art such as, but not limited to, silicon, germanium, indium gallium arsenide, lead (II) sulfide and/or mercury cadmium telluride. In some embodiments, the photodetector detects light at one or more wavelengths in the Ultraviolet, Visible, Near-infrared and/or Mid-infrared.

In some embodiments, the spectrometer 7120 includes two or more photodetectors configured to detect spectral data at different wavelengths and/or for detecting different analytes. For example, in some embodiments, the spectrometer 7120 may include a plurality of photodetectors configured to detect light or spectra at different wavelengths associated with the detection of different analytes, such as proteins, fats and/or carbohydrates. Optionally, the spectrometer 20 includes an array of photodetectors.

In some embodiments, the photodetector is a 2-dimensional array, optionally a two-dimensional array of photodiodes. For example, in some embodiments, the photodetector is a 40x40 array with 1600 pixels. Larger or smaller arrays may also be used depending on the resolution desired for hyperspectral data. Examples of two-dimensional arrays that may be used in an ingestible device as described herein include smaller arrays with low power consumption and a spectral range suitable for detection of light across wavelengths in one or more of UV, VIS, NIR and/or MIR. Accordingly, in some embodiments the spectral data generated using an ingestible device described herein (e.g., the ingestible device 7110) is hyperspectral data. NIR hyperspectral imaging of biological materials is described in greater detail in Manley, Near-infrared spectroscopy and hyperspectral imaging: non-destructive analysis of biological materials Chem. Soc. Rev., 2014, 43, 8200.

Hyperspectral imaging may be advantageously used in the methods and devices described herein as the additional spatial dimensions may allow for images to be analyzed in order to identify non-homogeneous samples and/or obtain spectra from different areas of the image such as to focus on liquid sample or particular matter such as food particles, etc. The results of this analysis may be included in the digestion profile for a subject, or may be used in conjunction with a digestion profile to infer information about the subject or provide recommendations for the subject. For example, spectral data may be used to detect the presence of a particular chemical or macronutrient present within the sample over time, which may indicate the subject's ability to absorb the chemical or macronutrient from the sample as it transits through the GI tract of the subject.

In some embodiments, spectrometer 7120 generates spectral data in the Near Infrared (NIR) spectrum. For example, in some embodiments, the spectral data includes intensity, absorbance, reflectance, transmission or fluorescence at one or more wavelengths between about 600 nm and about 2600 nm, between about 600 nm and about 1500 nm or between about 800 nm and about 2000 nm. In some embodiments, spectrometer 7120 generates spectral data in the mid-infrared (MIR) spectrum. For example, in some embodiments, the spectral data includes intensity, absorbance, reflectance, transmission or fluorescence at one or more wavelengths between about 1500 nm and about 5600 nm, between about 2000 nm and about 4000 nm, or between about about 2500 nm and about 5000 nm. The spectrometer 7120 may also generate spectral data that overlaps two or more areas of the electromagnetic spectrum. For example, in some embodiments, the spectral data may include wavelengths from the NIR and MIR, such as wavelengths between about 1000 nm and 2500 nm. In some embodiments, the spectral data may include wavelengths from the VIS and NIR, such as between about 500 nm and 1500 nm. In one aspect of the disclosure, generating spectral data at one or more predetermined wavelengths allows for the detection of particular analytes within the sample. For example, spectral data in the low NIR such as 700-1100 nm may be suitable for detecting for food and macronutrients. Spectral data may also be used for detecting the presence of liquid water as well. For example, in the NIR range liquid water has absorption bands around 1950 nm, 1450 nm, 1200 nm and 970 nm. In some embodiments, generating spectral data at one or more pre-determined wavelengths allows for information to be obtained regarding the sample and/or subject without necessarily identifying specific analytes. For example, the spectral data gathered by an ingestible device 7110 may be used to search a database of spectral standards or digestion profiles (e.g., via an Internet connected base station 7140), and it may be determined that similar spectral data is indicative of individuals with a vegetarian diet who have recently eaten a particular type of ingestible standard.

Spectral data generated using an ingestible device as described herein may be analyzed in order to extract information from complex biological samples in the GI tract of a subject. For example, the analysis of spectral data of biological samples to determine various characteristics such as the relative level of macronutrients, gross energy content and/or utilizable energy content are disclosed in Fusch et al., "Rapid measurement of macronutrients in breast milk: How reliable are infrared milk analyzers?" Clinical Nutrition 34 (2015) 465-476; Manley, "Near-infrared spectroscopy and hyperspectral imaging: non-destructive analysis of biological materials" Chem. Soc. Rev., 2014, 43, 8200; Szigedi et al., "Fourier Transform Near-Infrared Spectroscopy to Predict the Gross Energy Content of Food Grade Legumes" Food Anal. Methods (2013) 6:1205-1211; Kays and Barton, "Rapid Prediction of Gross Energy and Utilizable Energy in Cereal Food Products Using Near-Infrared Reflectance Spectroscopy" J. Agric. Food Chem. 2002, 50, 1284-1289.

Spectrometer 7120 may optionally include detection chamber 7122. In some embodiments, spectrometer 20 includes a light source and a photodetector defining a light path through detection chamber 7122. For example, a light source and a photodetector may be positioned on either side of the detection chamber 7122 such that light emitted from the light source passes through the detection chamber 7122 before being detected by the protodetector. In general, detection chamber 7122 may be used to contain a sample from the GI tract of the subject in order to generate spectral data of the sample *in vivo.*

In some embodiments, detection chamber 7122 is exposed to the exterior of device 7110. In operation, fluid from the GI tract may enter into the detection chamber of ingestible device 7110 *in vivo* through surface tension, movement of the subject and/or peristaltic effects. In some embodiments, the detection chamber may be coated with a hydrophilic coating to encourage fluid to flow into the detection chamber. In some embodiments, detection chamber 22 is formed by a depression along the exterior surface of the enclosure of ingestible device 10. In some embodiments, ingestible device 7110 may include a cover (not shown) movable to expose detection chamber 7122 to the exterior of the device.

In some embodiments, detection chamber 7122 may be located within device 7110. A fluid sample from the GI tract of the subject may be actively or passively brought into the detection chamber in order to generate spectral data of the sample. For example, in some embodiments, device 7110 includes one or more ports, valves, pumps and/or conduits for controlling the transfer of the fluid sample from the GI tract into the detection chamber.

In some embodiments, spectrometer 7120 includes a light source and a photodetector positioned on the exterior of ingestible device 7110, or positioned behind the enclosure of ingestible device 7110 and directed towards the exterior of ingestible device 7110 (e.g., spectrometer 7120 includes a hyperspectral camera). In some embodiments, the light source is configured to transmit light radially towards the environment external to the device and the photodetector is configured to detect a reflectance from the environment external to the device. In some embodiments, the light source is adjacent to the photodetector on the exterior of device 10. In some embodiments, spectrometer 7120 is configured to generate reflectance spectral data. In some embodiments, the spectrometer 7120 is positioned either fully or partially behind a material that is transparent to one or more spectra of light, such as optically transparent plastic. For example, the spectrometer 7120 may include a light source and photodetector that operate in the Ultraviolet, Visible, Near-infrared and/or Mid-infrared, and the light source and/or the photodetector may be placed behind a portion of the device enclosure that is formed from a material that is transparent to light in the Ultraviolet, Visible, Near-infrared and/or Mid-infrared spectra. In some embodiments, the spectrometer 7120 may be positioned either fully or partially behind one or more lenses. For example, the photodetector of spectrometer 7120 may be positioned behind a lens configured to focus light onto the surface of the photodetector. In some embodiments, the spectrometer 7120 may include a light source positioned behind a lens configured to collimate the light into a beam. The collimated beam of light may be directed into a detection chamber 7120, and oriented in the direction of one or more photodetectors positioned on the other side of the detection chamber. In some embodiments, the collimated beam of light may be configured to travel along a pre-defined light path through the detection chamber. In embodiments described in this paragraph, ingestible device 7110 may or may not include detection chamber 7122.

Generally, ingestible device 7110 may have one or more of the various features of ingestible device described throughout this disclosure. As an example, ingestible device 7110 may include one or more additional environmental sensors. As another example, ingestible device 7110 may include or more connectors (e.g., one or more hooks) to connect to tissue (e.g., tissue of the GI tract).

In some embodiments, ingestible device 7110 includes processing unit 7150 configured to generate a digestion profile for the subject based on the spectral data and optionally environmental data and/or one or more inputs. Alternatively, processing unit 7150 may be located within base station 7140 located *ex vivo.* In some embodiments, ingestible device 7110 and/or base station 7140 are configured to transmit spectral data, and optionally environmental data and one or more inputs, to a server and receive information such as a digestion profile from the server. For example, spectral data and unit inputs may be transmitted to the server by the base station 7140, and the server may generate base station 7140, which may be a computer or a personal electronic device such as a watch, phone, tablet or physical activity tracker or fitness monitor. In some embodiments, base station 7140 may be a stand-alone chip or set of circuitry that may be incorporated into a computer, or other personal electronic device.

In embodiments in which it is desirable to use the spectral data to a digestion profile for a subject, the digestion profile for the subject may be generated by comparing spectral data for a sample from the GI tract of the subject to one or more spectral standards and/or spectra contained in other digestion profiles. For example, information associated with a sample may be obtained by comparing the spectral data gathered by the ingestible device 7110 with one or more spectral standards representative of typical spectral data gathered under known conditions. For example, a particular spectral standard may represent the typical spectral data that would be observed in a healthy individual from a partially digested ingestible standard with half of the nutrients remaining. If the spectral data gathered by the ingestible device is similar to a particular spectral standard, information about the spectral standard may then be incorporated into the digestion profile for the subject. For example, if the spectral data is similar to spectral standards gathered from individuals who are diabetic, the digestion profile for the subject may indicate that the subject has produced similar spectral data to diabetic individuals, and therefore may aid in determining whether the subject is at risk of developing or has diabetes.

In general, in embodiments in which it is desirable to us the generated spectral to determine the presence and/or amount of an analyte, a spectral standard may represent typical spectral data gathered from a sample comprising an analyte or from a subject having an analyte in the subject's GI tract. In some embodiments, the spectral standard may represent typical spectral data gathered from a subject having an analyte bound to a detection agent in the subject's GI tract and/or spectral data gathered from a sample comprising an analyte bound to a detection agent (e.g., a detection agent comprising a fluorescent probe). In some embodiments, the spectral standard may represent typical spectral data gathered from individuals having a particular condition (e.g., a disease or disorder described herein). In some embodiments, spectral standards may represent typical spectral data gathered from a sample having a predetermined level of an analyte and/or a predetermined level of an analyte bound to a detection agent. These standards may be used in the methods described herein to compare spectral data gathered by ingestible device 7110 with the spectral data of the spectral standards in order to detect, quantify and/or analyze a sample obtained from the GI tract of a subject or the GI tract of the subject. For example, in some embodiments, the methods described herein comprise comparing the spectral data gathered by the ingestible device with the spectral data of a spectral standard to determine whether a particular analyte or a combination of analytes is present in the GI tract of a subject and/or a sample from the GI tract of the subject. In some embodiments, the methods described herein comprise comparing the spectral data gathered by the ingestible device with the spectral data of a spectral standard to determine a level of analyte present in the GI tract of a subject and/or GI tract of the subject. In some embodiments, the methods described herein comprising comparing the spectral data gathered by the ingestible device with the spectral data of a spectral standard to determine whether the subject has or is at risk of developing a disease or disorder.

Spectral standards may be stored in a database accessible to either the ingestible device 7110 or the base station 7140, and the ingestible device 7110 or the base station 7140 may use any suitable criteria for identifying spectral standards in the spectral standards database or selecting one or more spectral standards in the spectral standards database to compare against the spectral data gathered by the ingestible device 7110.

In some embodiments, spectral standards and/or spectra contained in other digestion profiles are selected for comparison in order to match the sample type used to generate the spectral data. For example, if the spectral data gathered by the ingestible device 10 was obtained from a particular type of sample (e.g., a particular pre-determined ingestible standard), the spectral data may be compared to spectral standards that represent typical spectral data gathered for that same type of sample. This may control for any differences or similarities between the spectral data and the spectral standards, which may be due to differences between the different samples used to generate the respective data, and may be used to generate a more accurate digestion profile indicative of information associated with the spectral data or the subject.

In some embodiments, spectral standards and/or spectra contained in other digestion profiles are selected for comparison in order to match the region in the GI tract where the spectral data was generated for the subject with spectral standards that are representative of spectral data for samples gathered from the same region in the GI tract (e.g., one or more of the stomach, proximal and distal duodenum, jejunum, ileum, descending colon, ascending colon and transverse colon). In some embodiments, a digestion profile is generated by comparing the spectral data to spectral standards that are gender-matched, age-matched, and/or matched for the presence or absence of a particular condition.

In some embodiments, the spectral standards and/or spectra contained in other digestion profiles are selected for comparison in order to match the subject used to generate the spectral data. For example, the gathered spectral data may be compared to spectra obtained from the subject at an earlier time point. Accordingly, the methods and devices described herein may be used for monitoring the GI tract of a subject over time. For example, in some embodiments, this comparative data may be used to detect changes in how the subject absorbs nutrients or digests particular ingestible standards over time. In some embodiments, the comparative data may be used to analyze the progression of a disease or disorder (e.g., a GI disease or disorder). In some embodiments, the comparative data may be used to determine the effectiveness of a particular course of treatment.

In some embodiments, the spectral standards are spectra associated with digestion profiles for individuals or groups of individuals in a database of digestion profiles. For example, the spectral standards may be a set of spectra in a database of digestion profiles associated with a particular ingestible standard, analyte, desired outcome, or medical condition (e.g., a disease or disorder described herein). In one aspect, digestion profiles of subjects generated using the ingestible device described herein are saved in a database of digestion profiles. The database of digestion profiles containing spectral data and information regarding other individuals or groups of individuals, such as, but not limited to, medical conditions, analytes, food sensitivities, desired outcomes, or the effect of ingesting a substance, and can then be used to inform the digestion profile of a subject by comparing the digestion profile of the subject to the digestion profiles contained in the database.

Spectral standards may also be generated that are associated with a particular condition such as the level of one or more analytes or a desired outcome. For example, spectral standards representative of the level of a particular analyte may be generated using a benchtop spectrometer and a GI tract model such as the TNO Gastro-Intestinal Model (TIM) described in Mans Minekus, Chapter 5: The TNO Gastro-Intestinal Model (TIM) in K. Verhoeckx et al. (eds.), The Impact of Food Bio-Actives on Gut Health, Springer International Publishing (2015).

Spectral standards may also be generated by preparing samples *in vitro* that simulate samples of digested or partially digested food and spectra for each sample may be generated ex vivo. Base samples may include, for example, a predetermined amount of macronutrients or an ingestible standard. Various components such as saliva, gastric acid, pancreatic enzymes, bile and/or bacteria may be added to the base samples in order to generate samples that reflect various regions of the GI tract.

Spectral data may be compared to spectral standards or other spectral data using various techniques known in the art. For example, the intensity and/or location of peaks or troughs in the spectra may be compared. In some embodiments, spectral data may be compared using algorithms or statistical methodologies for quantifying the difference or similarity between two or more spectra. Optionally, the spectra data may be pre-processed prior to comparing the spectra or sets of spectra. For example, in some embodiments, machine-learning, genetic algorithms, multivariate data analysis, chemometric methods, pattern recognition methods and/or or principle component analysis (PCA) may be used for comparing spectra or a set of spectra. In some embodiments, an unsupervised classification method is used to compare spectra or set of spectra such as PCA. In another embodiment, a supervised classification method is used such as soft independent modelling of class analogy (SIMCA), linear discriminant analysis (LDA), multiple discriminant analysis (MDA), factorial discriminant analysis (FDA), PLS discriminant analysis (PLS-DA), canonical variate analysis (CVA), artificial neural networks (ANNs) and/or k-nearest neighbor (k-NN) analysis. Hyperspectral data may be compared using Multivariate Image Analysis (MIA) techniques. Various mathematical techniques and methods for comparing and analyzing spectral data including hyperspectral data are described in Manley, "Near-infrared spectroscopy and hyperspectral imaging: non-destructive analysis of biological materials" Chem. Soc. Rev., 2014, 43, 8200.

In some embodiments, processing unit 7150 is configured to generate desired data (e.g., a digestion profile) based on the spectral data and one or more inputs. The inputs may provide additional information regarding the sample and/or the subject, or allow the digestion profile to provide information that is indicative of a particular characteristic such as, but not limited to, a medical condition, analyte, food or drug sensitivity, effectiveness of a treatment regimen (e.g., with a therapeutic agent), desired outcome, or the effect of ingesting a substance. For example, spectral data gathered by the ingestible device 7110 may be transmitted to base station 40, where a processing unit 7150 located within the base station 7140 combines the spectral data with input from the user in order to generate the desired data (e.g., a full digestion profile). While certain examples of spectral data and spectral standards are discussed in this paragraph, the disclosure is not limited in this sense. Any spectral data, or combination of spectral data, as well as corresponding spectral standards, can likewise be implemented.

The one or more inputs may include information on the subject or criteria selected by a user. In some embodiments, the one or more inputs are entered or selected by a subject using a computer interface, which then configures processing unit 7150 to generate desired data (e.g., a digestion profile) based on the one or more inputs. For example, a user may select criteria using a computer interface and/or display located on base station 7140. While certain examples of spectral data and spectral standards are discussed in this paragraph, the disclosure is not limited in this sense. Any spectral data, or combination of spectral data, as well as corresponding spectral standards, can likewise be implemented.

In some embodiments, the information on the subject includes at least one of weight, height, sex, diet, exercise, medical condition (e.g., a disease or disorder described herein), medication, genotype, phenotype, BMI, race, age, exercise routine, tobacco use, and/or alcohol use, heart rate, pulse and place of residence. Examples of medical conditions include, cancer (e.g., gastric cancer or colorectal cancer), metabolic disease, prediabetes, diabetes, irritable bowel syndrome (IBS), inflammatory bowel disease, short bowel syndrome, malabsorption syndromes such as carbohydrate malabsorption or bile acid malabsorption and/or other bile acid diseases, pancreatic insufficiency/chronic pancreatitis (i.e., inability to digest fat in small intestine), nutritional insufficiencies linked to old age, an allergy (e.g., a food allergy), kidney disease, epilepsy, protein malnutrition such as from lysinuric protein intolerance or low gastric acid in stomach (hypochlorhydria), gastroparesis (slow gastric emptying) and chronic constipation. For example, the methods and devices described herein may be useful for monitoring a ketogenic diet to help control seizures in subjects with epilepsy. In some embodiments, the methods and devices described herein are useful for detecting blood and/or bile in the GI tract of a subject. In some embodiments, a bile acid metabolism profile may be generated. Bile acid metabolism profiles may be indicative of the presence or absence of C. *difficile.* A bile acid metabolism profile of a subject may be generated to determine the presence or absence of C. *difficile,* for example, after a fecal microbiota transplant or treatment with an antibiotic. In certain embodiments, one or more intra-colonic bile acid profiles may be used to predict the success of fecal microbiota transplantation (FMT).

In some embodiments, the methods and devices described herein are useful for monitoring subjects following a "Fermentable, Oligo-, Di-, Mono-saccharides And Polyols" (FODMAP) diet for managing Irritable Bowel Syndrome (IBS). For example, the ingestible device and methods described herein may be used to determine the absorption of short-chain carbohydrates such as fructose, lactose, polyols, fructans, and galacto-oligosaccharides within the GI tract of a subject with IBS. Diets low in FODMAP have been shown to be effective at managing symptoms in subjects with IBS (see, for example, Halmos et al. Gastroenterology 2014; 146:67-75, Gibson and Shepherd, Journal of Gastroenterology and Hepatology 25 (2010) 252-258).

For example, in some embodiments, a user may specify that the subject is a female subject 35 years of age with type II diabetes by providing user input to the base station 7140. Processing unit 7150 may then be configured to generate a digestion profile based on spectral data gathered by the ingestible device 7110 using a given ingestible standard and the information that the subject is a 35-year-old female with type II diabetes. This digestion profile may then be stored in a digestion profile database, may be used to search for similar digestion profiles, may be used to provide dietary or lifestyle recommendations for the subject, or may be used for any other suitable purpose.

In some embodiments, a digestion profile may be generated based on one or more criteria selected by a user. For example, a user may select criteria indicative of a particular medical condition, analyte, food sensitivity, desired outcome, or the effect of ingesting a substance. In some embodiments, the desired outcome is to lose weight, gain weight, lose body fat, gain body fat, lose muscle mass, manage a medical condition (e.g., a disease or disorder), treat a medical condition, or increase or decrease the absorption of a macronutrient. Processing unit 50 may then be configured to generate a digestion profile based on spectral data and the criteria selected by the user. For example, a user may input criteria identifying a subset of medical conditions of the subject, different types on ingestible standards that will be consumed concurrently with the ingestible device 7110, or personal subject goals for the subject. These criteria may be used along with the spectral data to generate a digestion profile, and may be used to identify a subset of individuals or groups of individuals in a database of digestion profiles. The spectral data for a subject may then be compared to the spectral data for the selected subset of individuals or groups of individuals.

In some embodiments, information regarding the individuals or groups of individuals with similar digestion profiles is added to the digestion profile for the subject. For example, this information may be used to recommend foods that the subject should avoid or foods that the subject should consume in order to obtain a desired outcome. For example, if the subject desires to lose weight, the digestion profile database may be searched for profiles of subjects of the same sex, age, and/or having the same medical condition who have lost weight. The identified digestion profiles may contain information about typical foods consumed by subjects falling within the searched demographic group who have lost weight, and this information may be presented to the user (e.g., using a display located on base station 7140) in order to recommend foods that the subject should consume in order for the subject to achieve weight loss. In some embodiments, processing unit 7150 is configured to transmit the spectral data to a server and receive the digestion profile from the server. For example, the server may also store previously stored user input, and combine the user input with the spectral data to create the digestion profile. Optionally, processing unit 7150 is configured to transmit the spectral data, environmental data and/or one or more inputs to a server and receive the digestion profile from the server.

While the discussion in this section of the disclosure has provided examples of data, as well as the utilization and manipulation thereof, this section of the disclosure is not limited in this sense. Any spectral data, or combination of spectral data, as well as corresponding spectral standards, can likewise be implemented.

In some embodiments, a digestion profile generated using the methods and/or devices described herein is indicative of calories intake and/or absorbed by the subject. In another embodiment, a digestion profile is indicative of the weight or mass in grams of one or more analytes ingested and/or absorbed by the subject. In some embodiments, the methods and devices described herein are useful for tracking whether the substances ingested by a subject fall within the parameters of a specific diet, such as the total number of calories consumed, metabolized and/or absorbed by a subject, or the relative amount of calories from different macronutrients such as proteins, carbohydrates and fats consumed, metabolized and/or absorbed by the subject.

In some embodiments, the methods and/or devices described herein may provide a user information such as lifestyle and/or dietary recommendations for a subject. For example, if the spectral data gathered by the ingestible device 7110 for a given subject indicates that the subject is unable to digest a particular macronutrient efficiently, a digestion profile may be generated that recommends that the subject supplement their diet with foods that have higher concentrations of that macronutrient. In some embodiments, processing unit 7150 is configured to generate a digestion profile that is indicative of lifestyle and/or diet recommendations for a subject based on outcomes associated with certain lifestyles (such as an exercise routine) and/or diets in the database of digestion profiles. For example, if the subject desires to gain weight and/or increase muscle mass, the user may search a digestion profile database for profiles of subjects with similar demographics to obtain spectral data of subjects who have successfully gained weight and/or increased muscle mass. Information from the identified digestion profiles, such as common foods consumed by individuals who have successfully gained weight and/or increased muscle mass, may then be incorporated into the digestion profile for the subject as a dietary recommendation. It will be understood that any other suitable type of information contained in the identified digestion profiles, such as typical caloric intake and/or typical exercise regimens for individuals associated with the identified digestion profiles, may be presented to the subject as well.

In some embodiments, there is provided methods and systems for predicting the effect of ingesting a substance (e.g., an analyte) by a subject. In some embodiments, the method includes identifying a substance to be ingested and predicting the effect of ingesting the substance based on a digestion profile for the subject as described herein. For example, a user may identify a given substance (e.g., a particular pre-packaged meal) by providing user input to the base station 40, and the digestion profile for the subject may be used to predict how the subject will digest, absorb and/or metabolize the substance. In some embodiments, the effect of ingesting the substance includes the absorption of calories or macronutrients in the GI tract of the subject. For example, the digestion profile for the subject may be used to predict how efficiently the subject will absorb and/or metabolized an analyte (e.g., the fat, protein, and carbohydrates present in the substance). In some embodiments, the method includes obtaining nutritional data on the substance (e.g., a food stuff) and predicting the effect of ingesting the substance based on the digestion profile for the subject and the nutritional data. For example, nutritional data about the various amounts of fat, protein, and carbohydrates within the substance may be found on a central database or repository, and the subject's digestion profile may be used to estimate how much of the fat, protein, and carbohydrates contained in the substance will actually be absorbed by the subject. Optionally, the method includes predicting the effect of ingesting the substance based on a digestion profile for the subject and database of digestion profiles and/or standard spectra as described herein. For example, the amount of an analyte (e.g., fat, protein, and carbohydrates) absorbed from a given substance may be predicted using empirical data from digestion profiles similar to the subject's digestion profile. For instance, if the other digestion profiles indicate that individuals typically absorb half of the macronutrients in a given substance (e.g., a food stuff), it may be inferred that the subject will absorb approximately half the macronutrients in a given sample on average. The information in the other digestion profiles may also be used to generate a range of possible outcomes to the subject. For example, if the digestion profiles on the database indicate that there is a wide variance in the amount of macronutrients absorbed from a given substance, this information may be used to predict a possible range of outcomes for the subject. While certain examples of spectral data spectral standards are discussed in this paragraph, the disclosure is not limited in this sense. Any spectral data, or combination of spectral data, as well as corresponding spectral standards, can likewise be implemented as discussed herein.

In some embodiments, spectral data obtained using a spectrometer is transmitted to a processing unit (which may be external to the ingestible device, e.g., outside the subject's body). Optionally, environmental data obtained from an environmental sensor is transmitted to the processing unit. A processing unit is configured by a user using interface and/or display (e.g., as described elsewhere herein) by inputting information on the subject and/or selecting criteria for generating a digestion profile that is, e.g., indicative of a particular analyte or predictive of the effect of ingesting a particular substance or food. The processing unit generates a digestion profile by transmitting spectral data and information inputted by the user to a server. The server contains a searchable database of digestion profiles and/or standard spectra. Information based on comparing the spectral data to the database of digestion profiles and/or standard spectra is then presented to the user. In some embodiments, the information is displayed to the user on a display. Optionally, the server may provide the information based on the digestion profile to a user in the form of an electronic communication such as email or text. In some embodiments, the digestion profile for the subject may be added to the searchable database of digestion profiles stored on the server. While certain examples of spectral data spectral standards are discussed in this paragraph, the disclosure is not limited in this sense. Any spectral data, or combination of spectral data, as well as corresponding spectral standards, can likewise be implemented as discussed herein.

In some embodiments, an ingestible device includes a portion (e.g., a portion of the housing through which a sample is to be collected, and/or a container for delivering one or more substances) that is electrolytically erodible for forming opening in the ingestible device (e.g., at a desired location in the gastrointestinal tract). The ingestible device can then absorb a sample (e.g., using a construction, such as one including one or more absorptive materials, disclosed elsewhere herein) and/or to deliver a substance (e.g., as described elsewhere herein). The device includes an exposed metal surface that acts as a valve to open to its surrounding environment. The exposed metal anode material acting as valve can include a metal alloy or substantially pure metal that is acceptable for human ingestion from consideration of its biocompatibility in the amounts electrolyzed during opening of the valve. A wide variety of stainless steel including SAE grades 303, 304, 304L, 316, 316L, 440 may be used, for example. From consideration of nickel content, purity, and/or traceability, it may be desirable to choose from stainless steel grades approved for use as surgical implant materials including ASTM grades F138, F1314, F1586, F2229, or F2581. If the exposed area of metal is small, a variety of other materials of construction may be used that may have advantages for manufacturability such as nickel, cobalt nickel alloy, or plain steel.

Optionally, the exposed metal surface can be quite small relative to the total surface area of the device. At the time of valve actuation, the metal portion o is biased with a positive voltage relative to a metal cathode element. The bias is provided by the batteries (power supply). In the case of an external electrolytic circuit (electrolytically erodible surface being on the exterior of the device), the surrounding gastric fluids are the electrolyte that completes an electrolytic circuit between anode and cathode. With sufficient bias voltage (e.g., 1.5-15 volts, such as 3-5 volts), the anode will dissolve or erode electrolytically and thus form an opening to its surrounding environment within a desired time interval. The appropriate bias voltage depends upon the chosen anode and cathode materials, cathode area relative to anode area, proximity of the cathode to anode, and desired performance. Increased voltage typically increases the rate of metal erosion unless bubbles are created that insulate the exposed metal from surrounding electrolyte. The voltage is chosen to be high enough such that electrolytic erosion occurs within an acceptable time without being so high that excessive bubbling occurs at the anode. Pulsing the DC voltage applied between anode and cathode can help reduce the amount of bubbling at the anode and make the electrolytic erosion process faster and/or more reliable. In the case of using pulsed DC voltage, frequency of pulsing can be in the range 5 to 500 hz, with 50 hz found experimentally to give good results. Duty cycle of the pulsed DC voltage relates to the percentage of time that the voltage is applied between anode and cathode, with the remainder of the time the applied voltage is at or near zero. An effective duty cycle for the purpose of making the electrolytic erosion process faster and/or more reliable is in the range 20% or 80% with 50% found experimentally to give good results.

Keeping the area of exposed metal small can reduce the volume of metal to be electrolyzed, which can be desirable (e.g., in terms of biological exposure to the patient), and can reduce the total current required to open the valve. In some embodiments, the area is from 0.01 mm² to 0.20 mm². As an example, the area of the exposed metal can be 0.03 mm², and/or the metal portion can have a diameter of 8.5 mm and a length of 14 mm. Other dimensions for the area of exposed metal that can be used are disclosed elsewhere herein.

Optionally, a nonconductive coating can be present on the metal. Exemplary nonconductive coating materials include polyimide, poly(p-xylylene) polymer (trade name Parylene), polyurethane, PDMS (polydimethylsiloxane) or other silicone, polyester, polyamide, epoxy, Teflon or other fluoropolymer, polyethylene, polypropylene, acrylate polymer, polyvinylchloride, polyethylene vinyl acetate.

In some embodiments, external portions of the ingestible device are covered by one or more coatings that are sensitive to the environment. For example, some polymer coatings (e.g., enteric coatings) are pH-sensitive and respond by degrading at a selected pH. Thus, incorporating an environmentally-sensitive material to the device allows for an event (e.g., erode the valve, or actuate a force for dispensing a substance or collecting a sample) to be triggered once the ingestible device has encountered a target condition or a target location *in vivo.* In some embodiments a threshold sensor on the outside of the device could be present so that polymer erosion is sensed by a controlling electronics system which then starts the electrolysis circuit. In certain embodiments, a polymer piece is present between two electrodes with mechanical bias like a spring or shape memory so that, as the polymer erodes, the electrodes come into contact completing the electrolysis circuit. Such an approach can be implemented with reduced electronics (e.g., without a microcontroller). For example, the power could be provided by a battery or another power source with a relatively small volume.

In some embodiments, the ingestible device can include one or more coatings (e.g., one or more hydrophilic coatings) that can enhance wetting of the exterior of the device and therefore assist in completing the circuit (e.g., when fluid of the GI tract is the electrolytic fluid).

It can be desirable to have the thickness of metal in the valve area be small (e.g., to reduce the time and amount of current used to open the valve). For example, the metal portion can be 0.025 mm thick across a diameter of 0.60 mm in the vicinity of the valve. In general, the thickness of the metal in the valve area can be in the range 0.002 mm to 0.200 mm. Other dimensions for the thickness of exposed metal that can be used are disclosed elsewhere herein.

For illustrative purposes the disclosure focuses primarily on a number of different example embodiments of an ingestible device, and example embodiments of methods for determining a location of an ingestible device within a GI tract. However, the possible ingestible devices that may be constructed are not limited to these embodiments, and variations in the shape and design may be made without significantly changing the functions and operations of the device. Similarly, the possible procedures for determining a location of the ingestible device within the GI tract are not limited to the specific procedures and embodiments discussed (e.g., process 65500, process 65600, process 65900, process 651200, process 651300, process 651400 and process 651500). Also, the applications of the ingestible devices described herein are not limited merely to gathering data, sampling and testing portions of the GI tract, or delivering medicament. For example, in some embodiments the ingestible device may be adapted to include a number of chemical, electrical, or optical diagnostics for diagnosing a number of diseases. Similarly, a number of different sensors for measuring bodily phenomenon or other physiological qualities may be included on the ingestible device. For example, the ingestible device may be adapted to measure elevated levels of certain chemical compounds or impurities in the GI tract, or the combination of localization, sampling, and appropriate diagnostic and assay techniques incorporated into a sampling chamber may be particularly well suited to determine the presence of small intestinal bacterial overgrowth (SIBO).

At least some of the elements of the various embodiments of the ingestible device described herein that are implemented via software (e.g., software executed by control circuitry within a PCB 65120) may be written in a high-level procedural language such as object oriented programming, a scripting language or both. Accordingly, the program code may be written in C, C⁺⁺ or any other suitable programming language and may include modules or classes, as is known to those skilled in object oriented programming. Alternatively, or in addition, at least some of the elements of the embodiments of the ingestible device described herein that are implemented via software may be written in assembly language, machine language or firmware as needed. In either case, the language may be a compiled or an interpreted language.

At least some of the program code used to implement the ingestible device can be stored on a storage media or on a computer readable medium that is readable by a general or special purpose programmable computing device having a processor, an operating system and the associated hardware and software to implement the functionality of at least one of the embodiments described herein. The program code, when read by the computing device, configures the computing device to operate in a new, specific and predefined manner in order to perform at least one of the methods described herein.

Furthermore, at least some of the programs associated with the systems, devices, and methods of the example embodiments described herein are capable of being distributed in a computer program product including a computer readable medium that bears computer usable instructions for one or more processors. The medium may be provided in various forms, including non-transitory forms such as, but not limited to, one or more discettes, compact discs, tapes, chips, and magnetic and electronic storage. In some embodiments, the medium may be transitory in nature such as, but not limited to, wire-line transmissions, satellite transmissions, internet transmissions (e.g. downloads), media, digital and analog signals, and the like. The computer useable instructions may also be in various formats, including compiled and non-compiled code.

The techniques described above can be implemented using software for execution on a computer. For instance, the software forms procedures in one or more computer programs that execute on one or more programmed or programmable computer systems (which may be of various architectures such as distributed, client/server, or grid) each including at least one processor, at least one data storage system (including volatile and non-volatile memory and/or storage elements), at least one input device or port, and at least one output device or port.

The software may be provided on a storage medium, such as a CD-ROM, readable by a general or special purpose programmable computer or delivered (encoded in a propagated signal) over a communication medium of a network to the computer where it is executed. All of the functions may be performed on a special purpose computer, or using special-purpose hardware, such as coprocessors. The software may be implemented in a distributed manner in which different parts of the computation specified by the software are performed by different computers. Each such computer program is preferably stored on or downloaded to a storage media or device (e.g., solid state memory or media, or magnetic or optical media) readable by a general or special purpose programmable computer, for configuring and operating the computer when the storage media or device is read by the computer system to perform the procedures described herein. The inventive system may also be considered to be implemented as a computer-readable storage medium, configured with a computer program, where the storage medium so configured causes a computer system to operate in a specific and predefined manner to perform the functions described herein.

For illustrative purposes the examples given herein focus primarily on a number of different example embodiments of an ingestible device. However, the possible ingestible devices that may be constructed are not limited to these embodiments, and variations in the general shape and design may be made without significantly changing the functions and operations of the device. For example, some embodiments of the ingestible device may feature a sampling chamber substantially towards the middle of the device, along with two sets of axial sensing sub-units, each located on substantially opposite ends of the device. In addition, the applications of the ingestible device are not limited merely to gathering data, sampling and testing portions of the GI tract, or delivering medicament. For example, in some embodiments the ingestible device may be adapted to include a number of chemical, electrical, or optical diagnostics for diagnosing a number of diseases. Similarly, a number of different sensors for measuring bodily phenomenon or other physiological qualities may be included on the ingestible device. For example, the ingestible device may be adapted to measure elevated levels of certain analytes, chemical compounds or impurities in the GI tract, or the combination of localization, sampling, and appropriate diagnostic and assay techniques incorporated into a sampling chamber may be particularly well suited to determine the presence of small intestinal bacterial overgrowth (SIBO). It is also noted that although embodiments described herein focus on an ingestible device in the GI tract, such ingestible device described in FIGs. 1-34 may be used for delivering substances including medicaments and therapeutics in other parts of the body.

The various embodiments of systems, processes and apparatuses have been described herein by way of example only. It is contemplated that the features and limitations described in any one embodiment may be applied to any other embodiment herein, and flowcharts or examples relating to one embodiment may be combined with any other embodiment in a suitable manner, done in different orders, or done in parallel. It should be noted, the systems and/or methods described above may be applied to, or used in accordance with, other systems and/or methods. Various modifications and variations may be made to these example embodiments. The appended embodiments should be given the broadest interpretation consistent with the description as a whole.

Implementations of the subject matter and the operations described in this specification can be implemented by digital electronic circuitry, or via computer software, firmware, or hardware, including the structures disclosed in this specification and their structural equivalents, or in combinations of one or more of them. Implementations of the subject matter described in this specification can be implemented as one or more computer programs, i.e., one or more modules of computer program instructions, encoded on computer storage medium for execution by, or to control the operation of, data processing apparatus.

A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, discs, or other storage devices).

The operations described in this specification can be implemented as operations performed by a data processing apparatus on data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., a FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto optical discs, or optical discs. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic discs, e.g., internal hard discs or removable discs; magneto optical discs; and CD ROM and DVD-ROM discs. The processor and the memory can be supplemented by, or incorporated in, special purpose logic circuitry.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's user device in response to requests received from the web browser.

Implementations of the subject matter described in this specification can be implemented in a computing system that includes a back end component, e.g., as a data server, or that includes a middleware component, e.g., an application server, or that includes a front end component, e.g., a user computer having a graphical display or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include users and servers. A user and server are generally remote from each other and typically interact through a communication network. The relationship of user and server arises by virtue of computer programs running on the respective computers and having a user-server relationship to each other. In some implementations, a server transmits data (e.g., an HTML page) to a user device (e.g., for purposes of displaying data to and receiving user input from a user interacting with the user device). Data generated at the user device (e.g., a result of the user interaction) can be received from the user device at the server.

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable sub combination. Moreover, although features may be described above as acting in certain combinations and even initially claimed as such, one or more features from a claimed combination can in some cases be excised from the combination, and the claimed combination may be directed to a sub combination or variation of a sub combination.

For the purpose of this disclosure, the term "coupled" means the joining of two members directly or indirectly to one another. Such joining may be stationary or moveable in nature. Such joining may be achieved with the two members or the two members and any additional intermediate members being integrally formed as a single unitary body with one another or with the two members or the two members and any additional intermediate members being attached to one another. Such joining may be permanent in nature or may be removable or releasable in nature.

It should be noted that the orientation of various elements may differ according to other exemplary implementations, and that such variations are intended to be encompassed by the present disclosure. It is recognized that features of the disclosed implementations can be incorporated into other disclosed implementations.

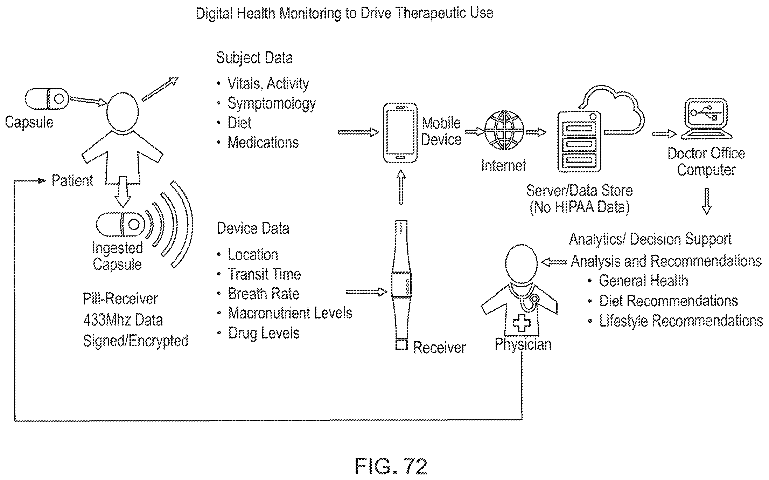

FIG. 72 illustrates a nonlimiting example of a system for collecting, communicating and/or analyzing data about a subject, using an ingestible device as disclosed herein. For example, an ingestible device may be configured to communicate with an external base station. As an example, an ingestible device can have a communications unit that communicates with an external base station which itself has a communications unit. FIG. 72 illustrates exemplary implementation of such an ingestible device. As shown in FIG. 72, a subject ingests an ingestible device as disclosed herein. Certain data about the subject (e.g., based on a collected sample) and/or the location of the ingestible device in the GI tract of the subject is collected or otherwise available and provided to a mobile device, which then forwards the data via the internet and a server/data store to a physician's office computer. The information collected by the ingestible device is communicated to a receiver, such as, for example, a watch or other object worn by the subject. The information is then communicated from the receiver to the mobile device which then forwards the data via the internet and a server/data store to a physician's office computer. The physician is then able to analyze some or all of the data about the subject to provide recommendations, such as, for example, general health recommendations, dietary health recommendations and/or lifestyle recommendations. While FIG. 72 shows a particular approach to collecting and transferring data about a subject, the disclosure is not limited. As an example, one or more of the receiver, mobile device, internet, and/or server/data store can be excluded from the data communication channel. For example, a mobile device can be used as the receiver of the device data, e.g., by using a dongle. In such embodiments, the item worn by the subject need not be part of the communication chain. As another example, one or more of the items in the data communication channel can be replaced with an alternative item. For example, rather than be provided to a physician's office computer, data may be provided to a service provider network, such as a hospital network, an HMO network, or the like. In some embodiments, subject data may be collected and/or stored in one location (e.g., a server/data store) while device data may be collected and/or stored in a different location (e.g., a different server/data store).

An ingestible device may include one or more environmental sensors. Environmental sensor may be used to generate environmental data for the environment external to device in the GI tract of the subject. Environmental data may be used to further characterize the GI tract of the subject either alone or in combination with the spectral data. In some embodiments, environmental data is generated at the same location within the GI tract of the subject where a sample is procured. Examples of environmental sensor include, but are not limited to a capacitance sensor, a temperature sensor, an impedance sensor, a pH level sensor, a heart rate sensor, acoustic sensor, image sensor, and/or a movement sensor. In some embodiments, the ingestible device includes a plurality of different environmental sensors for generating different kinds of environmental data. In some embodiments, the image sensor is a video camera suitable for obtaining images *in vivo* of the tissues forming the GI tract of the subject. In some embodiments, the environmental data is used to help determine one or more characteristics of the GI tract the subject such as for the diagnosis of a medical condition. In some embodiments, the ingestible device may include a camera for generating video imaging data of the GI tract which can be used to determine, among other things, the location of the device. Examples of video imaging capsules include Medtronic's PillCam^{™}, Olympus' Endocapsule^{®}, and IntroMedic's MicroCam^{™} (see Basar et al. "Ingestible Wireless Capsule Technology: A Review of Development and Future Indication" International Journal of Antennas and Propagation (2012); 1-14). Other imaging technologies include thermal imaging cameras, and those that employ ultrasound or Doppler principles to generate different images (see Chinese patent disclosure CN104473611: "Capsule endoscope system having ultrasonic positioning function"). In another embodiment, the ingestible device described herein may be localized using a gamma scintigraphy technique or other radio-tracker technology as employed by Phaeton Research's Enterion^{™} capsule (See Teng, Renli, and Juan Maya. "Absolute bioavailability and regional absorption of ticagrelor in healthy volunteers." Journal of Drug Assessment 3.1 (2014):43-50), or monitoring the magnetic field strength of permanent magnet in the ingestible device (see T. D. Than, et al., "A review of localization systems for robotic endoscopic capsules," IEEE Trans. Biomed. Eng., vol. 59, no. 9, pp. 2387-2399, Sep. 2012). In some embodiments, the one or more environmental sensors measure pH, temperature, transit times, or combinations thereof. Examples of devices useful to detect pH changes include Medimetrics' IntelliCap^{®} technology (see Becker, Dieter, et al. "Novel orally swallowable IntelliCap® device to quantify regional drug absorption in human GI tract using diltiazem as model drug." AAPS PharmSciTech 15.6 (2014):1490-1497) and Rani Therapeutics' Auto-Pill^{™} technology (see U.S. Patent 9,149,617).

### Systems and Methods For Extracting a Sample From an Ingestible Device

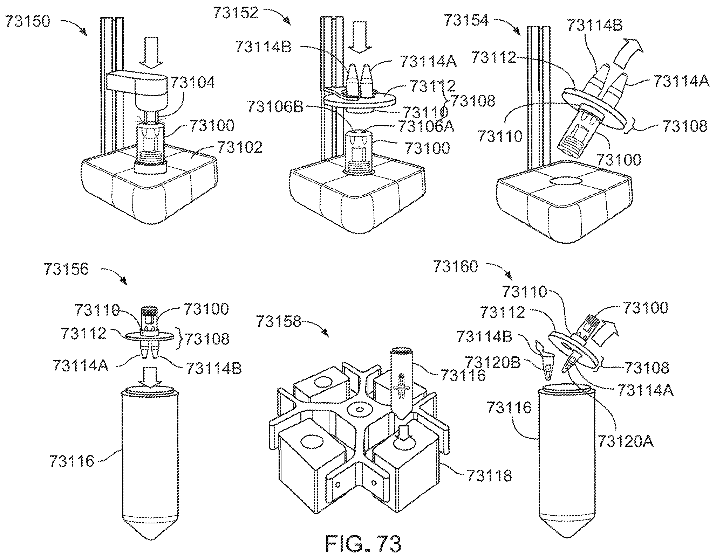

FIG. 73 shows an illustrative embodiment of a method for extracting a sample from an ingestible device. The method includes causing an opening to be created in the housing of the ingestible device. An example of creating an opening is shown in diagram 73150. The method includes inserting the ingestible device at least partially into a fitted sleeve that connects the opening in the housing of the ingestible device to a tube. An example of this process is shown in diagrams 73152 and 73154. The method also includes centrifuging the ingestible device, while the ingestible device is at least partially inserted into the fitted sleeve, to transfer at least a portion of the sample contained in the sampling chamber into the tube. An example of a centrifuging process is shown in diagrams 73156, 73158, and 73160.

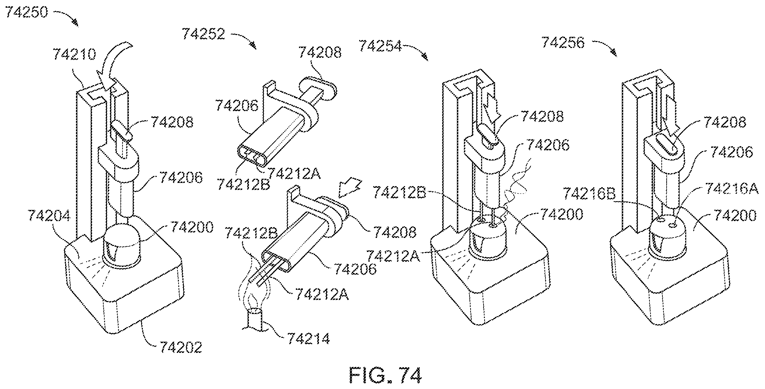

As shown in diagram 73150, an opening is created in the housing of the ingestible device 73100. The ingestible device 73100 is placed into a holder 73102, and a lancing mechanism 73104 including two lancets is used to pierce the housing of the ingestible device and create two openings. Other examples of methods and systems for creating an opening in an ingestible device are discussed in relation to FIG. 74, FIG. 75, and FIG. 76.

In general, ingestible devices may contain one or more sampling chambers, and each of the created openings may connect directly to one or more of the sampling chambers. In the example shown in diagram 73150, the ingestible device 73100 has sampling chambers located near the top of the device, which is where the two openings are created by the lancing mechanism 73104. However, it is possible for any number of openings to be connected to a single sampling chamber, and it may also be possible for each opening to be connected to different sampling chambers.

The lancing mechanism 73104 is depicted in diagram 73150 as having two lancets and creates two openings in the housing of the ingestible device 73100. In general, the lancing mechanism 73104 may include any number of lancets, where each lancet may be used to create an opening in the housing. Moreover, the lancing mechanism 73104 may include one or more lancets that are not used to create an opening in one ingestible device, but are used to create one or more openings in another, larger ingestible device with a larger number of sampling chambers, for example.

As shown in diagram 73152, the ingestible device 73100 is at least partially inserted into a sleeve device 73108. After the openings 73106A and 73106B (generally, openings 73106) are created in the ingestible device 73100, the sleeve device 73108 is connected to the ingestible device 73100. The sleeve device 73108 includes a sleeve portion 73110, and a base portion 73112. The sleeve portion 73110 may be sized and shaped to fit snugly around a portion of the ingestible device, and the base portion 73112 may be sized and shaped to connect the openings 73106A and 73106B to the tubes 73114A and 73114B respectively.

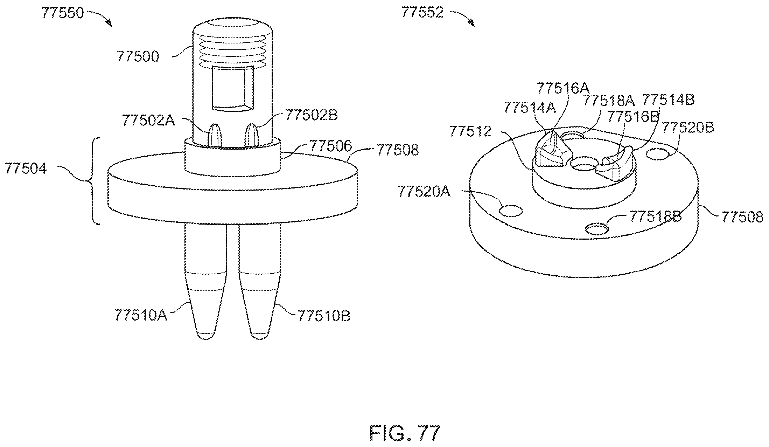

Diagram 73154 shows removing the ingestible device 73100 from the holder 73102 after the ingestible device 73100 is connected to the sleeve device 73108. In general, the sleeve portion 73110 of the sleeve device 73108 may fit snugly around the ingestible device 73100, and form a seal around a portion of the ingestible device 73100. The base portion 73112 of the sleeve device 73108 may form a seal around each of the openings in the ingestible device 73100, connecting each of the openings directly to one of the tubes 73114A or 73114B. An example of a sleeve device 73108, and how the connection to the tubes 73114A and 73114B may be created, is discussed in detail in relation to FIG. 77.

As shown in diagrams 73156, 73158, and 73160, the ingestible device is centrifuged. The centrifuging occurs while the ingestible device is at least partially inserted into the fitted sleeve and is performed in order to transfer at least a portion of the sample contained in the sampling chamber into the tube. Diagram 73156 shows the ingestible device 73100 being inserted into a centrifuge tube 73116 while the ingestible device 73100 is connected to the tubes 73114A and 73114B via the sleeve device 73108. Generally, the ingestible device 73100 and the sleeve device 73108 may be placed within the centrifuge tube 73116. The tubes 73114A and 73114B may be oriented near the bottom of the centrifuge tube 73116, and the ingestible device 73100 may be oriented near the top of the centrifuge tube 73116. This may ensure that centrifugal forces transfer a portion of the sample contained within the ingestible device 73100 into the tubes 73114A and 73114B.

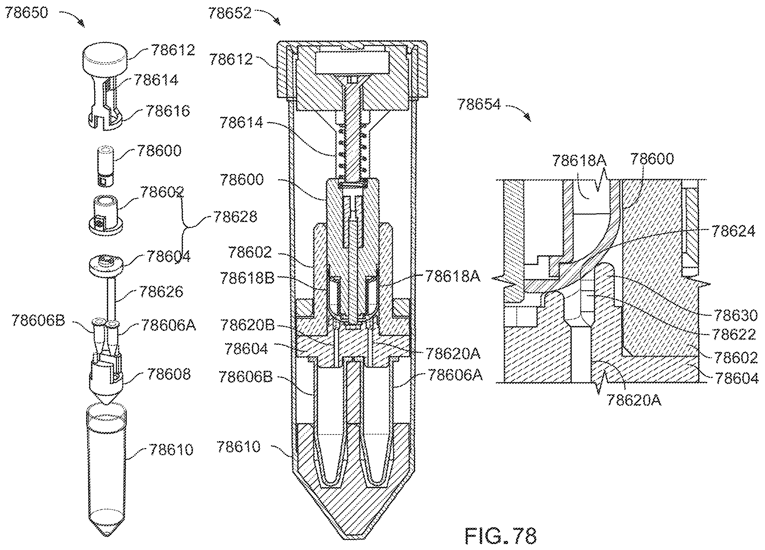

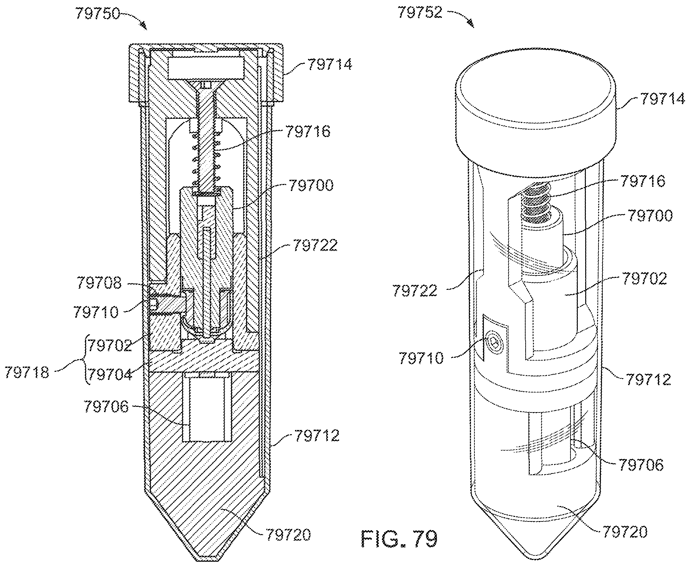

The centrifuge tube 73116 may be a commercially available centrifuge tube, such as a Falcon^{™} brand 50mL conical centrifuge tube. However, in some embodiments, the centrifuge tube 73116 is modified in order to better secure the ingestible device 73100 and the sleeve device 73108 within the centrifuge tube 73116. Examples of optional centrifuge mechanisms, which may allow the sleeve device 73108 and the ingestible device 73100 to be secured within the centrifuge tube 73116, are discussed in relation to FIG. 78 and FIG. 79.

Diagram 73158 shows the centrifuge tube 73116 (which contains the ingestible device 73100, the sleeve device 73108, and the tubes 73114A and 73114B) being inserted into a centrifuge 73118. After the centrifuge tube 73116 is inserted into the centrifuge 118, the centrifuge 73118 may be turned on, thereby centrifuging the ingestible device 73100. It will be understood that the speed and length of time that the ingestible device 73100 is centrifuged may depend on any number of factors, such as the physical properties of the ingestible device 73100, the properties of the sample contained within the ingestible device 73100, and the amount of the sample to be extracted from within the ingestible device 73100. However, for typical applications involving an ingestible device of the type described in PCT Application No. PCT/CA2013/000133 containing a sample acquired from the small intestine, centrifuging the ingestible device for 4 minutes at 3700 rpm is sufficient to transfer a portion of the sample from the ingestible device 73100 into the tubes 73114A and 73114B.

Diagram 73160 shows the ingestible device 73100, the sleeve device 73108, and the tubes 73114A and 73114B being removed from the centrifuge tube 73116, after the ingestible device 73100 and the sleeve device 73108 have been centrifuged. The centrifugal forces during the centrifuge process described in relation to diagram 73158 have transferred portions of the sample 73120A and 73120B (generally, portions of the sample 73120) from the ingestible device 73100 into the tubes 73114A and 73114B. The tubes 73114A and 73114B containing the samples 73120A and 73120B may then be disconnected from the sleeve device 73108. The tubes 73114A and 73114B containing the samples 73120A and 73120B may then be used in any type of standard laboratory diagnostics, and the ingestible device 73100 and the sleeve device 73108 may be disposed of, recycled and sanitized, or repurposed for another use.

In some embodiments, as a result of the centrifuging, tubes 73114A and 73114B may contain portions of samples 73120A and 73120B that were contained in separate sampling chambers within the ingestible device 73100. This may be the case if opening 73106A connects to a first sampling chamber, opening 73106B connects to a second sampling chamber, and if each sampling chamber holds a separate portion of the sample 73120A and 73120B prior to the centrifuging. In some embodiments, the portions of the sample 73120A and 73120B may be absorbed by a sponge contained within the sampling chambers prior to be being transferred into the tube. For example, the ingestible device 73100 may use a sponge within each of the sampling chambers in order to better acquire samples from the ingestible device while the ingestible device 73100 traverses the GI tract. In these situations, the force of the centrifuging may extract a portion of the sample from the sponge before the sample is transferred into the tubes 73114A and 73114B.

The ingestible device 73100 may have sampling chambers located near the top of the device, and these sampling chambers are connected to the created openings 73106A and 73106B. However, some ingestible devices may have sampling chambers located in other positions within the ingestible device. In some embodiments, the positioning and placement of an ingestible device within the holder 73102 is altered such that the opening created by the lancing mechanism 73104 still connects to the sampling chamber of the ingestible device. Similarly, the general shape of the sleeve device 73108 may be altered to fit around any portion of an ingestible device, and to accommodate one or more openings made at any location on the housing of the ingestible device.

In some embodiments, the sleeve device 73108 creates the openings in the ingestible device 73100. For example, two needles may be attached to the bottom side of the sleeve device 73108. As the sleeve device 73108 is connected to the ingestible device 73100, these needles may pierce the housing of the ingestible device 73100, thereby creating the openings 73106A and 73106B. This may allow openings to be created in the housing of the ingestible device 73100 without the use of a lancing mechanism 73104.

In some embodiments, a modified version of the sleeve device 73108 connects multiple openings 73106A and 73106B to a single tube 73114. For example, the ingestible device 73100 may have multiple sampling chambers, each of which is connected to a different one of the created openings 73106A and 73106B. The sleeve device 73108 may connect both of these openings 73106A and 73106B to a single tube 73114. This may allow the substances contained in each of the sampling chambers to be mixed together within the tube 73114 as portions of the samples are transferred into the tube 73114.

In the examples shown in diagrams 73150, 73152, 73154, 73156, 73158, and 73160, two openings 73106A and 73106B are simultaneously created in the housing of the ingestible device 73100, and the sleeve device 73108 is designed to simultaneously connect two tubes 73114A and 114B to the ingestible device 73100. However, in some embodiments, only a single opening is created in the housing of the ingestible device 73100, and the sleeve device 73108 is designed to connect only a single tube to the ingestible device 73100. More generally, in some embodiments, any number of openings may be created in the ingestible device 73100, either sequentially or simultaneously, and the sleeve device 73108 is designed to connect any number of tubes to any number of openings. In some embodiments, the sleeve device 73108 may also be designed to connect multiple ingestible devices to any number of tubes.

In the example shown in diagrams 73150 and 73152, the two openings 73106A and 73106B are simultaneously created by a lancing mechanism 73104. However, in some embodiments, other types of mechanisms may be used to create any number of openings in the ingestible device 73100, either sequentially or simultaneously.

In some embodiments, the sleeve device 73108 and the tubes 73114A and 73114B may not be involved in the sample extraction process, and the ingestible device 73100 may be loaded directly into the centrifuge tube 73116 after the one or more openings are created in the ingestible device 73100. This may result in a portion of the sample being transferred directly into the centrifuge tube 73116, and the centrifuge tube 73116 containing the portion of the sample may then be used for diagnostics and laboratory testing. In some of these embodiments, a first opening is created to access a first sampling chamber in the ingestible device 73100, and a first sample is transferred into the centrifuge tube 73116 from the first sampling chamber. Afterwards, a second opening is created to access a second sampling chamber in the ingestible device 73100, and a second sample is transferred into a different centrifuge tube 73116 from the second sampling chamber. This process may be continued for additional samples in additional sampling chambers, and may allow portions of multiple different samples to be transferred into separate centrifuge tubes without the need for the sleeve device 73108.

In general, the systems and methods and systems discussed in relation to FIG. 73 may be adapted or combined with other systems and methods. For example, the sleeve device 73108, the centrifuge tube 73116, and the centrifuge 73118 may be used to load substances into an ingestible device. An ingestible device may have one or more openings, and a modified sleeve device 73108 may connect the openings to tubes or funnels containing substances to be loaded into the ingestible device, such as a medicament to be delivered to a certain portion of the gastrointestinal tract. The ingestible device and the modified sleeve device 73108 may be placed into the centrifuge tube 73116, with the ingestible device oriented beneath the modified sleeve device 73108, closer to the bottom of the centrifuge tube 73116. In this arrangement, the centrifugal forces generated by the centrifuge 73118 may force the substances through the modified sleeve device 73108 and into the ingestible device 73100. After the substances have been loaded into the ingestible device, the openings in the ingestible device may be sealed, and the ingestible device may be administered to a patient. In some embodiments, the material used to seal the openings may be made of a different material than the rest of the housing of the ingestible device.

In some embodiments, a modified version of the sleeve device 73108 may allow substances to be loaded into an ingestible device without the use of the centrifuge tube 73116 or the centrifuge 73118. For example, instead of the tubes 73114A and 73114B, the sleeve device 73108 may allow one or more openings in an ingestible device to be connected to a needle or syringe. The needle or syringe may then be used to load the substances directly into the appropriate location within the ingestible device. The openings in the ingestible device may then be resealed prior to the ingestible device being administered to a patient.

As an alternate example, the centrifuge tube 73116 and the centrifuge 73118 may be used to move substances within an ingestible device, or mix substances previously contained within multiple chambers of an ingestible device. For example, there may be multiple chambers within the ingestible device, each chamber containing a different substance, and the multiple chambers may be connected together by one or more low-pressure check-valves. Loading the ingestible device into the centrifuge tube 73116, and centrifuging the ingestible device using the centrifuge 73118, may cause one or more of the low-pressure check-valves to break, thereby allowing the different substances to mix together within the ingestible device. This may allow the ingestible device to be used in a wider variety of applications. For example, a powdered medication may be activated when it is mixed with a solvent or reagent. The medication and the solvent may be pre-loaded into separate chambers of the ingestible device, and a centrifuge may be used to mix the substances together and activate the medication only when the ingestible device is about to be administered to a patient.

In general, this type of mixing may also be performed as substances are loaded into an ingestible device, with or without the use of a centrifuge 73118 or check-valves. For example, multiple openings in the housing of an ingestible device may connect to a single chamber within the ingestible device. A modified version of the sleeve device 73108 may be used to load multiple different substances into the ingestible device through each of the different openings. This may allow the substances to mix together once they are inside the ingestible device, and prevent the need for substances to be mixed together outside of the ingestible device.

As an alternate example, after the openings 73106A and 73106B are created in the housing of the ingestible device 73100, the sleeve device 73108 may connect to a vacuum mechanism instead of tubes 73114A and 73114B. In this case, the sample is directly sucked from the ingestible device 73100 and deposited into a tube or other receptacle attached to the vacuum mechanism. This may be done instead of centrifuging the ingestible device 73100, or it may be done either before or after a portion of the sample has been extracted by centrifuging. To assist in this process, multiple openings may be created in the housing of the ingestible device 73100 that connect to a single sampling chamber within the ingestible device 73100. A modified version of the sleeve device 73108 may allow one of these openings to be connected to a vacuum mechanism, and allow another of these openings to be connected to a source of air or flushing fluid. This may allow air or flushing fluid to enter the sampling chamber as the sample is being removed from the sampling chamber. This may prevent pressure from building up within the sampling chamber as the sample is sucked out by the vacuum mechanism, and allow the sample to be sucked out of the sampling chamber more easily.

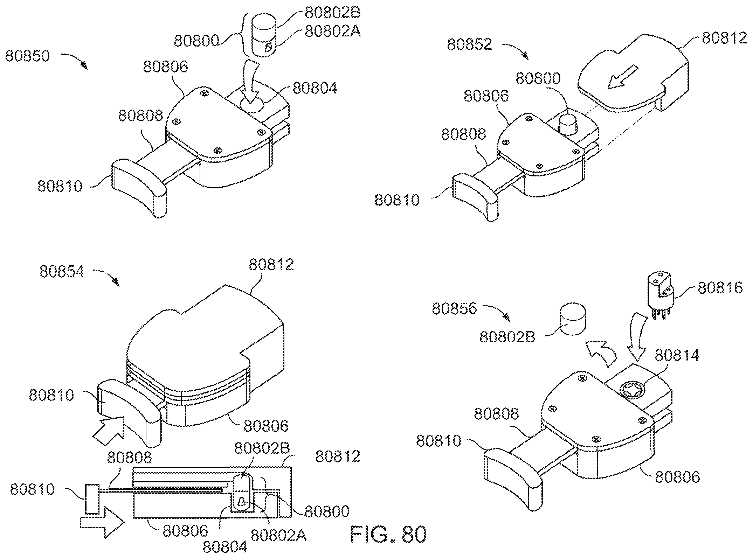

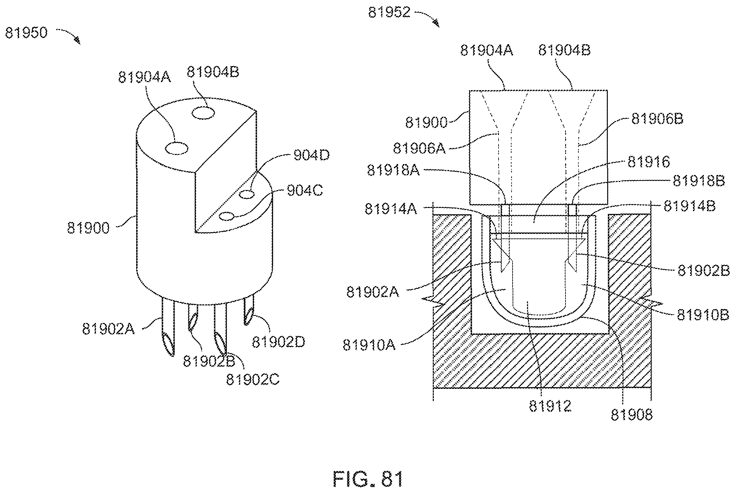

As yet another example, the systems and methods discussed in relation to FIG. 73 may be adapted or combined with any of the systems and methods discussed in relation to FIGS. 74-81. For example, FIG. 80 and FIG. 81 show an illustrative embodiment of a method for separating the ingestible device into multiple portions, and connecting a portion of the ingestible device to an adapter. This adapter may function in a similar manner as the sleeve device 73108, and connect the sampling chambers within a portion of an ingestible device 73100 to one or more tubes 73114. The centrifuge tube 73116 and the centrifuge 73118 may then be used to extract a sample from the portion of an ingestible device, similar to the manner shown in diagrams 73156, 73158, and 73160.

It is also understood that any of the systems and methods discussed in relation to FIG. 73 may be adapted into automated systems or combined with automated systems. For example, after an ingestible device 73100 is inserted into the holder 73102, a button or other suitable triggering mechanism may be used to automatically lower the lancing mechanism 73104 and create the openings 73106A and 73106B in the ingestible device 73100. Other processes, such as attaching the sleeve device 73108 onto the ingestible device 73100, or loading the ingestible device 73100 and the sleeve device 73108 into the centrifuge tube 73116, may be similarly automated. In general, the entire process of extracting a sample from an ingestible device described in relation to FIG. 73 may, if desired, be automated.

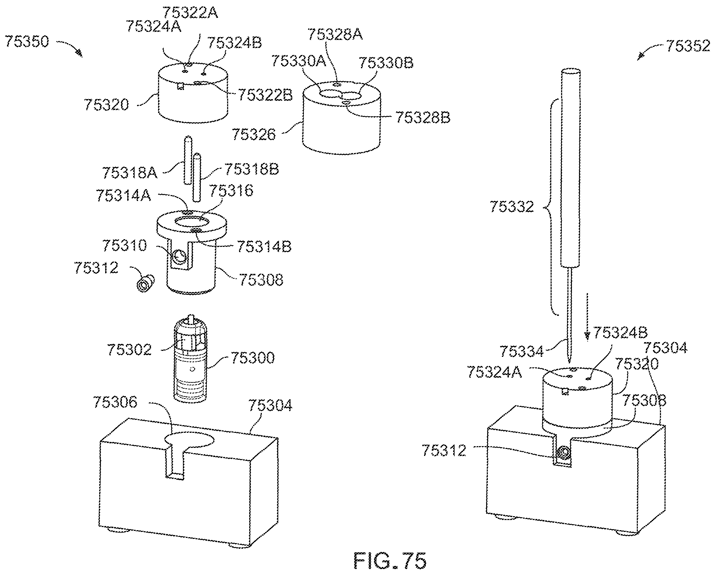

FIG. 74 shows an illustrative embodiment of a method for creating an opening in an ingestible device using a heated lancet, which may be used in conjunction with the method illustrated by FIG. 73, or which may be combined either wholly or in part with any of the systems and methods discussed in relation to FIGS. 75-81.

Diagram 74250 shows the ingestible device 74200 placed in a holder 74202 of a lancing jig in preparation for two openings to be created in the ingestible device 74200 by the lancing mechanism 206. Any one or more characteristics of the ingestible device 73100, the holder 73102, and the lancing mechanism 73104 described in relation to FIG. 73 may be applicable to the ingestible device 74200, the holder 74202, and the lancing mechanism 74206 described in relation to FIG. 74, respectively.

The holder 74202 is depicted in diagram 74250 with orientation markings 74204, which may allow the ingestible device 74200 to be aligned in a particular manner within the holder 74202. For example, aligning physical characteristics or markings on the ingestible device 74200 with the orientation markings 74204 may ensure that the lancing mechanism 74206 creates openings in the appropriate locations in the housing of the ingestible device 74200. In some embodiments, the ingestible device 74200 may only be placed into the holder 74202 when the ingestible device 74200 has a particular orientation relative to the holder 74202. For example, the ingestible device 74200 may have a window, or other physical feature, on one side of the ingestible device 74200, and the holder 74202 may have an opening which is sized and shaped to fit snugly around the ingestible device 74200 only when the window is oriented in a particular direction (e.g., oriented towards the orientation markings 74204 shown in FIG. 74). In some embodiments, the ingestible device 74200 may snap into place when it is inserted into the holder 74202 in a predetermined orientation, preventing it from moving further. For example, the ingestible device 74200 may include a window that is slightly receded from the housing of the ingestible device 74200, and the opening of the holder 74202 may include a slight protrusion. When the ingestible device 74200 is inserted into the holder 74202 with the window oriented in a particular position (e.g., oriented towards the orientation markings 74204 shown in FIG. 74), the protrusion aligns with the window and fills the receded area. This may prevent the orientation of the ingestible device 74200 from changing further once it has been snapped into place.

After the ingestible device 74200 has been positioned into the holder 74202, the lancing mechanism 74206 may be placed into a guide 74210. The guide 74210 may restrict the motion of the lancing mechanism 74206, and ensure that the openings created by the lancing mechanism 74206 are positioned correctly. For example, the guide 74210 may only permit the lancing mechanism 74206 to be lowered to a predetermined depth, and prevent the lancing mechanism 74206 from being lowered further. A plunger 74208 on an end of the lancing mechanism 74206 may be operated in order to push one or more lancets out of the lancing mechanism 74206, exposing them, or retract the lancets back into the lancing mechanism 74206. In general, the lancing mechanism 74206 may include a protective outer sheath, and the lancets are housed within the sheath when the lancets are retracted into the lancing mechanism 74206. Retracting the lancets into the protective outer sheath may prevent injuries due to mishandling the lancing mechanism 74206. Fully depressing the plunger 74208, or depressing the plunger 74208 past a predetermined depth, may also cause any exposed lancets to automatically retract back into the lancing mechanism 74206. This may be accomplished by means of a spring mechanism.

Diagram 74252 shows the operation of the plunger 74208 and the lancets 74212A and 74212B being heated prior to creating the openings in the ingestible device 74200. Allowing the lancets 74212A and 74212B to be retracted into the lancing mechanism 74206 may reduce the chance of injury by mishandling or misusing the lancing mechanism 74206. This may also prevent the lancets 74212A and 74212B from being contaminated by the outside environment.

By partially depressing the plunger 74208, the lancets 74212A and 74212B are pushed out of the lancing mechanism 74206. A heat source 74214 may be used to heat the lancets 74212A and 74212B. In general, heating the lancets 74212A and 74212B may sterilize the lancets 74212A and 74212B, and may reduce the risk of inadvertently contaminating the samples contained within the ingestible device 74200 when the openings are created. In some embodiments, the heat source 74214 may also heat the lancets 74212A and 74212B to a temperature that is above the melting point of a material used to form the housing of the ingestible device 74200. This may allow the lancets 74212A and 74212B to easily melt or penetrate the housing of the ingestible device 74200, thereby creating openings in the housing of the ingestible device 74200.

In general, the temperature of the lancets 74212A and 74212B is high enough to allow the lancets 74212A and 74212B to easily penetrate the housing of the ingestible device 74200, but not so high that unwanted damage is caused to the ingestible device 74200 or any samples contained within the ingestible device 74200. For example, the housing of ingestible device 74200 may be partially made of polycarbonate that has a melting temperature of about 147 degrees Celsius, and may begin to flow readily at 155 degrees Celsius. In this case, a Bunsen burner or torch may be used as the heat source 74214, and the temperature of the lancets 74212A and 74212B may be raised to approximately 250-300 degrees Celsius prior to being inserted into the ingestible device 74200. Having a temperature which is higher than the melting point of the polycarbonate may allow the openings 74216A and 74216B to be created quickly, and reduce the amount of time that the lancets 74212A and 74212B are in contact with the ingestible device 74200. In turn, this may reduce the risk of damaging the sample, for example, by denaturing analytes contained in the sample fluid. However, keeping the temperature of the lancets 74212A and 74212B below 300 degrees Celsius may minimize the chance of inadvertently damaging the sample or burning the polycarbonate used to form the housing of the ingestible device 74200. It is understood that the temperature ranges above are provided only as examples, and are not limiting. The optimal temperature of the lancets 74212A and 74212B depends on numerous factors, including the physical properties of the lancets 74212A and 74212B, the physical properties of the housing of the ingestible device 74200 and the sample within the ingestible device 74200, the desired size and shape of the openings 74216A and 74216B, the geometry and shape of the tips of the lancets 74212A and 74212B, and any number of other factors.

In some embodiments, the lancets 74212A and 74212B may be replaced by electrically heated tips. For example, the lancets 74212A and 74212B may be replaced by soldering iron tips that are heated by means of an electrical current instead of an external heat source 74214. Replacing the lancets 74212A and 74212B with electrically heated tips may allow the temperature to be set and maintained accurately.

Diagram 74254 shows the lancing mechanism 74206 being used to create the openings in the ingestible device 74200. By positioning the lancing mechanism74206 slightly above the ingestible device 74200, and partially depressing the plunger 74208, the heated lancets 74212A and 74212B are brought into contact with, and penetrate, the housing of the ingestible device 74200.

Diagram 74256 shows the openings 74216A and 74216B (generally, openings 74216) created in the housing of the ingestible device 74200 by the lancets 74212A and 74212B, after the plunger 74208 has been fully depressed. By fully depressing the plunger 74208, the lancets 74212A and 74212B are automatically retracted back into the lancing mechanism 74206. This may be done, for example, by means of a spring mechanism within the lancing mechanism 74206. Automatically retracting the lancets 74212A and 74212B when the plunger 74208 is fully depressed may reduce the time that a sample contained within the ingestible device 74200 is exposed to the heated lancets 74212A and 74212B. In some embodiments, the lancets 74212A and 74212B are automatically retracted back into the lancing mechanism 74206 when the plunger 74208 is depressed past a predetermined depth, and the plunger 74208 does not need to be fully depressed.

In some embodiments, fully depressing the plunger 74208, or depressing the plunger 74208 past a predetermined depth, does not automatically retract the lancets 74212A and 74212B back into the lancing mechanism 74206. Instead, the lancets 74212A and 74212B may be removed by lifting the plunger 74208, or lifting the entire lancing mechanism 74206, away from the ingestible device 74200. In some embodiments, the lancets 74212A and 74212B are automatically retracted a predetermined period of time after the plunger 74208 has been depressed.

In some embodiments, the holder 74202 does not include orientation markings 74204. For example, there may be only a single sampling chamber within the ingestible device 74200, and the precise positioning of the openings 74216A and 74216B may be unimportant. As an alternate example, there may be marking on the ingestible device 74200 that may be aligned directly with the lancets 74212A and 74212B. As an alternate example, the portion of the housing of the ingestible device 74200 where the openings are to be created are made with a different material than the surrounding portion of the housing. For example, the openings 74216A and 74216B may more easily created if only a certain portion of the housing of the ingestible device 74200 has a lower melting temperature, or is made with a softer material, than the rest of the housing of the ingestible device 74200. As yet another example, the ingestible device 74200 may include slight indentations on the surface of the housing, and the lancing mechanism 206 may be aligned with the indentations, and create the openings 74216A and 74216B at the same location as the indentations.

FIG. 74 depicts two openings being created in the housing of the ingestible device 74200 simultaneously. However, the lancing mechanism 74206 may be modified to create any number of openings in the ingestible device 74200, including a single opening in the ingestible device 74200. Additionally, different openings may be made at different times. For example, a first opening may be made at a first location by using a lancing mechanism, the ingestible device 74200 may be reoriented within the holder 74202, and a second opening may be made in a second location using the same lancing mechanism a second time.

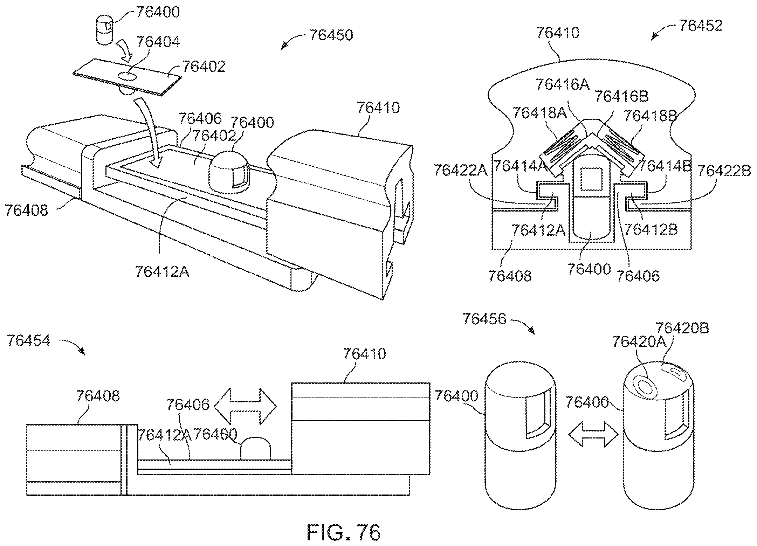

In general, the systems and methods discussed in relation to FIG. 74 may be combined with any other systems and methods, including the systems and methods discussed in relation to FIG. 73 or FIGS. 75-81. For example, the lancing mechanism 73104 depicted in FIG. 73 may have heated lancets, similar to the lancets 74212A and 74212B. As an alternate example, the lancing mechanism discussed in relation to FIG. 75 may be a heated lancet, or may automatically retract after the lancet penetrates the housing of an ingestible device. The general concept of heating a blade or surface to better penetrate or create openings in the housing of an ingestible device may also be directly applicable to the systems and methods described in FIGS. 76 and 80. In general, it is understood the any of the systems and methods discussed in relation to FIG. 74 may be incorporated into an automated system for creating openings within an ingestible device. For example, the process of heating and inserting the lancets 74212A and 74212B may be controlled by one or more sensors or actuators working in tandem with a computer or microcontroller. This could be accomplished, for example, by replacing the lancets 74212A and 74212B with electrically heated tips, and using threading within the guide 74210 to raise and lower the lancing mechanism 74206 with a motor. This may allow the openings 74216A and 74216B to be created with increased precision, and reduce the time that the heated lancets 74212A and 74212B are in contact with the ingestible device 74200.

FIG. 75 shows an illustrative lancing device, which may be used to create an opening in an ingestible device, and may be used in conjunction with the method illustrated by FIG. 73, or which may be combined either wholly or in part with any of the systems and methods discussed in relation to FIG. 74 or FIGS. 76-81. Diagram 350 shows an exploded view of the lancing device, with different components illustrated in detail. Diagram 75352 shows the fully assembled lancing device, which allows a lancet 75332 to be inserted into an ingestible device.

The ingestible device 75300 may be placed into an opening 75316 of a sleeve device 75308 in a manner similar to the way the ingestible device 73100 is inserted into the sleeve portion 73110 of the sleeve device 73108 discussed in relation to FIG. 73. The ingestible device 75300 may be placed in the sleeve device 75308, and the sleeve device 75308 may be placed into an opening 75306 in the holder 75304, which may perform a similar function as holder 73102 discussed in relation to FIG. 73.

The sleeve device 75308 has an aperture 75310, which is located on a protruding portion of the sleeve device 75308. The opening 75306 of the holder 75304 is sized and shaped to accommodate sleeve device 75308, including the protruding portion, and may ensure that the sleeve device 75308 has a predetermined orientation relative to the holder 75304 when the sleeve device 75308 and the ingestible device 75300 are inserted into the opening 75306 on the holder 75304.

In order to ensure that the ingestible device 75300 is oriented properly relative to the sleeve device 75308, the aperture 75310 may be used to view the ingestible device 75300 while it is within the sleeve device 75308. The ingestible device 75300 has a physical feature or marking 75302 that may be a sample acquisition window located on the side of the ingestible device 75300. The ingestible device 75300 may be rotated in order to line up the feature or marking 75302 with the aperture 75310, thereby orienting the ingestible device 75300 properly within the sleeve device 75308. Once the ingestible device 75300 is oriented properly relative to the sleeve device 75308 and the holder 75304, a fitting screw 75312 may be placed into the aperture 75310. The fitting screw 75312 may secure the ingestible device 75300 within the sleeve device 75308 and prevent the ingestible device 75300 from moving or rotating relative to the sleeve device 75308.

The openings 75314A and 75314B (generally, opening 75314) on the sleeve device 75308 are sized and shaped to accommodate two dowels 75318A and 75318B (generally, dowel 75318). The dowels 75318A and 75318B may connect the sleeve device 75308 to a lancing guide 75320, and ensure that the lancing guide 75320 has a particular orientation relative to the sleeve device 75308. The bottom surface of the lancing guide 75320 has a similar pair of openings (not shown), which may allow the lancing guide 75320 to slide smoothly onto the dowels 75318A and 75318B, and come into contact with the top of the sleeve device 75308 when the lancing guide 75320 is oriented properly relative to the sleeve device 75308.

The top surface of the lancing guide 75320 may include two openings 75322A and 75322B (generally, openings 75322). Depending on the length of the dowels 75318A and 75318B, the dowels 75318A and 75318B may extend all the way through the lancing guide 75320, and extend out of the openings 75322A and 75322B. This may allow additional guides or additional types of devices to be connected to the top surface of the lancing guide 75320 and the sleeve device 75308. However, in some embodiments, the length of the dowels 75318A and 75318B may be shorter, and the dowels 75318A and 75318B do not extend out of the top surface of the lancing guide 75320. In this case, the top surface of the lancing guide 75320 may not include the openings 75332A and 75332B.

Referring now to diagram 75352, the openings 75324A and 75324B (generally, opening 75324) in the top surface of the lancing guide 75320 are sized and shaped to accommodate a pointed end 75334 of a lancet 75332. Because the lancing guide 75320 may have a particular orientation relative to the sleeve device 75308, and the sleeve device 75308 may have a particular orientation relative to the ingestible device 75300, the opening may be created at a predetermined site on the ingestible device 75300 when the pointed end 75334 of the lancet 75332 is inserted through the opening 75324A and into the housing of the ingestible device.

As discussed in relation to FIG. 74, the pointed end 75334 of the lancet 75332 may be heated prior to being inserted into the opening 75324. This may allow the pointed end 75334 of the lancet 75332 to easily slide through the housing of the ingestible device 75300. The pointed end 75334 may, for example, be heated until it has a temperature within a predetermined range, which may be high enough to melt a material used to create the housing of the ingestible device 75300, but not so hot that it damages the lancing mechanism or the sample contained within the ingestible device 75300. After a first opening in the ingestible device 75300 has been created by inserting the lancet through the opening 75324A and penetrating a first site on the housing of the ingestible device 75300, a second opening may be created by inserting a lancet through the opening 75324B and penetrating a second site on the housing of the ingestible device 75300.

In some embodiments, the lancet 75332 may be replaced by a soldering iron, and the lancing guide 75320 may be replaced with a soldering iron guide 75326. The soldering iron guide 75326 may be connected to the sleeve device 75308 via the dowels 75318A and 75318B, and it may have openings 75328A and 75328B (generally, opening 75328) which function similarly to the openings 75322A and 75322B. One difference between the soldering iron guide 75326 and the lancing guide 75320 is that the openings 75330A and 75330B (generally, opening 75330) are sized and shaped to fit an end of a soldering iron, as opposed to the pointed end 75334 of the lancet 75332. The heated point of a soldering iron may then be inserted into the openings 75330A and 75330B in order to create openings in the housing of the ingestible device 75300. Similar to the lancet 75332, the heated point of the soldering iron may be heated to a temperature that is above a melting point of a material used to create the ingestible device 75300.

In some embodiments, the openings 75324A and 75324B may be replaced with any number of openings, including a single opening at the top of the lancet guide. In some embodiments, there may be no aperture 75310, no fitting screw 75312, no feature or marking 75302 on the ingestible device 75300, or any suitable combination thereof. For example, if there is only a single sampling chamber within the ingestible device 75300, or if the precise positioning of the opening created in the ingestible device 75300 is unimportant, there may be no need for the ingestible device 75300 to be oriented a particular way relative to the sleeve device 75308.

In general, the systems and methods discussed in relation to FIG. 75 may be modified or altered to accommodate the particular ingestible device and the particular application that the opening in the ingestible device is needed for. This may include, for example, incorporating the systems and methods discussed in relation to FIG. 75 into one or more automated systems for creating openings in an ingestible device, which may be operated by an appropriate combination of sensors, actuators, microcontrollers, mechanized systems, robotic systems, and the like. Additionally, the systems and methods discussed in relation to FIG. 75 may be combined with any other systems and methods, including the systems and methods discussed in relation to FIG. 73, FIG. 74, or FIGS. 76-81. For example, after creating openings in the ingestible device 75300, the sleeve device 75308 may be connected to or more tubes by a base portion, similar to the base portion 73112 of the sleeve device 73108 depicted in FIG. 73, or the base portion 77508 which is discussed in relation to FIG. 77. The ingestible device 75300, the sleeve device 75308, and the attached base portion connected to tubes may then be inserted into a centrifuge in order to extract a portion of the sample from the ingestible device, similar to the method discussed in relation to FIG. 73. As an alternate example, the lancet 75332 may be replaced with a modified version of the lancing mechanism 74206 discussed in relation to FIG. 74. The modified version of the lancing mechanism 74206 may be designed to be inserted into the openings 75324A and 75324B simultaneously. As yet another example, a modified version of the lancet 75332 may be configured to automatically retract the pointed end 75334 of the lancet 75332 once it is fully inserted into the opening 75324A. This may be done, for example, through a spring-loaded mechanism, which is triggered when the base of the lancet 75332 (i.e., the point where the pointed end 75334 of the lancet 75332 is connected to the remaining portion of the lancet 75332) comes into contact with the top of the lancing guide 75320.

FIG. 76 shows an illustrative embodiment of a grating device, which may be used to create an opening in an ingestible device, and may be used in conjunction with the method illustrated by FIG. 73, or which may be combined either wholly or in part with any of the systems and methods discussed in relation to FIG. 74, FIG. 75, or FIGS. 77-81. In general, the grating device may remove a portion of a housing of the ingestible device 76400, exposing one or more sampling chambers contained within the ingestible device 76400. Diagram 76450 shows an ingestible device 76400 that is loaded into the grating device. Diagram 76452 shows a view from one end of the grating device, after the ingestible device 76400 has been loaded into the grating device. Diagram 76454 shows a profile view of the grating device, and indicates a motion of the slide block 76410 used to remove a portion of the housing of the ingestible device 76400. Diagram 76456 shows the ingestible device 76400, before and after the grating device is used to remove a portion of the housing of the ingestible device 76400.

As shown in diagram 76450, the grating device includes a holder 76406 having a built-in platform 76402, which includes a recessed area 76404 that is sized and shaped to snugly hold a bottom portion of the ingestible device 76400. In some embodiments, the platform 76402 may be removable from the holder 76406, which may allow the ingestible device to be easily inserted and oriented within the recessed area 76404 before the platform 76402 is connected to the rest of the grating device. The ingestible device 76400 is partially inserted into the recessed area 76404 of the holder 76406, such that the top portion of the ingestible device is exposed.

The side of the holder 76406 forms a rail 76412A, and the other side of the holder 76406 may form a second rail 76412B (shown in diagram 76452). The grating device includes a slide block 76410 which is configured to move along the rails 76412A and 76412B (generally, rail 76412), and pass over the exposed top portion of the ingestible device 76400.

Diagram 76452 shows a view from one end of the grating device. As shown in diagram 76452, two grating surfaces 76416A and 76416B (generally, grating surface 76416) are connected to the inside of the slide block 76410. The grating surfaces 76416A and 76416B may be positioned so that when the slide block 76410 passes over the exposed top portion of ingestible device 76400, the grating surfaces 76416A and 76416B scrape against the top portion of the ingestible device 76400. This scraping may remove a portion of the housing of the ingestible device 76400, potentially exposing one or more sampling chambers within the ingestible device. It will be understood that the slide block 76410 may be passed over the exposed top portion of the ingestible device 76400 several times before enough of the housing is removed for the sampling chambers within the ingestible device 76400 to be exposed.

In general, the grating surfaces 76416A and 76416B may be made of any type of sufficiently sharp blade, or sufficiently abrasive material. For example, the grating surfaces 76416A and 76416B may include grated blades formed from metal or ceramic, a roughened or abraded surface similar to a rasp or file, or other types of rough materials, such as sand paper or Emory cloth.

In diagram 76452, the grating surfaces 76416A and 76416B are depicted as oriented substantially perpendicularly to each other. This may help to simplify the geometry of the slide block 76410, and may allow the grating device to expose two sampling chambers within the ingestible device simultaneously. In some embodiments, the orientation of the grating surfaces 76416A and 76416B may have a different orientation relative to one another, in order to accommodate the shape and size of the ingestible device 76400, or to alter the location of the portion of the housing of the ingestible device 76400 that is removed.

The grating surfaces 76416A and 76416B may be connected to the slide block 76410 any number of ways. For example, the grating surfaces 76416A and 76416B may be connected by magnetic plates attached to the inside of the slide block 76410, or by mechanical fasteners such as bolts, screw, springs, and the like. Certain types of connections, such as the use of magnetic plates, may allow the grating surfaces 76416A and 76416B to be removed from the slide block 76410. In turn, this may allow the grating surfaces 76416A and 76416B to be periodically replaced with new grating surfaces once old grating surfaces wear down from use. Two spring mechanisms 76418A and 76418B (generally, spring mechanism 76418) are positioned between the grating surfaces 76416A and 76416B and the interior wall of the slide block 76410. The spring mechanisms 76418A and 76418B may push the grating surfaces 76416A and 76416B towards the ingestible device 76400 when the slide block 76410 is passing over the top portion of the ingestible device 76400. This may allow the grating device to accommodate different types of ingestible devices, to ensure that a new portion of the housing of the ingestible device 76400 is removed each time that the slide block 76410 moves over the ingestible device 76400, or to compensate for slight variations for how firmly the ingestible device 76400 is seated into the recessed area 76404 of the holder 76406. However, in some embodiments, the spring mechanisms may not be included in the grating device, and the grating surfaces 76416A and 76416B are simply connected to the slide block 76410. In some embodiments, the spring mechanisms 76418A and 76418B only push the grating surfaces 76416A and 76416B to a predetermined depth. For example, the grating surfaces 76416A and 76416B may be connected to a certain type of fastener that only allows the grating surfaces 76416A and 76416B to move a certain distance away from the inner wall of the slide block 76410. This may, in turn, restrict the amount of material that may be remove from the housing of the ingestible device 76400, and limit the size of any openings created in the housing of the ingestible device 76400.

Diagram 76452 shows the holder 76406 connected to the base 76408 of the grating device. Rails 76412A and 76412B extending from the sides of the holder 76406 are suspended slightly above the base 76408, forming small gaps 76422A and 76422B (generally gap 76422). The slide block 76410 has two groove-shaped slots 76414A and 76414B (generally, groove-shaped slot 76414) that extend along the length of the slide block 76410. The groove-shaped slots 76414A and 76414B are shaped to fit at least partially around the rails 76412A and 76412B (e.g., around three surfaces) as the slide block 76410 moves along the rails 76412A and 76412B, and may guide the motion of the slide block 76410. Generally, the movement of the slide block 76410 is restricted by the two rails 76412A and 76412B, and the slide block 76410 may move along the two rails 76412A and 76412B simultaneously as the slide block 76410 is passed over the exposed top surface of the ingestible device 76400.

Diagram 76454 shows a profile view of the grating device, and indicates the motion of the slide block 76410 used to remove a portion of the housing of the ingestible device 76400. The slide block 76410 may be positioned at one side of the base 76408, and held in place by the rails 76412A and 76412B on the sides of the holder 76406. The slide block 76410 may slide along the rails 76412A and 76412B, moving along the length of the base 76408. This may allow the grating surfaces 76416A and 76416B to scrape against the exposed top portion of the ingestible device 76400. The slide block 76410 may be moved back and forth along the rails 76412A and 76412B any number of times, and this may allow additional portions of the housing of the ingestible device 76400 to be removed as the grating surfaces 76416A and 76416B repeatedly scrape along the top portion of the ingestible device 76400.

Diagram 76456 shows the ingestible device 76400, before and after the grating device is used to remove a portion of the housing of the ingestible device 76400. When enough of the housing of the ingestible device 76400 is removed, two openings 76420A and 76420B (generally, opening 76420) are created in the housing of the ingestible device. Each of these openings 76420A and 76420B may expose sampling chambers within the ingestible device 76400. Once the openings 76420A and 76420B have been created, a portion of the sample contained in the sampling chambers may be extracted from the ingestible device 76400. This may be done using any appropriate system or method, such as the systems and methods discussed in relation to FIG 73. For example, the ingestible device 76400 may be connected to a sleeve device, which connects the openings 76420A and 76420B to a set of tubes, similar to the sleeve device 73108 and tubes 73114A and 73114B discussed in relation to FIG. 73. At that point, the ingestible device 76400 and the sleeve device may be placed into a centrifuge tube, and a portion of the sample may be transferred from the ingestible device 76400 to the tubes by centrifuging the ingestible device 76400, the sleeve device, and the tubes.

In some embodiments, the holder 76406 or the platform 76402 may include a set of orientation markings near the recessed area 76404. These orientation markings may indicate the portion of the housing of ingestible device 76400 that is removed when the slide block 76410 passes over the exposed top portion of the ingestible device 76400. This may be particularly advantageous if it is known where the sampling chambers within the ingestible device 76400 are located, and if there are any noticeable features or markings on the ingestible device 76400 which may be aligned with the orientation markings.

For illustrative purposes, FIG. 76 depicts two rails 76412A and 76412B, and two groove-shaped slots 76414A and 76414B. However, in some embodiments there may be only one rail on the holder 76406, or one groove-shaped slot on the slide block 76410. In these embodiments, it may be advantageous to change the shape of the rail and the groove-shaped slot, to ensure that a single rail is sufficient to restrict the motion of the slide block 76410. For example, in some embodiments, a "T"-shaped rail may be used to further restrict the motion of the slide block 76410.

In some embodiments, the positioning or shape of the rails 76412A and 76412B may be different, and the shape and location of the groove-shaped slots 76414A and 76414B may be altered to match. For example, two "T"-shaped rails may come directly up from the base 76408, and the groove-shaped slots 76414A and 76414B slots may be reshaped accordingly and repositioned to the bottom of the slide block 76410.

For illustrative purposes, FIG. 76 depicts two grating surfaces 76416A and 76416B. However, in some embodiments there may be only one grating surface. If multiple sampling chambers within the ingestible device 76400 need to be exposed, it may be possible to use a single grating surface to expose a first sampling chamber, reposition the ingestible device 76400 within the holder 76406, and then use the same grating surface to expose another sampling chamber within the ingestible device 76400. This process may be repeated several times over, as necessary, to expose any number of sampling chambers within the ingestible device 76400. Alternatively, the grating device may include more than two grating surfaces 76416.

In general, the systems and methods discussed in relation to FIG. 76 may be modified or altered to accommodate different types of ingestible device, or accommodate the particular application that necessitate exposing a sampling chamber within the ingestible device. For example, if there is only a single sampling chamber located within the top of the ingestible device, the grating device may be modified to include only a single grating surface, and the position of the grating surface within the slide block 76410 may be repositioned accordingly. As an alternate example, in some embodiments the slide block 76410 is in a fixed position relative to the base 76408, and the holder 76406 or the platform 76402 is able to be moved in order to bring the ingestible device 76400 in contact with a grating surface 76416 within the slide block 76410. In some embodiments, the holder 76406 may be replaced with a conveyer belt, to move the ingestible device 76400 into contact with the grating surface 76416 within the slide block 76410.

Additionally, the systems and methods discussed in relation to FIG. 76 may be combined with any other systems and methods, including automated systems, and the systems and methods discussed in relation to FIGS. 73-75, or FIGS. 77-81. For example, the orientation of the ingestible device 76400 within the holder 76406 may be improved by first placing the ingestible device 76400 into a sleeve device, similar to sleeve device 75308 discussed in connection with FIG. 75. The orientation of the ingestible device 76400 within the sleeve device 75308 may be guaranteed by use of an aperture and fitting screw (e.g., the aperture 75310 and fitting screw 75312 of FIG. 75), and the recessed area 76404 may be sized and shaped so the sleeve device only fits into the recessed area in a particular orientation. As another example, the movement of the slide block 76410 may be automated, and an appropriately configured control system may ensure that the slide block 76410 continues to move back and forth over the top of the ingestible device 76400 until a predetermined portion of the housing of the ingestible device 76400 is removed.

FIG. 77 shows an illustrative embodiment of a sleeve device, which may connect an ingestible device to one or more tubes, and may be used in conjunction with the method illustrated by FIG. 73, or which may be combined either wholly or in part with any of the systems and methods discussed in relation to FIGS. 74-76 or FIGS. 78-81. Diagram 77550 shows an ingestible device 77500 with two sampling chambers 77502A and 77502B (generally, sampling chamber 77502) inserted into a sleeve device 77504, which is used to connect openings in the housing of the ingestible device (not shown) to the tubes 77510A and 77510B (generally, tube 77510). Diagram 77552 shows a detailed view of an example base portion 77508, which may be included in the sleeve device 77504.

Sleeve device 77504 connects to the end of the ingestible device 77500, and may generally be used in extracting samples from the ingestible device 77500. Ingestible device 77500 is shown with sampling chambers 77502A and 77502B, and openings (not shown) in the portion of the housing of the ingestible device inserted into the sleeve device 77504 lead to the sampling chambers 77502A and 77502B. The sleeve device 77504 has a sleeve portion 77506 and a base portion 77508. The sleeve portion 77506 is shaped to fit snugly around the circumference of the ingestible device, and the base portion 77508 connects the openings in the bottom of the ingestible device 77500 to the tubes 77510A and 77510B.

The top surface of the base portion 77508 has openings 77516A and 77516B (shown in diagram 77552), each of which may connect to an opening in the housing of the ingestible device 77500. Tunnels run through the base portion 77508, and connect the openings 77516A and 77516B on the top surface of the base portion 77508 to similar openings (not shown) in the bottom surface of the base portion 77508.

In general, the tubes 77510A and 77510B are removable tubes, which may be attached or detached from the bottom of the base portion 77508. Each of the tubes 77510A and 77510B have a tube opening, which may face the openings on the bottom of the base portion 77508 when the tubes 77510A and 77510B are attached to the bottom of the base portion 77508.

The top surface of the base portion 77508 may be connected to the end of the sleeve portion 77506 in order to form the sleeve device 77504. This connection may be made any number of ways. For example, the openings 77520A and 77520B (generally, opening 77520) may be sized and shaped to accommodate a dowel or a bolt, and the sleeve portion 77506 may be connected to the base portion 77508 through the use of an appropriately sized dowel or bolt. In this example, the sleeve portion 77506 may also have dowel-shaped openings, similar to the sleeve device 75308 discussed in relation to FIG. 75, and the sleeve portion 77506 and the base portion 77508 may be connected by a dowel joint. In some embodiments, another type of mechanism may be used to connect the sleeve portion 77506 and the base portion 77508. For example, part of the base portion 77508 may form a pair of pegs, which fit into the openings 77520A and 77520B, which allow the sleeve portion 77506 and the base portion 77508 to be connected together, similar to a mortise and tenon joint. In general, it may be possible to use any type of convenient mechanism, such as magnetic or mechanical fasteners, to connect the sleeve portion 77506 and the base portion 77508. In some embodiments, depending on the mechanism being used to connect the sleeve portion 77506 and the base portion 77508, the openings 77520A and 77520B may not be included on the base portion 77508.

In some embodiments, the openings 77520A and 77520B on the base portion 77508 may be sized and shaped to accommodate a dowel, and dowel-shaped tunnels connect the openings 77520A and 77520B to similar dowel-shaped openings on the bottom of the base portion 77508. The sleeve portion 77506 may have similar dowel-shaped openings, which connect to another set of dowel-shaped tunnels that extend into the sleeve portion 77506 (e.g., similar to the sleeve device 75308 of FIG. 75). This configuration may allow a dowel to be inserted all the way through the base portion 77508, through the openings on the bottom of the base portion 77508, and connect the base portion 77508 to the sleeve portion 77506.

The top surface of the base portion 77508 includes a sealing portion 77512, that is sized and shaped to fit the end of the ingestible device 77500. The sealing portion 77512 forms watertight seals between openings in the housing of the ingestible device and the openings 77516A and 77516B. These watertight seals may be created by the protruding portions 77514A and 77514B (generally, protruding portion 77514). These protruding portions 77514A and 77514B may be sized and shaped to fit the curvature of the ingestible device. When the end of the ingestible device 77500 is inserted into the base portion 77508, each of the protruding portions 77514A and 77514B may contact a portion of the housing of the ingestible device 77500 that surrounds one of the openings on the end of the housing of the ingestible device 77500. This may cause each of the protruding portions 77514A and 77514B to form a watertight seal that encircles the openings in the housing in the ingestible device 77500.

In some embodiments, the protruding portions 77514A and 77514B are able to compress and conform to the shape of the ingestible device 77500. For example, the protruding portions 77514A and 77514B, or the entire base portion 77508, may be made from silicone. The shape of the silicone protruding portions 77514A and 77514B may deform slightly to accommodate the portion of the ingestible device 77500 that contacts the protruding portions 77514A and 77514B. This may help the protruding portions 77514A and 77514B form watertight seals around the openings in the housing of the ingestible device 77500.

Diagram 77552 shows two magnets 77518A and 77518B (generally, magnet 77518) included as part of the base portion 77508. These magnets may be used to connect the tubes 77510A and 77510B to the bottom of the base portion 77508 of the sleeve device 77504. This, in turn, may allow the tubes 77510A and 77510B to be easily connected or disconnected from the sleeve device. In some embodiments, base portion 77508 may not include magnets 77518A and 77518B, and some other suitable type of mechanism may be used to connect the tubes 77510A and 77510B to the sleeve device 77504. For example, this may be accomplished by mechanically fastening the tubes 77510A and 77510B to the base portion 77508 of the sleeve device 77504. In some embodiments, the bottom (not shown) of the base portion 77508 is shaped to conform to the opening of the tubes 77510A or 77510B. For example, there may be a slight protrusion on the bottom of the base portion 77508, which is sized and shaped to fit snugly around the outer circumference of the tubes 77510A and 77510B, or to fill the inner circumference of the tubes 77510A and 77510B. This may help to ensure that the tubes 77510A and 77510B are properly oriented relative to the base portion 77508, and reduce the chance of any of the sample from within the ingestible device 77500 spilling out from the tubes 77510A and 77510B.

In some embodiments, a window or aperture on the side of the sleeve portion 77506 of the sleeve device 77504 may be used to view the ingestible device 77500 and orient the ingestible device 77500 within the sleeve device 77504. This may be done in order to ensure that any openings within the housing of the ingestible device 77500 are aligned with the openings 77516A and 77516B in the base portion 77508. In some embodiments, the orientation of the ingestible device 77500 relative to the sleeve device 77504 may be fixed. For example, a peg or fitting screw may be inserted into an aperture on the side of the sleeve portion 77506 in order to prevent the orientation of the ingestible device 77500 from changing. As another example, the ingestible device 77500 and the sleeve device 77504 may be shaped such that the ingestible device 77500 can only be inserted into the sleeve device 77504 in a predetermined orientation. This may help to ensure that openings in the housing of the ingestible device 77500 are properly aligned with the protruding portions 77514A and 77514B of the base portion 77508.

For illustrative purposes, FIG. 77 depicts two sampling chambers within the ingestible device 77500, two openings 77516A and 77516B within the base portion 77508 of the sleeve device 77504, and two tubes 77510A and 77510B. However, the general size and shape of the sealing portion 77512 and the base portion 77508 may be modified to connect any number of sampling chambers to any number of tubes.

In general, the systems and methods discussed in relation to FIG. 77 may be modified or altered to accommodate different types of ingestible devices. For example, it is understood that the general design and shape of the sleeve device 77504 may be easily modified to accommodate different types of ingestible devices, openings in the housing of an ingestible device which are located in different positions around the ingestible device. For example, if there is only a single sampling chamber located within the top of an ingestible device, the openings 77516A and 77516B may be replaced with a single opening in the center of the sealing portion 77512, and this opening may connect the ingestible device to a single tube.

Additionally, the systems and methods discussed in relation to FIG. 77 may be combined with any other systems and methods, including automated systems, and the systems and methods discussed in relation to FIGS. 73-76, or FIGS. 78-81. For example, the orientation of the ingestible device 77500 may be fixed within the sleeve device 77504 by inserting a positioning pin or a fitting screw into an aperture on the side of the sleeve portion 77506 of the sleeve device 77504 (e.g., similar to the aperture 75310 and fitting screw 75312 of FIG. 75). As an alternate example, the sleeve device 77504 may be modified to accommodate any ingestible device with an opening or exposed sampling chamber, such as the ingestible devices 73100, 74200, 75300, and 76400. Additionally, any ingestible device connected to sleeve device 77504 may be inserted into a centrifuge in order to extract a portion of the sample from the ingestible device, similar to the methods discussed in relation to FIG. 73.FIG. 78 shows illustrative diagrams of a centrifuge mechanism, which may allow a sleeve device and an ingestible device to be inserted into a centrifuge, and may be used in conjunction with the method illustrated by FIG. 73, or which may be combined, either wholly or in part, with any of the systems and methods discussed in relation to FIGS. 74-77 or FIGS. 79-81. Diagram 78650 shows an exploded view of the centrifuge mechanism, and shows details of the different components which may be included in the centrifuge mechanism. Diagram 78652 shows a cross-sectional view of the assembled centrifuge mechanism. Diagram 78654 shows a detailed view of the interface between an ingestible device and a sleeve device when placed together within the centrifuge mechanism.

The ingestible device 78600 is placed within a sleeve portion 78602, and the end of the ingestible device 78600 rests at the top of a base portion 78604. Together, the sleeve portion 78602 and the base portion 78604 form a sleeve device 78628, and are able to connect the ingestible device 78600 to two tubes 78606A and 78606B (generally, tube 78606). In some embodiments, these elements (e.g., the ingestible device 78600, the sleeve portion 78602, the base portion 78604, and the tubes 78606) may be the same as the ingestible device 77500, the sleeve portion 77506 and base portion 77508 that form the sleeve device 77504, and tubes 77510A and 77510B discussed in relation to FIG. 77. In general, the sleeve portion 78602 and the base portion 78604 may be made of any suitable material, such as machined polycarbonate or molded silicone.

The ends of the tubes 78606A and 78606B are placed into a tube holder 78608, and the tip of the tube holder 78608 may be sized and shaped to fit snugly within the end of the centrifuge tube 78610. The tube holder 78608 may have an extended portion 78626, which comes up from the tube holder 78608 and may be positioned near the top of the centrifuge tube 78610 when the centrifuge mechanism is fully assembled. Pulling on the extended portion 78626 may be used to remove the contents of the centrifuge tube 78610. This may be particularly useful if the centrifuging mechanism needs to be disassembled once the centrifuging has been completed.

Once the ingestible device 78600, the sleeve device 78628, the tubes 78606A and 78606B, and the tube holder 78608 are assembled together within the centrifuge tube 78610, a cap 78612 may be used to cover the centrifuge tube. The bottom surface of the cap 78612 is connected to a spring 78614, and a brace 78616 extends from the bottom of the cap 78612. The spring 78614 may be used to help maintain the position of the ingestible device 78600 relative to the sleeve device 78628 while the ingestible device 78600 is inserted into the sleeve device 78628. This may be done by using a spring 78614 that is sized and shaped to fill the space between the cap 78612 and ingestible device 78600, and apply a downward pressure to the ingestible device 78600. The brace 78616 is sized and shaped to fill the space around the sides of the ingestible device 78600, between the cap 78612 and the sleeve device 78628. This may help hold the ingestible device 78600 within position in the center of the centrifuge tube 78610, and also help to maintain the position of the sleeve device 78628 and the tube holder 78608 at the bottom of the centrifuge tube 78610.

In some embodiments, the spring 78614 is not attached to the cap 78612, and is a separate component. For example, the spring 78614 may be placed onto the ingestible device 78600 after the ingestible device 78600 and the sleeve device 78628 have been inserted into the centrifuge tube 78610. The cap 78612 may then be screwed or fitted onto the top of the centrifuge tube 78610. This may compress the spring 78614, and cause the spring 78614 to push the ingestible device 78600 towards the bottom of the centrifuge tube 78610.

As shown in diagram 78654, an opening 78624 in the housing of the ingestible device 78600 is pressed against an opening 78622 in the base portion 78604 of the sleeve device 78628 when the ingestible device 78600 and the sleeve device 78628 are connected together. A protruding portion 78630 of the base portion 78604 contacts the housing of the ingestible device 78600 around the opening 78624, and forms a seal between the opening 78624 in the housing of the ingestible device 78600, and the opening 78622 in the base portion 78604. This may be similar to the system discussed in relation to FIG. 77, where the protruding portions 77514A and 77514B of the sealing portion 77512 of the base portion 77508 contact the ingestible device 77500 to form a watertight seal.

A tunnel 78620A runs through the base portion 78604, and connects the opening 78622 on the top surface of the base portion 78604 to a similar opening on the bottom surface of the base portion 78604. This may form path for a portion of the sample to flow through when it is transferred from the sampling chamber 78618A into the tube 78606A. Generally, the tunnels 78620A and 78620B (generally, tunnel 78620) are able to connect the sampling chambers 78618A and 78618B (generally, sampling chamber 78618) to one or more tubes.

After the centrifuge mechanism is assembled, the centrifuge tube 78610 containing the ingestible device 78600, the sleeve device 78628, and the tubes 78606A and 78606B may be placed into a centrifuge. When the centrifuge is turned on, the centrifugal forces may force any sample contained within the sampling chambers 78618A and 78618B in a downward direction, towards the bottom of the centrifuge tube 78610. This may force the samples out of the sampling chambers 78618A and 78618B, through the tunnels 78620A and 78620B in the base portion 78604 of the sleeve device 78628, and into the tubes 78606A and 78606B.

After a sufficient amount of the sample has been transferred into the tubes 78606A and 78606B, the centrifuge may be turned off, the centrifuge mechanism disassembled, and the tubes 78606A and 78606B may be recovered by detaching them from the base portion 78604.

For illustrative purposes, FIG. 78 depicts two sampling chambers 78618A and 78618B within the ingestible device 78600, two tunnels 78620A and 78620B running through the base portion 78604, and two tubes78 606A and 78606B being used to collect the sample. However, the centrifuge mechanism may be modified to connect any number of sampling chambers to any number of tubes through any number of tunnels.

For illustrative purposes, FIG. 78 depicts a centrifuge mechanism that includes a spring 78614 and a brace 78616. However, in some embodiments, the spring 78614 and/or the brace 78616 may not be included. In these embodiments, centrifugal forces alone may be enough to hold the ingestible device 78600 within the sleeve device 78628. Similarly, the general size and shape of the sleeve device 78628 may be altered to fit snugly with the centrifuge tube 78610, or to better secure the ingestible device within the centrifuge tube 78610.

In some embodiments, when the sample is to be collected in the bottom of the centrifuge tube 78610 instead of the tubes 78606A and 78606B, the tubes 78606A and 78606B and the tube holder 78608 may not be included in the centrifuge mechanism. It is also possible that the design of the sleeve device 78628 may be altered accordingly. For example, the sleeve device 78628 may be redesigned to simply suspend the ingestible device 78600 above the bottom of the centrifuge tube 78610, with an opening in the housing of the ingestible device 78600 facing towards the bottom of the centrifuge tube 78610.

In general, the systems and methods discussed in relation to FIG. 78 may be modified or altered to accommodate different types of ingestible devices. For example, it is understood that the general design and shape of the sleeve device 78628 may be easily modified to accommodate different types of ingestible devices, or openings in the housing of an ingestible device which are located in different positions around the ingestible device. For example, if there is only a single sampling chamber located within the bottom of an ingestible device, a single tunnel running through the base portion 78604 may connect an opening in the bottom of the ingestible device to a single tube.

Additionally, the systems and methods discussed in relation to FIG. 78 may be combined with any other systems and methods, including automated systems, and the systems and methods discussed in relation to FIGS. 73-77, or FIGS. 79-81. For example, the sleeve device 57704 discussed in relation to FIG. 77 may be incorporated into the centrifuge mechanism as the sleeve device 78628. As an alternate example, the centrifuge mechanism may, as a whole, be used to replace the centrifuge tube 73116 discussed in relation to FIG. 73. It will also be understood that any of the systems or methods discussed in relation to FIG. 78 may be combined, either wholly or in part, with the centrifuge mechanism discussed in relation to FIG. 79.

FIG. 79 shows an illustrative diagram of a centrifuge mechanism, which may allow a sleeve device and an ingestible device to be inserted into a centrifuge, and may be used in conjunction with the methods described in relation to FIG. 73. The centrifuge mechanism of FIG. 79 includes several modifications which may be made to the centrifuge mechanism discussed in relation to FIG. 78, and may be combined, either wholly or in part, with any of the systems and methods discussed in relation to FIGS. 74-78, FIG. 80, or FIG. 81. Diagram 79750 shows an internal view of an assembled centrifuge mechanism, which includes a modified sleeve device. Diagram 79752 shows an external view of the same centrifuge mechanism shown by diagram 79750.

The ingestible device 79700 is positioned within a sleeve portion 79702, and the end of the ingestible device 79700 rests at the top of a base portion 79704. Together, the sleeve portion 79702 and the base portion 79704 form a sleeve device 79718, and connect the ingestible device 79700 to a tube 79706. The ingestible device 79700, the sleeve device 79718, and the tube 79706 may be similar to the arrangement of the ingestible device 78600, the sleeve device 78628, and the tubes 78606A and 78606B discussed in relation to FIG. 78.

The end of the tube 79706 is placed in a tube holder 79720, and the tip of the tube holder 79720 may be sized and shaped to fit snugly within the end of the centrifuge tube 79712. The centrifuge tube 79712 is covered by a cap 79714. Similar to cap 78612 discussed in relation to FIG. 78, cap 79714 is connected to a spring 79716 and a brace 79722. The spring 79716 may be used to help maintain the position of the ingestible device 79700 relative to the sleeve device 79718, and the brace 79722 is sized and shaped to fill the space around the sides of the ingestible device 79700, between the cap 79714 and the sleeve device 79718.

On the side of the sleeve portion 79702 is an aperture 79708, which may be used to view the orientation of the ingestible device 79700 within the sleeve device 79718. A moveable fitting screw 79710 is inserted into aperture 79708, and may be used to restrict the motion of the ingestible device 79700 relative to the sleeve device 79718. In some embodiments, the moveable fitting screw 79710 is inserted through the side of the centrifuge tube 79712, and the moveable fitting screw 79710 may be used to restrict the motion of the ingestible device 79700 relative to the centrifuge tube 79712 as well. In some embodiments, the moveable fitting screw 79710 is made of a transparent material. This may allow the orientation of the ingestible device 79700 within the sleeve device 79718 to be viewed through the aperture 79708 when the moveable fitting screw 79710 is inserted into the aperture 79708.

For illustrative purposes, FIG. 79 shows a single tube 79706 being used to collect samples from the ingestible device 79700. If the ingestible device 79700 has multiple sampling chambers, multiple tunnels may run through the base portion 79704, each tunnel connecting a different sampling chamber to the same tube 79706 through openings in the housing of the ingestible device 79700. Furthermore, in some embodiments, the base portion 79704 may be modified to connect any number of sampling chambers within the ingestible device 79700 to any number of tubes.

In general, the systems and methods discussed in relation to FIG. 79 may be modified or altered to accommodate different types of ingestible devices. For example, it is understood that the general design and shape of the sleeve device 79718 may be easily modified to accommodate different types of ingestible devices, or accommodate openings in the housing of an ingestible device which are located in other positions.

Additionally, the systems and methods discussed in relation to FIG. 79 may be combined with any other systems and methods, including the systems and methods discussed in relation to FIGS. 73-78, FIG. 80, or FIG. 81. For example, any of systems and methods of the centrifuge mechanism discussed in relation to FIG. 78 may be applied to the centrifuge mechanism of FIG. 79, and the individual components used in different centrifuge mechanism embodiments may be interchangeable with one another. As an alternate example, the sleeve device 79718 may be replaced with any other suitable type of device connecting sampling chambers of an ingestible device to an appropriately sized tube, and the size and shape of the other components of the centrifuge mechanism may be adapted accordingly. Examples of other systems and methods for accessing sampling chambers of an ingestible device and connecting the sampling chambers to tubes, which may be incorporated into a centrifuge mechanism similar to the mechanisms discussed in relation to FIG. 78 and FIG. 79, are discussed in relation to FIG. 80 and FIG. 81.

FIG. 80 shows an illustrative embodiment of a method for separating the ingestible device into multiple portions, and extracting a sample from a portion of the ingestible device. The method includes separating the ingestible device into a first portion and a second portion, the first portion comprising a sampling chamber that contains at least a portion of the sample. An example system and method for separating an ingestible device into portions is shown in diagrams 80850, 80852, and 80854. The method also includes connecting the sampling chamber to an adapter. Connecting the sampling chamber to the adapter is shown in diagram 80856, and an example adapter is discussed in relation to FIG. 81. The adapter, in turn, may be connected to a tube. The method includes centrifuging the first portion of the ingestible device, while the sampling chamber is connected to the adapter, to transfer at least the portion of the sample from the sampling chamber into the tube. This may be done by inserting the adapter, the ingestible device, and the tube into a centrifuge mechanism (e.g., variations of the centrifuge mechanisms discussed in relation to FIG. 78 and FIG. 79) and making use of a centrifuge similar to the centrifuge 73118 discussed in relation to FIG. 73.

As shown in diagram 80850, an ingestible device 80800 may have a first portion 80802A that houses the sampling chambers within the ingestible device, and a second portion 80802B. The ingestible device 80800 is inserted into a cutting mechanism 80806 by positioning the ingestible device 80800 within a recessed area 80804 of the cutting mechanism 80806. The cutting mechanism 80806 has a moveable blade 80808 which may be operated by means of a handle 80810. In general, the first portion 80802A of the ingestible device 80800 is positioned within the recessed area 80804, and the second portion 80802B is exposed. The moveable blade 80808 may be used to cut the ingestible device 80800 and form two separate portions 80802A and 80802B, while the recessed area 80804 holds the ingestible device 80800.

As shown in diagram 80852, after the ingestible device 80800 is inserted into the cutting mechanism 80806, a back-piece 80812 may be connected to the cutting mechanism 80806. The back-piece 80812 may further restrain the ingestible device 80800, and provide additional resistance when the end of the moveable blade 80808 is inserted into the ingestible device 80800.

Diagram 80854 shows the back-piece 80812 and the cutting mechanism 80806 connected together. When the back-piece 80812 and the cutting mechanism 80806 are connected together, the walls of the back-piece 80812 and the recessed area 80804 form a chamber that holds the ingestible device 80800. As noted above, this may effectively restrain the ingestible device 80800 while it is being cut by the moveable blade 80808, and provide additional resistance when the end of the moveable blade 80808 is inserted into the ingestible device 80800.

By pushing the handle 80810 towards the body of the cutting mechanism 80806, the moveable blade 80808 is pressed into the ingestible device 80800. If sufficient pressure is placed on the handle 80810, the moveable blade 80808 may cut through the ingestible device 80800, separating the ingestible device 80800 into a first portion 80802A and a second portion 80802B. Once the first portion 80802A and the second portion 80802B have been formed, the back-piece 80812 may be disconnected and removed from the cutting mechanism 80806. At this point, the second portion 80802B may be removed, and the handle 80810 may be used to retract the moveable blade 80808.

Diagram 80856 shows the cutting mechanism 80806 after ingestible device 80800 has been separated into two portions. Removing the second portion 80802B exposes a barrier 80814 within the first portion 80802A of the ingestible device 80800. The barrier 80814 may cover the sampling chambers contained within the first portion 80802A of the ingestible device 80800. In some embodiments, the barrier 80814 may form a portion of the outer surface of the sampling chamber, or it may be part of a structure within the first portion 80802A that houses the sampling chambers.

After the barrier 80814 is exposed, an adapter 80816 is inserted into the first portion 80802A of the ingestible device 80800. The bottom of the adapter 80816 may include hollow needles, and inserting the adapter 80816 into the first portion 80802A may cause the hollow needles to penetrate the barrier 80814, and enter one or more sampling chambers within the ingestible device 80800. An example of an adapter 80816 is discussed in detail in relation to FIG. 81.

In some embodiments, there may visible features or markings on the barrier 80814, which indicate the position of the sampling chambers within the first portion 80802A. This may ensure that the hollow needles of the adapter 80816 are inserted into the correct locations of the first portion 80802A. In some embodiments, there may be small indentations on the barrier 80814. These indentations may be used to guide the hollow needles of the adapter 80816, and the hollow needles of the adapter 80816 may penetrate the barrier 80814 at the location of the indentations.

After the adapter 80816 is inserted into the first portion 80802A, one or more tubes (not shown) may be connected to the top of the adapter 80816. These tubes may be sized and shaped similar to the tubes 77510A and 77510B (FIG. 77), and may be connected to the adapter 80816 similar to how the tubes 77510A and 77510B connect to base portion 77508, as discussed in relation to FIG. 77. The first portion 80802A may then be loaded into a centrifuge tube while it is connected to the adapter 80816, and run through a centrifuge (e.g., the centrifuge 73118 of FIG. 73). This may cause at least a portion of the sample to be transferred from the sampling chambers within the first portion 80802A into the tubes. After the centrifuging is completed, the tubes containing portions of the sample may be disconnected from the adapter 80816, and may be used in laboratory testing.

As an alternate example, instead of tubes, the adapter 80816 may connect the sampling chambers within the first portion 80802A to a vacuum mechanism. This may allow the sample to be directly sucked from the first portion 80802A of the ingestible device 80800. The sample may then be deposited into a tube or other suitable receptacle attached to the vacuum mechanism. This may be done instead of centrifuging the first portion 80802A and the adapter 80816, or it may be done either before or after a portion of the sample has been extracted by centrifuging. To assist in this process, the adapter 80816 may simultaneously connect a vacuum mechanism and a source of air or flushing fluid to a single sampling chamber within the first portion 80802A of the ingestible device 80800. This may allow air or flushing fluid to enter the sampling chamber as the sample is being sucked out of the sampling chamber, allowing the sample to be easily sucked out.

In some embodiments, the adapter 80816, the first portion 80802A, and the tubes are inserted into a centrifuge mechanism prior to being centrifuged. For example, the centrifuge mechanisms discussed in relation to FIG. 78 and FIG. 79 may be modified to hold the adapter 80816 while it is connected to an ingestible device, instead of the sleeve devices 78628 and 79718. More generally, any of the various systems or methods discussed in relation to FIG. 78 and FIG. 79 may be modified to accommodate the first portion 80802A and adapter 80816. For example, this may include inserting the adapter 80816 and the first portion 80802A into a centrifuge tube (e.g., the centrifuge tube 78610 or 79712), and attaching the centrifuge tube to a cap having a spring at the bottom of the cap (e.g., the cap 78612 or 79714 attached to spring 78614 or 79716). This may cause the spring to maintain a position of the first portion 80802A relative to the adapter 80816 while they are centrifuged. As an alternate example, a moveable fitting screw or positioning pin (e.g., the moveable fitting screw 79710) may be used to restrict the movement of the first portion 80802A relative to the centrifuge tube. This may be done by inserting the moveable fitting screw into an aperture on the side of the adapter 80816.

In general, the systems and methods discussed in relation to FIG. 80 may be modified or altered to accommodate different types of ingestible device. For example, the size and shape of the recessed area 80804, and the positioning of the ingestible device within the recessed area 80804, may be altered. This may change where the moveable blade 80808 cuts the ingestible device, and determine where the separation between the first portion and second portion is made. Similarly, the general design of the adapter 80816 may be altered to accommodate the size and shape of the ingestible device, and may connect any number of sampling chambers within the ingestible device to any number of tubes. For example, the adapter 80816 may have a single hollow needle that connects a single sampling chamber to a single tube, or the adapter 80816 may have two hollow needles that connect two sampling chambers to two separate tubes.

Additionally, the systems and methods discussed in relation to FIG. 80 may be combined with any other systems and methods, including automated systems, and the systems and methods discussed in relation to FIGS. 73-79, or FIG. 81. For example, moveable blade 80808 may automatically retract into the cutting mechanism 80806 when the handle 80810 is fully inserted into the cutting mechanism 80806, similar to the lancing mechanism 74206 and the plunger 74208 discussed in relation to FIG. 74. As another example, similar to the heated lancet discussed in relation to FIG. 74, the moveable blade 80808 may be heated to a temperature that is above the melting temperature of the housing of the ingestible device 80800. This may allow the moveable blade 80808 to easily cut into the ingestible device 80800, thereby forming the first portion 80802A and the second portion 80802B. This may be done by incorporating a heating element, such as an electric heater, into the cutting mechanism 80806. This may also be done by removing the moveable blade 80808 from the cutting mechanism 80806, and heating it using an external heat source. As one example, the motion of the moveable blade 80808 may be fully automated, and the cutting mechanism 80806 automatically cuts the ingestible device 80800 into two portions 80802A and 80802B upon detecting that the back-piece 80812 is connected to the cutting mechanism 80806. Similarly, an automated system may connect the back-piece 80812 to the cutting mechanism 80806, remove and dispose of the second portion 80802B, and connect the adapter 80816 to the first portion 80802A. This may be done using an appropriate set of sensors, actuators, microcontrollers, computerized systems, robotic systems, or the like.

FIG. 81 shows an illustrative adapter device, which may connect a portion of an ingestible device to a tube, and may be used in conjunction with the method illustrated by FIG. 80. Furthermore, the adapter device discussed in relation to FIG. 81 may be combined either wholly or in part with any of the systems and methods discussed in relation to FIGS. 74-80.

Diagram 81950 shows a detailed external view of the adapter device, and diagram 81952 shows a detailed internal cross-sectional view of the adapter device when it is connected to an ingestible device portion 81908. As shown in diagram 81950, the top surface of the adapter device 81900 includes four openings 81904A, 81904B, 81904C, and 81904D (generally, opening 81904). Each of these openings 81904A, 81904B, 81904C, and 81904D are connected to similar openings (e.g., the openings 81918A and 81918B shown in diagram 81952) on the bottom surface of the adapter device 81900 by tunnels (e.g., the tunnels 81906A and 81906B, shown in diagram 81952) which run through the body of the adapter device 81900. Hollow needles 81902A, 81902B, 81902C, and 81902D (generally, hollow needle 81902) protrude from the bottom surface of the adapter device 81900, and each hollow needle 81902A, 81902B, 81902C, and 81902D has a lumen that connects to one of the openings on the bottom surface of the adapter device 81900.

For simplicity, diagram 81952 only shows two openings 81904A and 81904B, that connect via two tunnels 81906A and 81906B (generally, tunnels 81906) to two openings 81918A and 81918B (generally, openings 81918). These openings 81918A and 81918B are connected to two hollow needles 81902A and 81902B, which connect with sampling chambers 81910A and 81910B (generally, sampling chamber 81910) within the ingestible device portion 81908. However, it will be understood that the openings 81904C and 81904D and the hollow needles 81902C and 81902B may function in a similar manner, and any of the systems and methods discussed in relation to one of the openings 81904 or hollow needles 81902 may apply to all of the others.

As shown in diagram 81952, tunnel 81906A connects the opening 81904A on the top surface of the adapter device 81900 to the opening 81918A on the bottom surface of the adapter device 81900. The hollow needle 81902A protrudes from the bottom surface of the adapter device 81900, and has a lumen connected to the opening 81918A. The hollow needle 81902A may penetrate the barrier 81914A, and connect to the sampling chamber 81910A. It is understood that the barriers 81914A and 81914B (generally, barrier 81914) may be penetrated by any hollow needle 81902.

The lumen of hollow needle 81902A, the opening 81918A, the tunnel 81906A, and the opening 81904A form a fluid channel, and the hollow needle 81902A is sized and shaped such that a portion of the sample contained in the sampling chamber 81910A is able to flow through this fluid channel. A similar fluid channel is formed by the combination of the lumen of hollow needle 81902B, the opening 81918B, the tunnel 81906B, and the opening 81904B, which allows a portion of the sample to flow out from the sampling chamber 81910B.

In general, the ingestible device portion 81908 may include a wiper 81912, which contacts the internal walls of the housing of the ingestible device portion 81908, and forms part of the boundaries of the sampling chambers 81910A and 81910B. The wiper 81912 may act as a watertight barrier, isolating samples in the sampling chambers 81910A and 81910B until the top portion of the wiper 81912 that forms the barriers 81914A and 81914B is penetrated by the hollow needles 81902A and 81902B. The ingestible device portion 81908 is also depicted with a secondary barrier 81916. This secondary barrier 81916 may act as a fail-safe to ensure that the sample remains with ingestible device portion 81908 in case the barriers 81914A and 81914B formed by the wiper 81912 are damaged. The secondary barrier 81916 may be constructed from a similar material as the wiper 81912, and may be penetrated by the hollow needles 81902A and 81902B in a similar fashion.

The openings 81904A, 81904B, 81904C, and 81904D may be connected with removable tubes, similar to the tubes 77510A and 77510B connected to the sleeve device 77504 in FIG. 77. These tubes may have tube openings which face the openings 81904A, 81904B, 81904C, and 81904D when the tubes are connected to the top surface of the adapter device 81900. These tubes may be connected to the adapter device by any convenient means, such as through the use of mechanical fasteners or magnets. In some embodiments, the adapter device 81900 includes magnets, which may be used to connect tubes to the appropriate locations of the adapter device 81900 near the openings 81904A, 81904B, 81904C, and 81904D.

In some embodiments, the adapter device 81900 may be configured to have two hollow needles simultaneously connected to a single sampling chamber within the ingestible device portion 81908. This may allow a sample to be removed from the sampling chamber without the need for centrifuging the ingestible device portion 81908 and the adapter device 81900. For example, both of the hollow needles 81902B and 81902C may be connected simultaneously to the sampling chamber 81910B. In these embodiments, one hollow needle (e.g., hollow needle 81902B) may be used to remove a sample from the sampling chamber 81910B, and the other hollow needle (e.g., the hollow needle 81902C) may allow air or fluid to enter sampling chamber 81910B. For example, the hollow needle 81902B may be connected to a vacuum mechanism (e.g., by connecting the vacuum mechanism to the opening 81904B), which applies suction to the sampling chamber 81910B. At the same time, the hollow needle 81902C may allow air to flow into the sampling chamber 81910B, preventing negative pressure from building up within the sampling chamber 81910B and resisting the force of the suction. As an alternate embodiment, the hollow needle 81902C may pump fluid into the sampling chamber 81910B, such as water or saline. This may be done by attaching the water source to the opening 81904C. This may flush out the sampling chamber 81910B, causing any of the sample contained in the sampling chamber 81910B to be forced out of the other hollow needle 81902B.

In some embodiments, the adapter device 81900 may be used to load substances into the ingestible device portion 81908. For example, after a hollow needle 81902 has been inserted into the ingestible device portion 81908, the substance may be inserted into the ingestible device portion 81908 through the opening 81904. The opening 81904 may be connected to a funnel, tube, needle, syringe, or any other mechanism which allows substances to be placed into the opening 81904. Afterwards, the ingestible device portion 81908 may be connected to another ingestible device portion, in order to form a single ingestible device that may be administered to a patient. In general, this method may allow any type of substance to be loaded into an ingestible device through the use of the adapter device 81900.

In some embodiments, one or more of the hollow needles 81902A, 81902B, 81902C, and 81902D may include a retractable sleeve around the needle. After a hollow needle (e.g., hollow needle 81902A) is inserted into a sampling chamber (e.g., the sampling chamber 81910A), the sleeve around the hollow needle 81902A is retracted. This may result in the hollow needle 81902A penetrating the barrier 81914A through an opening which is slightly wider than the hollow needle 81902A. This may provide a path for air to enter the sampling chamber 81910A as the hollow needle 81902A is used to remove the sample from the sampling chamber 81910A. For example, this may be particularly advantageous if the opening 81904A connected to the hollow needle 81902A is connected to a vacuum mechanism which sucks a portion of the sample out of the sampling chamber 81910A.

In some embodiments, one or more secondary needles may be inserted through the hollow needles 81902A, 81902B, 81902C, and 81902D. After a hollow needle (e.g., hollow needle 81902A) is inserted into a sampling chamber (e.g., the sampling chamber 81910A), a thinner secondary needle is inserted through the hollow needle and into the sampling chamber. This secondary needle may be inserted, for example, through one of the openings 81904 of the top surface of the adapter device 81900. The secondary needle may then be used to extract the sample from the sampling chamber 81910A, and the area between the outer circumference of the secondary needle and the inner circumference of the hollow needle 81902A may provide a path for air to enter the sampling chamber 81910A.

For illustrative purposes, the adapter device 81900 of FIG. 81 is depicted with the top surface of the adapter device 81900 being divided into an upper level and a lower level. The openings 81904A and 81904B are located on the upper level, and the openings 81904C and 81904D are located on the lower level. This may visually distinguish the different openings 81904, or allow only certain types of devices or mechanisms to be connected to each of the openings 81904. For example, a vacuum mechanism may be configured to only connect to openings on the upper level, and a source of flushing fluid may be configured to only connect to openings on the lower level. However, in some embodiments, the design and shape of the adapter device 81900 may be altered, and all of the openings 81904 may be placed on a single level.

In general, the systems and methods discussed in relation to FIG. 81 may be modified or altered to accommodate different types of ingestible devices. For example, it is understood that the general design and shape of the adapter device 81900 may be modified to accommodate different types of ingestible devices, and the number and positioning of the various openings and hollow needles may be altered to accommodate the number and position of the sampling chambers within the ingestible device portion. For example, if there is only a single chamber located in the center of the ingestible device, there may be only a single hollow needle extending from center of the bottom of the adapter device 81900. As an alternate example, in some embodiments the hollow needles can penetrate the housing of an ingestible device. This may allow the adapter device 81900 to connect to an ingestible device without first separating the ingestible device into a first and second portion.

Additionally, the systems and methods discussed in relation to FIG. 81 may be combined with any other systems and methods, including automated systems, and the systems and methods discussed in relation to FIGS. 73-80. For example, the adapter device 81900 may be directly incorporated into the systems and methods discussed in connection with FIG. 80 as the adapter 80816. As an alternate example, the adapter device 81900 may be used in a similar fashion as the sleeve device 77504 discussed in relation to FIG. 77, in order to connect an ingestible device to one or more tubes. The adapter device 81900, may be included as part of the centrifuging mechanisms discussed in relation to FIG. 78 and FIG. 79, and the systems and methods discussed in relation to FIG. 73 may be adapted to centrifuge the adapter device 81900, a portion of an ingestible device, and a tube, in order to transfer a portion of the sample within the portion of the ingestible device into the tube.

Moreover, while examples have been provided in which holes are generated at the top of an ingestible device, other approaches are also within the disclosure.

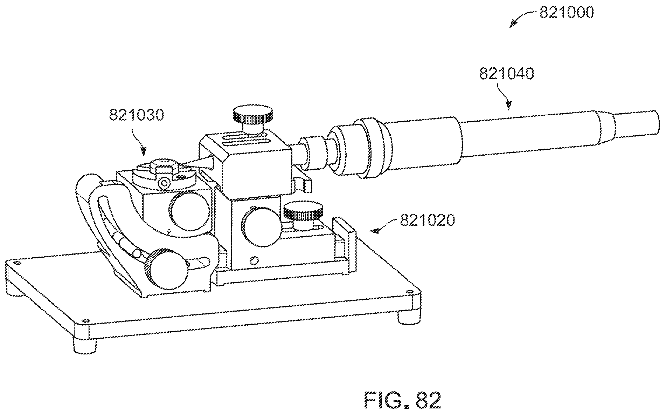

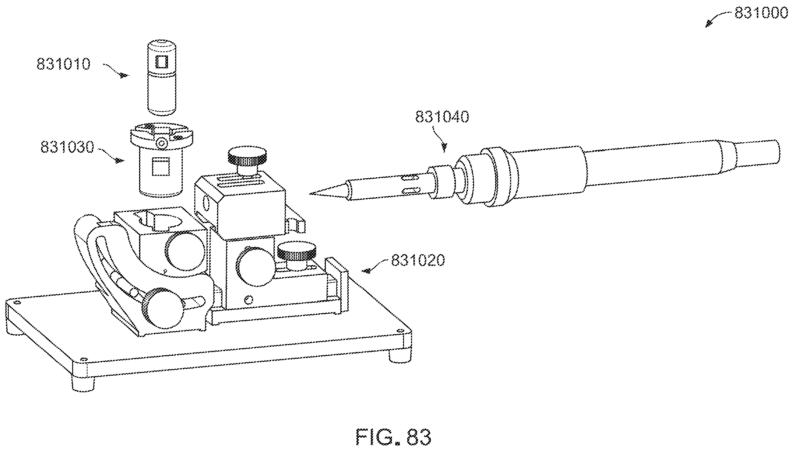

FIGs. 82 and 83 illustrate a system 821000 that can be used to pierce one or more holes in the side of an ingestible device 831010. System 821000 includes a tool 821020, a device holder 821030 and a soldering iron 821040.

In some embodiments, soldering iron tool 821020 includes primarily machined aluminum components. Tool 821020 is designed to assist in the production of two holes in the side of device 831010 through which sample contained within device 831010 is extracted. Tool 821020 has a keyed cradle which accepts device holder 821030. The cradle has two keying features which position holder 821030 and device 831010 into two distinct radial positions to produce the holes in device 831010 in the target locations in the side of each sampling chamber. A guide channel in tool 821020 accurately positions the tip of soldering iron 821040 in the correct position and at the correct depth to control the location and size of the melted holes in device 831010. Tool 821020 also has knobs to adjust the height, distance and penetration depth of soldering iron 821040 and to adjust the angle of device 831010 with respect to soldering iron 821040. Alternatively, or additionally, safety screws may be used to lock the positions of the parts of tool 821020, which can, for example, reduce the possibility of accidental misalignment during use of tool 821020. Optionally, soldering iron is operated manually 821040. However, in some embodiments, soldering iron 821040 is operated automatically. In certain embodiments, soldering iron 821040 is attached to tool 821020 such that movement of iron 821040 is automated with a motor to accurately control penetration depth, exposure time and applied force. In some embodiments, soldering iron 821040 is on a rail fixed to the tool 821020 and uses a spring loaded trigger mechanism to snap soldering iron 821040 back automatically once it has been pushed forward and reached the target.

Device holder 821030 can be made of machined polycarbonate. Holder 821030 holds device 831010 during the hole creation and centrifugation processes. In some embodiments, holder 821030 uses a set screw or the like (e.g., a ball detent, snap, spring loaded tab, or other keying feature) to lock device 831010 in place by keying into the window of device 831010. When positioned correctly, the set screw does not apply pressure to device 831010, but simply protrudes into the window of device 831010. Holder 821030 is placed into the cradle in soldering iron tool 821020. An external keying feature on the side of holder 821030 helps locate holder 821030 into one of two positions within tool 821020. The two positions ensure that the tip of soldering iron 821040 is guided into the two hole locations. After the holes in device 831010 have been produced, holder 821030 is removed from tool 821020 and is used to key device 831010 into a centrifuge jig which is then centrifuged to extract the samples (see discussion above).

In some embodiments, soldering iron 821040 is a hand-held tool that has a heatable tip. As an example, the tip can be heated electrically, and the temperature can be set using an interface of a control system of system 821000. The heatable tip can be a commercially available tip, or it can be custom made. After the tip of soldering iron 821040 is heated to the target temperature, the tip is inserted into a guide in soldering iron tool 821020. The guide aligns the tip to the correct location on device 831010 and restricts the depth to which soldering iron 821040 can be pushed. The tip is pushed into the device 831010 (e.g., polycarbonate housing) to produce the holes. The heat of the tip melts the polycarbonate, allowing for penetration while minimizing the applied force. Optionally, the tip can be a single use tip.

While system 821000 has been described with a soldering iron, other embodiments are possible. For example, additionally or alternatively, system 821000 can use one or more heated lancets (see discussion above). In some embodiments, soldering iron 821040 can be replaced by rotary cutting tool, such as a dremel, to drill holes into capsules. In certain embodiments of a fully automated system, soldering iron 821040 can be replaced by custom heated tips that could be provided, for example, in cartridges which are attached to tool 821000. In such embodiments, tool 821000 can be configured to dispense the tips, heat the tips (e.g., using an onboard electrical system), move one tip forward at a time to melt the holes in the capsule, and eject the tip. Optionally, tips could then be discarded. In some embodiments, the number of components in system 821000 can be simplified to reduce the number of adjustment options. Alternatively, system 821000 could be configured so that there are more adjustment options. In certain embodiments, components of system 821000 can be adjusted by adding rails and lead screws or rack and pinion systems to allow positions of components to be adjusted more precisely.

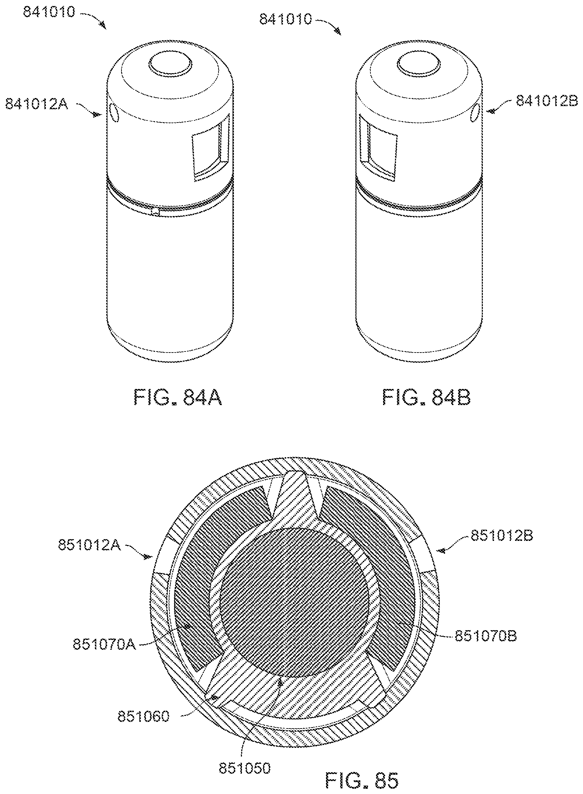

FIGs. 84A and 84B illustrate device 841010 with holes 841012A and 841012B formed, for example, using the approach noted above. In general holes 841012A and 841012B are centered on corresponding, respective sampling chamber of device 841010. Thus, in some embodiments 841012A and 841012B are not separated from each other by 180°. The location of holes 841012A and 841012B allows sample to exit the sample chambers when device 841010 is centrifuged upside down with holes facing away from the centrifuge's rotor.

FIG. 85 illustrates a cross-sectional view of an embodiment of device 851010 with holes 851012A and 851012B. Device 841010 includes a gearmotor 851050 and a wiper 851060. A shaft of gearmotor 851050 is connected to the top cap of device 841010 so that the activation of gearmotor 851050 spins the top cap of device 841010. This in turn allows the sampling window in the top cap to be rotated so that it aligns with the sampling chambers formed by wiper 851060. Wiper 851060 contacts the internal surface of device 841010 and forms part of the boundaries between holes 851012A and 851012B. Wiper 851060 may define a liquid tight barrier that isolates a sample in chamber 851012A from a sample in 851012B. As shown in FIG. 85, device 841010 may further include absorbent material members 851070A and 851070B. Members 851070A and 851070B may be, for example, sponges (e.g., hydrophilic sponges, hydrophobic sponges). Members 851070A and 851070B can assist in chambers 851012A and 851012B collecting and maintaining their respective samples.

While embodiments have been disclosed which involve centrifugation, the disclosure is not limited in this sense. In some embodiments, sample analysis does not involve centrifugation. More generally, any method, device and/or system can be used that is able to analyze a sample (e.g., analyze a sample as described elsewhere herein).

Further, while certain embodiments have been disclosed for removing one or more samples from an ingestible device, other approaches may be used as well. More generally, any method, device and/or system can be used that is able to remove a desired sample from an ingestible device for subsequent analysis (e.g., analysis as described elsewhere herein). Preferably, such approach result in little or no sample contamination.

### Detection Methods and Systems

### Live Cell Dye

Certain systems described herein employ methods, compositions and detection systems found to accurately and reliably correlate fluorescence to total bacteria count (TBC) in an autonomous, ingestible device, or other similarly-sized device. As used herein, the term "total bacteria count" is used to refer to a quantity of bacterial and/or archaeal cells. In some disclosures, the methods and devices described herein can be used for the detection of TBC in a sample from the gastrointestinal tract of the subject to determine whether the subject has or is at risk of developing a GI disorder (e.g., SIBO). The compositions include novel combinations of dyes, buffers and detergents that allow for the selective staining of viable bacterial and/or archaeal cells in samples that include non-bacterial and/or non-archaeal cells and other components that otherwise make detecting or quantifying live bacterial and/or archaeal cells challenging. In some disclosures, the systems allow for bacteria to be quantified in near real-time and the results to be shared telemetrically outside of the device. Above, various types of cells (e.g., bacterial cells and archaeal cells) are disclosed which can be detected using the methods described in this section.

In some disclosures, the disclosure provides a composition including a dye and optionally a reagent for selective lysis of eukaryotic cells. In some disclosures, the composition includes both a dye and a reagent for selective lysis of eukaryotic cells. In some disclosures, the composition further comprises one or more reagents independently selected from the group consisting of: a second reagent for selective lysis of eukaryotic cells, an electrolyte (e.g., MgCl₂), an anti-fungal reagent (e.g., amphotericin-B), and an antibiotic. In some disclosures, the composition comprises water and is in the form of an aqueous solution. In some disclosures, the composition is a solid or semi-solid. In some disclosures, the compositions described here are suitable for use in a kit or device for detecting or quantifying viable bacterial and/or archaeal cells in a sample. In some disclosures, such a device is an ingestible device for detecting or quantifying viable bacterial and/or archaeal cells *in vivo (e.g.,* in the GI tract). In some disclosures, viable bacterial and/or archaeal cells in a sample are detected or quantified in the presence of one or more antibiotics to determine antibiotic resistance of the bacteria and/or archaea in the sample. In some disclosures, anomalous bacterial and/or archaeal populations in a sample may be detected or quantified, for example through the use of a composition comprising a dye as disclosed herein, to determine whether a subject has an infection, such as Small Intestinal Bacterial Overgrowth (SIBO), or to characterize bacterial and/or archaeal populations within the GI tract for diagnostic or other purposes.

In some disclosures, the dye suitable for use in the composition of the present disclosure is a dye that is capable of being internalized by a viable cell (e.g., a bacterial and/or archaeal cell), binding to or reacting with a target component of the viable cell, and having fluorescence properties that are measurably altered when the dye is bound to or reacted with the target component of the viable cell. In some disclosures, the dye of the present disclosure is actively internalized by penetrating viable cells through a process other than passible diffusion across cell membranes. Such internalization includes, but is not limited to, internalization through cell receptors on cell surfaces or through channels in cell membranes. In some disclosures, the target component of a viable cell to which the dye is bound to or reacted with is selected from the group consisting of: nucleic acids, actin, tubulin, enzymes, nucleotide-binding proteins, ion-transport proteins, mitochondria, cytoplasmic components, and membrane components. In some disclosures, the dye suitable for use herein is a fluorogenic dye that is capable of being internalized and metabolized by a viable cell, and wherein the dye fluoresces when metabolized by the viable cell. In some disclosures, the dye is a chemiluminescent dye that is capable of being internalized and metabolized by a viable cell, and wherein the dye becomes chemiluminescent when metabolized by the viable cell.

In some disclosures, the composition includes a dye that fluoresces when bound to nucleic acids. Examples of such dyes include, but are not limited to, acridine orange (U.S. Pat. No. 4,190,328); calcein-AM (U.S. Pat. No. 5,314,805); DAPI; Hoechst 33342; Hoechst 33258; PicoGreen^{™}; SYTO^{®} 16; SYBR^{®} Green I; Texas Red^{®}; Redmond Red^{™}; Bodipy^{®} Dyes; Oregon Green^{™}; ethidium bromide; and propidium iodide.

In some disclosures, the composition includes a lipophilic dye that fluoresces when metabolized by a cell (e.g., a bacterial and/or archaeal cell). In some disclosures, the dye fluoresces when reduced by a cell or a cell component. Examples of dyes that fluoresce when reduced include, but are not limited to, resazurin; C¹²-resazurin; 7-hydroxy-9H-(1,3 dichloro-9,9-dimethylacridin-2-ol) N-oxide; 6-chloro-9-nitro-5-oxo-5H-benzo[a]phenoxazine; and tetrazolium salts. In some disclosures, the dye fluoresces when oxidized by a cell or a cell component. Examples of such dyes include, but are not limited to, dihydrocalcein AM; dihydrorhodamine 123; dihydroethidium; 2,3,4,5,6-pentafluorotetramethyldihydrorosamine; and 3'-(p-aminophenyl) fluorescein.

In some disclosures, the composition includes a dye that becomes chemiluminescent when oxidized by a cell or a cell component, such as luminol.

In some disclosures, the composition includes a dye that fluoresces when de-acetylated and/or oxidized by a cell or a cell component. Examples of such dyes include, but are not limited to, dihydrorhodamines; dihydrofluoresceins; 2',7'-dichlorodihydrofluorescein diacetate; 5-(and 6-)carboxy-2',7'-dichlorodihydrofluorescein diacetate; and chloromethyl-2',7'-dichlorodihydrofluorescein diacetate acetyl ester.

In some disclosures, the composition includes a dye that fluoresces when reacted with a peptidase. Examples of such dyes include, but are not limited to, (CBZ-Ala-Ala-Ala-Ala)2-R110 elastase 2; (CBZ-Ala-Ala-Asp)2-R110 granzyme B; and 7-amino-4-methylcoumarin, N-CBZ-L-aspartyl-L-glutamyl-L-valyl-L-aspartic acid amide.

In some disclosures, the composition of this disclosure includes a dye selected from the group consisting of resazurin, fluorescein diacetate fluorescein diacetate (FDA), Calcein AM, and SYTO^{®} 9. **In** some disclosures, the dye is FDA or SYTO^{®} 9. In some disclosures, the methods described herein may make use of more than one dye (e.g., two, three, four, five, six, seven, eight, nine, ten, or more dyes). The use of multiple dyes allows for the detection of multiple analytes (e.g., multiplexing), for example, when each dye is detectable at a different wavelength. More generally, multiple dyes operating with different fluorescent wavelengths can be used as appropriate.

SYTO^{®} 9, when used alone, labels nucleic acids of bacterial cells. The excitation/emission wavelengths for SYTO^{®} 9 is 480/500 nm, with the background remaining non-fluorescent. *See, e.g.,* J. Appl. Bacteriol. 72, 410 (1992); Lett. Appl. Microbiol. 13, 58 (1991); Curr. Microbiol. 4, 321 (1980); J. Microbiol. Methods 13, 87 (1991); and Microbiol. Rev. 51, 365 (1987); and J. Med. Microbiol. 39, 147 (1993).

FDA is a non-polar, non-fluorescent compound that can cross the membranes of cells (e.g., mammalian, archaeal, and bacterial cells). The acetyl esterases (present only within viable cells) hydrolyze the FDA into the fluorescent compound fluorescein. Fluorescein is a fluorescent polar compound that is retained within these cells. Living cells can be visualized in a photospectrometer when assayed with an excitation wavelength of 494 nm and an emission wavelength of 518 nm. *See, e.g.,* Brunius, G. (1980). Technical aspects of the use of 3', 6' - Diacetyl fluorescein for vital fluorescent staining of bacteria. Current Microbiol. 4:321-323; Jones, K. H. and Senft, J. A. (1985). An improved method to determine cell viability by simultaneous staining with fluorescein diacetate - propidium iodide. J. Histochem. Cytochem. 33:77-79; Ross, R. D. , Joneckis, C. C., Ordonez, J. V., Sisk, A. M., Wu, R. K., Hamburger, A. W., and Nora, R. E. (1989). Estimation of cell survival by flow cytometric quantification of fluorescein diacetate/propidium iodide viable cell number. Cancer Research. 49:3776 - 3782.

Calcein-AM, which is an acetoxylmethyl ester of calcein, is highly lipophilic and cell permeable. Calcein-AM in itself is not fluorescent, but the calcein generated by esterase in a viable cell emits a green fluorescence with an excitation wavelength of 490 nm and an emission of 515 nm. Therefore, Calcein-AM can only stain viable cells. *See, e.g.,* Kimura, K., et al., Neurosci. Lett., 208, 53 (1998); Shimokawa, I., et al., J. Geronto., 51a, b49 (1998); Yoshida, S., et al., Clin. Nephrol., 49, 273 (1998); and Tominaga, H., et al., Anal. Commun., 36, 47 (1999).

Resazurin (also known as Alamar Blue) is a blue compound that can be reduced to pink resorufin which is fluorescent. This dye is mainly used in viability assays for mammalian cells. C¹² -resazurin has better cell permeability than resazurin. When lipophilic C¹² -resazurin crosses the cell membranes, it is subsequently reduced by living cells to make a red fluorescent resorufin. The adsorption/emission of C¹² -resazurin is 563/587 nm. *See, e.g.,* Appl Environ Microbiol 56, 3785 (1990); J Dairy Res 57, 239 (1990); J Neurosci Methods 70, 195 (1996); J Immunol Methods 210, 25 (1997); J Immunol Methods 213, 157 (1998); Antimicrob Agents Chemother 41, 1004 (1997).

Also provided herein are methods for detecting or quantifying viable archaeal cells (e.g., methanogenic archaeal cells) in a sample obtained using an ingestible device described herein. Coenzyme F-420 is a redox-active 5-deazaflavin derivative present in all methanogens, yet absent in commensal microflora and human cells (see, e.g., Hendrickson and Leigh (2008) J. Bacteriol. 190(14):4818-4821; and Greening et al. (2016) Microbiology and Molecular Biology Reviews 80(2):451-93). In its oxidized state, the absorbance spectrum of F-420 peaks at 420 nm, and the emission spectrum peaks at 470 nm (Greening *et al.* (2016)). Thus, the disclosure provides methods for detecting the presence and/or amount of viable archaeal cells (e.g., methanogenic archaeal cells) in a sample obtained from the GI tract in the subject by measuring total fluorescence or rate of change of fluorescence as a function of time of the sample by exciting the sample, e.g., at 420 nm. This process can be performed within the ingestible device or *ex vivo* after the sample has been removed from the ingestible device described herein. In some disclosures, the sample is admixed with a composition comprising a dye described herein (e.g., resazurin). The sample may be excited at 420 nm, such that the coenzyme F-420 present in the sample (e.g., from lysed or intact archaea) is excited thereby acting as an energy donor that activates the dye.

In some disclosures, the composition of this disclosure optionally further includes a reagent for selective lysis of eukaryotic cells. In some disclosures, the composition includes a dye as described herein and a reagent for selective lysis of eukaryotic cells. In some disclosures, the reagent for selective lysis of eukaryotic cells is a detergent, such as a non-ionic or an ionic detergent. Examples of the reagent for selective lysis of eukaryotic cells include, but are not limited to, alkylglycosides, Brij 35 (C12E23 polyoxyethyleneglycol dodecyl ether), Brij 58 (C16E20 Polyoxyethyleneglycol dodecyl ether), Genapol, glucanids such as MEGA-8, -9, -10, octylglucoside, Pluronic F127, Triton X-100^{™} (C₁₄H₂₂O(C₂H₄O)ₙ), Triton X-114 (C₂₄H₄₂O₆), Tween 20 (Polysorbate 20) and Tween 80 (Polysorbate 80), Nonidet P40, deoxycholate, reduced Triton X-100^{™} and/or Igepal CA 630. In some disclosures, the composition of this disclosure includes a dye as described herein and deoxycholate (*e.g.,* sodium deoxycholate) as a reagent for selective lysis of eukaryotic cells. In some disclosures, the composition includes deoxycholate at a concentration from about 0.0001% to about 1 wt%. In some disclosures, the composition includes deoxycholate at a concentration of about 0.005 wt%. In some disclosures, the composition includes more than one reagent for selective lysis of eukaryotic cells.

In some disclosures, the composition may include two different reagents for selective lysis of eukaryotic cells. In some instances, when more than one selective lysis reagents are used, more effective and/or complete selective lysis of eukaryotic cells in a sample may be achieved. For example, the composition may include deoxycholate (*e.g.,* sodium deoxycholate) and Triton X-100^{™} as two different reagents for selective lysis of eukaryotic cells. In some disclosures, the composition includes deoxycholate (e.g., sodium deoxycholate) at a concentration of about 0.0001% to about 1 wt% *(e.g.,* about 0.005 wt%) and Triton X-100^{™} at a concentration selected of about 0.1 to about 0.05 wt%.

In some disclosures, after a sample *(e.g.,* a biological sample) is treated or contacted with a composition including a dye and one or more reagents for selective lysis of eukaryotic cells as described herein, the eukaryotic cells (*e.g.,* animal cells) in the sample are selectively lysed whereby a substantial percentage (*e.g.,* more than about 20%, about 40%, about 60%, about 80%, about 90% or even more than about 95%) of the bacterial and/or archaeal cells in the same sample remains intact or alive.

In some disclosures, the composition does not include a reagent for selective lysis of eukaryotic cells, and such a composition is useful for detecting or quantifying viable bacterial and/or archaeal cells in a sample *(e.g.,* an environmental sample such as a water sample) that does not contain any eukaryotic cells.

In some disclosures, the composition of this disclosure further includes an electrolyte, such as a divalent electrolyte (*e.g.,* MgCl₂). In some disclosures, the composition includes MgCl₂ at a concentration of about 0.1 mM to about 100 mM (*e.g.,* a concentration of about 0.5 mM to about 50 mM).

In some disclosures, the composition of this disclosure further includes water and is in a form of an aqueous solution. In some disclosures, the composition has a pH of about 5-8 (*e.g.,* a pH of about 6-7.8, such as pH of about 6.0). In some embodiments, the composition is a solid or a semi-solid.

In some disclosures, the composition further includes an anti-fungal agent. Suitable anti-fungal agents for use herein include, but are not limited to, fungicidal and fungistatic agents including terbinafine, itraconazole, micronazole nitrate, thiapendazole, tolnaftate, clotrimazole and griseofulvin. In some embodiments, the anti-fungal agent is a polyene anti-fungal agent, such as amphotericin-B, nystatin, and pimaricin.

In some disclosures, the composition does not contain any anti-fungal agent. In some embodiments, the composition contains broad spectrum antibiotics but not any anti-fungal agent. Such compositions that do not contain anti-fungal agents but contain broad spectrum antibiotics may be useful in detecting or quantifying fungi (*e.g.,* yeast) in a sample.

In some disclosures, the composition does not contain any anti-fungal agent or any antibiotics. Such compositions that do not selectively lyse mammalian cells may be useful in detecting or quantifying mammalian cells (e.g., cells from the GI tract) in a sample since many dyes have a higher affinity for mammalian as compared to bacteria or fungi cells. In some disclosures, the composition contains broad spectrum antibiotics and one or more anti-fungal agents. Such compositions that contain anti-fungal agents and broad spectrum antibiotics may be useful in detecting or quantifying mammalian cells (e.g., cells from the GI tract) in a sample. The detection or quantification of mammalian cells may be useful for determining cell turnover in a subject. High cell turnover is sometimes associated with a GI injury (e.g., lesion), the presence of a tumor(s), or radiation-induced colitis or radiation enteropathy.

In some disclosures, the composition further includes an antibiotic agent as described herein. Such a composition may be useful in detecting or quantifying antibiotic-resistant strains of bacteria and/or archaea in a sample.

In disclosuresembodiments, the composition of this disclosure includes Triton X-100, deoxycholate, resazurin, and MgCl₂. In some disclosures, the composition includes Triton X-100^{™}, deoxycholate, resazurin, amphotericin-B and MgCl₂. In some embodiments, the composition includes about 0.1 wt% or about 0.05 wt% Triton X-100^{™}; about 0.005 wt% deoxycholate; about 10 mM resazurin; about 2.5 mg/L amphotericin-B and about 50 mM MgCl₂. In some disclosures, the composition has a pH of about 6.0.

In some disclosures, the compositions of this disclosure are suitable for use in a kit or device, *e.g.,* for detecting or quantifying viable bacterial and/or archaeal cells in a sample. In some disclosures, such a device is an ingestible device for detecting or quantifying viable bacterial and/or archaeal cells *in vivo (e.g.,* in the GI tract).

This disclosure provides a method for detecting the presence of viable bacterial and/or archaeal cells in a sample, including: (a) contacting the sample with a composition as described herein; and (b) measuring total fluorescence or rate of change of fluorescence as a function of time of the sample, thereby detecting viable bacterial and/or archaeal cells in the sample.

In some disclosures, the total fluorescence or the rate of change of fluorescence as a function of time of the sample is measured over multiple time points for an extended period of time in step (b), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the method further includes correlating the total fluorescence or the rate of change of fluorescence as a function of time determined in step (b) to the number of viable bacterial and/or archaeal cells in the sample. In some disclosures, the method does not require *ex vivo* plating or culturing. In some disclosures, the method does not require aspiration. In some disclosures, the method is performed *in vivo (e.g.,* in an ingestible device *in vivo).* In some disclosures, the method includes communicating the results of the onboard assay(s) to an *ex vivo* receiver.

In some disclosures, a control may be employed in the method as described herein. Such a control may be a positive control, *e.g.,* a composition as described herein further including a known number of viable bacterial and/or archaeal cells. In some disclosures, the control may be a negative control, *e.g.,* a composition as described herein that has not been contacted with any viable bacterial and/or archaeal cells. In some disclosures, this disclosure provides a method for detecting the presence of viable bacterial and/or archaeal cells in a sample, including: (a) contacting the sample with a composition as described herein; (b) measuring total fluorescence or rate of change of fluorescence as a function of time of the sample; and (c) comparing the total fluorescence measured in step (b) to the total fluorescence produced by a control as described herein, or comparing the rate of change of fluorescence as a function of time measured in step (b) to the rate of change of fluorescence as a function of time produced by a control as described herein, thereby detecting viable bacterial and/or archaeal cells.

In some disclosuresof the method, the control may be (1) a composition identical to the one used in step (a) but has not been contacted with any viable bacterial and/or archaeal cells; or (2) a composition identical to the one used in step (a) further including a known number of viable bacterial and/or archaeal cells *(e.g.,* a composition identical to the one used in step (a) further including 10², 10³, 10⁴, 10⁵, 10⁶, or 10⁷ CFU/mL of bacterial and/or archaeal cells). In some disclosures, the total fluorescence or the rate of change of fluorescence as a function of time of the sample is measured over multiple time points for an extended period of time in step (b), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some embodiments, the method further includes correlating the comparative total fluorescence determined in step (c) to the number of viable bacterial and/or archaeal cells in the sample. In some disclosures, the rate of change of fluorescence as a function of time of the sample measured over multiple time points is determined and compared to the rate of change of fluorescence as a function of time of a control measured over the same time points to determine the number of viable bacterial and/or archaeal cells in the sample. In some disclosures, the method does not require *ex vivo* plating or culturing. In some disclosures, the method does not require aspiration. In some disclosures, the method is performed *in vivo (e.g.,* in an ingestible device *in vivo).* In some embodiments, the method includes communicating the results of the onboard assay(s) to an *ex vivo* receiver.

In some disclosures, methods as described herein are highly sensitive in detecting or quantifying viable bacterial and/or archaeal cells in various samples. In some disclosures, the lowest detection or quantification limit of the present methods is 10² CFU/mL. In some disclosures, the highest detection or quantification limit of the present methods is 10⁷ CFU/mL, 10⁸ CFU/mL, 10⁹ CFU/mL, 10¹⁰ CFU/mL or more. In some disclosures, the methods allow detection or quantification of 10² to 10⁷ CFU/mL bacterial and/or archaeal cells in various samples. In some disclosures, methods of this disclosure may be used to distinguish samples bases on the quantity of viable bacterial and/or archaeal cells contained therein. For instance, the methods may be used to distinguish among samples including 10², 10³, 10⁴, 10⁵, 10⁶, or 10⁷ CFU/mL of bacterial and/or archaeal cells.

This disclosure provides a kit including a composition as described herein and instructions, *e.g*., for detecting or quantifying viable bacterial and/or archaeal cells in a sample. This disclosure also provides a device *(e.g.,* an ingestible device) including a composition as described herein, *e.g.,* for detecting or quantifying viable bacterial and/or archaeal cells in a sample. The detection of live cells is the gold standard of viable plate counting and represents one of the advantages of exemplary compositions and methods described herein.

This disclosure also provides a method of assessing or monitoring the need to treat a subject suffering from or at risk of overgrowth of bacterial and/or archaeal cells in the GI tract, including: (a) obtaining a sample from the GI tract of the subject; (b) contacting the sample with a composition as described herein; (c) measuring total fluorescence or rate of change of fluorescence as a function of time of the sample; and (d) correlating the total fluorescence or the rate of change of fluorescence as a function of time measured in step (c) to the number of viable bacterial and/or archaeal cells in the sample, wherein the number of the viable bacterial and/or archaeal cells determined in step (d) greater than about 10⁵ CFU/mL indicates a need for treatment, *e.g*., with an antibiotic agent as described herein.

In some disclosures, the total fluorescence or the rate of change of fluorescence as a function of time of the sample is measured over multiple time points for an extended period of time in step (c). For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes In some disclosures, the total fluorescence or the rate of change of fluorescence as a function of time of the sample is measured continuously for a period of 0-330 minutes. In some disclosures, the method does not require *ex vivo* plating or culturing. In some disclosures, the method is performed *in vivo (e.g.,* in an ingestible device *in vivo).* In some disclosures, the method includes communicating the results of the onboard assay(s) to an *ex vivo* receiver.

In some disclosures, a control may be used in the method of assessing the need to treat a subject suffering from or at risk of overgrowth of bacterial and/or archaeal cells in the GI tract. Such a control may be a positive control, *e.g.,* a composition as described herein further including a known number of viable bacterial and/or archaeal cells. In some disclosures, the control may be a negative control, *e.g.,* a composition as described herein that has not been contacted with any viable bacterial and/or archaeal cells. In some disclosures, this disclosure provides a method of assessing or monitoring the need to treat a subject suffering from or at risk of overgrowth of bacterial and/or archaeal cells in the GI tract, including: (a) obtaining a sample from the GI tract of the subject; (b) contacting the sample with a composition as described herein; (c) measuring total fluorescence of the sample; (d) comparing the total fluorescence measured in step (c) to the total fluorescence produced by a control as described herein; and (e) correlating the comparative fluorescence determined in step (d) to the number of viable bacterial and/or archaeal cells in the sample, wherein the number of the viable bacterial and/or archaeal cells determined in step (e) greater than about 10⁵ CFU/mL indicates a need for treatment, *e.g*., with an antibiotic agent as described herein.

In some disclosures, this disclosure provides a method of assessing or monitoring the need to treat a subject suffering from or at risk of overgrowth of bacterial and/or archaeal cells in the GI tract, including: (a) obtaining a sample from the GI tract of the subject; (b) contacting the sample with a composition as described herein; (c) measuring rate of change of fluorescence as a function of time of the sample; (d) comparing the rate of change of fluorescence as a function of time measured in step (c) to the rate of change of fluorescence as a function of time produced by a control as described herein; and (e) correlating the comparative rate of change of fluorescence as a function of time determined in step (d) to the number of viable bacterial and/or archaeal cells in the sample. The number of the viable bacterial and/or archaeal cells determined in step (e) greater than about 10⁵ CFU/mL indicates a need for treatment, *e.g.*, with an antibiotic agent as described herein.

In some disclosuresof the method, the control may be (1) a composition identical to the one used in step (b) but has not been contacted with any viable bacterial and/or archaeal cells; or (2) a composition identical to the one used in step (b) further including a known number of viable bacterial and/or archaeal cells *(e.g.,* a composition identical to the one used in step (b) further including 10², 10³, 10⁴, 10⁵, 10⁶, or 10⁷ CFU/mL of bacterial and/or archaeal cells). In some disclosures, the total fluorescence or the rate of change of fluorescence as a function of time of the sample is measured over multiple time points for an extended period of time in step (c), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the rate of change of fluorescence as a function of time of the sample measured over multiple time points is determined and compared to the rate of change of fluorescence as a function of time of a control measured over the same time points to determine the number of viable bacterial and/or archaeal cells in the sample. In some embodiments, the method does not require *ex vivo* plating or culturing. In some disclosures, the method is performed *in vivo (e.g.,* in an ingestible device *in vivo).* In some disclosures, the method includes communicating the results of the onboard assay(s) to an *ex vivo* receiver. In some disclosures, the method may be further used to monitor the subject after the treatment (*e.g.,* with an antibiotic). In some disclosures, the method may be used to assess the efficacy of the treatment. For example, efficacious treatment may be indicated by the decrease of the number of viable bacterial and/or archaeal cells in a sample from the GI tract of the subject post-treatment. Efficacy of the treatment may be evaluated by the rate of decrease of the number of viable bacterial and/or archaeal cells in a sample from the GI tract of the subject post-treatment. In some disclosures, the method may be used to detect infection with antibiotic-resistant strains of bacteria and/or archaea in a subject. For instance, such infection may be indicated where the number of viable bacterial and/or archaeal cells in a sample from the GI tract of the subject does not substantially decrease after antibiotic treatment.

The present disclosure provides a member made of an absorptive material (*e.g.,* an absorptive sponge) having absorbed therein a composition (*e.g.,* a composition as described herein) including a dye and a reagent for selective lysis of eukaryotic cells. In some disclosures, the absorptive sponge is a hydrophilic sponge. In some disclosures, the absorptive sponge is selected from the group consisting of: fibers of cotton, rayon, glass, polyester, polyethylene, polyurethane, nitrocellulose, and the like. In some embodiments, the absorptive sponge is polyester or polyethylene. In some disclosures, the absorptive sponge is selected from the group consisting of: Ahlstrom Grade 6613H, Porex 1/16" Fine Sheet 4897, Porex 1/8" Fine Sheet 4898, Porex 4588 0.024" Conjugate release pad, Porex PSU-567, and Filter Papers. In some disclosures, the absorptive sponge is Ahlstrom Grade 6613H (Lot 150191) or Porex PSU-567.

The present disclosure further provides a method for preparing an absorptive sponge as described herein, including the step of injecting into the absorptive sponge an aqueous solution including a composition of the present disclosure. In some disclosures, the method including a step of drying the absorptive sponge having absorbed therein the aqueous solution at a temperature in the range of 0-100°C, 0-50°C, 0-40°C, 0-30°C, 0-20°C, 0-10°C, or 0-4°C), for a time period sufficient to reduce the total water content to below 50%, 40%, 30%, 20%, 15%, 10%, 7%, 5%, 3%, 1%, 0.7%, 0.5%, 0.3%, or 0.1% by weight.

In some disclosures, the absorptive sponge of this disclosure are suitable for use in a kit or device, *e.g.,* for detecting or quantifying viable bacterial and/or archaeal cells in a sample. In some embodiments, such a device is an ingestible device for detecting or quantifying viable bacterial and/or archaeal cells *in vivo (e.g.,* in the GI tract).

This disclosure provides a method for detecting the presence of viable bacterial and/or archaeal cells in a sample, including: (a) fully or partially saturating (*e.g.,* at 50% or half saturation) an absorptive sponge as described herein, or an absorptive sponge prepared according to a method as described herein, with the sample; and (b) measuring total fluorescence or rate of change of fluorescence as a function of time of the fully or partially saturated sponge *(e.g.,* 50% or half-saturated) prepared in step (a), thereby detecting viable bacterial and/or archaeal cells in the sample.

In some disclosures, the total fluorescence or the rate of change of fluorescence as a function of time of the sponge is measured over multiple time points for an extended period of time in step (b), thereby detecting viable bacterial and/or archaeal cells in the sample. For instance, in some disclosures, the total fluorescence or the rate of change of fluorescence as a function of time of the sample is measured continuously for a period of 0-1000 minutes, 0-900 minutes, 0-800 minutes, 0-700 minutes, 0-600 minutes, 0-500 minutes, 0-400 minutes, 0-350 minutes, or 0-330 minutes. In some disclosures, the total fluorescence or the rate of change of fluorescence as a function of time of the sample is measured continuously for a period of 0-330 minutes. In some disclosures, the method further includes correlating the total fluorescence or the rate of change of fluorescence as a function of time determined in step (b) to the number of viable bacterial and/or archaeal cells in the sample. In some disclosures, the method does not require *ex vivo* plating or culturing. In some disclosures, the method does not require aspiration. In some disclosures, the method is performed *in vivo (e.g.,* in an ingestible device *in vivo).* In some disclosures, the method includes communicating the results of the onboard assay(s) to an *ex vivo* receiver.

In some disclosures, a control may be employed in the method as described herein. Such a control may be a positive control, *e.g.,* an absorptive sponge as described herein further including a known number of viable bacterial and/or archaeal cells. In some embodiments, the control may be a negative control, *e.g.,* an absorptive sponge as described herein that has not been contacted with any viable bacterial and/or archaeal cells.

In some disclosures, this disclosure provides a method for detecting the presence of viable bacterial and/or archaeal cells in a sample, including: (a) fully or partially saturating *(e.g.,* at 50% or half saturation) an absorptive sponge as described herein, or an absorptive sponge prepared according to a method as described herein, with the sample; (b) measuring total fluorescence or rate of change of fluorescence as a function of time of the fully or partially saturated sponge *(e.g.,* at 50% or half saturation) prepared in step (a); and (c) comparing the total fluorescence measured in step (b) to the total fluorescence produced by a control as described herein, or comparing the rate of change of fluorescence as a function of time measured in step (b) to the rate of change of fluorescence as a function of time produced by a control as described herein, thereby detecting viable bacterial and/or archaeal cells.

In some disclosures of the method, the control may be (1) an absorptive sponge identical to the one used in step (a) that has not been contacted with any viable bacterial and/or archaeal cells, or (2) an absorptive sponge identical to the one used in step (a) and is fully or partially saturated with a solution including a known number of viable bacterial and/or archaeal cells (*e.g.,* an absorptive sponge identical to the one used in step (a) further including 10², 10³, 10⁴, 10⁵, 10⁶, or 10⁷ CFU/mL of bacterial and/or archaeal cells). In some disclosures, the total fluorescence or the rate of change of fluorescence as a function of time of the sponge is measured over multiple time points for an extended period of time in step (b), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the method further includes correlating the comparative total fluorescence determined in step (c) to the number of viable bacterial and/or archaeal cells in the sample. In some disclosures, the rate of change of fluorescence as a function of time of the fully or partially saturated sponge measured over multiple time points is determined and compared to the rate of change of fluorescence as a function of time of a control measured over the same time points to determine the number of viable bacterial and/or archaeal cells in the sample. In some disclosures, the method does not require *ex vivo* plating or culturing. In some disclosures, the method does not require aspiration. In some disclosures, the method is performed *in vivo (e.g.,* in an ingestible device *in vivo).* In some disclosures, the method includes communicating the results of the onboard assay(s) to an *ex vivo* receiver.

In some disclosures, methods as described herein are highly sensitive in detecting and quantifying viable bacterial and/or archaeal cells in various samples. In some disclosures, the lowest detection or quantification limit of the present methods is about 10² CFU/mL. In some disclosures, the highest detection or quantification limit of the present methods is about 10⁷ CFU/mL, about 10⁸ CFU/mL, about 10⁹ CFU/mL, about 10¹⁰ CFU/mL or more. In some disclosures, the methods allow detection or quantification of about 10² to about 10⁷ CFU/mL bacterial and/or archaeal cells in various samples. In some disclosures, methods of this disclosure may be used to distinguish samples bases on the quantity of viable bacterial and/or archaeal cells contained therein. For instance, the methods may be used to distinguish among samples that contain about 10², 10³, 10⁴, 10⁵, 10⁶, or 10⁷ CFU/mL of bacterial and/or archaeal cells.

This disclosure provides a kit including an absorptive sponge as described herein and instructions, *e.g*., for detecting or quantifying viable cells using the absorptive sponge. This disclosure also provides a device (*e.g.,* an ingestible device) including an absorptive sponge as described herein, *e.g.,* for detecting or quantifying viable bacterial and/or archaeal cells in a sample.

This disclosure provides a method of assessing or monitoring the need to treat a subject suffering from or at risk of overgrowth of bacterial and/or archaeal cells in the GI tract, including: (a) obtaining a sample from the GI tract of the subject; (b) fully or partially saturating (*e.g.,* at 50% or half saturation) an absorptive sponge as described herein, or an absorptive sponge prepared according to a method as described herein, with the sample; (c) measuring total fluorescence or rate of change of fluorescence as a function of time of the fully or partially saturated sponge *(e.g.,* at 50% or half saturation) prepared in step (b); and (d) correlating the total fluorescence or the rate of change of fluorescence as a function of time measured in step (c) to the number of viable bacterial and/or archaeal cells in the sample, wherein the number of the viable bacterial and/or archaeal cells determined in step (d) greater than about 10⁵ CFU/mL indicates a need for treatment, *e.g.,* with an antibiotic agent as described herein.

In some disclosures, the total fluorescence or the rate of change of fluorescence as a function of time of the fully or partially saturated sponge is measured over multiple time points for an extended period of time in step (c). For instance, in some disclosures, the total fluorescence or the rate of change of fluorescence as a function of time of the fully or partially saturated sponge is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the fully or partially saturated sponge is measured continuously for a period of 0-330 minutes. In some disclosures, the method does not require *ex vivo* plating or culturing. In some disclosures, the method does not require aspiration. In some disclosures, the method is performed *in vivo (e.g.,* in an ingestible device *in vivo).*

In some embodiments, a control may be used in the method of assessing the need to treat a subject suffering from or at risk of overgrowth of bacterial and/or archaeal cells in the GI tract. Such a control may be a positive control, *e.g.,* an absorptive sponge as described herein further including a known number of viable and/or archaeal bacterial cells. In some embodiments, the control may be a negative control, *e.g.,* an absorptive sponge as described herein that has not been contacted with any viable and/or archaeal bacterial cells.

In some disclosures, this disclosure provides a method of assessing or monitoring the need to treat a subject suffering from or at risk of overgrowth of bacterial and/or archaeal cells in the GI tract, including: (a) obtaining a sample from the GI tract of the subject; (b) fully or partially saturating (*e.g.,* at 50% or half saturation) an absorptive sponge as described herein, or an absorptive sponge prepared according to a method as described herein, with the sample; (c) measuring total fluorescence of the fully or partially saturated sponge (*e.g.,* at 50% or half saturation) prepared in step (b); (d) comparing the total fluorescence measured in step (c) to the total fluorescence produced by a control as described herein; and (e) correlating the comparative fluorescence determined in step (d) to the number of viable bacterial and/or archaeal cells in the sample, wherein the number of the viable bacterial and/or archaeal cells determined in step (e) greater than about 10⁵ CFU/mL indicates a need for treatment, *e.g.*, with an antibiotic agent as described herein.

In some disclosures, this disclosure provides a method of assessing or monitoring the need to treat a subject suffering from or at risk of overgrowth of bacterial and/or archaeal cells in the GI tract, including: (a) obtaining a sample from the GI tract of the subject; (b) fully or partially saturating (*e.g.,* at 50% or half saturation) an absorptive sponge as described herein, or an absorptive sponge prepared according to a method as described herein, with the sample; (c) measuring rate of change of fluorescence as a function of time of the fully or partially saturated sponge (*e.g.,* at 50% or half saturation) prepared in step (b); (d) comparing the rate of change of fluorescence as a function of time measured in step (c) to the rate of change of fluorescence as a function of time produced by a control as described herein; and (e) correlating the comparative rate of change of fluorescence as a function of time determined in step (d) to the number of viable bacterial and/or archaeal cells in the sample, wherein the number of the viable bacterial and/or archaeal cells determined in step (e) greater than about 10⁵ CFU/mL indicates a need for treatment, *e.g.*, with an antibiotic agent as described herein.

In some disclosures of the method, the control may be (1) an absorptive sponge identical to the one used in step (a) that has not been contacted with any viable bacterial and/or archaeal cells, or (2) an absorptive sponge identical to the one used in step (a) and is fully or partially saturated with a solution including a known number of viable bacterial and/or archaeal cells (*e.g.,* a composition identical to the one used in step (b) further including 10², 10³, 10⁴, 10⁵, 10⁶, or 10⁷ CFU/mL of bacterial and/or archaeal cells). In some disclosures, the total fluorescence or the rate of change of fluorescence as a function of time of the sponge is measured over multiple time points for an extended period of time in step (c), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the rate of change of fluorescence as a function of time of the fully or partially saturated sponge measured over multiple time points is determined and compared to the rate of change of fluorescence as a function of time of a control measured over the same time points to determine the number of viable bacterial and/or archaeal cells in the sample. In some disclosures, the method does not require *ex vivo* plating or culturing. In some disclosures, the method does not require aspiration. In some disclosures, the method is performed *in vivo (e.g.,* in an ingestible device *in vivo).* In some disclosures, the method includes communicating the results of the onboard assay(s) to an *ex vivo* receiver. In some disclosures, the method may be further used to monitor the subject after the treatment (*e.g.,* with an antibiotic). In some disclosures, the method may be used to assess the efficacy of the treatment. For example, efficacious treatment may be indicated by the decrease of the number of viable bacterial and/or archaeal cells in a sample from the GI tract of the subject post-treatment. Efficacy of the treatment may be evaluated by the rate of decrease of the number of viable bacterial and/or archaeal cells in a sample from the GI tract of the subject post-treatment. In some disclosures, the method may be used to detect infection with antibiotic-resistant strains of bacteria and/or archaea in a subject. For instance, such infection may be indicated where the number of viable bacterial and/or archaeal cells in a sample from the GI tract of the subject does not substantially decrease after antibiotic treatment.In some disclosures, fluorescence intensity is measured with an optical reader. The actual configuration and structure of the optical reader may generally vary as is readily understood by those skilled in the art. Typically, the optical reader contains an illumination source that is capable of emitting light at a defined wavelength and a detector that is capable of registering a signal (*e.g.,* transmitted, reflected, or fluorescence light). Optical readers may generally employ any known detection technique, including, for instance, luminescence *(e.g.,* fluorescence, phosphorescence, etc.), absorbance (*e.g.,* fluorescent or non-fluorescent), diffraction, etc. Exemplary optical readers, illumination sources and detectors are disclosed in U.S. Patent 7,399,608.

In some disclosures, the illumination source may be any device known in the art that is capable of providing electromagnetic radiation, such as light in the visible or near-visible range (*e.g.,* infrared or ultraviolet light). For example, suitable illumination sources that may be used in the present disclosure include, but are not limited to, light emitting diodes (LED), flashlamps, cold-cathode fluorescent lamps, electroluminescent lamps, and so forth. The illumination may be multiplexed and/or collimated. In some disclosures, the illumination may be pulsed to reduce any background interference. In some disclosures, filters may be used to improve optics. *See, e.g.,* Reichman, Jay, Handbook of optical filters for fluorescence microscopy, Chroma Technology Corporation (2000). In some disclosures, excitation source may be a LED with a band-pass filter, *e.g.,* a filter for 500 nm +/-10 nm wavelength to selectively excite a sample with 500 nm light. In some disclosures, to cut out any stray longer wavelengths from the green LED, a Thorlabs FESH0550 shortpass filter may be used for excitation (FIG. 72A). In some disclosures, the emission from a sample is captured at a 90° angle with an avalanche photodiode detector with a bandpass filter, *e.g.,* a filter for 590 nm +/- 20 nm wavelength, placed in front of the detector, to selectively capture light emitted at 590 nm. In some disclosures, a Thorlabs FB580-10 bandpass filter may be used as an emission filter (FIG. 72B). A cross sectional view of an exemplary fluorescent assay test fixture depicting collimating, focusing, and filtering lenses is shown in FIG. 72C. In some disclosures, a 5-50 nanosecond delay may be used before emission is measured. Typical fluorophore used for time delayed fluorescence are lanthanide metal chelates (Europium, Samarium, Terbium, etc.), ruthenium complexes and others known in the art. In some disclosures, illumination may be continuous or may combine continuous wave (CW) and pulsed illumination where multiple illumination beams are multiplexed (*e.g.,* a pulsed beam is multiplexed with a CW beam), permitting signal discrimination between a signal induced by the CW source and a signal induced by the pulsed source. For example, in some disclosures, LEDs (*e.g.,* aluminum gallium arsenide red diodes, gallium phosphide green diodes, gallium arsenide phosphide green diodes, or indium gallium nitride violet/blue/ultraviolet (UV) diodes) are used as the pulsed illumination source. In some disclosures, the illumination source may provide diffuse illumination to the dye. For example, an array of multiple point light sources (*e.g*., LEDs) may simply be employed to provide relatively diffuse illumination. In some disclosures, the illumination source is capable of providing diffuse illumination in a relatively inexpensive manner is an electroluminescent (EL) device. An EL device is generally a capacitor structure that utilizes a luminescent material (*e.g.,* phosphor particles) sandwiched between electrodes, at least one of which is transparent to allow light to escape. Disclosure of a voltage across the electrodes generates a changing electric field within the luminescent material that causes it to emit light.

In some disclosures, the detector may be any device known in the art that is capable of sensing a signal. In some disclosures, the detector may be an electronic imaging detector that is configured for spatial discrimination. Some examples of such electronic imaging sensors include high speed, linear charge-coupled devices (CCD), charge-injection devices (CID), complementary-metal-oxide-semiconductor (CMOS) devices, and so forth. Such image detectors, for instance, are generally two-dimensional arrays of electronic light sensors, although linear imaging detectors (*e.g.,* linear CCD detectors) that include a single line of detector pixels or light sensors, such as, for example, those used for scanning images, may also be used. Each array includes a set of known, unique positions that may be referred to as "addresses." Each address in an image detector is occupied by a sensor that covers an area (*e.g.,* an area typically shaped as a box or a rectangle). This area is generally referred to as a "pixel" or pixel area. A detector pixel, for instance, may be a CCD, CID, or a CMOS sensor, or any other device or sensor that detects or measures light. The size of detector pixels may vary widely, and may in some cases have a diameter or length as low as 0.2 micrometers.

In other disclosures, the detector may be a light sensor that lacks spatial discrimination capabilities. For instance, examples of such light sensors may include photomultiplier devices, photodiodes, such as avalanche photodiodes or silicon photodiodes, and so forth. Silicon photodiodes are sometimes advantageous in that they are inexpensive, sensitive, capable of high-speed operation (short risetime/high bandwidth), and easily integrated into most other semiconductor technology and monolithic circuitry. In addition, silicon photodiodes are physically small, which enables them to be readily incorporated into various types of detection systems. If silicon photodiodes are used, then the wavelength range of the emitted signal may be within their range of sensitivity, which is 400 to 1100 nanometers. In some disclosures, a photomultiplier may be used to increase the intensity of the signal.

The present disclosure provides ingestible devices containing a microscopic evaluation system. In some disclosures, bacterial and/or archaeal cells in a sample may be first labeled with fluorescent dyes (such as those described herein), and the fluorescently-labeled cells may be imaged and counted by the microscopic evaluation using an ingestible device as described herein. In other disclosures, the fluorescently-labeled cells are counted as they pass through an onboard flow system (*e.g.,* microfluidic single cell channeling). Examples of flow cytometry systems include hydrodynamic focusing, small diameter capillary tube flow, and rectangular capillary tube flow. As described herein, live bacterial and/or archaeal cells are labeled, and the principles of flow cytometry are used to quantify labeled cells. Generally speaking, the photons from an incident laser beam are absorbed by the fluorophore and raised to a higher, unstable energy level. Within less than a nanosecond, the fluorophore re-emits the light at a longer representative wavelength where it is passed through a series of dichroic filters. This reemitted light can be collected and interpreted as proportional to the number of labeled bacterial and/or archaeal cells. In some disclosures, a sheath fluid is not used as part of the flow system to help accommodate the volume restrictions of the device. In some disclosures, a rectangular capillary tube is used to achieve a sufficiently large cross-sectional area and relatively thin inspection area. The flow cytometry optical system operates parallel to the fluidics system and serves to observe the redirection of light passing through the cell and delivers information about the bacterial and/or archaeal cells. In some disclosures, rather than using a conventional laser and spherical lenses to focus the light to a point, an LED and cylindrical lenses are used to focus the light to a line across a rectangular capillary tube. In other disclosures, collimating lenses are used to make the light source parallel, while cylindrical lenses are used to refine the inspection area. An exemplary optical configuration for this arrangement can be seen in FIG. 30. In some disclosures, optical filters can be added to permit the use of fluorophores. The characteristic wavelength of reemitted light from the fluorophores can be isolated and detected with the use of dichroic, bandpass, and short or long wave pass filters. Generally, multiple dichroic lenses and photomultipliers are used, however, due to space limitations, only a single side-scatter detector and forward scatter detector may be used in certain disclosures.

One of the design challenges of integrating flow cytometry into the device is to provide a pumping mechanism. Without moving fluid, individual bacterial and/or archaeal cells cannot be identified and accounted for by flow cytometry within a fixed volume of fluid. In some disclosures, a gear motor is to move fluid through the device. For example, a micromotor including a planetary gearhead (*e.g.,* with a 25:1 reduction) can provide the desired amount of torque to create fluid flow. In another embodiment, a series of piezoelectric resistors embedded in the surface of a microfabricated plate is used to create flow. In yet another embodiment, a micropump that includes a pair of one-way valves and uses a magnetic pump membrane actuated by an external magnetic field is used to create flow.

In some disclosures, the system architecture includes an opening and sealing mechanism combined with a rotary wiper which creates a pressure driven flow via a gear motor. The gear motor can be used for other functions in the device. As shown in Fig. 31, the components of the optics and flow chamber systems fit within the device. In some disclosures, the sample fluid is absorbed via a flexible membrane at the top of the capsule. In some disclosures, the gear motor has 270° of permissible travel which serves to open and fill the fluid chamber. During closure, the motor closes the ingress port while simultaneously pushing the fluid through the rectangular capillary tube where the optical system is located. The threaded component allows the flexible membrane to close and seal the ingress channel without changing the wiper height. In some disclosures, the volume of the sample chamber is 25µL, 50µL, 75µL or more. In some disclosures, two or more samples are taken from the GI tract to procure a sufficient sample size. Referring to FIG. 31, an LED on the left side of the capillary tube and the two low-light detectors on the right for capturing forward and side scatter are shown. Once the fluid passes through the capillary tube, it exits the capsule via a one-way valve. In certain disclosures, the flow system allows for the detection of cell size and internal cell complexity, in addition to cell quantitation.

In some disclosures, the ingestible devices as described herein may be used to analyze samples (*e.g.,* samples from the GI tract) to detect or quantify viable bacterial and/or archaeal cells in a sample. In some disclosures, the devices of this disclosure may be used to measure the concentration of viable bacteria and/or archaea in specific regions of the GI tract. Such data may be used to determine whether a subject has a condition in need for treatment, such as an infection, Small Intestinal Bacterial Overgrowth (SIBO), or a SIBO-related condition, or to quantify bacterial and/or archaeal populations within the GI tract (or within specific regions of the GI tract) for other diagnostic purposes.

An ingestible device used in a live cell dye method can be configured so that one or more than one samples may be analyzed.

As an example, in some disclosures, the ingestible device has only one sample chamber. In such disclosures, the chamber can be used to analyze one sample. In certain disclosures, an ingestible device having a single sample chamber can be used to analyze multiple different disclosures. The sample chamber may include a sponge that is used such that the different samples are analyzed at different points in time, such as, for example, taken at different locations within the GI tract (e.g., duodenum, jejunum, ileum) as the device passes through the GI tract. For example, a given sponge may be contacted multiple times and used for analyte detection. In some disclosures, the sponge may be contacted with non-saturating amounts of sample multiple times and used for analyte detection. Alternatively or additionally, the sample chamber may be used with multiple dyes (e.g., used in a single reaction) that are detectable at different wavelengths. Multiple analytes can be detected, for example, using different antibodies (e.g., detecting fluorescence at different wavelengths).

As another example, in certain disclosures, the ingestible device has multiple chambers for analyzing samples. In such disclosures, each chamber can be used to analyze different samples. Features noted in the preceding paragraph may be implemented with an ingestible device having multiple sample chambers.

In some disclosures, data may be generated after the ingestible device has exited the subject, or the data may be generated *in vivo* and stored on the device and recovered *ex vivo.* Alternatively, the data can be transmitted wirelessly from the ingestible device while the device is passing through the GI tract of the subject.

This disclosure provides a method for detecting the presence of viable bacterial and/or archaeal cells in a sample, including: (a) providing an ingestible device as described herein; (b) transferring a fluid sample from the GI tract of a subject into a sampling chamber of the ingestible device *in vivo;* and (c) detecting the presence of viable bacterial and/or archaeal cells in the fluid sample (*e.g., in vivo*).

In some disclosures, the method for detecting the presence of viable bacterial and/or archaeal cells in a sample includes: (a) providing an ingestible device as described herein; (b) transferring a fluid sample from the GI tract of a subject into a sampling chamber of the ingestible device *in vivo,* wherein the sampling chamber of the device is configured to hold an absorptive sponge as described herein, or an absorptive sponge prepared according to the method for preparing an absorptive sponge as described herein; (c) fully or partially saturating (*e.g.,* at 50% or half saturation) the absorptive sponge with the fluid sample; and (d) measuring total fluorescence or rate of change of fluorescence as a function of time of the fully or partially saturated sponge (*e.g.,* 50% or half-saturated) prepared in step (c), thereby detecting viable bacterial and/or archaeal cells in the sample (*e.g., in vivo*).

In some disclosures, the method described herein further includes a step of calibrating the ingestible device, wherein the fluorescent properties of the absorptive sponge contained in the sampling chamber of the device are determined prior to the introduction of the sample. In some disclosures, each ingestible device is calibrated by measuring the fluorescence of the absorptive sponge held in the sampling chamber of the device and comparing the measured florescence to a positive or negative control as described herein. In some disclosures, each ingestible device is calibrated by measuring the fluorescence of the absorptive sponge held in the sampling chamber of the device to provide a baseline fluorescence. In some disclosures, the baseline fluorescence should be within a set number (900 +/- 450 FU). In some disclosures, a subset of ingestible devices may be treated with 0, 10⁴, 10⁵, 10⁶ and 10⁷ CFU bacteria and/or archaea in duodenal or jejunal aspirates to generate a calibration curve. The calibration curve may be stored and used to quantify bacteria and/or archaea in all the devices in the batch. See, *e.g.,* David Wild, *Standardization and Calibration,* The Immunoassay Handbook, Gulf Professional Publishing, 2005. In some disclosures, the total fluorescence or the rate of change of fluorescence as a function of time of the sponge is measured over multiple time points for an extended period of time in step (d), thereby detecting viable bacterial and/or archaeal cells in the sample. For instance, in some disclosures, the total fluorescence or the rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the method further includes correlating the total fluorescence or the rate of change of fluorescence as a function of time determined in step (d) to the number of viable bacterial and/or archaeal cells in the sample. In some disclosures, the method does not require *ex vivo* plating or culturing. In some disclosures, the method does not require aspiration. In some disclosures, the method is performed in the ingestible device *in vivo.*

In some disclosures, a control may be employed in the method as described herein. Such a control may be an internal control (*e.g.,* the fluorescence coming from resorufin impurity in resazurin (900 +/- 450 FU) in the sponge may be used as an internal control for optics and amount of dye in the sponge). In some disclosures, each ingestible device as described herein is individually calibrated wherein the fluorescent properties of the absorptive sponge contained in the sampling chamber of the device are determined prior to the introduction of sample.

In some disclosures, methods as described herein are highly sensitive in detecting and quantifying viable bacterial and/or archaeal cells in various samples. In some disclosures, the lowest detection or quantification limit of the present methods is 10² CFU/mL. In some disclosures, the highest detection or quantification limit of the present methods is 10⁷ CFU/mL, 10⁸ CFU/mL, 10⁹ CFU/mL, 10¹⁰ CFU/mL or more. In some disclosures, the methods allow detection or quantification of 10² to 10⁷ CFU/mL bacterial and/or archaeal cells in various samples. In some disclosures, methods of this disclosure may be used to distinguish samples bases on the quantity of viable bacterial and/or archaeal cells contained therein. For instance, the methods may be used to distinguish among samples that contain 10², 10³, 10⁴, 10⁵, 10⁶, or 10⁷ CFU/mL of bacterial and/or archaeal cells.

This disclosure provides a method of assessing or monitoring the need to treat a subject suffering from or at risk of overgrowth of bacterial and/or archaeal cells in the GI tract, including: (a) providing an ingestible device as described herein; (b) transferring a fluid sample from the GI tract of a subject into a sampling chamber of the ingestible device *in vivo;* and (c) quantifying viable bacterial and/or archaeal cells present in the fluid sample (*e.g., in vivo*), wherein the number of the viable bacterial and/or archaeal cells determined in step (c) greater than about 10⁵ CFU/mL indicates a need for treatment, *e.g*., with an antibiotic agent as described herein.

In some disclosures, the method of assessing or monitoring the need to treat a subject suffering from or at risk of overgrowth of bacterial and/or archaeal cells in the GI tract includes: (a) providing an ingestible device as described herein; (b) transferring a fluid sample from the GI tract of a subject into a sampling chamber of the device *in vivo,* wherein the sampling chamber of the device as described herein is configured to hold an absorptive sponge as described herein, or an absorptive sponge prepared according to the method for preparing an absorptive sponge as described herein; (c) fully or partially saturating (*e.g.,* at 50% or half saturation) the absorptive sponge in the sampling chamber with the fluid sample; (d) measuring total fluorescence of the fully or partially saturated sponge *(e.g.,* at 50% or half saturation) prepared in step (c); and (e) correlating the total fluorescence measured in step (d) to the number of viable bacterial and/or archaeal cells in the sample, wherein the number of the viable bacterial and/or archaeal cells determined in step (e) greater than about 10⁵ CFU/mL indicates a need for treatment, *e.g*., with an antibiotic agent as described herein.

In some disclosures, the method of assessing or monitoring the need to treat a subject suffering from or at risk of overgrowth of bacterial and/or archaeal cells in the GI tract includes: (a) providing an ingestible device as described herein; (b) transferring a fluid sample from the GI tract of a subject into a sampling chamber of the device *in vivo,* wherein the sampling chamber of the device as described herein is configured to hold an absorptive sponge as described herein, or an absorptive sponge prepared according to the method for preparing an absorptive sponge as described herein; (c) fully or partially saturating (*e.g.,* at 50% or half saturation) the absorptive sponge in the sampling chamber with the fluid sample; (d) measuring rate of change of fluorescence as a function of time of the fully or partially saturated sponge *(e.g.,* at 50% or half saturation) prepared in step (c); and (e) correlating the rate of change of fluorescence as a function of time measured in step (d) to the number of viable bacterial and/or archaeal cells in the sample, wherein the number of the viable bacterial and/or archaeal cells determined in step (e) greater than about 10⁵ CFU/mL indicates a need for treatment, *e.g*., with an antibiotic agent as described herein.

In some disclosures, the total fluorescence or the rate of change of fluorescence as a function of time of the sponge is measured over multiple time points for an extended period of time in step (d). For instance, in some disclosures, the total fluorescence rate of change of fluorescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total fluorescence or the rate of change of fluorescence as a function of time of the sample is measured continuously for a period of 0-330 minutes. In some disclosures, the method does not require *ex vivo* plating or culturing. In some disclosures, the method does not require aspiration. In some disclosures, the method is performed *in vivo (e.g.,* in an ingestible device *in vivo).* In some disclosures, the method includes communicating the results of the onboard assay(s) to an *ex vivo* receiver. In some disclosures, the ingestible device and the method may be further used to monitor the subject after the treatment (*e.g.,* with an antibiotic). In some disclosures, the ingestible device and the method may be used to assess the efficacy of the treatment. For example, efficacious treatment may be indicated by the decrease of the number of viable bacterial and/or archaeal cells in a sample from the GI tract of the subject post-treatment. Efficacy of the treatment may be evaluated by the rate of decrease of the number of viable bacterial and/or archaeal cells in a sample from the GI tract of the subject post-treatment. In some disclosures, the ingestible device and the method may be used to detect infection with antibiotic-resistant strains of bacteria and/or archaea in a subject. For instance, such infection may be indicated where the number of viable bacterial and/or archaeal cells in a sample from the GI tract of the subject does not substantially decrease after antibiotic treatment.

In some disclosures, the compositions, methods and devices described herein may use a combination of (e.g., two or more) analyte-binding agents to detect, characterize and/or quantitate the type of analyte (e.g., a microorganism, a protein, or a metabolite) present in a sample. For example, in some disclosures, the compositions, methods and devices described herein may be used to determine the types of microorganisms (e.g., bacteria, archaea, protozoans, or viruses) present in a sample. In some disclosures, a first analyte-binding agent that binds to an analyte and comprises a first fluorescent dye, may be used in combination with a second analyte-binding agent, wherein the second analyte-binding agent comprises a second fluorescent dye that exhibits increased fluorescence when spatially proximal to the first fluorescent dye. In some disclosures, spatial proximity between the first fluorescent dye and the second fluorescent dye results in energy transfer from the first fluorescent dye to the second fluorescent dye. The detection of the fluorescence emitted by the second fluorescent dye can be used, for example, to determine whether both analyte-binding agents are located in close proximity to each other in the sample. Alternatively, a first analyte-binding agent that binds to an analyte and comprises a first fluorogenic dye may be used in combination with a second analyte-binding agent, wherein the second analyte-binding agent comprises a second fluorescent dye that exhibits increased fluorescence when spatially proximal to the first fluorogenic dye. In some disclosures, the first fluorogenic dye exhibits no fluorescence or reduce fluorescence when the first-analyte binding agent is not bound to the analyte. In some disclosures, the first fluorogenic dye exhibits increased fluorescence upon binding of the first analyte-binding agent to the analyte. In some disclosures, spatial proximity between the first fluorogenic dye and the second fluorescent dye results in energy transfer from the first fluorogenic dye to the second fluorescent dye. The detection of the fluorescence emitted by the second fluorescent dye can be used, for example, to determine whether both analyte-binding agents are located in close proximity to each other in the sample. In some disclosures, the first and the second analyte-binding agents bind to the same region (e.g., epitope) of the analyte (e.g., a protein). For instance, in some disclosures, the first and the second analyte-binding agents comprise the same type of analyte-binding moiety or reagent (e.g., the same antibody). In some disclosures, the first and the second analyte-binding agents bind to separate regions (e.g., epitopes) of the analyte (e.g., a protein). In some disclosures, the first and the second analyte-binding agents bind to the separate regions of the analyte (e.g., a protein) that do not spatially overlap. In some disclosures, the first analyte-binding agent and the second analyte-binding agent are configured such that when both analyte-binding agents are bound to the analyte, their respective dyes are in close proximity (e.g., allowing for energy transfer to occur). In some disclosures, the first and/or second analyte binding agent(s) is an antigen-binding agent (e.g., an antibody). In some disclosures, the first and/or second analyte binding agent(s) is an affimer. In some disclosures, the first and/or second analyte binding agent(s) is an antigen-binding agent is an aptamer.

In some disclosures, the compositions, methods and devices described herein make use of fluorescent oxygen channeling immunoassay (FOCI) compositions and methods. FOCI is generally described in U.S. Patent Nos. 5,807,675; 5,616,719; and 7,635,571. In some disclosures, a first analyte-binding agent that is capable of binding to an analyte and comprises a photosensitizer is used in combination with a second analyte-binding agent comprising a fluorogenic dye. In some disclosures, the photosensitizer of the first analyte-binding agent generates singlet oxygen in an excited state thereby causing the fluorogenic dye of the second analyte-binding agent to emit fluorescence upon reacting with the singlet oxygen. In some disclosures, the emitted fluorescence can be detected to, e.g., determine the presence and/or absence of the analyte and/or to quantitate and/or analyze the analyte in a sample. In some disclosures, the first and the second analyte-binding agents bind to the same region (e.g., epitope) of the analyte (e.g., a protein). For example, in some disclosures, the first and the second analyte-binding agents comprise the same type of analyte-binding moiety or reagent (e.g., the same antibody). In some disclosures, the first and the second analyte-binding agents bind to separate regions (e.g., epitopes) of the analyte (e.g., a protein). In some disclosures, the first and the second analyte-binding agents bind to the separate regions of the analyte (e.g., a protein) that do not spatially overlap. In some disclosures, the first analyte-binding agent and the second analyte-binding agent are configured such that when both analyte-binding agents are bound to the analyte, the singlet oxygen generated by photosensitizer of the first analyte-binding agent is in close proximity to the fluorogenic dye of the second analyte-binding agent. In some disclosures, the first and/or second analyte binding agent(s) is an antigen-binding agent (e.g., an antibody). In some disclosures, the first and/or second analyte binding agent(s) is an affimer. In some disclosures, the first and/or second analyte binding agent(s) is an antigen-binding agent is an aptamer.

In some disclosures, the use of a combination of analyte-binding agents allows for the detection, analysis and/or quantitation of a multitude of analytes. Multiple combinations of analyte-binding agents may be used for the detection, analysis and/or quantitation of a complex mixture of analytes. For example, multiple analyte-binding agents having different dyes, and/or analyte specificities may be used to analyze a sample. For instance, in order to detect different species of bacteria and/or archaea ( or e.g., LTA vs. LPS) present in a sample, one can couple a live cell dye (F1) as described herein to an antibody or to an antibiotic that is microorganism-specific (e.g., bacteria specific or bacterial species-specific). Antibodies that specifically bind to a biomolecule (e.g., a surface antigen) present in a microorganism (e.g., a bacteria or an archaea) of a genus, species or strain of interest and do not cross-react with other microorganism biomolecules and/or eukaryotic biomolecules may also be used, including the exemplary antibodies described herein. A second antibody or antibiotic having a fluorescent dye (F2) that binds to the same microorganism and that gets excited (via an energy transfer from F1 to F2) when in close proximity to F1 may be employed to detect, analyze and/or quantitate the microorganism to which the antibodies and/or antibiotics bind.

### Analyte Diluting and Culturing

The present disclosure provides methods of obtaining, culturing, and/or detecting cells and/or analytes *in vivo* within the gastrointestinal (GI) tract o of a subject. Associated devices are also disclosed. The methods and devices described provide a number of advantages for obtaining and/or analyzing fluid samples from a subject. In some disclosures, diluting the fluid sample increases the dynamic range of analyte detection and/or reduces background signals or interference within the sample. For example, interference may be caused by the presence of non-target analytes or non-specific binding of a dye or label within the sample. In some disclosures, culturing the sample increases the concentration of cells (e.g., a specific type of cells) and/or analytes (e.g., a specific analyte of interest) produced by the cells thereby facilitating their detection and/or characterization. Above, various types of analytes are disclosed which may be detected and/or characterized as described herein.

In certain disclosures, the methods and devices a described herein may be used to obtain information regarding bacterial and/or archaeal populations in the GI tract of a subject. This has a number of advantages and is less invasive than surgical procedures such as intubation or endoscopy to obtain fluid samples from the GI tract. The use of an ingestible device as described herein also allows for fluid samples to be obtained and data to be generated on bacterial and/or archaeal populations from specific regions of the GI tract.

In some disclosures, the methods and devices described herein may be used to generate data such as by analyzing the fluid sample, dilutions thereof or cultured samples for one or more cells and/or analytes. The data may include, but is not limited to, the types of bacteria and/or archaea present in the fluid sample or the concentration of bacteria and/or archaea in specific regions of the GI tract. Such data may be used to determine whether a subject has an infection, such as Small Intestinal Bacterial Overgrowth (SIBO), or to characterize bacterial and/or archaeal populations within the GI tract for diagnostic or other purposes.

For example, in one disclosure, the data may include, but is not limited to, the concentration of bacteria and/or archaea in a specific region of the GI tract that is one or more of the duodenum, jejunum, ileum, ascending colon, transverse colon or descending colon. In one disclosure, the specific region of the GI tract is the duodenum. In one disclosure, the specific region of the GI tract is the jejunum. In one aspect, the specific region of the GI tract is the ileum. In one disclosure, the specific region of the GI tract is the ascending colon. In one disclosure, the specific region of the GI tract is the transverse colon. In one disclosure, the specific region of the GI tract is the descending colon. In a related embodiment, the data may be generated every one or more days to monitor disease flare-ups, or response to the therapeutic agents disclosed herein.

Data may be generated after the device has exited the subject, or the data may be generated *in vivo* and stored on the device and recovered *ex vivo.* Alternatively, the data can be transmitted wirelessly from the device while the device is passing through the GI tract of the subject.

In some disclosures, a method comprises: providing a device comprising one or more dilution chambers and dilution fluid; transferring all or part of a fluid sample obtained from the GI tract of the subject into the one or more dilution chambers *in vivo;* and combining the fluid sample and the dilution fluid to produce one or more diluted samples in the one or more dilution chambers.

In certain disclosures, a method comprises: providing an ingestible device comprising one or more dilution chambers; transferring all or part of a fluid sample obtained from the GI tract into the one or more dilution chambers comprising sterile media; culturing the sample *in vivo* within the one or more dilution chambers to produce one or more cultured samples; and detecting bacteria and/or archaea in the one or more cultured samples.

In some disclosures, a method comprises: providing a device comprising one or more dilution chambers; transferring all or part of a fluid sample obtained from the GI tract into the one or more dilution chambers; combining all or part of the fluid sample with a dilution fluid in the one or more dilution chambers; and detecting the analyte in the one or more diluted samples.

In certain disclosures, a device comprises: one or more dilution chambers for diluting a fluid sample obtained from the GI tract; and dilution fluid for diluting the sample within the one or more dilution chambers.

In some disclosures, the device comprises: one or more dilution chambers for culturing a fluid sample obtained from the GI tract; sterile media for culturing the sample within the one or more dilution chambers; and a detection system for detecting bacteria and/or archaea.

In certain disclosures, a device comprises: one or more dilution chambers for culturing a fluid sample obtained from the GI tract; sterile media for culturing the sample within the one or more dilution chambers; and a detection system for detecting bacteria and/or archaea.

Also provided is the use of a device as described herein for diluting one or more samples obtained from the GI tract of a subject. In one embodiment, there is provided the use of an ingestible device as described herein for detecting cells and/or analytes *in vivo* within the gastrointestinal (GI) tract of a subject.

Further provided is a system comprising a device as described herein and a base station. In one embodiment, the device transmits data to the base station, such as data indicative of the concentration and/or types of bacteria and/or archaea in the GI tract of the subject. In one embodiment, the device receives operating parameters from the base station. Some disclosures described herein provide an ingestible device for obtaining one or more samples from the GI tract of a subject and diluting and/or culturing all or part of the one or more samples. The ingestible device includes a cylindrical rotatable element having a port on the wall of the cylindrical rotatable element. The ingestible device further includes a shell element wrapping around the cylindrical rotatable element to form a first dilution chamber between the cylindrical rotatable element and the shell element. The shell element has an aperture that exposes a portion of the wall of the cylindrical rotatable element to an exterior of the ingestible device.

In some disclosures, the ingestible device includes one or more dilution chambers for receiving a fluid sample from the GI tract of a subject or a dilution thereof. In some disclosures, one or more dilutions of the fluid sample are cultured in one or more dilution chambers. In some disclosures, the dilution chambers each define a known volume, optionally the same volume or different volumes. In some disclosures, the dilution chambers define a fluid volume ranging from about 10 µL to about 1 mL. The dilution chambers may define a fluid volume less than or equal to about 500 µL, less than or equal to about 250 µL, less than or equal to about 100 µL, or less than or equal to about 50 µL. In some disclosures, the dilution chambers define a fluid volume of greater than or equal to about 10 µL, greater than or equal to about 20 µL, greater than or equal to about 30 µL, or greater than or equal to about 50 µL. In some disclosures, the dilution chambers define a fluid volume between about 10 µL and 500 µL, between about 20 µL and 250 µL, between about 30 µL and 100 µL or about 50 µL.

In some disclosures, dilution fluid in the device is combined with all or part of the fluid sample, or dilution thereof, to produce one or more dilutions. In some disclosures, the dilution fluid is sterile media suitable for culturing one or more cells within the dilution chambers.

In some disclosures, the one or more dilution chambers may be filled with the dilution fluid prior to a patient ingesting the ingestible device. Alternatively, in another embodiment, the dilution fluid may be added into the one or more dilution chambers *in vivo* from a reservoir of the ingestible device. Sampling and dilution of the GI fluid sample may take place *in vivo.* For example, an actuator of the ingestible device may pump the dilution fluid from the reservoir into a dilution chamber when it is determined that the ingestible device is located at a predetermined location within the GI tract. In some disclosures, the dilution chambers each contain a volume of sterile media suitable for culturing a fluid sample from the GI tract. In some disclosures, the dilution chambers are at least 95%, at least 97%, at least 98%, or at least 99% full of sterile media. In some disclosures, the dilution chambers each contain oxygen to facilitate aerobic bacteria growth. In another embodiment a non-dilution chamber includes oxygen and is added to one or more of the dilution chambers to facilitate aerobic bacteria growth.

In some disclosures, the culturing may take place *in vivo* immediately after the GI fluid sample has been diluted. Or alternatively, the culturing may take place *ex vivo,* e.g., when the ingestible device has been evacuated and recovered such that the dilution chamber containing the diluted GI fluid sample may be extracted and the culturing may be performed in a laboratory. The recovery of the ingestible device may be performed in a similar manner as disclosures described in International Application No. PCT/US17/61024, filed on November 10, 2017.

In some disclosures, the dilution fluid includes one or more agents for inhibiting the growth of fungus. In some disclosures, the anti-fungal agent is amphotericin B. In some disclosures, the dilution fluid contains about 2.5 mg/L of Amphotericin B.

In some disclosures, the media includes one or more antimicrobial agents in order to determine antibiotic sensitivity/resistance of bacteria and/or archaea within the fluid sample. For example, if bacteria and/or archaea grow in a dilution chamber containing media without antibiotics but do not grow in a separate dilution chamber containing media including antimicrobial agents, the sample may be identified as containing bacteria and/or archaea sensitive to that antibiotic. Alternatively, if bacteria and/or archaea grow in both dilution chambers (with and without antibiotics), the sample can be identified as containing bacteria and/or archaea resistant to that antibiotic. In some disclosures, the bacteria and/or archaea remaining in the dilution chamber(s) are quantified using, e.g., any detection and/or quantification method described herein. In some disclosures, the presence and/or absence of a particular type of bacteria and/or archaea in the dilution chamber (e.g., a bacteria and/or archaea of a particular genus, species and/or strain) is detected and/or quantified using a method described herein.

In another embodiment, the dilution fluid includes a substrate or reagent for measuring bacterial and/or archaeal activity or response. For example, in some disclosures, the dilution fluid includes one or more conjugated bile acids and deconjugated bile acids or a reduction in conjugated bile acids are detected in the diluted samples and/or cultured samples as a sign of bile salt hydrolase activity. In another embodiment, the substrate may be an enzyme, for example, glutamate dehydrogenase (GDH). In the case of GDH, it may be used to detect an antigen that is produced in high amounts by *C. difficile,* both toxin and non-toxin producing strains. This test indicates if *C*. *difficile* is present but not necessarily if the bacteria are producing toxins.

In another embodiment, products of the bacteria and/or archaea are detected or measured while the bacteria and/or archaea are being cultured in the media. For example, *Clostridium difficile* toxin A can be measured to detect if bacteria and/or archaea are producing toxins.

In some disclosures, the methods and devices described herein may be used to obtain, dilute, culture and/or detect eukaryotic cells from the subject. For example, epithelial cells or PBMC's from the GI tract can be diluted or cultured in the ingestible device. Optionally, the eukaryotic cells may be analyzed within the device and/or collected once the device has exited. In some disclosures, the dilution fluid further includes a substrate or reagent for measuring eukaryotic activity or response *in vivo.* For example, in some disclosures, a biomarker in the sample may be detected, analyzed and/or quantitated. The detection of the biomarker in the sample may be used to diagnose or monitor a disease or disorder or the treatment thereof.

In some disclosures, a fluid sample is transferred from the GI tract into one or more dilution chambers while the ingestible device is passing through the GI tract of a subject *in vivo.* By controlling when the fluid sample is transferred into the one or more dilution chambers, it is possible to obtain a fluid sample from a particular region of the GI tract. In operation, the exterior of the ingestible device will be in contact with biological fluids in the GI tract. As the ingestible device travels along the GI tract, it will typically be surrounded by fluid that is characteristic of that section of the GI tract. For example, when the ingestible device is in the stomach, the device will be in contact with stomach fluid which may include gastric acid, digestive enzymes, and partially digested food. When the ingestible device is in the jejunum, the device will be in contact with jejunal fluid.

In some disclosures, the device has one or more ports, valves and/or pumps that are used, either alone or in combination, for controlling the transfer of fluid from the GI tract into the one or more dilution chambers. The device may also contain one or more ports, valves and/or pumps for controlling the transfer of fluid between dilution chambers within the device, optionally to produce a serial dilution of the original fluid sample from the GI tract. Compositions and methods for sampling the GI tract are discussed in greater detail in International Application No. PCT/US2017/047476, filed August 18, 2017.

In some disclosures, the device includes a microcontroller for controlling the one or more ports, valves and/or pumps. In some disclosures, the microcontroller is programmed to control the ports, valves and/or pumps in response to data from the one or more environmental sensors. Alternatively or in addition, the microcontroller is programmed to control the ports, valves and/or pumps in response to wireless a signal from a base station or in response to a signal generated by the microcontroller, such as a timer.

In some disclosures the device includes a pump having an input conduit on the exterior of the device and an output conduit into a first dilution chamber. In some disclosures, the pump is operational to transfer fluid from the GI tract into the first dilution chamber. Preferably, the pump is controllable to transfer a predetermined volume of liquid from the GI tract into a dilution chamber. In some disclosures, the device includes a pump controllable to transfer a predetermined volume of liquid between dilution chambers, optionally the same pump or a different pump as used to transfer the fluid sample into the first dilution chamber. In some disclosures, the pump is a solenoid pump. In some disclosures, the pump is controllable to transfer a fluid volume from about 1 µL to about 50 µL, about 2 µL to about 20 µL, about 3 µL to about 15 µL, or about 5 µL.

In another embodiment, the device includes a port for receiving a fluid sample from the GI tract. In some disclosures, the port is exposed to the exterior of the device. Optionally, the device includes a cover movable to expose the port to the exterior of the device. In operation, when the port is exposed fluid from the GI tract enters into the port through surface tension, movement of the subject and/or peristaltic effects. Optionally, the port is coated with a hydrophilic coating to encourage fluid to flow into the port.

In some disclosures, the port is movable from an open position such that the port is exposed to the exterior of the device, to a first position such that the port is in fluid communication with a first dilution chamber. In some disclosures, moving the port from the open position to the first position transfers a predetermined volume of a fluid sample from the GI tract into the first dilution chamber. For example, in some disclosures the port defines a fluid volume of about 1 µL to about 50 µL, about 2 µL to about 20 µL, about 3 µL to about 15 µL, or about 5 µL.

A skilled person will appreciate that when the port is in fluid communication with the first dilution chamber, the port and the first dilution chamber will define a combined volume such that any fluid in the port and dilution chamber will mix to form a dilution. In some disclosures, the mixing of fluid will be enhanced through the peristaltic action of the GI tract as well as movement of the subject. In some disclosures, the first incubation chamber contains dilution fluid such as sterile media and moving the port to the first position produces a first dilution including a predetermined volume of fluid sample from the GI tract and a predetermined volume of dilution fluid. For example, in some disclosures the port has a fluid volume of 5µl and the first dilution chamber has 45 µL of dilution fluid, such that the first dilution is a 10 fold dilution of the fluid sample.

In some disclosures, the first dilution is cultured to produce a single cultured sample and cells and/or analytes are detected within the cultured sample. Alternatively, in some disclosures a portion of the first dilution is transferred to one or more additional dilution chambers to produce a serial dilution of the fluid sample from the GI tract. In some embodiments, the serial dilution is produced by controlling the transfer of fluid between the dilution chambers.

For example, in some disclosures the ingestible device includes a port movable to sequentially align with one or more additional dilution chambers, thereby transferring a portion of first dilution of the fluid sample to each additional dilution chamber. Alternatively or in addition, one or more pumps and/or valves may be used to sequentially transfer a portion of the fluid sample or dilution thereof to each dilution chamber.

In some disclosures, the device includes a port movable from a first position in fluid communication with a first dilution chamber to a second position such that the port is in fluid communication with a second dilution chamber. In some disclosures, the port is movable from the second position to a third position such that the port is in fluid communication with a third dilution chamber. In some disclosures, the port is movable from the third position to a fourth position such that the port is in fluid communication with a fourth dilution chamber. In some disclosures, the port is movable from the fourth position to a fifth position such that the port is in fluid communication with a fifth dilution chamber. Optionally, the device may contain more than five dilution chambers for producing more than five dilutions of the original fluid sample. In some disclosures, the dilution chambers are suitable for culturing the dilutions *in vivo* within the device.

In some disclosures, the port is a depression on the surface of a movable element. In some disclosures, the movable element is coupled to an actuator for moving the port relative to the positions of the one of more dilution chambers. Optionally, the actuator is an electric motor.

In some disclosures the port is a depression on the surface of a rotatable element. In some disclosures, the device includes an actuator coupled to the rotatable element for rotating the port to align with one or more dilution chambers. In some disclosures, the dilution chambers are positioned circumferentially around the axis of rotation of the rotatable element. In some disclosures, rotating the rotatable element sequentially moves the port from the open position to the first position and optionally one or more of the second position, third position and fourth position.

FIG. 27 shows one embodiment of a portion of an ingestible device 4000 with a port 4154b in an open position to the exterior of the ingestible device 400. The ingestible device 400 may include a cylinder-shaped rotatable element 4150 that includes sampling ports 4154a-b on the wall of the rotatable element 4150. The sampling chamber 4150 is wrapped by a shell element 4140 with dividers to form a series of dilution chambers 4151a-n between the shell element 4140 and the rotatable element 4150. In operation, when the ingestible device 4000 determines the device itself arrives at a target location within the GI tract, the rotatable element 4150 may be rotated into an open position such that an aperture of the shell element 4140 is aligned with the port 4154b on the wall of the rotatable element 4150 and the port 4154b is exposed to the exterior of the ingestible device 4000 through the aperture. In this way, fluid from the GI tract can enter the port 4154b and occupy the volume defined by the port 4154b. In the embodiment shown in FIG. 27, the port 4154b may be a depression on the surface of a rotatable element 4150 and a number of dilution chambers 4151a-n are positioned circumferentially around the axis of rotation of the rotatable element 4150. As previously discussed, each of the dilution chambers 4151a-n may store a dilution fluid. In some disclosures, the depression is a cylindrical depression. Optionally, the depression may be a rectangular depression, or any concave depression forming a regular or irregular shape. In another embodiment, the port 4154b may be connected to a chamber (not shown) within the rotatable element 4150 to create an enlarged space to store the GI fluid sample from the external environment of the ingestible device.

In some disclosures, the ingestible device 4000 may further include a controller and an actuator. The controller may determine that the ingestible device 4000 is located at a target location of the GI tract, and then the actuator may trigger the rotation of the rotatable element 4150 to align the port 4154b at the open position to initiate the sampling. For example, the housing of ingestible device 4000 may have a pH-sensitive enteric coating to detect or otherwise be sensitive to a pH level of the environment external to the ingestible device 4000, based on which the controller may determine whether the ingestible device has arrived at a target location. For another example, the ingestible device 4000 may include an optical sensing unit that transmits an illumination to the environment and collects a reflectance, based on which, the regio-specific location of the ingestible device 4000 may be identified based on optical characteristics of the reflectance. Further disclosures of localization of the ingestible device 4000 may be found in PCT International Application No. PCT/US2015/052500, filed on September 25, 2015.

FIG. 28 shows one embodiment of a portion of an ingestible device with a port 4154b at a first position aligned with a first dilution chamber 4151a. In operation, the rotatable element 4150 may be rotated to align the sampling port 4154b and the first dilution chamber 4151a such that the fluid sample from the GI tract stored within the volume of the sampling port 4154b can be combined with dilution fluid in the first dilution chamber to form a first dilution. The first dilution may then occupy the combined volume of the port 4154b and first dilution chamber 4151a. Optionally, the rotatable element 4150 may be subsequently rotated to a second position such that the port 4154b containing a portion of the first dilution is then moved to be aligned and in fluid communication with another dilution chamber, e.g., a second dilution chamber that is next to the first dilution chamber along the rotational direction. In this way, the first dilution stored within the port 154b may then again be diluted with the dilution fluid stored within the second dilution chamber. Similarly, if the rotatable element 4150 keeps rotating and allows the port 4154b to be serially aligned with each dilution chamber, then the original GI fluid sample may be diluted serially and each dilution chambers 4151a-n may be left with a diluted GI fluid sample at a different dilution ratio.

FIG. 29 shows one embodiment of an element 4140 forming part of a set of 5 dilution chambers (e.g., including 4151a-b) for surrounding a rotatable element (e.g., 4150 in FIGs. 28 and 29) in an ingestible device as described herein. In some disclosures, the device may contain a single dilution chamber. Alternatively, the device may contain 2, 3, 4, 5, 6, 7, 8 or greater than 8 dilution chambers.

In some disclosures, each dilution chamber 4151a-n may be filled with a dilution fluid prior to the ingestible device 4000 being administered. In another embodiment, the dilution fluid may be stored in a separate reservoir (not shown) within the ingestible device 4000. At the time when the ingestible device 4000 is determined to be at a target location within the GI tract, a pumping mechanism may pump the dilution fluid into one or more dilution chambers 4151a-b via one or more outlet (not shown) of the reservoir. The pumping mechanism and the reservoir that stores the dilution fluid, may take a form similar to the electromechanical delivery mechanism of an ingestible device as described in International Application No. PCT/US2017/050642, filed on September 8, 2017.

In some disclosures, the shell element 4140 may have valves or pumps (not shown) between the dilution chambers 4151a-n. For example, the diluted fluid from a first dilution chamber may be pumped into a second dilution chamber via a valve between the two chambers. The pump and valve mechanism may take a form similar to the electromechanical delivery mechanism of an ingestible device as described in International Application No. PCT/US2017/050642, filed on September 8, 2017.

In some disclosures, the method and devices described herein involve combining a fluid sample, or dilution thereof, with dilution fluid to produce one or more dilutions of the fluid sample. For example, in some disclosures the fluid sample is combined with dilution fluid in a first dilution chamber to produce a first dilution, a portion of the first dilution is combined with dilution fluid in a second dilution chamber to produce a second dilution, a portion of the second dilution is combined with dilution fluid in a third dilution chamber to produce a third dilution, and optionally a portion of the third dilution is combined with dilution fluid in a fourth dilution chamber to produce a fourth dilution, and optionally a portion of the fourth dilution is combined with dilution fluid in a fifth dilution chamber to produce a fifth dilution.

The relative dilution of the fluid sample will depend on the relative amount of fluid sample, or dilution thereof, and dilution fluid that is combined in each dilution chamber. In some disclosures, the fluid sample, or dilution thereof, is combined with dilution fluid at a ratio between about 1:1 and about 1: 1000, between about 1:1 and 1:100, between about 1:1 and about 1:20, or between about 1:1 and about 1: 10. Optionally, the relative amounts of fluid sample, or dilution thereof, and dilution fluid that are combined in each dilution chamber are varied such that different dilution chambers contain different dilutions. In some disclosures, the method and devices described herein produce a series of 10-fold dilutions of the fluid sample. In some disclosures, the methods and devices described herein produce a series of dilutions of a fluid sample such that for a given bacterial and/or archaeal concentration in the fluid sample, some of the dilutions will not be expected to contain any bacteria and/or archaea, and some of the dilutions will be expected to contain bacteria and/or archaea and therefore exhibit bacterial and/or archaeal growth when cultured.

As set out in examples below, determining the presence or absence of bacterial and/or archaeal growth in one or more dilutions can be used to estimate the concentration of bacteria and/or archaea within the original fluid sample from the GI tract. The use of a dilution series and a binary detection system that detects the presence or absence of bacterial and/or archaeal growth presents a number of advantages over more complicated detection systems that seek to directly quantify the concentration of bacteria and/or archaea within a sample. For example, binary detection systems are robust and amenable to miniaturization and therefore suitable for use in an ingestible device as described herein. Also, diluting the fluid sample increases the dynamic range while reducing interference. Accordingly, in some disclosures the methods and devices described herein include detecting the presence or absence of the growth of a cell, optionally bacterial and/or archaeal growth. In some disclosures, the methods and devices described herein include detecting the presence or absence of bacterial growth and/or archaeal growth in one or more dilutions of the fluid sample from the GI tract.

In some disclosures, the presence or absence of bacterial and/or archaeal growth in one or more dilutions is used to estimate the concentration of bacteria and/or archaea in the fluid sample. For example, in some disclosures a fluid sample of about 5 µL is diluted about 10000 times in one of the dilution chambers and detecting the presence of bacterial and/or archaeal growth in the dilution chamber is indicative of a bacterial and/or archaeal concentration of 10⁵ or greater colony forming units/mL (CFU/mL) in the fluid sample. 10 µL of a fluid sample with a bacterial and/or archaeal concentration of 10⁴ CFU/mL would contain about 100 CFU. A 10000-fold dilution of such a 10 µL fluid sample would be unlikely to contain any CFUs or bacteria and/or archaea (theoretically 0.01 bacteria and/or archaea) and therefore would not be expected to exhibit bacterial and/or archaeal growth when cultured.

Alternatively, in some disclosures the methods and devices described herein include detecting a level of bacterial and/or archaeal concentration within one or more cultured samples. For example, in some disclosures a quantifiable property of a cultured sample is measured in order to provide an estimate of the level of bacteria and/or archaea within the cultured samples in order to estimate the concentration of bacteria and/or archaea within the original fluid sample.

In some disclosures, the devices described herein include a detection system for detecting one or more cells and/or analytes. In some disclosures, the cells are bacteria (e.g., bacteria of a particular genus, species and/or strain). In some disclosures, the cells are archaea (e.g., archaea of a particular genus, species and/or strain). Different detection systems known in the art for detecting bacteria and/or archaea may be used with the device as described herein. In some disclosures, the detection system detects the presence or absence of bacterial and/or archaeal growth within a diluted sample. In some disclosures, the detection system detects the presence or absence of bacterial and/or archaeal growth within a cultured sample. Alternatively or in addition, the detection system detects a level of bacteria and/or archaea within one or more cultured samples. For example, in some disclosures a Coulter counter is used to detect and/or quantify bacteria and/or archaea in the fluid samples, dilutions thereof or cultured samples. In another embodiment, an optical detection system is used to detect and/or quantify bacteria and/or archaea within the fluid samples, dilutions thereof or cultured samples.

In some disclosures, the detection system detects cells and/or analytes in a dilution or cultured sample within the one or more dilution chambers. Alternatively, the device includes one or more separate detection chambers and the detection system detects cells and/or analytes in the fluid sample or dilutions therefor within the detection chamber. In some disclosures, fluid communication between one or more dilution chambers and one or more detection chambers is controlled by one or more ports, valves and/or pumps.

In some disclosures, the detection system detects cells and/or analytes at a plurality of time points. For example, in some disclosures the detection system detects bacteria and/or archaea within the sterile media prior to combining the fluid sample from the GI tract and the sterile media in order to ensure that any bacterial and/or archaeal growth is due to the bacteria and/or archaea introduced into the dilution chambers from the GI tract. In some disclosures, the detection system detects bacteria and/or archaea at a first time point and at a second time point. In some disclosures, the second time point is selected to allow for the growth of bacteria and/or archaea within the cultured fluid sample relative to the first time point. For example, in some disclosures the second time point is between about 1 hour and 6 hours, between about 1 hour and 4 hours, or between about 2 hours and 4 hours after the first time point. In some disclosures, detecting bacteria and/or archaea at the first time point serves as a control.

In some disclosures, the detection system detects the level of bacteria and/or archaea at three or more time points to determine a growth curve for bacteria and/or archaea in the one or more cultured samples. For example, the level of bacteria and/or archaea may be detected within one or more culture samples every 30 or 60 minutes after a sample is collected for a total of 2-12 hours; thereby producing a growth curve. The growth curve may then be compared to one or more standard growth curves. In some disclosures, the standard growth curves are representative of the growth of samples with a known concentration of bacteria and/or archaea. In some disclosures, the standard growth curves are representative of growth curves from subjects with Small Intestinal Bacterial Overgrowth (SIBO).

In some disclosures, the disclosures described herein use an optical detection system for detecting one or more cells and/or analytes. In some disclosures, the optical detection system includes a light source and a photodetector. In some disclosures, the light source and photodetector are operable to define a light path through a dilution chamber or detection chamber. In some disclosures, the optical detection system measures the absorbance of light or optical density along the light path at one or more wavelengths.

In some disclosures, the optical detection system measures the absorbance of light at one or more wavelengths between 400 nm and 1000 nm. In some disclosures, the optical detection system measures the absorbance of light at one or more wavelengths between about 500 and 700 nm. In some disclosures, the optical detection system measures the absorbance of light at about 600 nm.

In some disclosures, the device described herein includes one or more environmental sensors for measuring environmental data of the GI tract external to the device in the subject. In some disclosures, the environmental data is used to help determine one or more characteristics of the GI tract of the subject such as for the diagnosis of a medical condition. Alternatively or in addition, the environmental data is used to determine the location of the device within the GI tract of the subject. In some disclosures, the one or more environmental sensors include a capacitance sensor, a temperature sensor, an impedance sensor, a pH level sensor and/or a light sensor. In some disclosures, the one or more environmental sensors measure pH, temperature, transit times, or combinations thereof. Examples of devices that detect pH changes include Medimetrics' IntelliCap^{®} technology (see Becker, Dieter, et al. "Novel orally swallowable IntelliCap® device to quantify regional drug absorption in human GI tract using diltiazem as model drug." AAPS PharmSciTech 15.6 (2014):1490-1497) and Rani Therapeutics' Auto-Pill^{™} technology (see U.S. Patent 9,149,617).

In some disclosures, data regarding the location of the device within the GI tract of the subject is used to determine when to obtain a fluid sample from the GI tract and transfer the fluid sample into the one or more dilution chambers. Accordingly, in some disclosures the device includes a microcontroller configured to transfer the fluid sample from the GI tract of the subject to the one or more dilution chambers based on the location of the device within the GI tract.

In some disclosures, the device includes a communication sub-unit that is configured to receive operating parameters from an external base station and/or transmit data to an external base station. Also provided is a system including a device as described herein and an external base station. In some disclosures, the operating parameters include timing instructions for obtaining a fluid sample from the GI tract and transferring the sample into one or more dilution chambers. In some disclosures, the data transmitted to the external base station includes data indicative of the presence of absence of bacterial and/or archaeal growth in the cultured samples.

In general, it is possible for the ingestible device to obtain different samples from different predetermined regions of the GI tract, or for certain actions within the ingestible device to be triggered based on its location in the GI tract. For example, it may be possible for the ingestible device to use various combinations of light emitting diodes and sensors to determine whether the device is in the stomach, small intestine, or large intestine. This may be done by emitting light at different wavelengths, measuring the level of light reflected at each wavelength by the environment surrounding the ingestible device, and using this information to determine an approximate location of the ingestible device based on the different reflectance properties of the various different portions of the GI tract. Once the ingestible device determines that it is in a particular predetermined portion of the GI tract (e.g., the small intestine, or a specific part of the small intestine such as the jejunum), the ingestible device may be configured to obtain a sample from that portion of the GI tract, and store the sample in one or more sampling or incubation chambers within the ingestible device.

In another embodiment, an ingestible device may be localized using a gamma scintigraphy technique or other radio-tracker technology as employed by Phaeton Research's Enterion^{™} capsule (See Teng, Renli, and Juan Maya. "Absolute bioavailability and regional absorption of ticagrelor in healthy volunteers." Journal of Drug Assessment 3.1 (2014):43-50), or monitoring the magnetic field strength of permanent magnet in the ingestible device (see T. D. Than, et al., "A review of localization systems for robotic endoscopic capsules," IEEE Trans. Biomed. Eng., vol. 59, no. 9, pp. 2387-2399, Sep. 2012).

In still other disclosures, an ingestible device may include a camera for generating video imaging data of the GI tract which can be used to determine, among other things, the location of the device. Examples of video imaging capsules include Medtronic's PillCam^{™}, Olympus' Endocapsule^{®}, and IntroMedic's MicroCam^{™} (see Basar et al. "Ingestible Wireless Capsule Technology: A Review of Development and Future Indication" International Journal of Antennas and Propagation (2012); 1-14). Other imaging technologies include thermal imaging cameras, and those that employ ultrasound or Doppler principles to generate different images (see Chinese patent application CN104473611: "Capsule endoscope system having ultrasonic positioning function").

### LOCI

In some disclosures, the application provides an ingestible device for detecting an analyte in a sample, wherein the ingestible device includes a sampling chamber that is configured to hold a composition including: (1) a plurality of donor particles, each of the plurality of donor particles including a photosensitizer and having coupled thereto a first analyte-binding agent (e.g., an antigen-binding agent) that binds to the analyte, wherein the photosensitizer, in its excited state, is capable of generating singlet oxygen; and (2) a plurality of acceptor particles, each of the plurality of acceptor particles including a chemiluminescent compound and having coupled thereto a second analyte-binding agent (e.g., an antigen-binding agent) that binds to the analyte, wherein said chemiluminescent compound is capable of reacting with singlet oxygen to emit luminescence. In some disclosures, the first and the second analyte-binding agents are antigen-binding agents (e.g., antibodies). In some disclosures, the first and the second antigen-binding agents bind to the same epitope of the analyte (e.g., a protein). In some disclosures, the first and the second antigen-binding agents bind to separate epitopes of the analyte (e.g., a protein) that spatially overlap. In some disclosures, the first and the second antigen-binding agents bind to the separate epitopes of the analyte (e.g., a protein) that do not spatially overlap. In some disclosures, the first and/or second analyte binding agent(s) is an antibody. In some disclosures, the first and/or second analyte binding agent(s) is an affimer. In some disclosures, the first and/or second analyte binding agent(s) is an antigen-binding agent is an aptamer.

In some disclosures, this application provides an ingestible device for detecting an analyte in a sample, wherein the ingestible device includes a sampling chamber that is configured to hold a member made of an absorptive material (e.g., an absorptive pad or absorptive sponge) having absorbed therein a composition including: (1) a plurality of donor particles, each of the plurality of donor particles including a photosensitizer and having coupled thereto a first analyte-binding agent (e.g., an antigen-binding agent) that binds to the analyte, wherein the photosensitizer, in its excited state, is capable of generating singlet oxygen; and (2) a plurality of acceptor particles, each of the plurality of acceptor particles including a chemiluminescent compound and having coupled thereto a second analyte-binding agent (e.g., an antigen-binding agent) that binds to the analyte, wherein said chemiluminescent compound is capable of reacting with singlet oxygen to emit luminescence. In some disclosures, the first and the second analyte-binding agents are antigen-binding agents (e.g., antibodies). In some disclosures, the first and the second antigen-binding agents bind to the same epitope of the analyte (e.g., a protein). In some disclosures, the first and the second antigen-binding agents bind to separate epitopes of the analyte (e.g., a protein) that spatially overlap. In some disclosures, the first and the second antigen-binding agents bind to the separate epitopes of the analyte (e.g., a protein) that do not spatially overlap.

In some disclosures, the absorptive material is an absorptive sponge. In some disclosures, the absorptive sponge is a hydrophilic sponge. In some disclosures, the absorptive sponge is selected from the group consisting of: fibers of cotton, rayon, glass, polyester, polyethylene, polyurethane, nitrocellulose, and the like. In some disclosures, the absorptive sponge is polyester or polyethylene. In some disclosures, the absorptive sponge is selected from the group consisting of: Ahlstrom Grade 6613H, Porex 1/16" Fine Sheet 4897, Porex 1/8" Fine Sheet 4898, Porex 4588 0.024" Conjugate release pad, Porex PSU-567, and Filter Papers. In some disclosures, the absorptive sponge is Ahlstrom Grade 6613H (Lot 150191) or Porex PSU-567. The present application further provides a method for preparing an absorptive material as described herein, including the step of injecting into the absorptive material an aqueous solution including a composition of the present application. In some disclosures, the method including a step of drying the absorptive material having absorbed therein the aqueous solution at a temperature in the range of 0-100°C, 0-50°C, 0-40°C, 0-30°C, 0-20°C, 0-10°C, or 0-4°C), for a time period sufficient to reduce the total water content to below 50%, 40%, 30%, 20%, 15%, 10%, 7%, 5%, 3%, 1%, 0.7%, 0.5%, 0.3%, or 0.1% by weight.

In some disclosures, the disclosure provides a method of measuring the presence, absence or amount of one or more analytes from one or more samples in the gastrointestinal tract. In general, in disclosures involving LOCI, the analyte is capable of being bound by two analyte-binding agents at the same time to allow for detection of the analyte using the methods described herein. Exemplary analytes that can be used in disclosures involving LOCI include, but are not limited to, proteins, peptides, and microorganisms (e.g., bacteria and/or archaea). Various examples of analytes suitable for use in disclosures involving LOCI are described above.

In some disclosures the one or more analytes are measured multiple times, for example, at different time points or at different locations. In one embodiment, a single device measures one or more analytes or more time points or locations; thereby creating a "molecular map" of a physiological region. Measurements can be taken at any location in the gastrointestinal tract. For example, in one disclosure, analytes from samples from one or more of the duodenum, jejunum, ileum, ascending colon, transverse colon or descending colon can be measured to create a molecular map of the small and large intestine. In one disclosure, the sample is from the duodenum. In one disclosure, the sample is from the jejunum. In one disclosure, the sample is from the ileum. In one aspect, the sample is from the ascending colon. In one disclosure, the sample is from the transverse colon. In one disclosure, the sample is from the descending colon.

In another disclosure, a series of measurements can be taken over a shorter distance of the gastrointestinal tract (e.g., the ileum) to create a higher resolution molecular map. In some disclosures, previous endoscopic imaging may identify a diseased area for molecular mapping (e.g., biomarker mapping). For example, a gastroenterologist can use imaging (e.g., an endoscope equipped with a camera) to identify the presence of Crohn's disease in the ileum and cecum of a patient, and the methods and techniques of the present disclosure can be used to measure inflammation-associated analytes in this diseased area of the patient. In a related disclosures, the inflammation-associated analytes, or any analyte, may be measured every one or more days to monitor disease flare-ups, or response to therapeutics. Exemplary inflammation-associated analytes include anti-glycan antibodies; *anti-Saccharomyces cerevisiae* antibodies (ASCA); anti-laminaribioside antibodies (ALCA); anti-chitobioside antibodies (ACCA); anti-mannobioside antibodies (AMCA); anti-laminarin (anti-L) antibodies; anti-chitin (anti-C) antibodies; anti-outer membrane porin C (anti-OmpC) antibodies; anti-Cbir1 flagellin antibodies; anti-I2 antibodies (see, e.g., Mitsuyama et al. (2016) World J. Gastroenterol. 22(3):1304-10); autoantibodies targeting the exocrine pancreas (PAB); perinuclear anti-neutrophil antibody (pANCA); calprotectin; a cytokine such as vascular endothelial growth factor (VEGF), C-reactive protein (CRP), interleukin-6 (IL-6), or tumor necrosis factor alpha (TNF-α); an adhesion molecule such as intracellular adhesion molecule (ICAM) (e.g., ICAM-1) or vascular adhesion molecule (VCAM) (e.g., VCAM-1); or serum amyloid A (SAA).

Photosensitizers that are to be excited by light will be relatively photostable and will not react efficiently with singlet oxygen. Several structural features are present in most useful sensitizers. Most sensitizers have at least one and frequently three or more conjugated double or triple bonds held in a rigid, frequently aromatic structure. They will frequently contain at least one group that accelerates intersystem crossing such as a carbonyl or imine group or a heavy atom selected from rows 3-6 of the periodic table, especially iodine or bromine, or they may have extended aromatic structures. Typical sensitizers include acetone, benzophenone, 9-thioxanthone, eosin, 9,10-dibromoanthracene, methylene blue, metallo-porphyrins, such as hematoporphyrin, phthalocyanines, chlorophylls, rose bengal, buckminsterfullerene, etc., and derivatives of these compounds having substituents of 1 to 50 atoms for rendering such compounds more lipophilic or more hydrophilic and/or as attaching groups for attachment. Examples of other photosensitizers that can be utilized in the present methods and devices are those that have the above properties and are enumerated in N. J. Turro, "Molecular Photochemistry," page 132, W. A. Benjamin Inc., N.Y. 1965.

In some disclosures, the photosensitizers are relatively non-polar to assure dissolution into a lipophilic member when the photosensitizer is incorporated in an oil droplet, liposome, latex particle, etc.

In some disclosures, the photosensitizers suitable for use herein include other substances and compositions that can produce singlet oxygen with or without activation by an external light source. Thus, for example, molybdate (MoO₄ ⁼) salts and chloroperoxidase and myeloperoxidase plus bromide or chloride ion (Kanofsky, J. Biol. Chem. (1983) 259 5596) have been shown to catalyze the conversion of hydrogen peroxide to singlet oxygen and water. Either of these compositions can, for example, be included in particles and used in the assay method wherein hydrogen peroxide is included as an ancillary reagent, chloroperoxidase is bound to a surface and molybdate is incorporated in the aqueous phase of a liposome. Also included within the scope of the disclosure as photosensitizers are compounds that are not true sensitizers but which on excitation by heat, light, or chemical activation will release a molecule of singlet oxygen. The best known members of this class of compounds includes the endoperoxides such as 1,4-biscarboxyethyl-1,4-naphthalene endoperoxide, 9,10-diphenylanthracene-9,10-endoperoxide and 5,6,11,12-tetraphenyl naphthalene 5,12-endoperoxide. Heating or direct absorption of light by these compounds releases singlet oxygen.

A chemiluminescent compound is a substance that undergoes a chemical reaction with singlet oxygen to form a metastable intermediate that can decompose with the simultaneous or subsequent emission of light within the wavelength range of 250 to 1200 nm. Exemplary chemiluminescent compounds suitable for use in the present application include those described in U.S. Patent Nos. 6,251,581 and 7,709,273, and International Publication No. WO 1999/042838. Exemplary chemiluminescent compounds include the following:

| **Chemiluminescent** | **Half-Life** | **Emission Max** |
|---|---|---|
| Thioxene + Diphenyl anthracene | 0.6 seconds | 430 nm |
| Thioxene + Umbelliferone derivative | 0.6 seconds | 500 nm |
| Thioxene + Europium chelate | 0.6 seconds | 615 nm |
| Thioxene + Samarium Chelate | 0.6 seconds | 648 nm |
| Thioxene + terbium Chelate | 0.6 seconds | 540nm |
| N-Phenyl Oxazine + Umbelliferone derivative | 30 seconds | 500 nm |
| N-Phenyl Oxazine + Europium chelate | 30 seconds | 613nm |
| N-phenyl Oxazine + Samarium Chelate | 30 seconds | 648 nm |
| N-phenyl Oxazine + terbium Chelate | 30 seconds | 540nm |
| Dioxene + Umbelliferone derivative | 300 seconds | 500 nm |
| Dioxene + Europium chelate | 300 seconds | 613nm |
| Dioxene + Samarium Chelate | 300 seconds | 648 nm |
| N-phenyl Oxazine + terbium Chelate | 300 seconds | 540nm |

Emission will usually occur without the presence of an energy acceptor or catalyst to cause decomposition and light emission. In some disclosures, the intermediate decomposes spontaneously without heating or addition of ancillary reagents following its formation. However, addition of a reagent after formation of the intermediate or the use of elevated temperature to accelerate decomposition can be desirable for some chemiluminescent compounds. The chemiluminescent compounds are usually electron rich compounds that react with singlet oxygen, frequently with formation of dioxetanes or dioxetanones. Exemplary of such compounds are enol ethers, enamines, 9-alkylidenexanthans, 9-alkylidene-N-alkylacridans, aryl vinyl ethers, dioxenes, arylimidazoles and lucigenin. Other chemiluminescent compounds give intermediates upon reaction with singlet oxygen, which subsequently react with another reagent with light emission. Exemplary compounds are hydrazides such as luminol and oxalate esters.

The chemiluminescent compounds of interest will generally emit at wavelengths above 300 nanometers and usually above 400 nm. Compounds that alone or together with a fluorescent molecule emit light at wavelengths beyond the region where serum components absorb light will be of particular use in the present methods and devices. The fluorescence of serum drops off rapidly above 500 nm and becomes relatively unimportant above 550 nm. Therefore, when the analyte is in serum, chemiluminescent compounds that emit light above 550 nm, e.g., above 600 nm may be suitable for use. In order to avoid autosensitization of the chemiluminescent compound, in some disclosures, the chemiluminescent compounds do not absorb light used to excite the photosensitizer. In some disclosures, the sensitizer is excited with light wavelengths longer than 500 nm, it will therefore be desirable that light absorption by the chemiluminescent compound be very low above 500 nm.

Where long wavelength emission from the chemiluminescent compound is desired, a long wavelength emitter such as a pyrene, bound to the chemiluminescent compound can be used. Alternatively, a fluorescent molecule can be included in the medium containing the chemiluminescent compound. In some disclosures, fluorescent molecules will be excited by the activated chemiluminescent compound and emit at a wavelength longer than the emission wavelength of the chemiluminescent compound, usually greater than 550 nm. It is usually also desirable that the fluorescent molecules do not absorb at the wavelengths of light used to activate the photosensitizer. Examples of useful dyes include rhodamine, ethidium, dansyl, Eu(fod)₃, Eu(TTA)₃, Ru(bpy)₃ ⁺⁺ (wherein bpy=2,2'-dipyridyl, etc. In general these dyes act as acceptors in energy transfer processes and in some disclosures, have high fluorescent quantum yields and do not react rapidly with singlet oxygen. They can be incorporated into particles simultaneously with the incorporation of the chemiluminescent compound into the particles.

In general, the particles are at least about 20 nm and not more than about 20 microns, usually at least about 40 nm and less than about 10 microns, *e.g.,* from about 0.10 to 2.0 microns diameter, normally having a volume of less than 1 picoliter. Exemplary particles (including both donor and acceptor particles) suitable for use in the present application include those described in U.S. Patent Nos. 6,251,581, and 7,709,273, and PCT International Publication No. WO1999/042838. In some disclosures, a particle as used herein may be a bead make of suitable material. The particle (e.g., a bead) may be organic or inorganic, swellable or non-swellable, porous or non-porous, having any density, but in some disclosures, of a density approximating water, generally from about 0.7 to about 1.5 g/mL, may be suspendible in water, and composed of material that can be transparent, partially transparent, or opaque. The particles may or may not have a charge, and when they are charged, in some disclosures, they are negative. The particles may be solid (*e.g.,* polymer, metal, glass, organic and inorganic such as minerals, salts and diatoms), oil droplets (*e.g.,* hydrocarbon, fluorocarbon, silicon fluid), or vesicles (*e.g.,* synthetic such as phospholipid or natural such as cells and organelles). The particles may be latex particles or other particles included of organic or inorganic polymers; lipid bilayers, *e.g.,* liposomes, phospholipid vesicles; oil droplets; silicon particles; metal sols; cells; and dye crystallites.

The organic particles will normally be polymers, either addition or condensation polymers, which are readily dispersible in the assay medium. The organic particles will also be adsorptive or functionalizable so as to bind at their surface, either directly or indirectly, an analyte-binding agent and to bind at their surface or incorporate within their volume a photosensitizer or a chemiluminescent compound.

The particles can be derived from naturally occurring materials, naturally occurring materials which are synthetically modified and synthetic materials. Natural or synthetic assemblies such as lipid bilayers, *e.g.,* liposomes and non-phospholipid vesicles, are suitable for use herein. Among organic polymers of particular interest are polysaccharides, particularly cross-linked polysaccharides, such as agarose, which is available as SEPHAROSE^{®} (Pharmacia Biotech), dextran, available as SEPHADEX^{®} (Pharmacia Biotech) and SEPHACRYL^{®} (Pharmacia Biotech), cellulose, starch, and the like; addition polymers, such as polystyrene, polyacrylamide, homopolymers and copolymers of derivatives of acrylate and methacrylate, particularly esters and amides having free hydroxyl functionalities including hydrogels, and the like. Inorganic polymers include silicones, glasses, available as Bioglas, and the like. Sols include gold, selenium, and other metals. Particles may also be dispersed water insoluble dyes such as porphyrins, phthalocyanines, etc., which may also act as photosensitizers. Particles may also include diatoms, cells, viral particles, magnetosomes, cell nuclei and the like.

Where the particles are commercially available, the particle size may be varied by breaking larger particles into smaller particles by mechanical means, such as grinding, sonication, agitation, etc.

In some disclosures, the particles are polyfunctional or are capable of being polyfunctionalized or are capable of being bound or coupled to or associated with an analyte-binding agent, photosensitizer, or chemiluminescent compound through specific or nonspecific covalent or non-covalent interactions. A wide variety of functional groups are available or can be incorporated. Exemplary functional groups include carboxylic acids, aldehydes, amino groups, cyano groups, ethylene groups, hydroxyl groups, mercapto groups and the like. When covalent attachment of an analyte-binding agent, chemiluminescent compound or photosensitizer to the particle is employed, the manner of linking is well known and is amply illustrated in the literature. See for example Cautrecasas, J. Biol, Chem., 245:3059 (1970). The length of a linking group may vary widely, depending upon the nature of the compound being linked, the nature of the particle, the effect of the distance between the compound being linked and the particle on the binding of analyte-binding agents and the analyte and the like.

The photosensitizer and/or chemiluminescent compound can be chosen to dissolve in or noncovalently bind to the surface of the particles. In some disclosures, these compounds may be hydrophobic to reduce their ability to dissociate from the particle and thereby cause both compounds to associate with the same particle. This possibly can be further reduced by utilizing particles of only one composition that are associated with either the photosensitizer or chemiluminescent compound or by using two types of particles that differ in composition so as to favor association of the photosensitizer with one type of particle and association of the chemiluminescent compound with the other type of particle.

The number of photosensitizer or chemiluminescent molecules associated with each particle will on the average usually be at least one and may be sufficiently high that the particle consists entirely of photosensitizer or chemiluminescent molecules. In some disclosures, the number of molecules will be selected empirically to provide the highest signal to background in the assay. In some cases this will be best achieved by associating a multiplicity of different photosensitizer molecules to particles. In some disclosures, the photosensitizer or chemiluminescent compound to analyte-binding agent ratio in the particles should be at least 1, such as at least 100 to 1 up to over 1,000 to 1.

Generally, oil droplets are fluid particles included of a lipophilic compound coated and stabilized with an emulsifier that is an amphiphilic molecule such as, for example, phospholipids, sphingomyelin, albumin and the like.

The phospholipids are based upon aliphatic carboxylic acid esters of aliphatic polyols, where at least one hydroxyl group is substituted with a carboxylic acid ester of from about 8 to 36, more usually of from about 10 to 20 carbon atoms, which may have from 0 to 3, more usually from 0 to 1 site of ethylenic unsaturation and at least 1, normally only 1, hydroxyl group substituted with phosphate to form a phosphate ester. The phosphate group may be further substituted with small aliphatic compounds which are of di or higher functionality, generally having hydroxyl or amino groups.

The oil droplets can be made in accordance with conventional procedures by combining the appropriate lipophilic compounds with a surfactant, anionic, cationic or nonionic, where the surfactant is present in from about 0.1 to 5, more usually from about 0.1 to 2 weight percent of the mixture and subjecting the mixture in an aqueous medium to agitation, such as sonication or vortexing. Illustrative lipophilic compounds include hydrocarbon oils, halocarbons including fluorocarbons, alkyl phthalates, trialkyl phosphates, triglycerides, etc.

An analyte-binding agent will usually be adsorbed to the surface of the oil droplet or bonded directly or indirectly to a surface component of the oil droplet. The analyte-binding agent may be incorporated into the liquid particles either during or after the preparation of the liquid particles. The analyte-binding agent will normally be present from about 0.5 to about 100, about 1 to about 90, about 5 to about 80 or about 50 to about 100 mole percent of the molecules present on the surface of the particle.

The following is a list, by way of illustration and not limitation, of amphiphilic compounds, which may be utilized for stabilizing oil droplets: phosphatidyl ethanolamine, phosphatidyl choline, phosphatidyl serine, dimyristoylphosphatidyl choline, egg phosphatidyl choline, diapalmitoylphosphatidyl choline, phosphatidic acid, cardiolipin, lecithin, galactocerebroside, sphingomyelin, dicetylphosphate, phosphatidyl inositol, 2-trihexadecylammoniumethylamine, 1,3-bis(octadecyl phosphate)-propanol, stearoyloxyethylene phosphate, phospholipids, dialkylphosphates, sodium dodecyl sulfate, cationic detergents, anionic detergents, proteins such as albumin, non-ionic detergents, etc.

Other compounds may also be used which have lipophilic groups and which have been described previously. For the most part, these compounds will be alkylbenzenes, having alkyl groups of from about 6 to about 20 carbon atoms, usually mixtures of alkyl groups, which may be straight or branched chain, and having a carboxyl group, an hydroxyl group, a polyoxy alkylene group (alkylene of from about 2 to about 3 carbon atoms), carboxylic group, sulfonic acid group, or amino group. Aliphatic fatty acids may be used which will normally be of from about about 10 to about 36, more usually of from about about 12 to 2 about 0 carbon atoms. Also, fatty alcohols having the carbon limits indicated for the fatty acids, fatty amines of similar carbon limitations and various steroids may also find use.

The oil droplets can include a fluorocarbon oil or a silicone oil (silicon particle). Such droplets are described by Giaever in U.S. Pat. Nos. 4,634,681 and 4,619,904. These droplets are formed by dispersing a fluorocarbon oil or silicone oil in an aqueous phase. The droplets are prepared by placing a small amount of the selected oil (generally, such oils are commercially available) in a container with a larger amount of the aqueous phase. The liquid system is subjected to agitation to bring about emulsification and then centrifuged. The homogeneous phase is removed and the residual droplets are resuspended in an aqueous buffered medium. The above centrifugation and decantation steps can be repeated one or more times before the droplets are utilized.

Analyte-binding agents can be bound to the droplets in a number of ways. As described by Giaever, the particular analyte-binding agents, particularly a proteinaceous analyte-binding agent, can be coated on the droplets by introducing an excess of the analyte-binding agent into the aqueous medium prior to or after the emulsification step. Washing steps are desirable to remove excess analyte-binding agent. Functionalization of the oil introduces functionalities described above for linking to analyte-binding agents. Such functionalities can also be employed to link the droplets to a photosensitizer or a chemiluminescent compound. On the other hand, the photosensitizer or chemiluminescent compound will frequently be chosen to be soluble in the oil phase of the oil droplet and will not be covalently bound. When the oil is a fluorocarbon, a fluorinated photosensitizer or chemiluminescent compound will often be more soluble than the corresponding unfluorinated derivation. Other oil droplets described by Giaever also find use in the present methods and devices.

In general, liposomes are microvesicles of approximately spherical shape and are one of the materials for use in the present methods and devices. The liposomes have a diameter that is at least about 20 nm and not more than about 20 microns, usually at least about 40 nm and less than about 10 microns. In some disclosures, the diameter of the liposomes will be less than about two microns so as to limit settling or floatation.

The outer shell of a liposome consists of an amphiphilic bilayer that encloses a volume of water or an aqueous solution. Liposomes with more than one bilayer are referred to as multilamellar vesicles. Liposomes with only one bilayer are called unilamellar vesicles. Multilamellar vesicles are suitable for use in the present methods and devices when using a lipophilic photosensitizer or chemiluminescent compound because of their ability to incorporate larger quantities of these materials than unilamellar vesicles. The amphiphilic bilayer is frequently included of phospholipids. Phospholipids employed in preparing particles utilizable in the present methods and devices can be any phospholipid or phospholipid mixture found in natural membranes including lecithin, or synthetic glyceryl phosphate diesters of saturated or unsaturated 12-carbon or 24-carbon linear fatty acids wherein the phosphate can be present as a monoester, or as an ester of a polar alcohol such as ethanolamine, choline, inositol, serine, glycerol and the like. Suitable phospholipids include, but are not limited to, L-α-palmitoyl oleoyl-phosphatidylcholine (POPC), palmitoyl oleoylphosphatidyl-glycerol (POPG), L-α-dioleoylphosphatidylglycerol, L-α(dioleoyl)-phosphatidyl ethanolamine (DOPE) and L-α(dioleoyl)-phosphatidyl β-(4-(N-maleimidomethyl)-cyclohexane-1-carboxyamido)ethanol (DOPE-MCC).

The phospholipids in the bilayer may be supplemented with cholesterol and may be replaced with other amphiphilic compounds that have a polar head group, usually charged, and a hydrophobic portion usually including two linear hydrocarbon chains. Examples of such substituents include dialkylphosphate, dialkoxypropylphosphates wherein the alkyl groups have linear chains of about 12-20 carbon atoms, N-(2,3-di(9-(Z)-octa-decenyloxy))-prop-1-yl-N,N,N,-trimethyl-ammonium chloride (DOTMA) (as disclosed in U.S. Patent Application Ser. No. 811,146 filed on Dec. 19, 1985), sphingomyelin, cardiolipin, and the like.

In some disclosures, liposomes utilized in the present methods and devices have a high negative charge density to stabilize the suspension and to prevent spontaneous aggregation.

For use in the present methods and devices, the liposomes should be capable of binding to an analyte-binding agent and be capable of having a photosensitizer or chemiluminescent compound associated with either the aqueous or the nonaqueous phase. The liposomes utilized in the present methods and devices will usually have analyte-binding agents bound to the outer surface of the lipid vesicle.

Liposomes may be produced by a variety of methods including hydration and mechanical dispersion of dried phospholipid or phospholipid substitute in an aqueous solution. Liposomes prepared in this manner have a variety of dimensions, compositions and behaviors. One method of reducing the heterogeneity and inconsistency of behavior of mechanically dispersed liposomes is by sonication. Such a method decreases the average liposome size. Alternatively, extrusion is usable as a final step during the production of the liposomes. U.S. Pat. No. 4,529,561 discloses a method of extruding liposomes under pressure through a uniform pore-size membrane to improve size uniformity.

Preparation of liposomes containing a hydrophobic or amphiphilic photosensitizer or a chemiluminescent compound dissolved in the lipid bilayer can be carried out in a variety of methods, including a method described by Olsen, et al., Biochemica et Biophysica Acta, 557(9), 1979. Briefly, a mixture of lipids containing the appropriate compound in an organic solvent such as chloroform is dried to a thin film on the walls of a glass vessel. The lipid film is hydrated in an appropriate buffer by shaking or vortexing. Thereafter, the lipid suspension is extruded through a series of polycarbonate filter membranes having successively smaller pore sizes, for example, 2.0, 1.0, 0.8, 0.6, 0.4, and 0.2 microns. Repeated filtration through any of the filters, and in particular through the smallest filter, is desirable. The liposomes can be purified by, for example, gel filtration, such as through a column of SEPHACRYL^{®} S-1000 (Pharmacia Biotech). The column can be eluted with buffer and the liposomes collected. Storage in the cold prolongs shelf-life of the liposomes produced by this method. Alternatively the photosensitizer or chemiluminescent compound can be added to the liquid suspension following preparation of the liposomes.

Labeling of droplets and liposomes will often involve, for example, inclusion of thiol or maleimide or biotin groups on the molecules including the lipid bilayer. Photosensitizers, chemiluminescent molecules or analyte-binding agents may then be bound to the surface by reaction of the particles with one of these materials that is bound to a sulfhydryl reactive reagent, a sulfhydryl group, or avidin, respectively. Sulfhydryl reactive groups include alkylating reagents such as bromoacetamide and maleimide.

Analyte-binding agents can be attracted to the surface of the liposome particles by weak hydrophobic interactions, however such interactions are not generally sufficient to withstand the shear force encountered during incubation and washing. It is possible to covalently bond analyte-binding agents to a liposome particle that has been functionalized, for example by use of DOPE-MCC, as shown above, by combining said liposome with the selected analyte-binding agent functionalized with a mercaptan group. For example, if the analyte-binding agent is an antibody, it may be reacted with S-acetyl-mercaptosuccinic anhydride (SAMSA) and hydrolyzed to provide a sulfhydryl modified antibody.

Generally, latex signifies a particulate water suspendible water insoluble polymeric material usually having particle dimensions of 20 nm to 20 µm, *e.g.,* 100 to 1000 nm in diameter. The latex is frequently a substituted polyethylene such as: polystyrene-butadiene, polyacrylamide polystyrene, polystyrene with amino groups, poly-acrylic acid, polymethacrylic acid, acrylonitrile-butadiene, styrene copolymers, polyvinyl acetate-acrylate, polyvinyl pyrridine, vinyl-chloride acrylate copolymers, and the like. Non-crosslinked polymers of styrene and carboxylated styrene or styrene functionalized with other active groups such as amino, hydroxyl, halo and the like are suitable for use herein. In some disclosures, copolymers of substituted styrenes with dienes such as butadiene will be used.

The association of the photosensitizer or chemiluminescent compound with latex particles utilized in the present methods and devices can involve incorporation during formation of the particles by polymerization but will usually involve incorporation into preformed particles, usually by noncovalent dissolution into the particles. Usually a solution of the chemiluminescent compound or sensitizer will be employed. Solvents that may be utilized include alcohols, including ethanol, ethylene glycol and benzyl alcohol; amides such as dimethyl formamide, formamide, acetamide and tetramethyl urea and the like; sulfoxides such as dimethyl sulfoxide and sulfolane; and ethers such as carbitol, ethyl carbitol, dimethoxy ethane and the like, and water. The use of solvents having high boiling points in which the particles are insoluble permits the use of elevated temperatures to facilitate dissolution of the compounds into the particles and are particularly suitable. The solvents may be used singly or in combination. In some disclosures, solvents for incorporating photosensitizer are those that will not quench the triplet excited state of the photosensitizer either because of their intrinsic properties or because they can subsequently be removed from the particles by virtue of their ability to be dissolved in a solvent such as water that is insoluble in the particles. Aromatic solvents are also suitable for use herein, such as solvents that are soluble in the particle. For incorporating chemiluminescent compounds in particles a solvent should be selected that does not interfere with the luminescence because of their intrinsic properties or ability to be removed from the particles. In some disclosures, aromatic solvents may be used. Typical aromatic solvents include dibutylphthalate, benzonitrile, naphthonitrile, dioctylterephthalate, dichlorobenzene, diphenylether, dimethoxybenzene, etc.

Except when the photosensitizer or chemiluminescent compound is to be covalently bound to the particles, it may be suitable to use electronically neutral photosensitizers or chemiluminescent compounds. In some disclosures, the liquid medium selected does not soften the polymer beads to the point of stickiness. One technique includes suspending the selected latex particles in a liquid medium in which the photosensitizer or chemiluminescent compound has at least limited solubility. In some disclosures, the concentrations of the photosensitizer and chemiluminescent compound in the liquid media will be selected to provide particles that have the highest efficiency of singlet oxygen formation and highest quantum yield of emission from the chemiluminescent compound in the media but less concentrated solutions will sometimes be used. Distortion or dissolution of the particles in the solvent can be prevented by adding a miscible cosolvent in which the particles are insoluble.

Generally, the temperature employed during the procedure will be chosen to maximize the singlet oxygen formation ability of the photosensitizer labeled particles and the quantum yield of the chemiluminescent compound particles with the proviso that the particles should not melt or become aggregated at the selected temperature. Elevated temperatures are normally employed. The temperatures for the procedure will generally range from 20° C to 200° C, more usually from 50° C to 170° C. It has been observed that some compounds that are nearly insoluble at room temperature are soluble in, for example, low molecular weight alcohols, such as ethanol and ethylene glycol and the like, at elevated temperatures. Carboxylated modified latex particles have been shown to tolerate low molecular weight alcohols at such temperatures.

An analyte-binding agent may be physically adsorbed on the surface of the latex particle or may be covalently bonded to the particle. In cases wherein the analyte-binding agent is only weakly bound to the surface of the latex particle, the binding may in certain cases be unable to endure particle-to-particle shear forces encountered during incubation and washings. Therefore, it may be suitable to covalently bond analyte-binding agents to the latex particles under conditions that will minimize adsorption. This may be accomplished by chemically activating the surface of the latex. For example, the N-hydroxysuccinimide ester of surface carboxyl groups can be formed and the activated particles to reduce nonspecific binding of assay components to the particle surface are then contacted with a linker having amino groups that will react with the ester groups or directly with an analyte-binding agent that has an amino group. The linker will usually be selected to reduce nonspecific binding of assay components to the particle surface and will in some disclosures, provide suitable functionality for both attachment to the latex particle and attachment of the analyte-binding agent. Suitable materials include maleimidated aminodextran (MAD), polylysine, aminosaccharides, and the like. MAD can be prepared as described by Hubert, et al., Proc. Natl. Acad. Sci., 75(7), 3143, 1978.

In one method, MAD is first attached to carboxyl-containing latex particles using a water soluble carbodiimide, for example, 1-(3-dimethylaminopropyl)-3-ethyl carbodiimide. The coated particles are then equilibrated in reagents to prevent nonspecific binding. Such reagents include proteins such as bovine gamma globulin (BGG), and detergent, such as Tween^{®} 20, (ICI Americas, Inc.) TRITON X-100^{®} (Rohm and Haas Company) and the like. xAn analyte-binding agent having a sulfhydryl group, or suitably modified to introduce a sulfhydryl group, is then added to a suspension of the particles, whereupon a covalent bond is formed between the analyte-binding agent and the MAD on the particles. Any excess unreacted analyte-binding agent can then be removed by washing.

In general, metal sols are particles included of a heavy metal, *e.g.,* a metal of atomic number greater than 20 such as a Group IB metal, *e.g.,* gold or silver or chalcogens such as selenium or tellurium.

Metal sol particles are described, for example, by Leuvering in U.S. Pat. No. 4,313,734. Such sols include colloidal aqueous dispersion of a metal, metal compound, or polymer nuclei coated with a metal or metal compound.

The metal sols may be of metals or metal compounds, such as metal oxides, metal hydroxides and metal salts or of polymer nuclei coated with metals or metal compounds. Examples of such metals are platinum, gold, silver mercury, lead, palladium, and copper, and of such metal compounds are silver iodide, silver bromide, copper hydrous oxide, iron oxide, iron hydroxide or hydrous oxide, aluminum hydroxide or hydrous oxide, chromium hydroxide or hydrous oxide, vanadium oxide, arsenic sulphide, manganese hydroxide, lead sulphide, mercury sulphide, barium sulphate and titanium dioxide. In general, the metals or metal compounds useful may be readily demonstrated by means of known techniques.

In some disclosures, it may advantageous to use sols included of dispersed particles consisting of polymer nuclei coated with the above mentioned metals or metal compounds. These particles have similar properties as the dispersed phase of pure metals or metal compounds, but size, density and metal contact can be optimally combined.

The metal sol particles may be prepared in a large number of ways which are in themselves known. For example, for the preparation of a gold sol Leuvering refers to an article by G. Frens in Nature Physical Science 241:20 (1973).

The metal sol particles can be modified to contain various functional groups as described above for linking to an analyte-binding agent or a photosensitizer or a chemiluminescent compound. For example, polymeric bonding agents can be used to coat the particles such as polymers containing thiol groups that bond strongly to many heavy metals or silylating agents that can bond and form polymeric coatings as, for example, by reaction of metal particles with trialkoxy aminoalkylsilanes as described in EPO Patent Appl. 84400952.2 by Advanced Magnetics for coating magnetic particles.

Generally, dye crystallites are microcrystals of pure or mixed solid water-insoluble dyes, such as those described herein. The dye crystallites useful in the present methods and devices have a size range of 20 nm to 20 µm.

One method for preparing dye crystallites is described in U.S. Pat. No. 4,373,932 (Gribnau, et al.). Gribnau describes colloidal dye particles and aqueous dispersions of a hydrophobic dye or pigment, which may have an immunochemically reactive component directly or indirectly attached. The dye particles are prepared in general by dispersing a dye in water and then centrifuging. A dye pellet is obtained and resuspended in water, to which glass beads are added. This suspension is rolled for several days at room temperature. The liquid is decanted and centrifuged, and the dye particles are obtained after aspiration of the liquid.

Another method for preparing dye crystallites is by slow addition of a solution of the dye in a water miscible solvent to water. Another method is by sonication of a suspension of the solid dye in water.

Binding of analyte-binding agents to the dye particles can be achieved by direct or indirect adsorption or covalent chemical attachment. The latter is governed by the presence of suitable functional groups in any coating material and in the dye. For example, functional groups can be introduced onto the surface of a dye crystallite by coupling a compound containing a diazotized aromatic amino group and the desired functional group to a phenolic or anilino group of the dye.

Where the dye has a carboxyl group, the dye crystallite can be activated by a carbodiimide and coupled to a primary amino component. Aliphatic primary amino groups and hydroxyl groups can be activated, for example, by cyanogen bromide or halogen-substituted di- or tri-azines, after which attachment with a primary amino component or with, for example, a component containing a -SH, or -OH or group can take place. Use can also be made of bifunctional reactive compounds. For example, glutaraldehyde can be used for the mutual coupling of primary amino components of the dye and an analyte-binding agent, and, for example, a hetero-bifunctional reagent such as N-succinimidyl 3-(2-pyridyldithio) propionate can be employed for the coupling of a primary amino component to a component containing a thiol group.

In some disclosures, the composition for use in the ingestible devices of the present application further includes a medium having suspended therein said plurality of donor particles and said plurality of acceptor particles. In some disclosures, the medium is an aqueous medium. In some disclosures, the aqueous medium has a pH selected from 5-8 (e.g., a pH selected from 6-7.8, such as pH being 6.0). The standard buffer was 50 mM sodium phosphate/0.15 M NaCl, pH 7.0. St.Av Donar beads are incubated in 1um HABA ((2-(4-hydroxyphenylazo)benzoic acid) before depositing them on the pad. The assay buffer was 0.1 M Tris HCl/0.3 M NaCl/bovine serum albumin (1 mg/mL), pH 8.2.with 50 mM hydroxyl propyl cyclodextrin and tween-20-0.1%.

Suitable acceptor particles for use in the ingestible devices of this application may be any type of particles as described herein. In some disclosures, the acceptor particles are selected from the group consisting of latex particles, lipid bilayers, oil droplets, silica particles, and metal sols. In some disclosures, the acceptor particles are latex particles, such as, but not limited Polystyrene latex particles (175 nm) having about 8.3 carboxyl groups per nm² of surface, and/or the like.

Suitable chemiluminescent compounds for use in the ingestible devices of this application include those chemiluminescent compounds or substances as described in PCT International Publication No. WO 1999/042838 A1 (Table 1); and U.S. Patent No. 7,709, 273. In some disclosures, the chemiluminescent compounds are selected from the group consisting of example chemiluminescent compounds described in PCT International Publication No. WO 1999/042838 A1 (Table 1); and U.S. Patent No. 7,709, 273.

Suitable donor particles for use in the ingestible devices of this application may be any type of particles as described herein. In some disclosures, the donor particles are selected from the group consisting of latex particles, lipid bilayers, oil droplets, silica particles, and metal sols. In some disclosures, the donor particles are latex particles, such as, but not limited to Polystyrene latex particles (175 nm) having about 8.3 carboxyl groups per nm² of surface. Latex particles can vary between 175 nm to 800 nm. In some disclosures, the donor particles are latex particles (e.g., any type of latex particles described herein) that are coated with streptavidin.

Suitable photosensitizers for use in the ingestible devices of this application include any of the photosensitizers as described U.S. Patent Nos. 6,251,581, 5,516,636, 8,907,081, 6,545,012, 6,331,530, 8,247,180, 5,763,602, 5,705,622, 5,516,636, 7,217,531, and U.S. Patent Publication No. 2007/0059316. In some disclosures, the photosensitizers are selected from the group consisting of t-Butyl Silicon Phthalocyanine, Chlorophyll, and Silicon Napthalo cyanine.

The photosensitizer and chemiluminescent compound can be incorporated into donor and acceptor particles, respectively, by virtue of being soluble in at least one phase of the particles, in which case the photosensitizer and chemiluminescent compound will be at much higher concentration within the particle than in the bulk assay medium. When the photosensitizer and chemiluminescent compound are covalently bound to donor and acceptor particles, respectively, the photosensitizer and chemiluminescent compound or the particles, or components thereof, are functionalized to provide a means of attaching the photosensitizer and chemiluminescent compounds and the particles. Example ways to incorporate photosensitizer and chemiluminescent compounds in latex particles can be found in PCT International Publication No. WO1999/042838 and U.S. Patent No. 7,709,273. For particles that are oil droplets or liposomes the photosensitizer and chemiluminescent compound can be attached to one or more long hydrocarbon chains, each generally having at least 10 to 30 carbon atoms. If the particles are droplets of a fluorocarbon, the photosensitizer or chemiluminescent compound incorporated into these particles may be fluorinated to enhance solubility and reduce exchange into other particles bound with the other label, and the hydrocarbon chain used for linking will preferably be replaced with a fluorocarbon chain. For silicon fluid particles the photosensitizer and chemiluminescent compound can be bound to a polysiloxane. In order to maximize the number of photosensitizer or chemiluminescent compound molecules per particle, in some disclosures, it may be desirable to minimize the charge and polarity of the photosensitizer or chemiluminescent compound so that it resides within the non-aqueous portion of the particle. When the particle is a liposome and it is desired to retain the photosensitizer or chemiluminescent compound in the aqueous phase of the liposome, in some disclosures, photosensitizers or chemiluminescent compounds that are highly polar or charged may be used.

In some disclosures, the ratio of the number of donor particles to the number of the acceptor particles ranges from 10:1 to 1:10. In some disclosures, the ratio of the number of donor particles to the number of the acceptor particles ranges from 5:1 to 1:10. In some disclosures, the ratio of the number of donor particles to the number of the acceptor particles ranges from 5:1 to 10:1.

Suitable analytes to be detected, quantified, or measured by the ingestible devices of this application include any of the analytes as described herein. In some disclosures, the analyte is selected from the group consisting of proteins, peptides, cell surface receptors, receptor ligands, nucleic acids, carbohydrates, cells, microorganisms, and fragments thereof. In some disclosures, the analyte is selected from the group consisting of TNFα, lipoteichoic acid (LTA), lipopolysaccharide (LPS), lipopolysaccharide binding protein (LBP), calprotectin, cytokines and chemokines, IL12/23, IL-6, IL-10, MADCAM, α4β7 integrin, HGF, EGF, HB-EGF, TGFb, Adalimumab, Infliximab, Cimzia, Vedolizumab, Tysabri, Simponi, Remsima, bevacizumab (Avastin), and cetuximab (Erbitux).

The first analyte-binding agent coupled to or associated with the donor particles and the second analyte-binding agent coupled to or associated with the acceptor particles are capable of binding to the same analyte. The analyte, when present, therefore affects the amount of the photosensitizer of the donor particles and the chemiluminescent compound of the acceptor particles that can come into close proximity, wherein the short-lived singlet oxygen generated by the photosensitizer can react with the chemiluminescent compound prior to its spontaneous decay and upon reaction, the chemiluminescent compound produces luminescence. The intensity of luminescence produced is related to the amount of analyte in the sample. The chemiluminescent compound is capable of activation by singlet oxygen, and the photosensitizer catalyzes the formation of singlet oxygen usually in response to photoexcitation followed by energy transfer to molecular oxygen.

In some disclosures, an analyte causes molecules of the photosensitizer and the chemiluminescent compound to be closer to each other than their average distance in the bulk solution of the assay medium. This partitioning will depend upon the amount of analyte present in the sample to be analyzed. The photosensitizer molecules that do not become associated with the chemiluminescent compound produce singlet oxygen that is unable to reach the chemiluminescent compound before undergoing decay in the aqueous medium. However, when the photosensitizer and the chemiluminescent compound come in close proximity with each other in response to the amount of the analyte, the singlet oxygen produced upon irradiation of the photosensitizer can activate the chemiluminescent compound before undergoing decay.

In some disclosures, the first analyte-binding agent coupled to or associated with the donor particles and the second analyte-binding agent coupled to or associated with the acceptor particles are independently selected from the group consisting of antibodies, aptamers, cell surface receptors, receptor ligands, biotin, streptavidin, avidin, protein A, G, and L, and derivatives thereof. In some disclosures, the first analyte-binding agent is the same as the second analyte-binding agent. In some disclosures, the first analyte-binding agent is different from the second analyte-binding agent. In some disclosures, the first analyte-binding agent is an antibody or a derivative thereof (e.g., a biotinylated antibody). In some disclosures, the second analyte-binding agent is an antibody or a derivative thereof (e.g., a biotinylated antibody). In some disclosures, the second analyte-binding agent is an antibody covalently conjugated to the acceptor particles. In some disclosures, the first analyte-binding agent is a biotinylated antibody and the donor particles are coated with streptavidin. In some disclosures, the first analyte-binding agent is a biotinylated antibody and the donor particles are coated with streptavidin, and the second analyte-binding agent is an antibody covalently conjugated to the acceptor particles.

In some disclosures, the first analyte-binding agent coupled to or associated with the donor particles is an anti-bacterial antibody. In some disclosures, the antibody is selected from the group consisting of anti-Gram positive bacteria antibodies, anti-Gram positive bacteria LTA antibodies, anti-Gram negative bacteria antibodies, anti-lipoteichoic acid antibodies, anti-*E. coli* antibodies, anti-lipid A antibodies, anti-TNFα antibodies, and derivatives thereof. In some disclosures, the antibody is selected from the group consisting of MA1-7401 antibody, MA1-40134 antibody, ab127996 antibody, ab35654 antibody, ab35654 antibody, ab137967 antibody, ab8467 antibody, and derivatives or fragments thereof.

In some disclosures, the second analyte-binding agent coupled to or associated with the acceptor particles is an antibody selected from the group consisting of anti-bacterial antibodies. In some disclosures, the antibody is selected from the group consisting of anti-Gram positive bacteria antibodies, anti-Gram positive bacteria LTA antibodies, anti-Gram negative bacteria antibodies, anti-lipoteichoic acid antibodies, anti-*E. coli* antibodies, anti-lipid A antibodies, anti- TNFα antibodies, and derivatives thereof. In some disclosures, the antibody is selected from the group consisting of MA1-7401 antibody, MA1-40134 antibody, ab127996 antibody, ab35654 antibody, ab35654 antibody, ab137967 antibody, ab8467 antibody, and derivatives or fragments thereof.

In some disclosures, the donor particles include more than one type of analyte-binding agent. In some disclosures, the acceptor particles include more than one type of analyte-binding agent. For example, analyte-specific reagents can be used on donor and acceptor beads. For Analyte 1, parameters are set as excitation at 680nm, emission at 615 nm (half-life 0.6 seconds). For Analyte 2, parameters are set as excitation at 680nm, emission at 615 nm (half-life 30 seconds). For Analyte 3, parameters are set as excitation at 680nm, emission at 615 nm (half-life 300 seconds). After the first excitation, emission for analyte 1 is measure from: 0-6 seconds; emission for analyte 2 is measured from 6 - 300 seconds; and emission for analyte 3 is measured from 300-600 seconds. Signals are deconvoluted as described in further detail in PCT International Publication No. WO1999/042838. In some disclosures, emission wavelengths may be used to evaluate multiple analytes as discussed herein.

In some disclosures, the composition for use in the ingestible devices of the present application further includes cyclodextrin with a concentration range of about 25-50 mM, or about 1-500 mM.

This application provides a kit including an ingestible device as described herein. In some disclosures, the kit further includes instructions, *e.g.,* for detecting or quantifying an analyte in a sample. In some disclosures, such a device is an ingestible device for detecting or quantifying viable bacterial and/or archaeal cells *in vivo* (*e.g.,* in the GI tract).

Certain illustrative disclosures will now be described, including various systems and methods for obtaining samples using ingestible devices. In particular, techniques are described that allow an ingestible device to obtain a sample from within a gastrointestinal (GI) tract. These samples may include any of the fluids, solids, particulates, or other substances found within the GI tract. However, it will be understood by one of ordinary skill in the art that the systems and methods described herein may be adapted and modified as is appropriate for the applications being addressed, and that the systems and methods described herein may be employed in other suitable applications, and that such other additions and modifications will not depart from the scope of the present disclosure. Generally, the ingestible devices described herein may include actuators, sensors, valves, chambers, logic devices, telemetry systems, microcontrollers or other devices and processors that may be configured using a combination of hardware, firmware, and software to carry out one or more of the methods described herein.

In some disclosures, the ingestible device further includes an illuminating source. The illuminating source is capable of irradiating the composition held in the sampling chamber of the ingestible devices with light having a wavelength with energy sufficient to convert the photosensitizer to an excited state and thereby render it capable of activating molecular oxygen to singlet oxygen. The excited state for the photosensitizer capable of exciting molecular oxygen is generally a triplet state which is more than about 20, e.g., at least 23, Kcal/mol more energetic than the photosensitizer ground state. In some disclosures, the composition is irradiated with light having a wavelength of about 450 to 950 nm although shorter wavelengths can be used, for example, 230-950 nm. The luminescence produced may be measured in any convenient manner such as photographically, visually or photometrically to determine the amount thereof, which is related to the amount of analyte in the medium.

In some disclosures, the 632.6 nm emission line of a helium-neon laser is an inexpensive light source for excitation. Photosensitizers with absorption maxima in the region of about 620 to about 650 nm are compatible with the emission line of a helium-neon laser and are, therefore, useful illuminating sources for use in the present application. Example irradiating wavelengths for diode lasers include 680nm, 780nm, and/or the like. In some disclosures, the illuminating source is capable of irradiating the composition with light having a wavelength selected from the group consisting of 678 nm, 633nm, and 780nm.

Other means of excitation of the photosensitizer are also contemplated herein. In some disclosures, excitation of the photosensitizer may be achieved by energy transfer from an excited state of an energy donor such as a second photosensitizer. When a second photosensitizer is used, wavelengths of light can be used which are inefficiently absorbed by the photosensitizer but efficiently absorbed by the second photosensitizer. The second photosensitizer may be bound to an assay component that is associated/coupled, or becomes associated/coupled, with the first photosensitizer, for example, bound to a surface or incorporated in the particle having the first photosensitizer. When a second photosensitizer is employed it will usually have a lowest energy triplet state at higher energy than the lowest energy triplet state of the first photosensitizer.

In some disclosures, the ingestible device includes a detector for detecting the luminescence emitted by the chemiluminescent compound. In some disclosures, the detector is a photodiode that is capable of detecting the luminescence emitted by the chemiluminescent compound at a wavelength selected from 613 nm and 660 nm. Additional suitable detection wavelengths include, but are not limited to, 430 nm, 500 nm, 540 nm, 615 nm, and 680 nm.

In some disclosures, chemiluminescent intensity is measured with an optical reader. The actual configuration and structure of the optical reader may generally vary as is readily understood by those skilled in the art. Typically, the optical reader contains an illumination source that is capable of emitting light at a defined wavelength and a detector that is capable of registering a signal (e.g., transmitted, reflected, or fluorescence light). Optical readers may generally employ any known detection technique, including, for instance, luminescence (e.g., fluorescence, phosphorescence, etc.), absorbance (e.g., fluorescent or non-fluorescent), diffraction, etc. Exemplary optical readers, illumination sources and detectors are disclosed in U.S. Patent 7,399,608.

In some disclosures, the illumination source may be any device known in the art that is capable of providing electromagnetic radiation, such as light in the visible or near-visible range (e.g., infrared or ultraviolet light). The illumination may be multiplexed and/or collimated. In some disclosures, multiplexed analysis is enabled.

In some disclosures, the detector may be any device known in the art that is capable of sensing a signal. In some disclosures, the detector may be an electronic imaging detector that is configured for spatial discrimination. In other disclosures, the detector may be a light sensor that lacks spatial discrimination capabilities.

The present application provides ingestible devices containing a microscopic evaluation system. In some disclosures, bacterial and/or archaeal cells in a sample may be first labeled with fluorescent dyes (such as those described herein), and the fluorescently-labeled cells may be imaged and counted by the microscopic evaluation using an ingestible device as described herein. In other disclosures, the fluorescently-labeled cells are counted as they pass through an onboard flow system (e.g., microfluidic single cell channeling). Examples of flow cytometry systems include hydrodynamic focusing, small diameter capillary tube flow, and rectangular capillary tube flow. As described herein, live bacterial and/or archaeal cells are labeled, and the principles of flow cytometry are used to quantify labeled cells. Generally speaking, the photons from an incident laser beam are absorbed by the fluorophore and raised to a higher, unstable energy level. Within less than a nanosecond, the fluorophore re-emits the light at a longer representative wavelength where it is passed through a series of dichroic filters. This reemitted light can be collected and interpreted as proportional to the number of labeled bacterial and/or archaeal cells. In some disclosures, a sheath fluid is not used as part of the flow system to help accommodate the volume restrictions of the device. In some disclosures, a rectangular capillary tube is used to achieve a sufficiently large cross-sectional area and relatively thin inspection area. The flow cytometry optical system operates parallel to the fluidics system and serves to observe the redirection of light passing through the cell and delivers information about the bacterial and/or archaeal cells. In some disclosures, rather than using a conventional laser and spherical lenses to focus the light to a point, an LED and cylindrical lenses are used to focus the light to a line across a rectangular capillary tube. In other disclosures, collimating lenses are used to make the light source parallel, while cylindrical lenses are used to refine the inspection area. An exemplary optical configuration for this arrangement can be seen in FIG. 30. In some disclosures, optical filters can be added to permit the use of fluorophores. The characteristic wavelength of reemitted light from the fluorophores can be isolated and detected with the use of dichroic, bandpass, and short or long wave pass filters. Generally, multiple dichroic lenses and photomultipliers are used, however, due to space limitations, only a single side-scatter detector and forward scatter detector may be used in certain disclosures.

Where the analyte is bacterial and/or archaeal cells, one of the design challenges of integrating flow cytometry into the device is to provide a pumping mechanism. Without moving fluid, individual bacterial and/or archaeal cells cannot be identified and accounted for by flow cytometry within a fixed volume of fluid. In some disclosures, a gear motor is to move fluid through the device. For example, a micromotor including a planetary gearhead (e.g., with a 25:1 reduction) can provide the desired amount of torque to create fluid flow. In another embodiment, a series of piezoelectric resistors embedded in the surface of a microfabricated plate is used to create flow. In yet another embodiment, a micropump that includes a pair of one-way valves and uses a magnetic pump membrane actuated by an external magnetic field is used to create flow.

In some disclosures, the system architecture includes an opening and sealing mechanism combined with a rotary wiper which creates a pressure driven flow via a gear motor. The gear motor can be used for other functions in the device. As shown in FIG. 31, the components of the optics and flow chamber systems fit within the device. In some disclosures, the sample fluid is absorbed via a flexible membrane at the top of the capsule. In some disclosures, the gear motor has 270° of permissible travel which serves to open and fill the fluid chamber. During closure, the motor closes the ingress port while simultaneously pushing the fluid through the rectangular capillary tube where the optical system is located. The threaded component allows the flexible membrane to close and seal the ingress channel without changing the wiper height. In some disclosures, the volume of the sample chamber is about 25 µL, 50 µL, 75 µL or more (e.g., within the range of about 10-500 µL). In some disclosures, two or more samples are taken from the GI tract to procure a sufficient sample size. Referring to FIG. 31, an LED on the left side of the capillary tube and the two low-light detectors on the right for capturing forward and side scatter are shown. Once the fluid passes through the capillary tube, it exits the capsule via a one-way valve. In certain disclosures, the flow system allows for the detection of cell size and internal cell complexity, in addition to cell quantitation. The sampling chamber may have a capacity of 100 µL (total volume). The sampling chamber may also have multiple compartments, each storing a different assay mix for a different location within the GI tract. The compartments of the sampling chamber may each have a capacity of about 10-20 µL.

In some disclosures, the ingestible device further includes an internal calibrator. The miniaturized device may include 5 µg of OMNI Beads (Silicon Naphthalocyanine + Thioxene + Europium Chelate), having parameters set as: excitation: 780 nm; 50 millisecond delay; record emission at 615 nm for 6 seconds. The OMNI beads may be embedded on the pad and signals may be used to characterize signal uniformity and reliability of the miniaturized device. The ingestible device can be calibrated during manufacturing in the factory and before the patients ingests the device.

Internal standards may be used to calibrate the signal from the analyte of interest. The signal from internal standard will be measured at the same time as the analyte of interest. Donor beads with SA and HABA (10 µgs) + Biotin- PEG - 2,4 DNP (5 nmoles) + Anti-2,4 DNP labeled acceptor beads (10 µgs) with thioxene and Samarium Chelate or N-phenyl oxazine and Samarium Chelate may be used. Sample addition to the pad results in acceptor and donar beads binding to Biotin-PEG-2,4-DNP and forming a dimer which when excited with 680 nm light generates a chemiluminescent signal at 648 nm, *e.g.,* with either 0.6 or 30 seconds half-life depending on the analyte(s) of interest. The internal control may correct sample effects and instrument (the ingestible device) drift.

The present application provide methods for detecting, quantifying, or measuring an analyte in a sample using the ingestible devices as described herein. In some disclosures, the ingestible devices as described herein may be used to analyze samples (e.g., samples from the GItract) to detect or quantify analytes, such as viable bacterial and/or archaeal cells, in a sample *in vivo.* In some disclosures, the devices of this application may be used to measure the concentration of analytes (e.g., viable bacterial and/or archaeal cells) in various specific regions of the GI tract. Such data may be used to determine whether a subject has a condition in need for treatment (such as an infection, e.g., Small Intestinal Bacterial Overgrowth (SIBO), or a SIBO-related condition), the site of disease for treatment, or to quantify bacterial and/or archaeal populations within the GI tract (or within specific regions of the GI tract) for other diagnostic purposes. In some disclosures, the ingestible devices described herein may be used to detect and/or quantify specific genera, species, or strains of microorganisms present in a sample *in vivo.*

In some disclosures, data may be generated after the ingestible device has exited the subject, or the data may be generated *in vivo* and stored on the device and recovered *ex vivo.* Alternatively, the data can be transmitted wirelessly from the ingestible device while the device is passing through the subject (e.g., passing through the GI tract of the subject).

In some disclosures, this disclosure provides methods for detecting an analyte in a sample, such as a fluid sample, of a subject. The methods can include: (1) providing an ingestible device; (2) transferring the fluid sample of the subject into the sampling chamber of said ingestible device *in vivo;* (3) irradiating the composition held in the sampling chamber of the ingestible device with light to excite the photosensitizer; and (4) measuring total luminescence or rate of change of luminescence emitted from the composition held in the sampling chamber of the ingestible device as a function of time, thereby detecting the analyte in the fluid sample. In some disclosures, the presence of an analyte in the sample is used to diagnose and/or monitor a disease or disorder in the subject. In some disclosures, the absence of an analyte in the sample is used to diagnose and/or monitor a disease or disorder in the subject.

In some disclosures, this disclosure provides methods of determining the level of an analyte in a sample, such as a fluid sample, of a subject. The methods can include: (1) providing an ingestible device; (2) transferring the fluid sample of the subject into the sampling chamber of said ingestible device *in vivo;* (3) irradiating the composition held in the sampling chamber of the ingestible device with light to excite the photosensitizer; and (4) measuring total luminescence or rate of change of luminescence emitted from the composition held in the sampling chamber of the ingestible device as a function of time, thereby determining the level of the analyte in the fluid sample. In some disclosures, the method further comprises comparing the level of the analyte in the fluid sample with the level of analyte in a reference sample (e.g., a reference sample obtained from a healthy subject). In some disclosures, the level of the analyte in the sample is used to diagnose and/or monitor a disease or disorder in the subject or the treatment thereof.

In some disclosures, the total luminescence or the rate of change of luminescence as a function of time of the sponge is measured over multiple time points for an extended period of time in step (4). For instance, in some disclosures, the total luminescence or rate of change of luminescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total luminescence or the rate of change of luminescence as a function of time of said sample is measured continuously for a period of 0-330 minutes. In some disclosures, the method is performed *in vivo.* In some disclosures, the method includes communicating the results of the onboard assay(s) to an *ex vivo* receiver.

In certain disclosures, the disclosure provides methods of determining the level of an analyte in a sample of a subject, such as a human subject. The methods can include: (1) providing an ingestible device, said device including a sampling chamber that is configured to hold a member made of an absorptive material (*e.g.,* an absorptive pad or absorptive sponge) having absorbed therein a composition, as described herein; (2) transferring the fluid sample of the subject into the sampling chamber of said ingestible device *in vivo;* (3) fully or partially saturating the absorptive material held in the sampling chamber of the ingestible device with the fluid sample; (4) irradiating the absorptive material held in the sampling chamber of the ingestible device with light to excite the photosensitizer; and (5) measuring total luminescence or rate of change of luminescence emitted from the composition held in the sampling chamber of the ingestible device as a function of time, thereby detecting the analyte in the fluid sample. In some disclosures, the presence of an analyte in the sample is used to diagnose and/or monitor a disease or disorder in the subject. In some disclosures, the absence of an analyte in the sample is used to diagnose and/or monitor a disease or disorder in the subject.

In certain disclosures, the disclosure provides methods of determining the level of an analyte in a sample, such as a fluid sample, of a subject. The methods can include: (1) providing a ingestible device, said device including a sampling chamber that is configured to hold an a member made of absorptive material (e.g., an absorptive pad and/or an absorptive sponge) having absorbed therein a composition, as described herein; (2) transferring the fluid sample of the subject into the sampling chamber of said ingestible device *in vivo;* (3) fully or partially saturating the absorptive material held in the sampling chamber of the ingestible device with the fluid sample; (4) irradiating the absorptive material held in the sampling chamber of the ingestible device with light to excite the photosensitizer; and (5) measuring total luminescence or rate of change of luminescence emitted from the composition held in the sampling chamber of the ingestible device as a function of time, thereby determining the level of the analyte in the fluid sample. In some disclosures, the method further comprises comparing the level of the analyte in the fluid sample with the level of analyte in a reference sample (e.g., a reference sample obtained from a healthy subject). In some disclosures, the level of the analyte in the sample is used to diagnose and/or monitor a disease or disorder in the subject. In some disclosures, the method further comprises comparing the level of the analyte in the fluid sample with the level of analyte in a reference sample (e.g., a reference sample obtained from a healthy subject). In some disclosures, the level of the analyte in the sample is used to diagnose and/or monitor a disease or disorder in the subject.

In some disclosures, the total luminescence or the rate of change of luminescence as a function of time of the sponge is measured over multiple time points for an extended period of time in step (5). For instance, in some disclosures, the total luminescence or rate of change of luminescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total luminescence or the rate of change of luminescence as a function of time of said sample is measured continuously for a period of 0-330 minutes. In some disclosures, the method is performed *in vivo.* In some disclosures, the method includes communicating the results of the onboard assay(s) to an *ex vivo* receiver.

In some disclosures, the disclosure provides a method of assessing or monitoring the need to treat a subject suffering from or at risk of overgrowth of bacterial and/or archaeal cells in the gastrointestinal (GI) tract, including: (1) providing a ingestible device of the present application for detecting an analyte; (2) transferring a fluid sample from the GI tract of the subject into the sampling chamber of said ingestible device *in vivo;* (3) irradiating the composition held in the sampling chamber of the ingestible device with light to excite the photosensitizer; (4) measuring total luminescence or rate of change of luminescence emitted from the composition held in the sampling chamber of the ingestible device as a function of time; (5) correlating the total luminescence or the rate of change of luminescence as a function of time measured in step (4) to the amount of the analyte in the fluid sample; and (6) correlating the amount of the analyte in the fluid sample to the number of viable bacterial and/or archaeal cells in the fluid sample. In some disclosures, a number of viable bacterial and/or archaeal cells determined in step (6) greater than a control number of viable bacterial and/or archaeal cells, indicates a need for treatment (e.g., with an antibiotic agent described herein). In some disclosures, the control number of viable bacterial and/or archaeal cells is 10³, 10⁴, 10⁵, 10⁶, 10⁷, 10⁸, 10⁹, or more. For example, in some disclosures, a number of viable bacterial and/or archaeal cells determined in step (6) greater that about 10³ CFU/mL indicates a need for treatment. In some disclosures, a number of viable bacterial and/or archaeal cells determined in step (6) greater that about 10⁴ CFU/mL indicates a need for treatment. In some disclosures, a number of the viable bacterial and/or archaeal cells determined in step (6) greater than about 10⁵ CFU/mL indicates a need for treatment, *e.g.,* with an antibiotic agent as described herein. In some disclosures, a number of viable bacterial and/or archaeal cells determined in step (6) greater that about 10⁶ or more CFU/mL indicates a need for treatment.

In some disclosures, the disclosure provides a method of assessing or monitoring the need to treat a subject suffering from or at risk of a gastrointestinal (GI) tract microbial infection (e.g., with a pathogenic microorganism), including: (1) providing an ingestible device of the present application for detecting analyte; (2) transferring a fluid sample from the GI tract of the subject into the sampling chamber of said ingestible device *in vivo;* (3) irradiating the composition held in the sampling chamber of the ingestible device with light to excite the photosensitizer; (4) measuring total luminescence or rate of change of luminescence emitted from the composition held in the sampling chamber of the ingestible device as a function of time; (5) correlating the total luminescence or the rate of change of luminescence as a function of time measured in step (4) to the amount of the analyte in the fluid sample; and (6) correlating the amount of the analyte in the fluid sample to the number of microorganisms (e.g., pathogenic microorganisms) in the fluid sample. In some disclosures, the analyte is specific for a particular genus, species, or strain of microorganism. In some disclosures, a number of microorganisms determined in step (6) greater than a control number of microorganisms indicates a need for treatment (e.g., with an antibiotic agent described herein). In some disclosures, the control number of microorganisms is 0, 1, 10², 10³, 10⁴, 10⁵, 10⁶, 10⁷, 10⁸, 10⁹, or more. For example, in some disclosures, a number of microorganisms determined in step (6) greater that about 0 indicates a need for treatment. In some disclosures, a number of microorganisms determined in step (6) greater that about 1 indicates a need for treatment. In some disclosures, a number of microorganisms determined in step (6) greater that about 10² indicates a need for treatment. In some disclosures, a number of microorganisms determined in step (6) greater that about 10³ indicates a need for treatment. In some disclosures, a number of microorganisms determined in step (6) greater that about 10⁴ indicates a need for treatment. In some disclosures, a number of the microorganisms determined in step (6) greater than about 10⁵ indicates a need for treatment, e.g., with an antibiotic agent as described herein. In some disclosures, a number of microorganisms determined in step (6) greater that about 10⁶ indicates a need for treatment. In some disclosures, the control number of microorganisms is 0, 1, 10², 10³, 10⁴, 10⁵, 10⁶, 10⁷, 10⁸, 10⁹, or more CFU/mL. For example, in some disclosures, a number of microorganisms cells determined in step (6) greater that about 0 CFU/mL indicates a need for treatment. In some disclosures, a number of microorganisms determined in step (6) greater that about 1 CFU/mL indicates a need for treatment. In some disclosures, a number of microorganisms determined in step (6) greater that about 10² CFU/mL indicates a need for treatment. In some disclosures, a number of microorganisms determined in step (6) greater that about 10³ CFU/mL indicates a need for treatment. In some disclosures, a number of microorganisms determined in step (6) greater that about 10⁴ CFU/mL indicates a need for treatment. In some disclosures, a number of the microorganisms determined in step (6) greater than about 10⁵ CFU/mL indicates a need for treatment, e.g., with an antibiotic agent as described herein. In some disclosures, a number of microorganisms determined in step (6) greater that about 10⁶ or more CFU/mL indicates a need for treatment.

In some disclosures, the total luminescence or the rate of change of luminescence as a function of time of the sponge is measured over multiple time points for an extended period of time in step (4). For instance, in some disclosures, the total luminescence or rate of change of luminescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total luminescence or the rate of change of luminescence as a function of time of said sample is measured continuously for a period of 0-330 minutes. In some disclosures, the method is performed *in vivo.* In some disclosures, the method includes communicating the results of the onboard assay(s) to an *ex vivo* receiver. In some disclosures, the ingestible device and the method may be further used to monitor the subject after the treatment (*e.g.,* with an antibiotic). In some disclosures, the ingestible device and the method may be used to assess the efficacy of the treatment. For example, efficacious treatment may be indicated by the decrease of the number of viable bacterial and/or archaeal cells in a sample from the GI tract of the subject post-treatment. Efficacy of the treatment may be evaluated by the rate of decrease of the number of viable bacterial and/or archaeal cells in a sample from the GI tract of the subject post-treatment. In some disclosures, the ingestible device and the method may be used to detect infection with antibiotic-resistant strains of bacteria and/or archaea in a subject. For instance, such infection may be indicated where the number of viable bacterial and/or archaeal cells in a sample from the GI tract of the subject does not substantially decrease after antibiotic treatment.

In some disclosures, the presence of bacteria and/or archaea of a specific genera, species, and/or strains may be detected using an ingestible device described herein in order to ascertain which bacteria and/or archaea are resistant and/or sensitive to an antibiotic administered to the subject. For example, in some disclosures, the ingestible device and the methods described herein may be used to monitor a subject before and after treatment with an antibiotic to determine the presence and/or absence of a bacteria and/or archaea of a specific genera, species, and/or strain. A comparison of the type of bacteria and/or archaea that are present in the GI tract of the subject before and after the treatment with the antibiotic can be used to assess which types of bacteria and/or archaea were susceptible and/or resistant to the treatment with the antibiotic.

In some disclosures, the ingestible device and the methods described herein may be used to detect the presence of bacteria and/or archaea of a specific genera, species, and/or strains in a sample from the GI tract from a subject in order to select an antibiotic treatment that will render the detected bacteria and/or archaea either susceptible or resistant to the antibiotic treatment.

In certain disclosures, the disclosure provides methods of assessing or monitoring the need to treat a subject suffering from or at risk of overgrowth of bacterial and/or archaeal cells in the GI tract. The methods can include: (1) providing an ingestible device for detecting an analyte, said device including a sampling chamber that is configured to hold an absorptive material (*e.g.,* an absorptive pad and/or and absorptive sponge) having absorbed therein a composition; (2) transferring a fluid sample from the GI tract of the subject into the sampling chamber of said ingestible device *in vivo;* (3) fully or partially saturating the absorptive material held in the sampling chamber of the ingestible device with the fluid sample; (4) irradiating the absorptive material held in the sampling chamber of the ingestible device with light to excite the photosensitizer; (5) measuring total luminescence or rate of change of luminescence emitted from the composition held in the sampling chamber of the ingestible device as a function of time; (6) correlating the total luminescence or the rate of change of luminescence as a function of time measured in step (5) to the amount of the analyte in the fluid sample; and (7) correlating the amount of the analyte in the fluid sample to the number of viable bacterial and/or archaeal cells in the fluid sample. In some disclosures, a number of viable bacterial and/or archaeal cells determined in step (7) greater than a control number of viable bacterial and/or archaeal cells indicates a need for treatment (e.g., with an antibiotic agent described herein). In some disclosures, the control number of viable bacterial and/or archaeal cells is 10³, 10⁴, 10⁵, 10⁶, 10⁷, 10⁸, 10⁹, or more. For example, in some disclosures, a number of viable bacterial and/or archaeal cells determined in step (7) greater that about 10³ CFU/mL indicates a need for treatment. In some disclosures, a number of viable bacterial and/or archaeal cells determined in step (7) greater that about 10⁴ CFU/mL indicates a need for treatment. In some disclosures, a number of the viable bacterial and/or archaeal cells determined in step (7) greater than about 10⁵ CFU/mL indicates a need for treatment, *e.g.,* with an antibiotic agent as described herein. In some disclosures, a number of viable bacterial and/or archaeal cells determined in step (7) greater that about 10⁶ or more CFU/mL indicates a need for treatment.

In some disclosures, the disclosure provides a method of assessing or monitoring the need to treat a subject suffering from or at risk of a gastrointestinal (GI) tract microbial infection (e.g., with a pathogenic microorganism), including: (1) providing an ingestible device for detecting an analyte, said device including a sampling chamber that is configured to hold a member made of an absorptive material (*e.g.,* an absorptive pad and/or and absorptive sponge) having absorbed therein a composition; (2) transferring a fluid sample from the GI tract of the subject into the sampling chamber of said ingestible device *in vivo;* (3) fully or partially saturating the absorptive material held in the sampling chamber of the ingestible device with the fluid sample; (4) irradiating the absorptive material held in the sampling chamber of the ingestible device with light to excite the photosensitizer; (5) measuring total luminescence or rate of change of luminescence emitted from the composition held in the sampling chamber of the ingestible device as a function of time; (6) correlating the total luminescence or the rate of change of luminescence as a function of time measured in step (5) to the amount of the analyte in the fluid sample; and (7) correlating the amount of the analyte in the fluid sample to the number of microorganisms (e.g., pathogenic microorganisms) in the fluid sample. In some disclosures, the analyte is specific for a particular genus, species, or strain of microorganism. In some disclosures, a number of microorganisms determined in step (7) greater than a control number of microorganisms indicates a need for treatment (e.g., with an antibiotic agent described herein). In some disclosures, the control number of microorganisms is 0, 1, 10², 10³, 10⁴, 10⁵, 10⁶, 10⁷, 10⁸, 10⁹, or more. For example, in some disclosures, a number of microorganisms determined in step (7) greater that about 0 indicates a need for treatment. In some disclosures, a number of microorganisms determined in step (7) greater that about 1 indicates a need for treatment. In some disclosures, a number of microorganisms determined in step (7) greater that about 10² indicates a need for treatment. In some disclosures, a number of microorganisms determined in step (7) greater that about 10³ indicates a need for treatment. In some disclosures, a number of microorganisms determined in step (7) greater that about 10⁴ indicates a need for treatment. In some disclosures, a number of the microorganisms determined in step (7) greater than about 10⁵ indicates a need for treatment, e.g., with an antibiotic agent as described herein. In some disclosures, a number of microorganisms determined in step (7) greater that about 10⁶ indicates a need for treatment. In some disclosures, the control number of microorganisms is 0, 1, 10², 10³, 10⁴, 10⁵, 10⁶, 10⁷, 10⁸, 10⁹, or more CFU/mL. For example, in some disclosures, a number of microorganisms cells determined in step (7) greater that about 0 CFU/mL indicates a need for treatment. In some disclosures, a number of microorganisms determined in step (7) greater that about 1 CFU/mL indicates a need for treatment. In some disclosures, a number of microorganisms determined in step (7) greater that about 10² CFU/mL indicates a need for treatment. In some disclosures, a number of microorganisms determined in step (7) greater that about 10³ CFU/mL indicates a need for treatment. In some disclosures, a number of microorganisms determined in step (7) greater that about 10⁴ CFU/mL indicates a need for treatment. In some disclosures, a number of the microorganisms determined in step (7) greater than about 10⁵ CFU/mL indicates a need for treatment, e.g., with an antibiotic agent as described herein. In some disclosures, a number of microorganisms determined in step (7) greater that about 10⁶ or more CFU/mL indicates a need for treatment.

In some disclosures, the total luminescence or the rate of change of luminescence as a function of time of the sponge is measured over multiple time points for an extended period of time in step (4). For instance, in some disclosures, the total luminescence or rate of change of luminescence as a function of time of the sample is measured continuously for a period of 0-1800 minutes, 0-1600 minutes, 0-1500 minutes, 0-1440 minutes, 0-1320 minutes, 0-1000 minutes, 0-900 minutes, 0-800 minutes, 0-700 minutes, 0-600 minutes, 0-500 minutes, 0-400 minutes, 0-350 minutes, 0-330 minutes, 0-300 minutes, 0-270 minutes, or 0-220 minutes. In some disclosures, the total luminescence or the rate of change of luminescence as a function of time of said sample is measured continuously for a period of 0-330 minutes. In some disclosures, the method is performed *in vivo.* In some disclosures, the method includes communicating the results of the onboard assay(s) to an *ex vivo* receiver. In some disclosures, the ingestible device and the method may be further used to monitor the subject after the treatment (*e.g.,* with an antibiotic). In some disclosures, the ingestible device and the method may be used to assess the efficacy of the treatment. For example, efficacious treatment may be indicated by the decrease of the number of viable bacterial and/or archaeal cells in a sample from the GI tract of the subject post-treatment. Efficacy of the treatment may be evaluated by the rate of decrease of the number of viable bacterial and/or archaeal cells in a sample from the GI tract of the subject post-treatment. In some disclosures, the ingestible device and the method may be used to detect infection with antibiotic-resistant strains of bacteria and/or archaea in a subject. For instance, such infection may be indicated where the number of viable bacterial and/or archaeal cells or pathogens in a sample from the GI tract of the subject does not substantially decrease after antibiotic treatment or other treatments.

In some disclosures, the disclosure provides a method of measuring the presence, absence or amount of one or more analytes from one or more samples in the gastrointestinal tract. In some disclosures, the one or more analytes are measured multiple times, for example, at different time points or at different locations. In one embodiment, a single device measures one or more analytes or more time points or locations; thereby creating a "molecular map" of a physiological region. Measurements can be taken at any location in the gastrointestinal tract. For example, in one disclosure, analytes from samples from one or more of the duodenum, jejunum, ileum, ascending colon, transverse colon or descending colon can be measured to create a molecular map of the small and large intestine. In one disclosure, the sample is from the duodenum. In one disclosure, the sample is from the jejunum. In one disclosure, the sample is from the ileum. In one disclosure, the sample is from the ascending colon. In one disclosure, the sample is from the transverse colon. In one disclosure, the sample is from the descending colon.

In another disclosure, a series of measurements can be taken over a shorter distance of the gastrointestinal tract (e.g., the ileum) to create a higher resolution molecular map. In some disclosures, previous endoscopic imaging may identify a diseased area for molecular mapping. For example, a gastroenterologist may use imaging (e.g., an endoscope equipped with a camera) to identify the presence of Crohn's Disease in the ileum and cecum of a patient, and the methods and techniques of the present methods and devices herein may be used to measure inflammation-associated analytes in this diseased area of the patient. In a related embodiment, the inflammation-associated analytes, or any analyte, may be measured every one or more days to monitor disease flare-ups, or response to therapeutics.

In certain disclosures, the disclosure provides methods of generating a molecular map of the gastrointestinal (GI) tract. The methods can include: (1) providing an ingestible device of the present application for detecting an analyte; (2) transferring a fluid sample from the GI tract of the subject into the sampling chamber of said ingestible device *in vivo;* (3) irradiating the composition held in the sampling chamber of the ingestible device with light to excite the photosensitizer; (4) measuring total luminescence or rate of change of luminescence emitted from the composition held in the sampling chamber of the ingestible device as a function of time; (5) correlating the total luminescence or the rate of change of luminescence as a function of time measured in step (4) to the amount of the analyte in the fluid sample; and (6) correlating the amount of the analyte in the fluid sample to a marker of disease in the fluid sample.

The presence or amount of one or more markers of disease indicates a need for treatment, e.g., with an anti-inflammatory agent as described herein. Markers of disease are described herein and include, but are not limited, inflammatory markers like cytokines and chemokines. In one example, TNFα, calprotectin and C-reactive protein (CRP) are measured at multiple locations of the colon (ascending, transverse, descending) to identify "hot spots" in a subject suspected of suffering from ulcerative colitis. In some disclosures, the same ingestible device, or one administered subsequently, is also able to deliver a therapeutic agent at the site of disease as described in the above embodiment. Means for delivering a therapeutic agent are described in US Provisional Application Nos. 62/385,553 and 62/478,955, filed September 9, 2016 and March 30, 2017, respectively.

In some disclosures, the method described herein further includes a step of calibrating the ingestible device, e.g., by using OMNI beads as discussed herein.

In some disclosures, methods as described herein are highly sensitive in detecting and quantifying an analyte (e.g., viable bacterial cells) in various samples. Where the analyte is viable bacterial and/or archaeal cells, in some disclosures, the lowest detection or quantification limit of the present methods is 10² CFU/mL. In some disclosures, the highest detection or quantification limit of the present methods is 10⁷ CFU/mL, 10⁸ CFU/mL, 10⁹ CFU/mL, 10¹⁰ CFU/mL or more. In some disclosures, the methods allow detection or quantification of 10² to 10⁷ CFU/mL bacterial and/or archaeal cells in various samples. In some disclosures, methods of this application may be used to distinguish samples bases on the quantity of viable bacterial and/or archaeal cells contained therein. For instance, the methods may be used to distinguish among samples that contain 10², 10³, 10⁴, 10⁵, 10⁶, or 10⁷ CFU/mL of bacterial and/or archaeal cells. The sensitivity of the assay is similar to that of the live cell assay.

In some disclosures, the compositions, methods and devices described herein may be used to determine the types of microorganisms (e.g., bacteria) present in a sample (e.g., for diagnostic purposes). Microorganisms (e.g., pathogenic microorganisms or commensal microorganisms) that may be detected using the compositions, methods, and devices described herein include bacteria, archaea, protozoans, viruses, and fungi.

This disclosure provides methods for detecting the presence of an analyte in a sample, wherein the analyte is a microorganism of interest (e.g., a microorganism of a particular genus, species and/or strain). In some disclosures, the microorganism of interest is a pathogenic microorganism. In some disclosures, the microorganism of interest is a commensal microorganism. Microorganisms of interest may be specifically detected using an analyte-binding agent described herein that specifically binds to the microorganism of interest (or to a molecule thereof (e.g., a protein or nucleic acid)). One of ordinary skill will understand that more than one genus, species, and/or strain of microorganism may be detected using the methods described herein by, for example, by utilizing analyte-binding agents (e.g., antibodies) that specifically bind to antigens (e.g., surface antigens) of the microorganisms that will be detected. Exemplary antibodies are described above.

In some disclosures, the methods may include: (a) providing a first composition to a subject comprising an analyte-binding agent that binds to a microorganism of interest (e.g., an antibody), wherein the analyte binding agent comprises a photosensitizer; (b) providing an ingestible device to the subject for detecting the analyte-binding agent in a sample; (c) transferring a sample of the subject into a sampling chamber of said ingestible device *in vivo;* (d) irradiating the composition held in the sampling chamber of the ingestible device with light to excite the photosensitizer of the analyte-binding agent; and (e) measuring total luminescence or rate of change of luminescence emitted from the composition held in the sampling chamber of the ingestible device as a function of time, thereby detecting the microorganism of interest in the sample. The first composition may be provided to the subject before, after, or concurrently with the ingestible device.

In some disclosures, the methods may include: (a) providing an ingestible device for detecting a microorganism of interest in a sample to a subject; (b) transferring the sample of the subject into a sampling chamber of said ingestible device *in vivo,* wherein the sampling chamber comprises an analyte-binding agent that binds to the microorganism of interest (*e.g.,* an antibody), wherein the analyte binding agent comprises a photosensitizer; (c) irradiating the composition held in the sampling chamber of the ingestible device with light to excite the photosensitizer of the analyte-binding agent; and (d) measuring total luminescence or rate of change of luminescence emitted from the composition held in the sampling chamber of the ingestible device as a function of time, thereby detecting the microorganism of interest in the sample. The first composition may be provided to the subject before, after, or concurrently with the ingestible device.

In some disclosures, the methods may include providing an ingestible device for detecting a microorganism of interest in a sample to a subject, wherein the ingestible device includes a sampling chamber that is configured to hold a composition including: (1) a plurality of donor particles, each of the plurality of donor particles including a photosensitizer and having coupled thereto a first analyte-binding agent (e.g., antibody) that binds to the microorganism of interest, wherein the photosensitizer, in its excited state, is capable of generating singlet oxygen; and (2) a plurality of acceptor particles, each of the plurality of acceptor particles including a chemiluminescent compound and having coupled thereto a second analyte-binding agent (e.g., an antigen-binding agent) that binds to the microorganism of interest, wherein said chemiluminescent compound is capable of reacting with singlet oxygen to emit luminescence.

In some disclosures, this methods may include providing an ingestible device for detecting a microorganism of interest in a sample to a subject, wherein the ingestible device includes a sampling chamber that is configured to hold a member made of an absorptive material (e.g., an absorptive pad and/or and absorptive sponge) having absorbed therein a composition including: (1) a plurality of donor particles, each of the plurality of donor particles including a photosensitizer and having coupled thereto a first analyte-binding agent (e.g., an antibody) that binds to the microorganism of interest, wherein the photosensitizer, in its excited state, is capable of generating singlet oxygen; and (2) a plurality of acceptor particles, each of the plurality of acceptor particles including a chemiluminescent compound and having coupled thereto a second analyte-binding agent (e.g., an antigen-binding agent) that binds to the microorganism of interest, wherein said chemiluminescent compound is capable of reacting with singlet oxygen to emit luminescence. In some disclosures, the first and the second analyte-binding agents are antigen-binding agents (e.g., antibodies). In some disclosures, the first and the second antigen-binding agents bind to the same epitope of the analyte (e.g., a protein). In some disclosures, the first and the second antigen-binding agents bind to separate epitopes of the analyte (e.g., a protein) that spatially overlap. In some disclosures, the first and the second antigen-binding agents bind to the separate epitopes of the analyte (e.g., a protein) that do not spatially overlap.

In some disclosures, the methods further comprise quantitating the amount of microorganism (e.g., by using a flow cytometry system present in the ingestible device).In some disclosures, the compositions, methods and devices described herein are used to detect and/or quantitate a microorganism of interest in a first region of the GI tract of the subject.

In some disclosures, the methods described herein are used to detect and/or quantitate a microorganism of interest in the GI tract of a subject before, after, or during a course of treatment with an antibiotic, thereby allowing for a determination as to whether a particular microorganism of interest is susceptible or resistant to the antibiotic. For example, when a subject is treated with an antibiotic in order to reduce the population of bacteria and/or archaea of a particular genus, species and/or strain, the methods can be used to evaluate and/or monitor the effectiveness of the antibiotic treatment *in vivo.*

### EXAMPLES

### Example A: Activity of Antibiotics Against SIBO Bacteria

### I. Summary

The *in vitro* activity of various antibiotics can be determined for various SIBO organisms. The *in vitro* activity, along with other pharmacodynamic parameters characteristic of the individual antibiotics, will provide a framework for doses to be evaluated in animal models. Ertapenem, meropenem, ceftriaxone, and piperacillin-tazobactam are safe, have low systemic absorption when orally-dosed, and are active against SIBO organisms. A summary of these antimicrobials and known activity against SIBO organisms is found below in Table 1.

**Table 1. Summary of PK, safety and in vitro activity data for ertapenem, meropenem, ceftriaxone, and piperacillin-tazobactam**

| **Parameter** | **ertapenem** | **meropenem** | **ceftriaxone** | **piperacillin-tazobactam** |
|---|---|---|---|---|
| **Rat oral exposure (Dose)** | 0.39 µM*h at 10 mg/kg | ~4% | 0.9 µg*hr/mL at 50 mg/kg (200-fold lower than iv) | NA |
| **Current Dosing** | IV: 500 mg every 8 hrs (skin & skin structure infections); 1 gram every 8 hrs (intra-abdominal infections) | IV: 500 mg every 8 hrs (skin & .skin structure infections); 1 gram every 8 hrs (intra-abdominal infections) | 1 g to 2 g, once per day or in equally divided doses twice per day, not to exceed 4g | Usual dose is 3.375 g every six hours, for a total of 13.5 g (12 g piperacillin/ 1.5 g tazobactam). For nosocomial pneumonia, dose is 4.5 g every six hours for a total of 18 g (16 g piperacillin/ 2 g tazobactam |
| **Safety** | | A threshold concentration of >64.2 mg/L indicated a 50% risk for developing a neurotoxicity event; and >44.45 mg/L indicated a 50% risk for developing nephrotoxicity | At a dose of 1 g BID iv, no serious adverse events reported, though transient neutropenia and thrombocytosis were reported by another group. The mean serum trough level of ceftriaxone was 42.1 µg/mL | |

| ***In vitro* Activity Against SIBO Bacteria** | | | | |
|---|---|---|---|---|
| | *Streptococcus agalactiae* | *Streptococcus agalactiae* | *Streptococcus agalactiae* | *Streptococcus agalactiae* |
| | *Streptococcus pneumoniae* (PSSP) | *Streptococcus pneumoniae* (PSSP) | *Streptococcus pneumoniae* | *Streptococcus pneumoniae* **(**PISP & PSSP) |
| | | | *Streptococcus pyogenes* | *Streptococcus pyogenes* |
| | *Escherichia coli* | *Escherichia coli* | *Escherichia coli* | *Escherichia coli* |
| | *Haemophilus influenzae* (*bla⁻* only) | *Haemophilus influenzae* (*bla⁻* only) | *Haemophilus influenzae* | *Haemophilus influenzae* (*bla⁻* only) |
| | | | *Haemophilus parainfluenzae* | *Klebsiella pneumoniae* |
| | *Klebsiella pneumoniae* | *Klebsiella pneumoniae* | *Klebsiella pneumoniae* | |
| | *Bacteroides fragilis* | *Bacteroides fragilis* | *Bacteroides fragilis* | *Bacteroides fragilis* |
| | *Bacteroides distasonis* | *Bacteroides distasonis* | | *Bacteroides distasonis* |
| | *Bacteroides ovatus* | *Bacteroides ovatus* | | |
| | *Bacteroides thetaiotaomicron* | *Bacteroides thetaiotaomicron* | | *Bacteroides thetaiotaomicron* |
| | *Bacteroides uniformis* | *Bacteroides uniformis* | | |
| | *Bacteroides vulgatus* | *Bacteroides vulgatus* | | |
| | | *Bacteroides urolyticus* | | |
| | *Fusobacterium* spp | *Fusobacterium* spp. | | |
| | *Prevotella bivia* | *Prevotella bivia* | *Prevotella bivia* | |
| | | *Prevotella intermedia* | *Porphyromonas (Bacteroides) melanogenicus* | |
| | | *Prevotella melanogenica* | | *Prevotella melanogenica* |
| | *Citrobacter freundii* | *Citrobacter freundii* | *Citrobacter freundii* | |
| | *Citrobacter koseri* | *Citrobacter diversus* | *Citrobacter diversus* | *Citrobacter koseri* |

Table 2 shows a more comprehensive summary of literature for activity of meropenem, a broad-spectrum agent that has FDA approval for treating a variety of different infections cause by many different bacterial pathogens, and ceftriaxone. Rifaximin can be a comparator for activity and is not approved for many infectious indications and therefore is not studied as much as meropenem. Table 2 is organized by organism or organism group, source of the isolates, MIC range, MIC₅₀ (concentration of drug required to inhibit the growth of 50% of the isolates), MIC₉₀ (concentration of drug required to inhibit the growth of 90% of the isolates), and percent susceptible. The latter category refers to the percentage of isolates that would be deemed "susceptible" or "clinically treatable with meropenem or the comparator rifaximin. Table 2 shows that meropenem is studied more often against the organisms of SIBO compared to rifaximin.

**Table 2: In vitro Activity of meropenem, ceftriaxone and rifaximin against SIBO organisms. No data entered means relevant MIC information is not available from the literature.**

| **SIBO Organisms (No. of isolates)** | **Source of Isolates** | **Compound** | **MIC Range (ug/mL** | **MIC₅₀ (ug/mL)** | **MIC₉₀ (ug/mL)** | **% S¹** | **Reference** |
|---|---|---|---|---|---|---|---|
| *Staphylococcus aureus* (12) | SIBO | rifaximin | ≤0.25 | ≤0.25 | ≤0.25 | 100 | Pistiki et al. 2014, Int J Antimicrob Agents. 43: 236-241 |
| *Staphylococcus aureus* (8,619) 53.6% MRSA | 2008-10 USA Hospitals | meropenem | ≤0.12 - >8 | ≤0.12 | 4 | | Jones et al. 2013, Diag Micro Inf Dis. 75:89-93 |
| *Staphylococci* (73) | Rifaximin treatment (37), placebo treatment (36), both baseline isolates | rifaximin | ≤0.06 - 64 | ≤0.06 | 64 | | Pimentel et al. 2017, Dig Dis Sci. 62:2455-2463 |
| *Staphylococcus aureus* (4426) | Bloodstream infections, US medical centers | ceftriaxone | 0.5 - >8 | 4 | >8 | 54.5 | Sader, et al. 2015, Antimicrob Chemother 70 (7): 2053 - 2056 |
| *Streptococcus mutans* | | | | | | | |
| *Streptococcus agalactiae* (15) | | ceftriaxone | 0.012 -0.1 | 0.025 | 0.1 | | Neu, et al. 1981, Antimicrob Agents Chemother. 19(3): 414 - 423 |
| *Enterococcus faecalis* (11) | SIBO | rifaximin | ≤0.25-16 | 4 | 16 | 100 | Pistiki *et al.* 2014 |
| *Enterococcus spp.* (73) | Rifaximin treatment (37), placebo treatment (36), both baseline isolates | rifaximin | ≤0.06-32 | 4 | 32 | | Pimentel *et al.* 2017 |
| *Enterobacteriaceae* (20,457) | 2006-2012 USA Hospitals | meropenem | ≤0.12 - >8 | ≤0.12 | ≤0.12 | 98.3 | Sader *et al.* 2014 |
| | | ceftriaxone | ≤1 - >16 | ≤1 | 8 | 87.5 | |
| ***MDR Enterobacteriaceae (1,359)*** | 2006-2012 USA Hospitals | meropenem | ≤0.12 - >8 | ≤0.12 | >8 | 75 | Sader *et al.* 2014 |
| *(Citrobacter spp. (29 strains), Enterobacter spp. (246 strains), E. coli (259 strains), Klebsiella oxytoca (45 strains), K. pneumoniae (464 strains), Morganella morganii (76 strains), Proteus mirabilis (104 strains), Providencia rettgeri (7 strains), Providencia stuartii (35 strains), Serratia marcescens (68 strains), and other species (26 strains).* | | | | | | | |
| ***XDR Enterobacteriaceae* (224)** | 2006-2012 USA Hospitals | meropenem | ≤0.12 - >8 | 8 | >8 | 20.5 | Sader *et al.* 2014 |
| *Citrobacter freundii (4 strains), Enterobacter cloacae complex (36 strains), E. coli (7 strains), K. pneumoniae (143 strains), M. morganii (13 strains), P. mirabilis (5 strains), P. stuartii (2 strains), S. marcescens (11 strains), and other species (3 strains).* | | | | | | | |
| *Enterobacteriaceae* (34,062) | 2012-14 non-USA isolates, all infections | meropenem | ≤0.004 - >8 | 0.03 | 0.12 | 97.2 | Karlowsky et al. 2016, Antimicrob Agent Chemother. 60:2849-2857 |
| *Enterobacteriaceae* (73) | Rifaximin treatment (37), placebo treatment (36), both baseline isolates | rifaximin | 4 - >128 | 32 | >128 | | Pimentel *et al.* 2017 |
| *Escherichia coli* (59) | 2000-2016 non SIBO | meropenem | ≤0.06->64 | ≤0.06 | 32 | 83.1 | Blais *et al.* 2018, doi: 10.1128/AAC.00552-18 |
| *Escherichia coli* (48) | SIBO | rifaximin | 4-64 | 16 | 64 | 85.4 | Pistiki *et al.* 2014 |
| *Escherichia coli* (443) | 2005-09 worldwide 80% stool. 20% blood isolates | rifaximin | 2 - >512 | 32 | 128 | | Farrell et al. 2011, Antimicrob Agent Chemother. 55:992-996 |
| *Escherichia coli* (2,866) | NYC hospital isolates | ceftriaxone | ≤0.25- >64 | ≤0.25 | 32 | 87 | Abdallah, et al., 2014, Antimicrob Agents Chemother. 59(3): 1802 - 1805 |
| ***Enterobacter spp.* (40)** | 2000-2016 non SIBO | meropenem | ≤0.06->64 | 0.5 | 64 | 57.5 | Blais *et al.* 2018 |
| *E. aerogenes* (4), *E. cloacae* (35), *E. hormaechei* (1) | | | | | | | |
| *Enterobacter spp.* (23) | SIBO | rifaximin | 32-128 | 64 | 128 | 34.8 | Pistiki *et al.* 2014 |
| *Lactobacillus spp. (3)* | Clinical isolates | ceftriaxone | 0.12 - 64 | 4 | 8 | | Rolfe & Finegold, 1982, Antimicrob Agents Chemother. 22(2): 338 - 341 |
| *Klebsiella pneumoniae* (81) | 2000-2016 non SIBO | meropenem | ≤0.06->64 | 8 | 64 | 38.3 | Blais *et al.* 2018 |
| *Klebsiella pneumoniae* (944) | NYC hospital isolates | ceftriaxone | ≤0.25 - >64 | ≤0.25 | >64 | 67 | Abdallah, *et al.*, 2014 |
| *Klebsiella spp.* (594) | 2013-15 non SIBO | meropenem | | ≤0.06 | >8 | 78.3 | Pfaller et al. 2017, Braz Inf Dis 21:627-637 |
| *Klebsiella spp.* (39) | SIBO | rifaximin | 8-128 | 64 | 128 | 43.6 | Pistiki *et al.* 2014 |
| ***Citrobacter spp.* (22)** | 2000-2016 non SIBO | meropenem | ≤0.06->64 | ≤0.06 | 4 | 86.4 | Blais *et al.* 2018 |
| *C. freundii (21), C. koseri (1)* | | | | | | | |
| ***Citrobacter spp.* (1,889)** | 2012-14 non-USA isolates, all infections | meropenem | ≤0.004 - >8 | 0.03 | 0.06 | 99 | Karlowsky *et al.* 2016 |
| *C. freundii (1,033 isolates), C. koseri (707), C. braakii (109), C. amalonaticus (21), C. murliniae (5), nonspeciated Citrobacter spp. (4), C. farmeri (4), C. sedlakii (3), C. gillenii (1), C. diversus (1), and C. youngae (1)* | | | | | | | |
| *Proteus mirabilis* (22) | 2000-2016 non SIBO | meropenem | 0.12-16 | 0.12 | 0.25 | 95.5 | Blais *et al.* 2018 |
| *Proteus mirabilis* (2,235) | 2012-14 non-USA isolates, all infections | meropenem | ≤0.004 - >8 | 0.06 | 0.12 | 99.4 | Karlowsky *et al.* 2016 |
| *Pseudomonas aeruginosa* (537) | 2013-15 non SIBO | meropenem | | 1 | >8 | 64.2 | Pfaller *et al.* 2017 |
| *Haemophilus influenzae* (15) | 2013-14 China non SIBO | meropenem | | ≤0.125 | 0.5 | | Yao et al. 2016, Molecules. 21:2-14 |
| *Bacteroides fragilis* (36) | 2009-2011 intra-abdominal infections | meropenem | 0.125 - >32 | 0.25 | 4 | 94.5 | Goldstein *et al. 2013, Antimicrob Agent* |
| | | | | | | | Chemother. 57:2620-2630 |
| *Bacteroides fragilis* (20) | bowel flora unknown age | rifaximin | 0.25 - >1024 | 0.25 | >1024 | | Finegold et al. 2009, Antimicrob Agent Chemother. 53:281-286 |
| *Bacteroides fragilis* (81) | Clinical isolates | ceftriaxone | 8 - >32 | 32 | >32 | 15 | Wexler et al., 2005, Antimicrob Agents Chemother. 44(8):2222 - 2224 |
| *Bacteroides spp* (92) | Rifaximin treatment (47), placebo treatment (45), both baseline isolates | rifaximin | 0.12 - >1024 | >1024 | >1024 | | Pimentel *et al.* 2017 |
| *Bacteroides spp.* (29) | Clinical isolates, 2008 | ceftriaxone | 1 - > 128 | >128 | >128 | | Dubreuil, et al., 2012, Int JAntimicrob Agents. 39:500 - 504 |
| *Bacteroides distasonis* | | | | | | | |
| *Bacteroides ovatus* (11) | bowel flora unknown age | rifaximin | 0.25 - >1024 | 1 | 1 | | Finegold *et al.* 2009 |
| *Bacteroides thetaiotaomicron* (18) | 2009-2011 intra-abdominal infections | meropenem | 0.125 - 1 | 0.25 | 0.5 | 100 | Goldstein *et al. 2013* |
| *Bacteroides thetaiotaomicron* (10) | bowel flora unknown age | rifaximin | 1 - > 1024 | 1 | >1024 | | Goldstein *et al. 2013* |
| *Bacteroides thetaiotaomicron* (42) | Clinical isolates | ceftriaxone | 16 - >32 | 32 | >32 | 2 | Wexler, *et al.*, 2005 |
| *Bacteroides uniformis* | | | | | | | |
| *Bacteroides vulgatus* (10) | 2009-2011 intra-abdominal infections | meropenem | 0.125 - 1 | 0.5 | 1 | 100 | Goldstein *et al. 2013* |
| *Bacteroides vulgatus* (11) | bowel flora unknown age | rifaximin | 0.25 - 4 | 0.25 | 0.5 | | Finegold *et al.* 2009 |
| *Bacteroides urolyticus* | | | | | | | |
| ***Fusobacterium* spp (42)** | 2009-2011 intra-abdominal infections | meropenem | ≤0.03 - 0.5 | 0.125 | 0.25 | 100 | Goldstein *et al. 2013* |
| *F. necrophorum* (10), *F. nucleatum* (11), *F. mortiferum* (10), *F. varium* (11) | | | | | | | |
| ***Fusobacterium* spp (34)** | bowel flora unknown age | rifaximin | 0.25 - >1024 | 8 | >1024 | | Finegold *et al.* 2009 |
| *F. nucleatum (10), F. mortiferum (6), F. necrophorum (11), Fusobacterium varium (5), and Fusobacterium species (2)*. | | | | | | | |
| *Fusobacterium nucleatum* (22) | Clinical isolates | ceftriaxone | 0.015 - 1 | 0.125 | 0.5 | 100 | Citron, et al., 2010, Antimicrob Agents Chemother. 54(4):1627 - 1632 |
| *Fusobacterium spp* (15) | Clinical isolates | ceftriaxone | 0.06 - >32 | 0.5 | 32 | 87 | Wexler *et al.*, 2005 |
| *Prevotella bivia* (10) | 2009-2011 | meropenem | ≤0.03 - 0.06 | 0.03 | ≤0.03 | 100 | Goldstein *et al. 2013* |
| | intra-abdominal infections | | | | | | |
| *Prevotella intermedia* | | | | | | | |
| *Prevotella melanogenica* (10) | 2009-2011 intra-abdominal infections | meropenem | ≤0.03-0.25 | 0.06 | 0.25 | 100 | Goldstein *et al. 2013* |
| ***Prevotella* spp. (31)** | bowel flora unknown age | rifaximin | 0.25 - 1 | 0.25 | 0.5 | | Finegold *et al.* 2009 |
| *P. bivia (5), P. corporis (4), P. disiens (6), P. intermedia*/*intermedia-nigrescens (5), P. loescheii (5), P. melaninogenica* (6). | | | | | | | |
| *Prevotella spp.* (20) | Clinical isolates | ceftriaxone | ≤0.008- 64 | 1 | 8 | 90 | Citron, *et al.* 2010 |
| *Clostridium sporogenes* | | NA | | | | | |
| *Clostridium spp.* (24) | Clinical isolates | ceftriaxone | 0.015- >64 | 2 | 641 | 75 | Citron, *et al.* 2010 |
| *Veillonella* (10) | 2009-2011 intra-abdominal infections | meropenem | ≤0.03-0.25 | ≤0.03 | ≤0.03 | 100 | Goldstein *et al. 2013* |
| *Veillonella spp (19)* | Clinical isolates | ceftriaxone | 0.03 - 8 | 4 | 8 | 79 | Citron, *et al.*, 2010 |

Using the MIC₉₀ as a guide, meropenem retains very good activity against many of the SIBO organisms. The Enterobacteriaceae is a larger group of gram-negative gut bacteria made up of *Escherichia coli, Klebsiella, Proteus, Enterobacter, Salmonella,* and *Morganella*, to name a few. A 20,457-isolate study by Sader et al. (MIC₉₀ ≤0.12 □g/mL) and a 34,062-isolate study by Karlowski et al. (MIC₉₀ 0.12 □g/mL) showed that meropenem was very potent against a wide variety of gram-negative pathogens. However, it is common for there to be meropenem-resistant isolates for each organism group, otherwise the percent susceptible would be 100. There are carbapenemase-producing bacteria within the Enterobacteriaceae group, and meropenem either would not be active at all, or huge doses would be required to inhibit growth and produce a recordable MIC. In sum, meropenem is active against SIBO organisms, and the data in Table 2 suggest that oral dosing could achieve levels of drug at or above the MIC₉₀ values for SIBO organisms. Where available from the literature, rifaximin MIC data are included in Table 2 for comparison. Though rifaximin is a broad-spectrum agent, MIC₉₀ values were often much higher than those for meropenem.

### II. Preclinical Study of Meropenem for the Treatment of SIBO

Rats and minipigs can be the species for preclinical/efficacy, as rats have been used as an animal model for IBS/SIBO and minipigs have similar GI systems to humans.
- Rat and minipig oral PK studies will be carried out with different doses of meropenem. Plasma and feces will be collected to demonstrate and compare exposure between these two samples even at high doses.
- Different meropenem formulations will be evaluated in rats and minipigs through monitoring alterations in stool consistency. In rats, drug and microbial levels in small intestinal fluid will be quantified following euthanasia.
- *In vitro* activity of meropenem will be determined against SIBO organisms:
   ∘ MIC studies
   ∘ Time-kill studies
   ∘ Resistance development studies

### III. Efficacy of Antimicrobial Formulations

The Pimentel (2008) *Campylobacter jejuni* rat model of SIBO will be used to evaluate and prioritize choice of antimicrobial formulations. Pimentel et al. 2008. A new rat model links two contemporary theories in irritable bowel syndrome. Dig Dis Sci. 53:982 - 989. Briefly, rats will be infected with *C. jejuni* and three months later it will be determined which animals have evidence of SIBO based on alterations in stool consistency and decreases in body weights. Said animals will be divided into different groups including a control group and dosed with the different antimicrobial formulations to monitor whether the animals' weights and stool consistencies normalize. This will be followed by bacterial quantitation of jejunal contents by PCR as described in Pimentel (2008). Further, resistance to meropenem developed *in vivo,* if any, will be monitored.

### IV. Clinical Study (to be conducted after formulation evaluation)

An oral PK study will be conducted with both fed and fasted humans. The dose of the antimicrobial will be in the range of from about one-quarter to about two times of a standard dose (for ertapenem, meropenem, ceftriaxone, and piperacillin-tazobactam see, e.g., Table 1).

Oral dosing will be performed with ¹⁴C-labeled meropenem or ¹⁴C-labeled ceftriaxone for recovery and analysis in urine and feces.

### Example B: Clinical study of an antimicrobial in treating SIBO

### I. Overview of study design

Participating subjects will be randomized in a 1: 1 ratio to receive either an antimicrobial (also referred to as the "study drug") or placebo. Subjects will receive study drug during a 2-4 week treatment phase, which will be followed by a 10-12 week post-treatment phase. The study drug dose regimen and treatment duration will be selected based on considerations from pharmacokinetic and scintigraphic study data, the published literature, consultations with thought leaders in SIBO, and results from *in vitro* and *in vivo* studies such as those described in Example A. The follow-up period can extended or shortened based on FDA recommendations for evaluating the study drug in SIBO.

### II. Subject Population

Patients or subjects selected for inclusion will have symptomology suggestive of SIBO. The current diagnostic criteria set by the FDA for SIBO is ≥10⁵ CFU/mL in a small intestinal fluid sample; however, this cutoff value is subject to change. For example, it may be lowered to ≥10⁴ or ≥10³ CFU/mL in a small intestinal fluid sample. To confirm bacterial levels in the small intestine, jejunal fluid can be sampled from the subject using a special sonde or via enteroscopy and directly cultured. Alternatively, an ingestible device may be used to detect the presence of bacterial growth in a dilution of a jejunal fluid sample. The ≥10⁵ CFU/mL may comprise any of the bacterial species associated with SIBO (see. e.g., the bacterial species listed in Table 2, or elsewhere in the specification). Depending on the study drug, the subject population may be further limited by the bacterial species detected in the jejunal fluid. For example, if the study drug is meropenem, only subjects having an overgrowth of bacterial species known to respond to meropenem per *in vitro and in vivo* preliminary studies will be included in the trial.

Alternatively, subjects can be selected based on a breath test for hydrogen and/or methane. To perform the breath hydrogen test, subjects will be required to undergo an overnight fast. At the conclusion of the fast (time zero), subjects will swallow 15 mL of Chronulac formula, delivering 10 g of lactulose. Every 5-20 min thereafter, for 2-4 hours, a 50 cm³ end-expiratory breath sample will be taken with an airtight sampling bag. Each breath sample will be analyzed for hydrogen content with a gas chromatograph, standardized as instructed by the manufacturer. Hydrogen peaks will be plotted before and after an antimicrobial treatment regimen for comparison.

### III. Study Objectives and Endpoints

Efficacy endpoints in the proposed study are based on subject responses to weekly questions regarding their SIBO symptoms (e.g., symptomology suggestive of SIBO) and responses to symptom-specific daily efficacy measures for SIBO symptoms such as bloating, diarrhea, flatulence, increased or decreased stool frequency, abdominal pain, constipation, weight loss, fever, abdominal tenderness, nausea, gastric stasis, and steatorrhea. Efficacy assessments will be collected over the full 12-16 weeks of subject observation.

The primary endpoint will be based on the proportion of subjects who achieve adequate relief of SIBO symptoms (*e.g.,* there is a decrease of the symptoms suggestive of SIBO). Adequate relief of SIBO symptoms can be defined as a response of "yes" to the following weekly SGA (subject global assessment): *"In regards to your SIBO symptoms, compared to the way you felt before you started study medication, have you*, *in the past 7 days, had adequate relief of your SIBO symptoms? [Yes*/*No]".* Secondary endpoints can be directed to other symptoms of SIBO, e.g., bloating, diarrhea, flatulence, stool frequency, abdominal pain, constipation, weight loss, fever, abdominal tenderness, nausea, gastric stasis, and steatorrhea. Agreement on these endpoints may be reached with the FDA.

In additions to subject responses, the presence of bacterial growth can be tested 1-2 times during the study (e.g., at the end of the 2-4 weeks of study drug administration and/or at the conclusion of the study (weeks 12-16)).

### Example C: Evaluation of in vitro activity of antimicrobials against SIBO organisms

In this study, the *in vitro* activity of meropenem and ceftriaxone in comparison to rifaximin was determined against a range of aerobic and anaerobic bacteria that have been implicated in SIBO. Minimal inhibitory concentration (MIC) values were determined by broth microdilution or agar dilution in accordance with guidelines from the Clinical and Laboratory Standards Institute (CLSI; (1) "Methods for Dilution Antimicrobial Susceptibility Tests for Bacteria That Grow Aerobically; Approved Standard; 11th ed." CLSI standard M07-A11, 2018; (2) "Performance Standards for Antimicrobial Susceptibility Testing; Twenty-Eighth Informational Supplement," CLSI document M100-S28, 2018; and (3) "Methods for Antimicrobial Susceptibility Testing of Anaerobic Bacteria; Approved Standard-Eighth Edition," CLSI document M11-A8, 2012).

### Materials and Methods

### Test Compounds

The test agents were provided by Micromyx (Kalamazoo, MI) and stored as directed by the manufacturer prior to use. Stock solutions were made of each test agent in the solvent recommended by CLSI (1) at 100X the desired highest concentration to be tested in the assay. All stock solutions were allowed to stand for at least 1 hr prior to use to auto-sterilize. Suppliers, catalog and lot numbers, solvents, and diluents were as follows:

| **Test Agents** | **Suppllier** | **Catalog No.** / **Lot Number** | **Solvent** / **Diluent** |
|---|---|---|---|
| Meropenem | USP | 1392454 / J0K434 | Water / Water |
| | | 1392455 / J0K435 | |
| Ceftriaxone | USP | 1098184 / R07420, G1D265 | Water / Water |
| Metronidazole | Sigma | M3761 / 095K0693 | DMSO / Water |
| Rifaximin | Sigma | 33999 / BCBT5109 | MeOH/Phosphate pH 7.4 + 0.45% SDS* |
| Moxifloxacin | ChemPacific | 35185 / CHCP062915MHV | Water / Water |
| Piperacillin | Sigma | P8396 / SLBD9564V | Water / Water |
| Tazobactam | USP | 1643383 / HOM200 | Water / Water |
| Vancomycin | Sigma | V2002 / 086M4003V | Water / Water |
| Penicillin | Sigma | P3032 / 071M0740V | Water / Water |
| Oxacillin | Sigma | 28221-5G / BCBF5635V | Water / Water |

| | | | |
|---|---|---|---|
| *SDS was not included in media for *Streptococcus* species | | | |

### Test Isolates

Test organisms consisted of clinical isolates from the Micromyx repository or reference strains acquired from the American Type Culture Collection (ATCC; Manassas, VA). They were initially received at Micromyx and streaked for isolation. Colonies were picked by sterile swab and suspended in the appropriate broth containing cryoprotectant. The suspensions were aliquoted into cryogenic vials and maintained at -80°C. Prior to testing, the isolates were streaked from frozen vials onto the appropriate agar. Tryptic soy agar (TSA) with 5% sheep blood was used for aerobes; anaerobes were grown on Brucella Agar with 5% sheep blood, hemin and vitamin K1. The streaked isolates were incubated under optimal conditions for growth. *Streptococcus* spp. were incubated in 3% CO₂ whereas anaerobes were incubated anaerobically. Quality control isolates were included during testing per CLSI guidelines (1)-(3).

Tested aerobically by broth microdilution:
- *Escherichia coli,* n = 50
- *Klebsiella* spp., n = 50
- *Pseudomonas aeruginosa,* n = 50
- *Streptococcus* spp., n = 50
- *Staphylococcus aureus,* n = 50
- *Enterococcus faecalis*, n = 50
- *Enterobacter aerogenes,* n = 50
- *Proteus mirabilis,* n = 50

Tested anaerobically by agar dilution:
- *Prevotella* spp., n = 30
- *Veillonella* spp., n = 30
- *Clostridium sporogenes,* n = 10
- *Bacteroides fragilis,* n = 30
- *Bacteroides* spp., n = 30 (incl. *B. vulgatus*)

### Test Media

Broth microdilution testing of aerobic bacteria was conducted in cation-adjusted Mueller Hinton broth (MHBII). For testing streptococci, lysed horse blood (LHB) was added to MHBII broth at a final concentration of 3%. Where LHB was used, it was added after filtering.

Details of media, supplements and components are summarized in the table below:

| **Media** | **Supplier** | **Cat. No.** | **Lot No.** |
|---|---|---|---|
| **Agar Growth Plates** | | | |
| Trypticase Soy Agar w/ 5% sheep blood (TSAII) | Becton Dickinson, Sparks, MD | 221261 | 8313526, 8306693, 8235998, 8256752 |
| Brucella Agar w/ 5% laked sheep blood, hemin, Vit K₁ | Becton Dickinson, Sparks, MD | 297716 | 8207613, 8264792 |

| **Broth MIC** | | | |
|---|---|---|---|
| Cation-adjusted Mueller Hinton broth (MHBII) | Becton Dickinson, Sparks, MD | 212322 | 8190586, 8096574, 7143896 |
| Brucella Broth | Becton Dickinson, Sparks, MD | 211088 | 7128995 |
| Lysed horse blood (LHB) | Hemostat, Dixon, CA | | 399694 |

| **Agar dilution** - **anaerobes** | | | |
|---|---|---|---|
| Brucella Agar | Becton Dickinson, Sparks, MD | 211086 | 7121860 |
| Hemin | Sigma, St. Louis, MO | H9039 | SLBP5720V |
| Vitamin K₁ | Sigma, St. Louis, MO | \/3501 | MKBN5958V |
| Laked sheen blood (LSB) | Hemostat, Dixon, CA | | 452421 |

### Susceptibility Testing

### Broth Microdilution MIC Assay

Broth microdilution susceptibility testing in 96-well microplates was conducted in accordance with CLSI methods (1) and (2). Automated liquid handlers were used to conduct serial dilutions and liquid transfers, including the Multidrop 384 (Labsystems, Helsinki, Finland), Biomek 2000, and the Biomek FX (Beckman Coulter, Fullerton, CA). All wells in columns 2 through 12 of a standard 96-well microdilution plate (Costar 3795) were filled with 150 µL of the appropriate diluent. Then, 300 µL of the test agents were added to the wells of column 1 of the plates at 100X the highest final concentration to be tested. Serial two-fold dilutions were made across the rows through column 11 using the Biomek 2000. The wells of column 12 contained no drug and served as the growth control wells. This plate served as the "mother plate" from which MIC assay plates or "daughter plates" were made.

The daughter plates were loaded with 190 µL per well of the appropriate medium using the Multidrop 384. The daughter plates were created using the Biomek FX which transferred 2 µL of drug solution from each well of a mother plate to each corresponding well of the daughter plate in a single step. A standardized inoculum of each test organism was prepared per CLSI methods (1) to equal a 0.5 McFarland standard, followed by a dilution of 1:20 for aerobes and 1:10 for anaerobes. The plates were then inoculated with 10 µL of the diluted inoculum using the Biomek 2000 from low to high drug concentration, resulting in a final concentration of approximately 5 x 10⁵ CFU/mL per well. An un-inoculated plate was incubated in parallel for each medium for the purpose of assessing solubility of the drug in the test media.

Inoculated plates were stacked 3 or 4 high, covered with a sterile lid on the top plate, placed into boxes with CO₂ planchets (streptococci) or plastic bags (all other isolates). Plates were incubated at 35 °C for 16-24 hr in ambient air for aerobes or for 42-48 hr in the anaerobic environment of the Bactron II anaerobe chamber (Sheldon Manufacturing Co.; Cornelius, OR). Following incubation, the microplates were removed from the incubator and viewed from the bottom using a plate viewer. The MIC values were read and recorded as the lowest concentration of drug that inhibited visible growth of the organism as per CLSI methods (1) and (2).

### Agar Dilution MIC Assay

MIC values for the anaerobic organisms were determined using an agar dilution method as recommended by the CLSI (3). The test agent was mixed with molten supplemented Brucella agar (50 - 55 °C) at a ratio of 0.5 mL of the 40X test agent to 19.5 mL agar containing 5% Laked Sheep Blood in a sterile tube, mixed gently, then poured manually into a sterile Petri plate. Plates were allowed to solidify at room temperature for approximately 1 hr. Plates containing no drug were inoculated as growth controls. The 0.5 McFarland standard suspensions were prepared in sterile saline, transferred to the wells of a stainless-steel replicator block, and stamped on agar plates containing the appropriate drug concentration. Following inoculation, the plates were incubated at 35 °C for 42-48 hr in the anaerobic environment of the Bactron II anaerobe chamber (Sheldon Manufacturing Co.; Cornelius, OR). The MIC was read per CLSI guidelines (1).

### Results

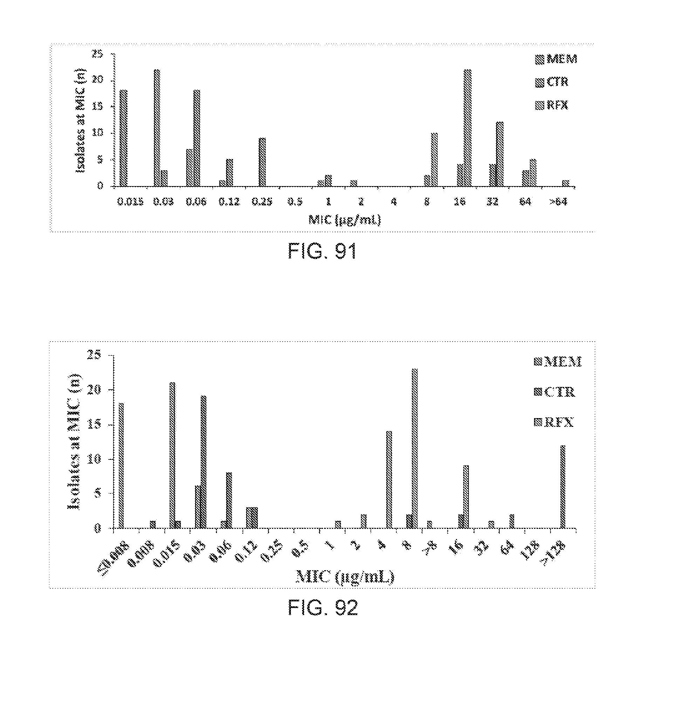

The *in vitro* activity of meropenem, ceftriaxone, rifaximin and comparators against organisms implicated in SIBO is displayed in Tables 3-34 below, where MEM = meropenem; CTR = ceftriaxone; RFX = rifaximin; MXF = moxifloxacin; and PTZ = piperacillin/tazobactam. These data are depicted graphically in FIGs. 91-106.

### Gram-negative aerobes

As shown in Table 3, against 50 *Enterobacter aerogenes* in this study, meropenem had an MIC₅₀ of 0.03 µg/mL and an MIC₉₀ of 0.06 µg/mL, with an MIC range of 0.015 to 2 µg/mL. Ninety-eight percent of the isolates in this panel were susceptible to meropenem, and 2% had an intermediate level of resistance. The MIC_{50/90} for ceftriaxone was 0.12/32 µg/mL, and the range was 0.03 to 64 µg/mL against this set of organisms, whereas for rifaximin the MIC₅₀ was 16 µg/mL, the MIC₉₀ 64 µg/mL, and the MIC range was 8 to >64 µg/mL. In this panel, 26% of isolates were ceftriaxone-resistant. Moxifloxacin and piperacillin-tazobactam were also tested against this panel, and meropenem was the most active drug evaluated. FIG. 91 displays these data graphically, and Table 4 shows the number and cumulative percentage of isolates at each MIC.

**Table 3. Summary of in vitro activity of five comparators against 50 Enterobacter aerogenes clinical isolates (µg/mL)**

| **Drug** | **MIC range** | **Mode** | **MIC₅₀** | **MIC₉₀** | **%S** | **%I** | **%R** |
|---|---|---|---|---|---|---|---|
| MEM | 0.015 - 2 | 0.03 | 0.03 | 0.06 | 98% | 2% | |
| CTR | 0.03 - 64 | 0.06 | 0.12 | 32 | 74% | | 26% |
| RFX | 8 - >64 | 16 | 16 | 64 | | | |
| MXF | 0.03 - 0.5 | 0.12 | 0.06 | 0.12 | 100%¹ | | |
| PTZ | 1 - >128 | 2 | 4 | 64 | 76% | 16% | 8% |

| | | | | | | | |
|---|---|---|---|---|---|---|---|
| ¹Susceptibility determined using FDA breakpoints | | | | | | | |

**Table 4. Summary of overall in vitro activity of five comparators against 50 Enterobacter aerogenes clinical isolates by number (top) and by cumulative percent (bottom)**

| **Drug** | **Number of test isolates at MIC (µg/mL)** | | | | | | | | | | | | | | | |
|---|---|---|---|---|---|---|---|---|---|---|---|---|---|---|---|---|
| | **0.015** | **0.03** | **0.06** | **0.12** | **0.25** | **0.5** | **1** | **2** | **4** | **8** | **16** | **32** | **64** | **>64** | **128** | **>128** |
| MEM | 18 | 22 | 7 | 1 | | | 1 | 1 | | | | | | | | |
| CTR | | 3 | 18 | 5 | 9 | | 2 | | | 2 | 4 | 4 | 3 | | | |
| RFX | | | | | | | | | | 10 | 22 | 12 | 5 | 1 | | |
| MXF | | 5 | 20 | 22 | 1 | 2 | | | | | | | | | | |
| PTZ | | | | | | | 2 | 17 | 12 | 6 | 1 | 6 | 2 | | 3 | 1 |

| **Drug** | **Cumulative percentage of isolates inhibited at MIC (µg/ml)** | | | | | | | | | | | | | | | |
|---|---|---|---|---|---|---|---|---|---|---|---|---|---|---|---|---|
| | **0.015** | **0.03** | **0.06** | **0.12** | **0.25** | **0.5** | **1** | **2** | **4** | **8** | **16** | **32** | **64** | **>64** | **128** | **>128** |
| MEM | 36.0 | 80.0 | 94.0 | 96.0 | | | 98.0 | 100.0 | | | | | | | | |
| CTR | | 6.0 | 42.0 | 52.0 | 70.0 | | 74.0 | | | 78.0 | 86.0 | 94.0 | 100.0 | | | |
| RFX | | | | | | | | | | 20.0 | 64.0 | 88.0 | 98.0 | 100.0 | | |
| MXF | | 10.0 | 50.0 | 94.0 | 96.0 | 100.0 | | | | | | | | | | |
| PTZ | | | | | | | 4.0 | 38.0 | 62.0 | 74.0 | 76.0 | 88.0 | 92.0 | | 98.0 | 100.0 |

Table 5 shows the results of evaluating the test agents against 50 *Escherichia coli* isolates, including those expressing extended spectrum beta-lactamase (ESBL) and a single New Delhi metallo-beta-lactamase (NDM-1) isolate. Meropenem demonstrated an MIC₅₀ of 0.015 µg/mL, an MIC₉₀ of 0.03 µg/mL, and an MIC range of ≤0.008 to >8 µg/mL. Ninety-eight percent of the isolates in this panel were susceptible to meropenem, and 2% were resistant. For ceftriaxone, the MIC₅₀ was 0.06 µg/mL, the MIC₉₀ was >128 µg/mL and the range was 0.008 to >128 µg/mL. Thirty-six percent of these isolates were resistant to ceftriaxone. The rifaximin MIC₅₀ against these isolates was 8 µg/mL, the MIC₉₀ was 16 µg/mL with an MIC range of 1 to 32 µg/mL. Moxifloxacin and piperacillin-tazobactam were also evaluated here, and meropenem was the most active drug tested. FIG. 92 displays these data graphically, and Table 6 shows the number and cumulative percentage of isolates at each MIC value.

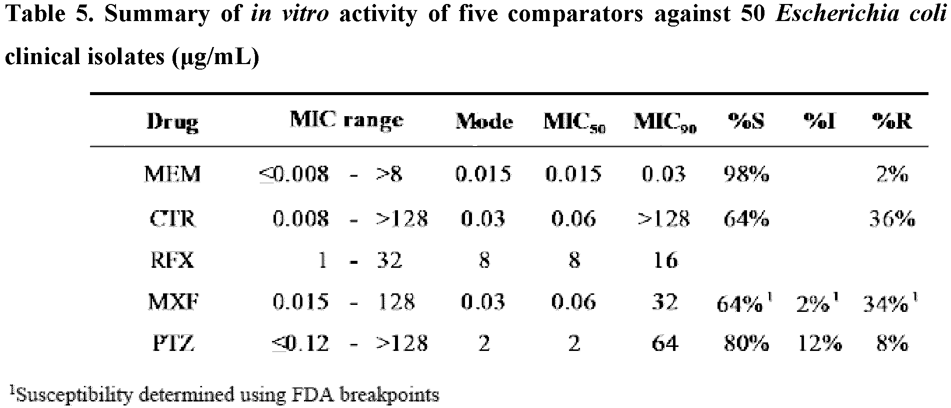

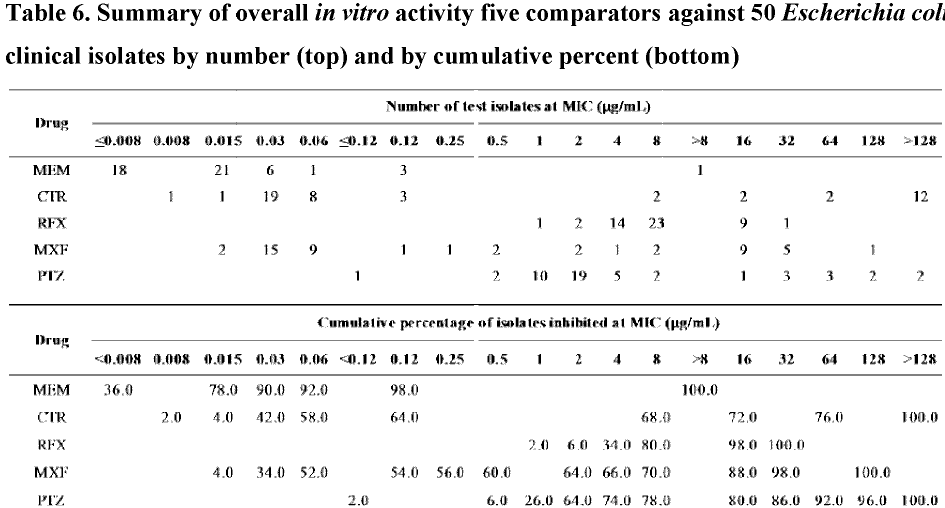

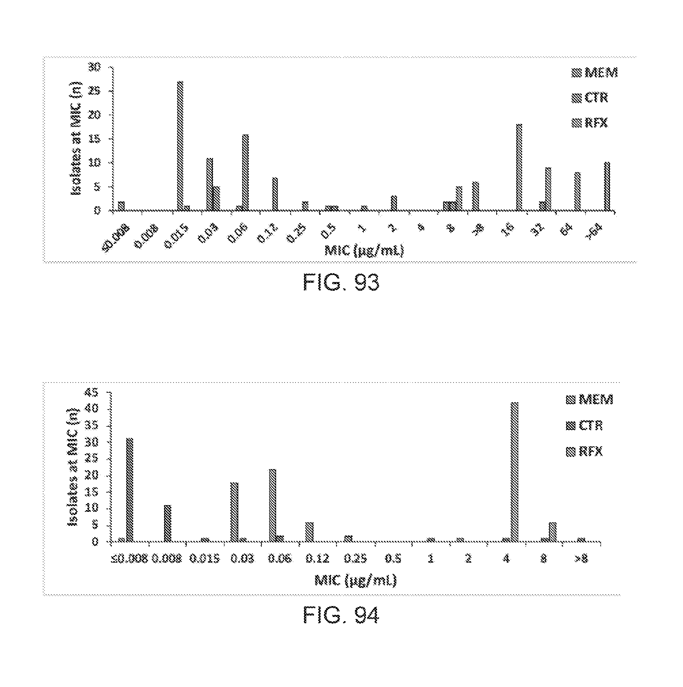

In this study there were 50 isolates of *Klebsiella* spp., 20 of which were *K. oxytoca* and the remaining 30 were *K. pneumoniae.* Against the 50 *Klebsiella* spp., meropenem had an MIC_{50/90} of 0.015/>8 µg/mL and an MIC range of ≤0.008 to >8 µg/mL (Table 7). Sixteen percent of these isolates were meropenem-resistant, and this was likely due to the presence of isolates expressing Klebsiella Pneumoniae Carbapenemase (KPC). For ceftriaxone, the MIC range was 0.015 to >128 µg/mL, and the MIC_{50/90} was 0.12/>128 µg/mL; 28% of the isolates in this panel were ceftriaxone-resistance, and 6% had intermediate resistance to this drug. The MIC₅₀ for rifaximin against these isolates was 32 µg/mL, and the MIC₉₀ was >64 µg/mL; the rifaximin range was 8 to >64 µg/mL. Moxifloxacin and piperacillin-tazobactam were also evaluated against these *Klebsiella* spp. isolates, and meropenem was the most active drug evaluated. Table 8 shows the number and cumulative percentage of isolates at each MIC, and FIG. 93 displays these data graphically.

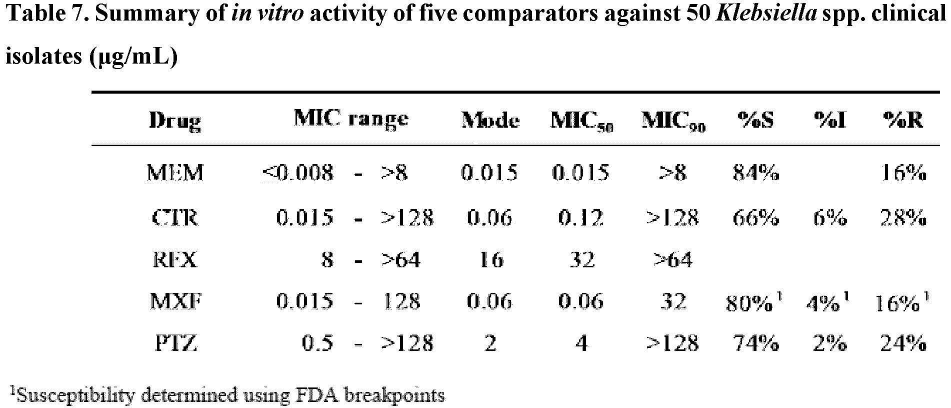

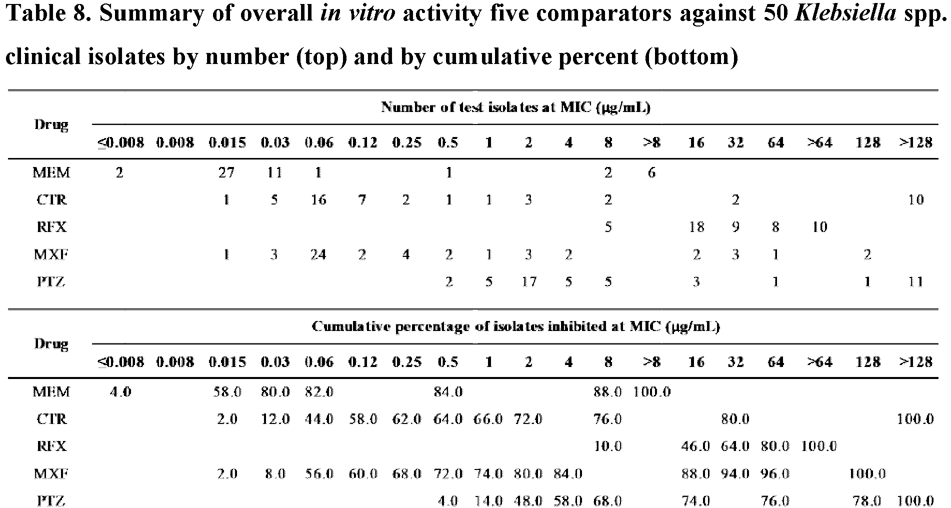

When evaluated against 50 isolates of *Proteus mirabilis,* meropenem had an MIC₅₀ of 0.06 µg/mL, an MIC₉₀ of 0.12 µg/mL, and an MIC range of ≤0.008 to 2 µg/mL (Table 9). Two percent of these isolates had intermediate resistance to meropenem. The MIC_{50/90} for ceftriaxone against these isolates was 0.004/0.06 µg/mL, and the range was ≤0.002 to >128 µg/mL. Six percent of these *P. mirabilis* isolates were ceftriaxone-resistant. For rifaximin, the MIC₅₀ was 4 µg/mL, the MIC₉₀ was 8 µg/mL, and the MIC range was 4 to >64 µg/mL. Moxifloxacin and piperacillin-tazobactam were comparators in this study, and ceftriaxone was the most active drug tested. FIG. 94 displays these data graphically, and Table 10 shows the number and cumulative percentage of isolates at each MIC.

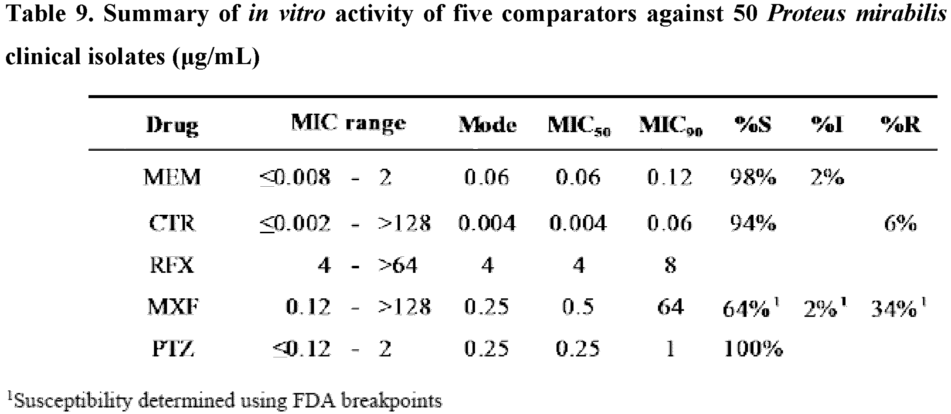

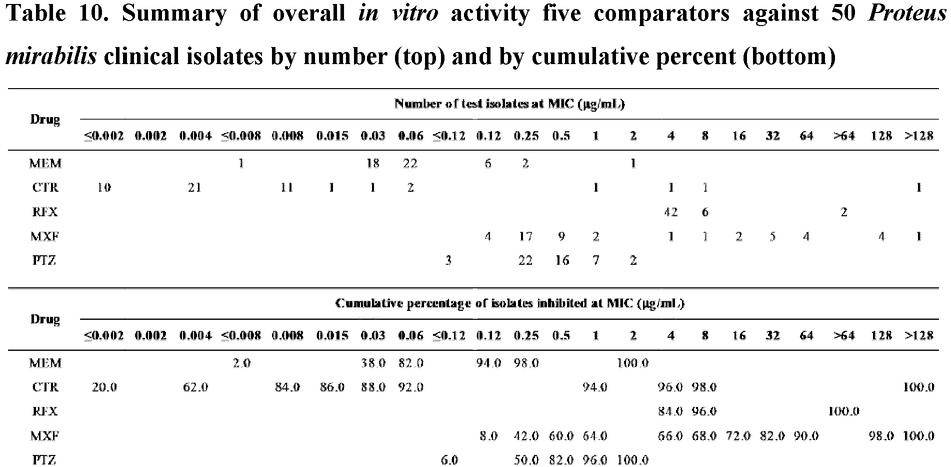

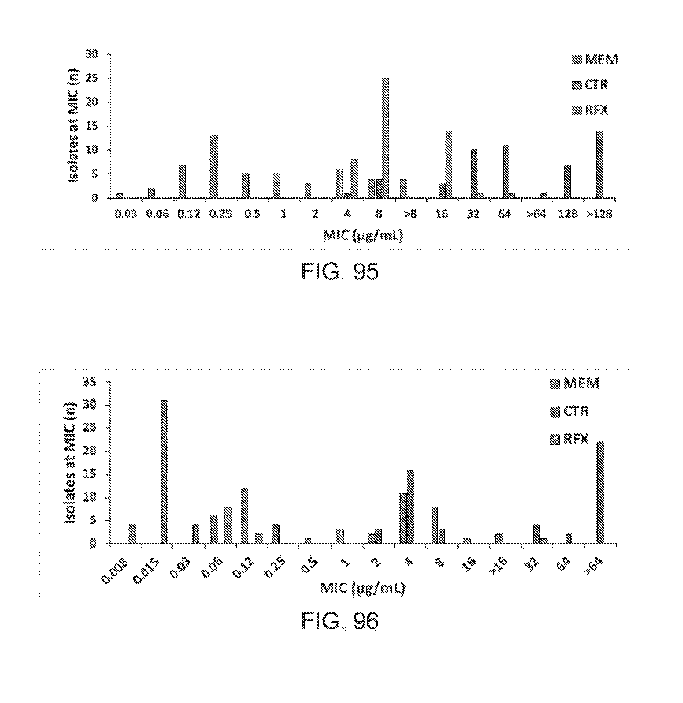

As shown in Table 11, against 50 *Pseudomonas aeruginosa* in this study, meropenem had an MIC₅₀ of 0.5 µg/mL and an MIC₉₀ of 8 µg/mL, with an MIC range of 0.03 to >8 µg/mL. Eighteen percent of the isolates in this panel were resistant to meropenem, and 10% had an intermediate level of resistance. The MIC_{50/90} for ceftriaxone was 64/>128 µg/mL, and the range was 4 to >128 µg/mL against this set of organisms, whereas for rifaximin the MIC₅₀ was 8 µg/mL, the MIC₉₀ 16 µg/mL, and the MIC range was 4 to >64 µg/mL. Moxifloxacin and piperacillin-tazobactam were comparators in this study, and meropenem was the most active drug evaluated. FIG. 95 displays these data graphically, and Table 12 shows the number and cumulative percentage of isolates at each MIC.

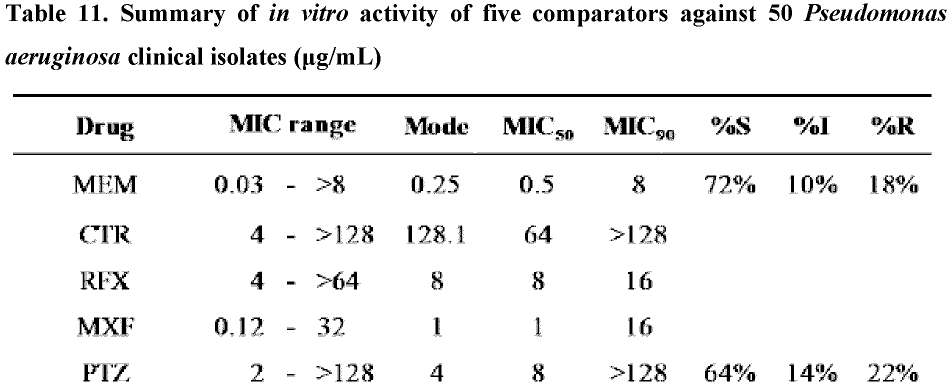

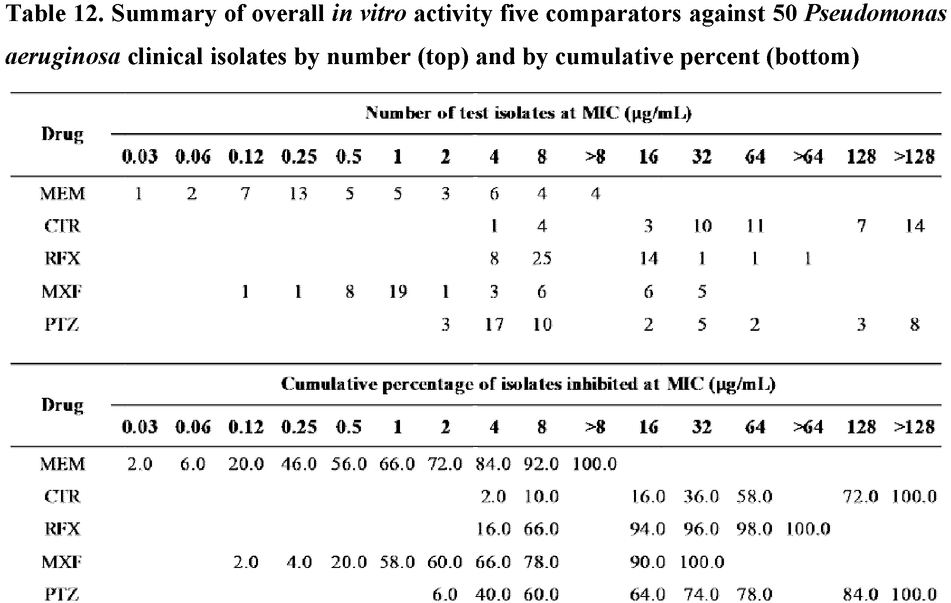

### Gram-positive aerobes

Table 13 shows the results of evaluating the test agents against 50 *Staphylococcus aureus* isolates, including 28 methicillin-resistant (MRSA) strains. Meropenem demonstrated an MIC₅₀ of 1 µg/mL, an MIC₉₀ of 8 µg/mL, and an MIC range of 0.06 - >16 µg/mL. For ceftriaxone, the MIC₅₀ was 32 µg/mL, the MIC₉₀ was >64 µg/mL and the range was 2 to >64 µg/mL. The presence of 28 MRSA isolates contributed to reduced susceptibility to meropenem, ceftriaxone, piperacillin-tazobactam, and oxacillin. The rifaximin MIC₅₀ against these isolates was 0.015 µg/mL, the MIC₉₀ was 0.06 µg/mL with an MIC range of 0.008 to 32 µg/mL. Moxifloxacin, piperacillin-tazobactam, vancomycin and oxacillin were comparators in this study, and rifaximin was the most active drug tested. FIG. 96 displays these data graphically, and Table 14 shows the number and cumulative percentage of isolates at each MIC value.

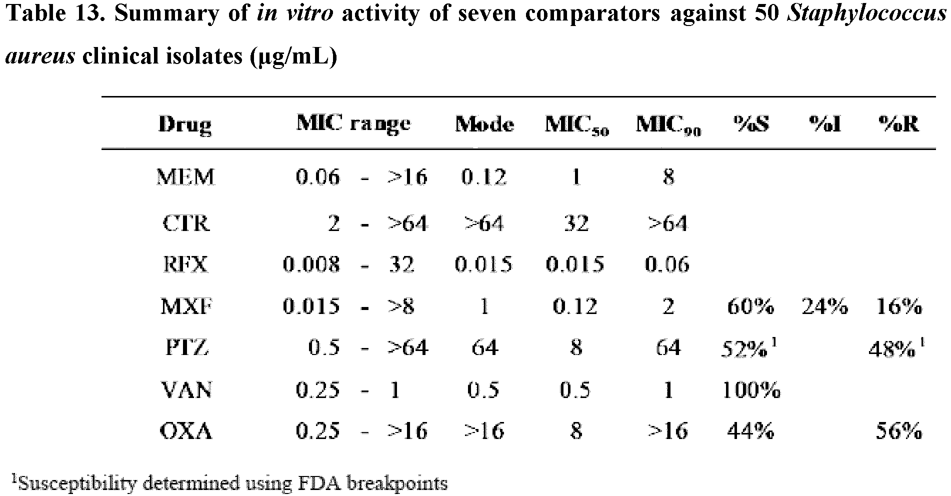

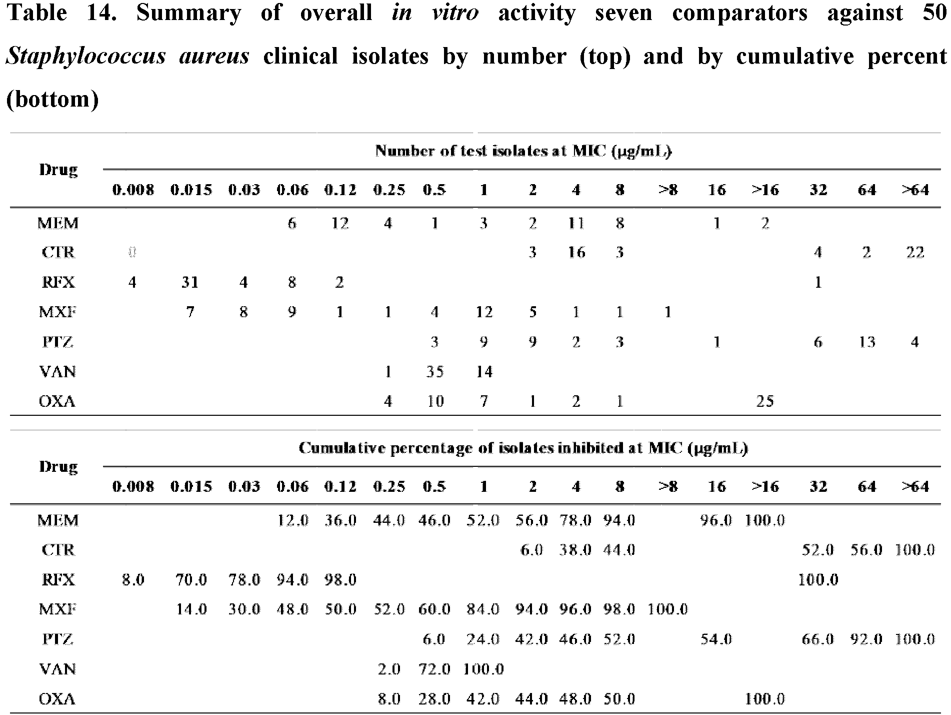

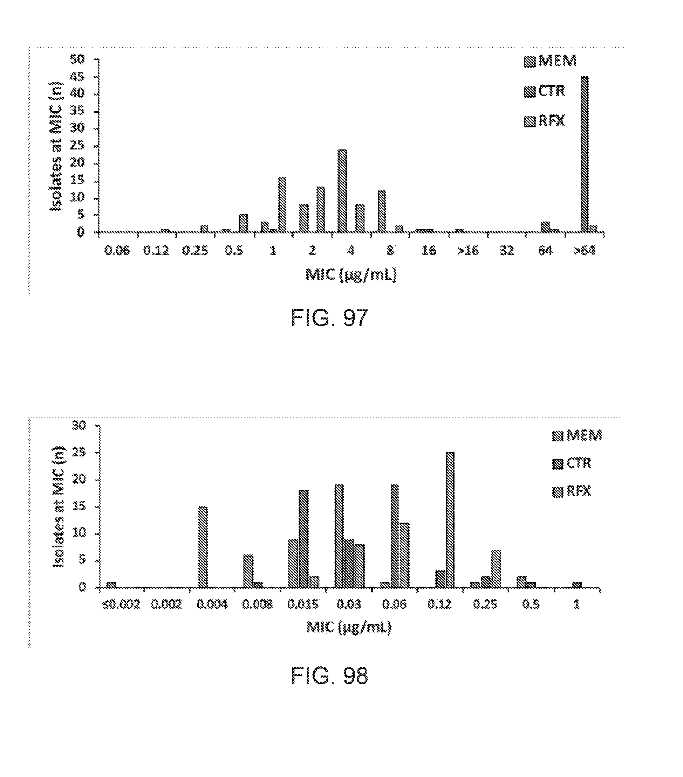

Against the 50 *Enterococcus faecalis* clinical isolates in this study, meropenem had an MIC_{50/90} of 4/8 µg/mL and an MIC range of 0.5 to >16 µg/mL (Table 15). For ceftriaxone, the MIC range was 1 to >64 µg/mL, and the MIC_{50/90} was >64/>64 µg/mL. The MIC₅₀ for rifaximin against these isolates was 2 µg/mL, and the MIC₉₀ was 4 µg/mL; the rifaximin range was 0.12 to >64 µg/mL. Overall, rifaximin was slightly more active than meropenem. Moxifloxacin, piperacillin-tazobactam, vancomycin and oxacillin were comparators in this study, and 26% of the isolates were vancomycin-resistant. Table 16 shows the number and cumulative percentage of isolates at each MIC, and FIG. 97 contains a graphical representation of these data.

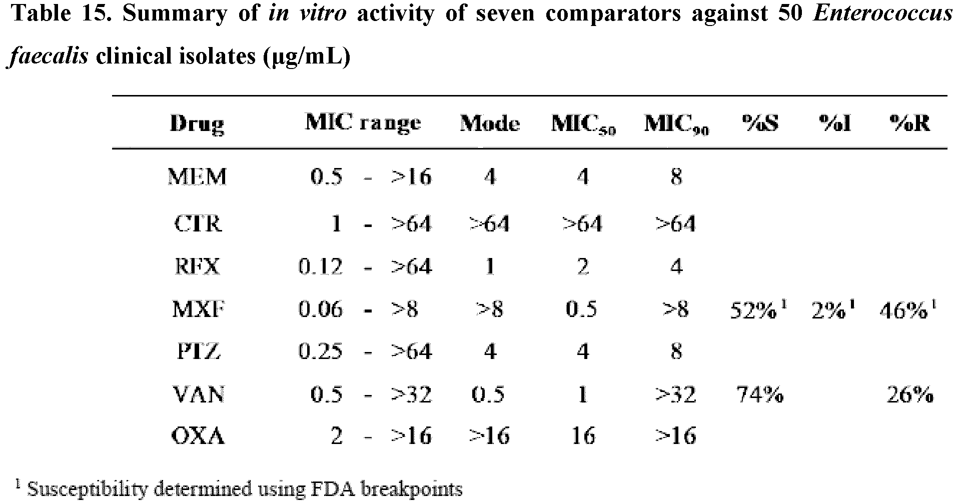

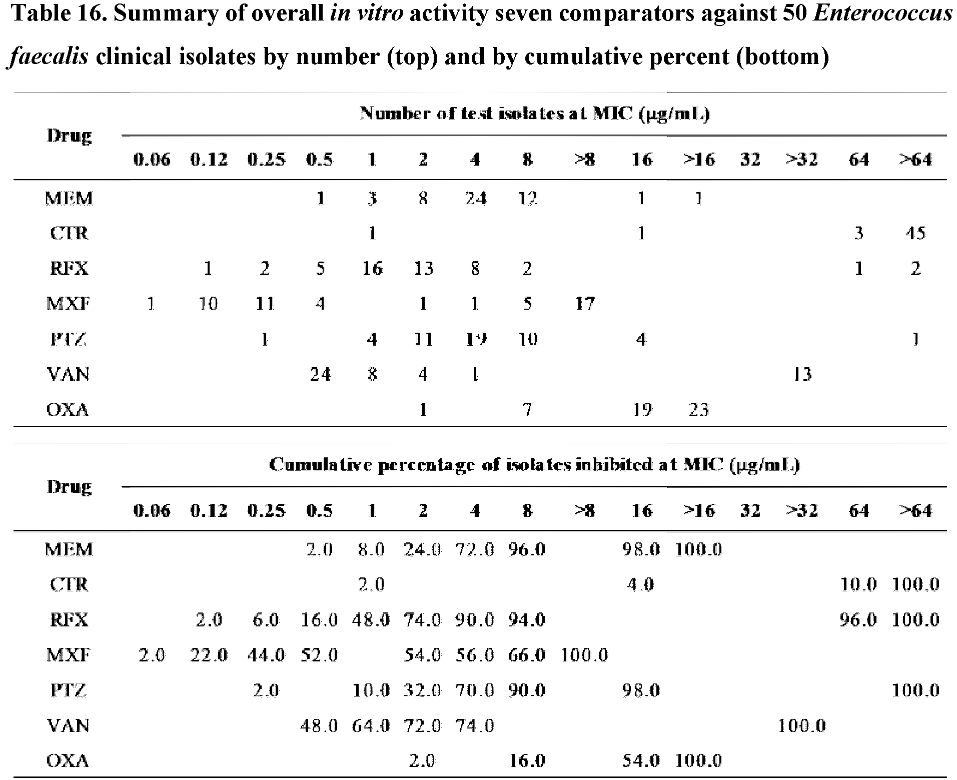

In this study, 54 *Streptococcus* spp. were tested, including 19 *S. pyogenes,* 20 *S. agalactiae* and 15 Viridans group *Streptococcus.* When evaluated against the 54 *Streptococcus* spp., meropenem had an MIC₅₀ of 0.015 µg/mL, an MIC₉₀ of 0.03 µg/mL, and an MIC range of ≤0.002 to 0.5 µg/mL (Table 17). All of these isolates were susceptible to meropenem. The MIC_{50/90} for ceftriaxone against these isolates was 0.03/0.12 µg/mL, and the range was 0.008 to 1 µg/mL, with 100% of isolates susceptible. For rifaximin, the MIC₅₀ was 0.12 µg/mL, the MIC₉₀ was 0.25 µg/mL, and the MIC range was 0.015 to 0.3 µg/mL. Moxifloxacin, piperacillin-tazobactam, vancomycin and oxacillin were comparators in this study, and meropenem was the most active drug evaluated. FIG. 98 displays these data graphically, and Table 18 shows the number and cumulative percentage of isolates at each MIC.

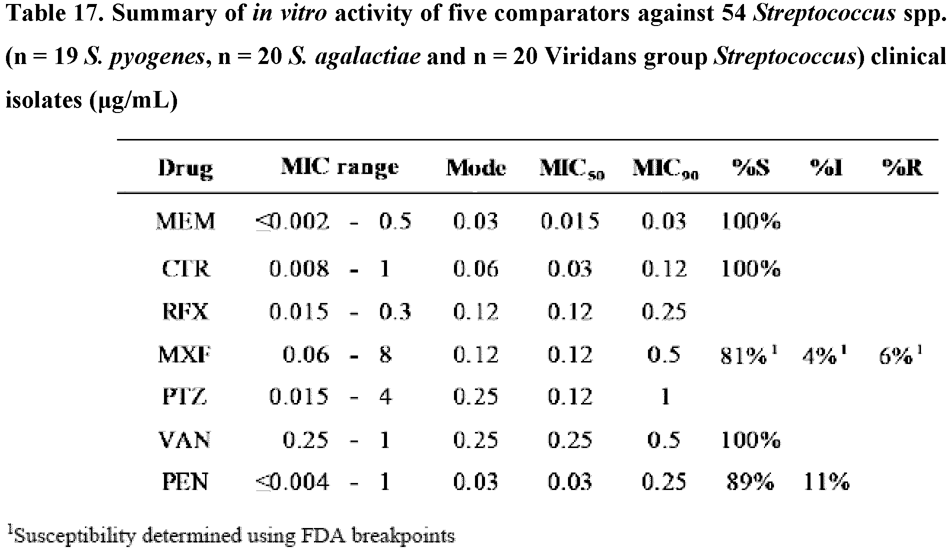

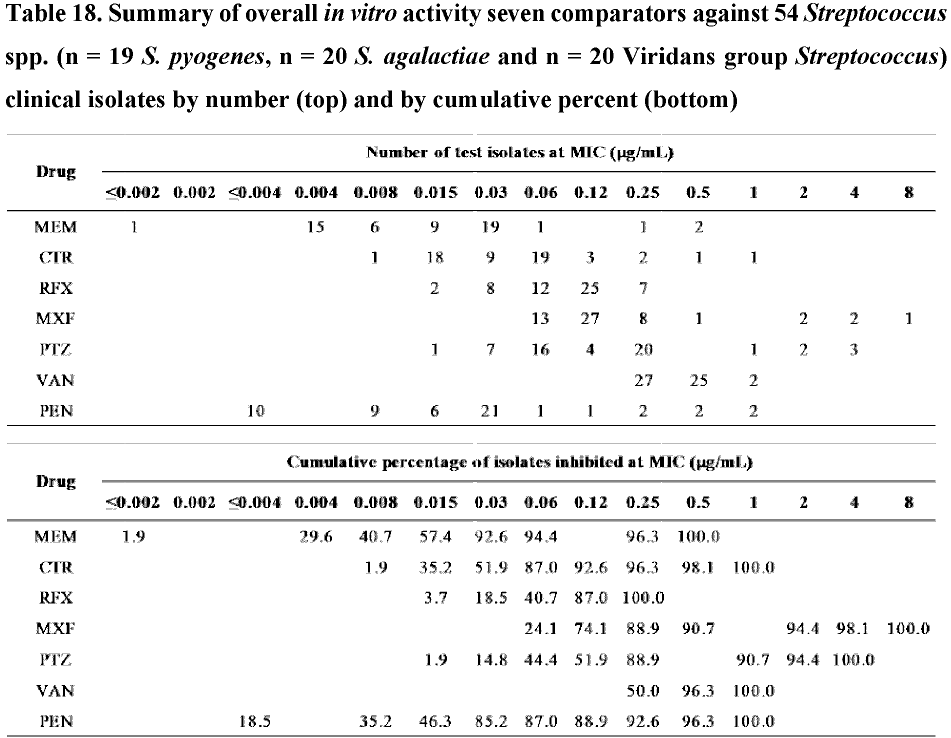

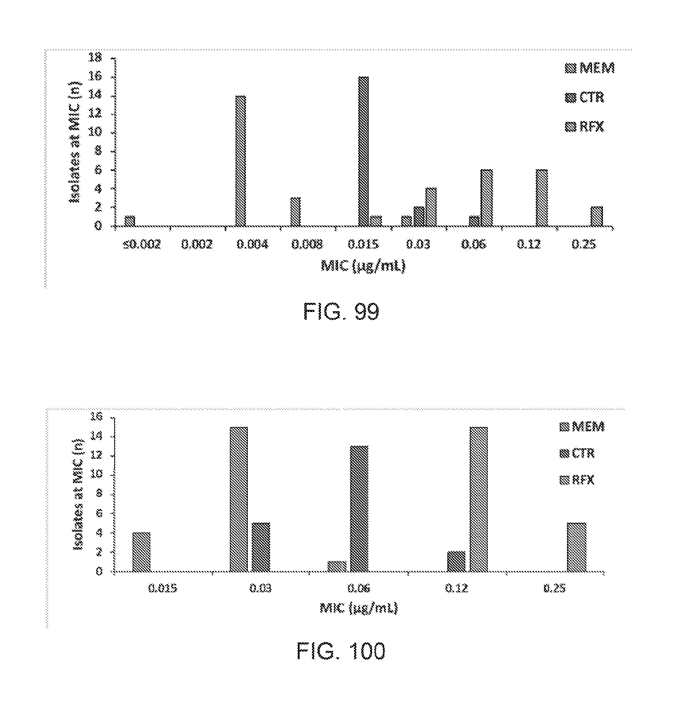

Against the 19 *S. pyogenes* tested, the meropenem MIC_{50/90} was 0.004/0.008 µg/mL, that for ceftriaxone was 0.015/0.03 µg/mL, and that for rifaximin was 0.06/0.25 µg/mL (Tables 19 and 20 and FIG. 99).

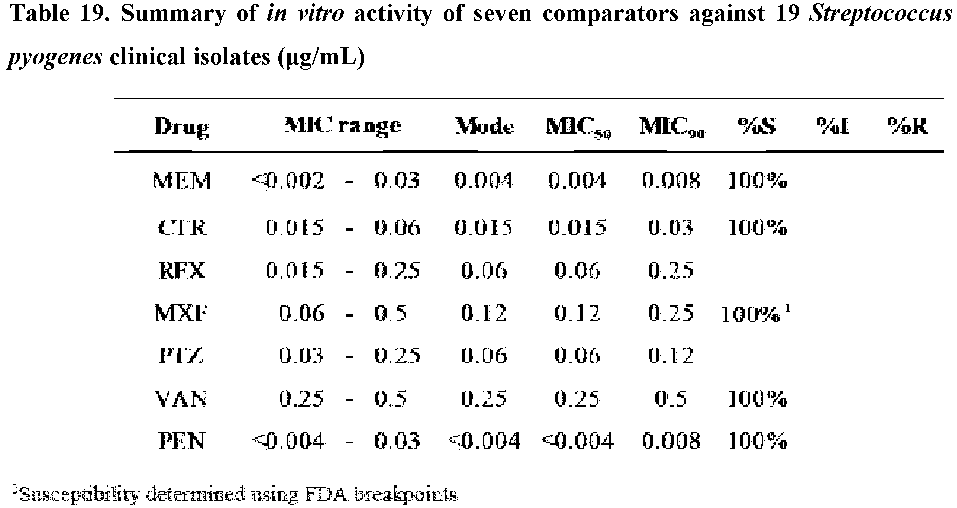

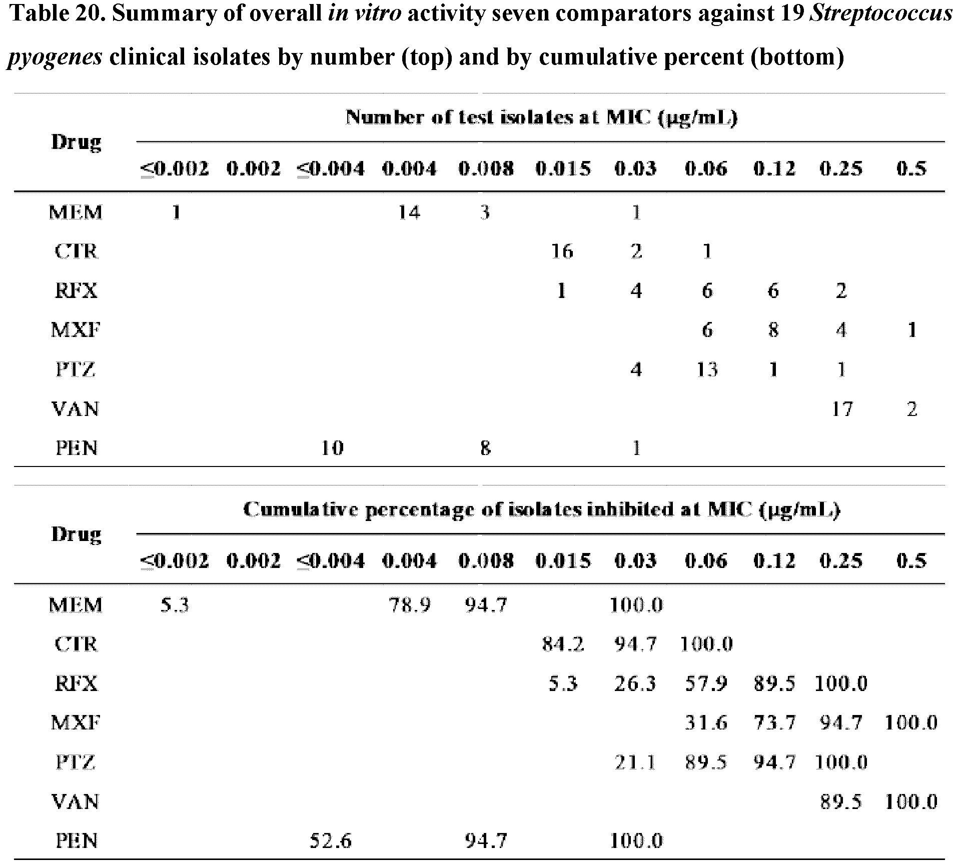

Against the 20 *S*. *agalactiae* tested, the meropenem MIC_{50/90} was 0.03/0.03 µg/mL, that for ceftriaxone was 0.06/0.06 µg/mL, and that for rifaximin was 0.12/0.12 µg/mL (Tables 21 and 22 and FIG. 100).

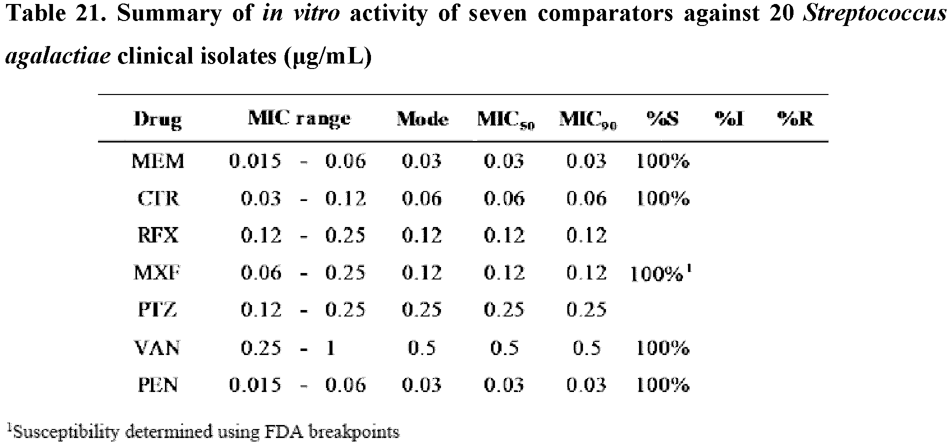

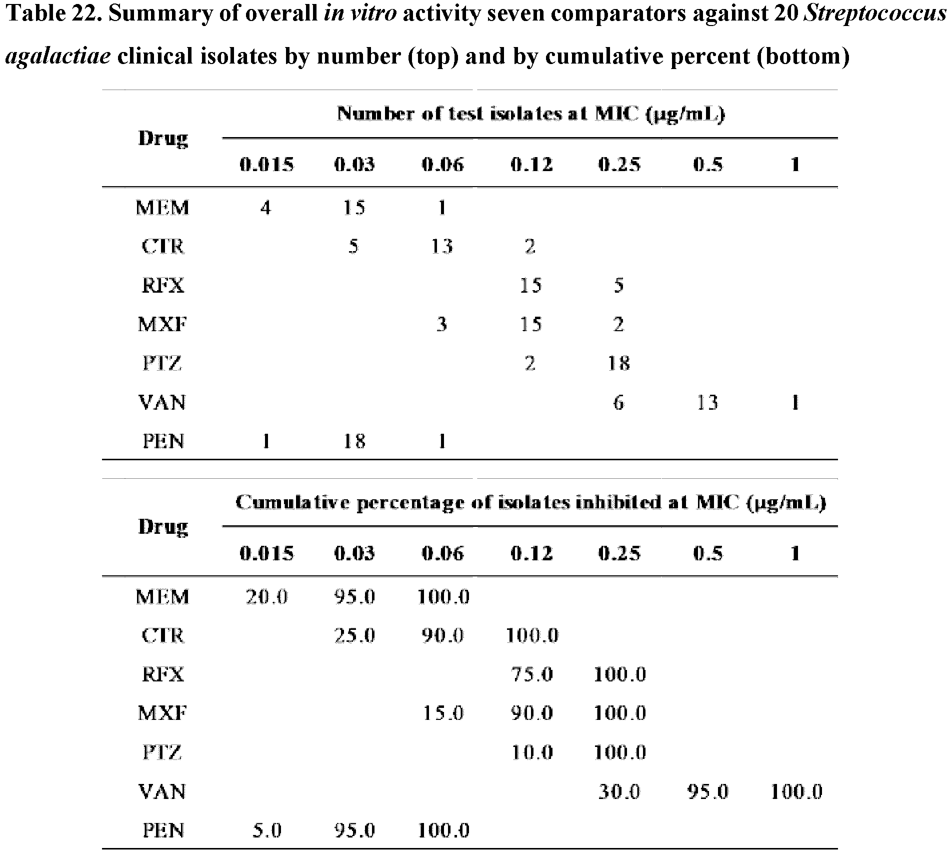

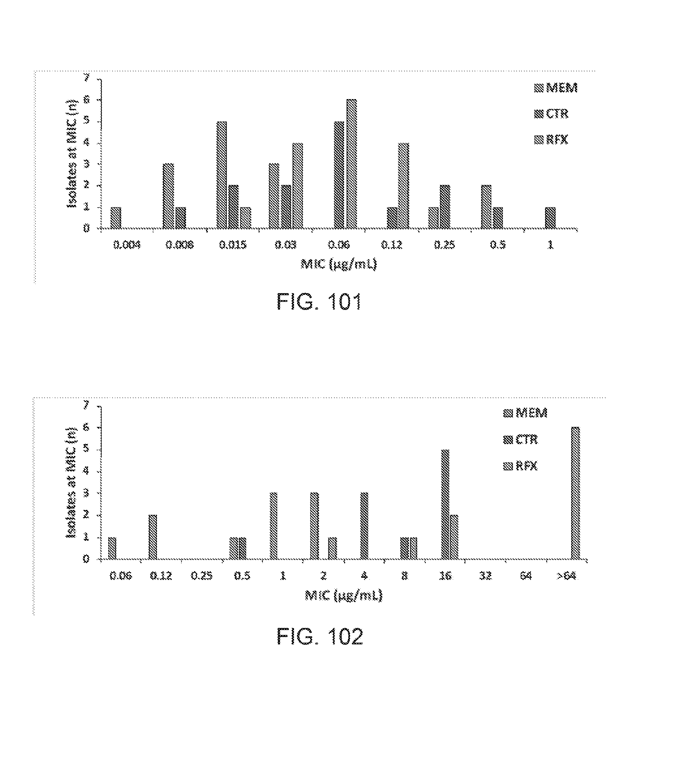

Against the 15 Viridans group *Streptococcus* tested, the meropenem MIC_{50/90} was 0.015/0.5 µg/mL, that for ceftriaxone was 0.06/0.5 µg/mL, and that for rifaximin was 0.06/0.12 µg/mL (Tables 23 and 24 and FIG. 101).

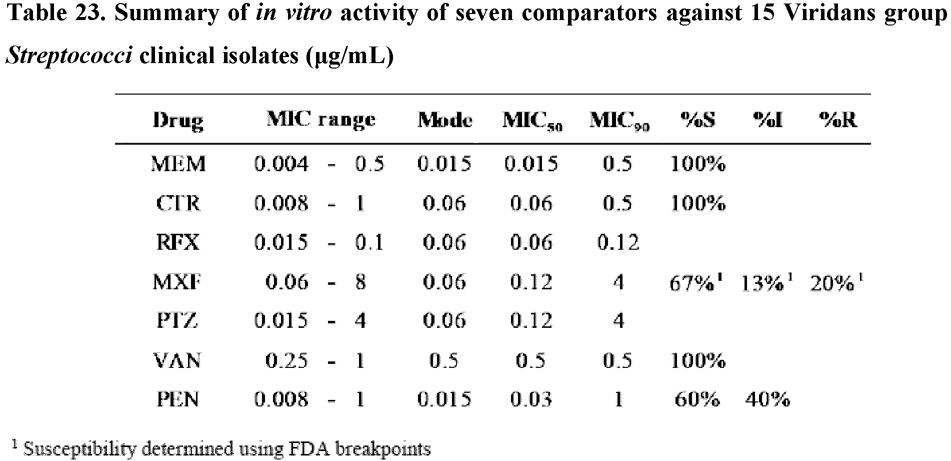

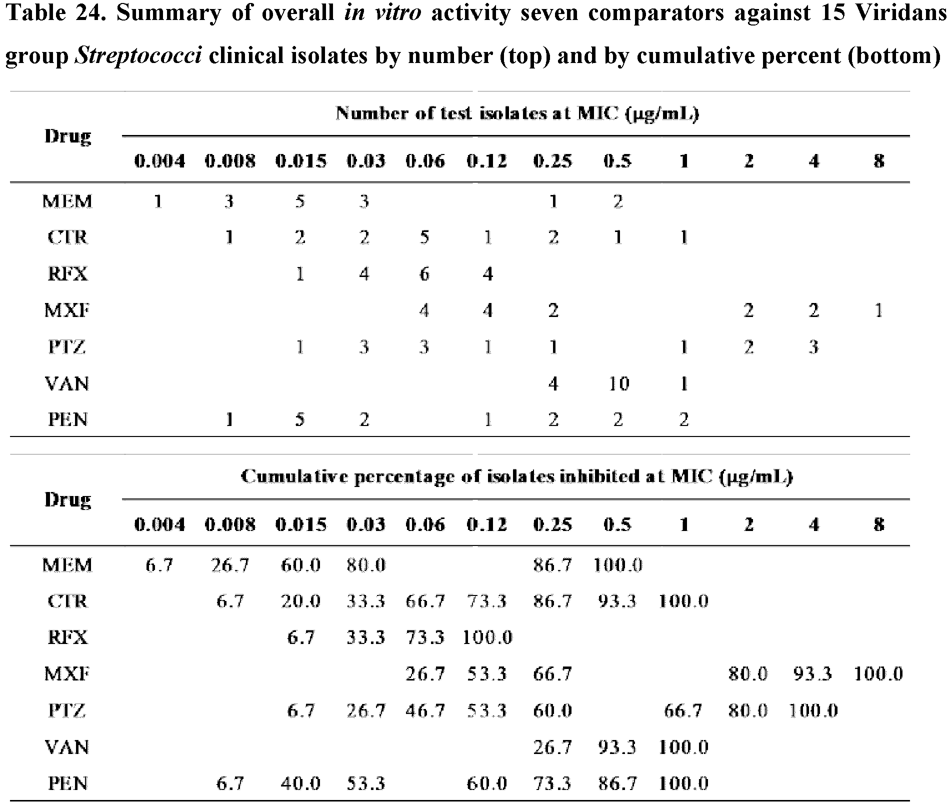

### Anaerobes

The panel of 10 *Clostridium* spp. in this study included 4 isolates of *C*. *sporogenes,* 2 isolates of *C*. *ramosum,* and 4 isolates of *C*. *innocuum.* As shown in Table 25 for all 10 *Clostridium* spp. in this study, meropenem had an MIC₅₀ of 1 µg/mL and an MIC₉₀ of 2 µg/mL, and an MIC range of 0.06 to 2 µg/mL. One hundred percent of the isolates in this panel were susceptible to meropenem. The MIC_{50/90} for ceftriaxone was 8/16 µg/mL, and the range was 0.5 to 16 µg/mL against this set of organisms, whereas for rifaximin the MIC₅₀ was >64 µg/mL, the MIC₉₀ >64 µg/mL, and the MIC range was 2 to >64 µg/mL. In this panel, all the isolates were susceptible to ceftriaxone. The comparator in this study was metronidazole, and meropenem and metronidazole had similar activity. FIG. 102 displays these data graphically, and Table 26 shows the number and cumulative percentage of isolates at each MIC.

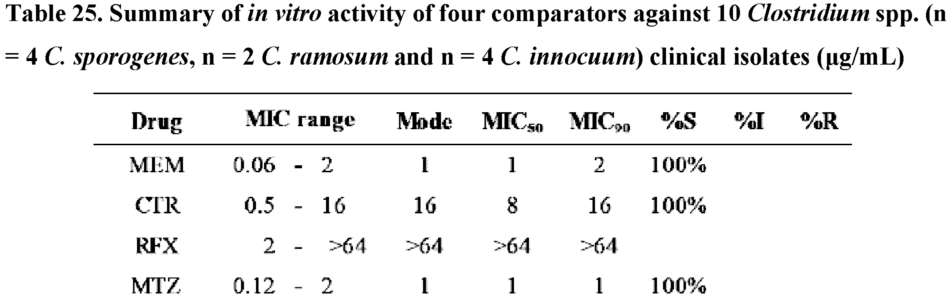

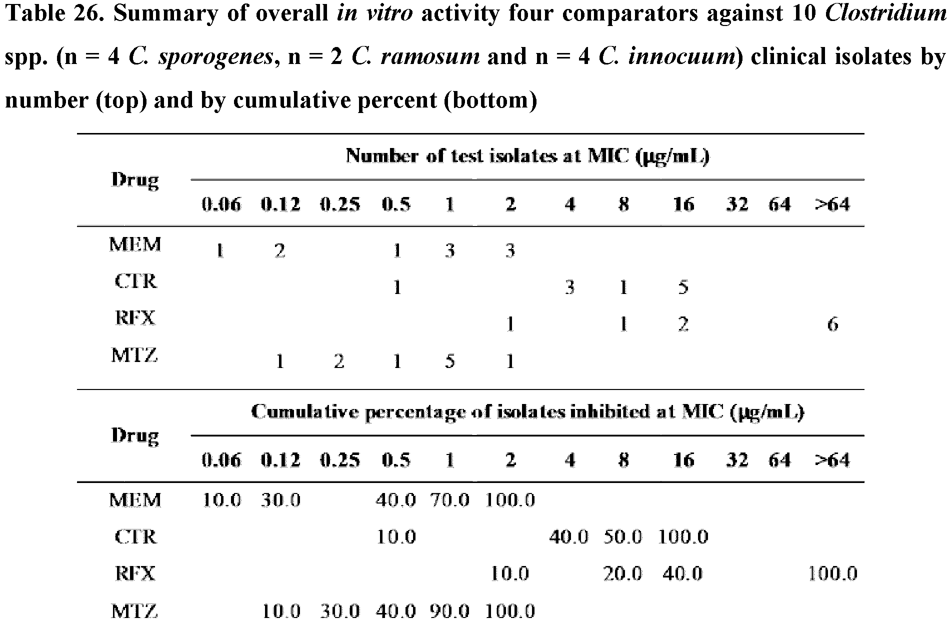

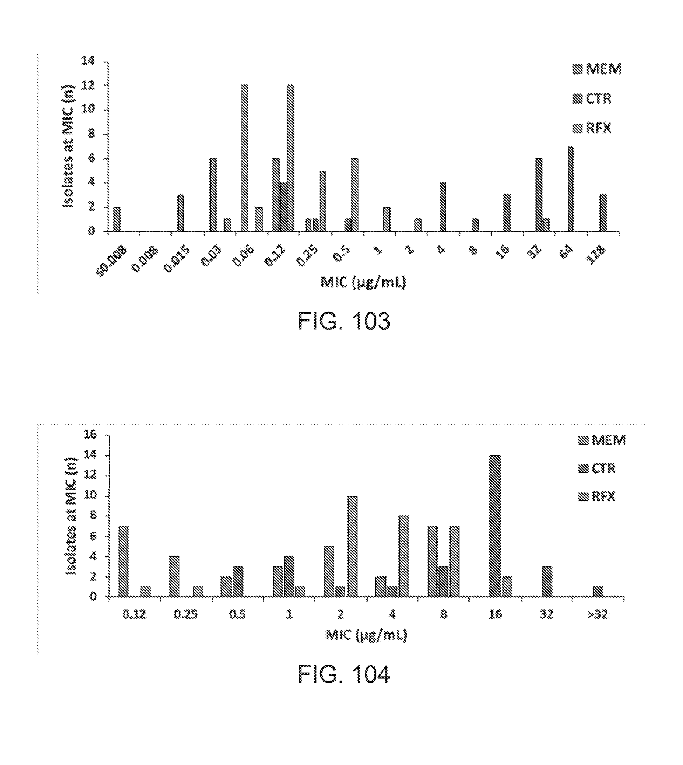

Among the panel of 30 *Prevotella* spp. tested were 5 *P. melanogenica,* 7 *P. bivia,* 11 *P. buccae,* and one isolate each of *P. nanceiensis, P. intermedia, P. denticola, P. nigrescens, P. corporis, P. bergensis,* and *P. disiens.* Table 27 shows the results of evaluating the agents against 30 *Prevotella* spp. isolates. Meropenem demonstrated an MIC₅₀ of 0.06 µg/mL, an MIC₉₀ of 0.12 µg/mL, and an MIC range of ≤0.008-0.3 µg/mL. All were susceptible to meropenem. For ceftriaxone, the MIC₅₀ was 32 µg/mL, the MIC₉₀ was 64 µg/mL and the range was 0.12-128 µg/mL; 33% of the isolates were ceftriaxone-resistant, and 20% had intermediate resistance to this drug. The rifaximin MIC₅₀ against these isolates was 0.12 µg/mL, the MIC₉₀ was 1 µg/mL with an MIC range of 0.03 to 32 µg/mL. The comparator in this study was metronidazole, and meropenem was the most active drug tested. FIG. 103 displays these data, and Table 28 shows the number and cumulative percentage of isolates at each MIC value.

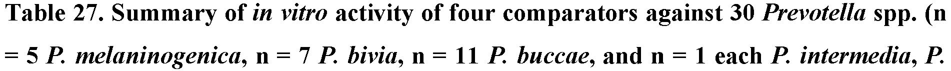

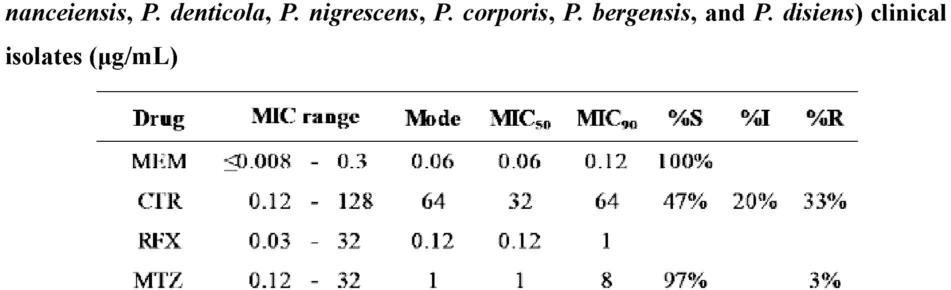

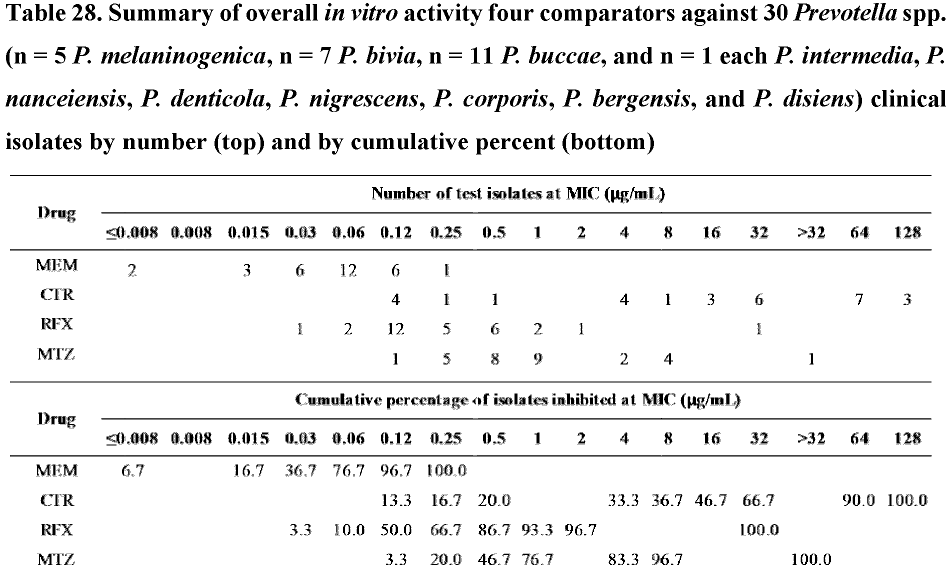

In this study, 30 *Veillonella* spp. were tested; 19 *V. parvula,* 9 *Veillonella* spp., 1 *Veillonella* dispr, and 1 *V. atypica.* When evaluated against the 30 *Veillonella* spp., meropenem had an MIC₅₀ of 1 µg/mL, an MIC₉₀ of 8 µg/mL, and an MIC range of 0.12 to 8 µg/mL (Table 29). Twenty-three percent of these isolates had intermediate resistance to meropenem. The MIC_{50/90} for ceftriaxone against these isolates was 16/32 µg/mL, and the range was 0.5 to >128 µg/mL, with 3% of isolates resistant and 10% of isolates with intermediate resistance to ceftriaxone. For rifaximin, the MIC₅₀ was 4 µg/mL, the MIC₉₀ was 8 µg/mL, and the MIC range was 0.12 to 16 µg/mL. The comparator in this study was metronidazole and meropenem was the most active drug evaluated. FIG. 104 displays these data graphically, and Table 30 shows the number and cumulative percentage of isolates at each MIC.

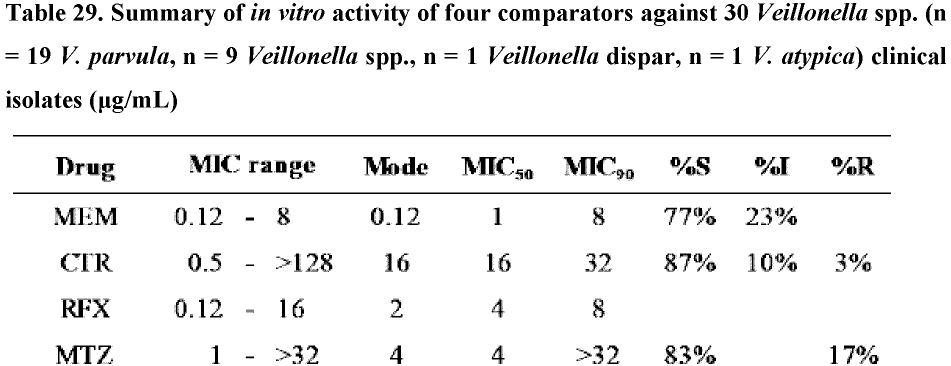

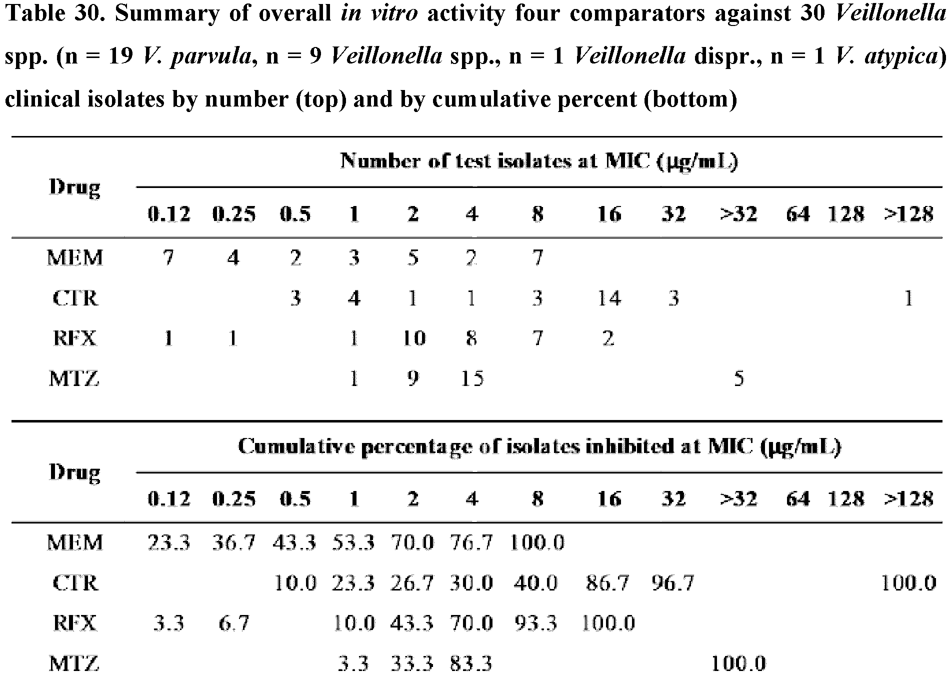

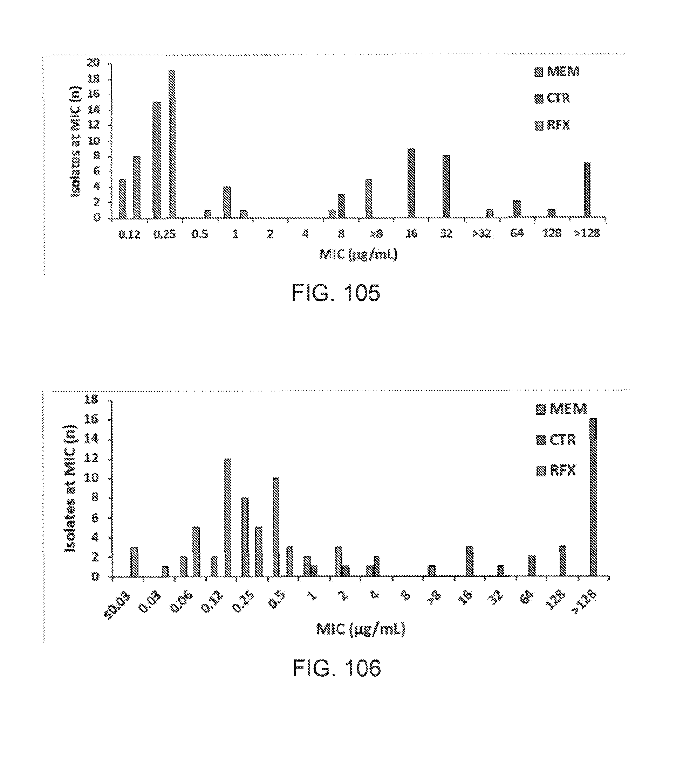

Against the 30 *Bacteroides fragilis* clinical isolates in this study, meropenem had an MIC_{50/90} of 0.25/>8 µg/mL and an MIC range of 0.12 to >8 µg/mL (Table 31). Twenty percent of isolates had intermediate resistance to meropenem. For ceftriaxone, the MIC range was 8 to 128 µg/mL, and the MIC_{50/90} was 32/>128 µg/mL. Thirty-three percent of these isolates were ceftriaxone-resistant, and 27% had intermediate resistance to this drug. The MIC₅₀ and MIC₉₀ for rifaximin against these isolates was 0.25 µg/mL, while the MIC range was 0.12 to >32 µg/mL. The comparator in this study was metronidazole, and meropenem and rifaximin had similar activity against these isolates. Table 32 shows the number and cumulative percentage of isolates at each MIC, and FIG. 105 contains a graphical representation of these data.

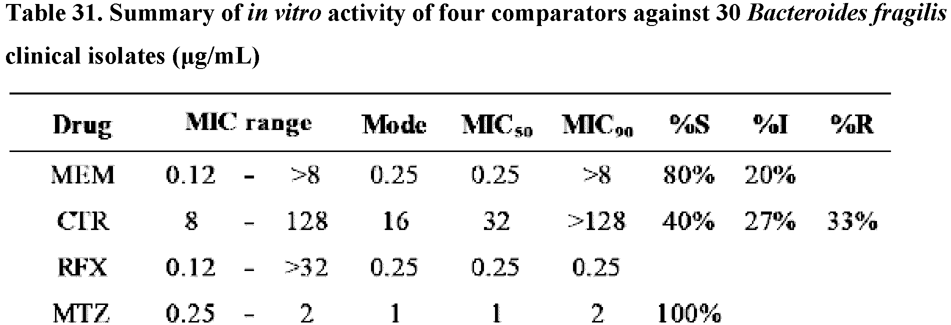

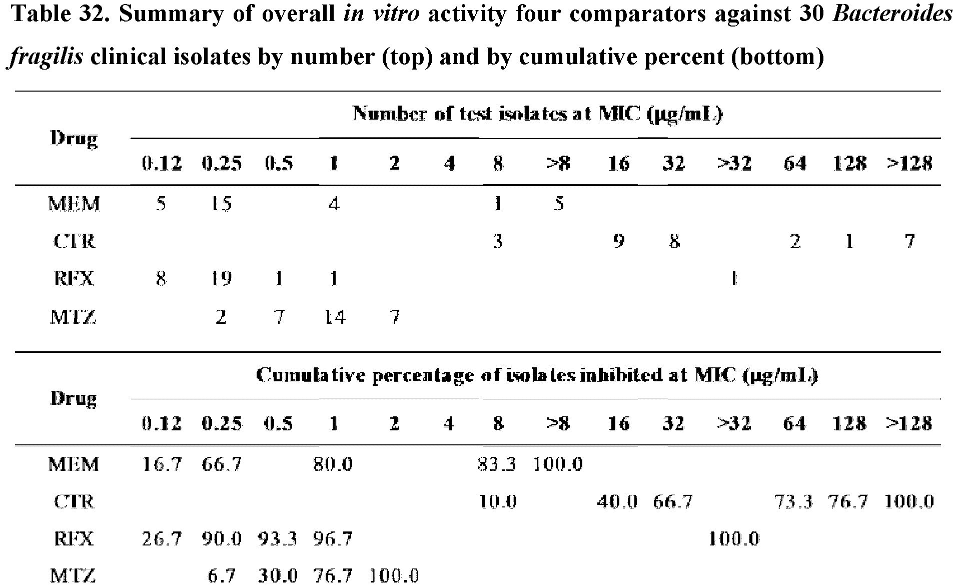

The panel of 29 *Bacteroides* non*-fragilis* isolates in this study included 3 isolates of *B. caccae,* 7 *B. thetaiotaomicron,* 4 *B. ovatus,* 5 *B. vulgatus,* 2 *B. uniformis,* 2 *B. stercoris,* 3 *B. xylanisolvens,* and 1 each of *B salyersiae, B. intestinalis,* and *B. faecis.* As shown in Table 33, against 29 *Bacteroides* non*-fragilis* isolates in this study, meropenem had an MIC₅₀ of 0.5 µg/mL and an MIC₉₀ of 2 µg/mL, with an MIC range of 0.06 to >8 µg/mL. Three percent of the isolates in this panel had intermediate resistance to meropenem. The MIC_{50/90} for ceftriaxone was >128/>128 µg/mL, and the range was 1 to 128 µg/mL against this set of organisms, whereas for rifaximin the MIC₅₀ was 0.12 µg/mL, the MIC₉₀ 0.5 µg/mL, and the MIC range was 0.03 to 0.5 µg/mL. In this panel, 72% the isolates were resistant to ceftriaxone and 3% had intermediate resistance. The comparator in this study was metronidazole and rifaximin was the most active. FIG. 106 displays these data graphically, and Table 34 shows the number and cumulative percentage of isolates at each MIC.

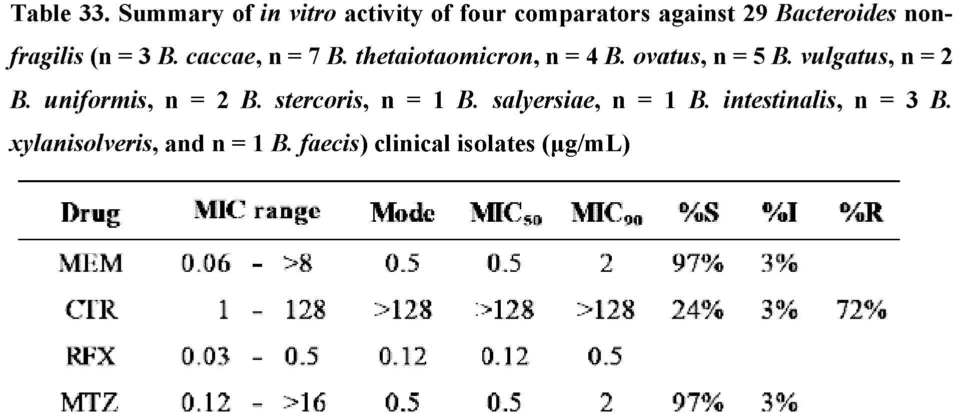

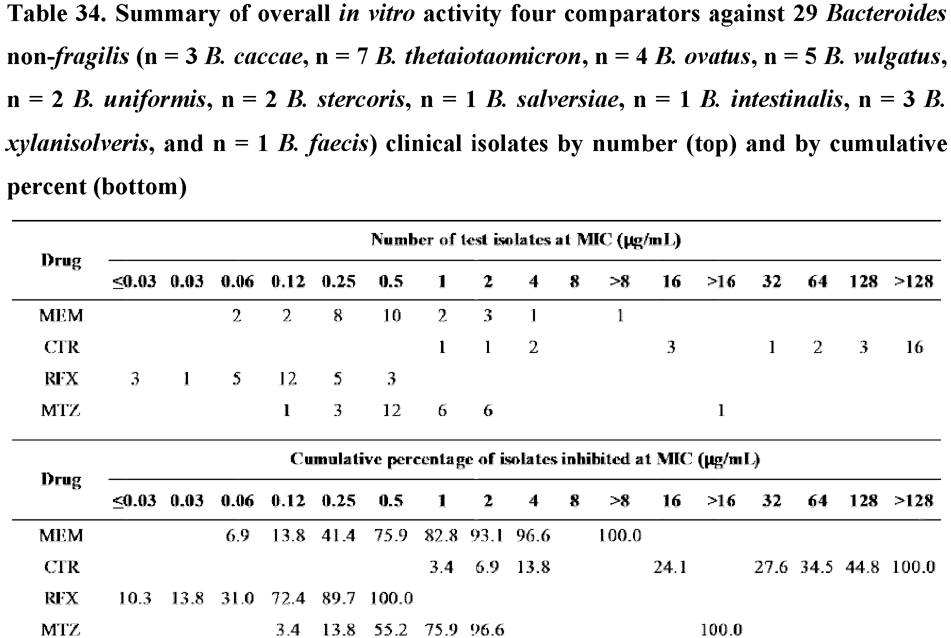

### Summary

In summary, meropenem, ceftriaxone and rifaximin were evaluated along with the appropriate comparators for *in vitro* activity against a broad range of Gram-positive and Gram-negative aerobic and anaerobic bacteria that are implicated as being relevant in SIBO. Since rifaximin has typically been used clinically as a topical antibiotic, resistance breakpoints were not available. The resistance breakpoints for meropenem, ceftriaxone, and the comparator agents were determined for systemic administration (see CLSI (1) and (2)). The results indicated that meropenem was the most active agent against *E. aerogenes, E. coli, P. aeruginosa, Streptococcus* spp., *Clostridium* spp., *Prevotella* spp., and *Veillonella* spp., while ceftriaxone was the most active agent tested against *P. mirabilis.* The MIC₉₀ value for these agents was greater than the highest concentration tested for *Klebsiella* spp., and therefore the order of potency could not be determined. However, the MIC₅₀ value for meropenem was the lowest.

### Example D: Time-kill kinetics of antimicrobials against SIBO organisms

The time-kill kinetics of the antibacterial agents meropenem and ceftriaxone against bacteria implicated in the pathogenesis of SIBO were compared with rifaximin which is used therapeutically for SIBO. Meropenem, ceftriaxone and rifaximin were evaluated against three isolates each of *Escherichia coli, Bacteroides* spp, and *Streptococcus* spp. at multiples of the minimum inhibitory concentration (MIC) (2X, 4X, and 8X the MIC). MIC values were determined by broth microdilution in accordance with guidelines from the Clinical and Laboratory Standards Institute (CLSI; (1) "Methods for Dilution Antimicrobial Susceptibility Tests for Bacteria That Grow Aerobically; Approved Standard; 11th ed.," CLSI standard M07, 2018; (2) "Performance Standards for Antimicrobial Susceptibility Testing; 28th ed.," CLSI supplement M100, 2018).

### Materials and Methods

### Test Compounds

Meropenem, rifaximin, and ceftriaxone were provided by Micromyx (Kalamazoo, MI).

### Organisms

Three isolates of each of *Escherichia coli, Bacteroides* spp, and *Streptococcus* spp. were used. Upon initial receipt, the isolates were streaked onto the appropriate agar medium. *E. coli* isolates were incubated overnight at 35 °C. *Streptococcus* isolates were incubated overnight at 35 °C in a CO₂ incubator and anaerobes were incubated in a Bactron Anaerobic Chamber for 48 hours at 35 °C. Using growth from these plates, frozen stocks were prepared in Trypticase Soy Broth (Brucella broth for anaerobic organisms) containing 20% sterile glycerol and were stored at -80 °C.

During testing, the isolates were cultured from frozen stocks onto Trypticase soy Agar with 5% Sheep blood (Becton Dickinson; Sparks, MD) for *E. coli* and *Streptococcus* in accordance with guidelines from the Clinical and Laboratory Standards Institute. Anaerobes were cultured on Supplemented Brucella Agar with 5% sheep blood (Becton Dickinson). All strains were incubated overnight (48 hours for anaerobic organisms) at 35 °C under the appropriate conditions before testing.

### Test Media

The medium employed during MIC and time-kill testing for *E*. *coli* was cation-adjusted Mueller Hinton broth (CAMHB; Becton-Dickinson [BD], Sparks, MD). For *Streptococcus* the medium was CAMHB with 3% laked horse blood (Hemostat Laboratories; Dixon, CA), and for anaerobes the medium was Supplemented Brucella broth (Becton Dickinson) containing 5 µg/mL hemin (Sigma, St. Louis, MO), 1 µg/mL vitamin K1 (Sigma), and 5% laked horse blood. All media were prepared and stored per CLSI guidelines (2).

Track plates for enumerating organisms were prepared using Trypticase Soy Agar (TSA; BD) for *E. coli,* Trypticase Soy Agar with 5% laked sheep blood (Hemostat Laboratories) for *Streptococci,* and Supplemented Brucella agar (BD) with 5% laked sheep blood for anaerobes.

### Broth Microdilution MIC

Meropenem, rifaximin, and ceftriaxone were first subjected to broth microdilution MIC testing with the appropriate QC organisms conducted in accordance with CLSI guidelines (1) and (2). Automated liquid handlers (Biomek 2000 and Biomek FX, Beckman Coulter, Fullerton CA) were used to conduct serial dilutions and liquid transfers.

To prepare the drug mother plate, which provided the serial drug dilutions for the replicate daughter plates, the wells of columns 2 through 12 of standard 96-well microdilution plates (Costar 3795) were filled with 150 µL of the designated diluent for each row of drug. The test article and comparator compound (300 µL at 100X the highest concentration to be tested) were dispensed into the appropriate wells in column 1. The Biomek 2000 was then used to make 2-fold serial dilutions in the mother plate from column 1 through column 11. The wells of Column 12 contained no drug and served as the organism growth control wells for the assay.

The daughter plates were loaded by hand with 188 µL per well of the appropriate media (CAMHB for *E. coli,* CAMHB +3% LHB for *S. pneumoniae,* and Supplemented Brucella broth + 5% LHB for *Bacteroides* spp.). The daughter plates were prepared on the Biomek FX which transferred 2 µL of drug solution from each well of a mother plate to the corresponding well of each daughter plate in a single step.

A standardized inoculum of each organism was prepared per CLSI methods (1) in the appropriate media to equal the turbidity of a 0.5 McFarland standard. To generate the final inoculum, the 0.5 McFarland suspensions were further diluted 1:20 (1:10 for *Bacteroides* spp.) in media, followed by dispensing into sterile reservoirs (Beckman Coulter 372788). The Biomek 2000 was used to deliver 10 µL of standardized inoculum into each well resulting in a final cell density of approximately 5 x 10⁵ CFU/mL. Daughter plates were placed on the Biomek 2000 work surface in reverse orientation so that inoculation took place from low to high drug concentration.

Plates were stacked 3 high, covered with a sterile lid on the top plate, placed in plastic bags, and incubated at 35°C in ambient atmosphere for approximately 18-20 hr. *Bacteroides* spp. were incubated at 35°C for approximately 48 hr under anaerobic conditions. Following incubation, the microplates were removed from the incubator and viewed from the bottom using a plate viewer. For each of the test compounds, an un-inoculated solubility control plate for each test medium was observed for evidence of drug precipitation. The MIC was read and recorded as the lowest concentration of drug that inhibited visible growth.

### Time-kill Assay

Time-kill was determined using methods described by CLSI ((3)"Methods for Determining Bactericidal Activity of Antimicrobial Agents; Approved Guideline," CLSI document M26-A, 1999) to evaluate the bactericidal activity of test agents. All compounds were tested at 2X, 4X and 8X the MIC determined by earlier testing.

A suspension was prepared from growth on a freshly-streaked agar plate to equal the turbidity of a 0.5 McFarland standard in the appropriate medium. *E coli* was prepared in CAMHB; *Streptococci* were prepared in Todd Hewitt Broth, and anaerobes were prepared in supplemented brucella broth. The suspensions were diluted 1:10 for *E coli,* 1:4 for *Streptococci* and anaerobes, and were grown in fresh media as follows to generate log-phase inocula: *E. coli* and *Streptococci* were incubated at 35 °C with shaking, while *Bacteroides* spp. were incubated statically in the anaerobe chamber at 35 °C. After approximately 2 to 3 hr of growth, the log-phase culture was adjusted to equal a 0.5 McFarland standard. The resulting log-phase suspension was used as the inoculum as described below.

Time-kill studies were conducted in sterile 125 mL Erlenmeyer flasks containing 18.8 mL of appropriate broth media as described above. To each test flask was added 0.2 mL of the appropriate 100X drug solution. A drug-free control flask was included to serve as the positive growth control. A 1 mL aliquot of the log-phase inoculum was added to each test flask, resulting in a final target cell density of approximately 5 x 10⁶ CFU/mL. After inoculation, *E coli* and *Streptococci* test flasks were immediately placed in a 35 °C ambient air incubator with shaking at 150 rpm. *Bacteroides* were incubated statically at 35 °C in an anaerobe chamber.

In order to enumerate the inoculum used to initiate the time-kill studies, 0.3 mL aliquots were removed from each individual organism inoculum, subjected to 10-fold serial dilutions in chilled sterile saline, and a 10 µL aliquot of each dilution was spotted in duplicate across the top of an agar plate. The plate was then tilted at a 45 - 90° angle to allow the 10 µL aliquot to track across the agar surface to the opposite side of the plate. The plates were laid flat, allowed to dry at room temperature, then inverted and incubated at 35 °C for approximately 24 hr. Colonies were manually counted to determine the viable count in CFU/mL.

During the time-kill study, the test flasks were sampled at 2, 6, and 24 hr for determination of viable counts using the track dilution method as described above. *Bacteroides* was also sampled at 48 hr. All *E. coli* plates were incubated for 20-24 hr at 35 °C, *Streptococci* for 20-24 hr at 35 °C in a CO₂ incubator, and *Bacteroides* for approximately 48 hr at 35 °C in the anaerobe chamber. Colonies were counted manually, and the CFU/mL was determined from the average count of the duplicate plates, followed by the calculation of the log₁₀ CFU/mL. The limit of detection for the assay was 50 CFU/mL. A kill of at least 3-logs at 24 hr was used to define bactericidal activity.

### Results

Meropenem, ceftriaxone and rifaximin were evaluated for *in vitro* bactericidal activity against organisms thought to be involved in small intestinal bacterial overgrowth (SIBO), with bactericidal being defined as a ≥3 log reduction in colony forming units (CFU) per mL after 24 hours of drug exposure. MIC results for these antibiotics and the comparator agents against the test isolates are shown in Table 35, and this data was used to determine the drug exposures at multiples of the MIC described below. Though the MIC values are shown, note that metronidazole and vancomycin are not clinically useful antibiotics for *E. coli.*

**Table 35. Activity of meropenem, ceftriaxone and rifaximin against the test isolates**

| **Isolate** | **MIC (µg/mL)** | | | | | |
|---|---|---|---|---|---|---|
| | **Meropenem** | **Ceftriaxone** | **Rifaximin** | **Metronidazole** | **Piperacillin/ tazobactam** | **Vancomycin** |
| *E. coli* ATCC 25922 | 0.015 (0.008-0.06)¹ | 0.06 (0.03-0.12) | 8 | >32 | 4/4 (0.5/4-4/4) | >16 |
| *E. coli* ATCC 35218 | 0.015 (0..008-0.06) | 0.03 | 4 | >32 | 2/4 (0.5/4-2/4) | >16 |
| *E. coli* MMX 1312 | 0.008 | 4 | 8 | >32 | 16/4 | >16 |
| *S*. *pneumoniae* ATCC 49619 | 0.06 (0.03-0.25)¹ | 0.06 (0.03-0.12) | 0.06 | >32 | 1 | 0.25 (0.12-0.5) |
| *S*. *pyogenes* ATCC 49399 | 0.004 | 0.015 | 0.12 | >32 | 0.06 | 0.25 |
| *S*. *agalactiae* ATCC 13813 | 0.008 | 0.015 | 0.25 | >32 | 0.06 | 0.5 |
| *B. fragilis* MMX 123 ATCC 25285 | 0.06 (0.03-0.25)¹ | 32 | 0.5 | 0.5 (0.25-2) | 8 (4-16) | >16 |
| *B. vulgatus* ATCC 8482 | 0.12 | 2 | 0.25 | 0.5 | 4 | >16 |
| *B. ovatus* MMX 3504 | 0.12 | >128 | 2 | 0.5 | >16 | >16 |

| | | | | | | |
|---|---|---|---|---|---|---|
| ¹CLSI QC ranges shown in parentheses | | | | | | |

### E. coli

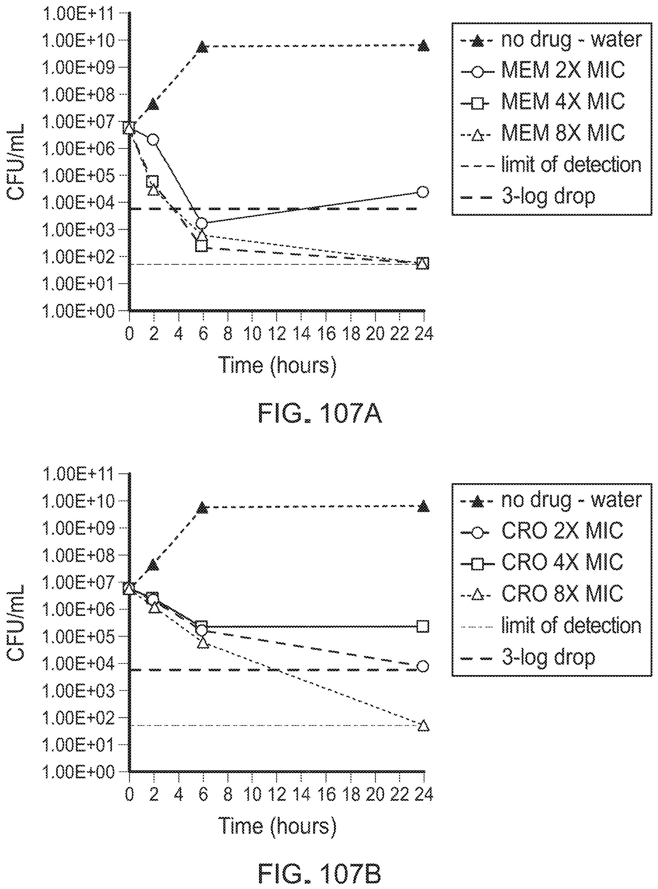

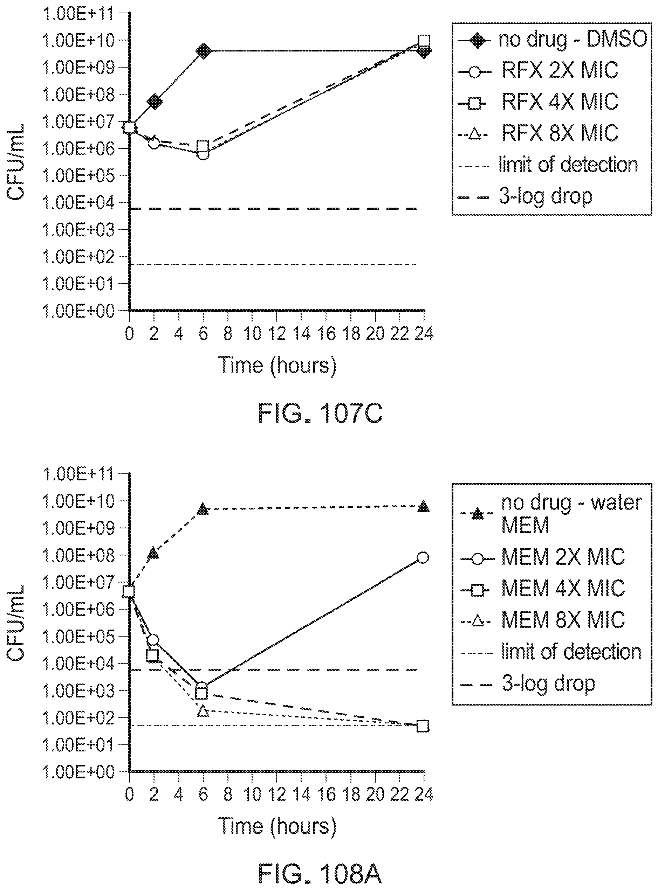

Broth microdilution MIC values for meropenem, ceftriaxone and rifaximin were 0.015, 0.06, and 8 µg/mL against *E. coli* ATCC 25922, respectively. The time-kill kinetics for meropenem, ceftriaxone and rifaximin against this organism are shown in FIG. 107, and the corresponding CFU/mL, log₁₀ CFU/mL, and change in log₁₀ CFU/mL relative to baseline are shown in Table 36. Meropenem at 2X, 4X and 8X MIC demonstrated rapid killing and bactericidal activity at the two higher concentrations at 24 hr. Ceftriaxone was bactericidal at 8X MIC at 24 hr, whereas rifaximin exhibited a bacteriostatic effect at 2-6 hr with regrowth between 6 and 24 hr.

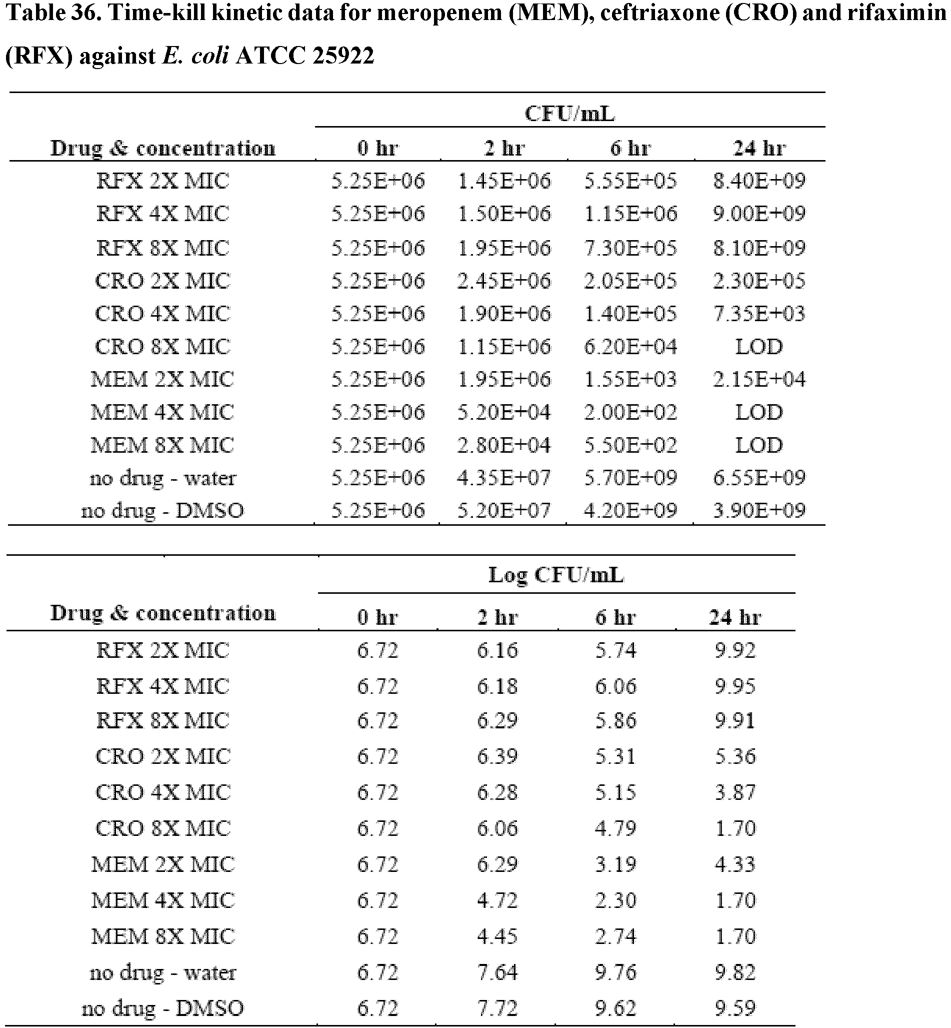

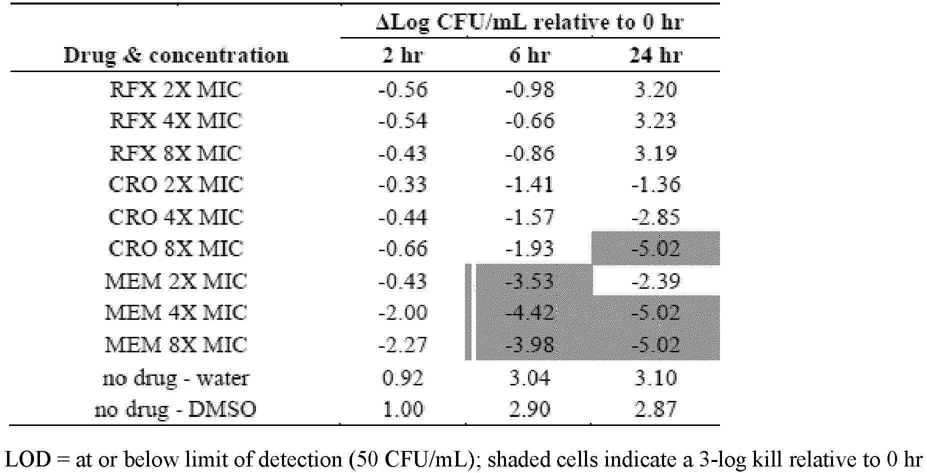

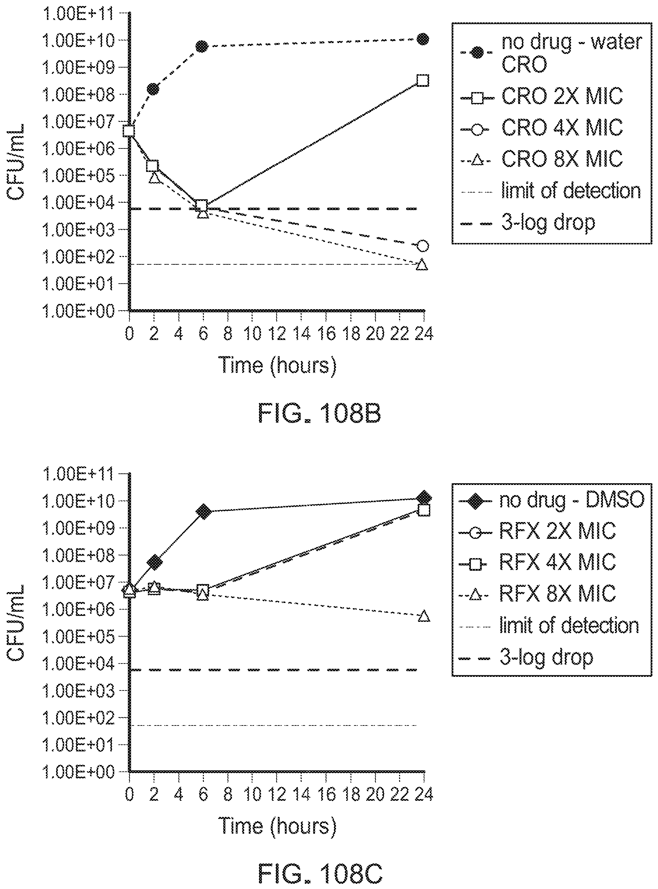

The MIC values for meropenem, ceftriaxone and rifaximin against *E. coli* ATCC 35218 (TEM-1 β-lactamase producer) were 0.015, 0.03, and 4 µg/mL, respectively. For this organism, the time-kill kinetics and corresponding data are shown in FIG. 108 and Table 37, respectively. Meropenem at 2X, 4X and 8X MIC demonstrated rapid killing and bactericidal activity at the two higher concentrations at 24 hr. Ceftriaxone was bactericidal at 24 hr at 4X and 8X MIC, whereas rifaximin was bacteriostatic at 8X the MIC with regrowth at 24 hr for both 2X and 4X the MIC.

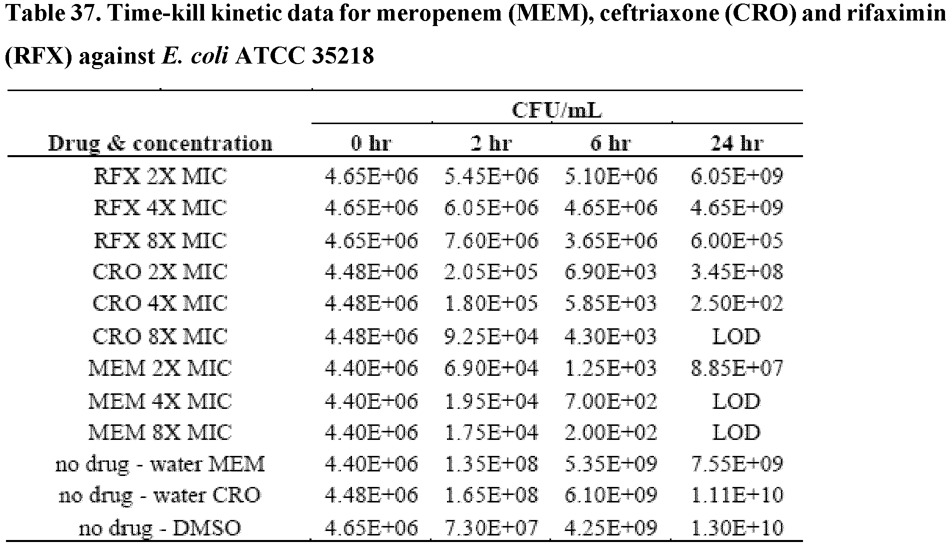

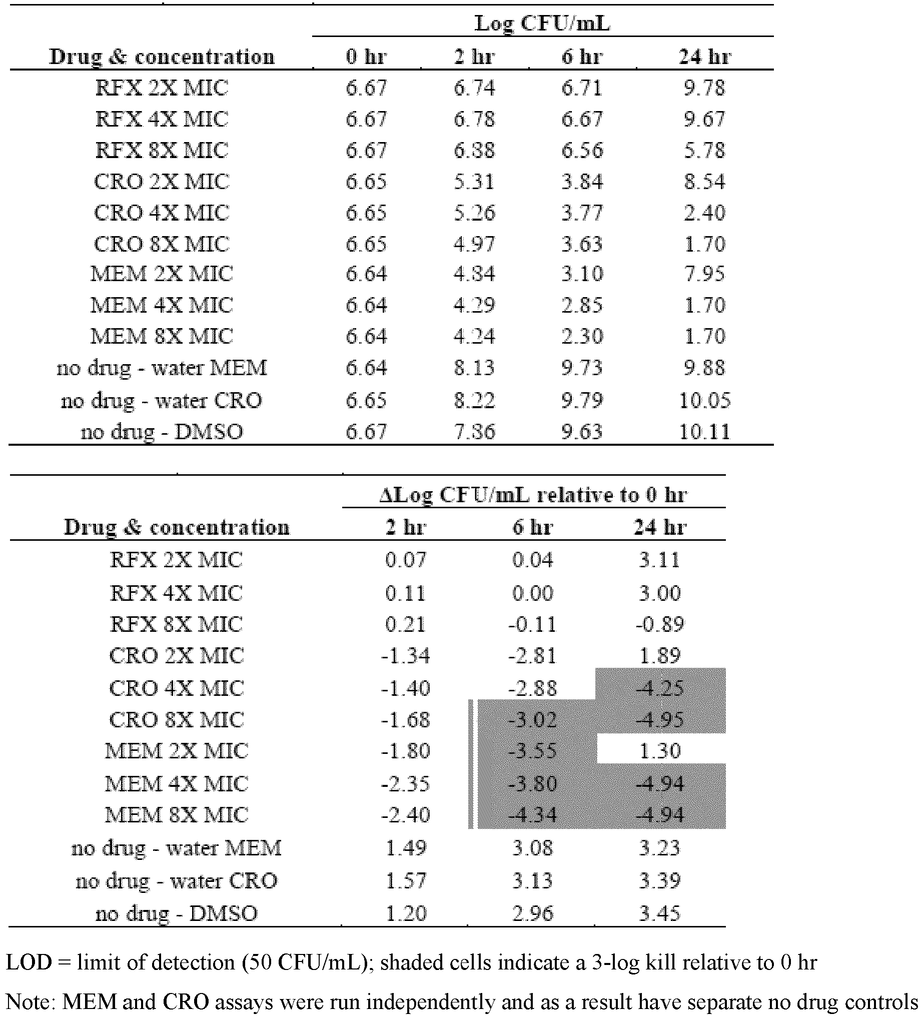

Against *E. coli* MMX 1312, meropenem, ceftriaxone and rifaximin had MIC values of 0.008, 4, and 8 µg/mL, respectively. The time-kill kinetics results for these drugs for this strain can be found in FIG. 108 and Table 38. Against this organism, no sustained bactericidal effect was observed for any drug; only meropenem at 8X MIC at the 6 hr timepoint exhibited a 3-log₁₀ CFU decrease but regrowth was observed at 24 hr for all concentrations. Ceftriaxone at 8X the MIC had killing at 6 and 24 hr that approached 3-logs. Regrowth was also observed for all concentrations of rifaximin.

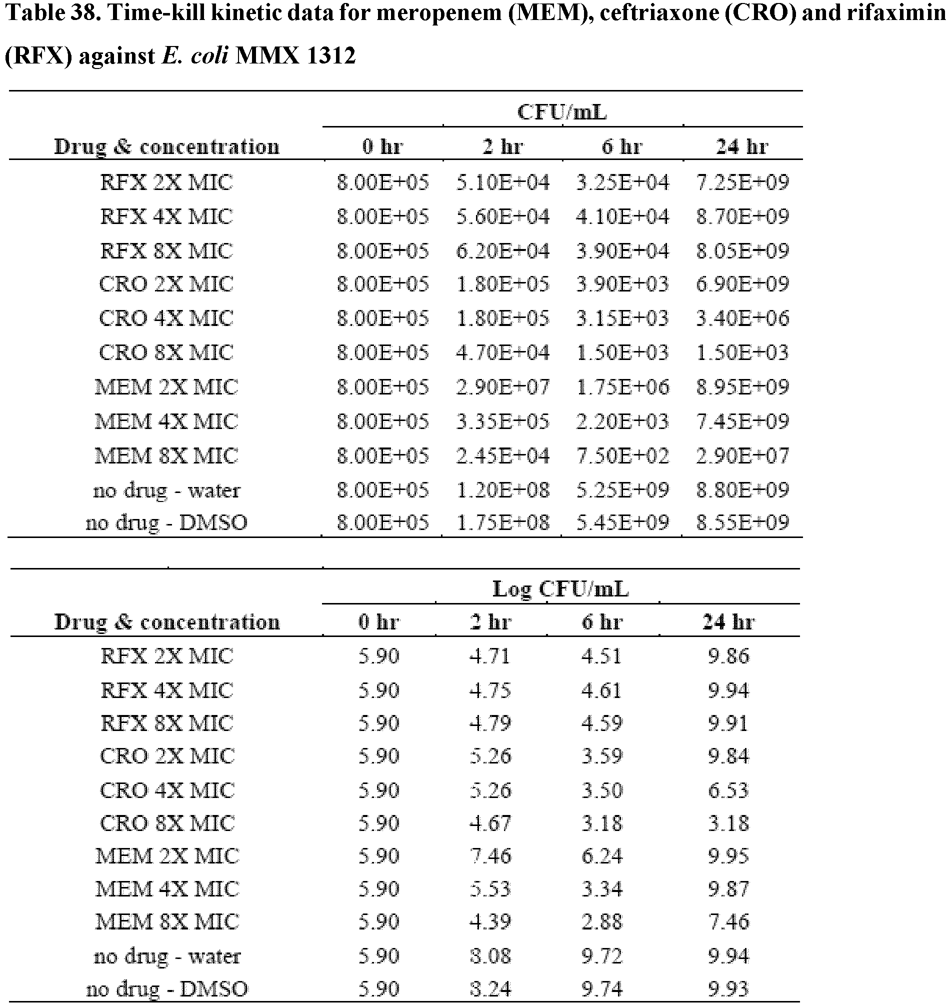

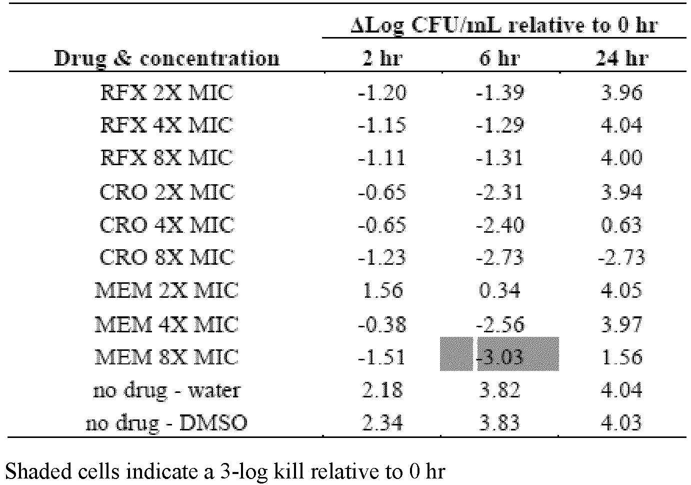

### Streptococcus spp.

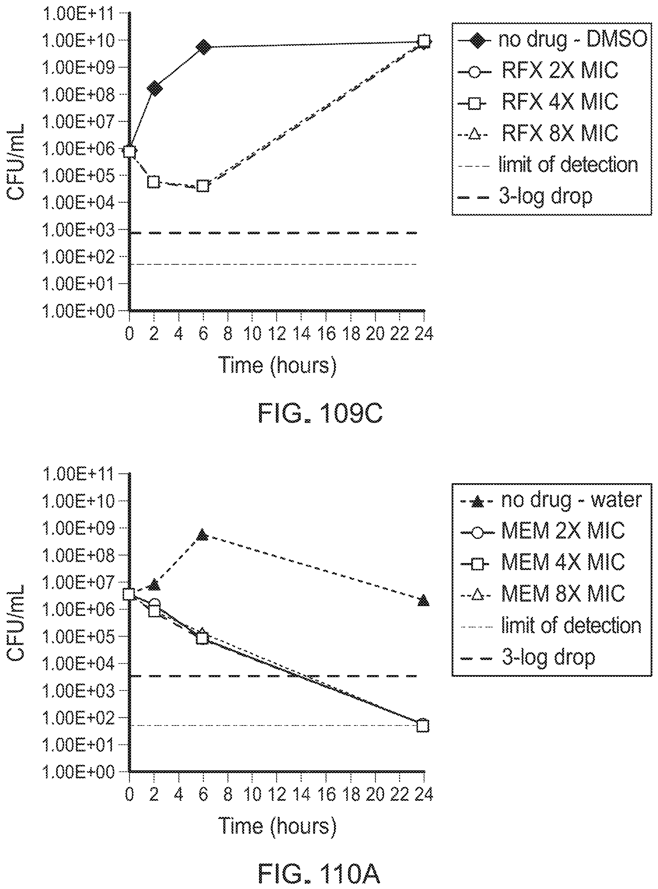

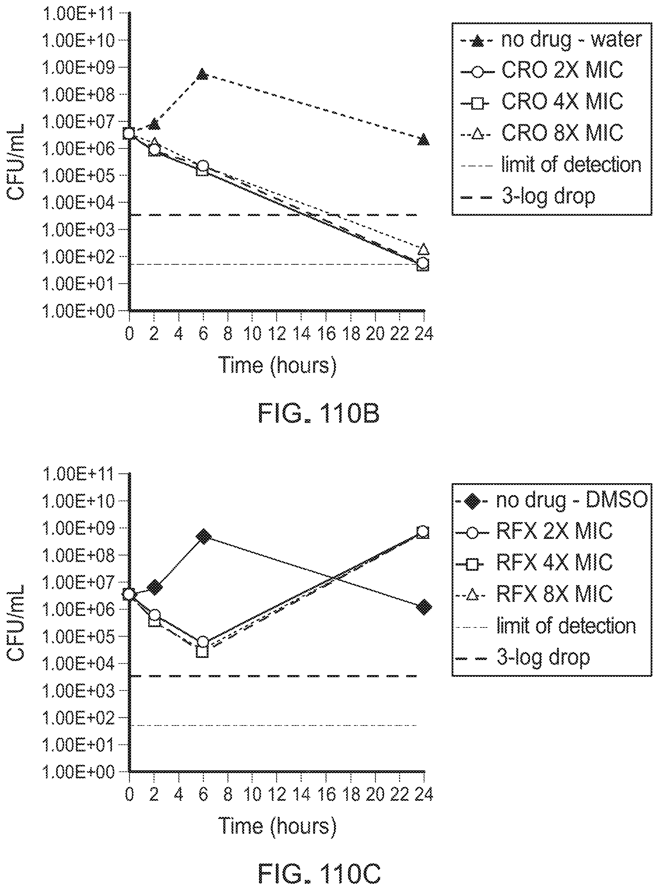

Meropenem, ceftriaxone and rifaximin all had MIC values of 0.06 µg/mL against *S*. *pneumoniae* ATCC 49619. The time-kill kinetics for meropenem, ceftriaxone and rifaximin against this organism are shown in FIG. 110, and the corresponding data are shown in Table 39. For meropenem and ceftriaxone, all three drug concentrations tested demonstrated bactericidal effects at 24 hr, whereas for rifaximin, there was an approximately 2.3 log₁₀ CFU decrease observed at all three concentrations at the 6 hr timepoint that rebounded to no-drug control levels at 24 hr.

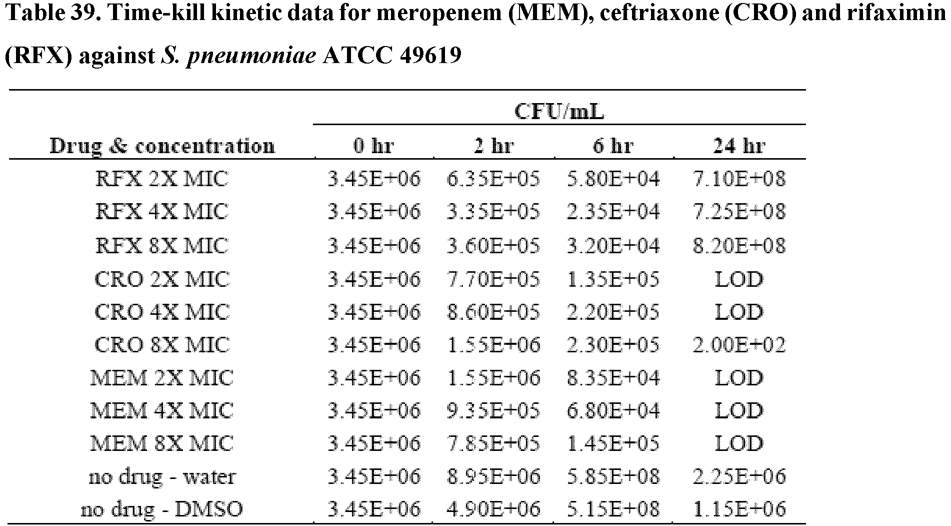

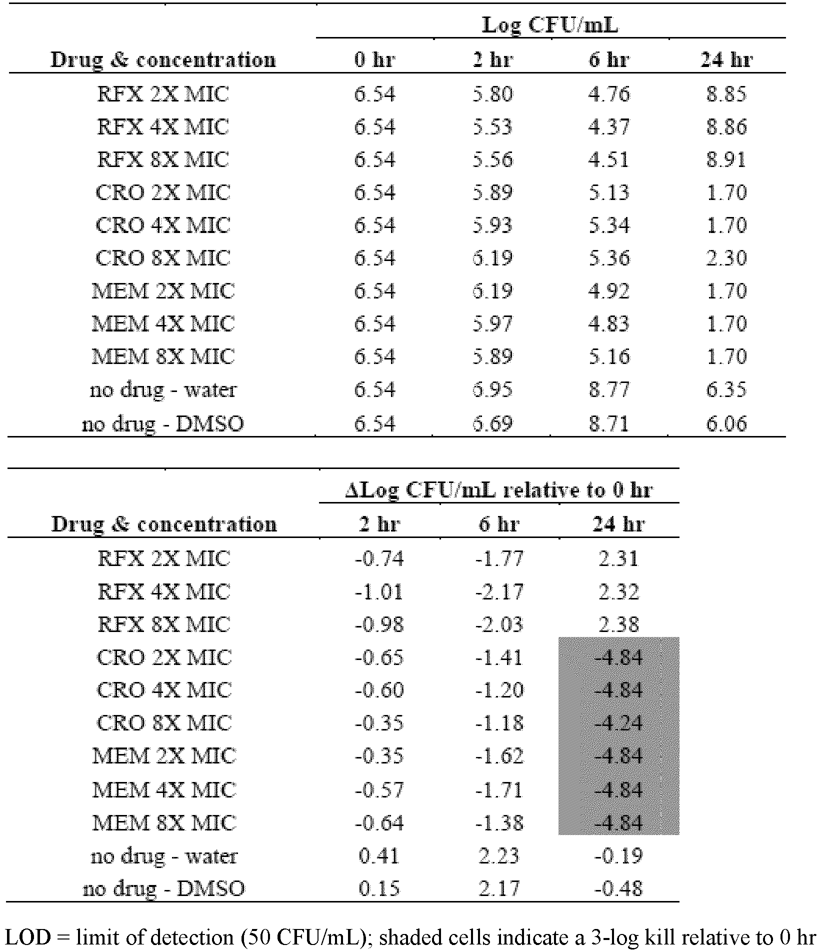

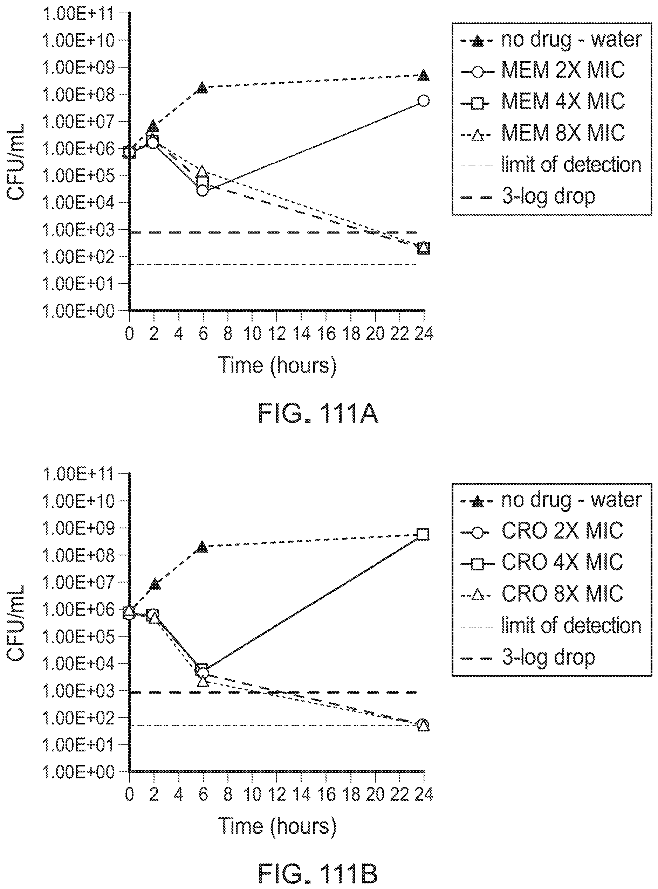

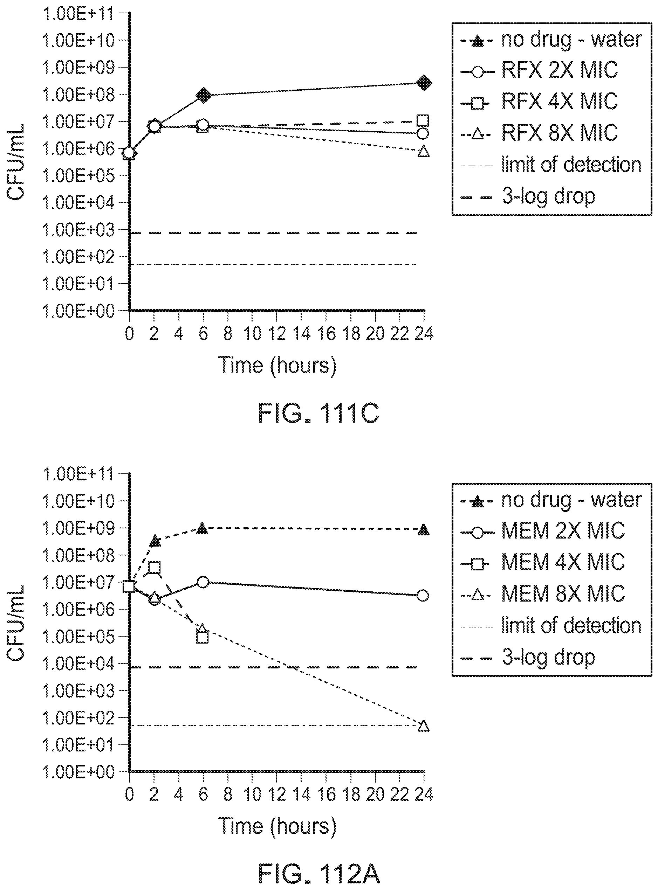

The MIC values for meropenem, ceftriaxone and rifaximin against *S. pyogenes* ATCC 49399 were 0.004, 0.015, and 0.12 µg/mL, respectively. For this organism, the time-kill kinetics and corresponding data are shown in FIG. 111 and Table 40, respectively. Meropenem showed a bactericidal effect at 4X and 8X MIC at 24 hr, as did ceftriaxone. Rifaximin demonstrated a bacteriostatic effect against this organism at 2X, 4X, and 8X the MIC.

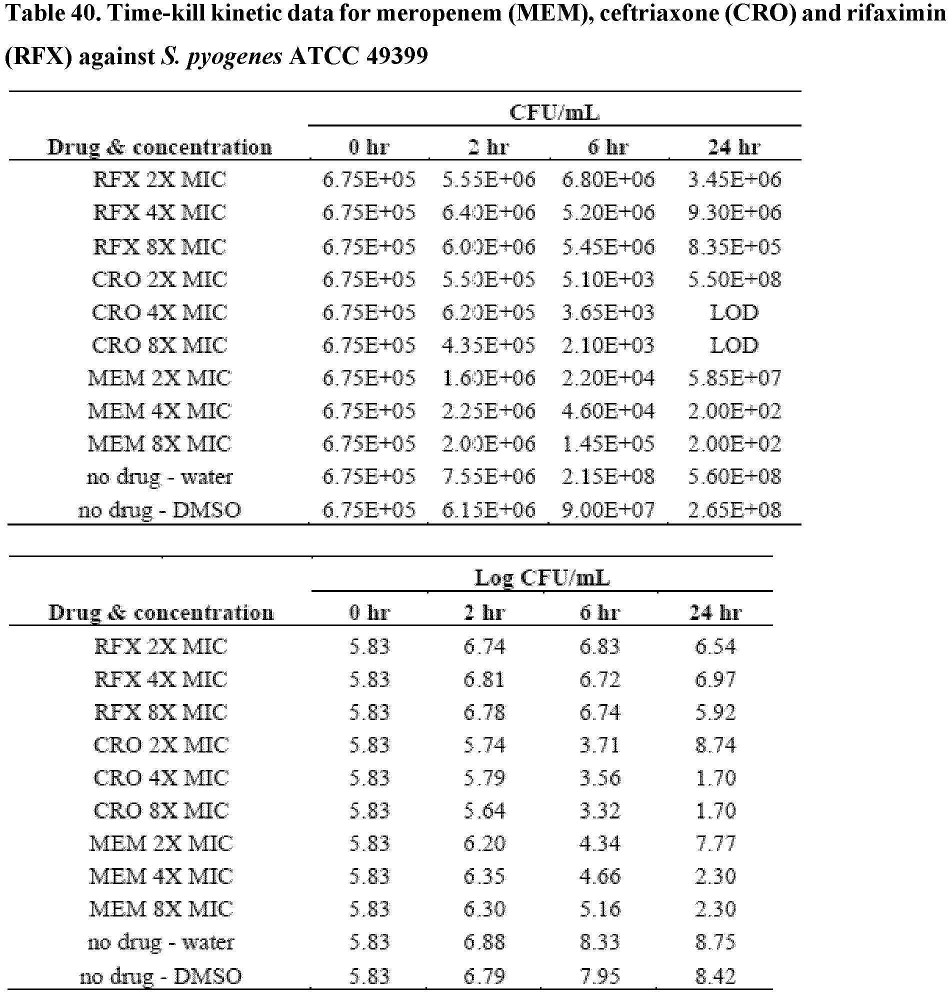

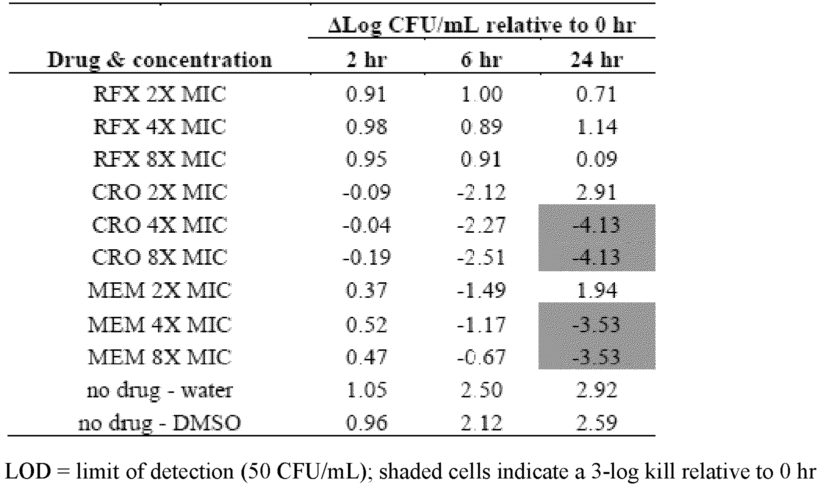

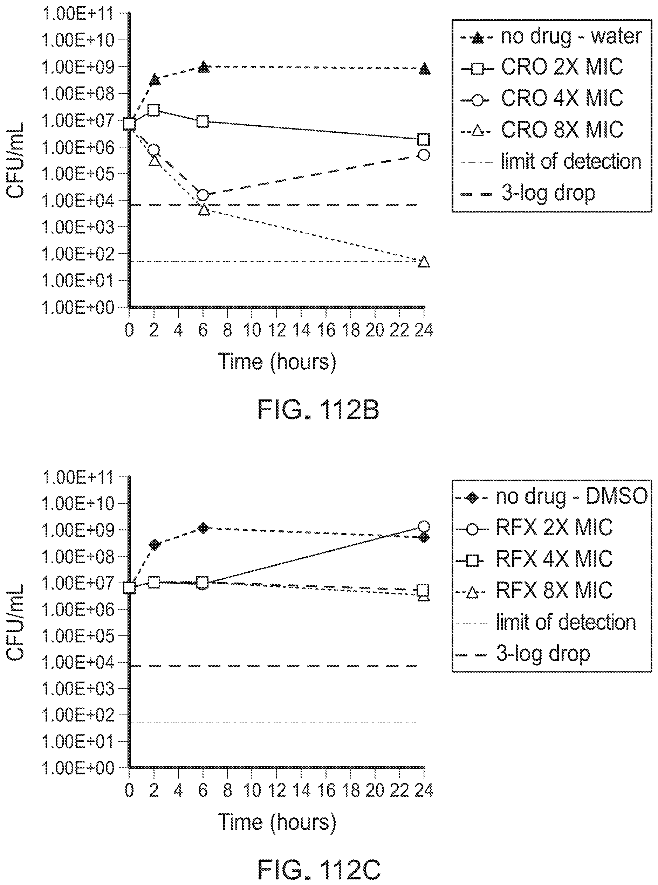

Against *S. agalactiae* ATCC 13813, meropenem, ceftriaxone and rifaximin had MIC values of 0.008, 0.015, and 0.25 µg/mL, respectively. The time-kill kinetics results for these drugs for this strain can be found in FIG. 112 and Table 41. Meropenem demonstrated a bactericidal effect at 24 hr at 8X MIC, as did ceftriaxone (6 and 24 hr), whereas rifaximin had a bacteriostatic effect at 4X and 8X the MIC against this organism.

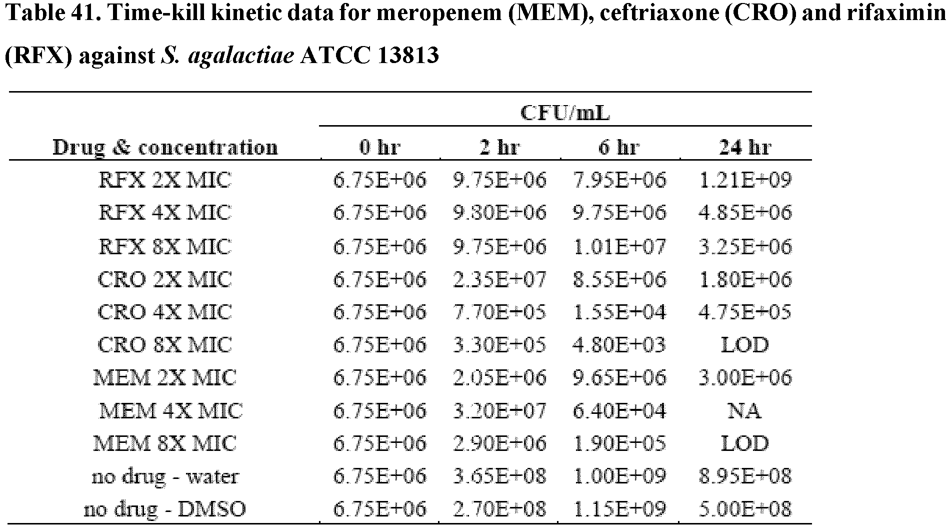

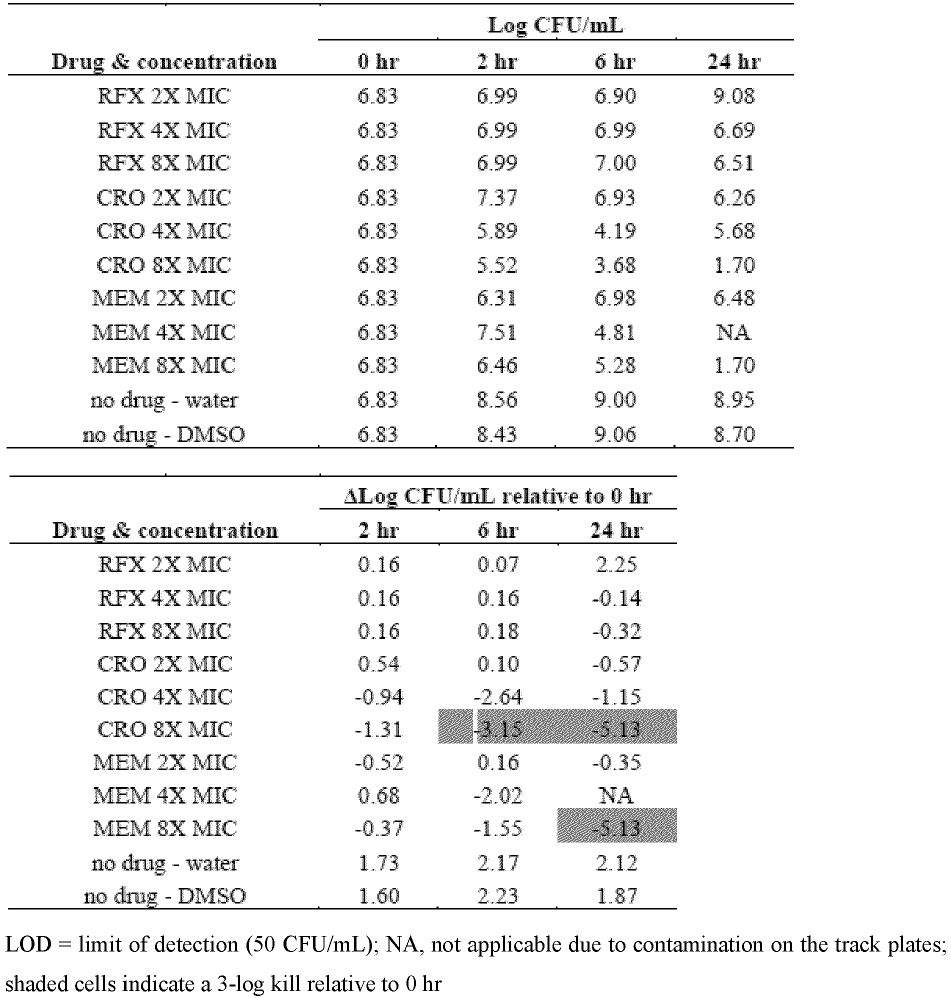

### Bacteroides spp.

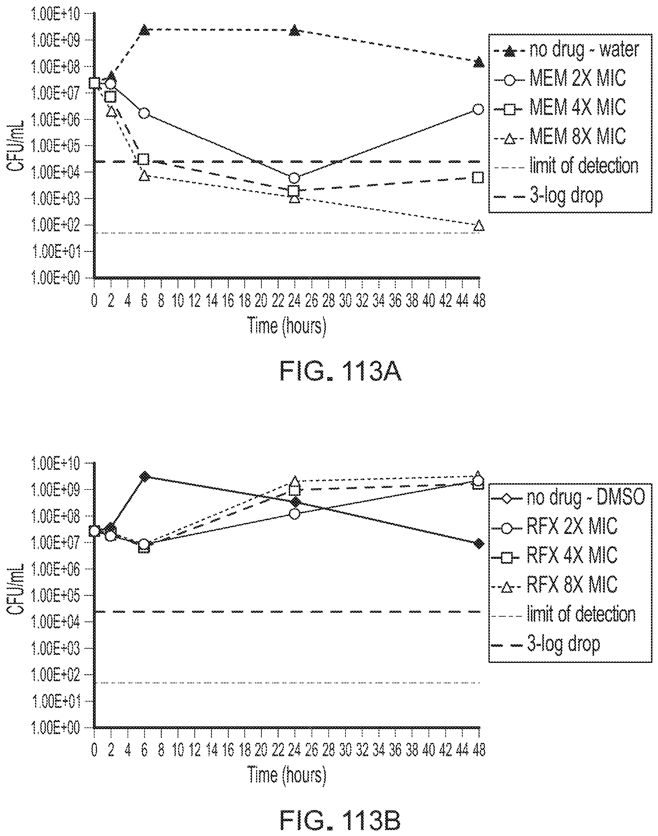

The MIC values for meropenem, ceftriaxone and rifaximin against *B. fragilis* ATCC 25285 were 0.06, 32, and 0.05 µg/mL, respectively. For this organism, the time-kill kinetics and corresponding data are shown in FIG. 113 and Table 42, respectively. Meropenem showed rapid bactericidal activity against this organism at 6, 24 and 48 hr at 8X MIC, 24 and 48 hr at 4X MIC and at 24 hr at 2X MIC (at 2X the MIC regrowth was observed at 48 hr). Rifaximin did not show evidence of killing against this strain. Time-kill kinetics were not conducted for ceftriaxone as the MIC against this organism was 32 µg/mL.

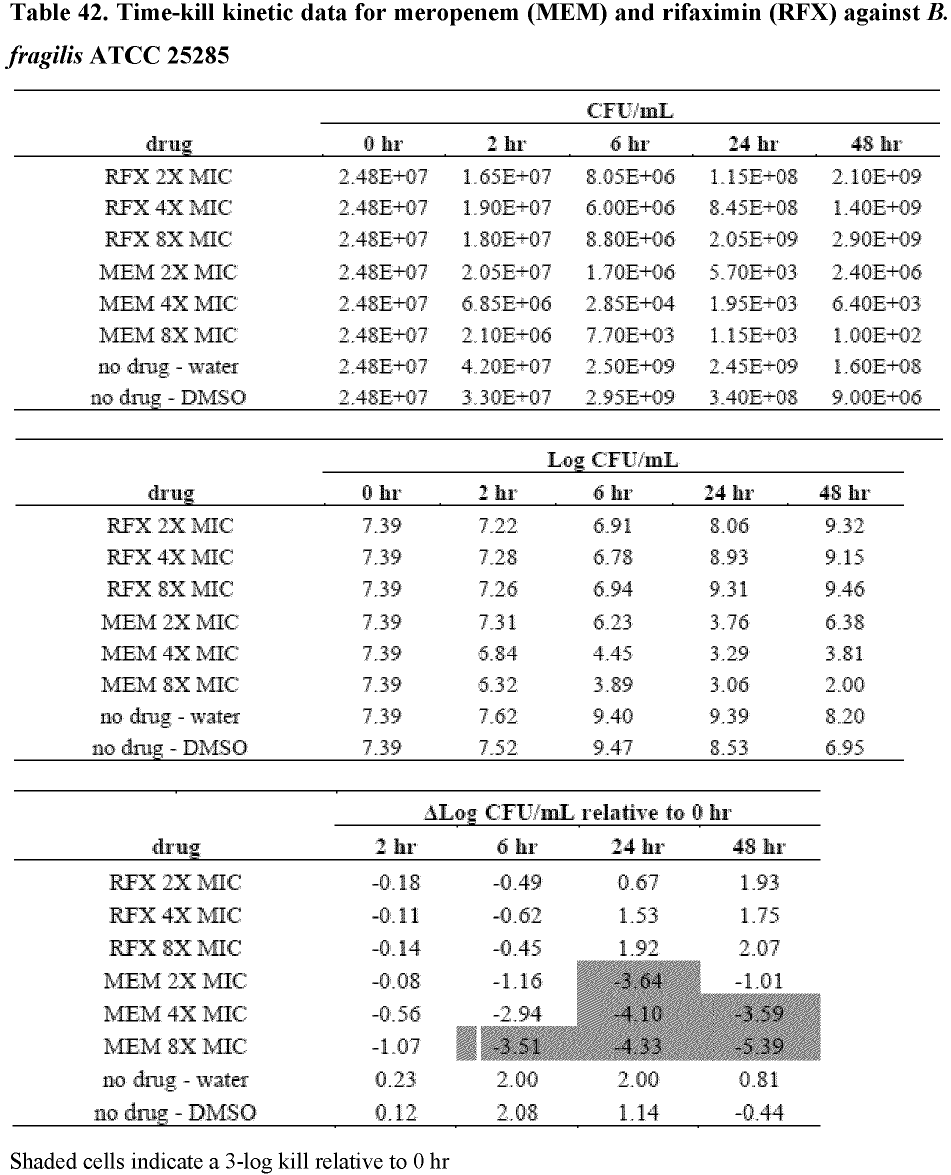

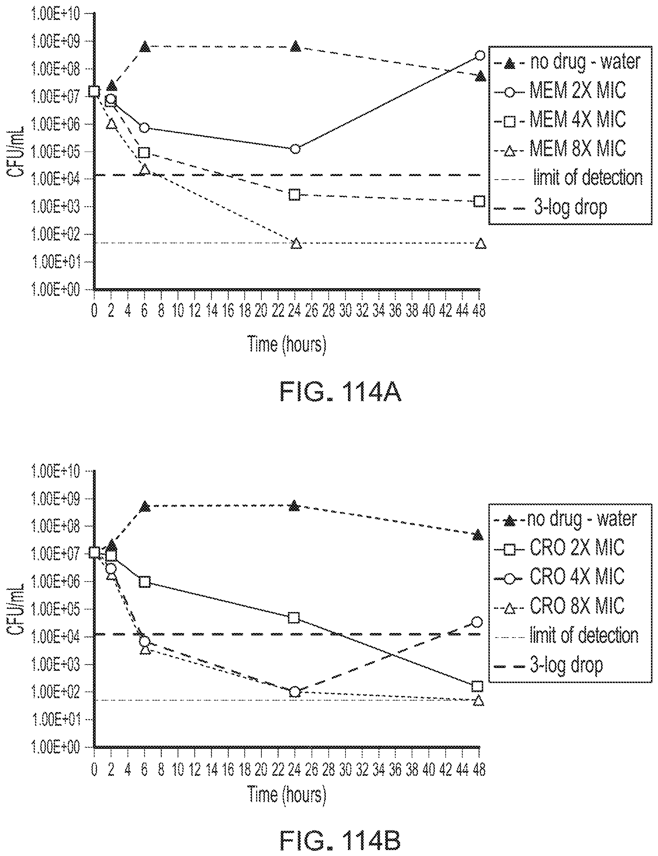

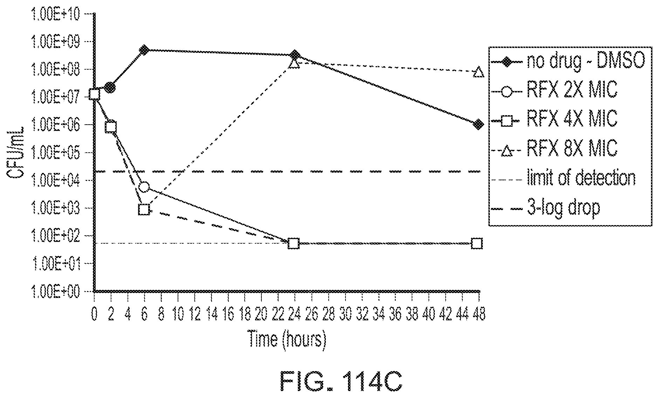

Against the *B. vulgatus* ATCC 8482 isolate, meropenem, ceftriaxone and rifaximin had MIC values of 0.12, 2, and 0.25 µg/mL, respectively. For this organism, the time-kill kinetics and corresponding data are shown in FIG. 114 and Table 43, respectively. Meropenem had a bactericidal effect at 24 and 48 hr at 4X and 8X MIC against this strain. For ceftriaxone, a bactericidal effect was observed at 8X MIC at 6, 24 and 48 hr; at 4X MIC at 6 and 24 hr; and at 2X MIC at 48 hr. Rifaximin was bactericidal against this organism at 8X MIC at 6 hr; and at 2 and 4X MIC at 6, 24 and 48 hr; interestingly at 8X the MIC there was rebound growth at 24 and 48 hr (growth at 48 hr was confirmed to be *B. vulgatus* at 48 hr by MALDI).

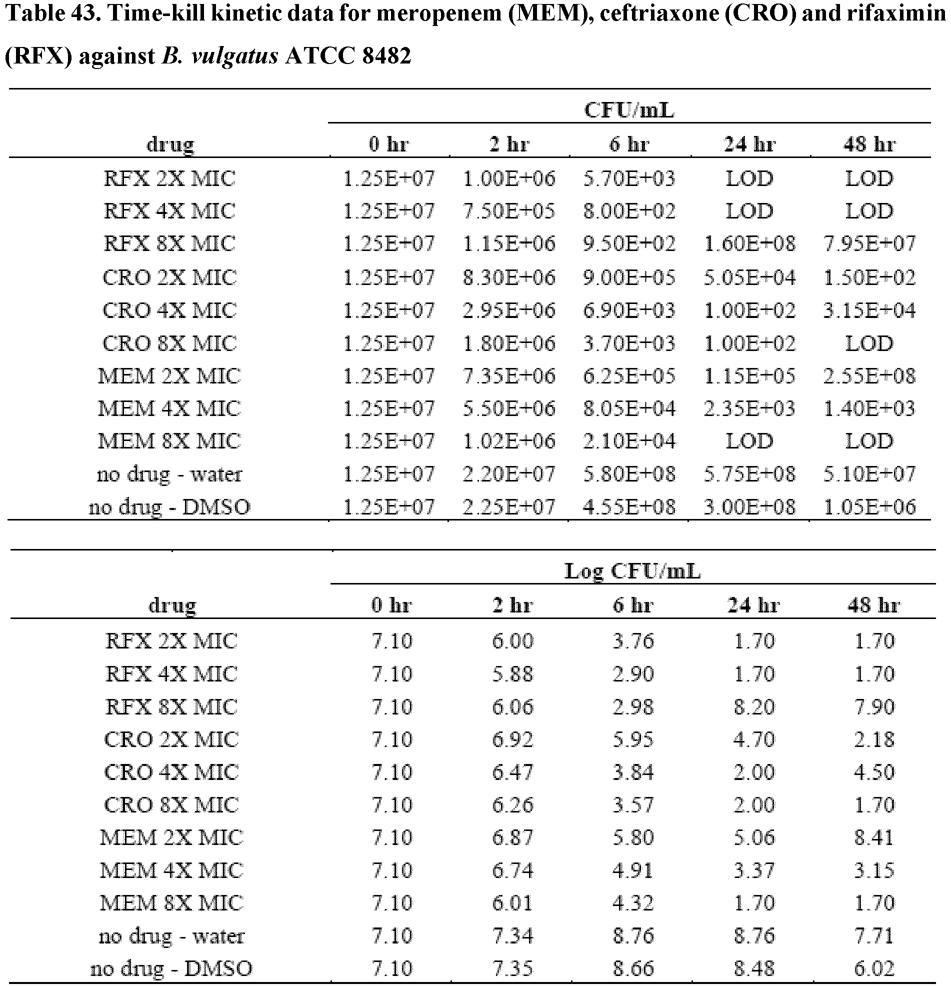

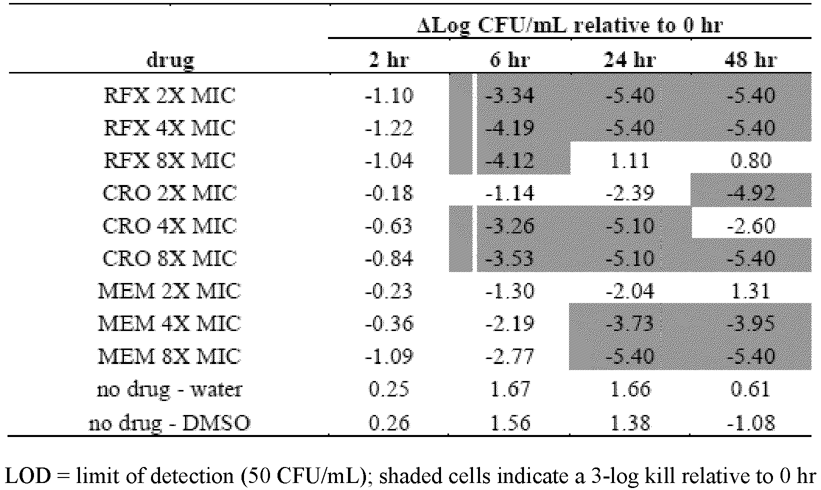

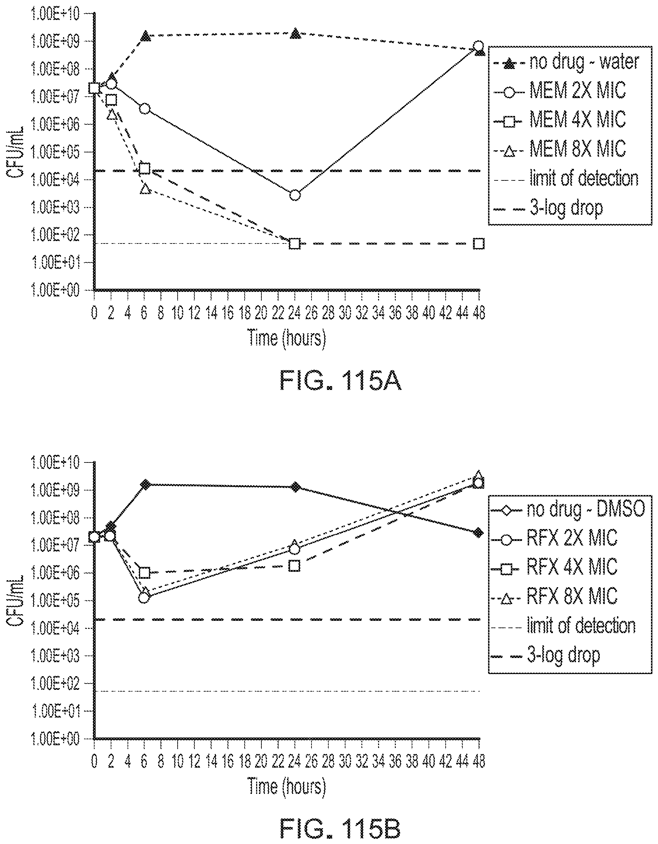

Meropenem, ceftriaxone and rifaximin had MIC values of 0.12, >128, and 1 µg/mL, respectively, against *B. ovatus* MMX 3503. For this organism, the time-kill kinetics and corresponding data are shown in FIG. 115 and Table 44, respectively. Meropenem was bactericidal at 8X MIC at 6, 24 and 48 hr; at 4X MIC at 24 and 48 hr; and at 2X MIC at 24 hr with rebound growth at 48 hr. Rifaximin was observed to have rapid killing (~2 log₁₀ CFU) at 6 hr that rebounded to no-drug control levels at later time points. Time-kill kinetics were not conducted for ceftriaxone as the MIC against this organism was 128 µg/mL.

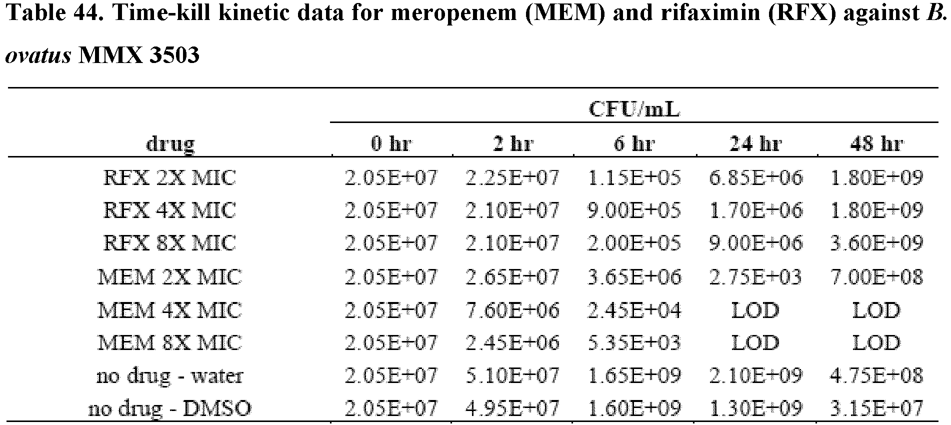

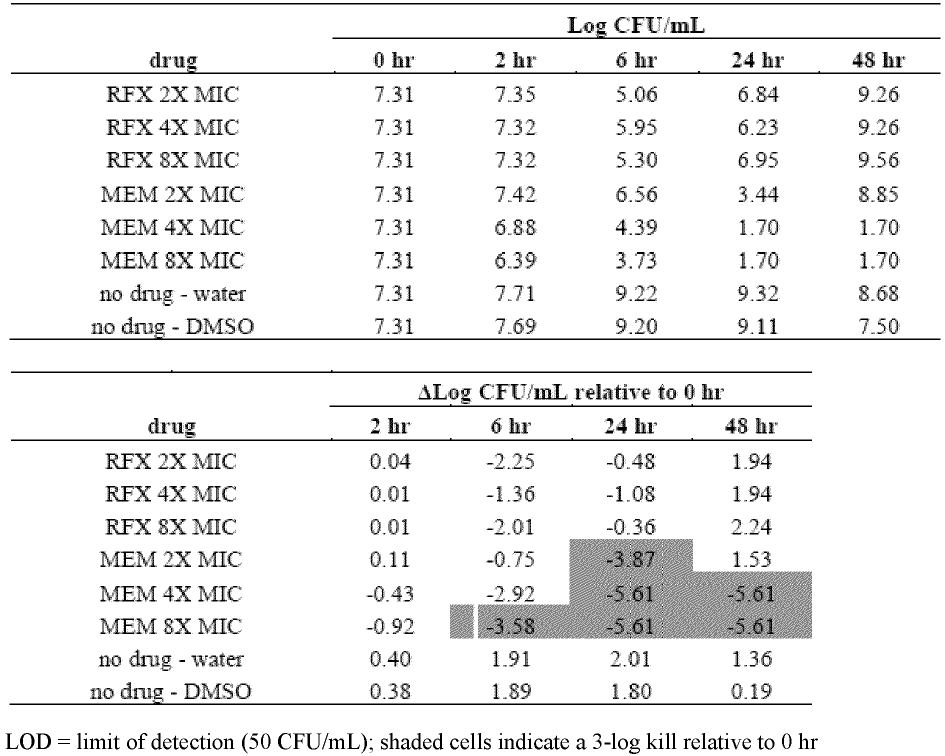

### Summary

Meropenem, ceftriaxone and rifaximin were evaluated for bactericidal activity against three isolates each of *E. coli, Streptococcus* spp. and *Bacteroides* spp., organisms implicated in SIBO. Overall, both meropenem and ceftriaxone demonstrated bactericidal activity against the evaluated isolates whereas bacteriostatic activity and regrowth was typically observed with rifaximin. The bactericidal activity of meropenem and ceftriaxone was surprising, especially as compared to rifaxim, which is generally considered the standard antibiotic for treatment of GI conditions. Not only was it surprising how poorly rifaximin worked, but perhaps more surprising was how well meropenem and ceftriaxone killed the bacterium and provided sustained bactericidal activity, which may be important for regulators, clinicians, and payors.

### Example E: Determination of the spontaneous mutation frequency for antimicrobials against SIBO organisms

The capacity for resistance development to meropenem and ceftriaxone in comparison to rifaximin was determined at multiples of the minimal inhibitory concentration (MIC) for three isolates each of *Escherichia coli, Bacteroides* spp., and *Streptococcus* spp. using the spontaneous mutation frequency assay. Initial values of the MIC were determined by agar dilution in accordance with guidelines from the Clinical and Laboratory Standards Institute (CLSI; (1) "Methods for Dilution Antimicrobial Susceptibility Tests for Bacteria That Grow Aerobically; Approved Standard; 11th ed.," CLSI standard M07, 2018; (2) "Methods for Antimicrobial Susceptibility Testing of Anaerobic Bacteria; Approved Standard-Eighth Edition," CLSI document M11-A8, 2012.).

### Materials and Methods

### Test Compounds

The test agents were supplied by Micromyx (Kalamazoo, MI). The rifaximin, ceftriaxone, and meropenem were prepared in the appropriate solvent at a stock concentration of 100X the highest test concentration for the agar dilution MIC assays. For the spontaneous mutation assay, drug stocks were prepared at 100X the highest final concentration tested. Final DMSO concentrations (v/v) were 1% for the testing of rifaximin in the spontaneous mutation assays.

### Organisms

The test organisms were as follows:
- *E. coli* ATCC 25922
- *E. coli* ATCC 35218
- *E. coli* MMX 1312
- *S. pneumoniae* ATCC 49619
- *S. pyogenes* ATCC 49399
- *S. agalactiae* ATCC 13813
- *B. fragilis* ATCC 25285
- *B. vulgatus* MMX 3483
- *B. ovatus* MMX 3503

Upon initial receipt at Micromyx, the isolates were streaked onto the appropriate agar medium. *E. coli* isolates were incubated overnight at 35 °C. *Streptococcus* spp. isolates were incubated overnight at 35 °C in a CO₂ incubator and anaerobes were incubated in a Bactron Anaerobic Chamber for 48 hours at 35 °C. Using growth from these plates, frozen stocks were prepared in Trypticase Soy broth (Brucella broth for anaerobic organisms) containing 20% sterile glycerol and were stored at -80 °C. During testing, the isolates were cultured from frozen stocks onto Trypticase Soy agar with 5% sheep blood (Becton Dickinson (BD); Sparks, MD) for *E. coli* and *Streptococcus* in accordance with guidelines from CLSI. Anaerobes were cultured on Supplemented Brucella Agar (SBA) with 5% sheep blood (BD). All strains were incubated overnight (48 hours for anaerobic organisms) at 35 °C under the appropriate conditions before testing.

### Test Media

Mueller-Hinton Agar (MHA II; BD; Sparks, MD), MHA II with 5% laked sheep blood (Hemostat) or supplemented Brucella agar (BD; Sparks, MD) were used as the test medium for the agar dilution MIC and spontaneous mutation assay.

### Spontaneous Mutation Assay

Prior to performing the spontaneous mutation assay, the MIC of meropenem, ceftriaxone, and rifaximin was determined for each isolate using the agar dilution method. The resulting MIC value was used to determine the selection concentrations for the spontaneous mutation assay (4X and 8X the agar dilution MIC).

### Preparation of agar plates containing drug

Serial dilutions of meropenem, ceftriaxone, and rifaximin were made in the appropriate diluents. The agar plates were prepared by mixing a ratio of 1.0 mL of concentrated drug to 49.0 mL of the appropriate molten (47 °C) agar; the drug-supplemented agar was mixed, poured into 15-by-150 mm petri dishes and allowed to solidify and dry at room temperature prior to inoculation. Duplicate plates were prepared containing the agents at 4X and 8X the agar dilution MIC. Each plate was allowed to cool at room temperature for at least 1 hr.

In addition, a single 4X MIC and 8X MIC plate (standard small petri plates) were prepared and inoculated with a standard inoculum density according to CLSI (1) for the agar dilution assay. This was to confirm that the drug content of the plates was above the MIC, validating that the stock solution of the drug was prepared properly.

### Inoculation and incubation of plates

The isolates were streaked onto the appropriate agar medium and incubated for 20 hr (aerobes) or 48 hr (Bacteroides under anaerobic conditions). Using a sterile swab, several well-isolated colonies were removed and resuspended in sterile saline at a heavy cell density. Each plate was inoculated with 0.5 mL of the cell suspension (each test concentration was inoculated in duplicate). The target inoculum for each spontaneous mutation plate was 10⁹⁻¹⁰ CFU. In addition, a portion of the inoculum from each isolate was enumerated by making dilutions, spreading on agar and counting colonies.

Once the inoculum was absorbed into the agar, the plates were incubated at 35 °C. Aerobes were examined at 24 and 48 hr, and resulting colonies were counted. Bacteroides were incubated anaerobically for 48 - 72 hr in the anaerobe chamber and colonies were counted.

Additionally, a suspension equivalent to a 0.5 McFarland standard was diluted 1:10 and 2 µL of the resulting suspension was spotted onto the surface of 100 x 15 mm agar plates containing 4X, and 8X the MIC of each test agent. This spot inoculation represented the standard inoculum density of approximately 10⁴ CFU/spot. The purpose of this inoculum was to confirm that supra-inhibitory concentrations of drug evaluated in the assay were inhibitory with an inoculum approximating the density used to determine the initial MIC by agar dilution. These plates were also incubated in ambient air for 48 hr at 35 °C prior to visually assessing growth.

Colony counts from plates used to enumerate the initial inoculum density and from the resulting spontaneous mutation frequency test plates were determined. The inoculum size was calculated as CFU inoculated per plate. The spontaneous mutation frequency was calculated using the counts from duplicate spontaneous mutation assay test plates as follows: $\frac{Average number of colonies on duplicate selection plates}{Total number of cells inoculated per plate}$

If there were no colonies on the antibiotic selection plates, the spontaneous mutation frequency was reported as less than the frequency observed with one colony to indicate that the spontaneous mutation frequency was less than the limit of detection (one CFU). For plates with colonies too numerous to count (TNTC), the frequency was reported as greater than the frequency calculated using a count of 500 CFU as an estimate. The mutation prevention concentration (MPC) was reported as the lowest concentration for which no mutants were selected.

Select isolates were subcultured onto agar containing drug at the selection concentration, were identified by MALDI, and were frozen for broth microdilution MIC testing as described below.

### Antibiogram Characterization of Spontaneous Mutants

To determine whether colonies growing on drug selection plates had elevated MIC values to the selecting antibiotic, susceptibility testing by broth microdilution was conducted on isolated mutants following methodology recommended by CLSI (1), (2), and (3) "Performance Standards for Antimicrobial Susceptibility Testing; Twenty-Eighth Informational Supplement.," CLSI document M100-S28, 2018.

### Test Articles and Comparators

The test agents were supplied by Micromyx. All test agents, manufacturer, lot numbers, storage, and solvents used during testing were as follows:

| **Compounds** | **Supplier** | **Lot Number** | **Solvent/Diluent** | **Conc. of Stock Solution (µg/mL)** |
|---|---|---|---|---|
| Rifaximin | Sigma | BCBT5109 | DMSO | 6400 |
| Ceftriaxone | USP | R07420 | Water | 12800 |
| Meropenem | USP | J0K434 | Water | 800 |
| Metronidazole | Sigma | 095K0693 | DMSO | 3200 |
| Clindamycin | Sigma | 021M1533 | Water | 800 |
| Moxifloxacin | ChemPacific | CHCP062915MHV | Water | 1600 |
| Gentamicin | Sigma-Aldrich | SLBT5354 | Water | 1600 |
| Colistin | Sigma | SLBV1747 | Water | 800 |
| Tetracycline | Sigma-Aldrich | 11.0M1693V | Water | 1600 |
| Penicillin | Sigma | 071M0740V | Water | 800 |
| Azithromycin | USP | R043P0 | water w/ acetic acid dropwise, water | 800 |

### Test Media

Tryptic Soy agar plates containing 5% sheep blood were used to streak aerobes (BD/BBL) and cation-adjusted Mueller Hinton broth (CAMHB II; BD) was used as the medium for testing aerobic bacterial isolates in the assay. *Bacteroides* spp. were streaked on Brucella plates (BD/BBL) containing 5% hemin (Sigma) and 1 mg/mL vitamin K1 (Sigma) and tested in Brucella Broth (BD/BBL) supplemented with 5 mg/mL hemin (Sigma), 1 mg/mL vitamin K (Sigma), and 5% Laked Horse blood (Hemostat).

### Broth Microdilution Susceptibility Testing

Microtiter plates were prepared in accordance with CLSI (1)-(3). To prepare the test plates, automated liquid handlers were used to conduct serial dilutions and liquid transfers, including the Multidrop 384 (Labsystems, Helsinki, Finland), the Biomek 2000 and the Biomek FX (Beckman Coulter, Fullerton CA).

All wells in columns 2 through 12 of a standard 96-well microdilution plate (Costar 3795) were filled with 150 µL of the appropriate diluent. Then, 300 µL of the test agents were added to the wells of column 1 of the plates at 100X the highest final concentration to be tested. Serial two-fold dilutions were made across the rows through column 11 using the Biomek 2000. The wells of column 12 contained no drug and served as the growth control wells. This plate served as the "mother plate" from which selected MIC assay plates or "daughter plates" were made.

For aerobes, the daughter plates were loaded with 190 µL per well of MHB II using the Multidrop 384. The daughter plates were then created using the Biomek FX which transferred
2 µL of drug solution from each well of a mother plate was added to each corresponding well of the daughter plate in a single step. A standardized inoculum of each aerobic test organism was prepared per CLSI methods to equal a 0.5 McFarland standard, followed by a dilution of 1:20 in MHB II. The plates were then inoculated with 10 µL of the diluted inoculum using the Biomek 2000 from low to high drug concentration, resulting in a final concentration of approximately 5 x 10⁵ CFU/mL. An un-inoculated plate was incubated for the purpose of assessing solubility of the drug in the test media.

The plates for the aerobic tests were stacked 3-4 high, covered with a sterile lid on the top plate, and incubated aerobically at 35 °C for 18-20 hr. The MIC was recorded as the lowest concentration of drug that inhibited visible growth of the organism.

Daughter plates for anaerobic organisms were transferred to a Bactron II anaerobic chamber (Sheldon Manufacturing Inc., Cornelius, OR) to reduce for 1 hour prior to inoculation. The inoculum for each organism was prepared in the anaerobic chamber and dispensed into sterile disposable reservoirs. Inoculation was completed using a multichannel pipette to transfer 10 µL of the inoculum into each well of the daughter plate. This yielded a final cell concentration in the daughter plates of approximately 1 x 10⁶ CFU/mL. Plates were stacked in BD Gaspak EZ Anaerobe containers, loaded with three BD Gaspak EZ Anaerobe sachets to maintain an anaerobic atmosphere and a BBL Dry Anaerobic Indicator Strip to monitor anaerobiosis, covered with a lid on the top plate, and incubated at 35 °C for approximately 46-48 hr, after which the MIC was determined as described above.

### Results

The agar dilution MIC values for meropenem, ceftriaxone, and rifaximin against the evaluated organisms are shown in Table 45. Results were within the established QC range for all QC isolates, and rifaximin MIC values were as expected based on prior test results with QC organisms (see Examples C and D, above). Spontaneous mutation rates are discussed for each drug below.

**Table 45. Agar MIC values (µg/mL) for meropenem, ceftriaxone and rifaximin against test organisms**

| **Isolate** | **Meropenem** | **Ceftriaxone** | **Rifaximin** |
|---|---|---|---|
| *E. coli* ATCC 25922 | 0.015 (0.008-0.06)¹ | 0.06 (0.03-0.12) | >4 |
| *E. coli* ATCC 35218 | 0.015 | 0.03 | >4 |
| *E. coli* MMX 1312 | 0.015 | 4 | 4 |
| *B. fragilis* ATCC 25285 | 0.12 (0.03-0.25) | 128 (32-128) | 0.25 |
| *B. vulgatus* ATCC 8482 | 0.12 | 4 | 0.12 |
| *B. ovatus* MMX 3503 | 0.12 | 128 | 0.5 |
| *S*. *pneumoniae* ATCC 49619 | 0.06 (0.03-0.25) | 0.06 (0.03-0.12) | 0.03 |
| *S. pyogenes* ATCC 49399 | 0.008 | 0.03 | 0.12 |
| *S*. *agalactiae* ATCC 13813 | 0.03 | 0.06 | 0.25 |

| | | | |
|---|---|---|---|
| ¹CLSI QC ranges shown in parentheses | | | |

### Meropenem

Meropenem had potent activity across the evaluated test isolates with MIC values of 0.008 - 0.12 µg/mL (see Table 45, above). Meropenem spontaneous mutation frequencies are summarized by organism in Table 46. No *Streptococcus* spp. or *Bacteroides* spp. colonies were selected, while only *E. coli* MMX 1312 demonstrated growth, as explained below.

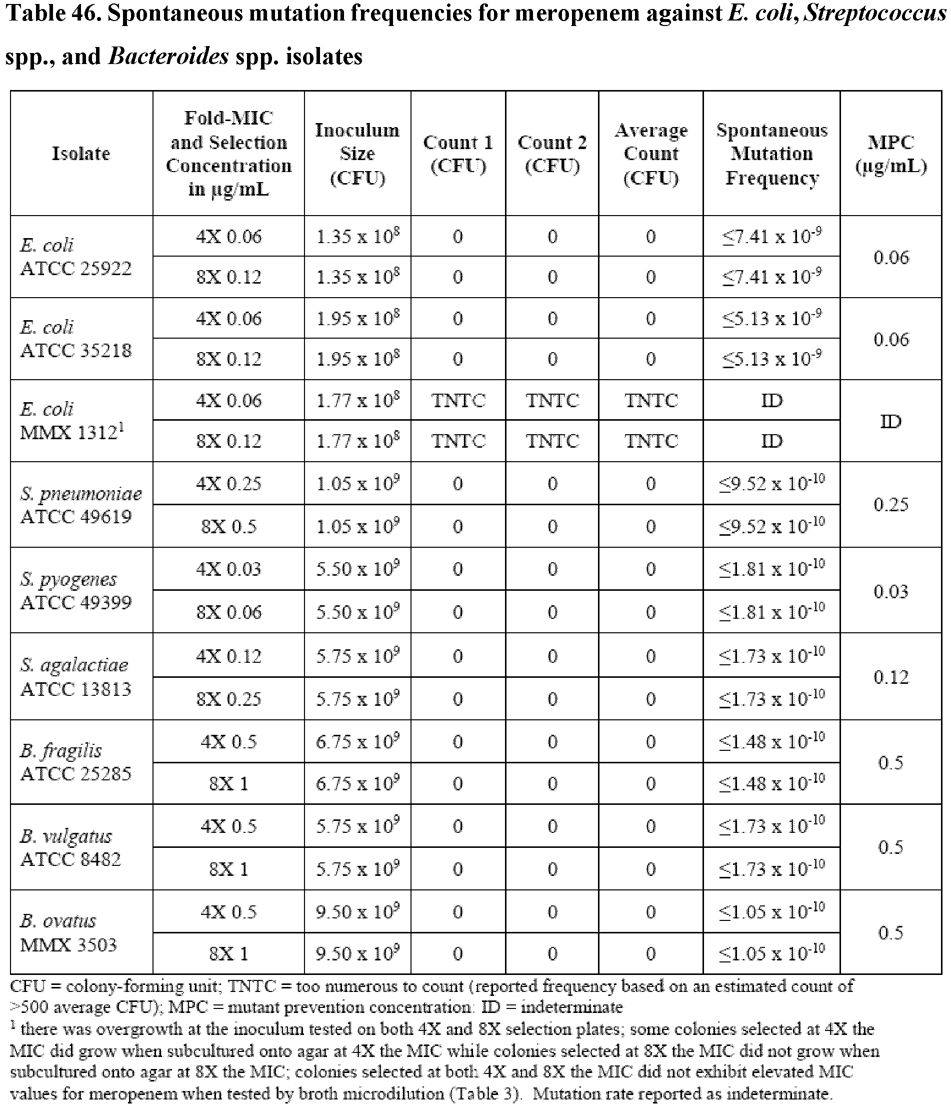

In the case of *E. coli* MMX 1312, colonies were too numerous to count during selection. However, only colonies selected at 4X the MIC were capable of growing upon subculture to fresh agar plates containing 4X the MIC of meropenem. Furthermore, a subset of the colonies selected at 4X and 8X the MIC subjected to broth microdilution MIC testing yielded MIC values consistent with that of the parent strain (Table 47). As a result, these colonies were not true mutants and likely were an artifact due to swamping of the plate with the inoculum; a mutation rate of "indeterminate" was reported for this isolate. For the remaining isolates, spontaneous mutation rates of ≤7.41 x 10⁻⁹ were observed for *E. coli,* ≤9.52 x 10⁻¹⁰ were observed for *Streptococcus* spp., and ≤1.73 x 10⁻¹⁰ were observed for *Bacteroides* spp. MPC values were equivalent to 4X the MIC across the evaluated isolates.

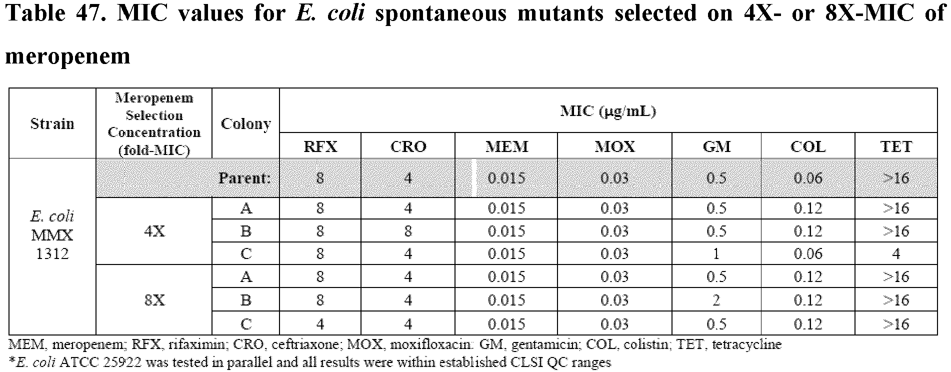

### Ceftriaxone

As shown in Table 45 above, ceftriaxone had potent activity against the *Streptococcus* spp. and *E. coli* with MIC values of 0.03 - 0.06 µg/mL, excluding *E. coli* MMX 1312, where an MIC value of 4 µg/mL was observed. Comparatively less activity was observed against *Bacteroides* spp. with MIC values of 4 - 128 µg/mL.

Ceftriaxone spontaneous mutation frequencies are summarized by organism in Table 48. No *E. coli, Streptococcus* spp., or *B. vulgatus* colonies were selected, with the exception of *E. coli* MMX 1312 and *B. vulgatus* ATCC 8482. Ceftriaxone mutation frequencies were not evaluated for *B. fragilis* and *B. ovatus* isolates due to high initial MIC values (128 µg/mL).

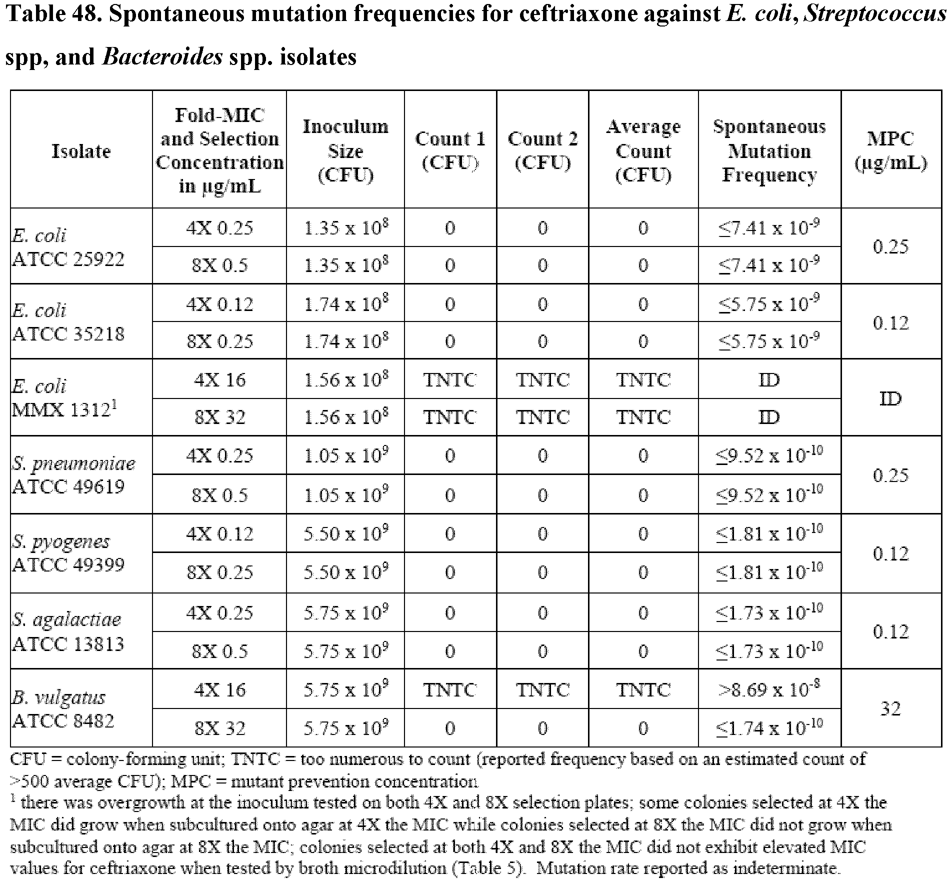

As was the case with meropenem for *E. coli* MMX 1312, colonies were too numerous to count during selection, but only colonies selected at 4X the MIC were capable of growing upon subculture to agar plates at 4X the MIC and a subset of the colonies selected at 4X and 8X the MIC subjected to broth microdilution MIC testing yielded MIC values consistent with that of the parent strain (Table 49). As a result, these colonies were not true mutants and likely were an artifact due to swamping of the plate with the inoculum; a mutation rate of "indeterminate" was reported for this isolate.

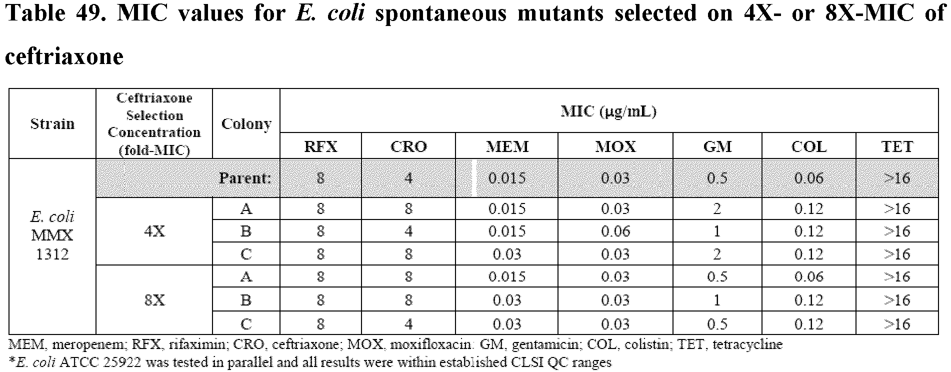

For *B. vulgatus* ATCC 8482, colonies were only observed at 4X the MIC; these colonies grew when subcultured onto agar containing 4X the MIC of ceftriaxone. One of two isolates evaluated for broth microdilution MIC was 4-fold higher than that observed with the parent (Table 50). As a result, for *B. vulgatus* ATCC 8482, a mutation rate of >8.69 x 10⁻⁸ was reported for 4X the MIC, and a mutation rate of ≤1.74 x 10⁻¹⁰ was reported for 8X the MIC, with an MPC of 32 µg/mL.

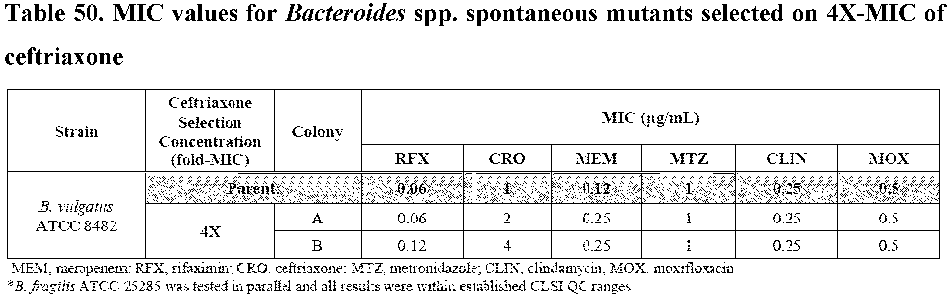

For the remaining isolates, spontaneous mutation rates of ≤7.41 x 10⁻⁹ were observed for *E. coli,* ≤9.52 x 10⁻¹⁰ for *Streptococcus* spp., and overall MPC values of 0.12-0.25 µg/mL.

### Rifaximin

Rifaximin was most active against the evaluated *Streptococcus* spp. and *Bacteroides* spp. with MIC values of 0.03-0.5 µg/mL, while less activity was observed against *E. coli* with MIC values ≥4 µg/mL (Table 45, above).

As summarized in Table 51, regardless of activity, spontaneous mutation rates for rifaximin were high for *E. coli* and *Streptococcus* spp. at both 4X and 8X the MIC. For *E. coli,* mutation frequencies were ≥2.37 x 10⁻⁸ and for *Streptococcus* spp. mutation rates were ≥2.77 x 10⁻⁸. For *Bacteroides* spp., mutation rates were lower and ranged from 8.7 x 10⁻¹⁰-1.19 x 10⁻⁹. Regardless of the organism, colonies selected for further analysis grew readily during subculture onto plates containing rifaximin at the corresponding selection concentration and broth MIC values for select isolates were several-fold higher than those observed with the respective parent strains (Table 52 - *E. coli*; Table 53 *- Streptococcus* spp.; Table 54 - *Bacteroides* spp.). In no instance was an MPC able to be reported as mutants were selected at every concentration evaluated for rifaximin in the study.

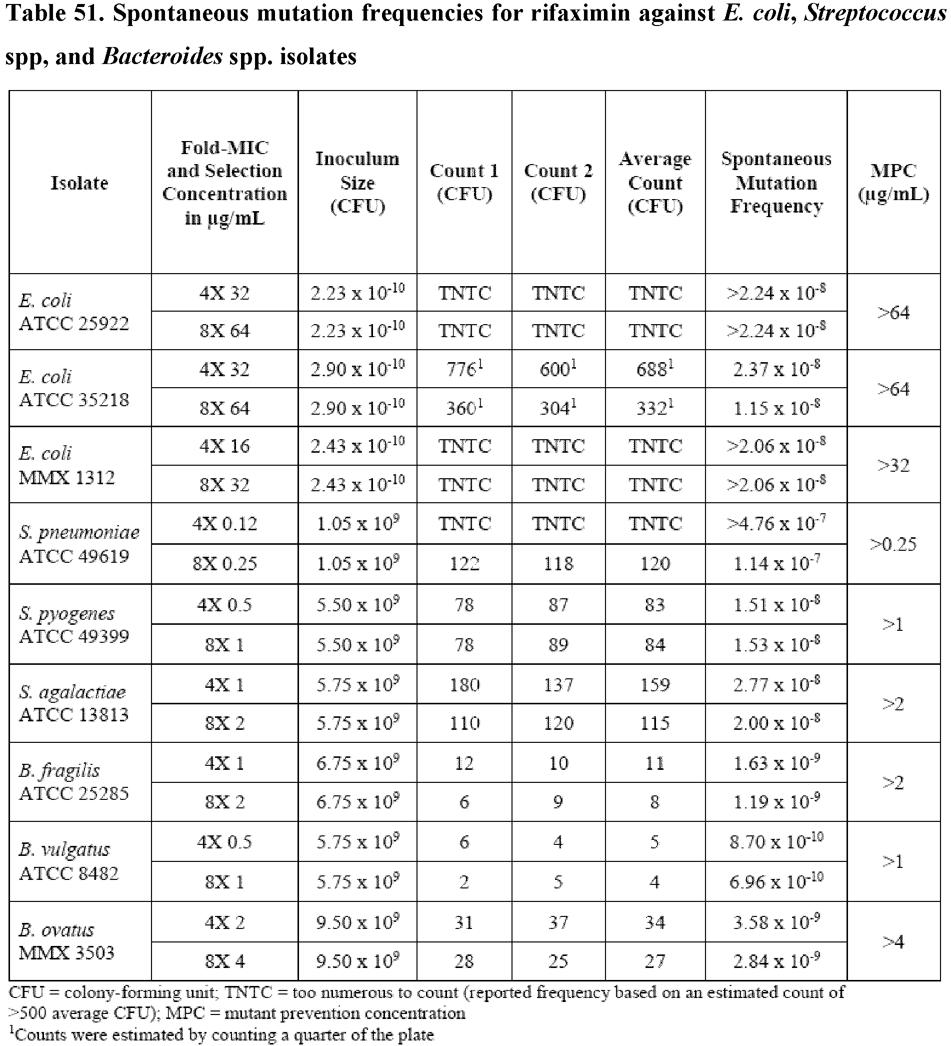

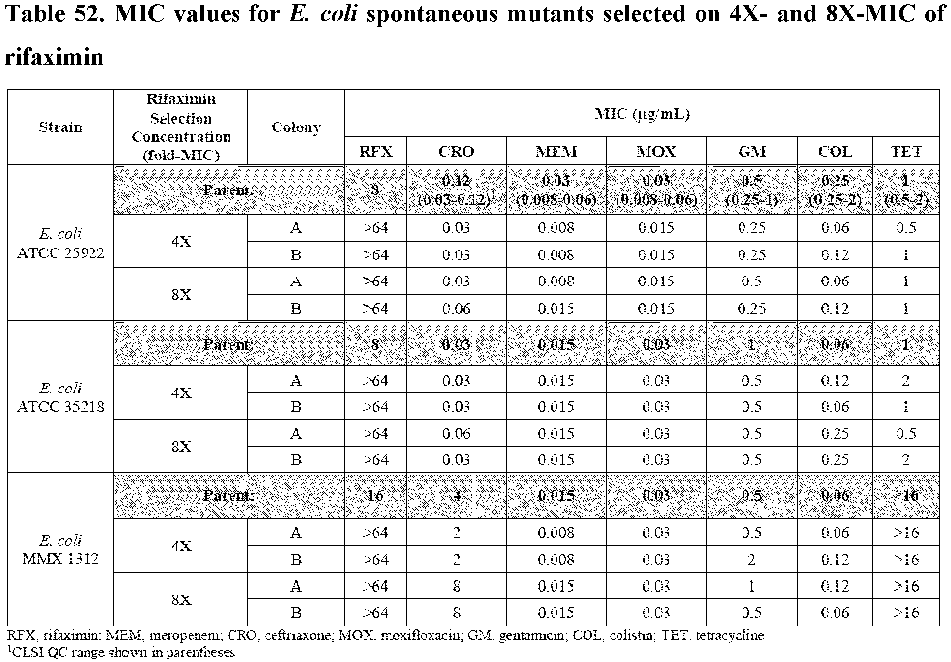

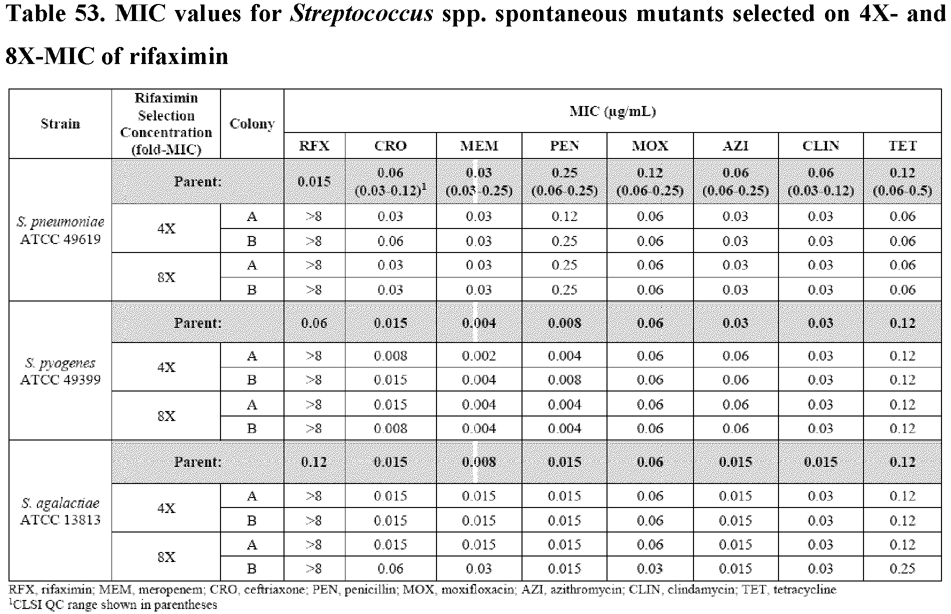

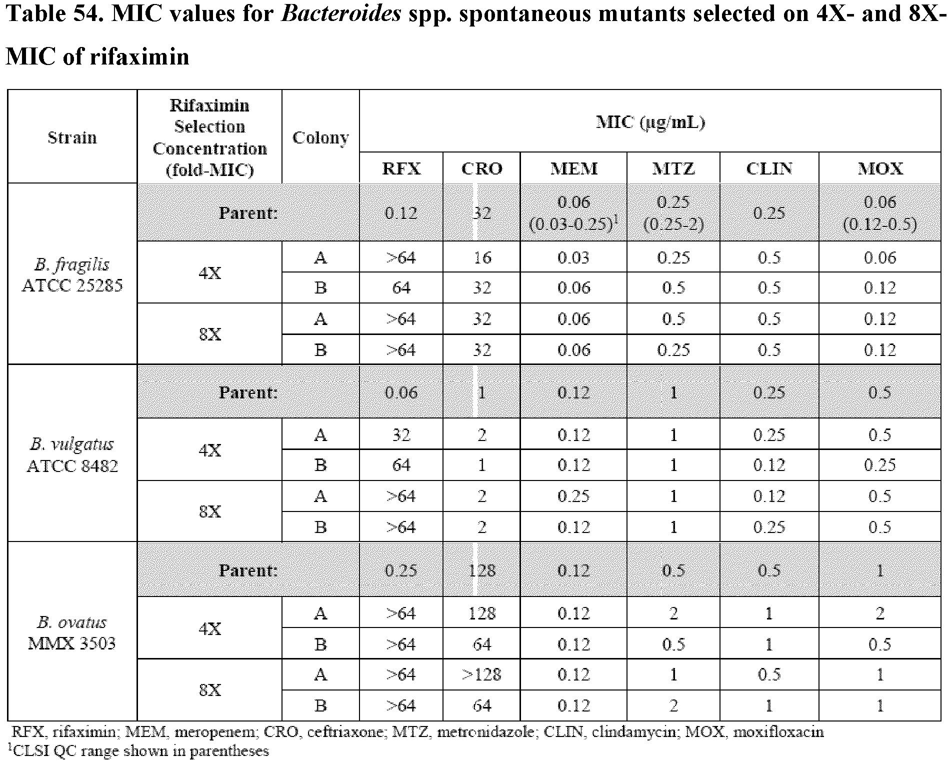

### Summary

In summary, there was little propensity for resistance to develop spontaneously to meropenem or ceftriaxone *in vitro* among SIBO pathogens. In the rare instances where colonies appeared on meropenem or ceftriaxone selection plates, based on further investigation, they were not true mutants as they typically did not grow when subcultured onto drug-containing agar and had MIC values consistent with that of the parent isolate. In contrast, spontaneous mutants with elevated MIC values were readily selected with rifampin at high rates (~10⁻⁸) for *E. coli* and *Streptococcus* spp. and less frequently for *Bacteroides* spp. (10⁻⁹-10⁻¹⁰). This result is consistent with the high mutation frequencies observed among bacteria with this antimicrobial class.

## Claims

1. A pharmaceutical formulation for use in a method for treating small intestinal bacterial overgrowth (SIBO) in a subject in need thereof, the method comprising:
orally administering an effective amount of the pharmaceutical formulation, which comprises an antimicrobial agent, to the subject, thereby treating SIBO in the subject,
wherein the antimicrobial agent is meropenem or ceftriaxone and wherein the pharmaceutical formulation is either: formulated for oral administration as a solid dosage form with an enteric coating; or is administered in an ingestible device and released into the small intestine.

2. The pharmaceutical formulation for the use according to claim 1, wherein the antimicrobial agent exhibits antimicrobial activity against a bacterium implicated in the pathogenesis of SIBO with either a MIC₅₀ or a MIC₉₀ value of 0.001 µg/mL to 64 µg/mL, 0.004 µg/mL to 32 µg/mL, 0.015 µg/mL to 16 µg/mL, 0.03 µg/mL to 8 µg/mL, or 0.5 µg/mL to 2 µg/mL.

3. The pharmaceutical formulation for the use according to claim 1 or claim 2, wherein the antimicrobial agent exhibits antimicrobial activity against a bacterium implicated in the pathogenesis of SIBO with a MIC range of 0.001 µg/mL to 128 µg/mL, 0.001 µg/mL to 64 µg/mL, 0.004 µg/mL to 32 µg/mL, 0.015 µg/mL to 16 µg/mL, 0.03 µg/mL to 8 µg/mL, or 0.5 µg/mL to 2 µg/mL, and/or wherein the bacterium is selected from the group consisting of a gram-positive bacterium, a gram-negative bacterium, an anaerobic bacterium, and combinations thereof.

4. The pharmaceutical formulation for the use according to claim 2 or claim 3 , wherein the bacterium is selected from the group consisting of *Enterobacter aerogenes, Escherichia coli, Klebsiella* spp., *K. oxytoca, K. pneumonia, Proteus mirabilis, Pseudomonas aeruginosa, Staphylococcus aureus, Enterococcus faecalis, Streptococcus* spp., *Streptococcus pyogenes, Streptococcus agalactiae,* Viridans group *Streptococcus, Clostridium* spp., *C*. *sporogenes, C. ramosum, C. innocuum, Prevotella* spp., *P. melanogenica, P. bivia, P. buccae, P. nanceiensis, P. intermedia, P. denticola, P. nigrescens, P. corporis, P. bergensis, P. disiens, Veillonella* spp., *V. parvula, Veillonella* dispr, *V. atypica, Bacteroides fragilis, Bacteroides non-fragilis, B. caccae, B. thetaiotaomicron, B. ovatus, B. vulgatus, B. uniformis, B. stercoris, B. xylanisolvens, B salyersiae, B. intestinalis,* and *B. faecis,* and/or wherein the antimicrobial agent exhibits bactericidal efficacy against the bacterium.

5. The pharmaceutical formulation for the use according to claim 4, wherein the antimicrobial agent exhibits bactericidal efficacy against the bacterium at a concentration of 0.5X MIC to 8X MIC, 1X MIC to 6X MIC, or 2X MIC to 4X MIC, and/or wherein the antimicrobial agent exhibits bactericidal efficacy of at least a 3-log reduction in colony forming units (CFU) per mL in 2 hours, 6 hours, 24 hours, or 48 hours.

6. The pharmaceutical formulation for the use according to any one of claims 4-5, wherein the antimicrobial agent exhibits a minimum 3-log reduction in CFU/mL of *E*. *coli, Streptococcus* spp., *Bacteroides* spp, or any combination thereof, after 24 hours of exposure to the antimicrobial agent, and/or wherein exposure of the bacterium to the antimicrobial agent prevents regrowth of the bacterium.

7. The pharmaceutical formulation for the use according to any one of claims 2-6, wherein the bacterium has a spontaneous mutation frequency of less than 7.45×10⁻⁹, 5.75×10⁻⁹, 5.15 × 10⁻⁹, 9.55× 10⁻¹⁰, 1.85× 10⁻¹⁰, 1.75× 10⁻¹⁰, 1.50× 10⁻¹⁰, or 1.05 ×10⁻¹⁰, and/or wherein the antimicrobial agent exhibits a mutation prevention concentration of 0.01 µg/mL to 32 µg/mL, 0.05 µg/mL to 1 µg/mL, or 0.1 µg/mL to 0.25 µg/mL against the bacterium.

8. The pharmaceutical formulation for the use according to claim 7, wherein the bacterium is selected from the group consisting of *Escherichia coli, Streptococcus* spp., *Bacteroides* spp., and combinations thereof, optionally wherein the bacterium is selected from the group consisting of *E*. *coli, B. fragilis, B. vulgatus, B. ovatus, S. pneumonia, S. pyogenes,* and *S. agalactiae,* and combinations thereof.

9. A pharmaceutical formulation for use in a method of preventing regrowth of a bacterium implicated in the pathogenesis of SIBO in a subject in need thereof, the method comprising:
orally administering an effective amount of the pharmaceutical formulation, which comprises an antimicrobial agent, to the subject, wherein the antimicrobial agent exhibits antimicrobial activity against a bacterium implicated in the pathogenesis of SIBO; and
decreasing the amount of bacterium by at least a 3-log reduction in CFU/mL, as compared to the amount of bacterium at the time of beginning administration of the formulation,
wherein exposure of the bacterium to the antimicrobial agent prevents regrowth of the bacterium, optionally wherein the bacterium is selected from the group consisting of *E*. *coli, Streptococcus* spp., *Bacteroides* spp, or any combination thereof, wherein the antimicrobial agent is selected from meropenem and ceftriaxone and wherein the pharmaceutical formulation is either: formulated for oral administration as a solid dosage form with an enteric coating; or is administered in an ingestible device and released into the small intestine.

10. A pharmaceutical formulation for use in a method of preventing the development of resistance to treatment by an antimicrobial agent of a bacterium implicated in the pathogenesis of SIBO in a subject in need thereof, the method comprising:
orally administering an effective amount of the pharmaceutical formulation, which comprises the antimicrobial agent, to the subject, wherein the antimicrobial agent is meropenem or ceftriaxone, wherein the pharmaceutical formulation is either: formulated for oral administration as a solid dosage form with an enteric coating; or is administered in an ingestible device and released into the small intestine, and wherein the bacterium has a spontaneous mutation frequency of less than 7.45×10⁻⁹, 5.75×10⁻⁹, 5.15× 10⁻⁹, 9.55×10⁻¹⁰, 1.85×10⁻¹⁰, 1.75× 10⁻¹⁰, 1.50×10⁻¹⁰, or 1.05×10⁻¹⁰.

11. The pharmaceutical formulation for the use according to claim 10, wherein the antimicrobial agent exhibits a mutation prevention concentration of 0.01 µg/mL to 32 µg/mL, 0.05 µg/mL to 1 µg/mL, or 0.1 µg/mL to 0.25 µg/mL against the bacterium.

12. The pharmaceutical formulation for the use according to claim 10 or 11, wherein the bacterium is selected from the group consisting of *Escherichia coli, Streptococcus* spp., *Bacteroides* spp., and combinations thereof.

13. The pharmaceutical formulation for the use according to claim 12, wherein the bacterium is selected from the group consisting of *E*. *coli, B. fragilis, B. vulgatus, B. ovatus, S*. *pneumonia, S. pyogenes,* and *S*. *agalactiae,* and combinations thereof.

14. The pharmaceutical formulation for the use according to any one of claims 10-13, wherein the antimicrobial agent is meropenem.

## Patentansprüche

1. Pharmazeutische Formulierung zur Verwendung in einem Verfahren zur Behandlung von bakterieller Überwucherung des Dünndarms (SIBO) bei einem bedürftigen Individuum, wobei das Verfahren Folgendes umfasst:
orales Verabreichen einer wirksamen Menge der pharmazeutischen Formulierung, die ein antimikrobielles Mittel umfasst, an das Individuum, wodurch SIBO beim Individuum behandelt wird,
wobei das antimikrobielle Mittel Meropenem oder Ceftriaxon ist und wobei die pharmazeutische Formulierung entweder: als feste Dosierungsform mit einer magensaftresistenten Beschichtung zur oralen Verabreichung formuliert ist; oder in einer einnehmbaren Vorrichtung verabreicht und im Dünndarm freigesetzt wird.

2. Pharmazeutische Formulierung zur Verwendung nach Anspruch 1, wobei das antimikrobielle Mittel antimikrobielle Aktivität gegen ein Bakterium, das an der Pathogenese von SIBO beteiligt ist, mit entweder einem MIC₅₀- oder einem MIC₉₀-Wert von 0,001 µg/ml bis 64 µg/ml, 0,004 µg/ml bis 32 µg/ml, 0,015 µg/ml bis 16 µg/ml, 0,03 µg/ml bis 8 µg/ml oder 0,5 µg/ml bis 2 µg/ml aufweist.

3. Pharmazeutische Formulierung zur Verwendung nach Anspruch 1 oder Anspruch 2, wobei das antimikrobielle Mittel antimikrobielle Aktivität gegen ein Bakterium, das an der Pathogenese von SIBO beteiligt ist, mit einem MIC-Bereich von 0,001 µg/ml bis 128 µg/ml, 0,001 µg/ml bis 64 µg/ml, 0,004 µg/ml bis 32 µg/ml, 0,015 µg/ml bis 16 µg/ml, 0,03 µg/ml bis 8 µg/ml oder 0,5 bis 2 µg/ml aufweist und/oder wobei das Bakterium aus der aus einem grampositiven Bakterium, einem gramnegativen Bakterium, einem anaeroben Bakterium und Kombinationen davon bestehenden Gruppe ausgewählt ist.

4. Pharmazeutische Formulierung zur Verwendung nach Anspruch 2 oder Anspruch 3, wobei das Bakterium aus der aus Enterobacter aerogenes, Escherichia coli, Klebsiella spp., K. oxytoca, K. pneumonia, Proteus mirabilis, Pseudomonas aeruginosa, Staphylococcus aureus, Enterococcus faecalis, Streptococcus spp., Streptococcus pyogenes, Streptococcus agalactiae, Streptococcus viridans, Clostridium spp., C. sporogenes, C. ramosum, C. innocuum, Prevotella spp., P. melanogenica, P. bivia, P. buccae, P. nanceiensis, P. intermedia, P. denticola, P. nigrescens, P. corporis, P. bergensis, P. disiens, Veillonella spp., V. parvula, Veillonella dispr, V. atypica, Bacteroides fragilis, Bacteroides non-fragilis, B. caccae, B. thetaiotaomicron, B. ovatus, B. vulgatus, B. uniformis, B. stercoris, B. xylanisolvens, B salyersiae, B. intestinalis und B. faecis bestehenden Gruppe ausgewählt ist und/oder wobei das antimikrobielle Mittel bakterizide Wirksamkeit gegen das Bakterium aufweist.

5. Pharmazeutische Formulierung zur Verwendung nach Anspruch 4, wobei das antimikrobielle Mittel in einer Konzentration von 0,5X MIC bis 8X MIC, 1X MIC bis 6X MIC oder 2X MIC bis 4X MIC bakterizide Wirksamkeit gegen das Bakterium aufweist und/oder wobei das antimikrobielle Mittel eine bakterizide Wirksamkeit von zumindest einer 3-log Verringerung der koloniebildenden Einheiten (CFU) pro ml in 2 Stunden, 6 Stunden, 24 Stunden oder 48 Stunden aufweist.

6. Pharmazeutische Formulierung zur Verwendung nach einem der Ansprüche 4 bis 5, wobei das antimikrobielle Mittel mindestens eine 3-log Verringerung der CFU/ml von E. coli, Streptococcus spp., Bacteroides spp. oder einer beliebigen Kombination davon innerhalb von 24 Stunden nach der Exposition gegenüber dem antimikrobiellen Mittel aufweist und/oder wobei die Exposition des Bakteriums gegenüber der antimikrobiellen Mittel das Wiedereinsetzen des Wachstums des Bakteriums verhindert.

7. Pharmazeutische Formulierung zur Verwendung nach einem der Ansprüche 2 bis 6, wobei das Bakterium eine Spontanmutationshäufigkeit von weniger als 7,45 x 10⁻⁹, 5,75 x 10⁻⁹, 5,15 x 10⁻⁹, 9,55 x 10⁻¹⁰, 1,85 x 10⁻¹⁰, 1,75 x 10⁻¹⁰, 1,50 x 10⁻¹⁰ oder 1,05 x 10⁻¹⁰ aufweist und/oder wobei das antimikrobielle Mittel eine Mutationspräventionskonzentration von 0,01 µg/ml bis 32 µg/ml, 0,05 µg/ml bis 1 µg/ml oder 0,1 µg/ml bis 0,25 µg/ml gegen das Bakterium aufweist.

8. Pharmazeutische Formulierung zur Verwendung nach Anspruch 7, wobei das Bakterium aus der aus Escherichia coli, Streptococcus spp., Bacteroides spp. und Kombinationen davon bestehenden Gruppe ausgewählt ist, wobei das Bakterium gegebenenfalls aus der aus E. coli, B. fragilis, B. vulgatus, B. ovatus, S. pneumonia, S. pyogenes und S. agalactiae und Kombinationen davon bestehenden Gruppe ausgewählt ist.

9. Pharmazeutische Formulierung zur Verwendung in einem Verfahren zur Prävention des Wiedereinsetzens des Wachstums eines Bakteriums, das an der Pathogenese von SIBO beteiligt ist, bei einem bedürftigen Individuum, wobei das Verfahren Folgendes umfasst:
orales Verabreichen einer wirksamen Menge der pharmazeutischen Formulierung, die ein antimikrobielles Mittel umfasst, an das Individuum, wobei das antimikrobielle Mittel antimikrobielle Aktivität gegen ein Bakterium aufweist, das an der Pathogenese von SIBO beteiligt ist; und
Verringern der Menge eines Bakteriums um zumindest eine 3-log Verringerung der CFU/ml im Vergleich zur Menge des Bakteriums bei Beginn der Verabreichung der Formulierung,
wobei die Exposition des Bakteriums gegenüber dem antimikrobiellen Mittel das Wiedereinsetzen des Wachstums des Bakteriums verhindert, wobei das Bakterium gegebenenfalls aus der aus E. coli, Streptococcus spp., Bacteroides spp. oder einer beliebigen Kombination davon bestehenden Gruppe ausgewählt ist, wobei das antimikrobielle Mittel aus Meropenem und Ceftriaxon ausgewählt ist und wobei die pharmazeutische Formulierung entweder: als feste Dosierungsform mit einer magensaftresistenten Beschichtung zur oralen Verabreichung formuliert ist; oder in einer einnehmbaren Vorrichtung verabreicht und im Dünndarm freigesetzt wird.

10. Pharmazeutische Formulierung zur Verwendung in einem Verfahren zur Prävention der Entwicklung von Resistenz eines Bakteriums, das an der Pathogenese von SIBO beteiligt ist, gegen eine Behandlung mit einem antimikrobiellen Mittel bei einem bedürftigen Individuum, wobei das Verfahren Folgendes umfasst:
orales Verabreichen einer wirksamen Menge der pharmazeutischen Formulierung, die das antimikrobielle Mittel umfasst, an das Individuum, wobei das antimikrobielle Mittel Meropenem oder Ceftriaxon ist, wobei die pharmazeutische Formulierung entweder: als feste Dosierungsform mit einer magensaftresistenten Beschichtung zur oralen Verabreichung formuliert ist; oder in einer einnehmbaren Vorrichtung verabreicht und im Dünndarm freigesetzt wird, und wobei das Bakterium eine Spontanmutationshäufigkeit von weniger als 7,45 x 10⁻⁹, 5,75 x 10⁻⁹, 5,15 x 10⁻⁹, 9,55 x 10⁻¹⁰, 1,85 x 10⁻¹⁰, 1,75 x 10⁻¹⁰, 1,50 x 10⁻¹⁰ oder 1,05 x 10⁻¹⁰ aufweist.

11. Pharmazeutische Formulierung zur Verwendung nach Anspruch 10, wobei das antimikrobielle Mittel eine Mutationspräventionskonzentration von 0,01 µg/ml bis 32 µg/ml, 0,05 µg/ml bis 1 µg/ml oder 0,1 µg/ml bis 0,25 µg/ml gegen das Bakterium aufweist.

12. Pharmazeutische Formulierung zur Verwendung nach Anspruch 10 oder 11, wobei das Bakterium aus der aus Escherichia coli, Streptococcus spp., Bacteroides spp. und Kombinationen davon bestehenden Gruppe ausgewählt ist.

13. Pharmazeutische Formulierung zur Verwendung nach Anspruch 12, wobei das Bakterium aus der aus E. coli, B. fragilis, B. vulgatus, B. ovatus, S. pneumonia, S. pyogenes und S. agalactiae und Kombinationen davon bestehenden Gruppe ausgewählt ist.

14. Pharmazeutische Formulierung zur Verwendung nach einem der Ansprüche 10 bis 13, wobei das antimikrobielle Mittel Meropenem ist.

## Revendications

1. Formulation pharmaceutique pour utilisation dans un procédé de traitement de la prolifération bactérienne de l'intestin grêle (SIBO) chez un sujet qui en a besoin, le procédé comprenant les étapes consistant à :
administrer au sujet par voie orale une quantité efficace de la formulation pharmaceutique, qui comprend un agent antimicrobien, en traitant ainsi la SIBO chez le sujet,
dans laquelle l'agent antimicrobien est le méropénème ou la ceftriaxone et dans laquelle la formulation pharmaceutique est soit : formulée pour une administration orale sous forme de dose solide avec un enrobage entérique ; soit administrée dans un dispositif pouvant être ingéré et libérée dans l'intestin grêle.

2. Formulation pharmaceutique pour utilisation selon la revendication 1, dans laquelle l'agent antimicrobien présente une activité antimicrobienne à l'encontre d'une bactérie impliquée dans la pathogenèse de SIBO avec une valeur CMI₅₀ ou CMI₉₀ de 0,001 µg/mL à 64 µg/mL, de 0,004 µg/mL à 32 µg/mL, de 0,015 µg/mL à 16 µg/mL, de 0,03 µg/mL à 8 µg/mL, ou de 0,5 µg/mL à 2 µg/mL.

3. Formulation pharmaceutique pour utilisation selon la revendication 1 ou la revendication 2, dans laquelle l'agent antimicrobien présente une activité antimicrobienne à l'encontre d'une bactérie impliquée dans la pathogenèse de SIBO avec une plage de CMI de 0,001 µg/mL à 128 µg/mL, de 0,001 µg/mL à 64 µg/mL, de 0,004 µg/mL à 32 µg/mL, de 0,015 µg/mL à 16 µg/mL, de 0,03 µg/mL à 8 µg/mL, ou de 0,5 µg/mL à 2 µg/mL, et/ou dans laquelle la bactérie est choisie dans le groupe constitué d'une bactérie à Gram positif, d'une bactérie à Gram négatif, d'une bactérie anaérobie, et de combinaisons de celles-ci.

4. Formulation pharmaceutique pour utilisation selon la revendication 2 ou la revendication 3, dans laquelle la bactérie est choisie dans le groupe comprenant *Enterobacter aerogenes, Escherichia coli, Klebsiella* spp., *K oxytoca, K pneumonia, Proteus mirabilis, Pseudomonas aeruginosa, Staphylococcus aureus, Enterococcus faecalis, Streptococcus* spp., *Streptococcus pyogenes, Streptococcus agalactiae,* Streptococcus de *groupe* Viridans, *Clostridium* spp., *C*. *sporogenes, C. ramosum, C. innocuum, Prevotella* spp., *P*. *melanogenica, P. bivia, P. buccae, P. nanceiensis, P*. *intermedia, P. denticola, P. nigrescens, P. corporis, P. bergensis, P*. *disiens, Veillonella* spp., *V parvula, Veillonella* dispr, *V. atypica, Bacteroides fragilis, Bacteroides non-fragilis, B. caccae, B. thetaiotaomicron, B. ovatus, B. vulgatus, B. uniformis, B. stercoris, B. xylanisolvens, B salyersiae, B. intestinalis* et *B. faecis,* et/ou dans laquelle l'agent antimicrobien présente une efficacité bactéricide à l'encontre de la bactérie.

5. Formulation pharmaceutique pour utilisation selon la revendication 4, dans laquelle l'agent antimicrobien présente une efficacité bactéricide à l'encontre de la bactérie à une concentration de 0,5X CMI à 8X CMI, 1X CMI à 6X CMI ou 2X CMI à 4X CMI, et/ou dans laquelle l'agent antimicrobien présente une efficacité bactéricide d'au moins une réduction de 3 log des unités formant colonie (UFC) par mL en 2 heures, 6 heures, 24 heures ou 48 heures.

6. Formulation pharmaceutique pour utilisation selon l'une quelconque des revendications 4 et 5, dans laquelle l'agent antimicrobien présente une réduction minimale de 3 log en UFC/mL d'*E*. *coli, Streptococcus* spp., *Bacteroides* spp, ou toute combinaison de celles-ci, après 24 heures d'exposition à l'agent antimicrobien, et/ou dans laquelle l'exposition de la bactérie à l'agent antimicrobien empêche une nouvelle croissance de la bactérie.

7. Formulation pharmaceutique pour utilisation selon l'une quelconque des revendications 2 à 6, dans laquelle la bactérie présente une fréquence de mutation spontanée inférieure à 7,45 × 10⁻⁹, 5,75 × 10⁻⁹, 5, 15 × 10⁻⁹, 9,55 × 1⁻¹⁰, 1,85 × 10⁻¹⁰,1,75 × 10⁻¹⁰, 1,50 × 10⁻¹⁰ ou 1,05 × 10⁻¹⁰, et/ou dans laquelle l'agent antimicrobien présente une concentration de prévention de mutation de 0,01 µg/mL à 32 µg/mL, de 0,05 ug/mL à 1 µg/mL ou de 0,1 ug/mL à 0,25 µg/mL à l'encontre de la bactérie.

8. Formulation pharmaceutique pour utilisation selon la revendication 7, dans laquelle la bactérie est choisie dans le groupe comprenant *Escherichia coli, Streptococcus* spp., *Bacteroides* spp., et des combinaisons de celles-ci, facultativement dans laquelle la bactérie est choisie dans le groupe comprenant *E. coli, B. fragilis, B. vulgatus, B. ovatus, S. pneumonia, S. pyogenes* et *S. agalactiae,* et des combinaisons de celles-ci.

9. Formulation pharmaceutique pour utilisation dans un procédé de prévention de la nouvelle croissance d'une bactérie impliquée dans la pathogenèse de SIBO chez un sujet qui en a besoin, le procédé comprenant les étapes consistant à :
administrer au sujet par voie orale une quantité efficace de la formulation pharmaceutique qui comprend un agent antimicrobien, dans laquelle l'agent antimicrobien présente une activité antimicrobienne à l'encontre d'une bactérie impliquée dans la pathogenèse de la SIBO ; et
diminuer la quantité de bactérie d'au moins une réduction de 3 log en UFC/mL, par comparaison à la quantité de bactérie au moment du début de l'administration de la formulation,
dans laquelle l'exposition de la bactérie à l'agent antimicrobien empêche une nouvelle croissance de la bactérie, facultativement dans laquelle la bactérie est choisie dans le groupe comprenant *E. coli, Streptococcus* spp., *Bacteroides* spp, ou toute combinaison de ceux-ci, dans laquelle l'agent antimicrobien est choisi parmi le méropénème et la ceftriaxone et dans laquelle la formulation pharmaceutique est soit : formulée pour une administration orale sous forme de dose solide avec un enrobage entérique ; soit administrée dans un dispositif ingérable et libérée dans l'intestin grêle.

10. Formulation pharmaceutique pour utilisation dans un procédé de prévention du développement d'une résistance au traitement par un agent antimicrobien d'une bactérie impliquée dans la pathogenèse de SIBO chez un sujet qui en a besoin, le procédé comprenant l'étape consistant à :
administrer au sujet par voie orale une quantité efficace de la formulation pharmaceutique qui comprend l'agent antimicrobien, dans laquelle l'agent antimicrobien est le méropénème ou la ceftriaxone, dans laquelle la formulation pharmaceutique est soit : formulée pour une administration orale sous forme de dose solide avec un enrobage entérique ; soit administrée dans un dispositif pouvant être ingéré et libérée dans l'intestin grêle, et dans laquelle la bactérie présente une fréquence de mutation spontanée inférieure à 7,45 × 10⁻⁹, 5,75 × 10⁻⁹, 5,15 × 10⁻⁹, 9,55 10⁻¹⁹, 1,85 × 10⁻¹⁰, 1,75 × 10⁻¹⁰, 1,50 × 10⁻¹⁹ ou 1,05 × 10⁻¹⁰.

11. Formulation pharmaceutique pour utilisation selon la revendication 10, dans laquelle l'agent antimicrobien présente une concentration de prévention de mutation de 0,01 µg/mL à 32 µg/mL, de 0,05 µg/mL à 1 µg/mL, ou de 0,1 µg/mL à 0,25 µg/mL à l'encontre de la bactérie.

12. Formulation pharmaceutique pour utilisation selon la revendication 10 ou 11, dans laquelle la bactérie est choisie dans le groupe comprenant *Escherichia coli, Streptococcus* spp., *Bacteroides* spp., et des combinaisons de celles-ci.

13. Formulation pharmaceutique pour utilisation selon la revendication 12, dans laquelle la bactérie est choisie dans le groupe comprenant *E. coli, B. fragilis, B. vulgatus, B. ovatus, S. pneumonia, S. pyogenes* et *S. agalactiae,* et des combinaisons de celles-ci.

14. Formulation pharmaceutique pour utilisation selon l'une quelconque des revendications 10 à 13, dans laquelle l'agent antimicrobien est le méropénème.
